# EUROPEAN PATENT APPLICATION

(11) **EP 4 755 881 A1**
(43) Date of publication of application: **10.06.2026**
(21) Application number: 24848221.8
(22) Date of filing: 29.07.2024
(51) Int. Cl.: C07D 401/14, C07D 401/04, C07D 413/04, A61K 31/4192, A61K 31/44, A61P 1/16, A61P 11/00, A61P 35/00, A61P 37/00

(54) **COMPOUND AS LPAR1 ANTAGONIST, PHARMACEUTICAL COMPOSITION THEREOF AND USE THEREOF**

(30) Priority: 28.07.2023 CN 202310941613; 07.02.2024 CN 202410173619; 30.04.2024 CN 202410539232
(71) Applicant: Wuhan Createrna Science and Technology Co., Ltd., Wuhan, Hubei 430075 (CN)
(72) Inventor: LOU, Jun, Wuhan, Hubei 430075 (CN); ZHAO, Jiaxing, Wuhan, Hubei 430075 (CN); LU, Xiaoqin, Wuhan, Hubei 430075 (CN); ZENG, Jing, Wuhan, Hubei 430075 (CN)
(74) Representative: Murgitroyd & Company
(86) International application number: PCT/CN2024/108122
(87) International publication number: WO 2025/026266

(57) **Abstract**

A compound as an LPAR1 antagonist, a pharmaceutical composition thereof and a use thereof. A compound represented by formula (I), as well as a racemate, stereoisomer, tautomer, nitrogen oxide, or pharmaceutically acceptable salt thereof. The compound of the LPAR1 antagonist has a good inhibitory effect on LPAR1, has excellent pharmacokinetic properties, and has good druggability.

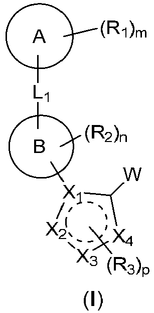

## Description

### SPECIFICATION

The present application claims the priorities of Chinese Patent Application No. 2023109416134 filed on July 28, 2023, Chinese Patent Application No. 2024101736196 filed on February 7, 2024, and Chinese Patent Application No. 2024105392328 filed on April 30, 2024. The contents of the Chinese patent application are incorporated herein by reference in their entirety.

### TECHNICAL FIELD

The present disclosure relates to the field of pharmaceuticals, specifically to a compound as an LPAR1 antagonist, a pharmaceutical composition thereof, and a use thereof.

### BACKGROUND

Lysophosphatidic acid (monoacyl-glycerol-3-phosphate, LPA) is a class of bioactive phospholipids that can be produced from lysophosphatidylcholine (LPC) and mediate a wide range of cellular responses such as proliferation, differentiation, survival, migration, adhesion, invasion, and morphogenesis through a family of seven-transmembrane domain G protein-coupled receptors (GPCRs). These receptors are collectively referred to as LPA receptors (LPARs). To date, six LPA receptors (LPARs) have been identified: LPAR1, LPAR2, LPAR3, LPAR4, LPAR5, and LPAR6. These six LPA receptors are commonly referred to interchangeably as LPAR1-6 or LPA1-6. All six LPA receptors have been characterized and found to exhibit distinct tissue distributions and downstream signaling pathways. LPA receptor-mediated signaling has been shown to influence numerous biological processes such as wound healing, immunity, carcinogenesis, angiogenesis, and neurogenesis. Aberrant upregulation of the LPA pathway has been implicated in various diseases, including cancer, inflammatory diseases, infertility, neuropathic pain, psychiatric and neurodegenerative conditions, atherosclerosis, as well as fibrosis of the skin, kidneys, lungs, and liver. Therefore, LPA receptors may serve as drug targets for various diseases, including cancer, fibrosis, inflammation, pain, and cardiovascular diseases.

Lysophosphatidic acid receptor 1 (LPAR1) is a G protein-coupled receptor that mediates the growth factor-like activity of lysophosphatidic acid (LPA) and plays an important role in the development of cancer, inflammation, fibrotic diseases, neurological diseases, urinary system diseases, and others. For example, siRNA silencing of LPA1 or the use of LPA1 antagonists can lead to reduced tumor burden in bone and soft tissues; LPA enhances the migration of human monocytes and is associated with T cell proliferation and infiltration, while LPA receptor antagonists can protect individuals from infection-induced inflammation; upregulation of LPAR1 activity is associated with fibrosis observed in systemic sclerosis. Studies have shown that in unilateral ureteral obstruction mice, a renal fibrosis animal model, LPA production and LPA1 overexpression occur, and renal fibrosis can be inhibited through LPA1 deficiency or LPA receptor antagonist administration; in bronchoalveolar lavage fluid from idiopathic pulmonary fibrosis patients, LPA concentrations are elevated, and LPA1 is most abundant in fibroblasts, which play a critical role in pulmonary fibrosis, with LPA promoting fibroblast migration; in bleomycin-induced subcutaneous scleroderma model mice, skin fibrosis can be inhibited via LPA1 deficiency or LPA receptor antagonist administration; LPA and LPA1 are also associated with neuropathic pain manifestations; LPAR1 can also be involved in urinary system diseases by contracting urethral resection specimens and prostate specimens, increasing intra-urethral pressure.

Recently, LPAR1 antagonists have been clinically studied in relation to fibrotic diseases, such as idiopathic pulmonary fibrosis (IPF) and systemic sclerosis, but there are no small molecule antagonists of LPAR1 on the market. Therefore, the development of LPAR1 small molecule antagonists with desired selectivity, good biological activity, and strong metabolic stability has positive significance for the treatment of the above diseases.

### SUMMARY

The technical problem to be solved by the present disclosure is the limited variety of small molecule antagonists for LPAR1 in the prior art. To address this, the present disclosure provides a compound as an LPAR1 antagonist, a pharmaceutical composition thereof, and a use thereof. The LPAR1 antagonist compound provided by the present disclosure exhibits good inhibitory effects against LPAR1, excellent pharmacokinetic properties, and good druggability.

The present disclosure provides a compound of formula (I), a racemate thereof, a stereoisomer thereof, a tautomer thereof, an N-oxide thereof, or a pharmaceutically acceptable salt of any one of the foregoing: 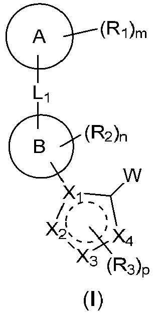
ring A is C₃₋₁₀ cycloalkyl, 3- to 10-membered heterocycloalkyl, or 5- to 10-membered heteroaryl (the C₃₋₁₀ cycloalkyl, 3- to 10-membered heterocycloalkyl, and 5- to 10-membered heteroaryl are each independently optionally substituted with m R¹ substituents);
the definitions of ring B and R₁ are selected from one of the following cases (1), (2), and (3):
case (1): ring B is 5- to 7-membered heteroarylene;
each R₁ is independently OH, COOH, CN, oxo (=O), halogen, NH₂, 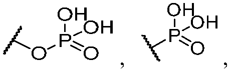 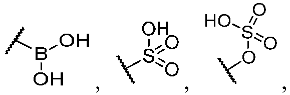 tetrazolyl, C₁₋₆ alkyl, C₁₋₆ alkoxy, -C₁₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, - C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
case (2): ring B is 8- to 10-membered heteroarylene;
each R₁ is independently OH, CN, oxo (=O), halogen, NH₂, 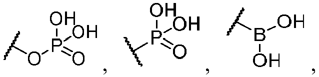 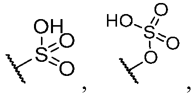 tetrazolyl, C₁₋₆ alkyl, C₁₋₆ alkoxy, -C₁₋₃ alkylene-COOH, -NHC(=O)OC₁₋₆ alkyl, - NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, - C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
case (3): ring B is C₆₋₁₀ arylene;
each R₁ is independently OH, CN, oxo (=O), halogen, NH₂, 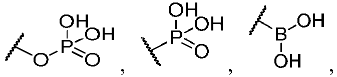 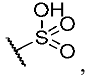 tetrazolyl, C₁₋₆ alkyl, C₁₋₆ alkoxy, -C₂₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, - C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, - C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₂₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
each Rₐ is independently C₁₋₄ alkyl, C₁₋₄ alkoxy, halogen, OH, oxo (=O), CN, or NH₂; when the number of Rₐ is 2 or more, the OH and oxo (=O) are not attached to the same carbon atom;
L₁ is C₂₋₆ alkynylene, -C₂₋₆ alkynylene-O-, or -C₂₋₆ alkynylene-NH-; the C₂₋₆ alkynylene, -C₂₋₆ alkynylene-O-, and -C₂₋₆ alkynylene-NH- are each independently optionally substituted with 1, 2, or 3 R_{b} substituents;
each R_{b} is independently halogen, COOH, or C₁₋₆ alkyl; the C₁₋₆ alkyl is optionally substituted with 1, 2, or 3 COOH or halogen substituents;
each R₂ is independently halogen, NH₂, OH, oxo (=O), CN, C₁₋₄ alkyl, C₁₋₄ alkoxy, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen or deuterium substituents;
X₁, X₂, X₃, and X₄ are each independently C, CH, CH₂, O, S, N, or NH; simultaneously, at least one of X₁, X₂, X₃, or X₄ is independently O, S, N, or NH;
the double line comprising solid and dashed lines represents a single bond or a double bond; simultaneously, 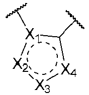 contains at least one double bond;
each R₃ is independently H, C₁₋₄ alkyl, C₁₋₄ alkoxy, halogen, NH₂, OH, oxo (=O), CN, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen or deuterium substituents;
W is 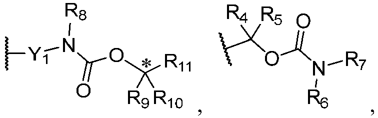 or -(CR₁₂R₁₃)_{q}-Y₂-R₁₅; the carbon atom marked with * is a chiral carbon atom or an achiral carbon atom; when the carbon atom is a chiral carbon atom, the configuration of the chiral carbon atom is R and/or S;
wherein
R₆ and R₇ are each independently H, C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl is optionally substituted with 1, 2, or more R_{c} substituents; the C₃₋₈ cycloalkyl, 3- to 10-membered heterocyclyl, C₆₋₁₀ aryl, and 5- to 10-membered heteroaryl are each independently optionally substituted with 1, 2, or more Rₑ substituents;
each R_{c} is independently deuterium, halogen, -OH, -CN, -COOH, C₁₋₆ alkoxy, C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl, C₁₋₆ alkoxy, C₃₋₈ cycloalkyl, 3- to 10-membered heterocyclyl, C₆₋₁₀ aryl, and 5- to 10-membered heteroaryl are each independently optionally substituted with 1, 2, or more R_{c-1} substituents;
each R_{c-1} is independently deuterium, halogen, -OH, -CN, -COOH, C₁₋₆ alkoxy, or C₁₋₆ alkyl;
R₄, R₅, and R₈ are each independently H, deuterium, halogen, C₁₋₄ alkyl, or C₃₋₆ cycloalkyl; the C₁₋₄ alkyl is optionally substituted with 1, 2, or more Rⱼ substituents;
each Rⱼ is independently deuterium or halogen;
Y₁ is a chemical bond or C₁₋₆ alkylene; the C₁₋₆ alkylene is optionally substituted with 1, 2, or more R_{d} substituents;
each R_{d} is independently deuterium, halogen, or C₃₋₆ cycloalkyl;
R₉ and R₁₀ are each independently H, deuterium, or C₁₋₆ alkyl; the C₁₋₆ alkyl is optionally substituted with 1, 2, 3, or 4 R_{f} substituents;
R₁₁ is C₁₋₆ alkyl, C₆₋₁₀ aryl, 5- to 10-membered heteroaryl, C₃₋₈ cycloalkyl, or 3- to 8-membered heterocycloalkyl; the C₁₋₆ alkyl, C₆₋₁₀ aryl, 5- to 10-membered heteroaryl, C₃₋₈ cycloalkyl, and 3- to 8-membered heterocycloalkyl are each independently optionally substituted with 1, 2, or more R_{g} substituents;
Rₑ, R_{f}, and R_{g} are each independently deuterium, halogen, -OH, -CN, -COOH, C₁₋₆ alkoxy, C₁₋₆ alkyl, or C₃₋₆ cycloalkyl; the C₁₋₆ alkyl, C₁₋₆ alkoxy, and C₃₋₆ cycloalkyl are each independently optionally substituted with 1, 2, or more deuterium or halogen substituents;
Y₂ is a chemical bond, N(R₁₄), -C(=O)N(R₁₄)-, or O;
R₁₂, R₁₃, and R₁₄ are each independently H, deuterium, halogen, or C₁₋₆ alkyl; the C₁₋₆ alkyl is optionally substituted with 1, 2, or more halogen or deuterium substituents;
R₁₅ is C₁₋₆ alkyl, 3- to 10-membered heterocyclyl, or 5- to 10-membered heteroaryl; the C₁₋₆ alkyl, 3- to 10-membered heterocyclyl, and 5- to 10-membered heteroaryl are each independently optionally substituted with 1, 2, or more Rᵢ substituents;
each Rᵢ is independently deuterium, halogen, oxo (=O), OH, COOH, NH₂, CN, C₃₋₆ cycloalkyl, C₁₋₆ alkyl, or C₁₋₆ alkoxy; the C₁₋₆ alkyl, C₃₋₆ cycloalkyl, and C₁₋₆ alkoxy are each optionally substituted with 1, 2, or more Rᵢ₋₁ substituents;
each Rᵢ₋₁ is independently deuterium, halogen, C₁₋₆ alkoxy, or C₃₋₆ cycloalkyl;
m is 0, 1, 2, 3, 4, or 5;
n and p are each independently 0, 1, 2, 3, or 4;
q is 0, 1, 2, or 3;
the heteroatoms in the 3- to 10-membered heterocycloalkyl, 5- to 7-membered heteroarylene, 8- to 10-membered heteroarylene, 3- to 6-membered heterocyclyl, 3- to 10-membered heterocyclyl, 5- to 10-membered heteroaryl, and 3- to 8-membered heterocycloalkyl are each independently selected from 1, 2, or 3 types of N, O, and S; the number of heteroatoms is independently 1, 2, or 3.

In a preferred embodiment, in the compound of formula (I), the racemate thereof, the stereoisomer thereof, the tautomer thereof, the N-oxide thereof, or the pharmaceutically acceptable salt of any one of the foregoing, certain groups have the following definitions, and the definitions of groups not mentioned are as described in any one of the embodiments of the present disclosure (hereinafter referred to as "in a preferred embodiment" for short).

In a preferred embodiment,
ring A is C₃₋₁₀ cycloalkyl or 3- to 10-membered heterocycloalkyl;
in case (1): each R₁ is independently OH, COOH, CN, oxo (=O), halogen, NH₂, 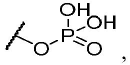 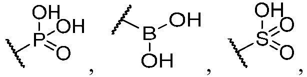 tetrazolyl, C₁₋₆ alkyl, C₁₋₆ alkoxy, -C₁₋₃ alkylene-COOH, -NHC(=O)OC₁₋₆ alkyl, -NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (2): each R₁ is independently OH, CN, oxo (=O), halogen, NH₂, 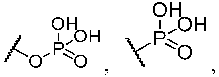 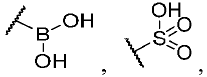 tetrazolyl, C₁₋₆ alkyl, C₁₋₆ alkoxy, -C₁₋₃ alkylene-COOH, -NHC(=O)OC₁₋₆ alkyl, - NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (3): each R₁ is independently OH, CN, oxo (=O), halogen, NH₂, 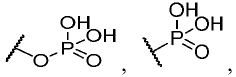 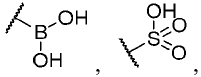 tetrazolyl, C₁₋₆ alkyl, C₁₋₆ alkoxy, -C₂₋₃ alkylene-COOH, -NHC(=O)OC₁₋₆ alkyl, - NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₂₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
R₆ and R₇ are each independently C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl is optionally substituted with 1, 2, or more R_{c} substituents; the C₃₋₈ cycloalkyl, 3- to 10-membered heterocyclyl, C₆₋₁₀ aryl, and 5- to 10-membered heteroaryl are each independently optionally substituted with 1, 2, or more Rₑ substituents.

In a preferred embodiment, ring A is C₃₋₁₀ cycloalkyl or 3- to 10-membered heterocycloalkyl;
in case (1): each R₁ is independently OH, COOH, CN, oxo (=O), halogen, NH₂, 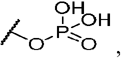 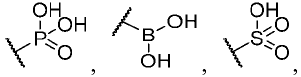 tetrazolyl, C₁₋₆ alkyl, C₁₋₆ alkoxy, -C₁₋₃ alkylene-COOH, -NHC(=O)OC₁₋₆ alkyl, -NHC(=O)C₁₋₆ alkyl, -C(=O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (2): each R₁ is independently OH, CN, oxo (=O), halogen, NH₂, 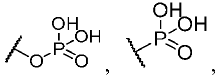 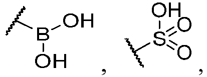 tetrazolyl, C₁₋₆ alkyl, C₁₋₆ alkoxy, -C₁₋₃ alkylene-COOH, -NHC(=O)OC₁₋₆ alkyl, - NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (3): each R₁ is independently OH, CN, oxo (=O), halogen, NH₂, 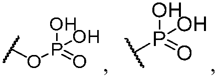 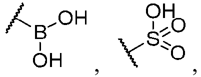 tetrazolyl, C₁₋₆ alkyl, C₁₋₆ alkoxy, -C₂₋₃ alkylene-COOH, -NHC(=O)OC₁₋₆ alkyl, - NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₂₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(-O)NHS(-O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
each R₂ is independently halogen, NH₂, OH, oxo (=O), CN, C₁₋₄ alkyl, C₁₋₄ alkoxy, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen substituents;
each R₃ is independently H, C₁₋₄ alkyl, C₁₋₄ alkoxy, halogen, NH₂, OH, oxo (=O), CN, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen substituents;
R₆ and R₇ are each independently C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl is optionally substituted with 1, 2, or more R_{c} substituents; the C₃₋₈ cycloalkyl, 3- to 10-membered heterocyclyl, C₆₋₁₀ aryl, and 5- to 10-membered heteroaryl are each independently optionally substituted with 1, 2, or more Rₑ substituents;
each R_{c} is independently halogen, -OH, -CN, -COOH, C₁₋₆ alkoxy, C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl, C₁₋₆ alkoxy, C₃₋₈ cycloalkyl, 3- to 10-membered heterocyclyl, C₆₋₁₀ aryl, and 5- to 10-membered heteroaryl are each independently optionally substituted with 1, 2, or more R_{c-1} substituents;
each R_{c-1} is independently halogen, -OH, -CN, -COOH, C₁₋₆ alkoxy, or C₁₋₆ alkyl;
R₉ and R₁₀ are each independently H or C₁₋₆ alkyl; the C₁₋₆ alkyl is optionally substituted with 1, 2, 3, or 4 R_{f} substituents;
Rₑ, R_{f}, and R_{g} are each independently halogen, -OH, -CN, -COOH, C₁₋₆ alkoxy, or C₁₋₆ alkyl; the C₁₋₆ alkyl and C₁₋₆ alkoxy are each independently optionally substituted with 1, 2, or more halogen substituents;
Y₂ is a chemical bond, NR₁₄, or O;
R₁₂, R₁₃, and R₁₄ are each independently H, halogen, or C₁₋₆ alkyl; the C₁₋₆ alkyl is optionally substituted with 1, 2, or more halogen substituents;
R₁₅ is 3- to 10-membered heterocyclyl or 5- to 10-membered heteroaryl; the 3- to 10-membered heterocyclyl and 5- to 10-membered heteroaryl are each independently optionally substituted with 1, 2, or more Rᵢ substituents;
each Rᵢ is independently halogen, oxo (=O), OH, COOH, NH₂, CN, C₃₋₆ cycloalkyl, C₁₋₆ alkyl, or C₁₋₆ alkoxy; the C₁₋₆ alkyl is optionally substituted with 1, 2, or more Rᵢ₋₁ substituents;
each Rᵢ₋₁ is independently halogen, C₁₋₆ alkoxy, or C₃₋₆ cycloalkyl;
q is 0, 1, or 2.

In a preferred embodiment, in R₁, R₂, R₃, R₄, R₅, R₈, R₁₂, R₁₃, R₁₄, Rₐ, R_{b}, R_{c}, R_{c-1}, R_{d}, Rₑ, R_{f}, R_{g}, Rⱼ, Rᵢ, and Rᵢ₋₁, each halogen is independently fluorine, chlorine, bromine, or iodine.

In a preferred embodiment, in R₁, R₆, R₇, R₉, R₁₀, R₁₁, R₁₂, R₁₃, R₁₄, R₁₅, R_{b}, R_{c}, R_{c-1}, Rₑ, R_{f}, R_{g}, and Rᵢ, each C₁₋₆ alkyl is independently methyl, ethyl, *n*-propyl, isopropyl, *n*-butyl, isobutyl, *sec-*butyl*, tert-*butyl*, n*-pentyl, -CH(CH₃)CH₂CH₂CH₃, -CH₂CH(CH₃)CH₂CH₃, -CH₂CH₂CH(CH₃)₂, -CH(C₂H₅)CH₂CH₃, - C(CH₃)₂CH₂CH₃, -CH(CH₃)CH(CH₃)₂, -CH₂C(CH₃)₃, or -CH(CH₃)CH₂CH(CH₃)₂; for example, methyl, ethyl, *n*-propyl, isopropyl, *n*-butyl, isobutyl, *sec*-butyl, *tert*-butyl*, n*-pentyl, -CH(CH₃)CH₂CH₂CH₃, - CH₂CH(CH₃)CH₂CH₃, -CH₂CH₂CH(CH₃)₂, -CH(C₂H₅)CH₂CH₃, -C(CH₃)₂CH₂CH₃, -CH(CH₃)CH(CH₃)₂, or -CH₂C(CH₃)₃.

In a preferred embodiment, in R₂, R₃, R₄, R₅, R₈, and Rₐ, each C₁₋₄ alkyl is independently methyl, ethyl, *n*-propyl, isopropyl, *n*-butyl, isobutyl, *sec*-butyl, or *tert-*butyl*.*

In a preferred embodiment, in R₁, each C₁₋₃ alkylene is independently methylene, -CH₂-CH₂-, - CH(CH₃)-, -CH₂-CH₂-CH₂-, -CH(CH₂-CH₃)-, -C(CH₃)₂-, or -CH₂-CH(CH₃)-, for example, methylene.

In a preferred embodiment, in R₁, each C₂₋₃ alkylene is independently -CH₂-CH₂-, -CH(CH₃)-, - CH₂-CH₂-CH₂-, -CH(CH₂-CH₃)-, -C(CH₃)₂-, or -CH₂-CH(CH₃)-.

In a preferred embodiment, in R₁, Rᵢ, R_{c}, Rₑ, R_{f}, R_{g}, R_{c-1}, and Rᵢ₋₁, each C₁₋₆ alkoxy is independently methoxy, ethoxy, *n*-propoxy, isopropoxy, *n*-butoxy, isobutoxy, *sec*-butoxy, or *tert*-butoxy.

In a preferred embodiment, in R₂, R₃, and Rₐ, each C₁₋₄ alkoxy is independently methoxy, ethoxy, *n*-propoxy, isopropoxy, *n*-butoxy, isobutoxy, *sec*-butoxy, or *tert*-butoxy.

In a preferred embodiment, in R₂, R₃, R₄, R₅, R₈, R_{d}, Rᵢ, Rᵢ₋₁, Rₑ, R_{f}, and R_{g}, each C₃₋₆ cycloalkyl is independently cyclopropyl, cyclobutyl, cyclopentyl, cyclohexyl, or bicyclo[1.1.1]pentyl; for example, cyclopropyl, cyclobutyl, cyclopentyl, or cyclohexyl.

In a preferred embodiment, in R₆, R₇, R₁₁, and R_{c}, each C₃₋₈ cycloalkyl is independently cyclopropyl, cyclobutyl, cyclopentyl, cyclohexyl, bicyclo[1.1.1]pentyl, or spiro[2.2]pentyl.

In a preferred embodiment, in R₆, R₇, R₁₁, and R_{c}, each C₃₋₈ cycloalkyl is independently cyclopropyl, cyclobutyl, cyclopentyl, cyclohexyl, bicyclo[1.1.1]pentyl, bicyclo[2.1.1]hexyl, spiro[2.2]pentyl, or spiro[2.3]hexyl; for example, cyclopropyl, cyclobutyl, cyclopentyl, cyclohexyl, bicyclo[1.1.1]pentyl, or spiro[2.2]pentyl.

In a preferred embodiment, in R₆, R₇, R₁₁, and R_{c}, each C₆₋₁₀ aryl is independently phenyl or naphthyl, for example, phenyl.

In a preferred embodiment, in R₆, R₇, R₁₁, and R_{c}, the heteroatom in each 5- to 10-membered heteroaryl is independently N, O, or S, and the number of the heteroatom may independently be 1 or 2.

In a preferred embodiment, the "more" as used in any one embodiment of the present disclosure independently refers to 3, 4, or 5.

In a preferred embodiment, in R₂ and R₃, the heteroatom in each 3- to 6-membered heterocyclyl is independently N or O, and the number of the heteroatom is independently 1 or 2.

In a preferred embodiment, in R₆, R₇, and R_{c}, each 3- to 10-membered heterocyclyl is independently 3- to 6-membered heterocyclyl, wherein the heteroatom in the 3- to 6-membered heterocyclyl is independently N, O, or S, and the number of the heteroatom is independently 1 or 2.

In a preferred embodiment, in R₁₁, the heteroatom of the 3- to 8-membered heterocycloalkyl in the 3- to 8-membered heterocycloalkyl optionally substituted with 1, 2, or more R_{g} substituents is N, and the number of the heteroatom is independently 1 or 2.

In a preferred embodiment, in ring A, the C₃₋₁₀ cycloalkyl is C₃₋₈ cycloalkyl, for example, cyclopropyl or C₄₋₈ cycloalkyl; for another example, C₃₋₆ cycloalkyl.

In a preferred embodiment, in ring A, the C₃₋₁₀ cycloalkyl is cyclopropyl, cyclobutyl, cyclopentyl, cyclohexyl, cycloheptyl, cyclooctyl, bicyclo[1.1.1]pentyl, spiro[2.3]hexyl, bicyclo[2.1.1]hexyl, bicyclo[3.1.0]hexyl, bicyclo[3.2.0]heptyl, spiro[2.4]heptyl, or bicyclo[2.2.2]octyl, for example, 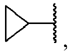 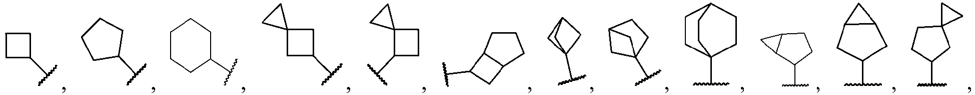 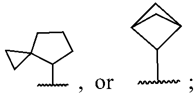 the C₃₋₁₀ cycloalkyl may also be 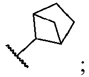 for another example,

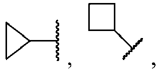

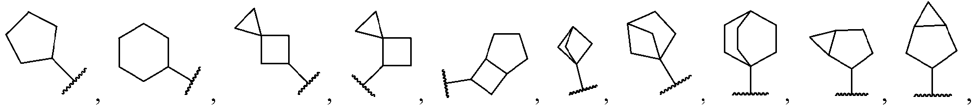

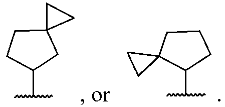

In a preferred embodiment, in ring A, the heteroatom in the 3- to 10-membered heterocycloalkyl is N or O, and the number of the heteroatom may be 1 or 2, for example, the number of the heteroatom is 1.

In a preferred embodiment, in ring A, the 3- to 10-membered heterocycloalkyl is 4- to 8-membered heterocycloalkyl, for example, 4- to 6-membered heterocycloalkyl.

In a preferred embodiment, in ring A, the 3- to 10-membered heterocycloalkyl is oxiranyl, oxetanyl, tetrahydrofuranyl, tetrahydropyranyl, oxepanyl, oxocanyl, aziridinyl, azetidinyl, pyrrolidinyl, piperidinyl, azepanyl, or azocanyl; for example, 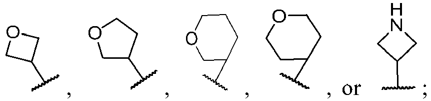 for another example,

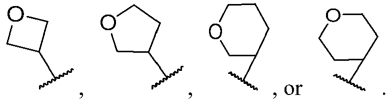

In a preferred embodiment, in ring A, the 5- to 10-membered heteroaryl is 5- to 6-membered heteroaryl, wherein the heteroatom in the 5- to 6-membered heteroaryl may be N, and the number of the heteroatom may be 1 or 2; for example, 5-membered heteroaryl or 6-membered heteroaryl.

In a preferred embodiment, in ring A, the 5- to 10-membered heteroaryl is pyridyl; for example,

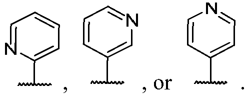

In a preferred embodiment, in R₁, the halogen is fluorine, chlorine, or bromine.

In a preferred embodiment, in R₁, each C₁₋₆ alkyl is independently C₁₋₄ alkyl; for example, methyl, ethyl, or n-propyl.

In a preferred embodiment, in Rₐ, the halogen is fluorine, chlorine, or bromine.

In a preferred embodiment, in R₁, the C₁₋₆ alkyl optionally substituted with 1, 2, or more Rₐ substituents is -CF₃, -CHF₂,

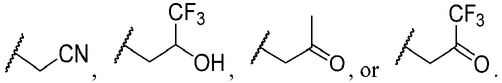

In a preferred embodiment, in R₁, the C₁₋₆ alkoxy in the C₁₋₆ alkoxy optionally substituted with 1, 2, or more Rₐ substituents is independently C₁₋₃ alkoxy; for example, methoxy or ethoxy.

In a preferred embodiment, in R₁, the unsubstituted -C₁₋₃ alkylene-COOH is

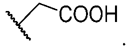

In a preferred embodiment, in R₁, the -C₁₋₃ alkylene-COOH substituted with 1, 2, or more Rₐ substituents is

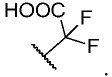

In a preferred embodiment, in R₁, the unsubstituted -NHC(=O)OC₁₋₆ alkyl is

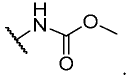

In a preferred embodiment, in R₁, the unsubstituted -C₁₋₃ alkylene-C(=O)NHC(=O)C₁₋₆ alkyl is

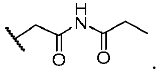

In a preferred embodiment, in R₁, the unsubstituted -C₁₋₃ alkyleneC(=O)NHS(=O)₂C₁₋₆ alkyl is

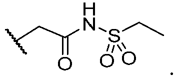

In a preferred embodiment, in R₁, the unsubstituted -C₁₋₃ alkylene-S(=O)₂OH is

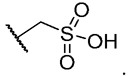

In a preferred embodiment, in R₁, the unsubstituted -C₁₋₃ alkylene-P(=O)(OH)₂ is

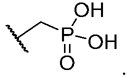

In a preferred embodiment, in R₁, the unsubstituted -C₁₋₃ alkylene-tetrazolyl is

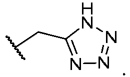

In a preferred embodiment, in R₁, the -NHS(=O)₂OH substituted with 1, 2, or more Rₐ substituents is

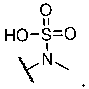

In a preferred embodiment, in R₁, the unsubstituted -C₁₋₃ alkylene-NHS(=O)₂OH is

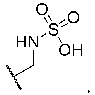

In a preferred embodiment, in R₁, the unsubstituted -C₁₋₃ alkylene-S(=O)₂C₁₋₆ alkyl is

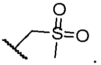

In a preferred embodiment, in L₁, each C₂₋₆ alkynylene is independently ethynylene, 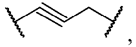 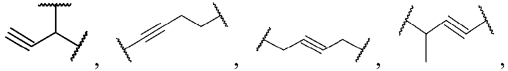 pentynylene, or hexynylene; preferably, each C₂₋₆ alkynylene is independently C₂₋₄ alkynylene, for example,

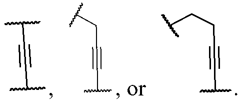

In a preferred embodiment, in R_{b}, the C₁₋₆ alkyl in the C₁₋₆ alkyl optionally substituted with 1, 2, or 3 COOH or halogen substituents is independently C₁₋₃ alkyl; for example, methyl.

In a preferred embodiment, in R_{b}, the C₁₋₆ alkyl substituted with 1, 2, or 3 COOH substituents is

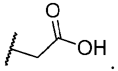

In a preferred embodiment, in L₁, the C₂₋₆ alkynyl substituted with 1, 2, or 3 R_{b} substituents is 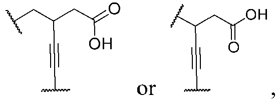 for example, 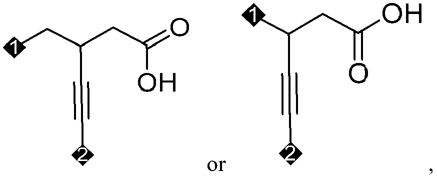 wherein the "1" position is connected to ring A and the "2" position is connected to ring B.

In a preferred embodiment, in ring B, the C₆₋₁₀ arylene is phenylene or naphthylene; for example, phenylene.

In a preferred embodiment, in ring B, the heteroatoms in the 8- to 10-membered heteroarylene and the 5- to 7-membered heteroarylene are N, and the number of heteroatoms is independently 1, 2, or 3.

In a preferred embodiment, in ring B, the 5- to 7-membered heteroarylene is 6-membered heteroarylene, wherein the heteroatom in the 6-membered heteroarylene is N, and the number of the heteroatom is 1, 2, or 3.

In a preferred embodiment, the ring B is pyridinylene, pyrimidinylene, pyridazinylene, pyrazinylene, or triazinylene; for example, 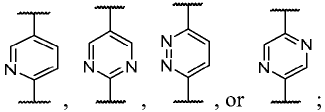 for another example, 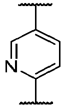 or 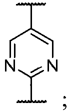 for yet another example,

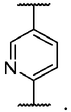

In a preferred embodiment, in R₂, each halogen is independently fluorine, chlorine, or bromine, for example, fluorine or chlorine.

In a preferred embodiment, in R₂, the C₁₋₄ alkyl in the C₁₋₄ alkyl optionally substituted with 1, 2, or more halogen or deuterium substituents is independently methyl or ethyl.

In a preferred embodiment, in R₂, the C₁₋₄ alkyl in the C₁₋₄ alkyl optionally substituted with 1, 2, or more halogen substituents is independently methyl or ethyl.

In a preferred embodiment, in R₂, the C₁₋₄ alkyl substituted with 1, 2, or more halogen or deuterium substituents is -CD₃, -CF₃, -CHF₂, or -CH₂F.

In a preferred embodiment, in R₂, the C₁₋₄ alkyl substituted with 1, 2, or more halogen substituents is -CF₃, -CHF₂, or -CH₂F.

In a preferred embodiment, in R₃, the halogen is fluorine, chlorine, or bromine.

In a preferred embodiment, in R₃, the C₁₋₄ alkyl in the C₁₋₄ alkyl optionally substituted with 1, 2, or more halogen or deuterium substituents is independently methyl or ethyl.

In a preferred embodiment, in R₆ and R₇, the C₁₋₆ alkyl in the C₁₋₆ alkyl optionally substituted with 1, 2, or more R_{c} substituents is independently methyl, ethyl, n-propyl, n-butyl, n-pentyl, 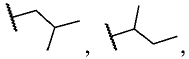 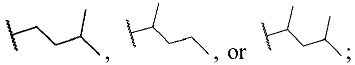 for example, methyl, ethyl, n-propyl, n-butyl, n-pentyl, 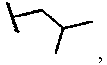 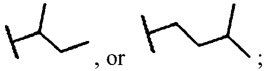 for another example, methyl, ethyl, n-propyl, n-butyl, 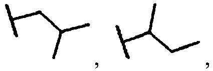 or

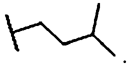

In a preferred embodiment, in R₆ and R₇, the C₃₋₈ cycloalkyl in the C₃₋₈ cycloalkyl optionally substituted with 1, 2, or more Rₑ substituents is independently C₃₋₆ cycloalkyl, for example, cyclopentyl.

In a preferred embodiment, in R₆ and R₇, the 5- to 10-membered heteroaryl in the 5- to 10-membered heteroaryl optionally substituted with 1, 2, or more Rₑ substituents is a monocyclic ring, for example, the 5-to 10-membered heteroaryl is independently 5- to 6-membered heteroaryl; the number of the heteroatom in the 5- to 6-membered heteroaryl may be 1 or 2; for another example, the 5- to 10-membered heteroaryl is independently pyridyl or thiazolyl, for yet another example,

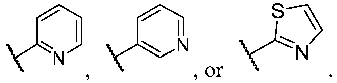

In a preferred embodiment, in R_{c}, the halogen is fluorine, chlorine, or bromine.

In a preferred embodiment, in R_{c}, the C₁₋₆ alkyl in the C₁₋₆ alkyl optionally substituted with 1, 2, or more R_{c-1} substituents is independently methyl or ethyl.

In a preferred embodiment, in R_{c}, the C₃₋₈ cycloalkyl in the C₃₋₈ cycloalkyl optionally substituted with 1, 2, or more R_{c-1} substituents is independently a monocyclic, bridged, or spiro ring.

In a preferred embodiment, in R_{c}, the C₃₋₈ cycloalkyl in the C₃₋₈ cycloalkyl optionally substituted with 1, 2, or more R_{c-1} substituents is independently cyclopropyl, cyclobutyl, cyclopentyl, bicyclo[1.1.1]pentyl, or spiro[2.2]pentyl; the spiro[2.2]pentyl may be 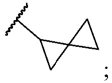 the bicyclo[1.1.1]pentyl may be

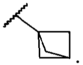

In a preferred embodiment, in R_{c}, the 5- to 10-membered heteroaryl in the 5- to 10-membered heteroaryl optionally substituted with 1, 2, or more R_{c-1} substituents is a monocyclic ring, for example, the 5-to 10-membered heteroaryl is independently 5- to 6-membered heteroaryl, wherein the number of the heteroatom in the 5- to 6-membered heteroaryl may be 1 or 2; for another example, the 5- to 10-membered heteroaryl is independently pyridyl, oxazolyl, or thiazolyl, for yet another example, the 5- to 10-membered heteroaryl is independently

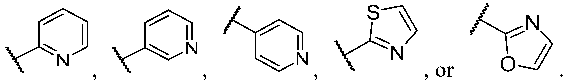

In a preferred embodiment, in R_{c-1}, the halogen is fluorine, chlorine, or bromine.

In a preferred embodiment, in R_{c-1}, the C₁₋₆ alkyl is C₁₋₃ alkyl; for example, methyl.

In a preferred embodiment, in R₆ and R₇, the C₁₋₆ alkyl substituted with 1, 2, or more R_{c} substituents is independently 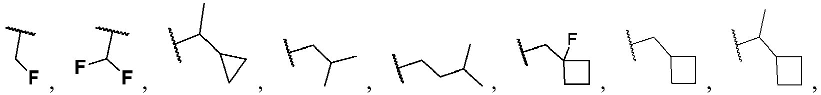 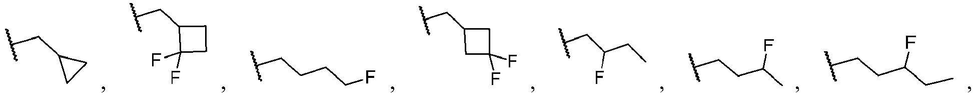 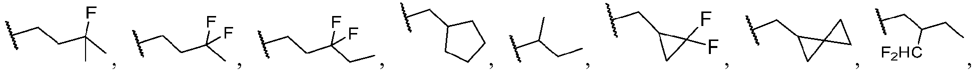 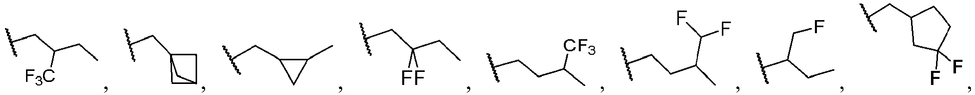 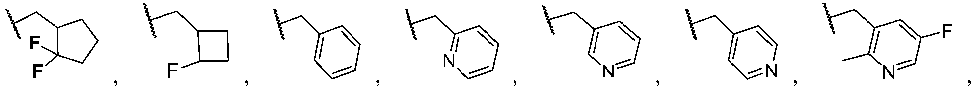 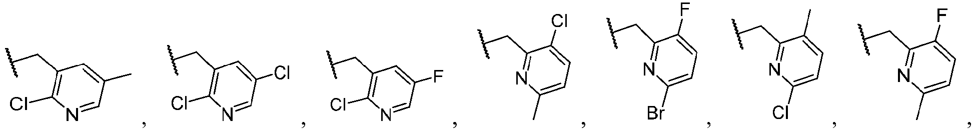 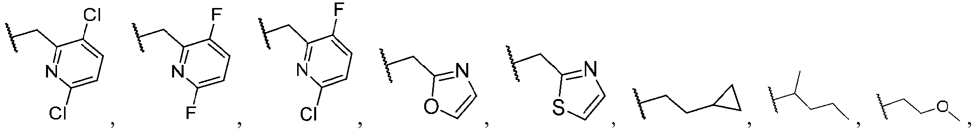 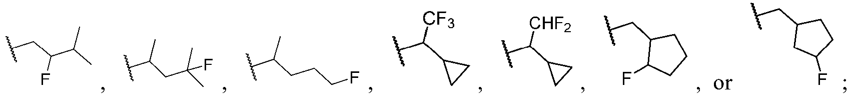 the C₁₋₆ alkyl substituted with 1, 2, or more R_{c} substituents may also be

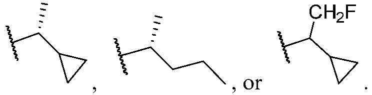

In a preferred embodiment, in Y₁, the C₁₋₆ alkylene in the C₁₋₆ alkylene optionally substituted with 1, 2, or more Rₐ substituents is independently methylene, -CH₂-CH₂-, -CH(CH₃)-, -CH₂-CH₂-CH₂-, -CH(CH₂-CH₃)-, -C(CH₃)₂-, -CH₂-CH(CH₃)-, or butylene, for example, methylene.

In a preferred embodiment, in R₉ and R₁₀, the C₁₋₆ alkyl in the C₁₋₆ alkyl optionally substituted with 1, 2, 3, or 4 R_{f} substituents is independently C₁₋₃ alkyl; for example, methyl.

In a preferred embodiment, in R_{f}, the halogen is fluorine, chlorine, or bromine.

In a preferred embodiment, in R₉ and R₁₀, the C₁₋₆ alkyl substituted with 1, 2, 3, or 4 R_{f} substituents is -CD₃, -CH₂F, or -CHF₂; for example, -CH₂F or -CHF₂.

In a preferred embodiment, in R₁₁, the C₁₋₆ alkyl in the C₁₋₆ alkyl optionally substituted with 1, 2, or more R_{g} substituents is independently C₁₋₄ alkyl; for example, ethyl, n-propyl, n-butyl, 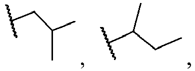 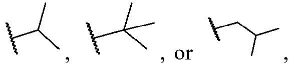 and may also be methyl; for another example, ethyl, n-propyl, n-butyl, or 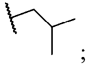 for yet another example, ethyl or n-propyl.

In a preferred embodiment, in R₁₁, the C₃₋₈ cycloalkyl in the C₃₋₈ cycloalkyl optionally substituted with 1, 2, or more R_{g} substituents is independently C₃₋₆ cycloalkyl, wherein the C₃₋₆ cycloalkyl is a monocyclic, spiro, or bridged ring; the C₃₋₈ cycloalkyl may independently be cyclopropyl, cyclobutyl, cyclopentyl, cyclohexyl, bicyclo[1.1.1]pentyl, spiro[2.2]pentyl, spiro[2.3]hexyl, or bicyclo[2.1.1]hexyl; for another example, cyclopropyl, cyclobutyl, cyclopentyl, bicyclo[1.1.1]pentyl, spiro[2.3]hexyl, or bicyclo[2.1.1]hexyl; bicyclo[1.1.1]pentyl may be 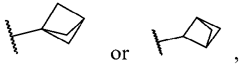 spiro[2.3]hexyl may be 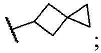 bicyclo[2.1.1]hexyl may be

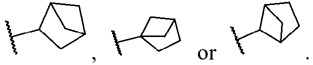

In a preferred embodiment, in R₁₁, the C₃₋₈ cycloalkyl in the C₃₋₈ cycloalkyl optionally substituted with 1, 2, or more R_{g} substituents is independently cyclopropyl, cyclobutyl, cyclopentyl, cyclohexyl, bicyclo[1.1.1]pentyl, or spiro[2.2]pentyl; for another example, cyclopropyl, cyclobutyl, or cyclopentyl.

In a preferred embodiment, in R_{g}, the halogen is fluorine, chlorine, or bromine.

In a preferred embodiment, in R₁₁, the C₁₋₆ alkyl substituted with 1, 2, or more R_{g} substituents is 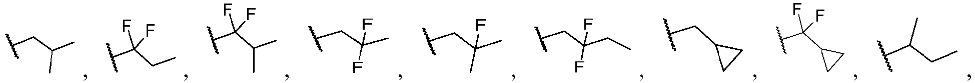 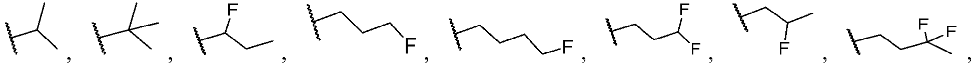 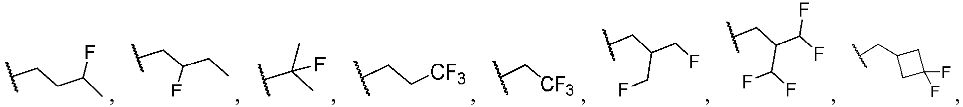 or 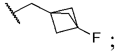 for example,

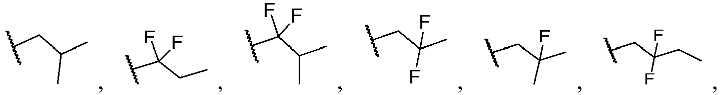

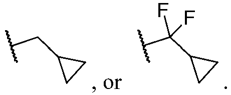

In a preferred embodiment, in R₁₁, the C₃₋₈ cycloalkyl substituted with 1, 2, or more R_{g} substituents is 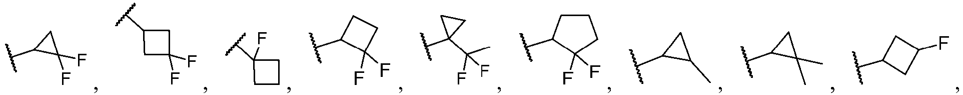 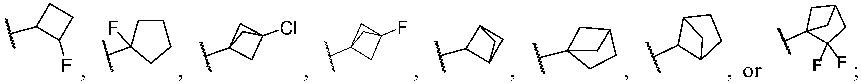 for example, 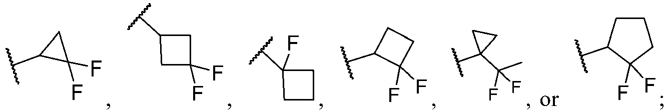 for another example,

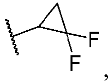

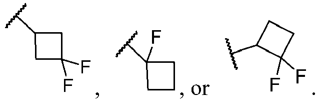

In a preferred embodiment, in R₁₁, the C₆₋₁₀ aryl substituted with 1, 2, or more R_{g} substituents is

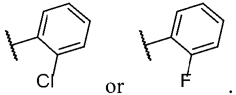

In a preferred embodiment, in R₁₁, the 5- to 10-membered heteroaryl in the 5- to 10-membered heteroaryl optionally substituted with 1, 2, or more R_{g} substituents is independently 5- to 6-membered heteroaryl, wherein the heteroatom in the 5- to 6-membered heteroaryl may be N, and the number of the heteroatom may be 1 or 2; preferably, the 5- to 10-membered heteroaryl is 6-membered heteroaryl; for example, pyridyl, for another example,

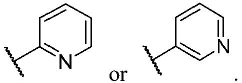

In a preferred embodiment, in R₁₁, the 5- to 10-membered heteroaryl substituted with 1, 2, or more R_{g} substituents is 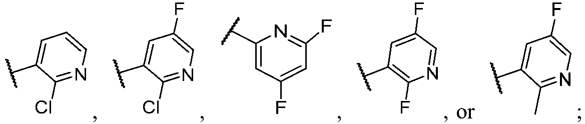 for example, 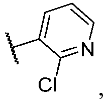 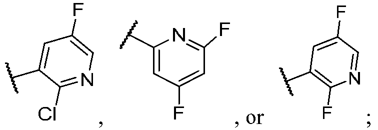 for another example,

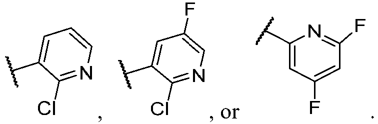

In a preferred embodiment, in R₁₅, the 3- to 10-membered heterocyclyl in the 3- to 10-membered heterocyclyl optionally substituted with 1, 2, or more Rᵢ substituents is independently 3- to 10-membered heterocycloalkyl or 3- to 10-membered heterocycloalkenyl, wherein the number of double bonds in the 3- to 10-membered heterocycloalkenyl is 1 or 2.

In a preferred embodiment, in R₁₅, the heteroatom of the 3- to 10-membered heterocyclyl in the 3-to 10-membered heterocyclyl optionally substituted with 1, 2, or more Rᵢ substituents is independently N or O, and the number of the heteroatom is independently 1 or 2; for example, the heteroatom is N and the number of the heteroatom is 1.

In a preferred embodiment, in R₁₅, the 3- to 10-membered heterocyclyl in the 3- to 10-membered heterocyclyl optionally substituted with 1, 2, or more Rᵢ substituents is independently 3- to 8-membered heterocyclyl, for example, 3- to 6-membered heterocyclyl.

In a preferred embodiment, in R₁₅, the 3- to 10-membered heterocyclyl in the 3- to 10-membered heterocyclyl optionally substituted with 1, 2, or more Rᵢ substituents is independently 3- to 6-membered heterocycloalkyl, for example, azetidinyl, pyrrolidinyl, or piperidinyl, for another example, 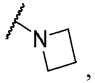 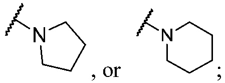 for yet another example,

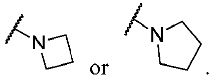

In a preferred embodiment, in R₁₅, the 3- to 10-membered heterocyclyl in the 3- to 10-membered heterocyclyl optionally substituted with 1, 2, or more Rᵢ substituents is independently 3- to 6-membered heterocycloalkenyl, wherein the number of double bonds in the 3- to 6-membered heterocycloalkenyl is 1 or 2; for example, the 3- to 6-membered heterocycloalkenyl is 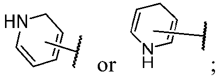 for another example,

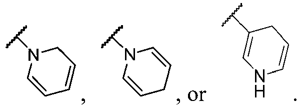

In a preferred embodiment, in R₁₅, the heteroatom of the 5- to 10-membered heteroaryl in the 5- to 10-membered heteroaryl optionally substituted with 1, 2, or more Rᵢ substituents is independently N, O, or S; for example, N; the number of the heteroatom may independently be 1, 2, or 3.

In a preferred embodiment, in R₁₅, the 5- to 10-membered heteroaryl in the 5- to 10-membered heteroaryl optionally substituted with 1, 2, or more Rᵢ substituents is 5- to 6-membered heteroaryl, for example, 6-membered heteroaryl.

In a preferred embodiment, in R₁₅, the 5- to 10-membered heteroaryl in the 5- to 10-membered heteroaryl optionally substituted with 1, 2, or more Rᵢ substituents is independently 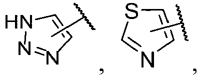 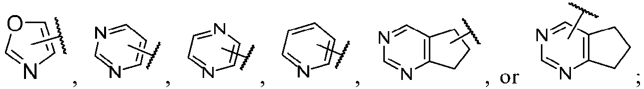 for example, 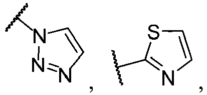 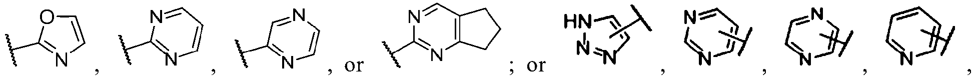 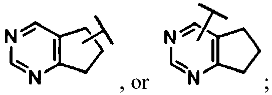 for another example,

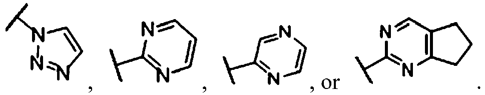

In a preferred embodiment, in Rᵢ, the C₁₋₆ alkyl in the C₁₋₆ alkyl optionally substituted with 1, 2, or more Rᵢ₋₁ substituents is independently C₁₋₄ alkyl; for example, methyl, ethyl, n-propyl, n-butyl, or isopropyl; preferably, C₁₋₃ alkyl; for another example, methyl, ethyl, or isopropyl.

In a preferred embodiment, in Rᵢ, the C₁₋₆ alkoxy is C₁₋₃ alkoxy; for example, ethoxy or isopropoxy.

In a preferred embodiment, in Rᵢ, the C₃₋₆ cycloalkyl optionally substituted with 1, 2, or more Rᵢ₋₁ substituents is cyclopropyl, cyclobutyl, or bicyclo[1.1.1]pentyl; bicyclo[1.1.1]pentyl may be

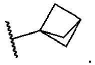

In a preferred embodiment, in Rᵢ, the C₃₋₆ cycloalkyl is cyclobutyl.

In a preferred embodiment, in Rᵢ₋₁, the C₃₋₆ cycloalkyl is cyclopropyl or cyclobutyl.

In a preferred embodiment, in Rᵢ₋₁, the C₁₋₆ alkoxy is methoxy or ethoxy; for example, methoxy.

In a preferred embodiment, in Rᵢ₋₁, the halogen is F.

In a preferred embodiment, the ring A is C₃₋₈ cycloalkyl, 4- to 6-membered oxacycloalkyl, or 4- to 6-membered azacycloalkyl; the number of the heteroatom in the 4- to 6-membered oxacycloalkyl and 4- to 6-membered azacycloalkyl is 1.

In a preferred embodiment, the ring A is C₄₋₈ cycloalkyl or 3- to 10-membered heterocycloalkyl, wherein the heteroatom in the 3- to 10-membered heterocycloalkyl is selected from 1, 2, or 3 types of N, O, and S, and the number of the heteroatom is 1, 2, or 3.

In a preferred embodiment, the ring A is C₃₋₈ cycloalkyl, 4- to 6-membered heterocycloalkyl, or 5-to 6-membered heteroaryl, wherein the heteroatom in the 4- to 6-membered heterocycloalkyl is N or O, and the number of the heteroatom is 1 or 2; the heteroatom in the 5- to 6-membered heteroaryl is N, and the number of the heteroatom is 1 or 2; for example, the ring A is C₃₋₈ cycloalkyl.

In a preferred embodiment, the ring A is 5- to 6-membered heteroaryl, wherein the heteroatom in the 5- to 6-membered heteroaryl is selected from 1, 2, or 3 types of N, O, and S, and the number of the heteroatom is 1, 2, or 3.

In a preferred embodiment, the ring A is cyclopropyl, cyclobutyl, cyclopentyl, cyclohexyl, cycloheptyl, cyclooctyl, bicyclo[1.1.1]pentyl, spiro[2.3]hexyl, bicyclo[2.1.1]hexyl, bicyclo[3.1.0]hexyl, bicyclo[3.2.0]heptyl, spiro[2.4]heptyl, bicyclo[2.2.2]octyl, oxiranyl, oxetanyl, tetrahydrofuranyl, tetrahydropyranyl, oxepanyl, oxocanyl, aziridinyl, azetidinyl, pyrrolidinyl, piperidinyl, azepanyl, azocanyl, or pyridinyl;
preferably, the ring A is cyclopropyl, cyclobutyl, cyclopentyl, cyclohexyl, cycloheptyl, cyclooctyl, spiro[2.3]hexyl, spiro[2.4]heptyl, bicyclo[1.1.1]pentyl, bicyclo[2.1.1]hexyl, bicyclo[3.1.0]hexyl, bicyclo[3.2.0]heptyl, bicyclo[2.2.2]octyl, oxetanyl, tetrahydrofuranyl (oxacyclopentyl), tetrahydropyranyl (oxacyclohexyl), azetidinyl, or pyridinyl;
more preferably, the ring A is 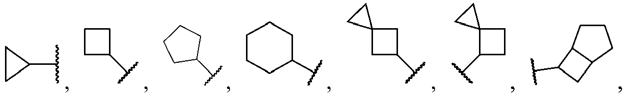 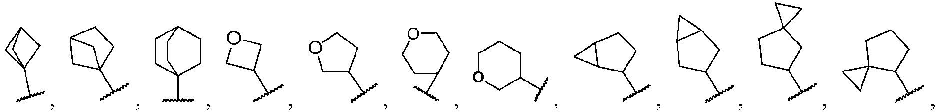 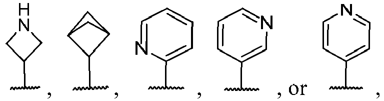 and may also be

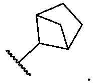

In a preferred embodiment, the ring A is 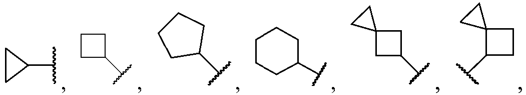 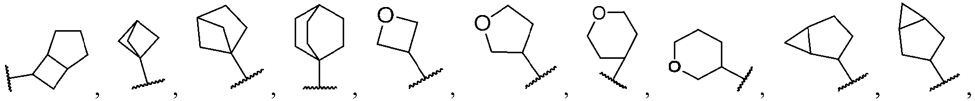 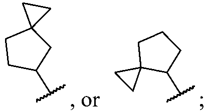 for example, cyclopropyl; preferably, the 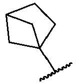 in ring A is

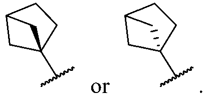

In a preferred embodiment, in case (1), each R₁ is independently OH, COOH, F, Cl, Br, CN, 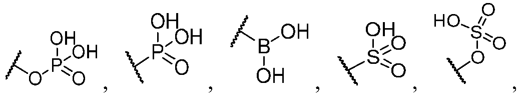 tetrazolyl, C₁₋₆ alkyl, C₁₋₆ alkoxy, -C₁₋₃ alkylene-COOH, -NHC(=O)OC₁₋₃ alkyl, -NHS(=O)₂OH, -C₁₋₃ alkylene-NHS(=O)₂OH, -C₁₋₃ alkylene-C(=O)NHC(=O)C₁₋₃ alkyl, -C₁₋₃ alkylene-C(=O)NHS(=O)₂C₁₋₃ alkyl, -C₁₋₃ alkylene-S(=O)₂OH, -C₁₋₃ alkylene-S(=O)₂C₁₋₆ alkyl, -C₁₋₃ alkylene-P(=O)(OH)₂, -C₁₋₃ alkylene-tetrazolyl, or -C₁₋₃ alkylene-C(=O)C₁₋₃ alkyl;
the C₁₋₆ alkyl, C₁₋₆ alkoxy, -C₁₋₃ alkylene-COOH, -NHC(=O)OC₁₋₃ alkyl, -NHS(=O)₂OH, -C₁₋₃ alkylene-NHS(=O)₂OH, -C₁₋₃ alkylene-C(=O)NHC(=O)C₁₋₃ alkyl, -C₁₋₃ alkylene-C(=O)NHS(=O)₂C₁₋₃ alkyl, -C₁₋₃ alkylene-S(=O)₂OH, -C₁₋₃ alkylene-S(=O)₂C₁₋₆ alkyl, -C₁₋₃ alkylene-P(=O)(OH)₂, -C₁₋₃ alkylene-tetrazolyl, and -C₁₋₃ alkylene-C(=O)C₁₋₃ alkyl are each independently optionally substituted with 1, 2, or more Rₐ substituents; preferably, R₁ is -C₁₋₃ alkylene-COOH.

In a preferred embodiment, in case (1), each Rₐ is independently methyl, oxo (=O), OH, F, Cl, or CN;
in a preferred embodiment, in case (1), each R₁ is independently CN, oxo (=O), halogen, NH₂, 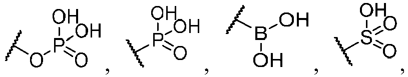 C₁₋₆ alkyl, C₁₋₆ alkoxy, -C₁₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, - C₁₋₃ alkylene-S(=O)₂NHC(=O)C₁₋₆ alkyl, -C₁₋₃ alkylene-P(=O)(OH)₂, or -C₁₋₃ alkyl-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents.

In a preferred embodiment, in case (3), each R₁ is independently 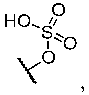 -NHS(=O)₂OH, or -C₁₋₃ alkylene-NHS(=O)₂OH; the -NHS(=O)₂OH and -C₁₋₃ alkylene-NHS(=O)₂OH are each independently optionally substituted with 1, 2, or more Rₐ substituents; each Rₐ is independently C₁₋₄ alkyl.

In a preferred embodiment, each R₁ is independently COOH or -C₁₋₃ alkylene-COOH.

In a preferred embodiment, in case (1), each R₁ is independently OH, COOH, F, Cl, CN, methyl, - CH₂F, -CHF₂, -CF₃, 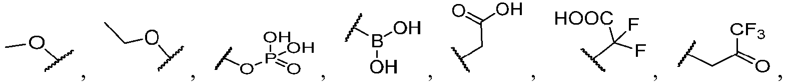 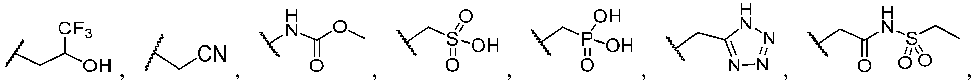 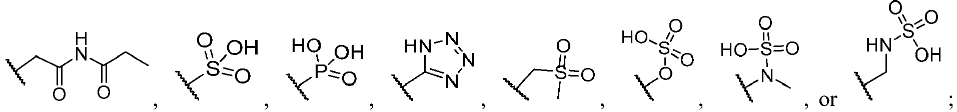 for example, OH, COOH, F, Cl, CN, methyl, -CH₂F, -CHF₂, -CF₃,

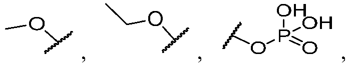

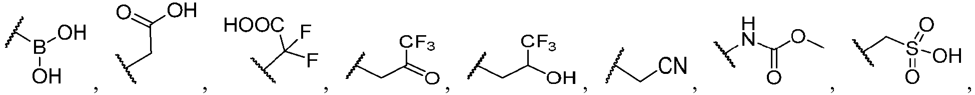

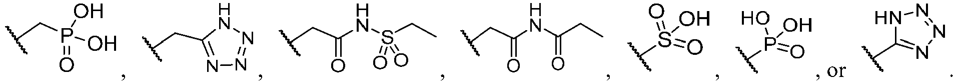

In a preferred embodiment, in case (2), each R₁ is independently OH, F, Cl, CN, methyl, -CH₂F, - CHF₂, -CF₃,

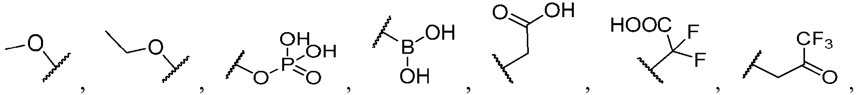

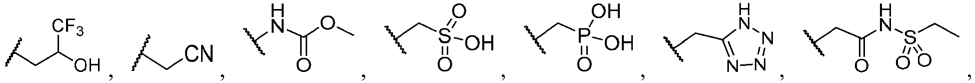

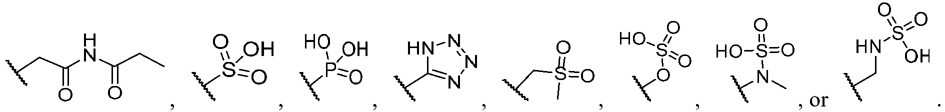

In a preferred embodiment, in case (3), each R₁ is independently OH, F, Cl, CN, methyl, -CH₂F, - CHF₂, -CF₃, 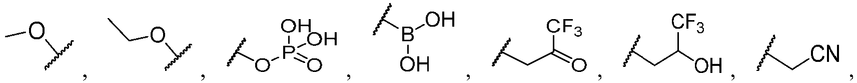 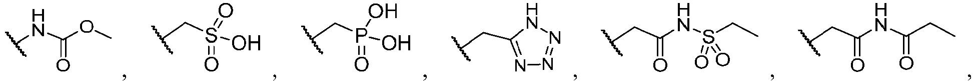 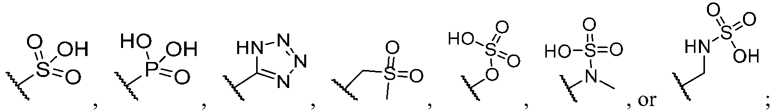 for example,

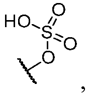

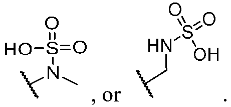

In a preferred embodiment, m is 0, 1, 2, or 3, for example, 1.

In the present disclosure, 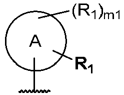 is also 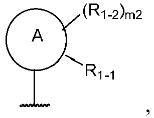 the definition of R₁₋₁ is the same as the definition of R₁ in any one embodiment of the present disclosure, and the definition of m2 is the same as the definition of m or m1 in any one embodiment of the present disclosure; in (R₁)ₘ₁, the definition of R₁ is the same as the definition of R₁₋₂, both being the definition of R₁ in any one embodiment of the present disclosure or the definition of R₁₋₂ in any one embodiment of the present disclosure; preferably, each R₁₋₂ is independently halogen, C₁₋₆ alkyl, C₁₋₆ alkoxy, OH, or CN; the C₁₋₆ alkyl and C₁₋₆ alkoxy are each independently optionally substituted with 1, 2, or more Rₐ substituents; for example, each R₁₋₂ is independently halogen, C₁₋₄ alkyl, C₁₋₄ alkoxy, OH, or CN; the C₁₋₄ alkyl and C₁₋₄ alkoxy are each independently optionally substituted with 1, 2, or more Rₐ substituents.

In a preferred embodiment, 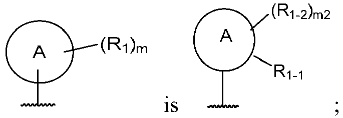 in case (1), ring B is 6-membered heteroarylene; R₁₋₁ is OH, COOH, C₁₋₆ alkyl, 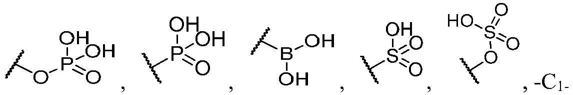 ₃ alkylene-COOH, -NHC(=O)OC₁₋₆ alkyl, -NHS(=O)₂OH, -C₁₋₃ alkylene-NHS(=O)₂OH, -C₁₋₃ alkyleneS(=O)₂OH, or -C₁₋₃ alkylene-S(=O)₂C₁₋₆ alkyl; the C₁₋₆ alkyl, -C₁₋₃ alkylene-COOH, -NHC(=O)OC₁₋₆ alkyl, - NHS(=O)₂OH, -C₁₋₃ alkylene-NHS(=O)₂OH, -C₁₋₃ alkylene-S(=O)₂OH, and -C₁₋₃ alkylene-S(=O)₂C₁₋₆ alkyl are each independently optionally substituted with 1, 2, or more Rₐ substituents;
or, R₁₋₁ is COOH, 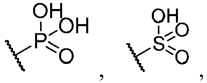 tetrazolyl, -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, or -C₁₋₃ alkylene-tetrazolyl; the -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, and -C₁₋₃ alkylene-tetrazolyl are each independently optionally substituted with 1, 2, or more Rₐ substituents;
in R₁₋₁, each Rₐ is independently C₁₋₄ alkyl, halogen, OH, oxo (=O), or CN; when the number of Rₐ is 2 or more, the OH and oxo (=O) are not attached to the same carbon atom;
each R₁₋₂ is independently halogen, C₁₋₄ alkyl, C₁₋₄ alkoxy, OH, or CN; the C₁₋₄ alkyl and C₁₋₄ alkoxy are each independently optionally substituted with 1, 2, or more Rₐ substituents;
in R₁₋₂, each Rₐ is independently halogen. In a preferred embodiment, each Rₐ is independently halogen.

In a preferred embodiment, 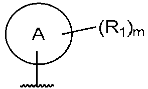 is 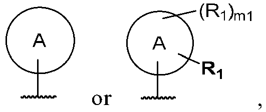 m1 is 0, 1, or 2; for example, 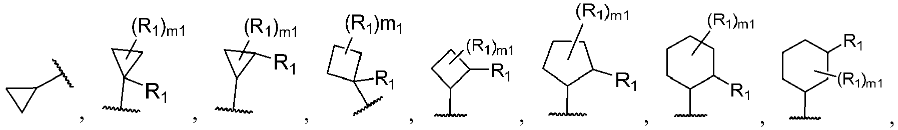 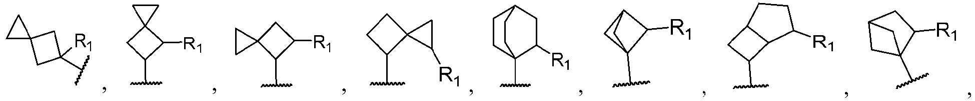 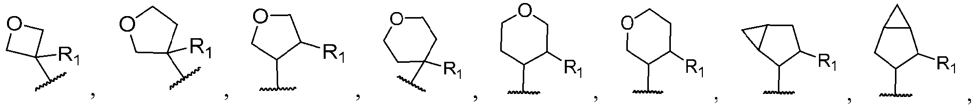 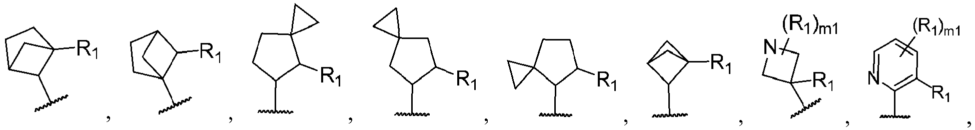 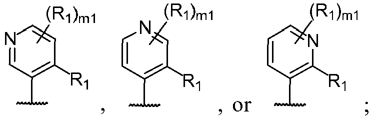 for another example, 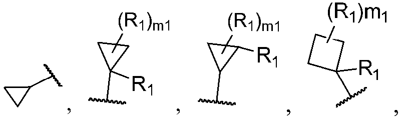 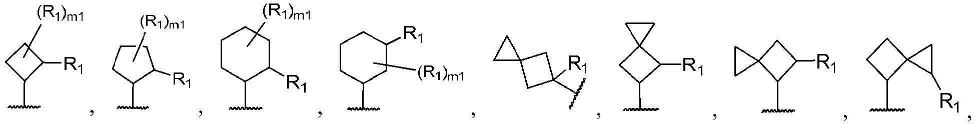 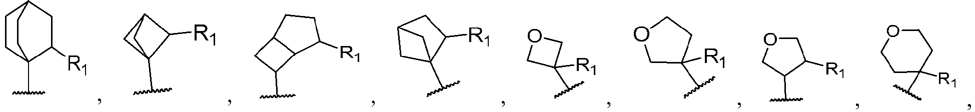 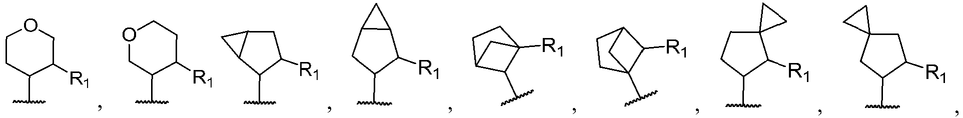 or

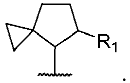

In a preferred embodiment, 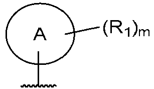 is 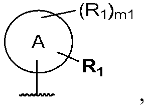 m1 is 0, 1, or 2; for example, 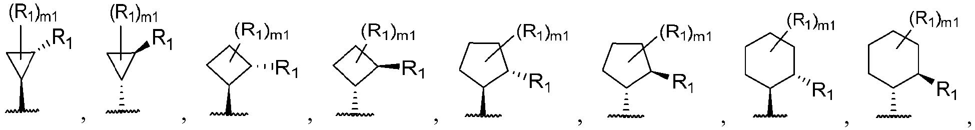 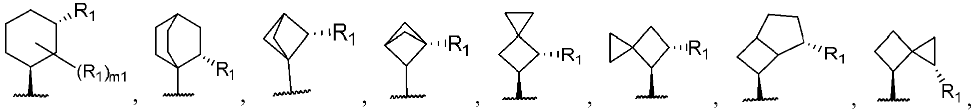 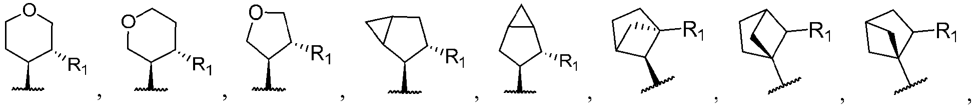 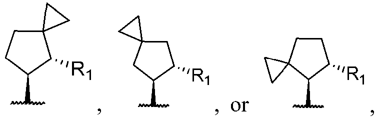 and may also be 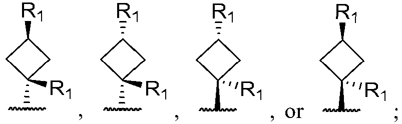 for another example, 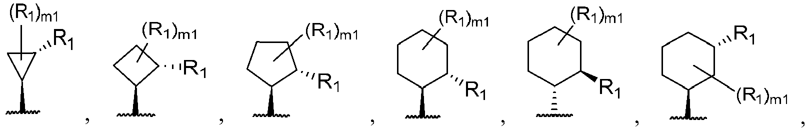 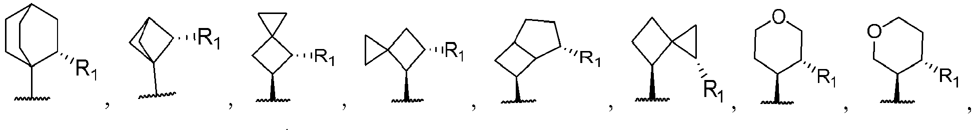 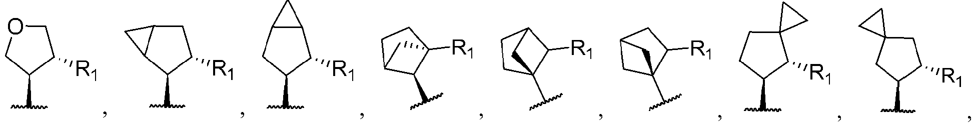 or

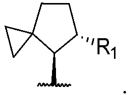

In a preferred embodiment, 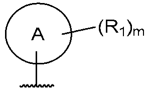 is 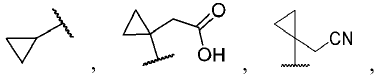 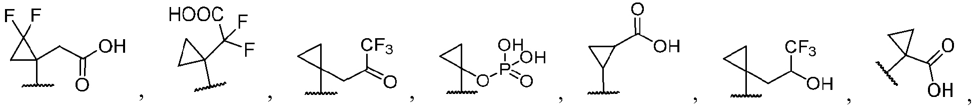 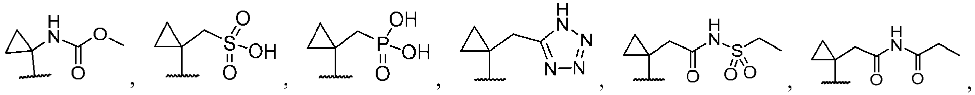 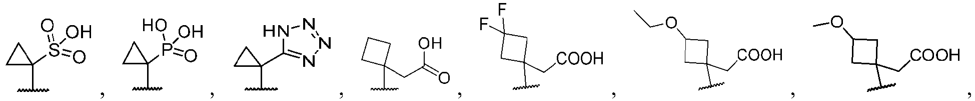 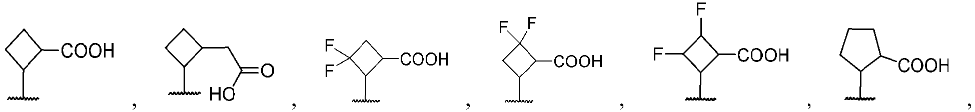 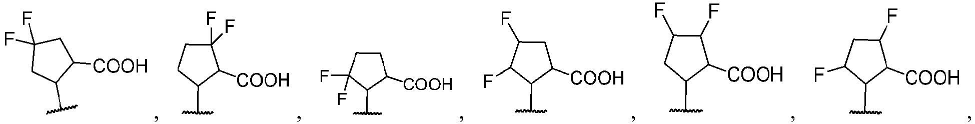 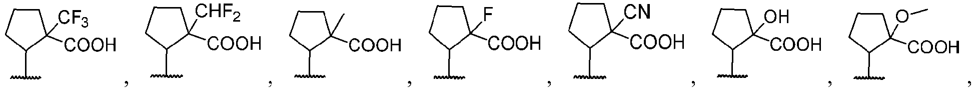 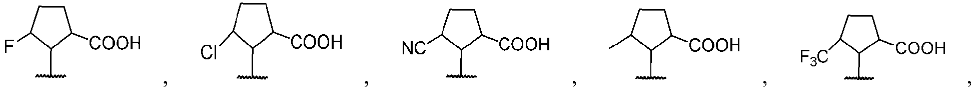 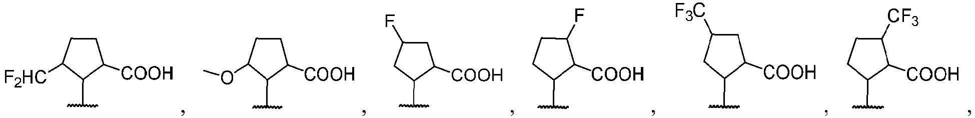 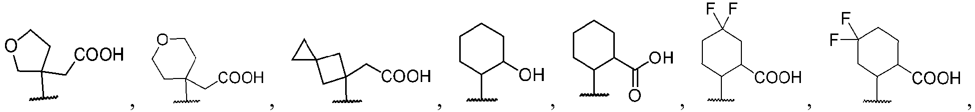 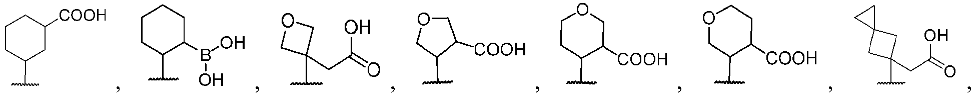 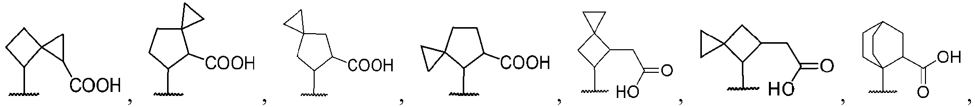 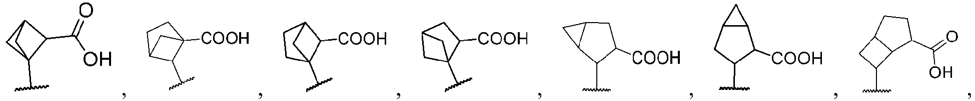 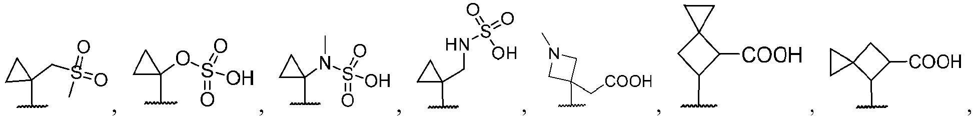 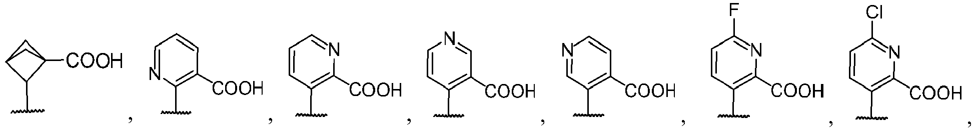 or 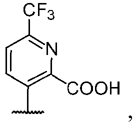 and may also be 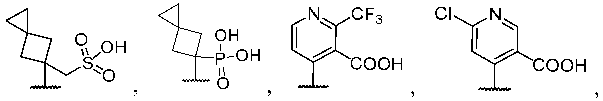 or

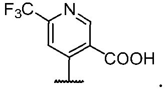

In a preferred embodiment, 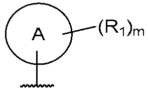 is 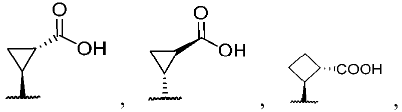 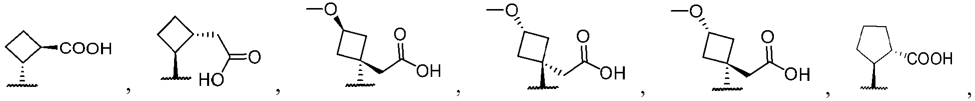 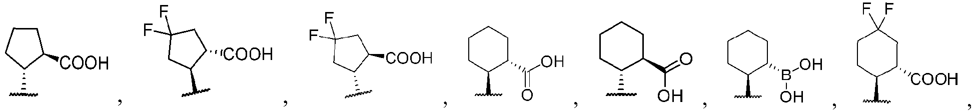 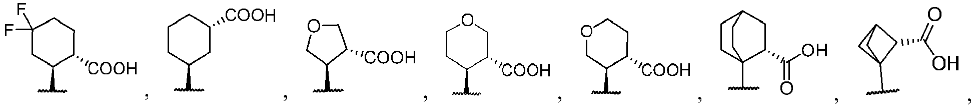 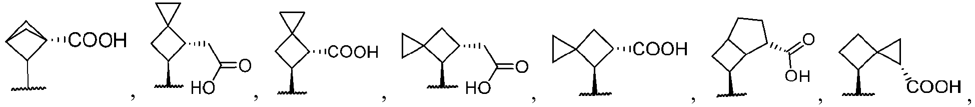 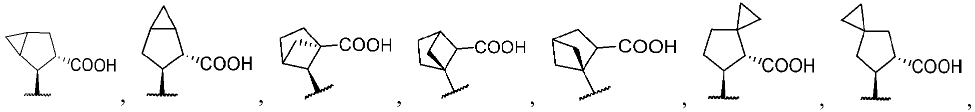 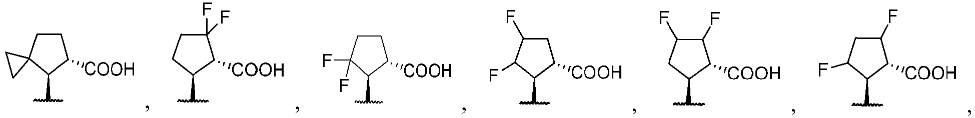 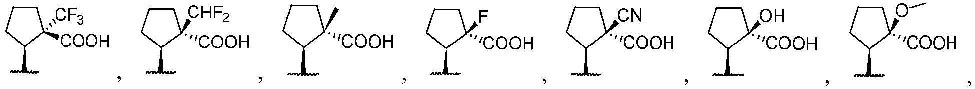 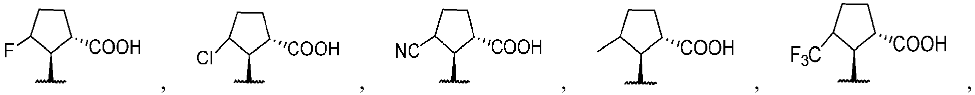 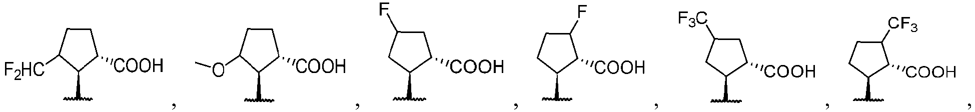 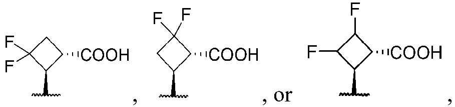 and may also be

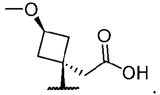

In a preferred embodiment, 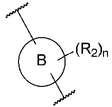 is 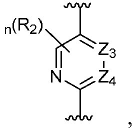 wherein Z₃ and Z₄ are each independently C, CH, or N.

In a preferred embodiment, each R₂ is independently halogen, -NH₂, C₁₋₄ alkyl, C₁₋₄ alkoxy, C₃₋₆ cycloalkyl, or 3- to 6-membered heterocyclyl; preferably, each R₂ is independently halogen, C₁₋₄ alkyl, methoxy, or cyclopropyl; the C₁₋₄ alkyl is optionally substituted with 1, 2, or more halogen or deuterium substituents.

In a preferred embodiment, each R₂ is independently halogen, C₁₋₄ alkyl, or C₃₋₆ cycloalkyl; the C₁₋₄ alkyl and C₃₋₆ cycloalkyl are each independently optionally substituted with 1, 2, or more halogen or deuterium substituents; for example, each R₂ is independently C₁₋₄ alkyl.

In a preferred embodiment, each R₂ is independently methyl, ethyl, F, Cl, Br, -CD₃, -CF₃, -CHF₂, - CH₂F, -NH₂, -OCH₃, or cyclopropyl; for example, methyl, ethyl, F, Cl, -CD₃, -CF₃, -CH₂F, -CHF₂, -OCH₃, or cyclopropyl; for example, methyl, ethyl, F, Cl, -CD₃, -CF₃, -CHF₂, -OCH₃, or cyclopropyl.

In a preferred embodiment, each R₂ is independently methyl, ethyl, F, Cl, Br, -CF₃, -CHF₂, -CH₂F, or -NH₂; for example, methyl, ethyl, F, Cl, -CF₃, or -CHF₂.

In a preferred embodiment, n is 0 or 1.

In a preferred embodiment, the 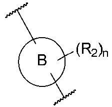 is 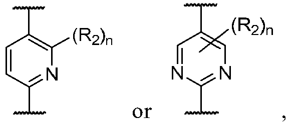 for example, 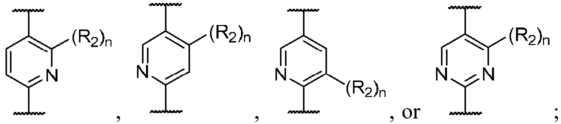 n is 0 or 1; for another example, 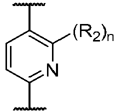 or 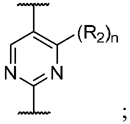 preferably, 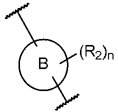 is 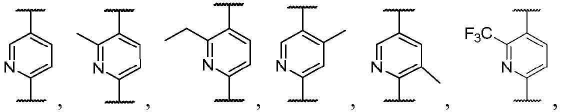 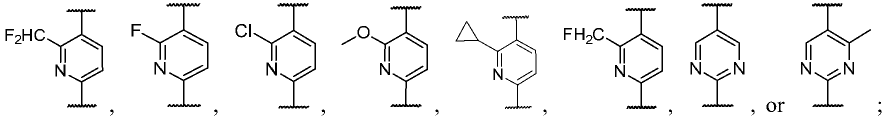 preferably, 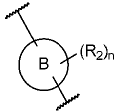 is 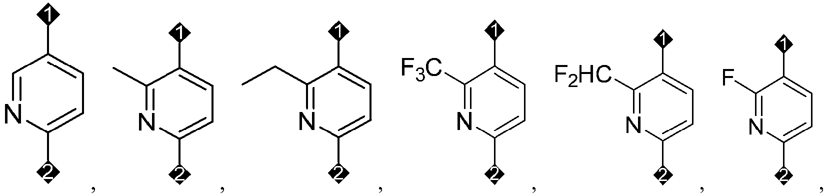 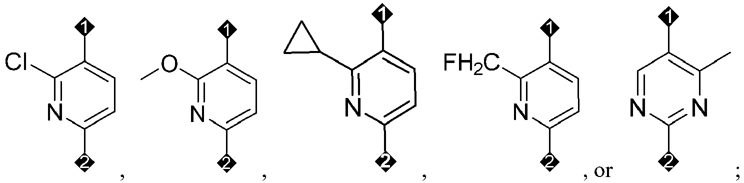 wherein the "1" position is connected to L₁, and the "2" position is connected to X₁ in

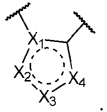

In a preferred embodiment, the 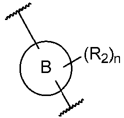 is 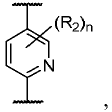 for example, 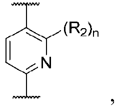 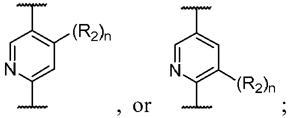 n is 0 or 1; preferably, 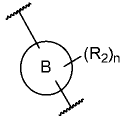 is 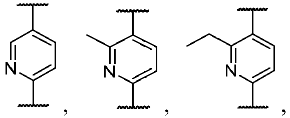 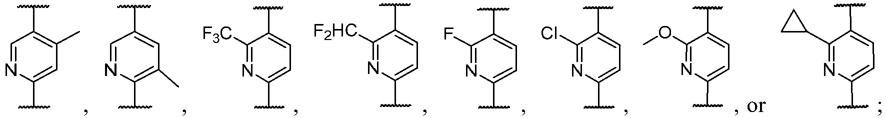 preferably, 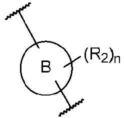 is 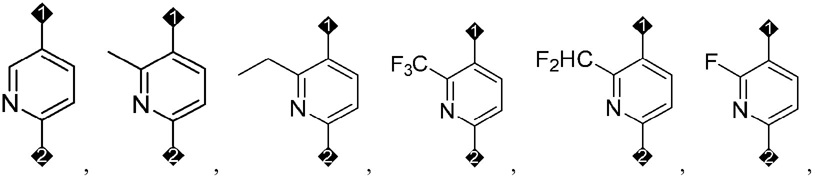 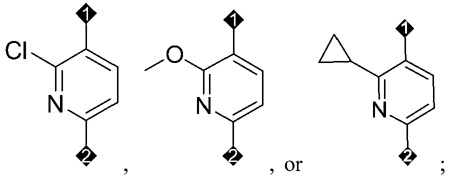 wherein the "1" position is connected to L₁, and the "2" position is connected to X₁ in

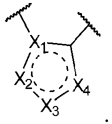

In a preferred embodiment, 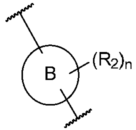 is 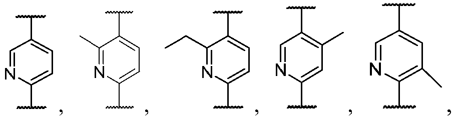 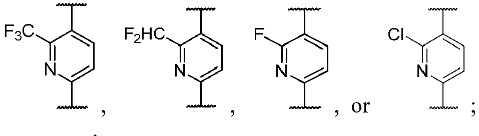 preferably, 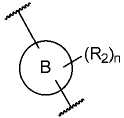 is 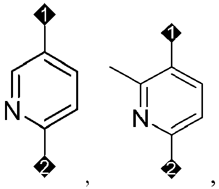 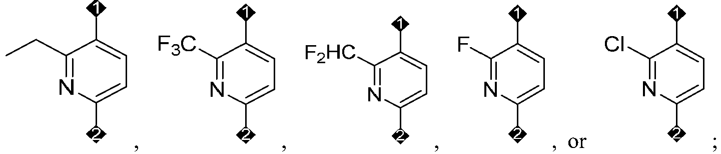 wherein the "1" position is connected to L₁, and the "2" position is connected to X₁ in

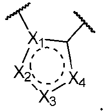

In a preferred embodiment, L₁ is C₂₋₆ alkynylene, wherein the C₂₋₆ alkynylene is optionally substituted with 1, 2, or 3 R_{b} substituents; preferably, L₁ is unsubstituted C₂₋₆ alkynylene.

In a preferred embodiment, each R_{b} is independently C₁₋₆ alkyl; the C₁₋₆ alkyl is optionally substituted with 1, 2, or 3 COOH substituents; preferably, the C₁₋₆ alkyl is optionally substituted with 1 COOH substituent.

In a preferred embodiment, L₁ is 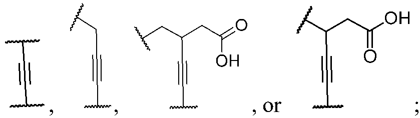 for example, 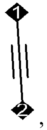 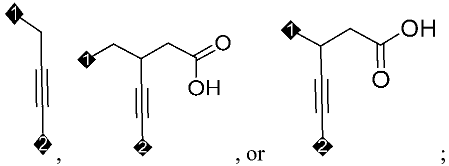 wherein the "1" position is connected to ring A, and the "2" position is connected to ring B.

In a preferred embodiment, X₁, X₂, X₃, and X₄ are each independently C, CH, CH₂, O, S, N, or NH; simultaneously, two or three of X₁, X₂, X₃, and X₄ are each independently O, S, N, or NH.

In a preferred embodiment, X₁ is C or N.

In a preferred embodiment, X₂ is CH, O, S, or N.

In a preferred embodiment, X₃ is C, CH₂, O, S, or N.

In a preferred embodiment, X₄ is C, CH, N, or NH.

In a preferred embodiment, 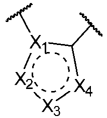 is triazolyl, pyrazolyl, imidazolyl, oxazolyl, tetrazolyl, isoxazolyl, thiazolyl, isothiazolyl, oxadiazolyl, furazanyl, thiadiazolyl, oxathiazolyl, 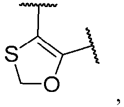 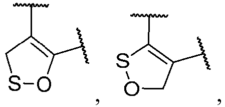 thienyl, furyl, or pyrrolyl;
preferably, 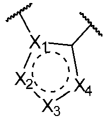 is 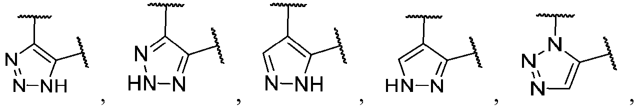 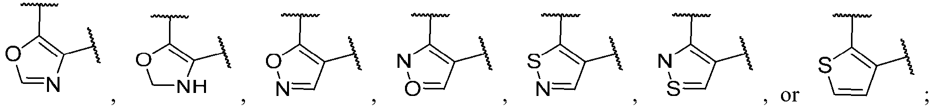 more preferably, 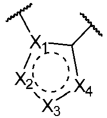 is 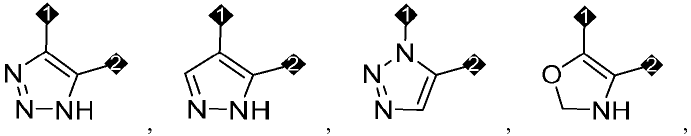 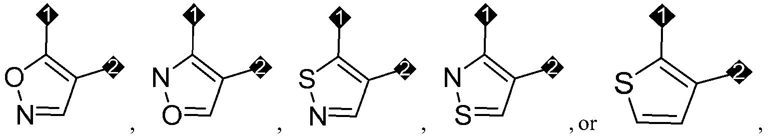 wherein the "1 " position is connected to ring B, and the "2" position is connected to W.

In a preferred embodiment, each R₃ is independently H, C₁₋₄ alkyl, oxo (=O), F, Cl, or Br; the C₁₋₄ alkyl is optionally substituted with 1, 2, or more halogen or deuterium substituents; preferably, each R₃ is independently H, C₁₋₄ alkyl, F, Cl, or oxo (=O); the C₁₋₄ alkyl is optionally substituted with 1, 2, or more halogen or deuterium substituents; or, each R₃ is independently C₁₋₄ alkyl, F, Cl, Br, or oxo, for example, each R₃ is independently C₁₋₄ alkyl.

Or, each R₃ is independently H, C₁₋₄ alkyl, oxo (=O), F, Cl, or Br; the C₁₋₄ alkyl is optionally substituted with 1, 2, or more halogen substituents; preferably, each R₃ is independently H, C₁₋₄ alkyl, F, Cl, or oxo (=O); the C₁₋₄ alkyl is optionally substituted with 1, 2, or more halogen substituents.

In a preferred embodiment, each R₃ is independently H, methyl, ethyl, oxo (=O), F, Cl, -CD₃, -CF₃, -CHF₂, or -CH₂F; preferably, each R₃ is independently H, methyl, ethyl, -CD₃, oxo (=O), or Cl;
or, each R₃ is independently H, methyl, ethyl, oxo (=O), F, Cl, -CF₃, -CHF₂, or -CH₂F; preferably, each R₃ is independently H, methyl, ethyl, oxo (=O), or Cl.

In a preferred embodiment, p is 0, 1, 2, or 3, for example, 1 or 2.

In a preferred embodiment, 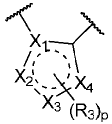 is 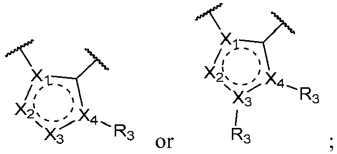 preferably, 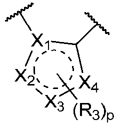 is 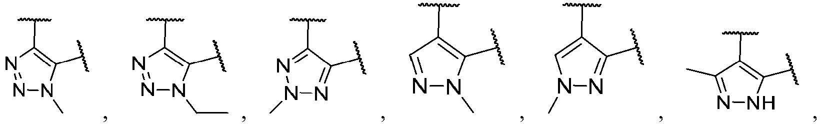 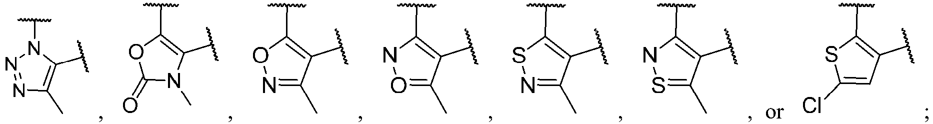 more preferably, 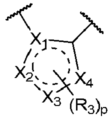 is 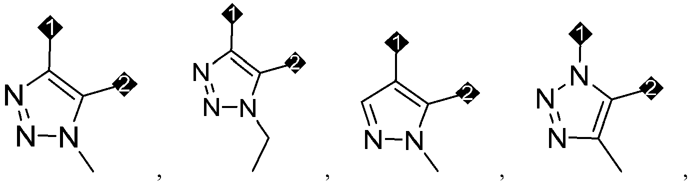 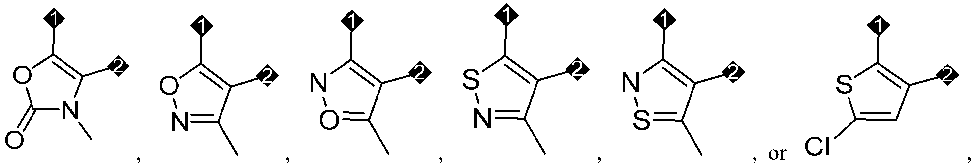 wherein the "1" position is connected to ring B and the "2" position is connected to W.

In a preferred embodiment, W is

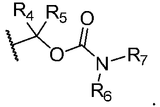

In a preferred embodiment, R₄ and R₅ are each independently H, halogen, or C₁₋₄ alkyl, wherein the C₁₋₄ alkyl is optionally substituted with 1, 2, or more halogen substituents; or, R₄ and R₅ are each independently H or deuterium; for example, R₄ and R₅ are H; R₆ and R₇ have the definitions as described in any one embodiment of the present disclosure; preferably, W is

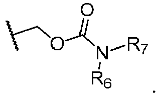

In a preferred embodiment, R₆ is H or C₁₋₆ alkyl; the C₁₋₆ alkyl is optionally substituted with 1, 2, or more R_{c} substituents.

In a preferred embodiment, in R₆, each R_{c} is independently halogen, for example, fluorine, chlorine, or bromine.

In a preferred embodiment, R₇ is C₁₋₆ alkyl, C₃₋₈ cycloalkyl, C₆₋₁₀ aryl, or 5- to 10-membered heteroaryl; the C₁₋₆ alkyl is optionally substituted with 1, 2, or more R_{c} substituents; the C₃₋₈ cycloalkyl, C₆₋₁₀ aryl, and 5- to 10-membered heteroaryl are each independently optionally substituted with 1, 2, or more Rₑ substituents; preferably, R₇ is C₁₋₆ alkyl, C₃₋₈ cycloalkyl, phenyl, or 5- to 6-membered heteroaryl; the C₁₋₆ alkyl is optionally substituted with 1, 2, or more R_{c} substituents; the C₃₋₈ cycloalkyl, phenyl, and 5- to 6-membered heteroaryl are each independently optionally substituted with 1, 2, or more Rₑ substituents; the heteroatom in the 5- to 6-membered heteroaryl is selected from 1 or 2 types of N, O, and S, and the number of the heteroatom is 1 or 2; more preferably, R₇ is C₁₋₆ alkyl, C₃₋₈ cycloalkyl, phenyl, or 5- to 6-membered heteroaryl; the C₁₋₆ alkyl is optionally substituted with 1, 2, or more R_{c} substituents.

In a preferred embodiment, in R₇, each R_{c} is independently deuterium, halogen, C₁₋₄ alkyl, C₃₋₈ cycloalkyl, C₆₋₁₀ aryl, or 5- to 10-membered heteroaryl; the C₁₋₄ alkyl, C₃₋₈ cycloalkyl, C₆₋₁₀ aryl, and 5- to 10-membered heteroaryl are each independently optionally substituted with 1, 2, or more R_{c-1} substituents.

In a preferred embodiment, each R_{c} is independently deuterium, halogen, C₁₋₆ alkoxy, C₃₋₈ cycloalkyl, C₆₋₁₀ aryl, or 5- to 10-membered heteroaryl; the C₁₋₆ alkoxy, C₃₋₈ cycloalkyl, C₆₋₁₀ aryl, and 5- to 10-membered heteroaryl are each independently optionally substituted with 1, 2, or more R_{c-1} substituents; preferably, the 5- to 10-membered heteroaryl is 5- to 6-membered heteroaryl; the heteroatom in the 5- to 6-membered heteroaryl is selected from 1 or 2 types of N, O, and S, and the number of the heteroatom is 1 or 2.

In a preferred embodiment, in R₇, each R_{c-1} is independently deuterium, halogen, or C₁₋₆ alkyl.

In a preferred embodiment, in R₇, each Rₑ is independently halogen or C₁₋₆ alkyl, for example, fluorine, chlorine, bromine, or methyl, or for example, deuterium or halogen.

In a preferred embodiment, each R_{c} is independently F, methyl, -CF₃, -CHF₂, methoxy, cyclopropyl, cyclobutyl, cyclopentyl,

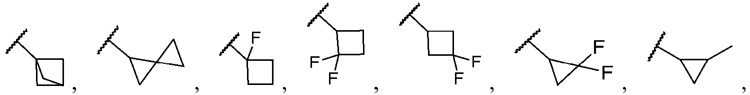

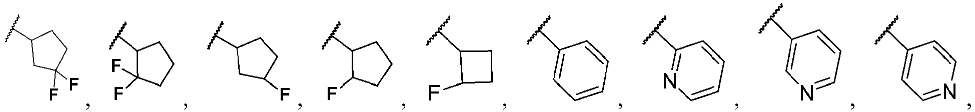

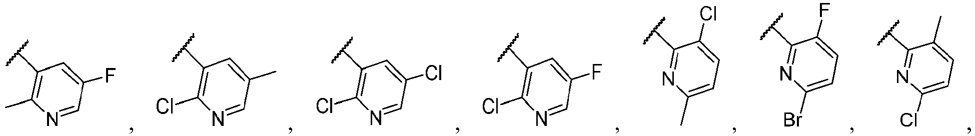

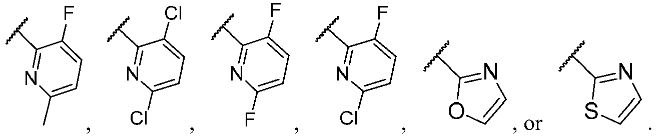

In a preferred embodiment, R₆ is H, methyl, ethyl, 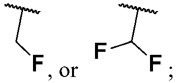 for example, methyl, ethyl,

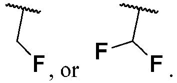

In a preferred embodiment, R₇ is

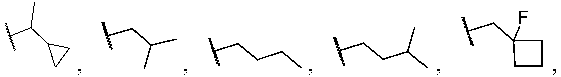

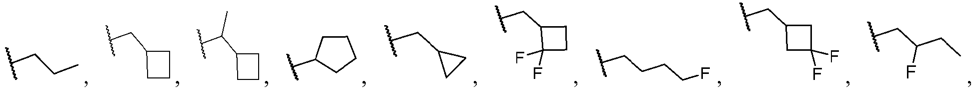

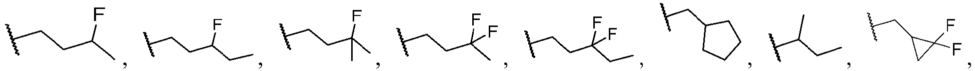

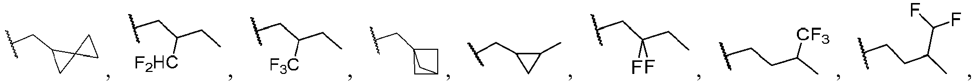

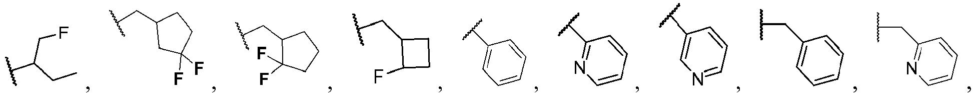

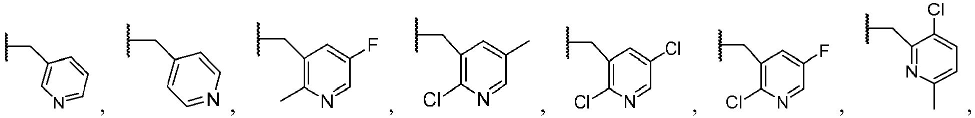

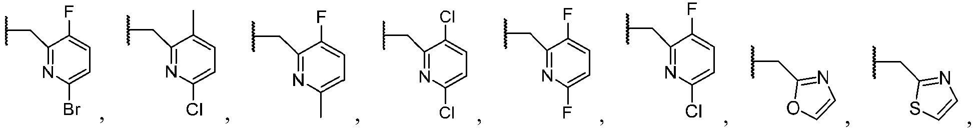

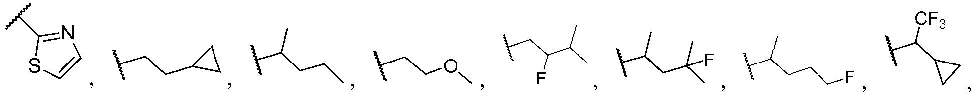

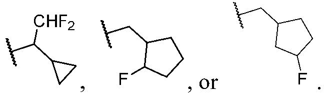

In a preferred embodiment, 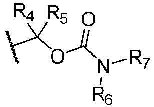 is

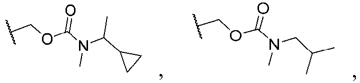

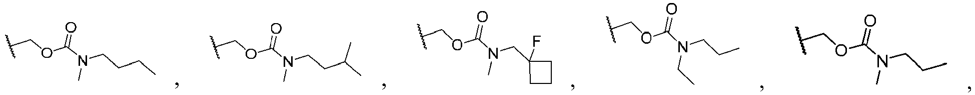

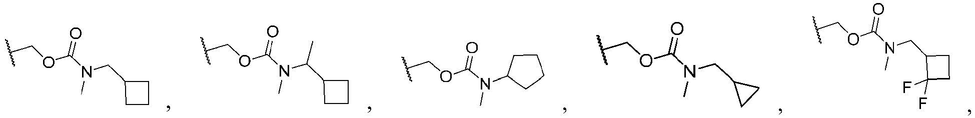

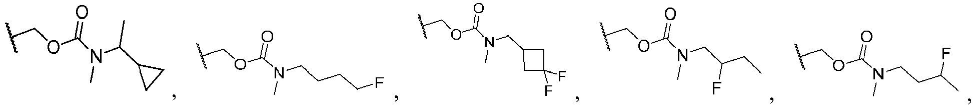

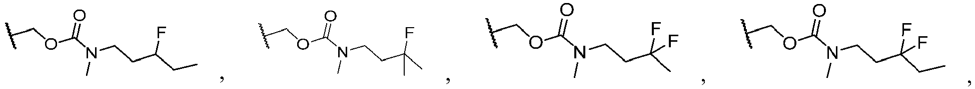

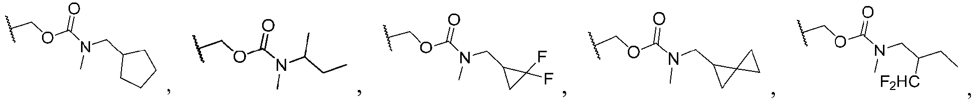

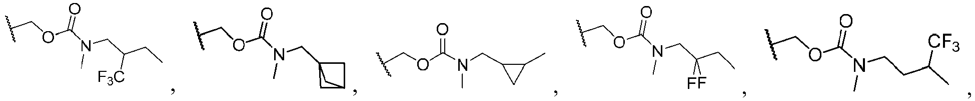

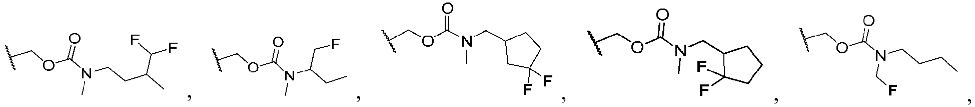

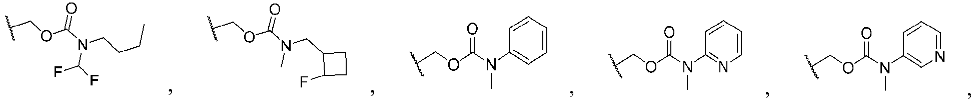

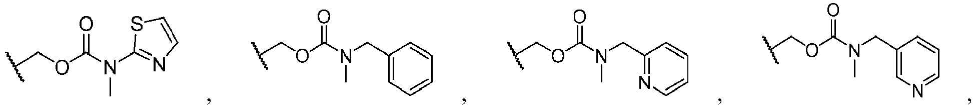

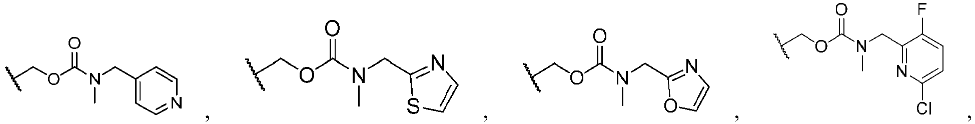

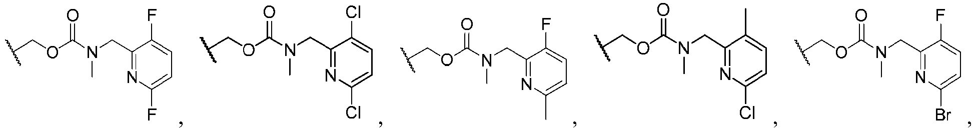

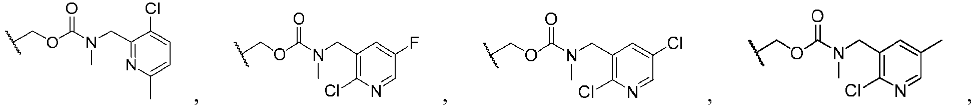

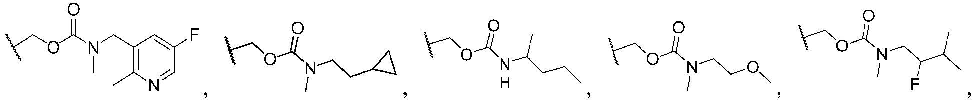

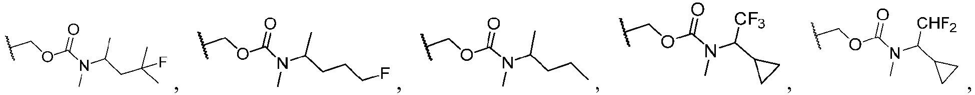

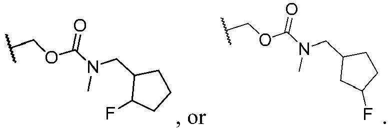

In a preferred embodiment, 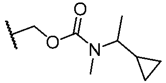 is

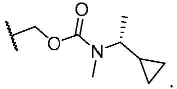

In a preferred embodiment, 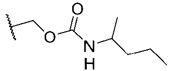 is

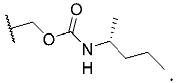

In a preferred embodiment, R₈ is H, deuterium, halogen, or C₁₋₄ alkyl; for example, H or deuterium; for another example, H.

In a preferred embodiment, Y₁ is a chemical bond.

In a preferred embodiment, Y₁ is C₁₋₆ alkylene; the C₁₋₆ alkylene is optionally substituted with 1, 2, or more R_{d} substituents; preferably, Y₁ is unsubstituted C₁₋₄ alkylene.

In a preferred embodiment, Y₁ is a single bond or C₁₋₆ alkylene.

In a preferred embodiment, R_{f} is halogen or deuterium; for example, halogen.

In a preferred embodiment, R₉ is H or deuterium; for example, H.

In a preferred embodiment, R₁₀ is H, deuterium, or C₁₋₄ alkyl; the C₁₋₄ alkyl is optionally substituted with 1, 2, 3, or 4 R_{f} substituents; for example, R₁₀ is H or C₁₋₄ alkyl; the C₁₋₄ alkyl is optionally substituted with 1, 2, 3, or 4 R_{f} substituents;

In a preferred embodiment, R₁₀ is H, methyl, ethyl, n-propyl, isopropyl, -CD₃, 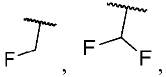 or 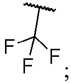 preferably, R₁₀ is H, methyl, -CD₃, 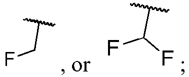 or, R₁₀ is H, methyl, ethyl, n-propyl, isopropyl, 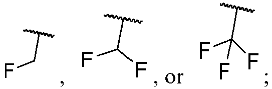 preferably, R₁₀ is H, methyl,

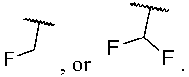

In a preferred embodiment, W is 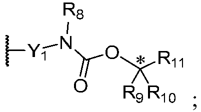 preferably, W is 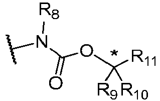 or

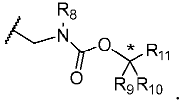

In a preferred embodiment, in 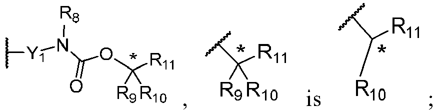 when the carbon atom marked with * is a chiral carbon atom, 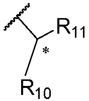 is 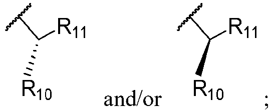 more preferably, 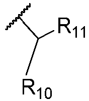 is

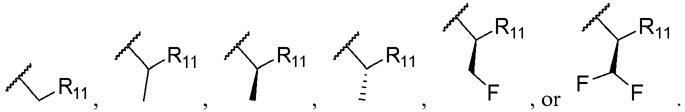

In a preferred embodiment, R₁₁ is C₁₋₆ alkyl, phenyl, 5- to 6-membered heteroaryl, or C₃₋₆ cycloalkyl; the C₃₋₆ cycloalkyl is a monocyclic, spiro, or bridged ring; the C₁₋₆ alkyl, phenyl, 5- to 6-membered heteroaryl, and C₃₋₆ cycloalkyl are each independently optionally substituted with 1, 2, or more R_{g} substituents; the heteroatom of the 5- to 6-membered heteroaryl is N, and the number of the heteroatom is 1 or 2; preferably, R₁₁ is phenyl or 5- to 6-membered heteroaryl; the phenyl or 5- to 6-membered heteroaryl is independently optionally substituted with 1, 2, or more R_{g} substituents.

In a preferred embodiment, R₁₁ is C₁₋₆ alkyl, C₆₋₁₀ aryl, 5- to 10-membered heteroaryl, or C₃₋₈ cycloalkyl; the C₁₋₆ alkyl, C₆₋₁₀ aryl, 5- to 10-membered heteroaryl, and C₃₋₈ cycloalkyl are each independently optionally substituted with 1, 2, or more R_{g} substituents; preferably, R₁₁ is C₁₋₆ alkyl, C₃₋₄ cycloalkyl, bicyclo[1.1.1]pentyl, spiro[2.3]hexyl, bicyclo[2.1.1]hexyl, bicyclo[1.1.1]pentyl, spiro[2.3]hexyl, or bicyclo[2.1.1]hexyl; the C₁₋₆ alkyl, C₃₋₄ cycloalkyl, bicyclo[1.1.1]pentyl, spiro[2.3]hexyl, bicyclo[2.1.1]hexyl, bicyclo[1.1.1]pentyl, spiro[2.3]hexyl, and bicyclo[2.1.1]hexyl are each independently optionally substituted with 1, 2, or more R_{g} substituents; or R₁₁ is cyclopentyl, cyclohexyl, phenyl, or 5- to 6-membered heteroaryl; the cyclopentyl, cyclohexyl, phenyl, or 5- to 6-membered heteroaryl is independently optionally substituted with 1, 2, or more R_{g} substituents.

In a preferred embodiment, R₁₁ is C₁₋₆ alkyl or C₃₋₄ cycloalkyl; the C₁₋₆ alkyl and C₃₋₄ cycloalkyl are each independently optionally substituted with 1, 2, or more R_{g} substituents.

In a preferred embodiment, each R_{g} is independently deuterium, halogen, C₁₋₆ alkyl, or C₃₋₆ cycloalkyl; the C₁₋₆ alkyl and C₃₋₆ cycloalkyl are optionally substituted with 1, 2, or more deuterium or halogen substituents; preferably, each R_{g} is independently deuterium or halogen.

In a preferred embodiment, each R_{g} is independently halogen or C₁₋₆ alkyl; the C₁₋₆ alkyl is optionally substituted with 1, 2, or more halogen substituents; preferably, each R_{g} is independently halogen.

In a preferred embodiment, each R_{g} is deuterium, F, Cl, methyl, ethyl, -CH₂F, -CHF₂, -CF₃, -CD₃, 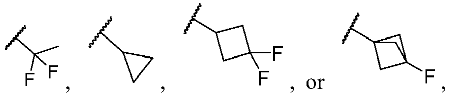 for example, deuterium, F, Cl, -CD₃,

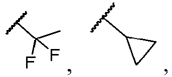

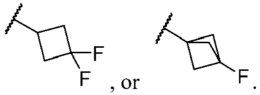

In a preferred embodiment, each R_{g} is F, Cl, methyl, ethyl, -CH₂F, -CHF₂, or -CF₃, for example, F or Cl.

In a preferred embodiment, R₁₁ is 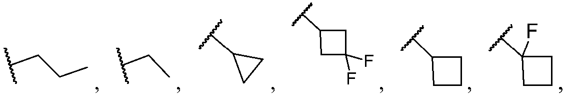 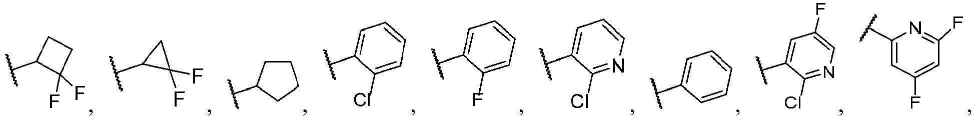 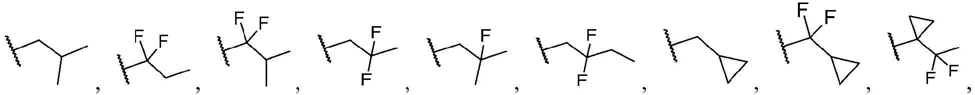 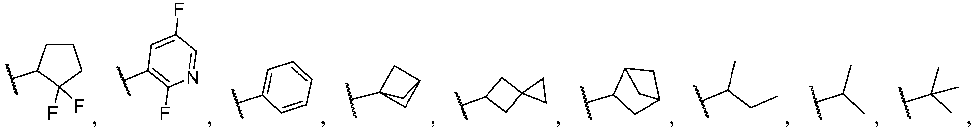 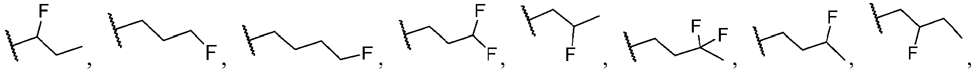 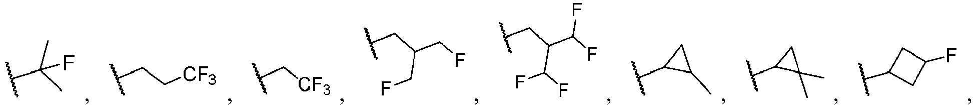 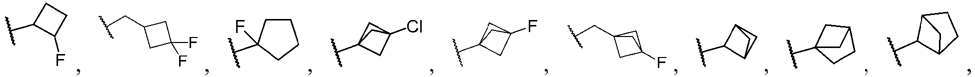 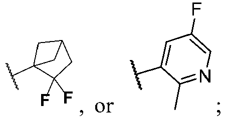 R₁₁ is

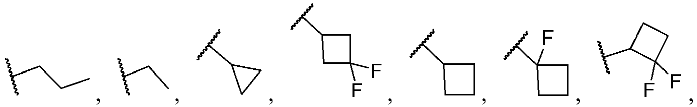

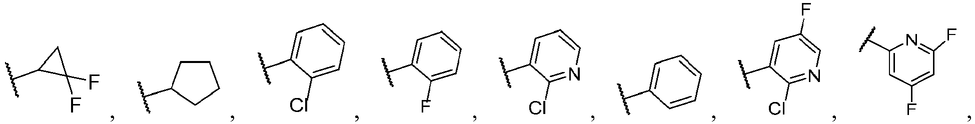

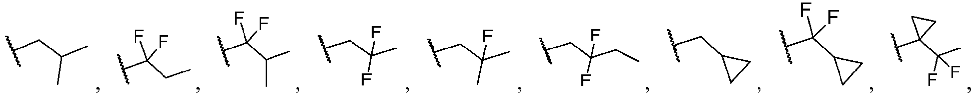

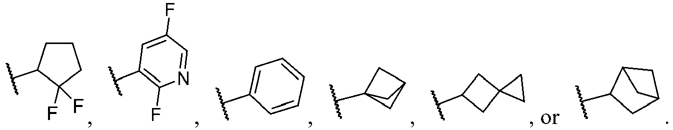

In a preferred embodiment, 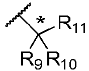 is 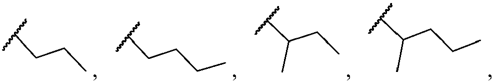 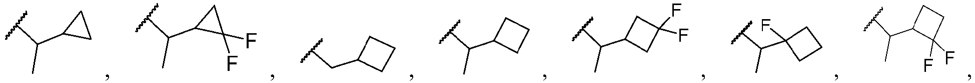 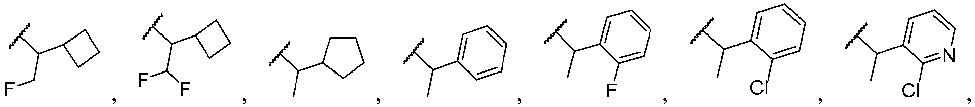 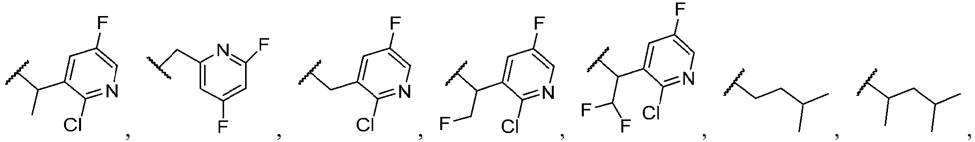 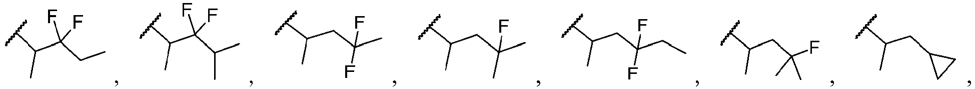 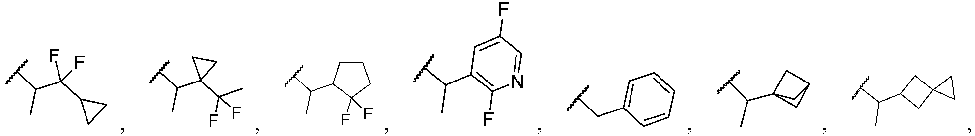 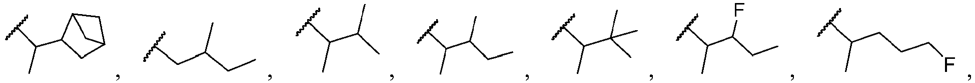 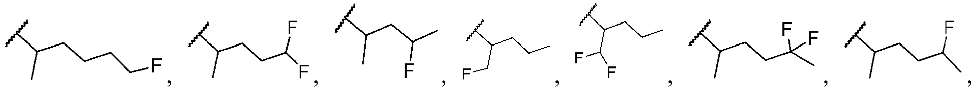 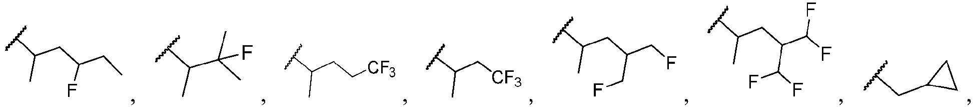 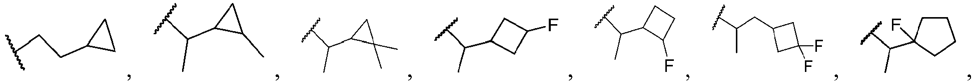 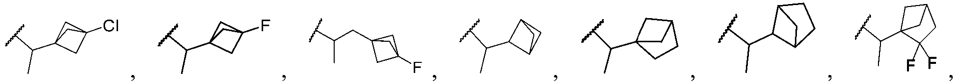 or 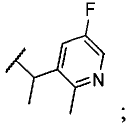 for example,

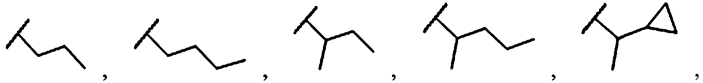

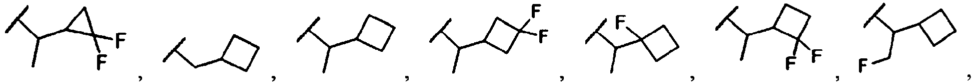

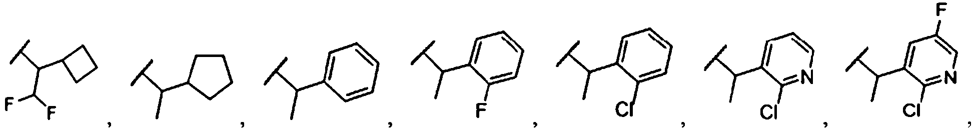

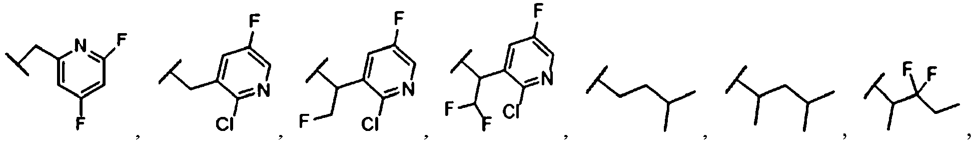

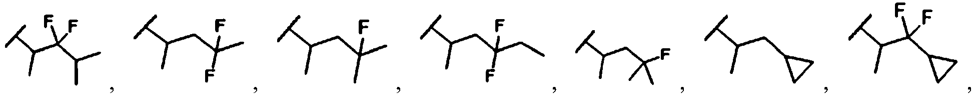

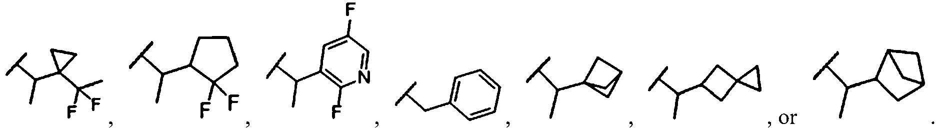

In a preferred embodiment, 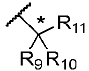 is 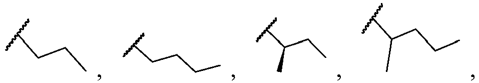 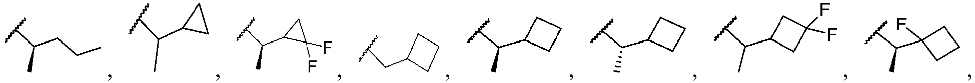 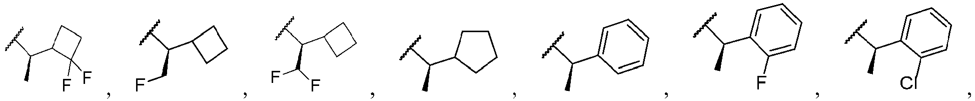 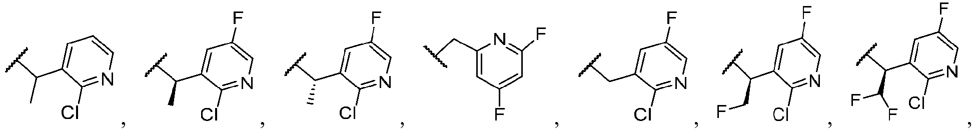 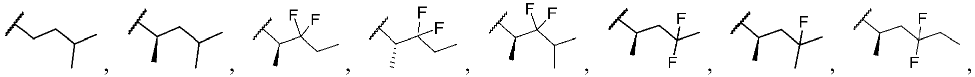 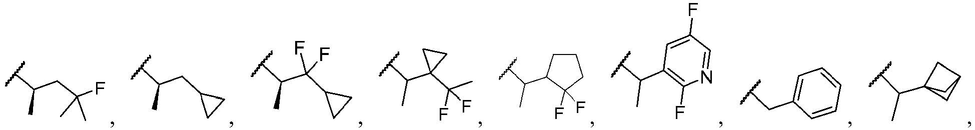 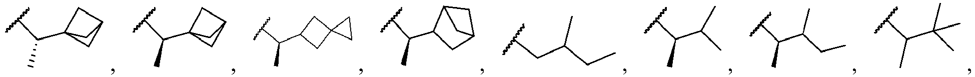 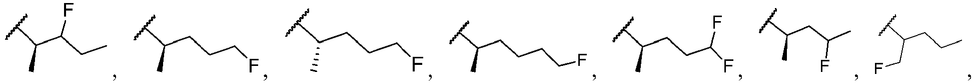 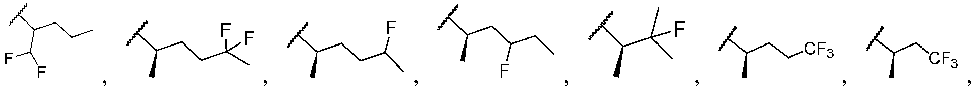 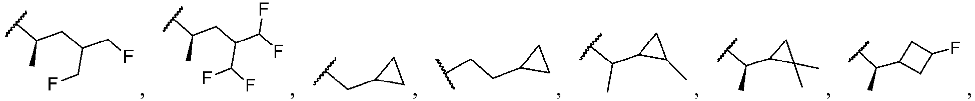 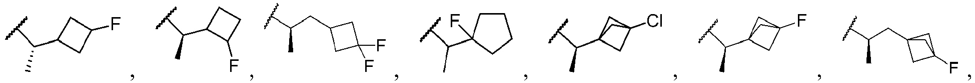 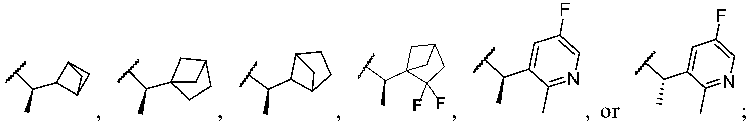 for example,

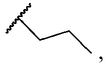

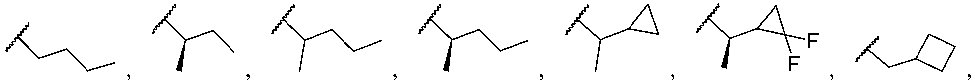

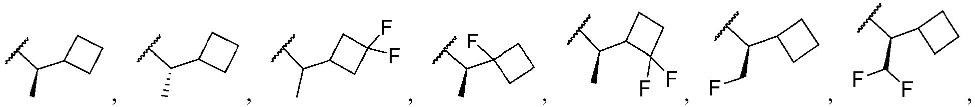

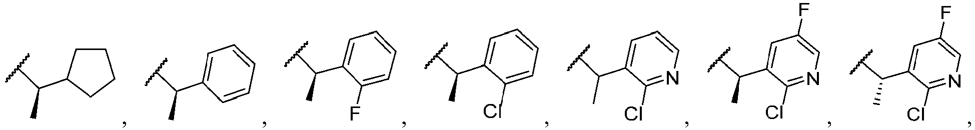

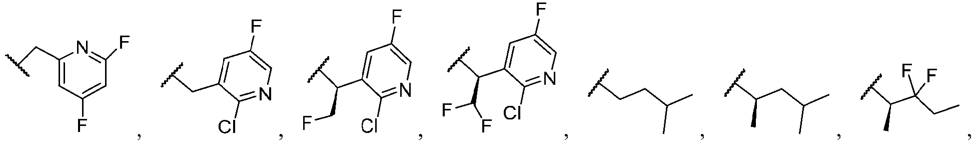

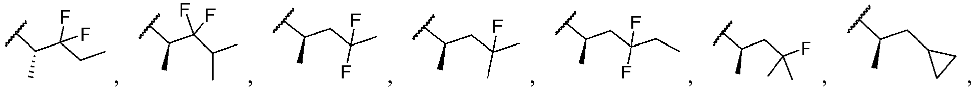

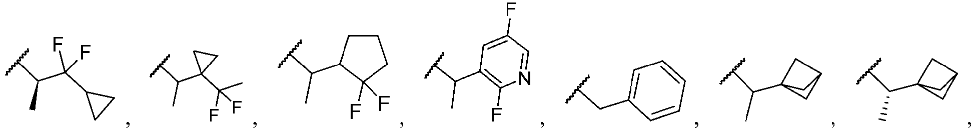

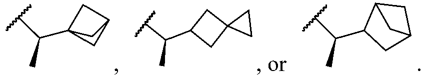

In a preferred embodiment, R₁₂ and R₁₃ are each independently H, deuterium, or halogen.

In a preferred embodiment, R₁₄ is H, deuterium, or C₁₋₆ alkyl.

In a preferred embodiment, R₁₂, R₁₃, and R₁₄ are each independently H, methyl, or F; for example, H.

In a preferred embodiment, Y₂ is a chemical bond, NH, -C(=O)N(CH₃)-, or O.

In a preferred embodiment, q is 0, 1, 2, or 3; for example, q is 0 or 1.

In a preferred embodiment, when q is 0, Y₂ is N(R₁₄), -C(=O)N(R₁₄)-, or O.

In a preferred embodiment, W is -(CR₁₂R₁₃)_{q}-Y₂-R₁₅; preferably, W is -N(R₁₄)-R₁₅, -C(R₁₂R₁₃)-R₁₅, -C(R₁₂R₁₃)-N(R₁₄)-R₁₅, -C(R₁₂R₁₃)-O-R₁₅, or -(CR₁₂R₁₃)₂-C(=O)-N(R₁₄)-R₁₅; more preferably, W is -NH-R₁₅, -CH₂-R₁₅, -CH₂-NH-R₁₅, -CH₂-O-R₁₅, or

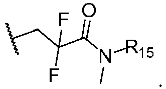

In a preferred embodiment, R₁₅ is C₁₋₆ alkyl, 3- to 6-membered heterocyclyl, or 5- to 6-membered heteroaryl; the C₁₋₆ alkyl, 3- to 6-membered heterocyclyl, and 5- to 6-membered heteroaryl are each independently optionally substituted with 1, 2, or more Rᵢ substituents; the heteroatom in the 3- to 6-membered heterocyclyl is independently N or O, and the number of the heteroatom is independently 1 or 2; the heteroatom in the 5- to 6-membered heteroaryl is independently N, O, or S, and the number of the heteroatom may independently be 1, 2, or 3.

In a preferred embodiment, each Rᵢ is independently halogen, oxo (=O), C₃₋₆ cycloalkyl, C₁₋₆ alkyl, or C₁₋₆ alkoxy; the C₁₋₆ alkyl, C₃₋₆ cycloalkyl, and C₁₋₆ alkoxy are each optionally substituted with 1, 2, or more Rᵢ₋₁ substituents.

In a preferred embodiment, each Rᵢ₋₁ is independently halogen, C₁₋₆ alkyl, C₃₋₆ cycloalkyl, or C₁₋₆ alkoxy.

In a preferred embodiment, each Rᵢ is independently methyl, n-propyl, isopropyl, F, oxo (=O),

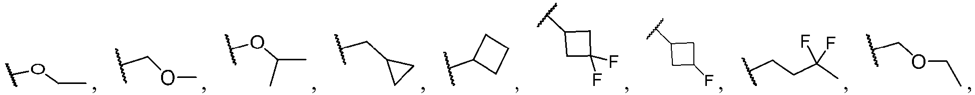

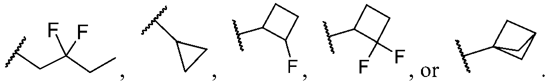

In a preferred embodiment, R₁₅ is 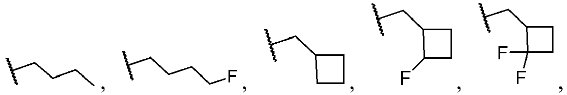 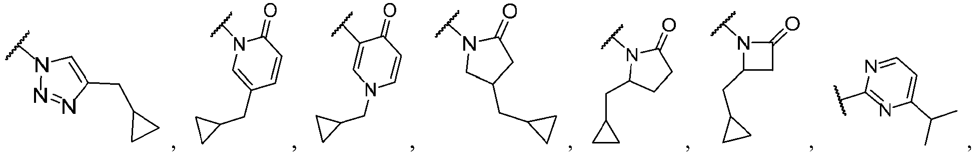 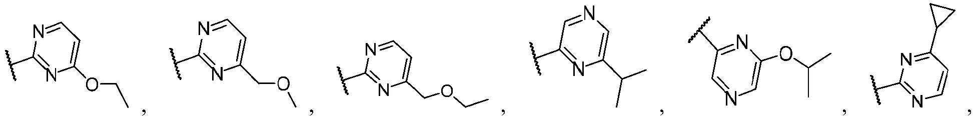 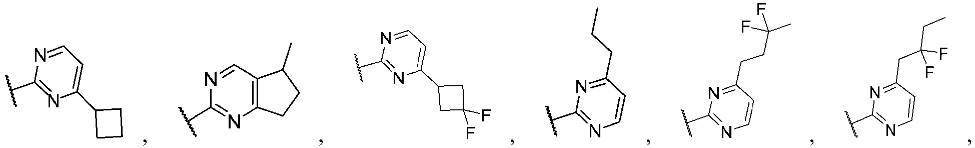 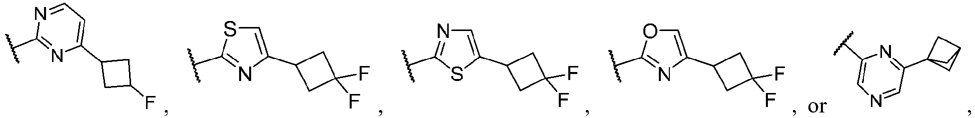 and may also be

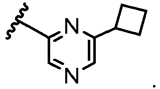

In a preferred embodiment, the compound of formula (I) has a structure shown in formula (I-I): 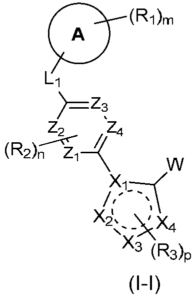 wherein Z₁, Z₂, Z₃, and Z₄ are each independently N or CH; simultaneously, at least one of Z₁, Z₂, Z₃, or Z₄ is N; n is 0, 1, or 2; ring A, ring B, L₁, R₁, R₂, R₃, W, X₁, X₂, X₃, X₄, m, p, and 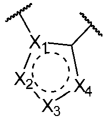 are as defined in any one embodiment of the present disclosure.

In a preferred embodiment, the compound of formula (I) has a structure shown in formula (I-II), (I-III), or (I-IV): 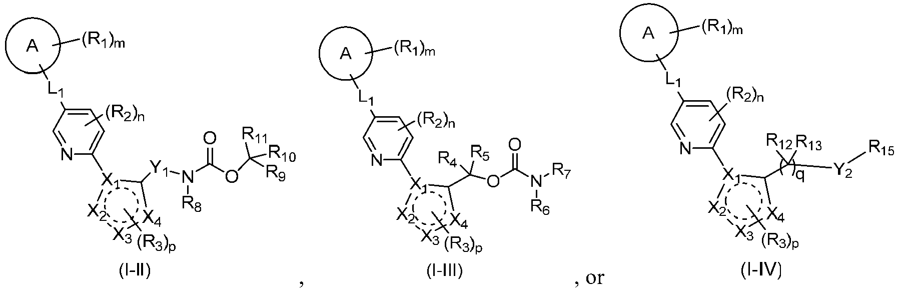
wherein L₁ is 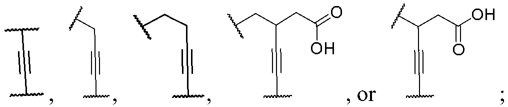
ring A, Y₁, Y₂, R₁, R₂, R₃, R₄, R₅, R₆, R₇, R₈, R₉, R₁₀, R₁₁, R₁₂, R₁₃, R₁₅, X₁, X₂, X₃, X₄, m, n, p, q, and 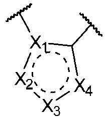 are as defined in any one embodiment of the present disclosure.

In a preferred embodiment, the compound of formula (I) has a structure shown in formula (I-V), (I-VI), or (I-VII): 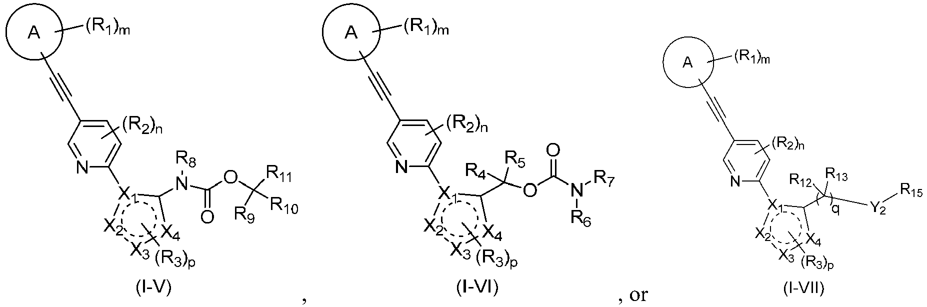 ring A, Y₂, R₁, R₂, R₃, R₄, R₅, R₆, R₇, R₈, R₉, R₁₀, R₁₁, R₁₂, R₁₃, R₁₅, X₁, X₂, X₃, X₄, m, n, p, q, and 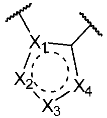 are as defined in any one embodiment of the present disclosure.

In a preferred embodiment, the compound of formula (I) is a compound of formula (I-VIII): 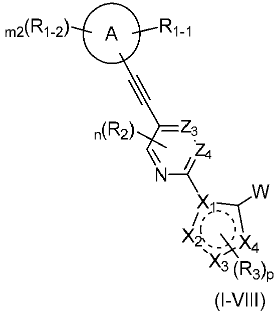
wherein R₁₋₁ is COOH, 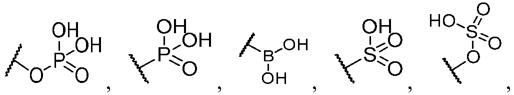 tetrazolyl, -C₁₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the -C₁₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
each R₁₋₂ is independently OH, CN, oxo (=O), halogen, NH₂, C₁₋₄ alkyl, or C₁₋₄ alkoxy; the C₁₋₄ alkyl is optionally substituted with 1, 2, or more halogen substituents;
each Rₐ is independently C₁₋₄ alkyl, C₁₋₄ alkoxy, or halogen;
Z₃ and Z₄ are each independently N or CH;
m2 is 0, 1, 2, or 3;
ring A, R₂, R₃, W, X₁, X₂, X₃, X₄, n, p, and 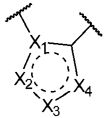 are as defined in any one embodiment of the present disclosure.

In a preferred embodiment, the compound of formula (I) is a compound of formula (I-VIII),
wherein R₁₋₁ is COOH, 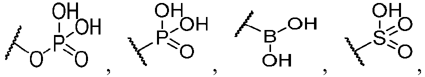 tetrazolyl, -C₁₋₃ alkylene-COOH, -NHC(=O)OC₁₋₆ alkyl, -NHC(=O)C₁₋₆ alkyl, -C(=O)NHC₁₋₆ alkyl, -C(=O)OC₁₋₆ alkyl, - C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, - C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the -C₁₋₃ alkylene-COOH, -NHC(=O)OC₁₋₆ alkyl, -NHC(=O)C₁₋₆ alkyl, -C(=O)NHC₁₋₆ alkyl, - C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents.

In a preferred embodiment, the compound of formula (I) is a compound of formula (I-VIII-1): 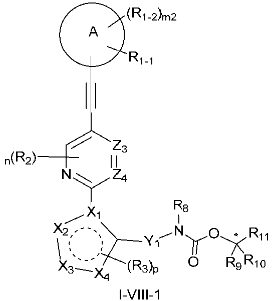
the carbon atom marked with * is a chiral carbon atom or an achiral carbon atom; when the carbon atom is a chiral carbon atom, the configuration of the chiral carbon atom is R and/or S;
ring A is C₃₋₁₀ cycloalkyl, 3- to 10-membered heterocycloalkyl, or 5- to 10-membered heteroaryl;
m2 is 0, 1, or 2;
R₁₋₁ is OH, COOH, C₁₋₆ alkyl, 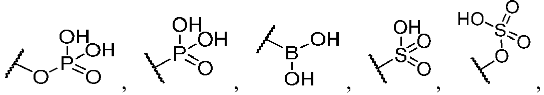 -C₁₋₃ alkylene-COOH, -NHC(=O)OC₁₋₆ alkyl, -NHS(=O)₂OH, -C₁₋₃ alkylene-NHS(=O)₂OH, -C₁₋₃ alkyleneS(=O)₂OH, or -C₁₋₃ alkylene-S(=O)₂C₁₋₆ alkyl; the C₁₋₆ alkyl, -C₁₋₃ alkylene-COOH, -NHC(=O)OC₁₋₆ alkyl, - NHS(=O)₂OH, -C₁₋₃ alkylene-NHS(=O)₂OH, -C₁₋₃ alkylene-S(=O)₂OH, and -C₁₋₃ alkylene-S(=O)₂C₁₋₆ alkyl are each independently optionally substituted with 1, 2, or more Rₐ substituents;
in R₁₋₁, each Rₐ is independently C₁₋₄ alkyl, halogen, OH, oxo (=O), or CN; when the number of Rₐ is 2 or more, the OH and oxo (=O) are not attached to the same carbon atom;
each R₁₋₂ is independently halogen, C₁₋₆ alkyl, C₁₋₆ alkoxy, OH, or CN; the C₁₋₆ alkyl and C₁₋₆ alkoxy are each independently optionally substituted with 1, 2, or more Rₐ substituents;
in R₁₋₂, each Rₐ is independently halogen;
Z₃ and Z₄ are each independently C, CH, or N;
each R₂ is independently halogen, C₁₋₄ alkyl, or C₃₋₆ cycloalkyl; the C₁₋₄ alkyl and C₃₋₆ cycloalkyl are each independently optionally substituted with 1, 2, or more halogen or deuterium substituents;
n is 0 or 1;
X₁ is C or N;
X₂ is CH, O, S, or N;
X₃ is C, CH₂, O, S, or N;
X₄ is C, CH, N, or NH;
and at least one of X₁, X₂, X₃, or X₄ is independently O, S, or N;
the double line comprising solid and dashed lines represents a single bond or a double bond; simultaneously, 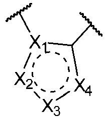 contains at least one double bond;
each R₃ is independently halogen, C₁₋₄ alkyl, or oxo;
p is 1 or 2;
Y₁ is a chemical bond or C₁₋₆ alkylene;
R₈ is H or deuterium;
R₉ is H or deuterium;
R₁₀ is independently H, deuterium, or C₁₋₆ alkyl; the C₁₋₆ alkyl is optionally substituted with 1, 2, 3, or 4 R_{f} substituents; each R_{f} is independently deuterium or halogen;
R₁₁ is C₁₋₆ alkyl, C₆₋₁₀ aryl, 5- to 10-membered heteroaryl, or C₃₋₈ cycloalkyl; the C₁₋₆ alkyl, C₆₋₁₀ aryl, 5- to 10-membered heteroaryl, and C₃₋₈ cycloalkyl are each independently optionally substituted with 1, 2, or more R_{g} substituents;
each R_{g} is independently deuterium, halogen, C₁₋₆ alkyl, or C₃₋₆ cycloalkyl; the C₁₋₆ alkyl and C₃₋₆ cycloalkyl are each independently optionally substituted with 1, 2, or more deuterium or halogen substituents;
the heteroatoms in the 3- to 10-membered heterocycloalkyl and 5- to 10-membered heteroaryl are each independently selected from 1, 2, or 3 types of N, O, and S, and the number of heteroatoms is independently 1, 2, or 3.

In a preferred embodiment, the compound of formula (I) is a compound of formula (I-VIII-2): 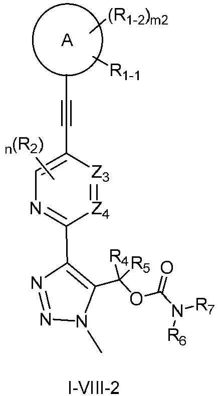
ring A is C₃₋₈ cycloalkyl, 4- to 6-membered oxacycloalkyl, or 4- to 6-membered azacycloalkyl; the number of heteroatoms in the 4- to 6-membered oxacycloalkyl and 4- to 6-membered azacycloalkyl is 1;
m2 is 0, 1, or 2;
R₁₋₁ is COOH, 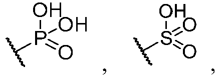 tetrazolyl, -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, or -C₁₋₃ alkylene-tetrazolyl; the -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, and -C₁₋₃ alkylene-tetrazolyl are each independently optionally substituted with 1, 2, or more Rₐ substituents;
each R₁₋₂ is independently halogen, C₁₋₆ alkyl, C₁₋₆ alkoxy, OH, or CN; the C₁₋₆ alkyl and C₁₋₆ alkoxy are each independently optionally substituted with 1, 2, or more Rₐ substituents;
each Rₐ is independently halogen;
Z₃ and Z₄ are each independently C, CH, or N;
each R₂ is independently halogen, C₁₋₄ alkyl, C₁₋₄ alkoxy, or C₃₋₆ cycloalkyl; the C₁₋₄ alkyl, C₁₋₄ alkoxy, and C₃₋₆ cycloalkyl are each independently optionally substituted with 1, 2, or more halogen or deuterium substituents;
n is 0 or 1;
R₄ and R₅ are each independently H or deuterium;
R₆ is C₁₋₆ alkyl; the C₁₋₆ alkyl is optionally substituted with 1, 2, or more R_{c} substituents;
in R₆, each R_{c} is independently halogen;
R₇ is C₁₋₆ alkyl, C₃₋₈ cycloalkyl, C₆₋₁₀ aryl, or 5- to 10-membered heteroaryl; the C₁₋₆ alkyl is optionally substituted with 1, 2, or more R_{c} substituents; the C₃₋₈ cycloalkyl, C₆₋₁₀ aryl, and 5- to 10-membered heteroaryl are each independently optionally substituted with 1, 2, or more Rₑ substituents;
in R₇, each R_{c} is independently deuterium, halogen, C₁₋₆ alkyl, C₃₋₈ cycloalkyl, C₆₋₁₀ aryl, or 5- to 10-membered heteroaryl; the C₁₋₆ alkyl, C₃₋₈ cycloalkyl, C₆₋₁₀ aryl, and 5- to 10-membered heteroaryl are each independently optionally substituted with 1, 2, or more R_{c-1} substituents;
each R_{c-1} is independently deuterium, halogen, or C₁₋₆ alkyl;
each Rₑ is independently deuterium or halogen;
the heteroatom in the 5- to 10-membered heteroaryl is independently selected from 1, 2, or 3 types of N, O, and S, and the number of the heteroatom is independently 1, 2, or 3.

In a preferred embodiment, the compound of formula (I) is a compound of formula (I-VIII-3): 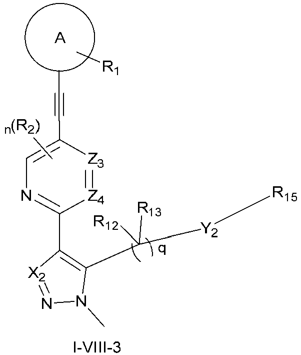
ring A is C₃₋₈ cycloalkyl;
R₁ is COOH or -C₁₋₃ alkylene-COOH;
each R₂ is independently C₁₋₄ alkyl;
n is 0 or 1;
Z₃ and Z₄ are each independently C, CH, or N;
X₂ is CH or N;
R₁₂ and R₁₃ are each independently H, deuterium, or halogen;
when q is 0, Y₂ is N(R₁₄), -C(=O)N(R₁₄)-, or O;
when q is 1, 2, or 3, Y₂ is a chemical bond, N(R₁₄), -C(=O)N(R₁₄)-, or O;
R₁₄ is H, deuterium, or C₁₋₆ alkyl; R₁₅ is C₁₋₆ alkyl, 3- to 10-membered heterocyclyl, or 5- to 10-membered heteroaryl; the C₁₋₆ alkyl, 3- to 10-membered heterocyclyl, and 5- to 10-membered heteroaryl are each independently optionally substituted with 1, 2, or more Rᵢ substituents;
each Rᵢ is independently halogen, oxo (=O), C₃₋₆ cycloalkyl, C₁₋₆ alkyl, or C₁₋₆ alkoxy; the C₁₋₆ alkyl, C₃₋₆ cycloalkyl, and C₁₋₆ alkoxy are each independently optionally substituted with 1, 2, or more Rᵢ₋₁ substituents;
each Rᵢ₋₁ is independently halogen, C₁₋₆ alkyl, C₃₋₆ cycloalkyl, or C₁₋₆ alkoxy;
the heteroatoms in the 3- to 10-membered heterocycloalkyl and 5- to 10-membered heteroaryl are each independently selected from 1, 2, or 3 types of N, O, and S, and the number of heteroatoms is independently 1, 2, or 3.

In a preferred embodiment, R₁₋₁ is COOH, 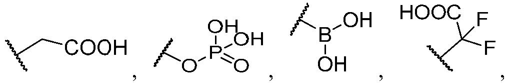 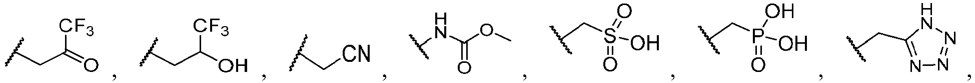 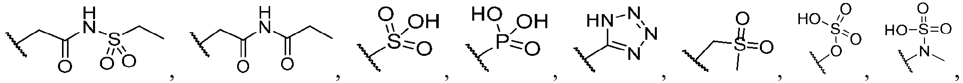 or 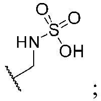 for example, COOH, 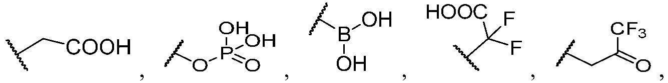 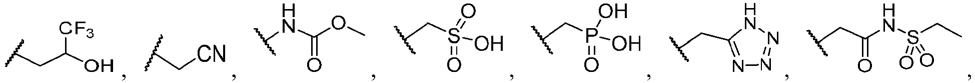 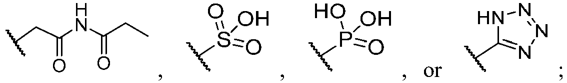 or, for example, COOH, 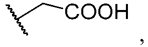 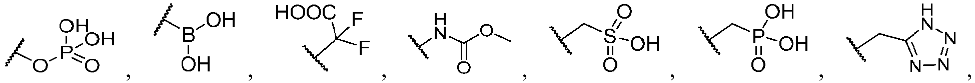 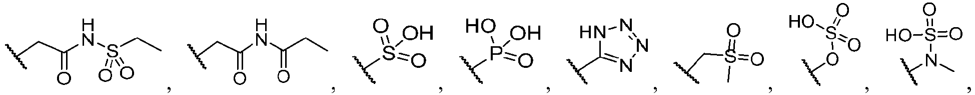 or

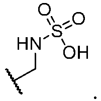

In a preferred embodiment, R₁₋₂ is independently OH, F, Cl, CN, methyl, -CH₂F, -CHF₂, -CF₃, 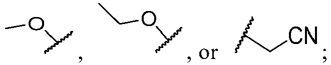 for example, OH, F, Cl, CN, methyl, -CH₂F, -CHF₂, -CF₃, 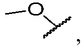 or

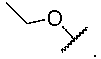

In a preferred embodiment, the compound of formula (I) is any one of the following compounds: 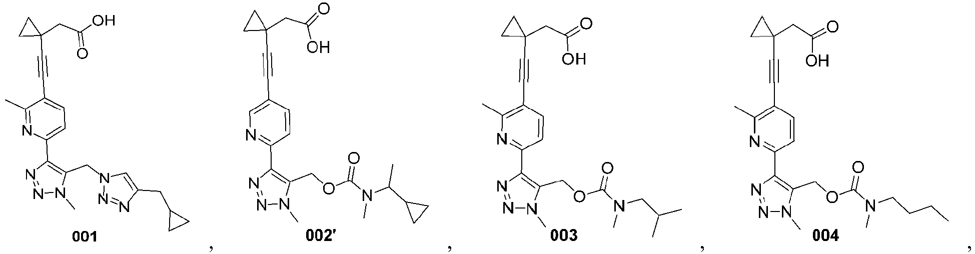 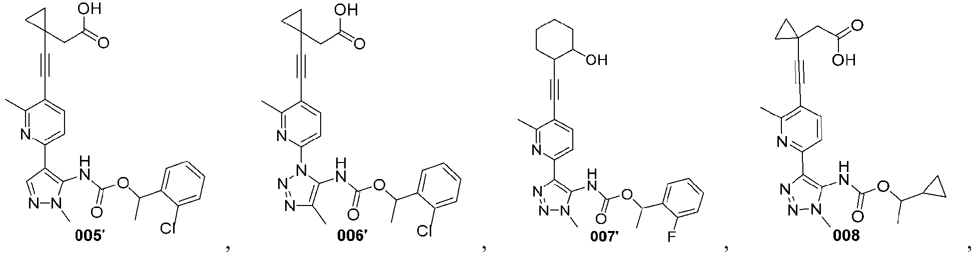 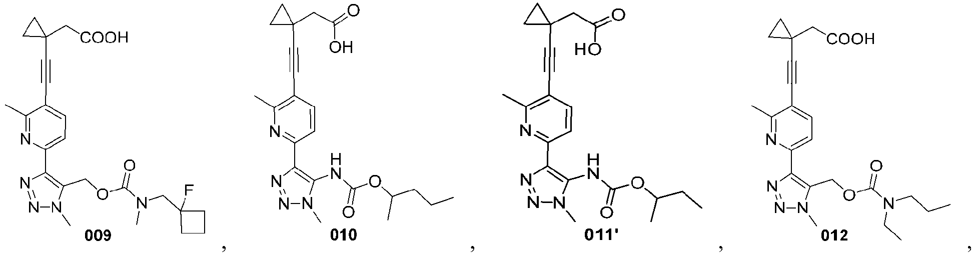 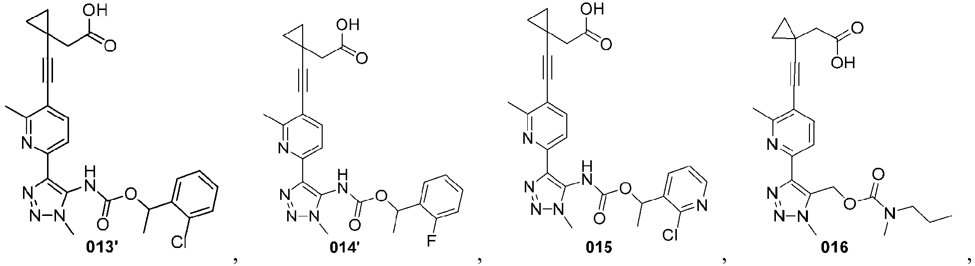 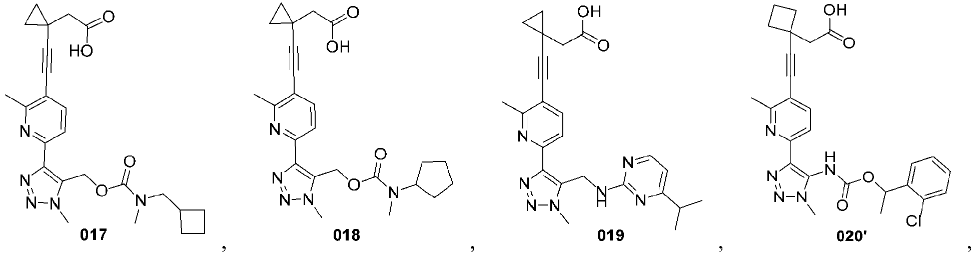 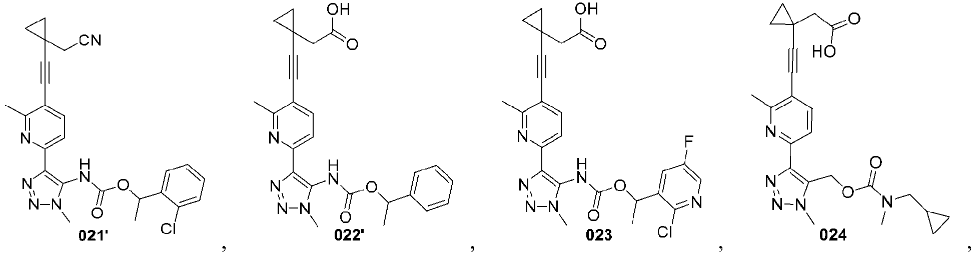 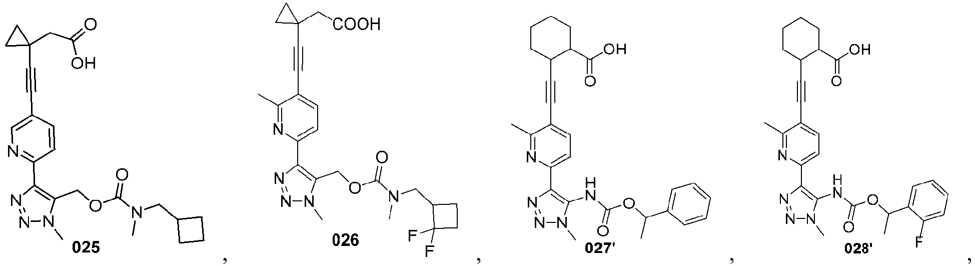 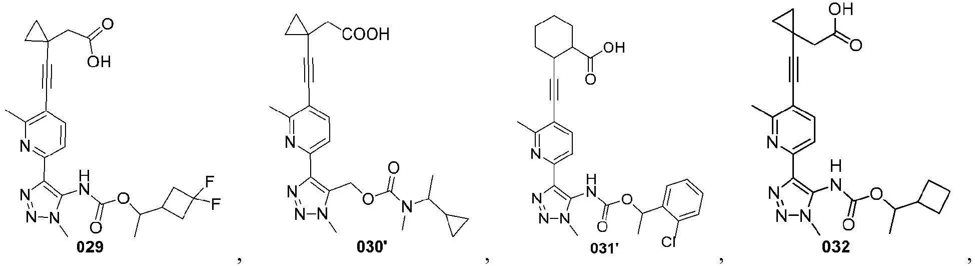 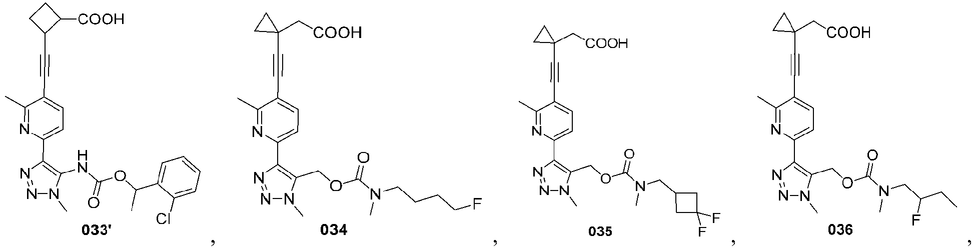 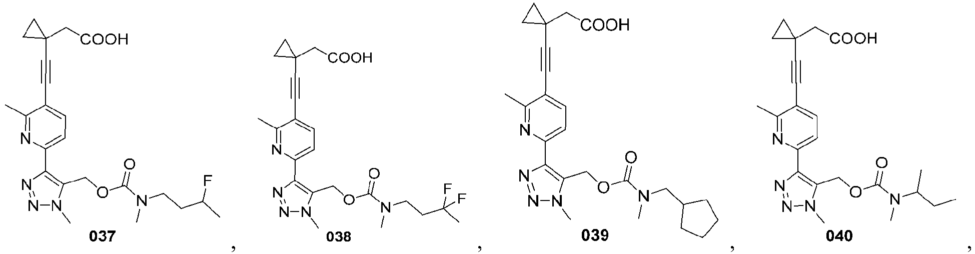 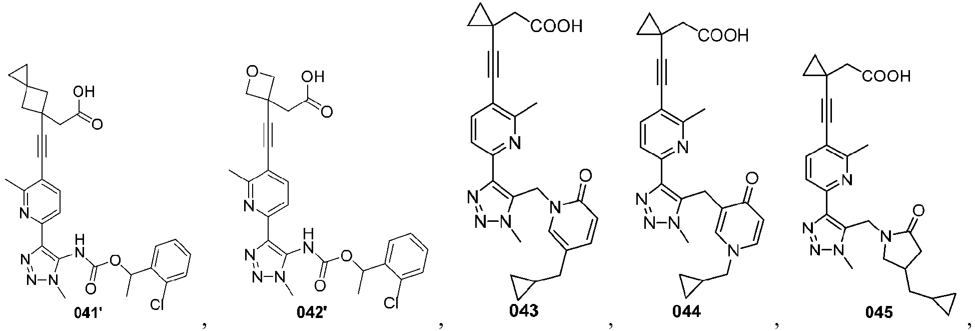 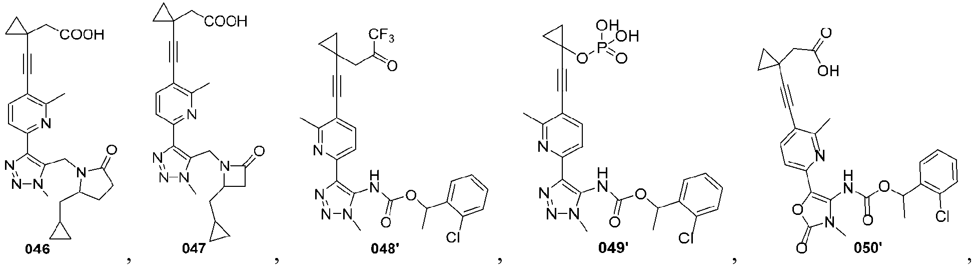 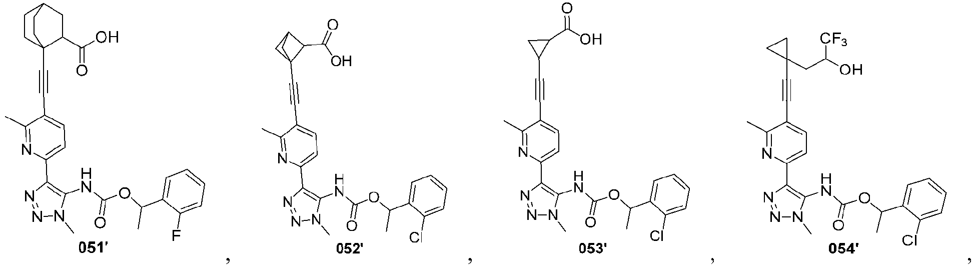 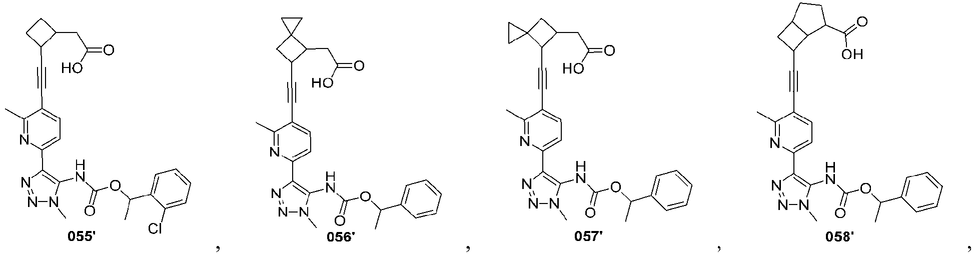 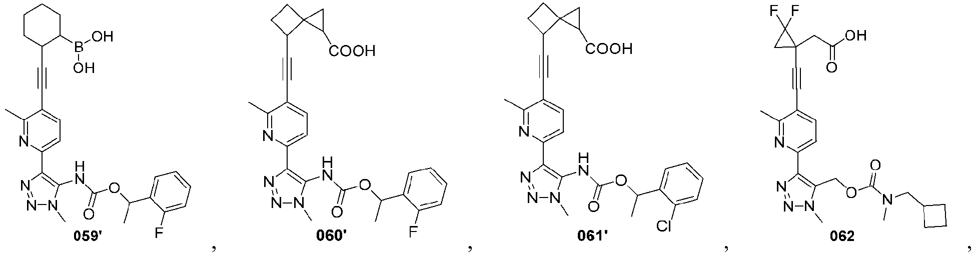 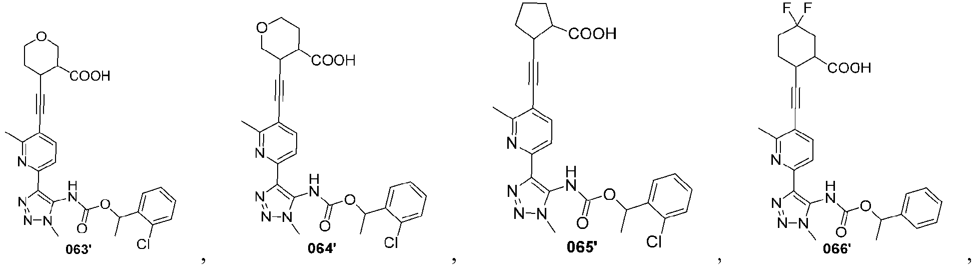 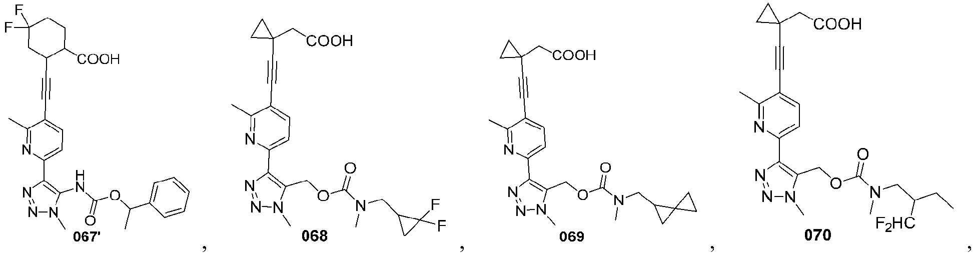 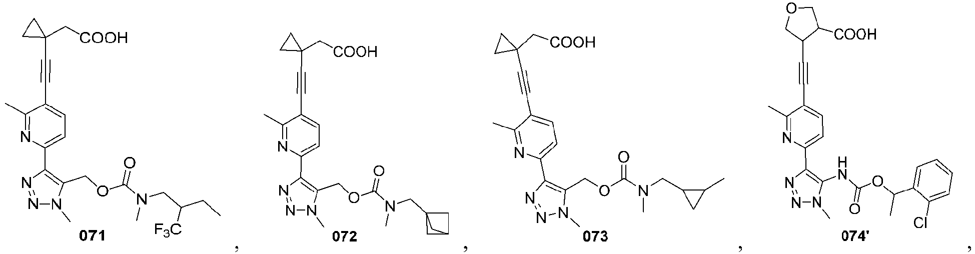 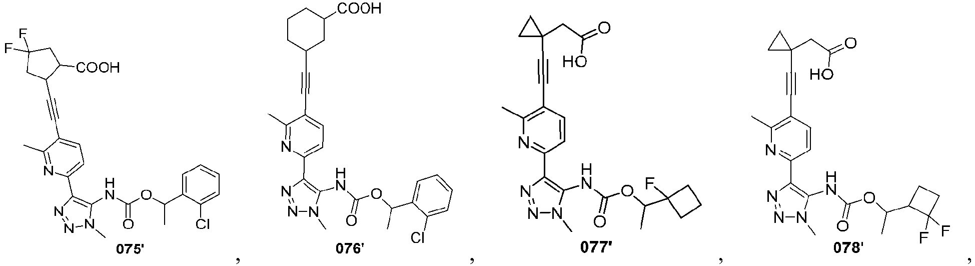 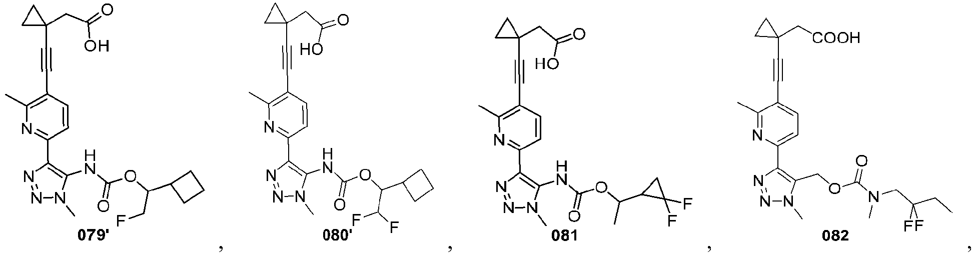 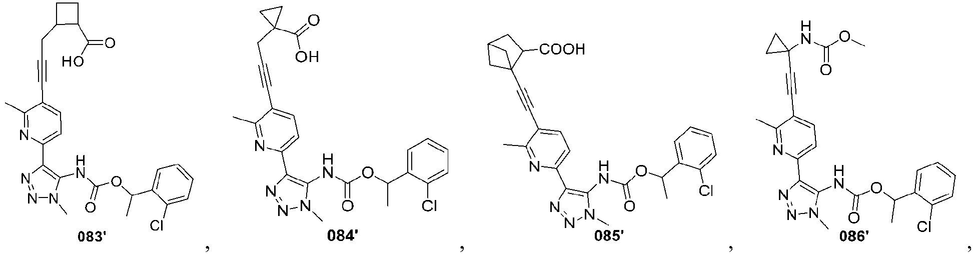 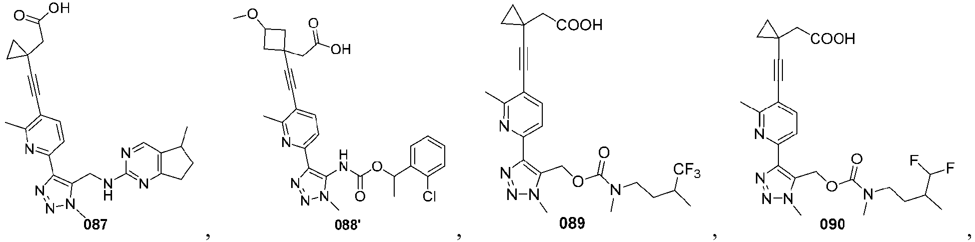 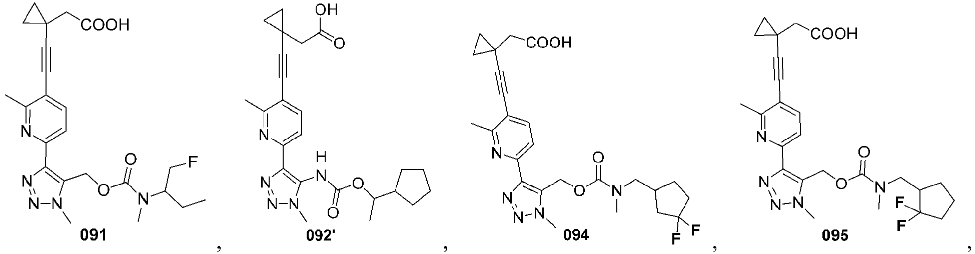 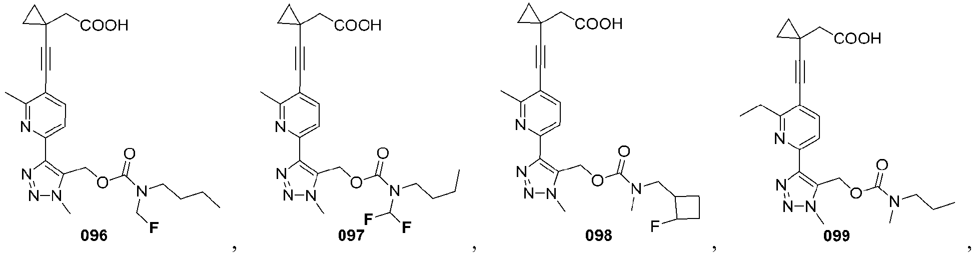 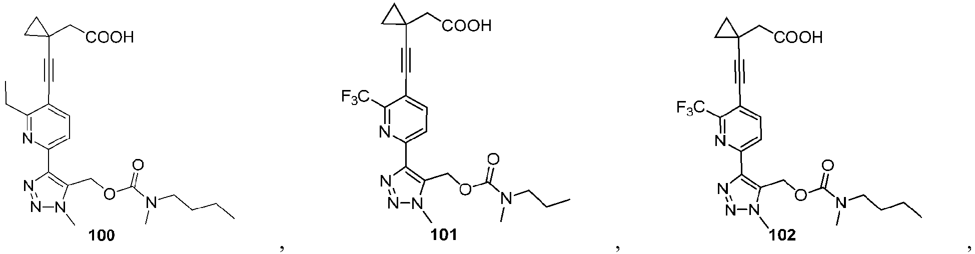 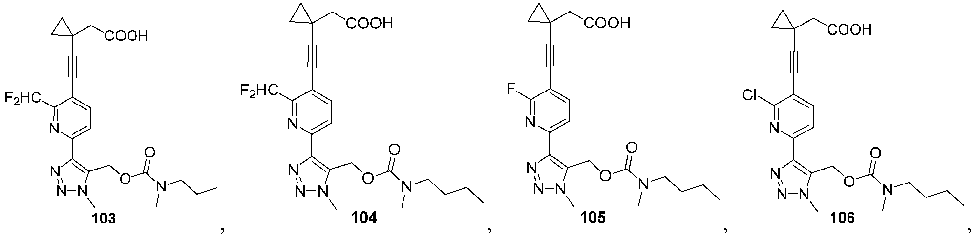 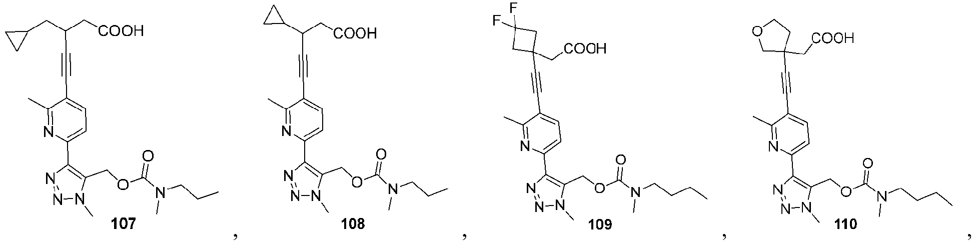 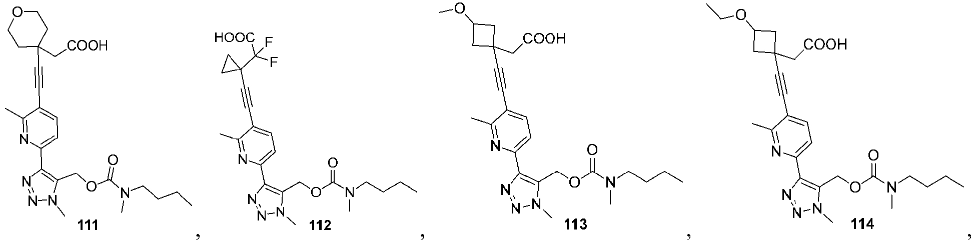 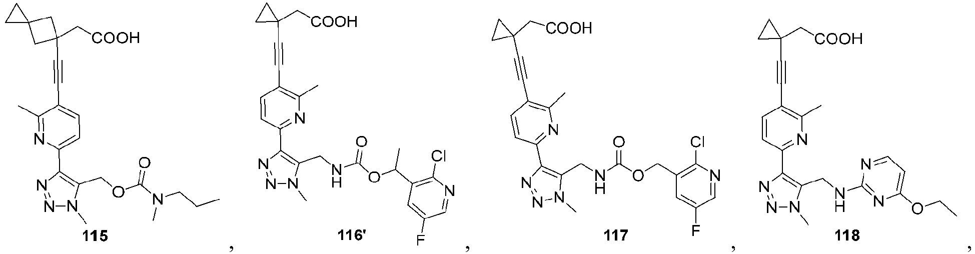 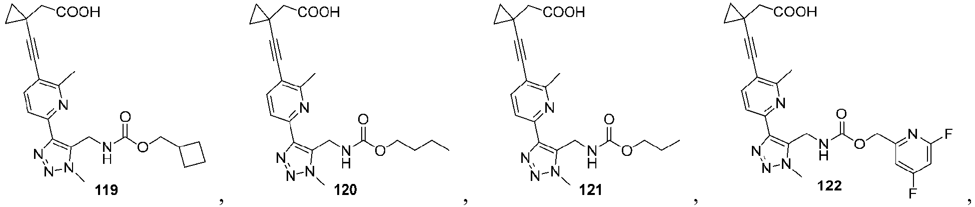 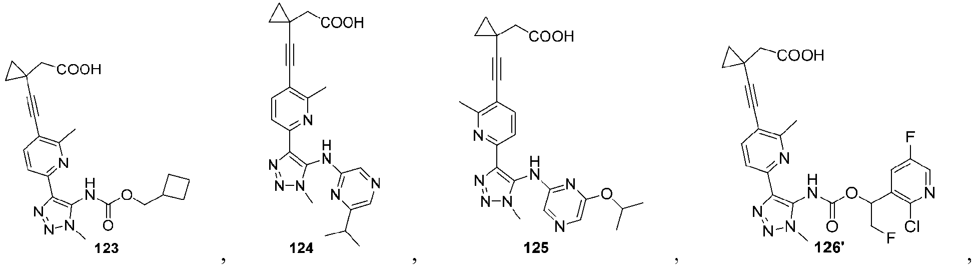 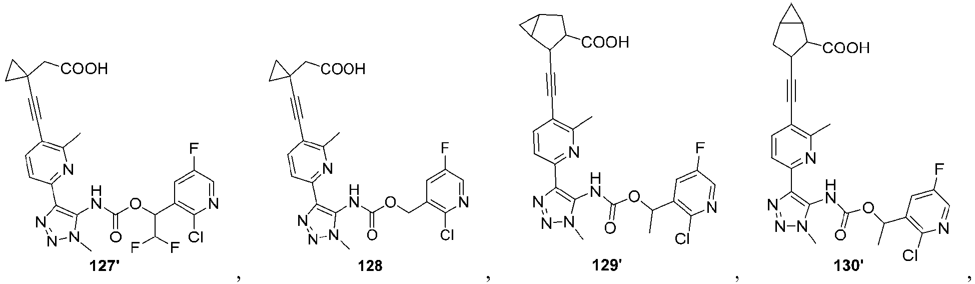 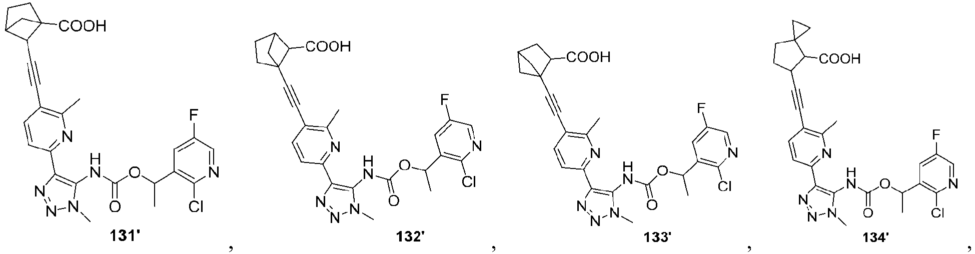 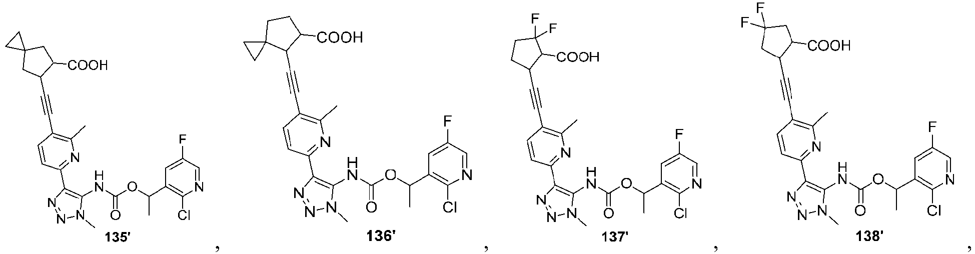 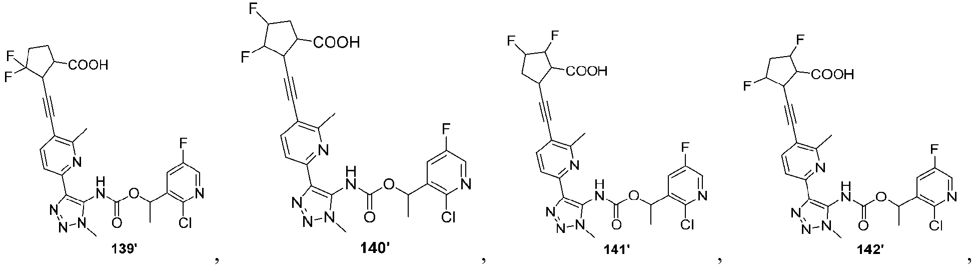 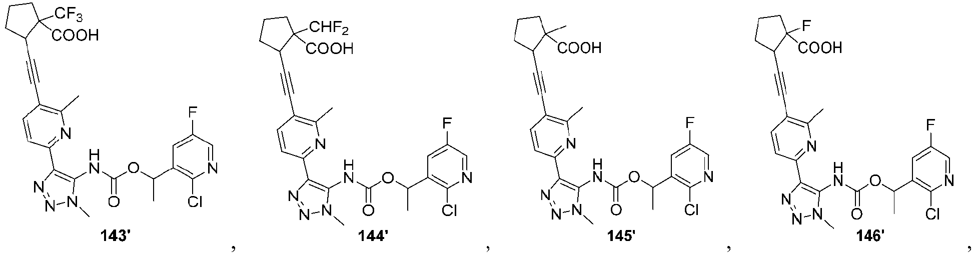 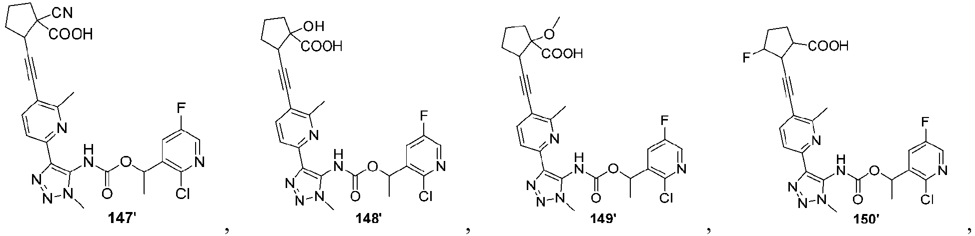 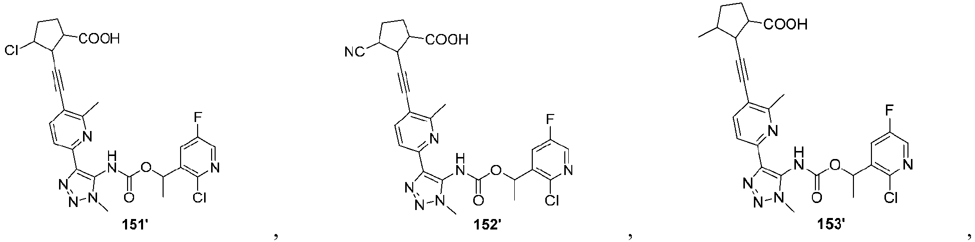 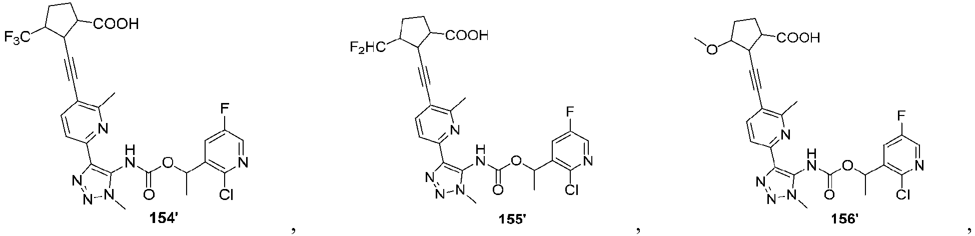 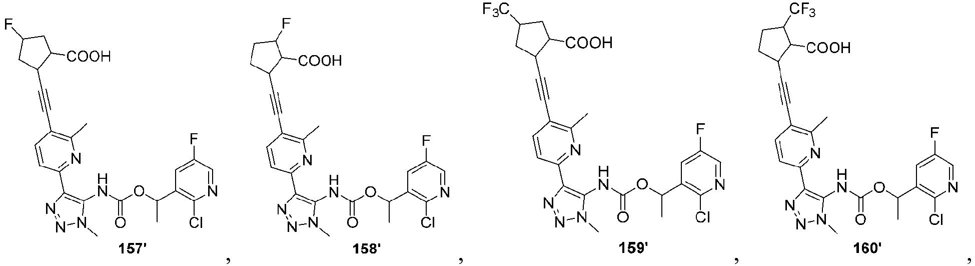 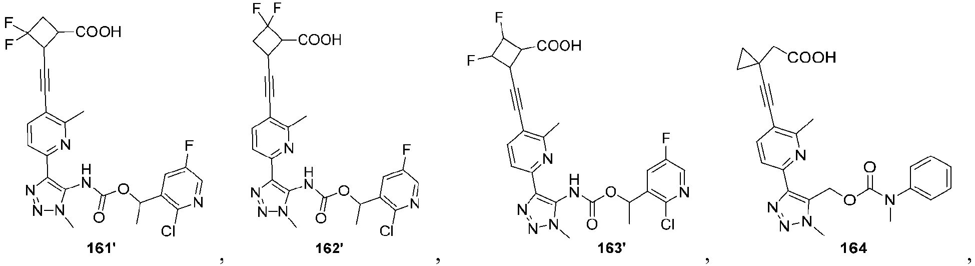 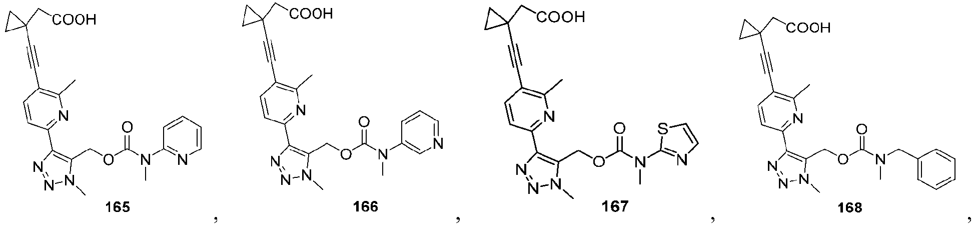 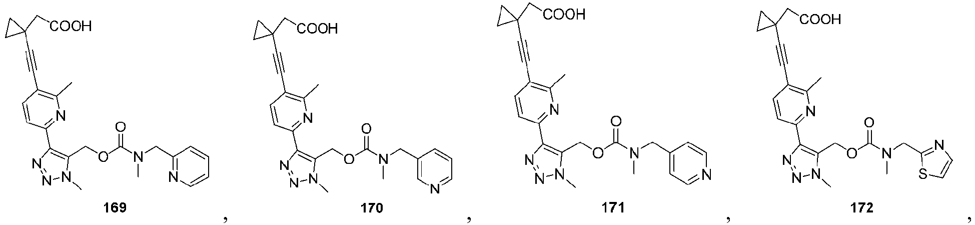 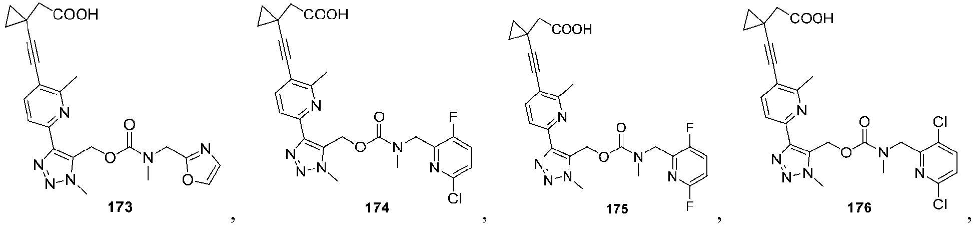 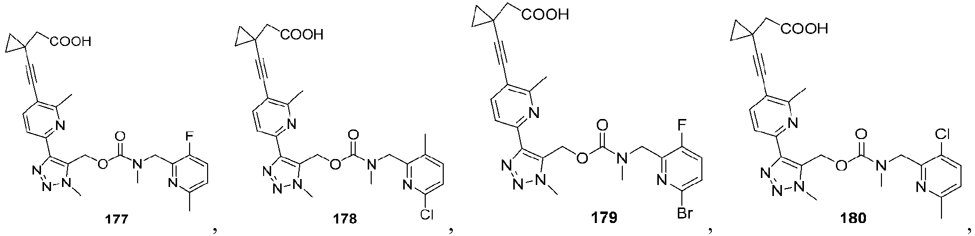 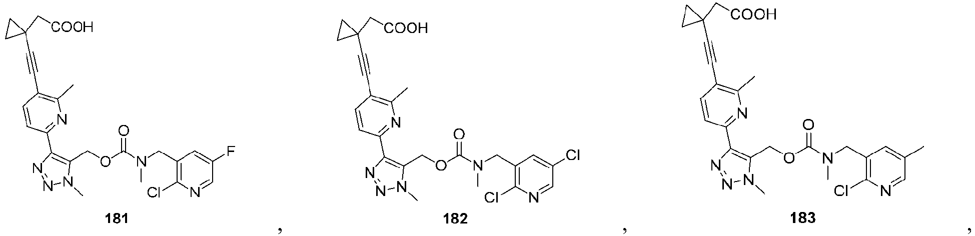 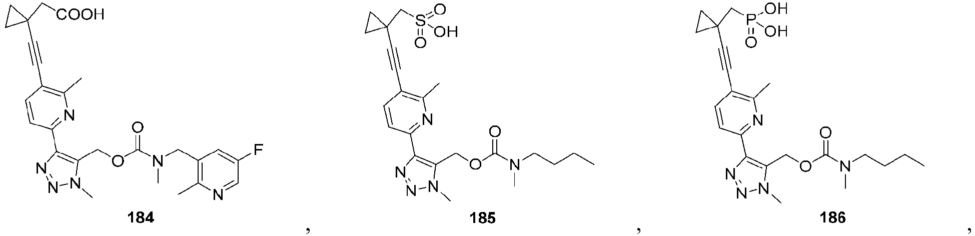 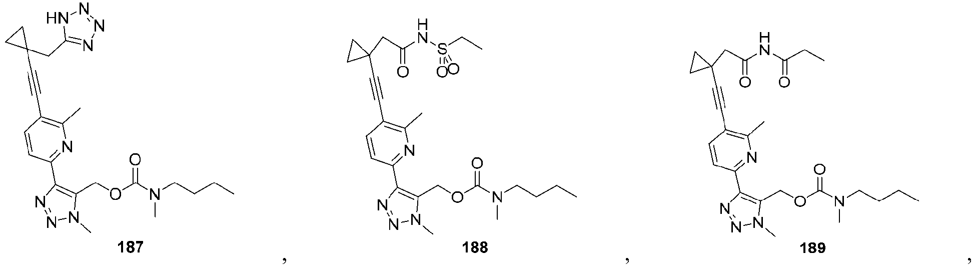 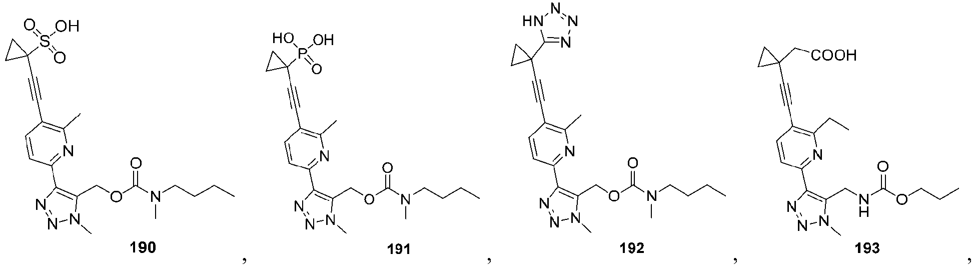 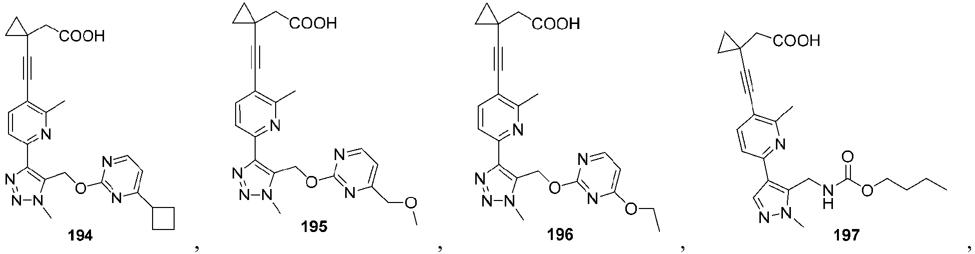 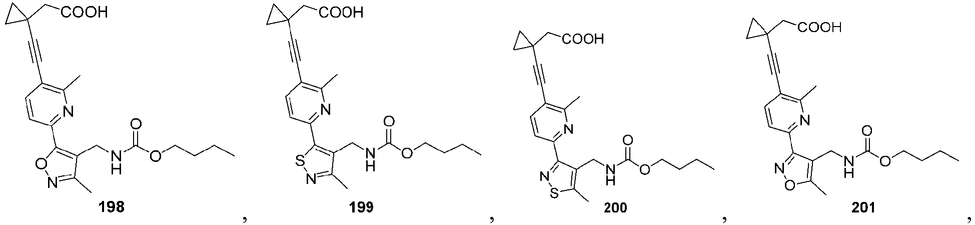 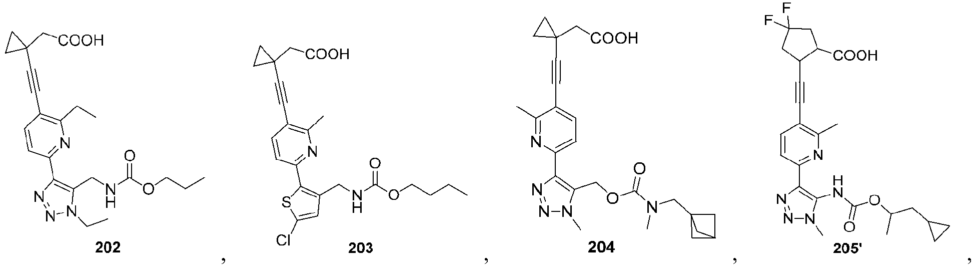 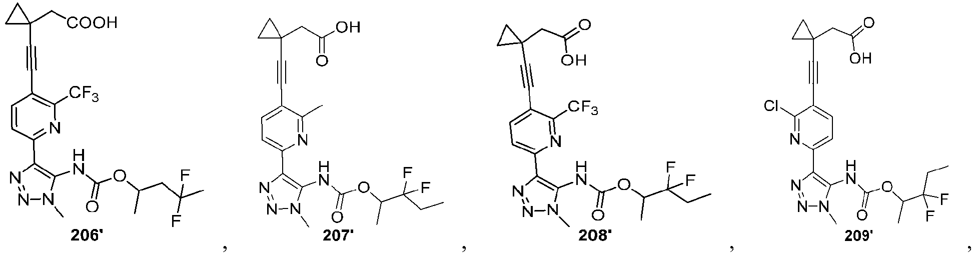 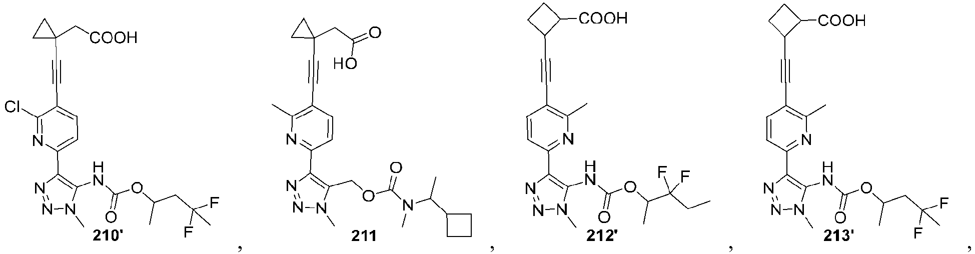 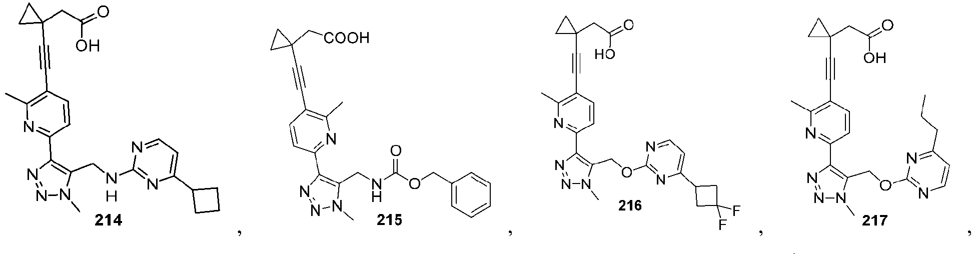 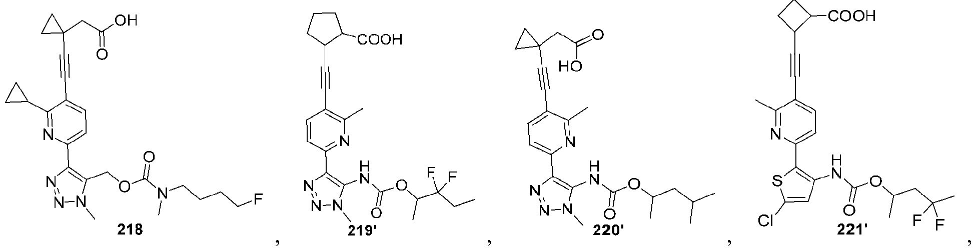 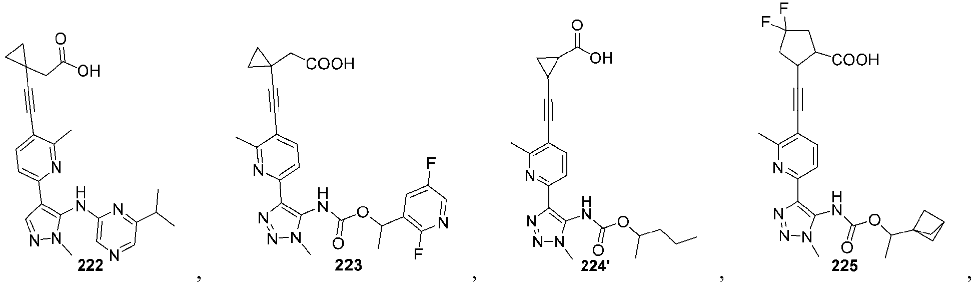 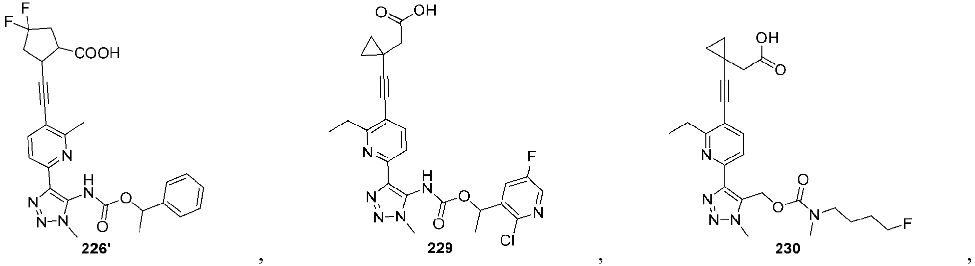 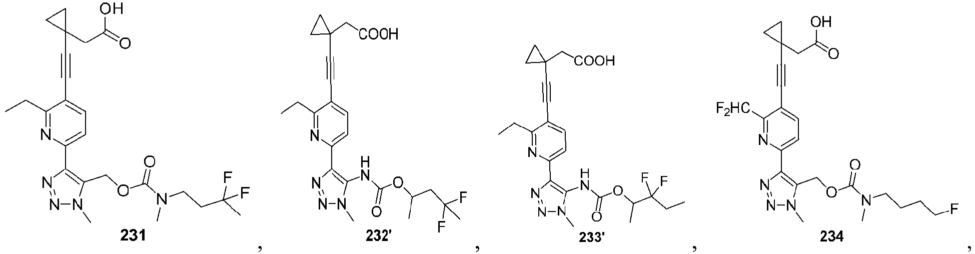 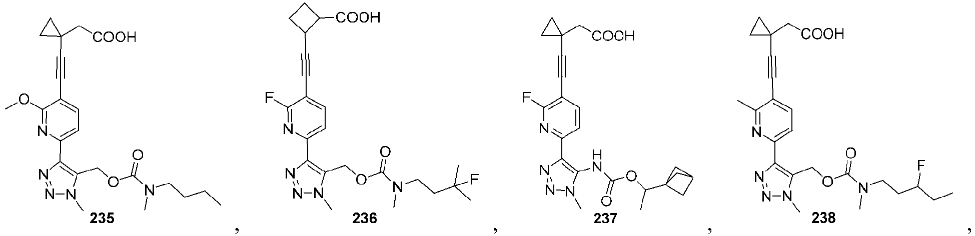 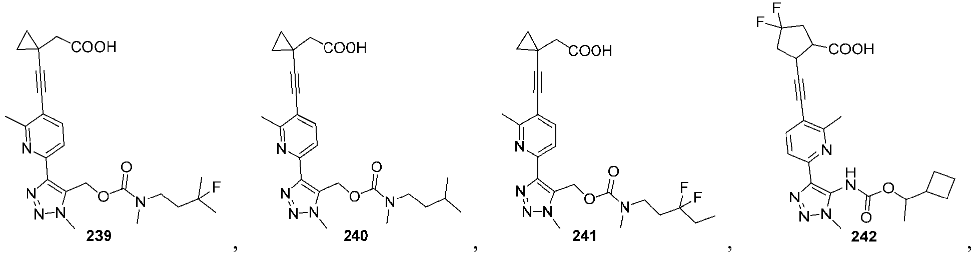 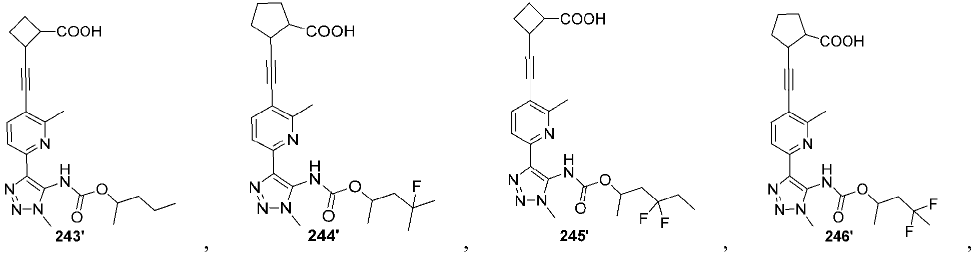 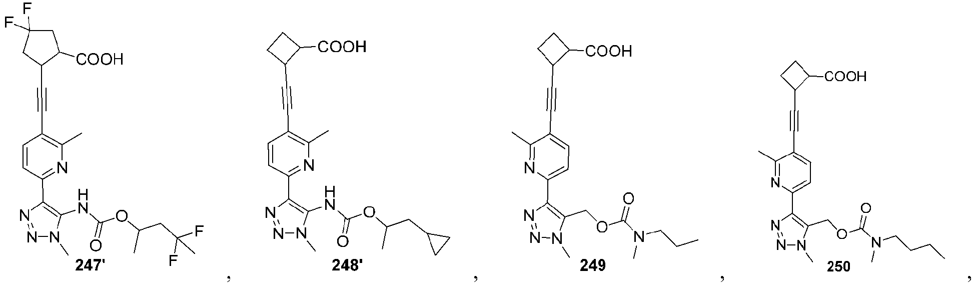 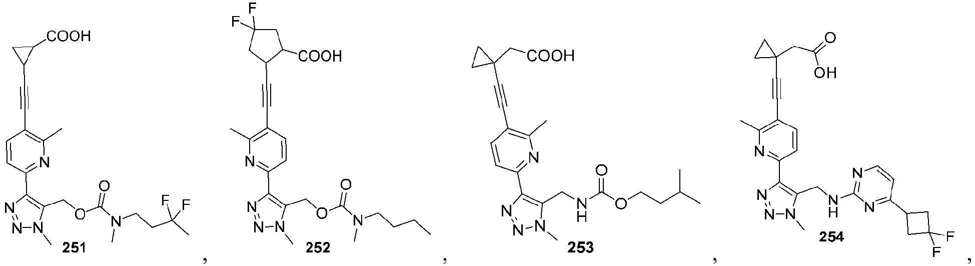 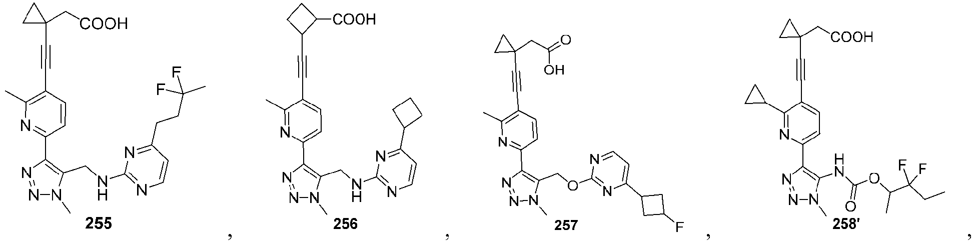 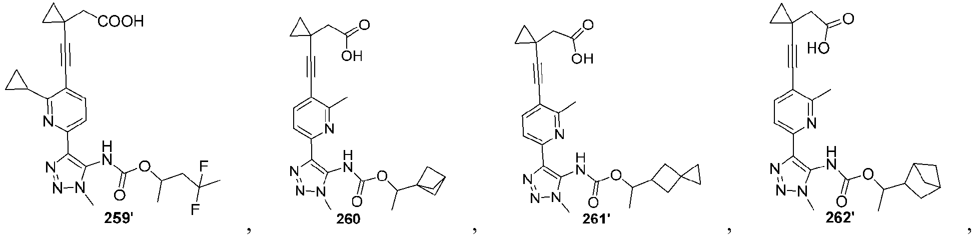 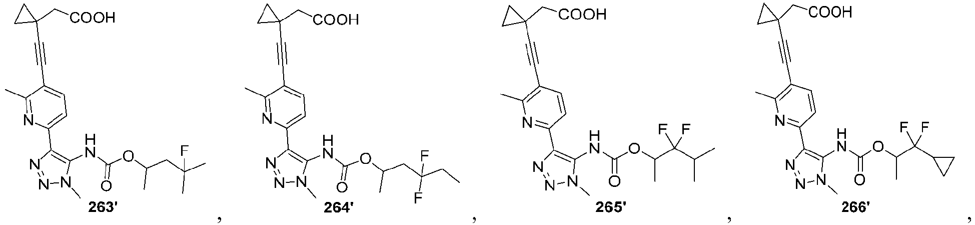 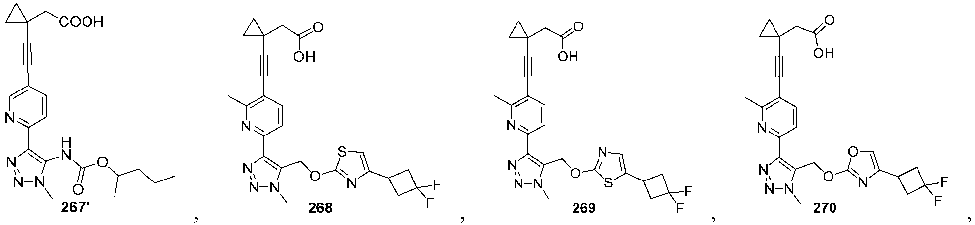 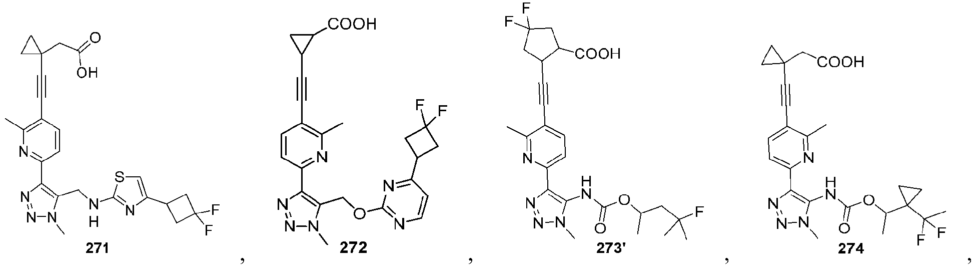 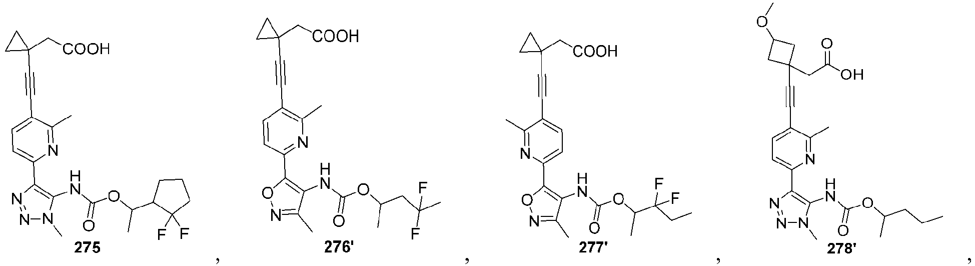 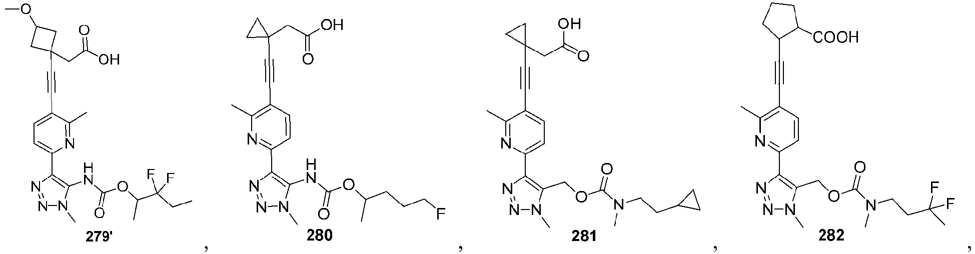 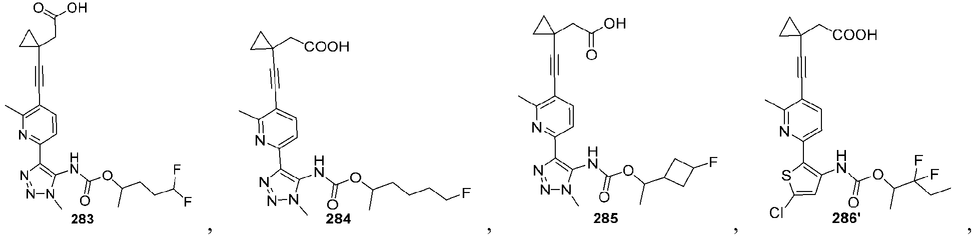 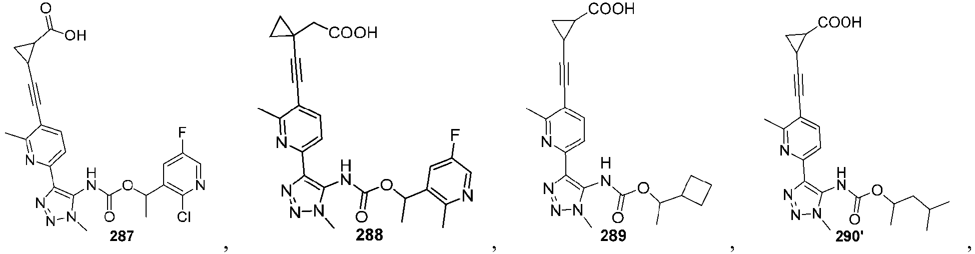 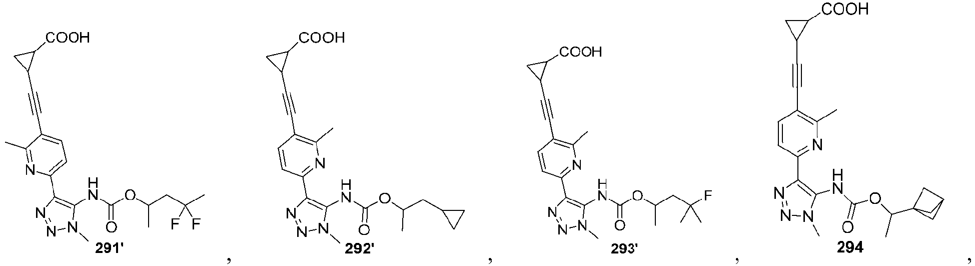 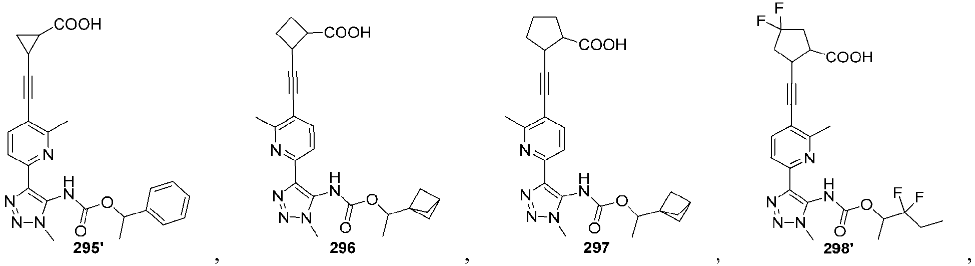 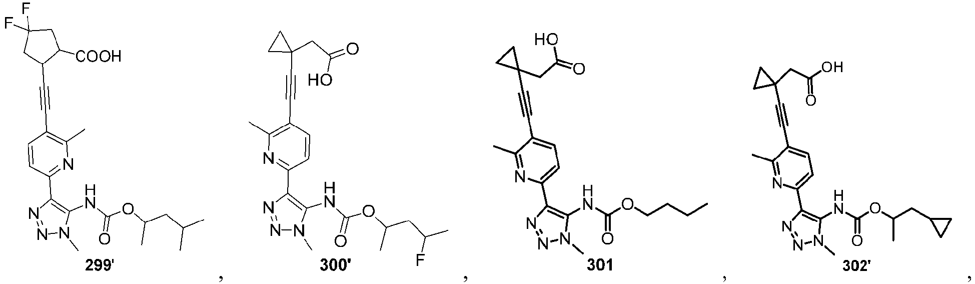 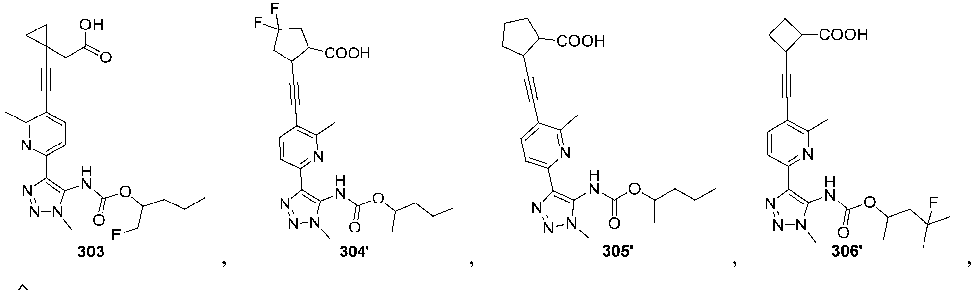 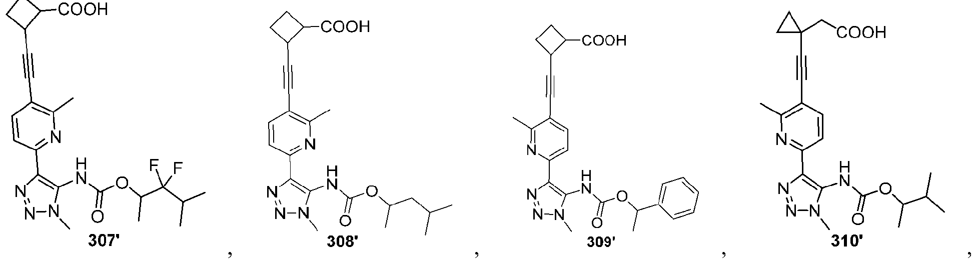 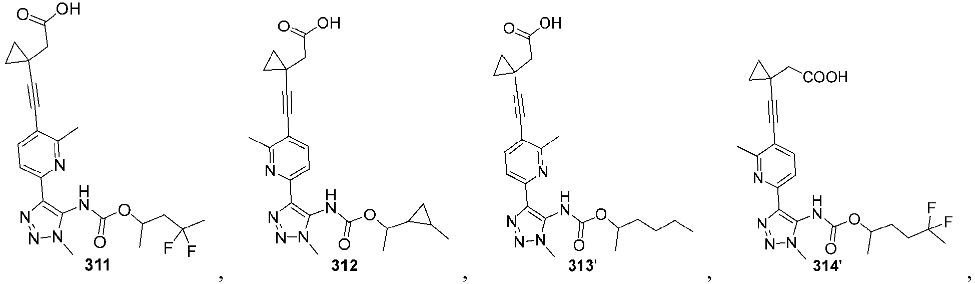 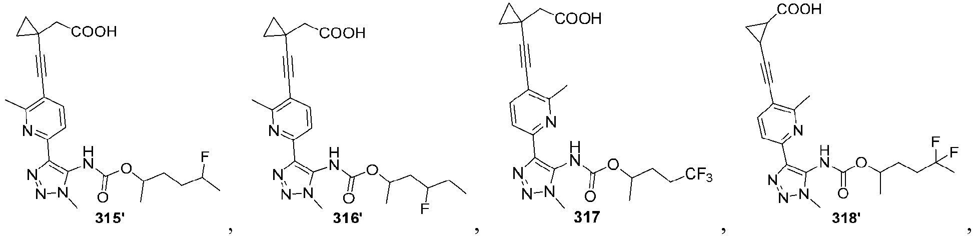 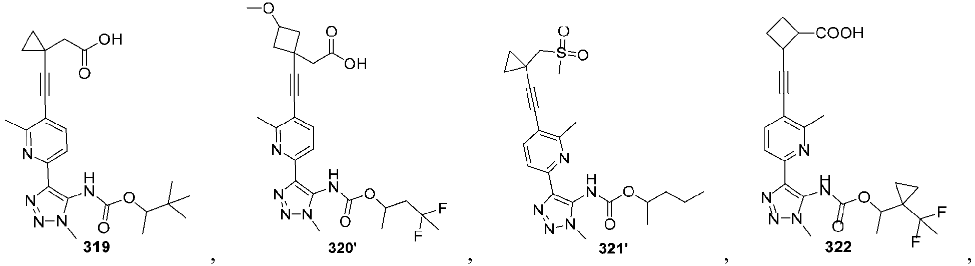 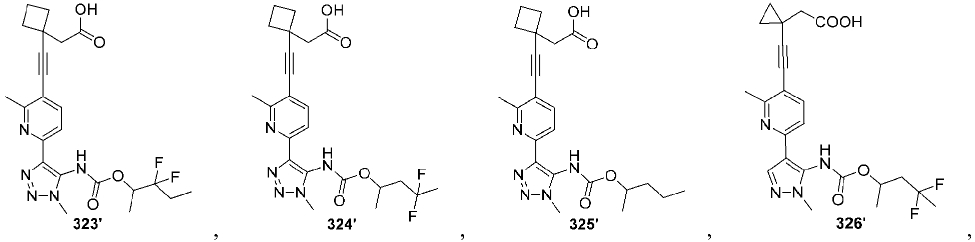 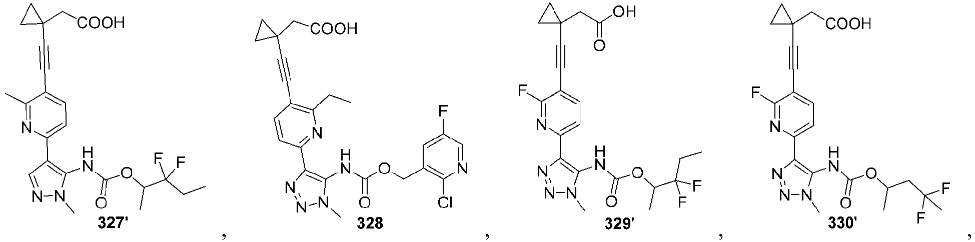 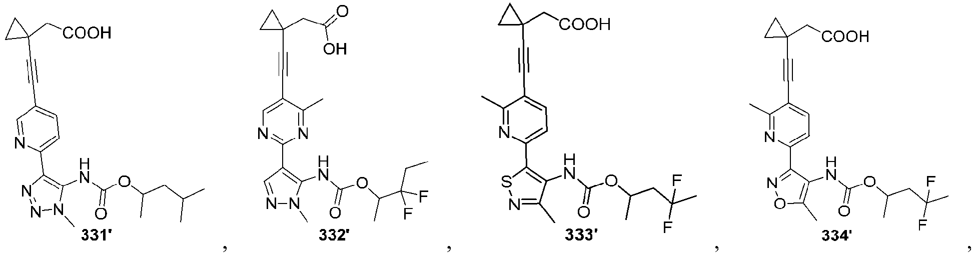 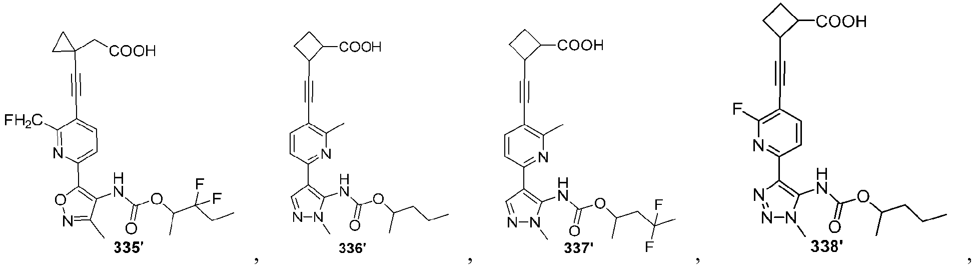 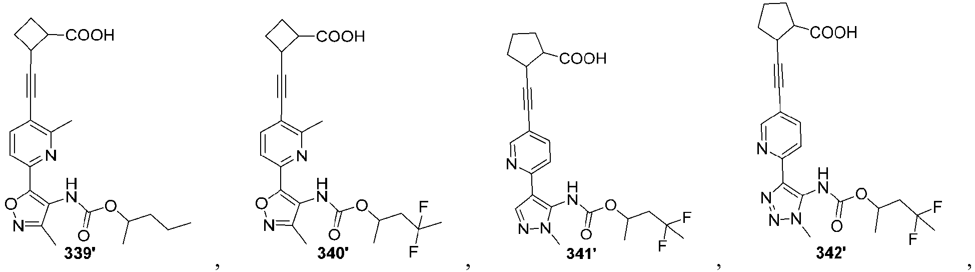 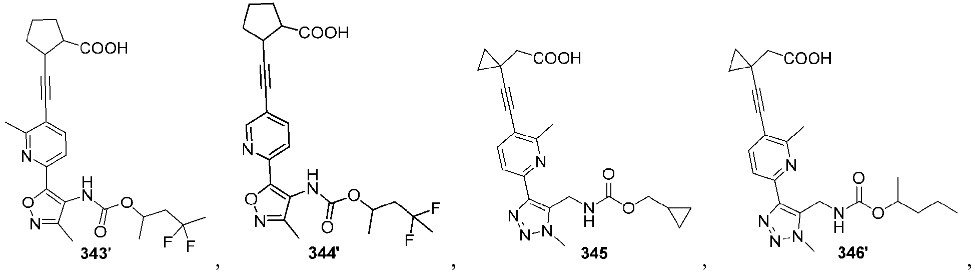 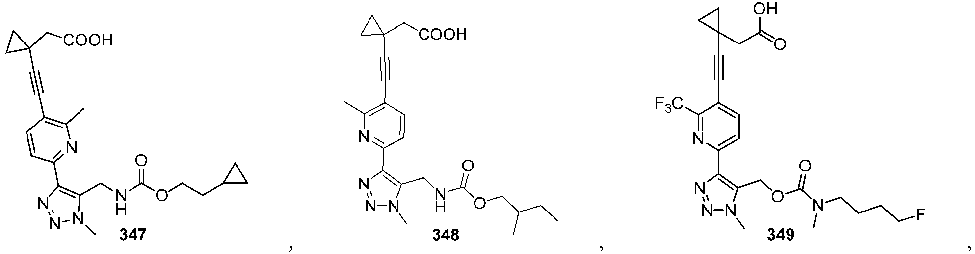 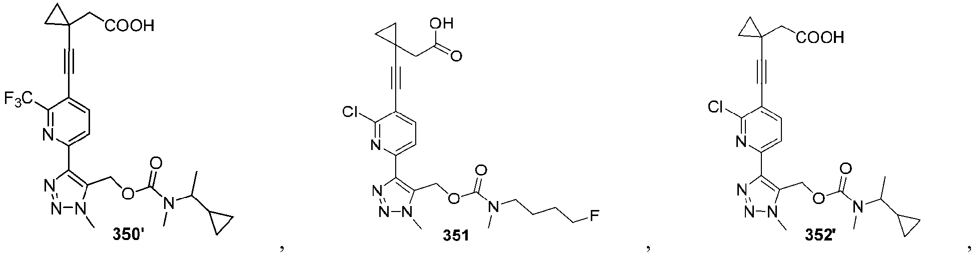 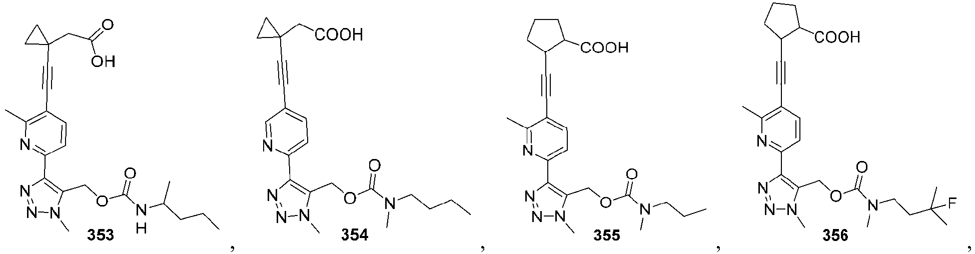 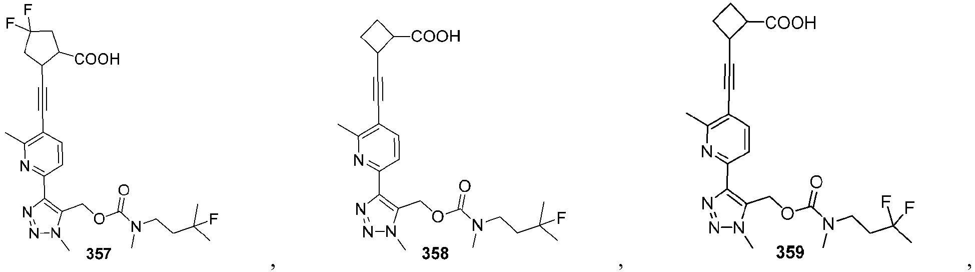 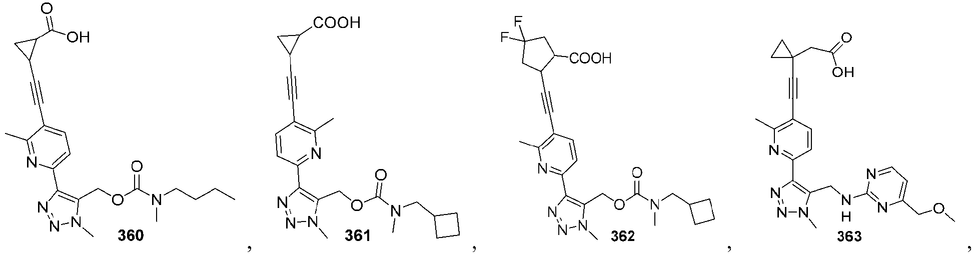 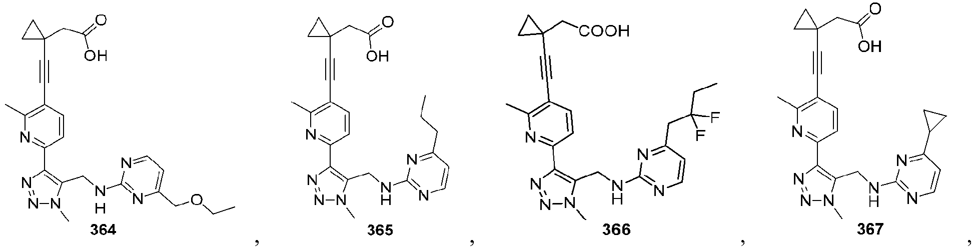 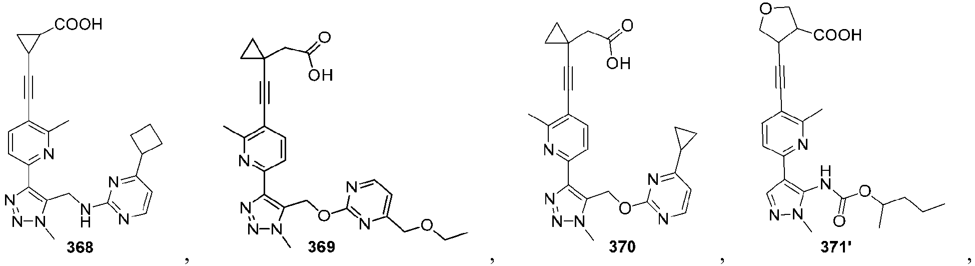 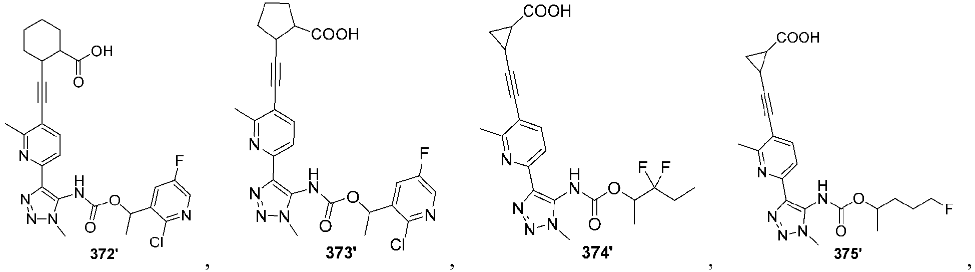 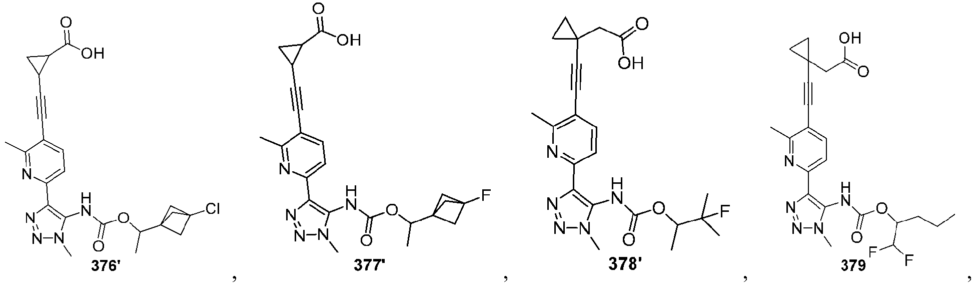 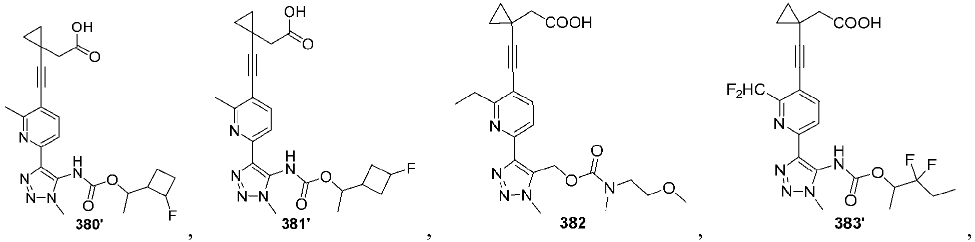 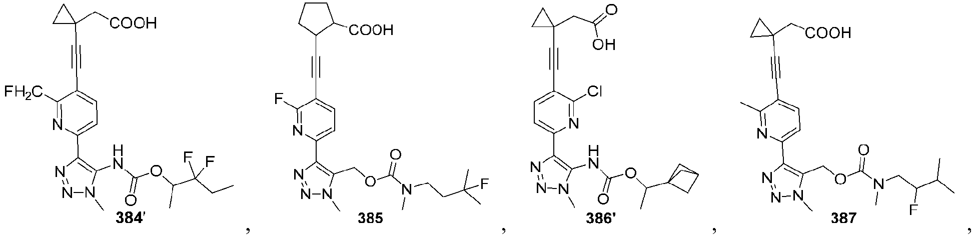 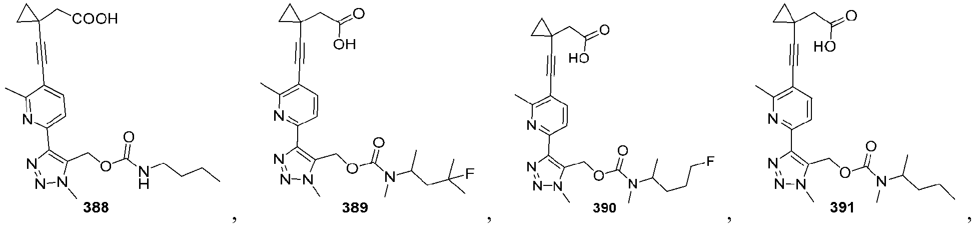 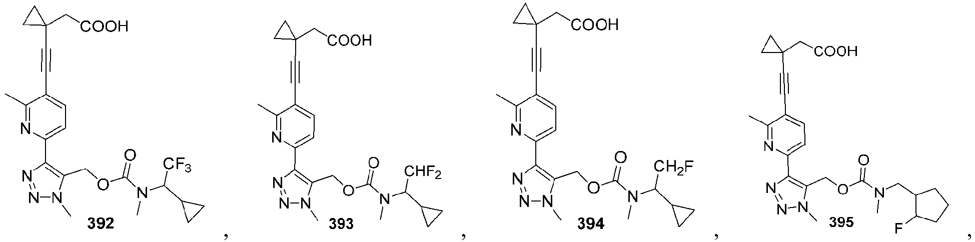 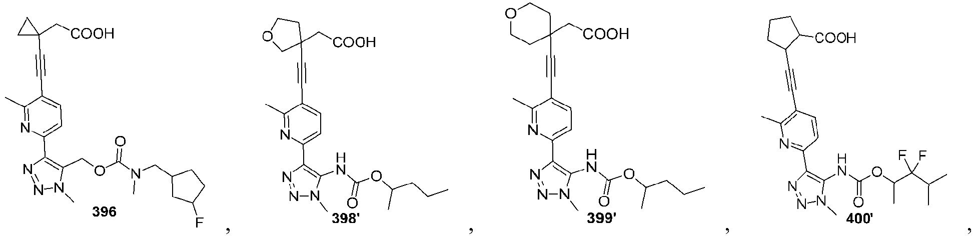 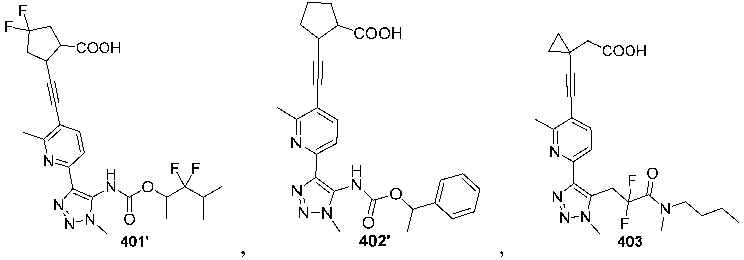 or its hydrochloride, 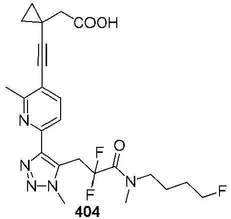 or its hydrochloride, 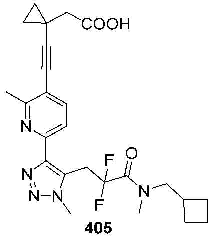 or its hydrochloride, 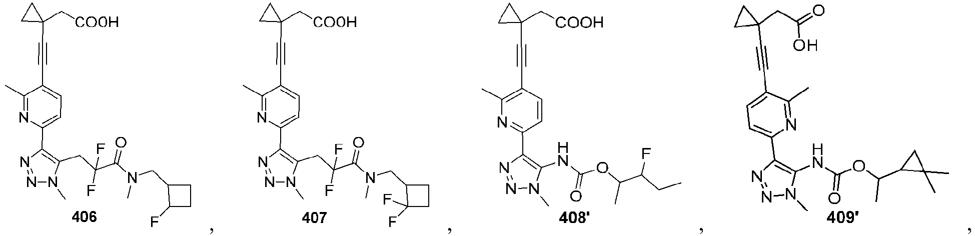 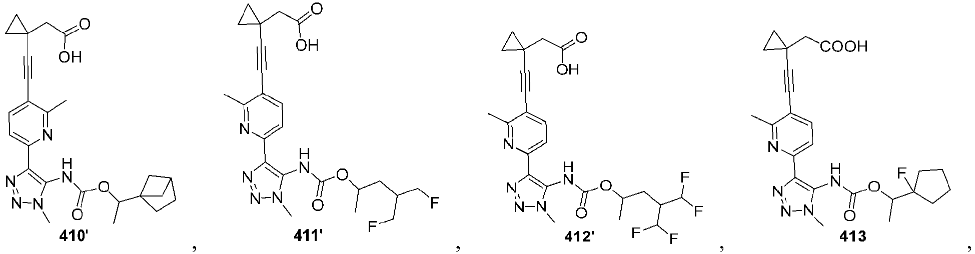 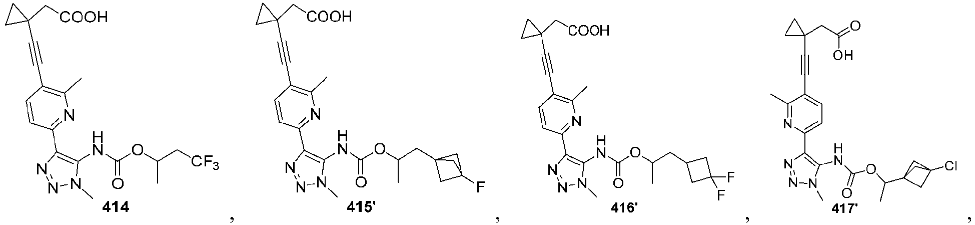 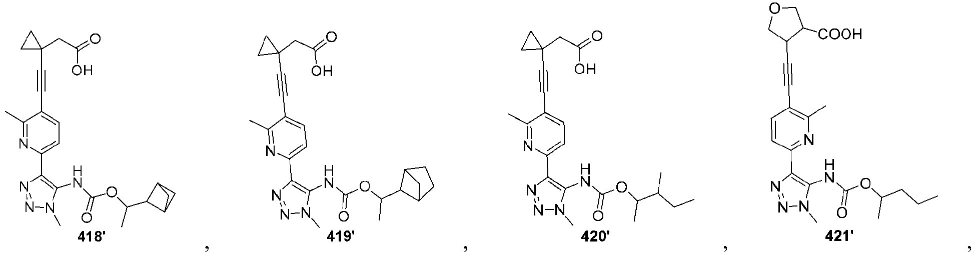 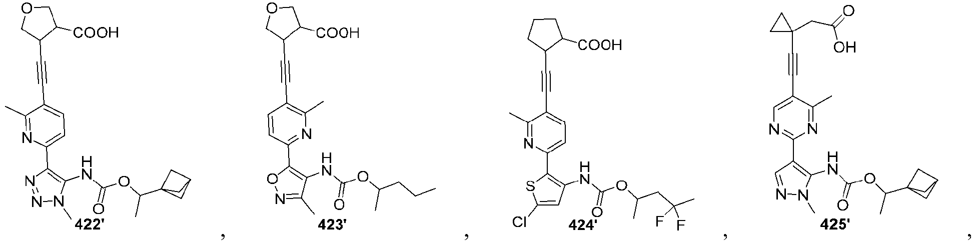 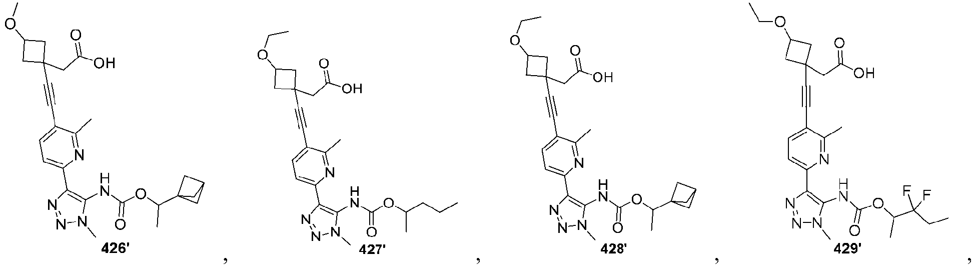 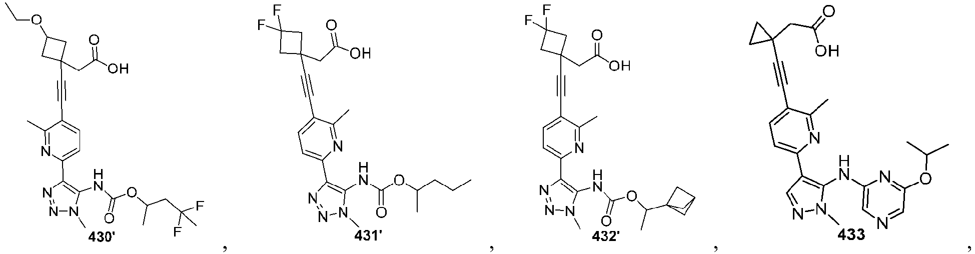 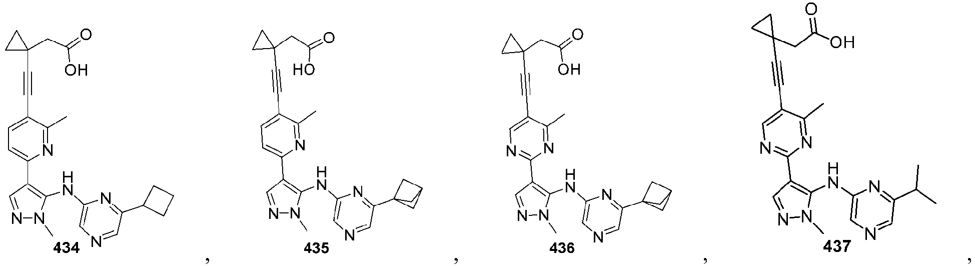 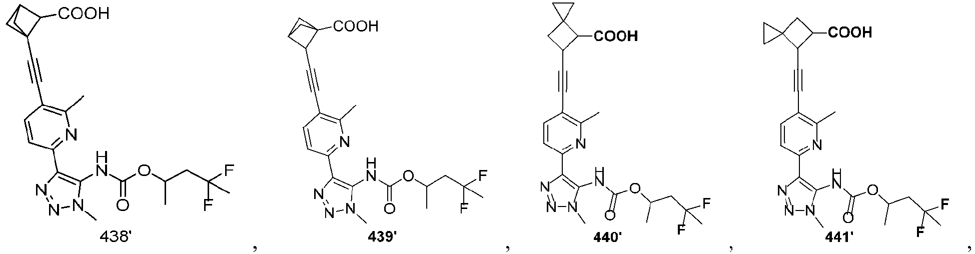 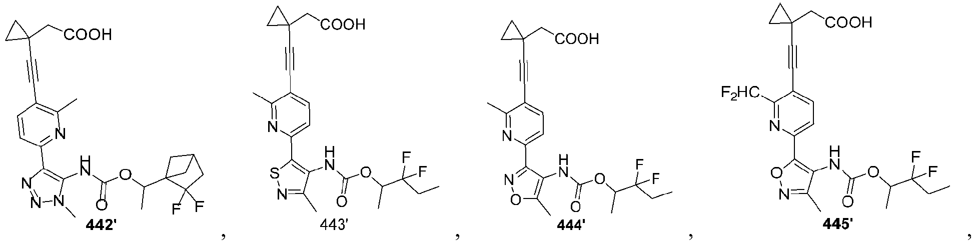 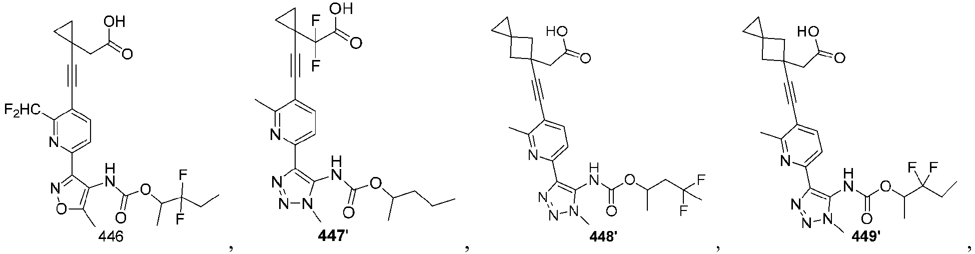 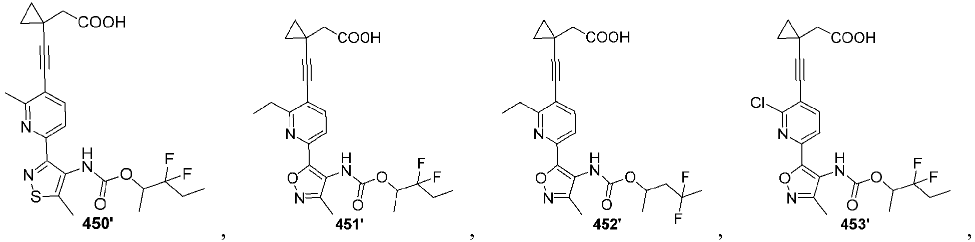 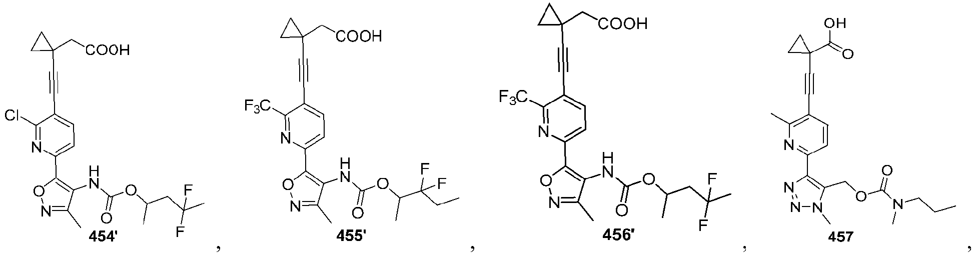 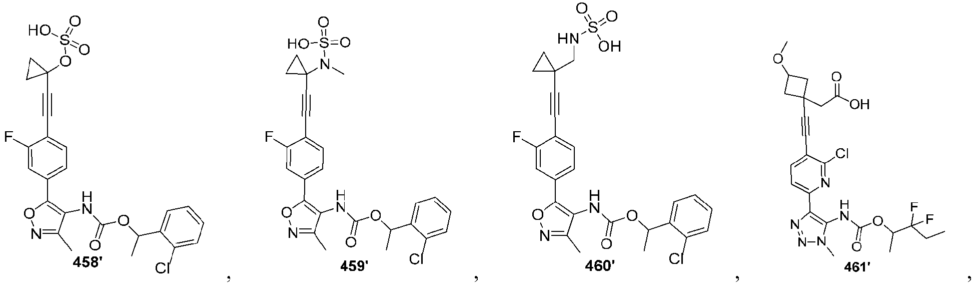 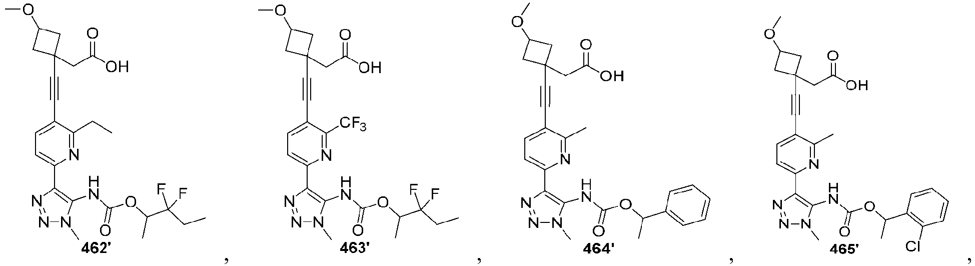 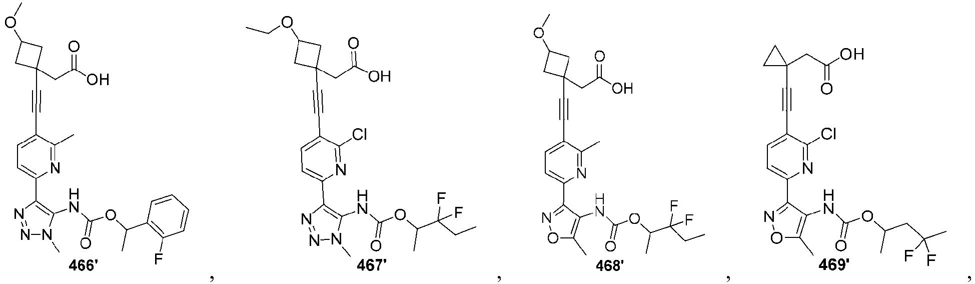 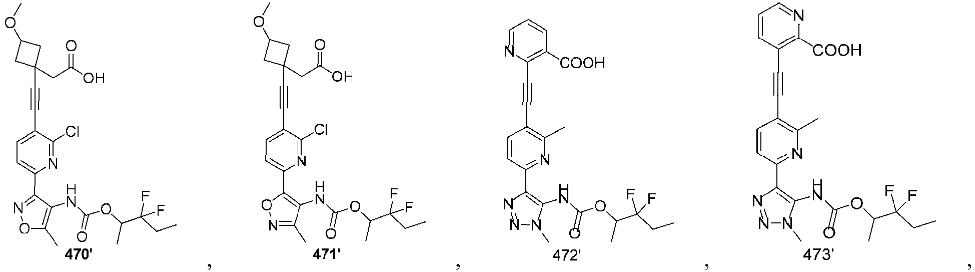 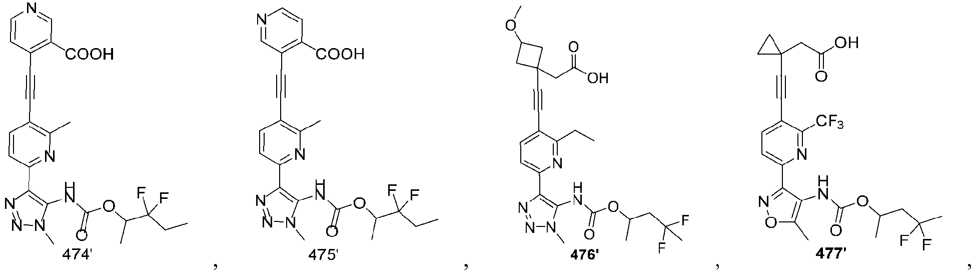 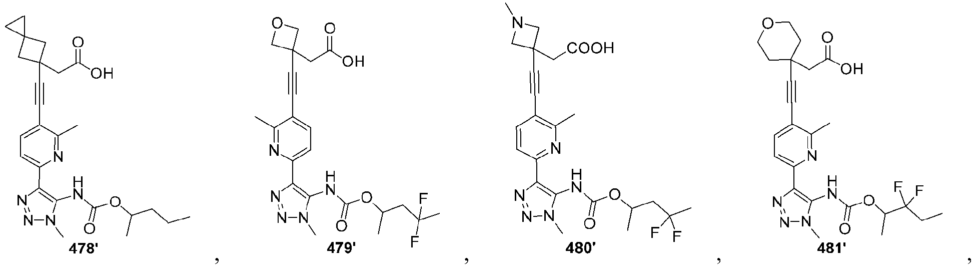 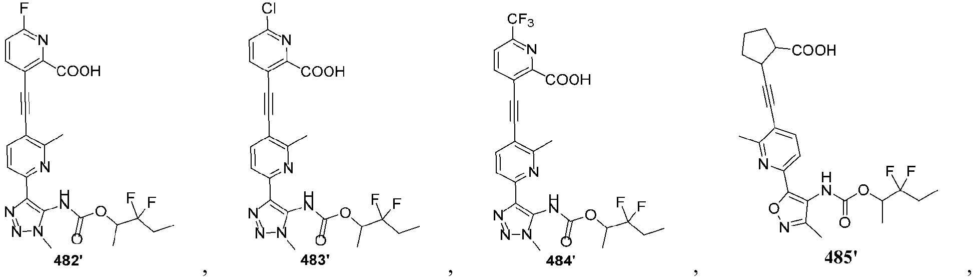 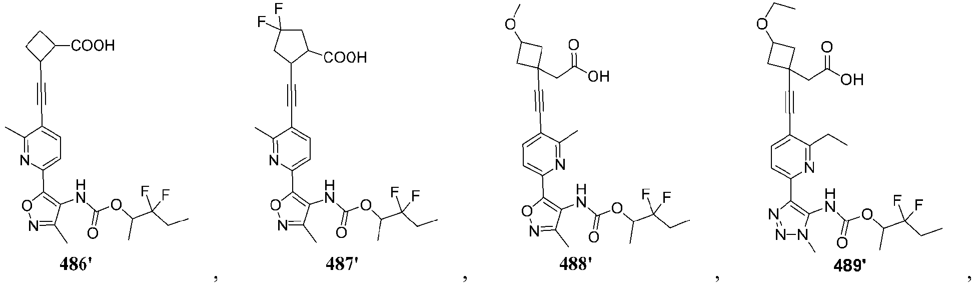 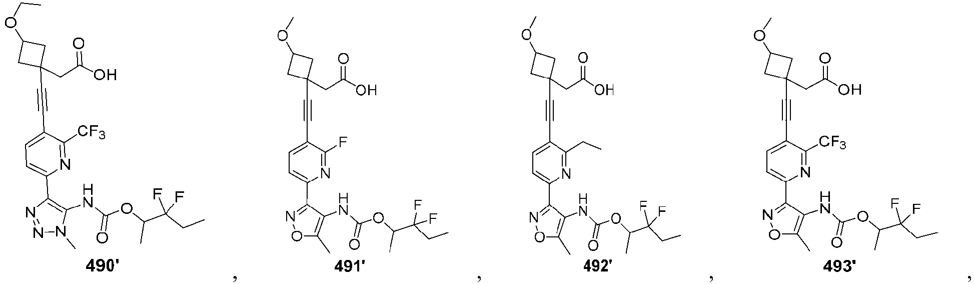 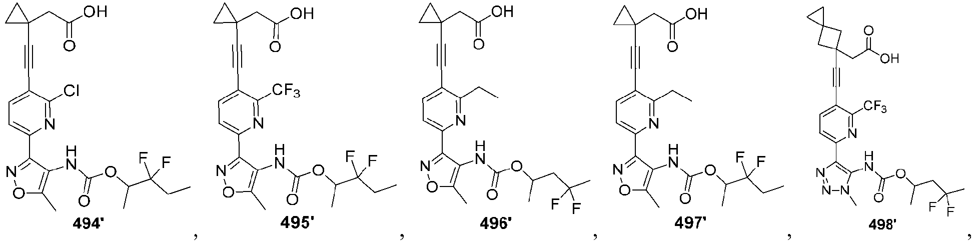 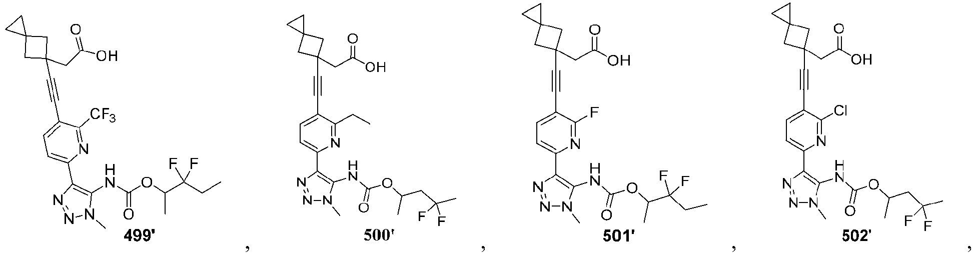 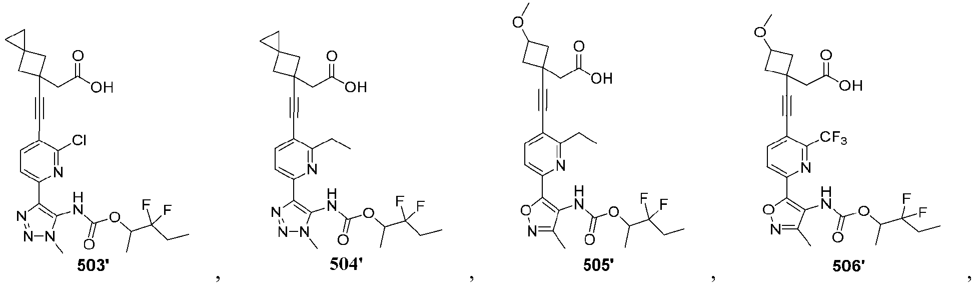 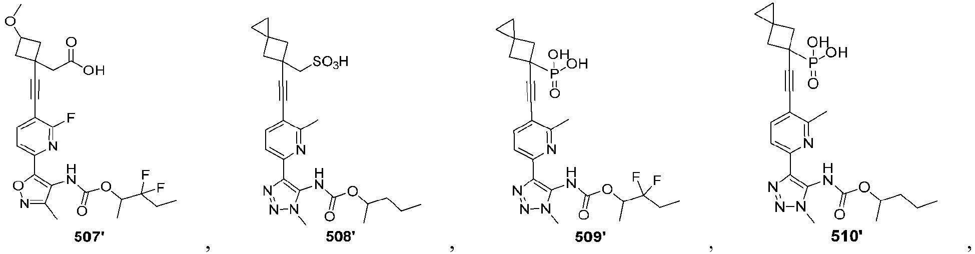 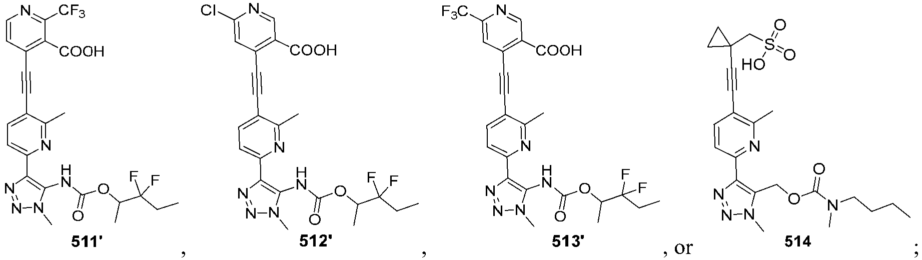 preferably, the compound of formula (I) is any one of the following compounds:

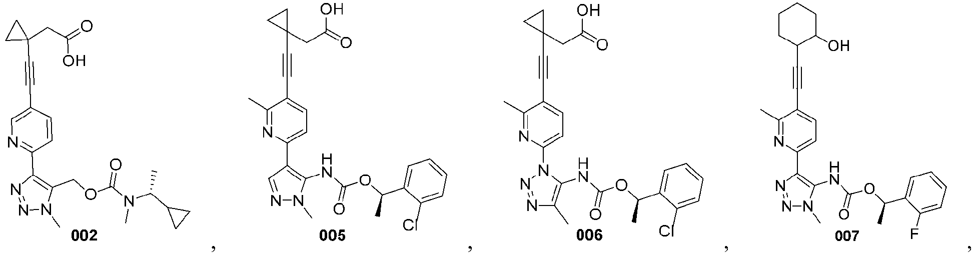

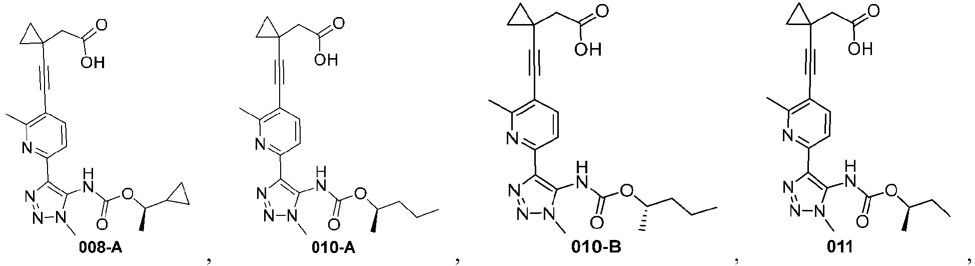

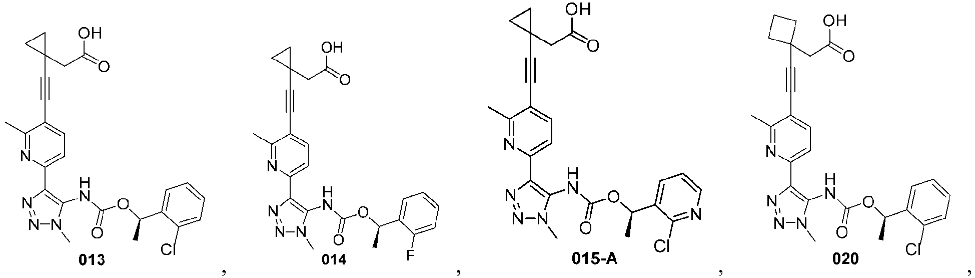

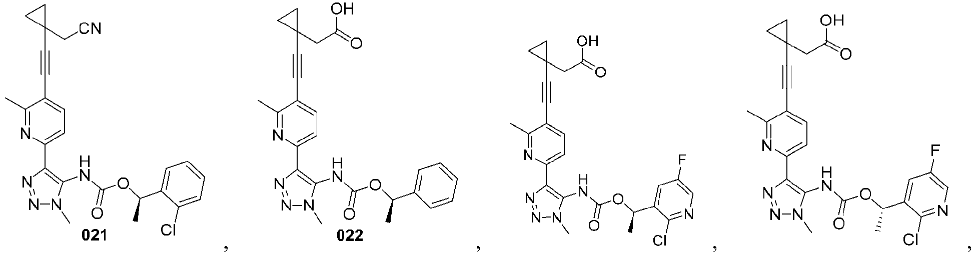

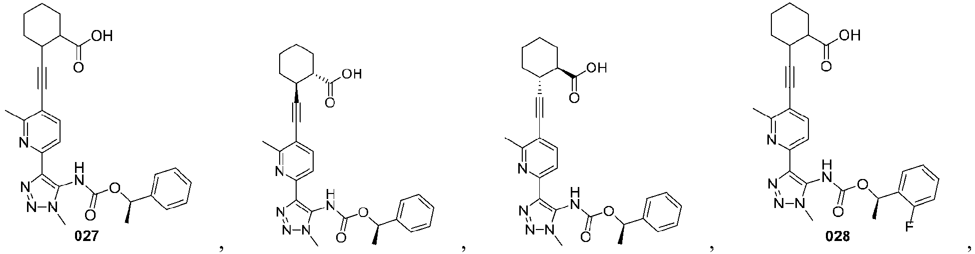

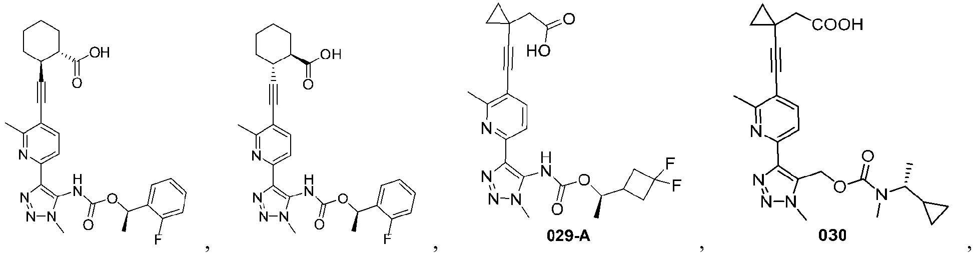

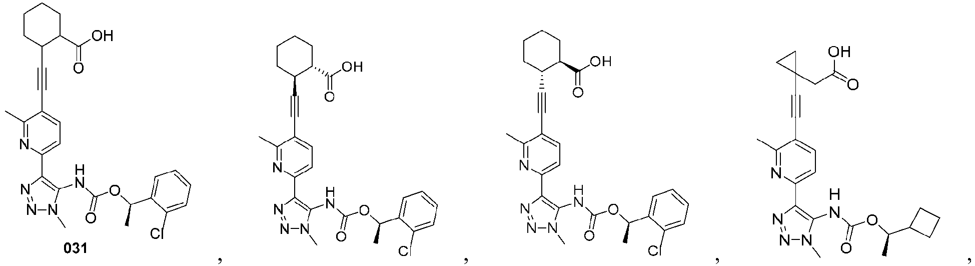

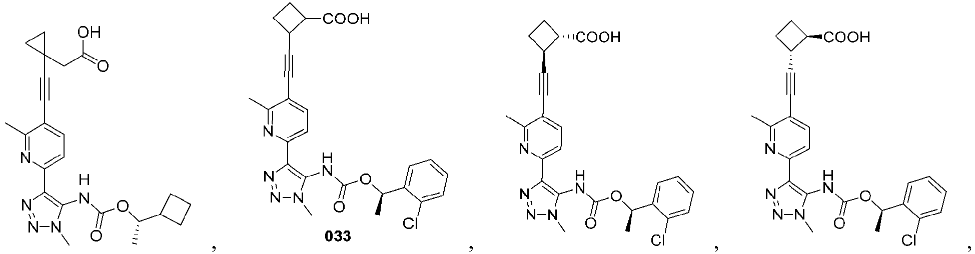

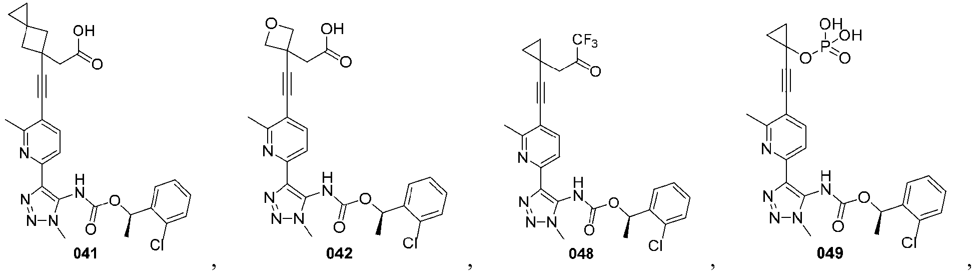

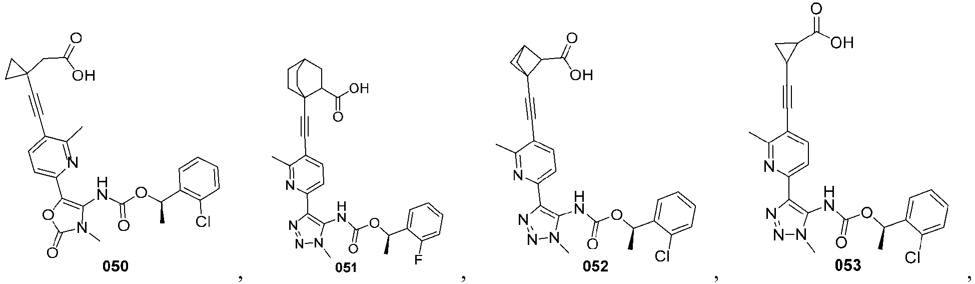

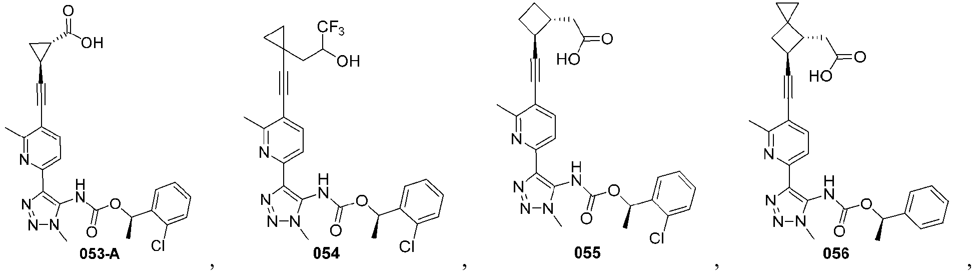

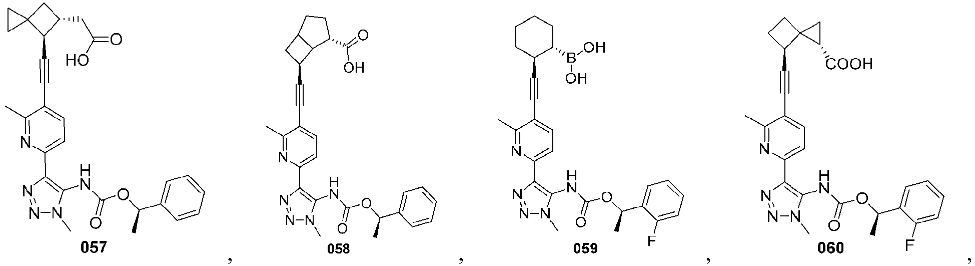

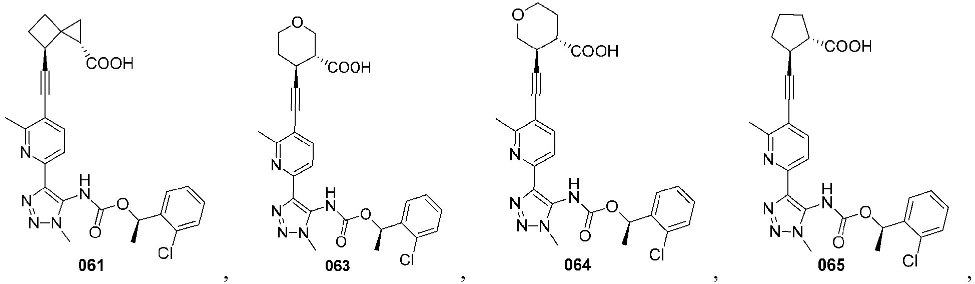

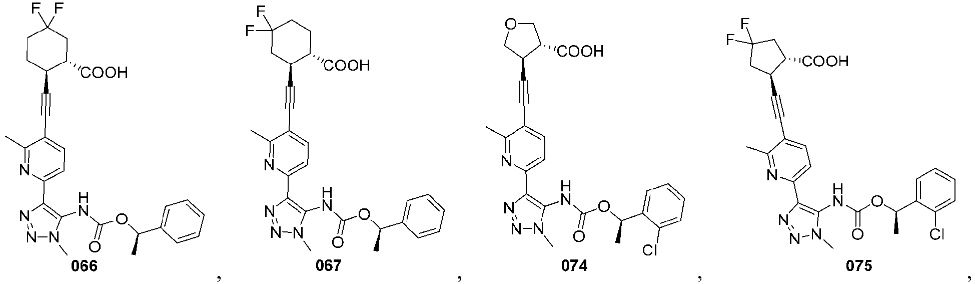

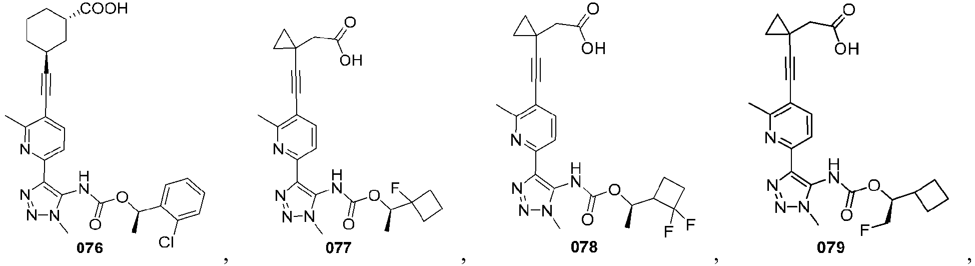

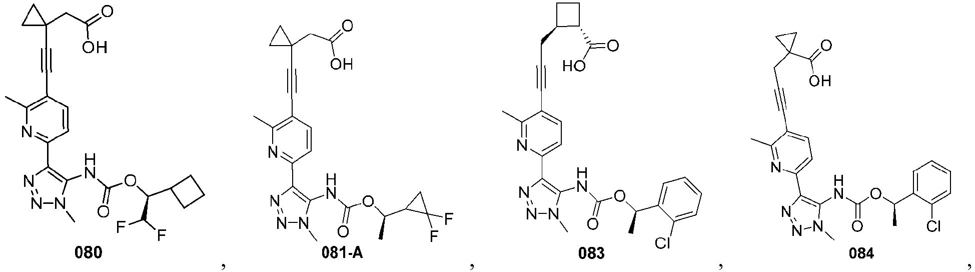

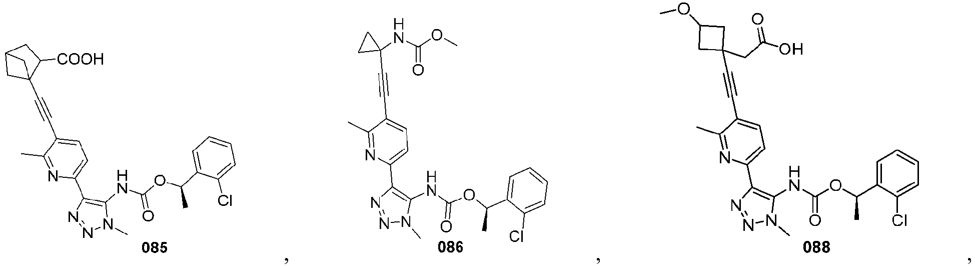

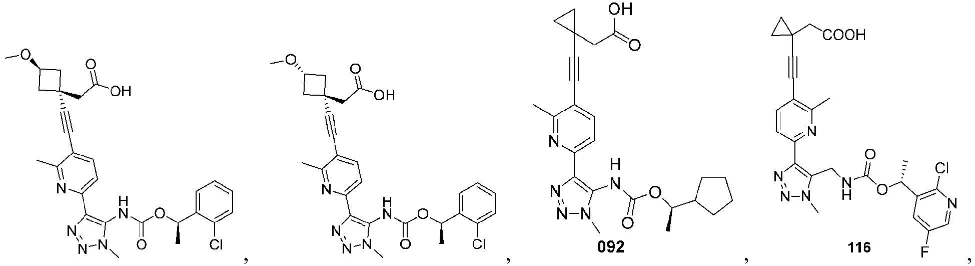

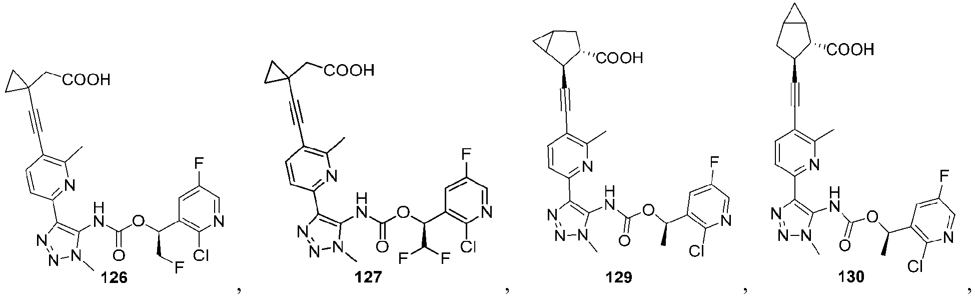

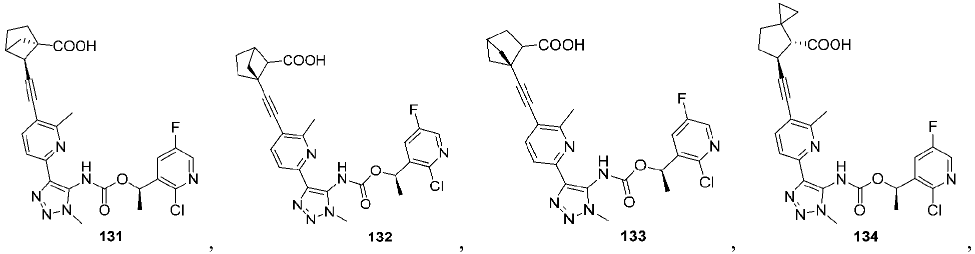

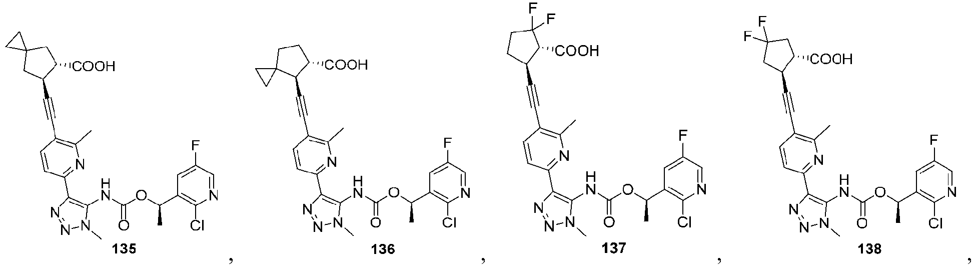

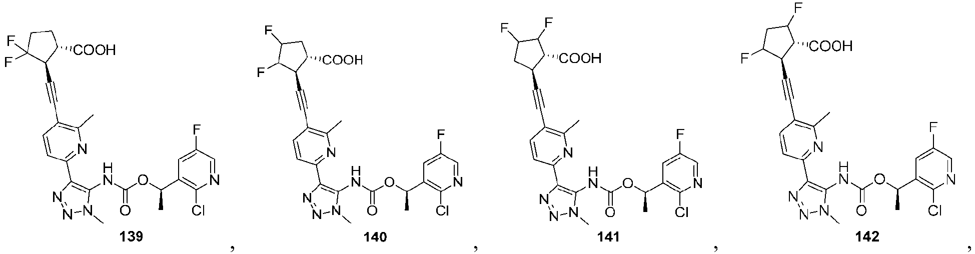

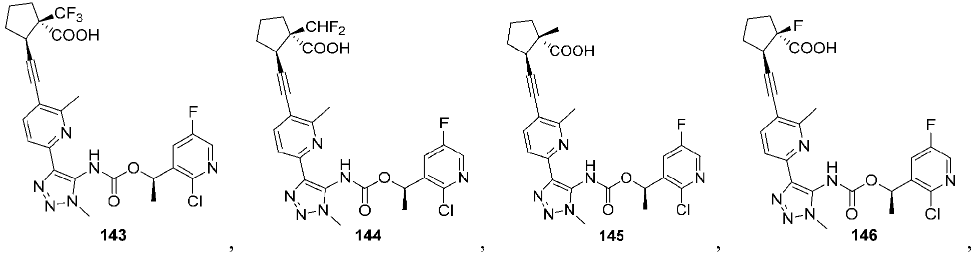

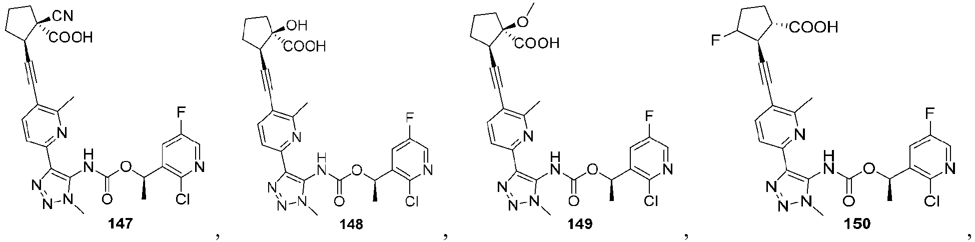

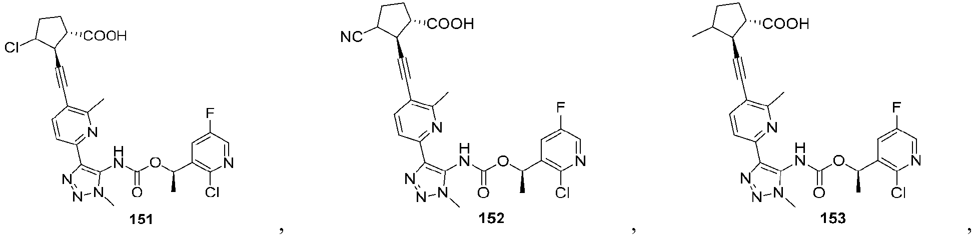

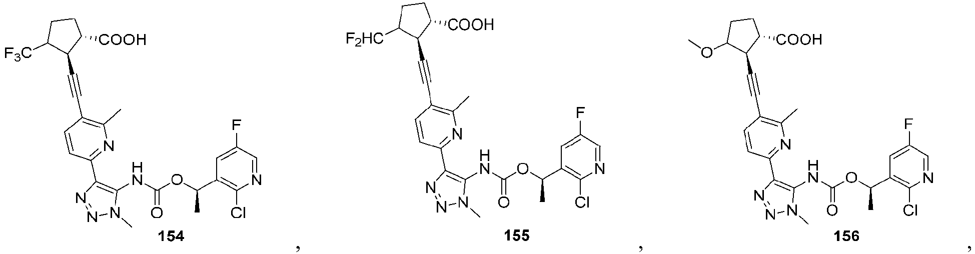

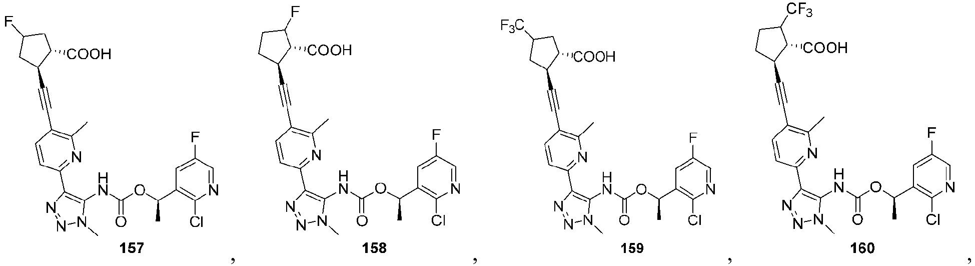

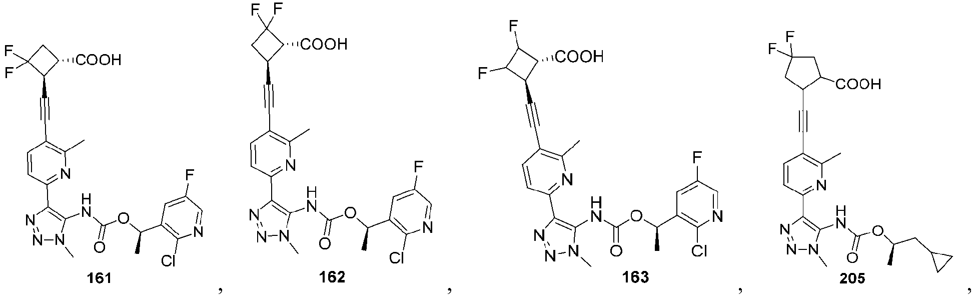

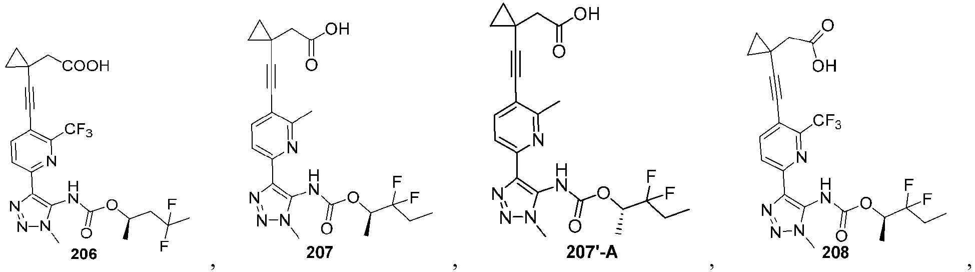

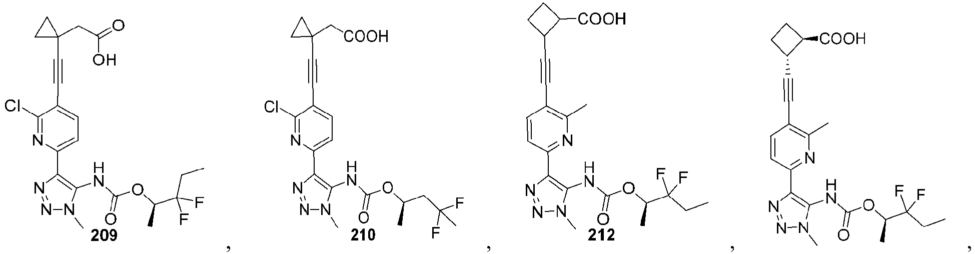

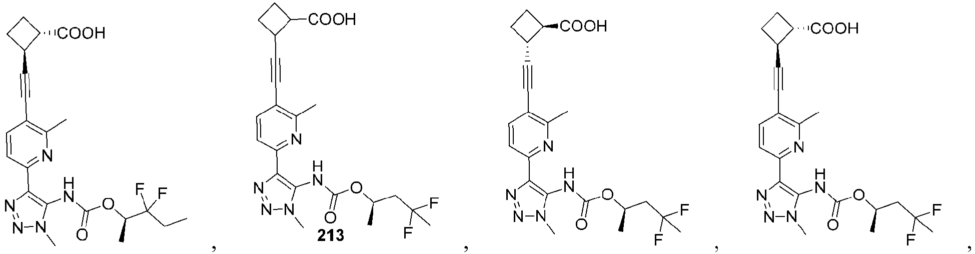

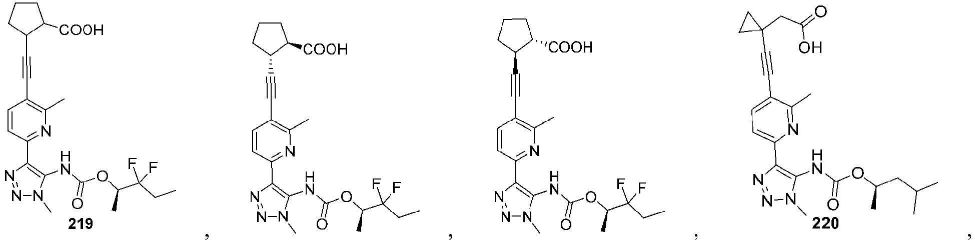

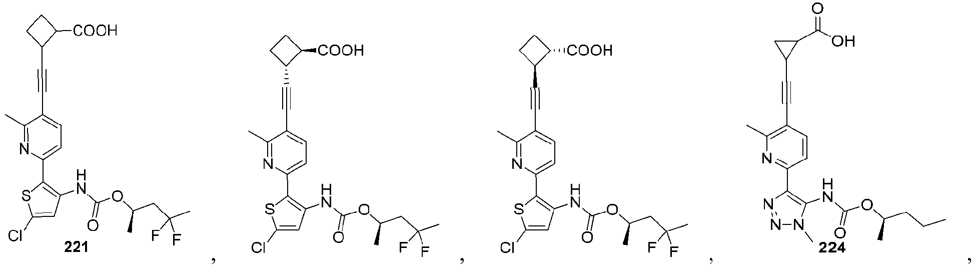

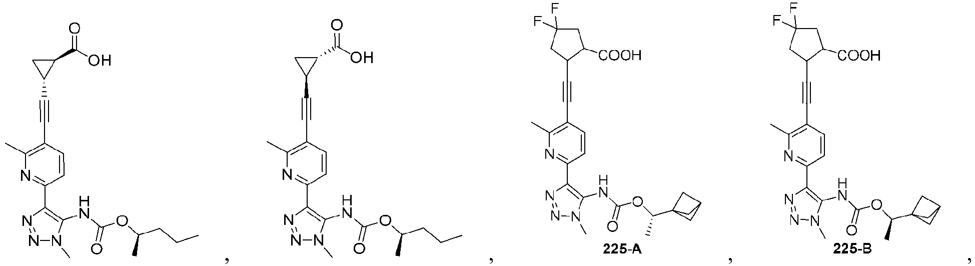

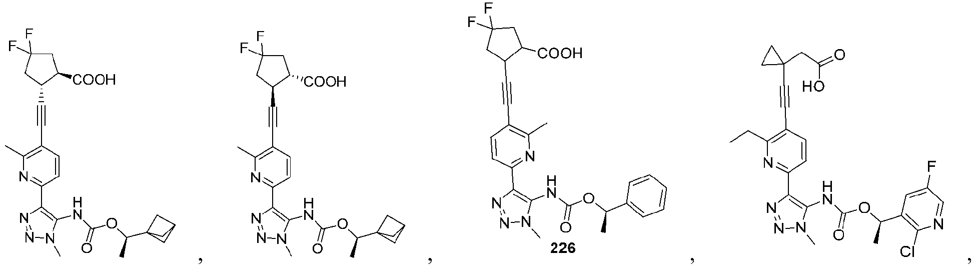

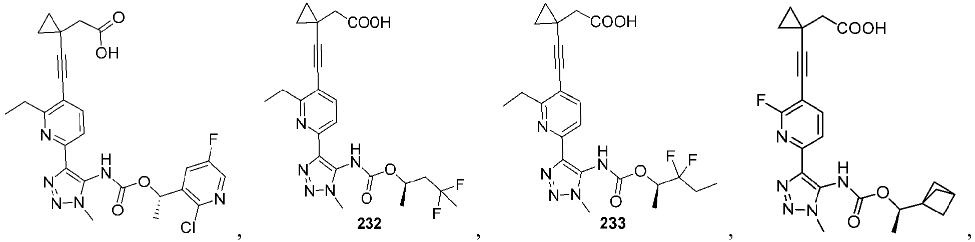

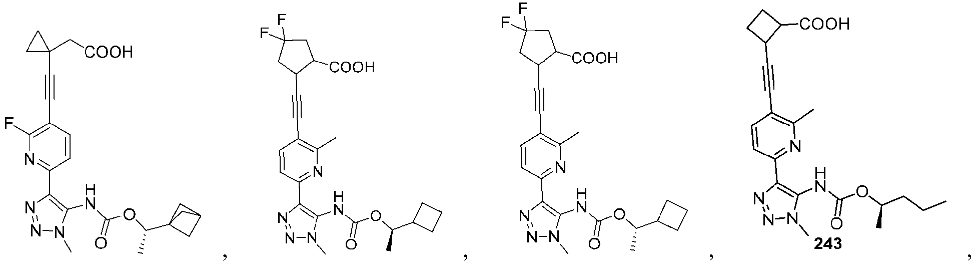

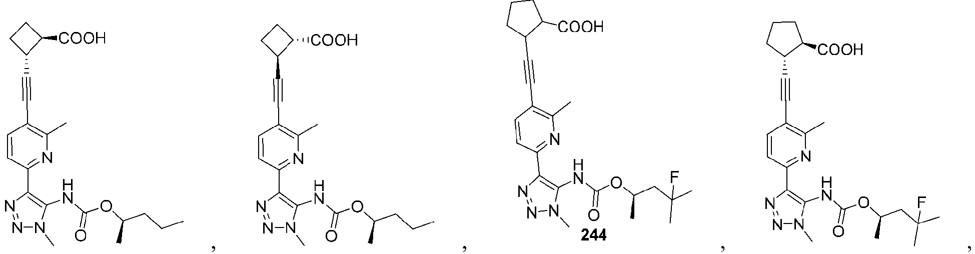

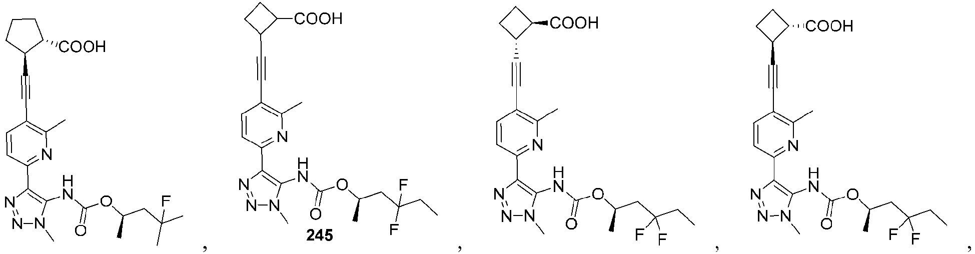

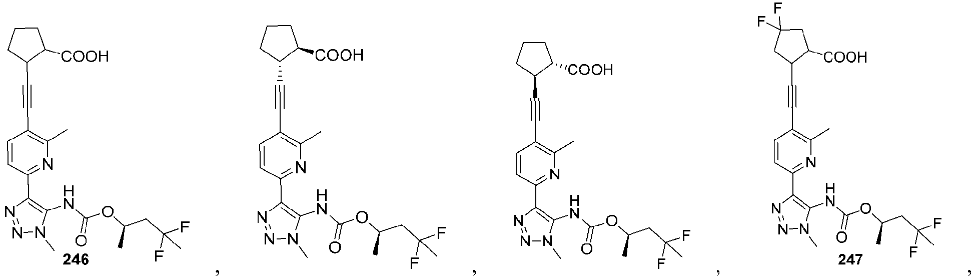

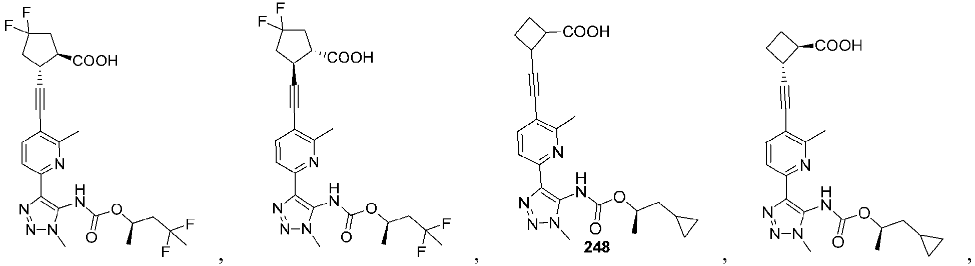

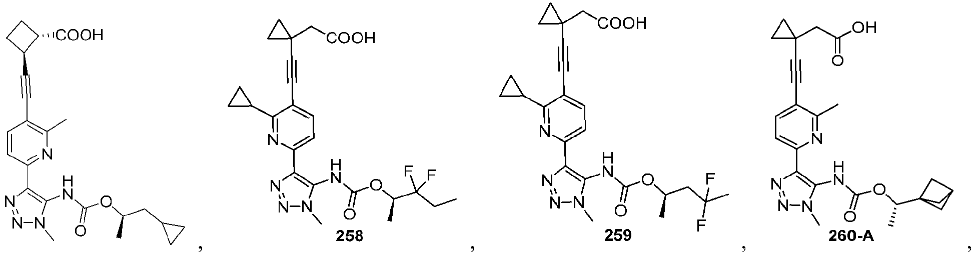

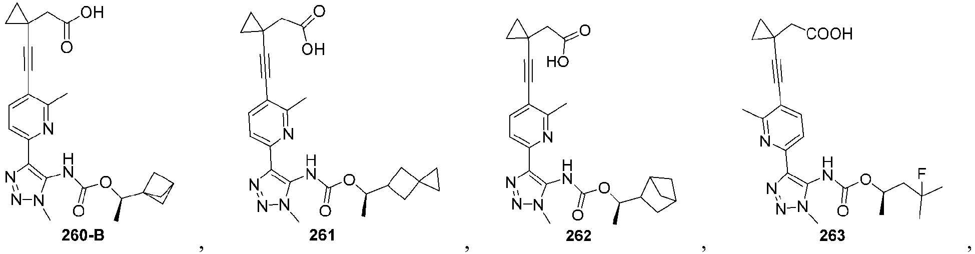

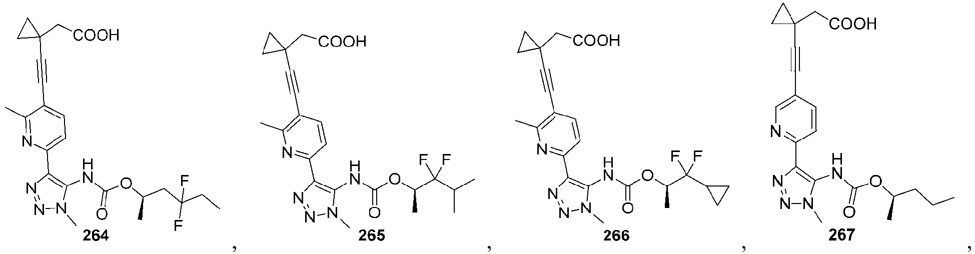

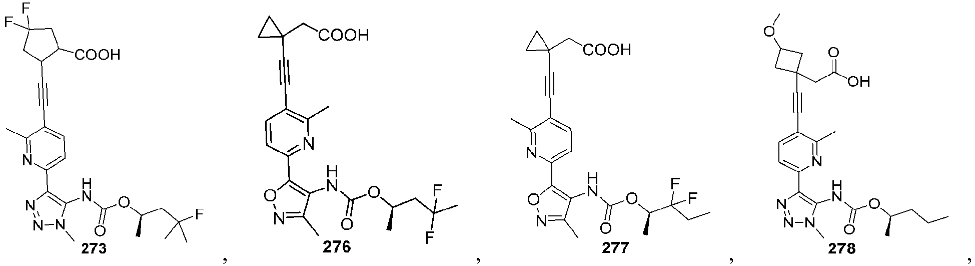

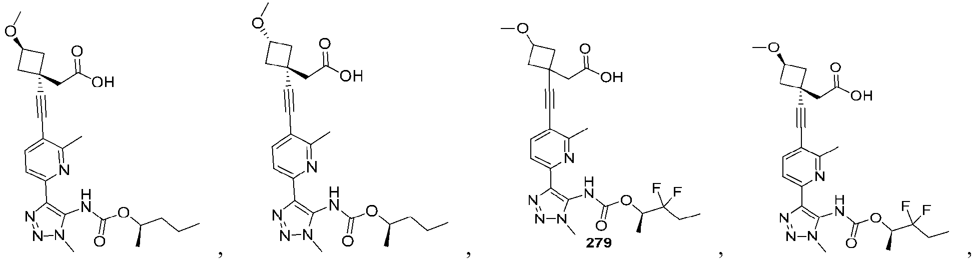

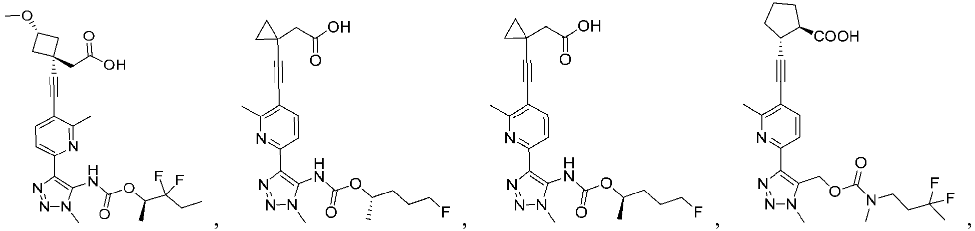

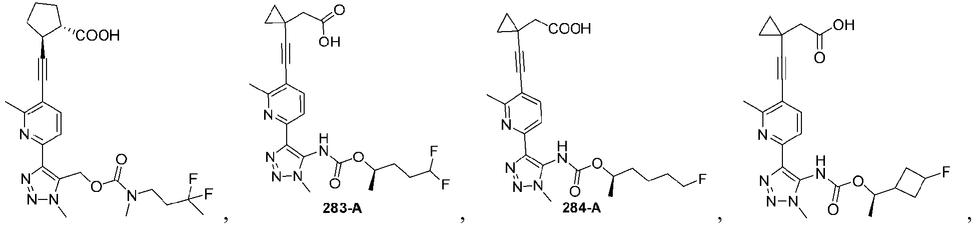

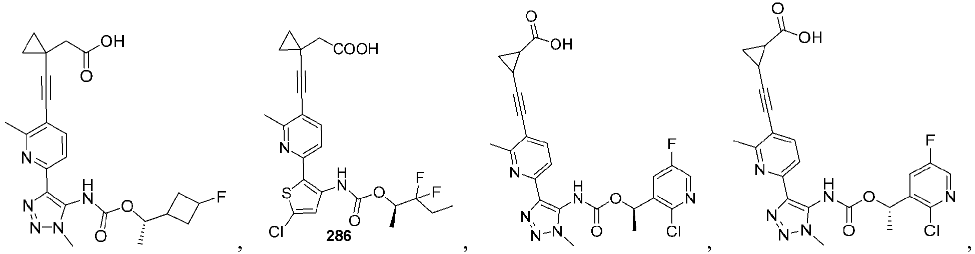

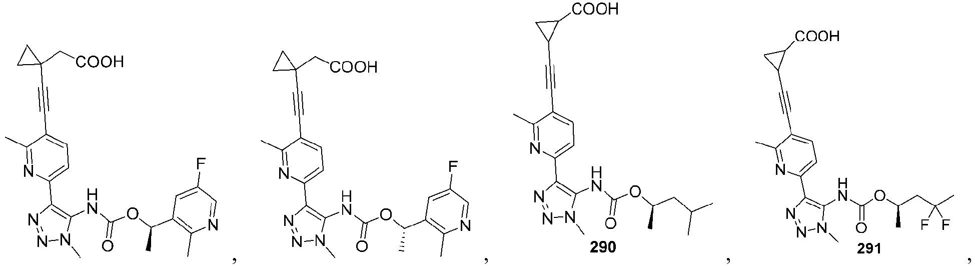

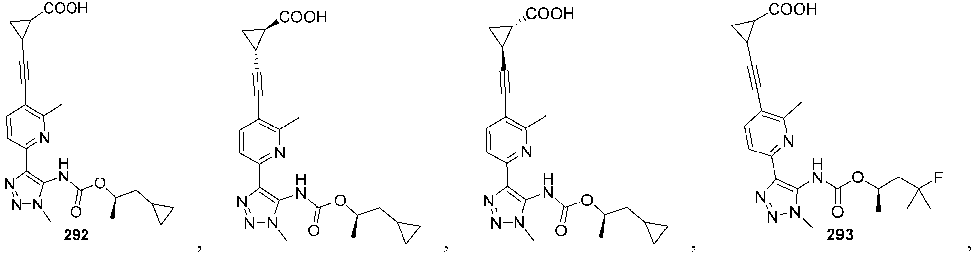

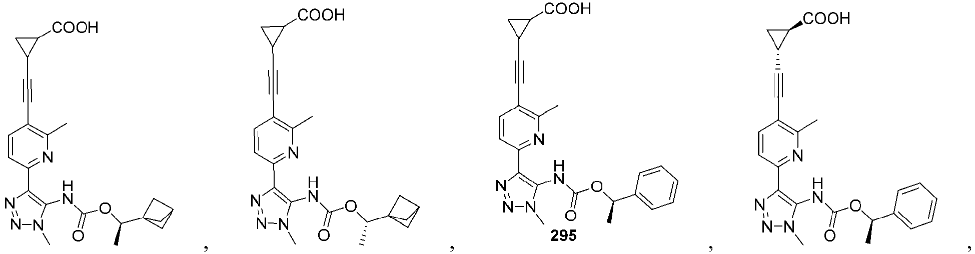

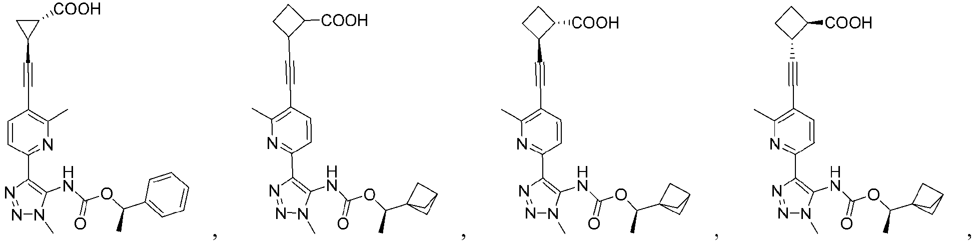

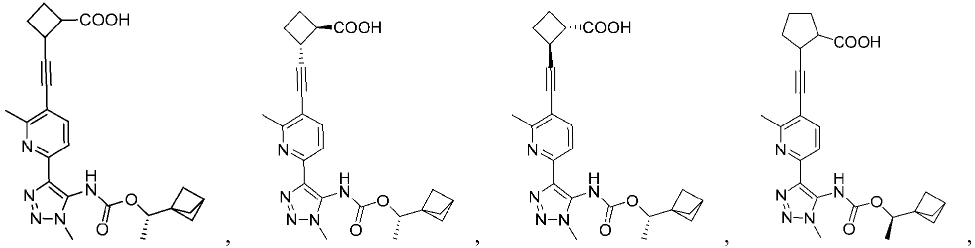

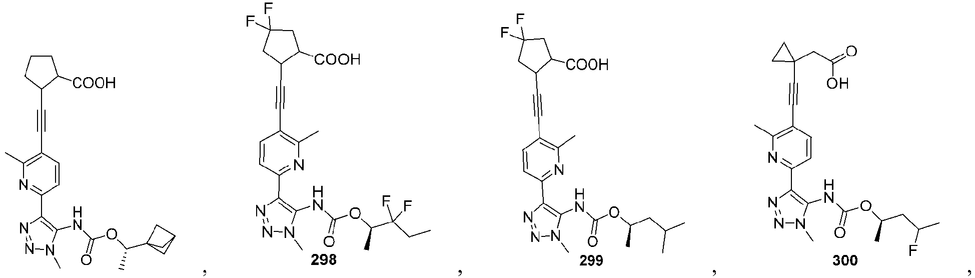

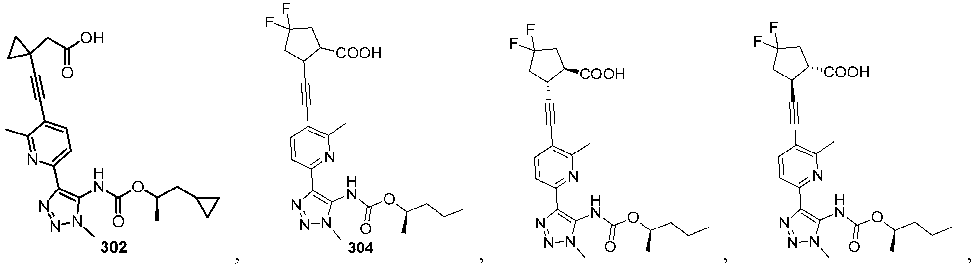

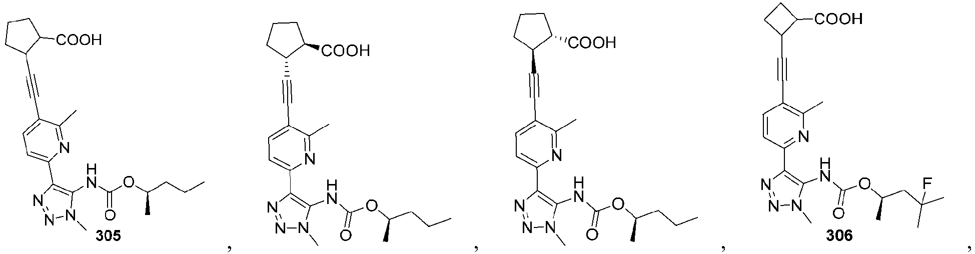

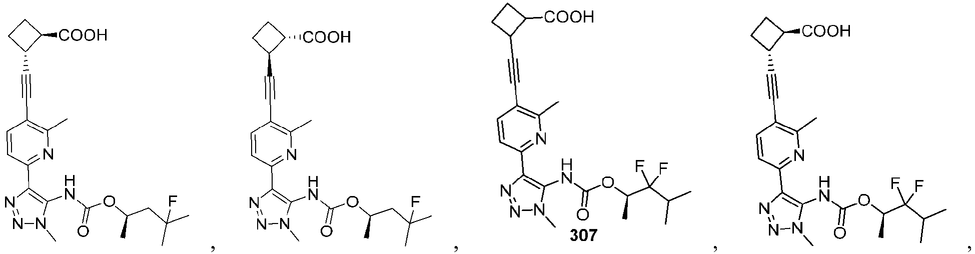

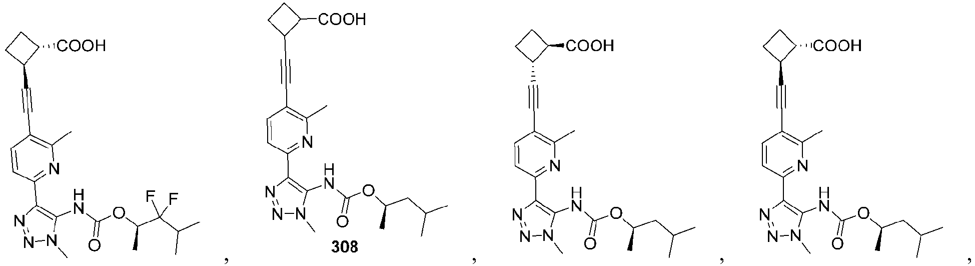

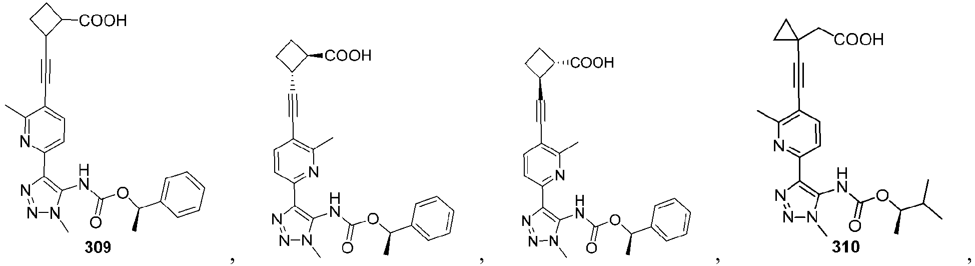

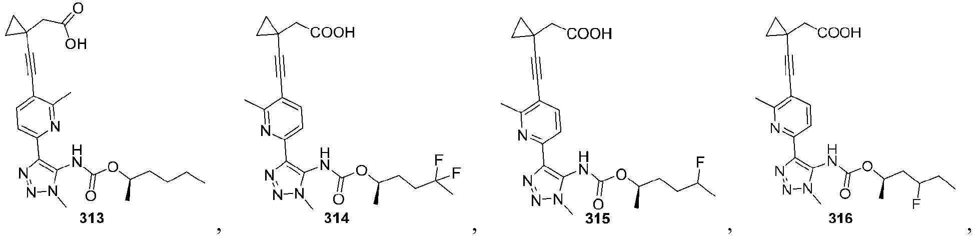

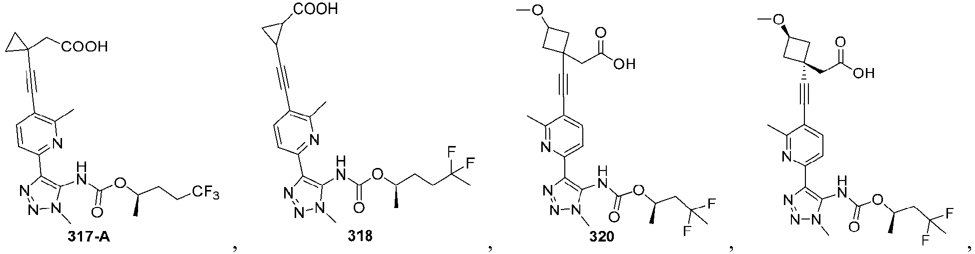

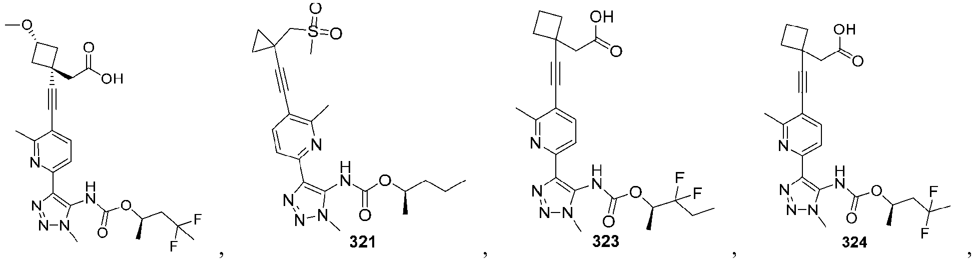

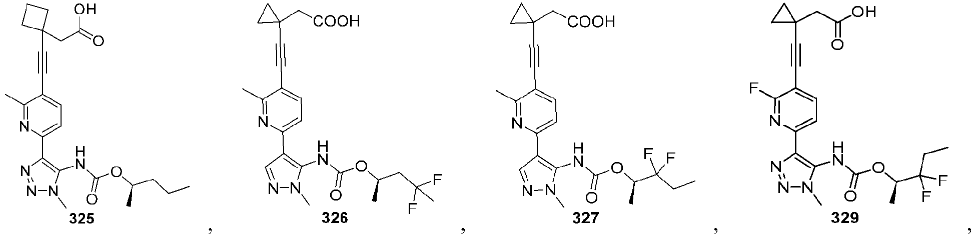

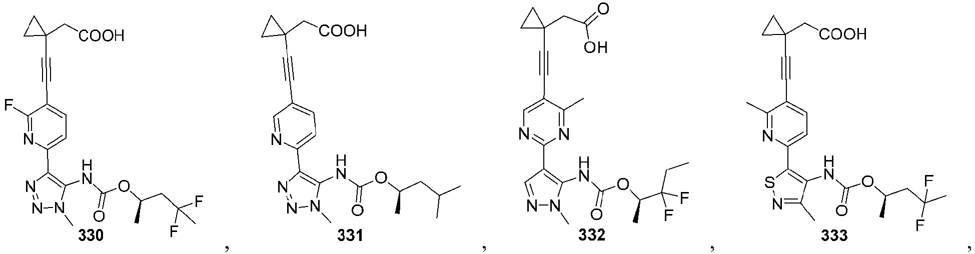

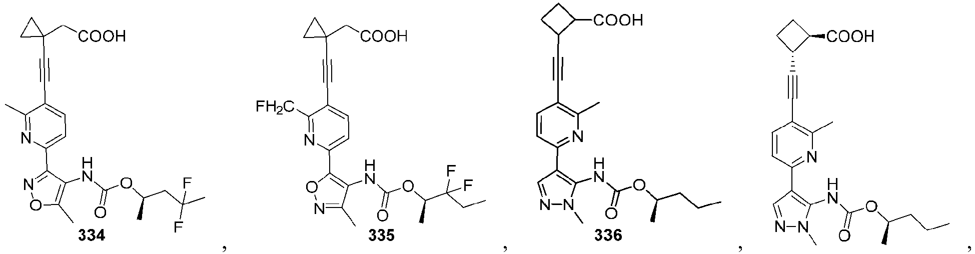

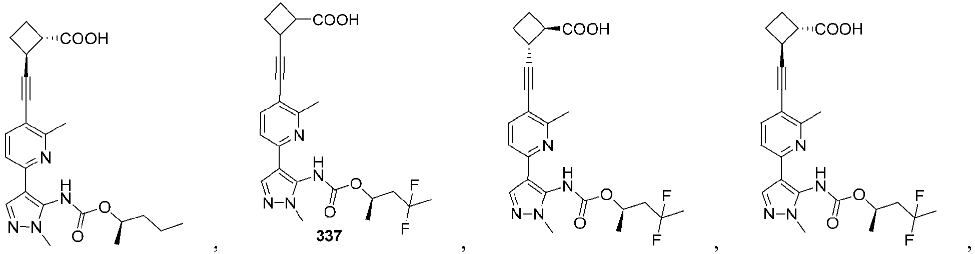

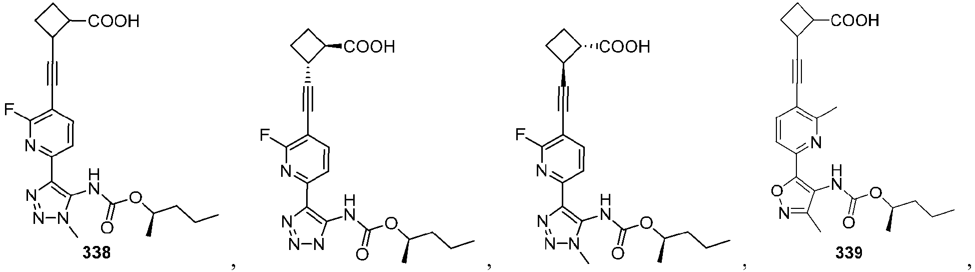

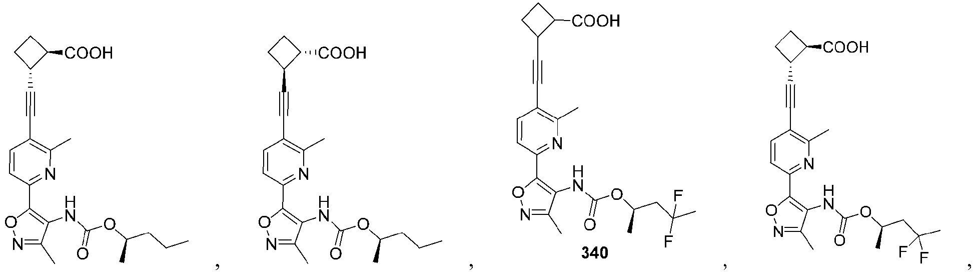

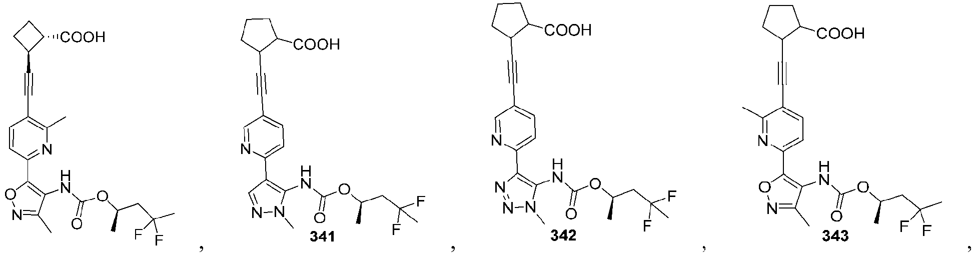

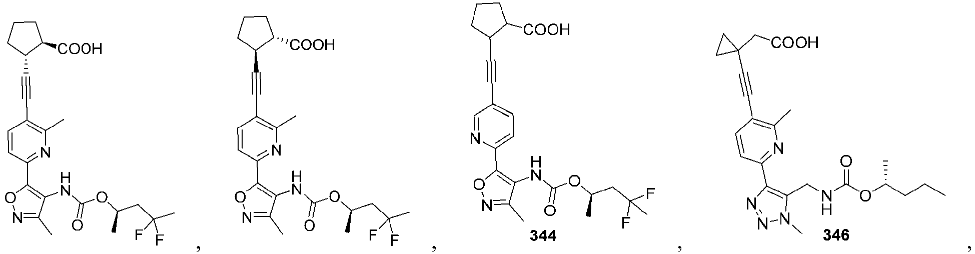

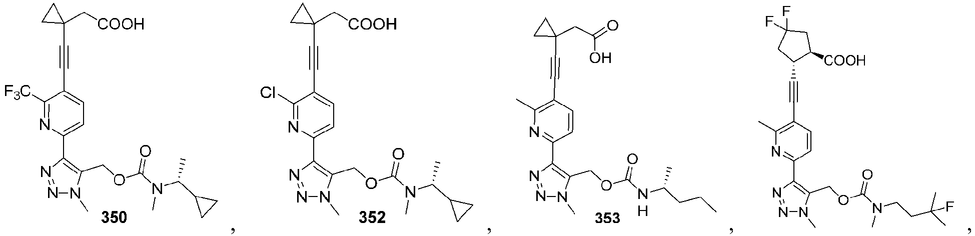

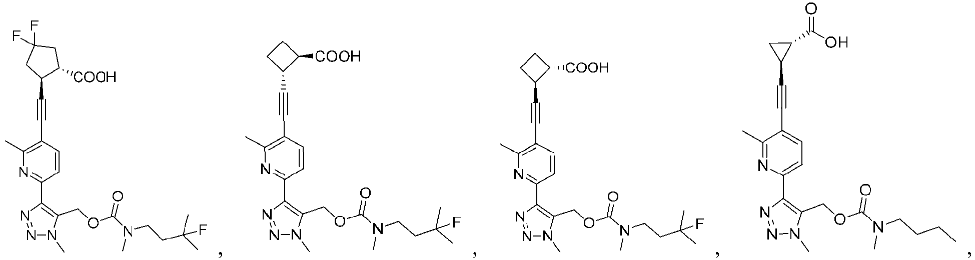

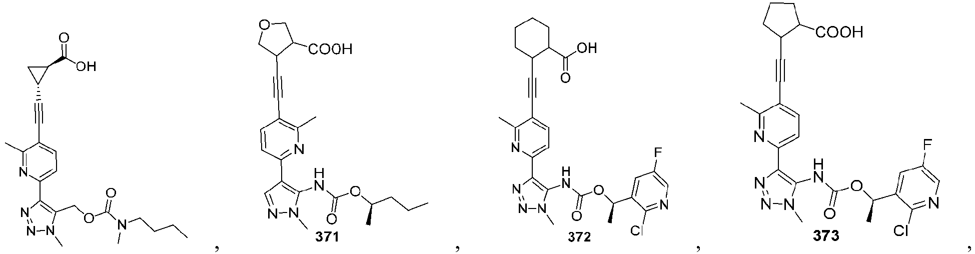

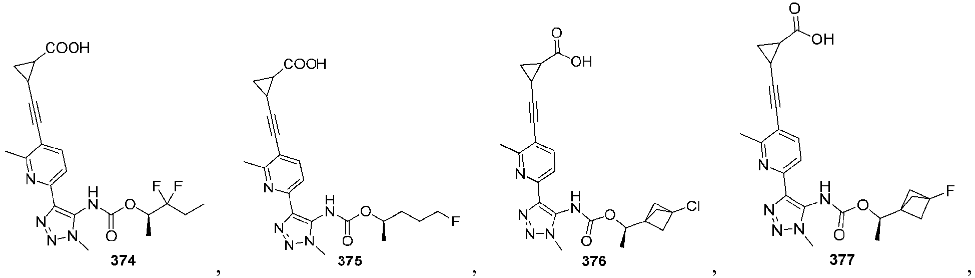

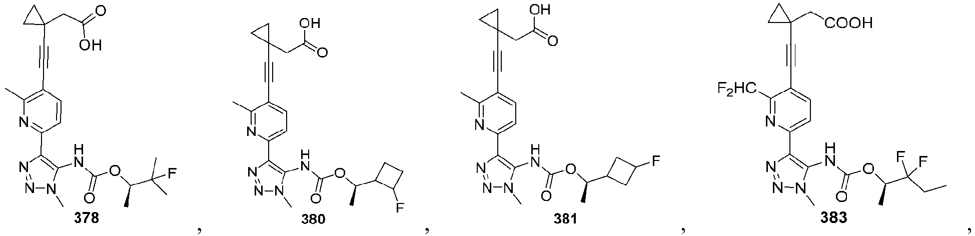

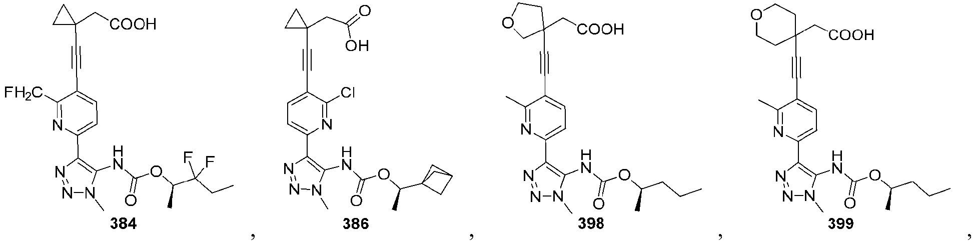

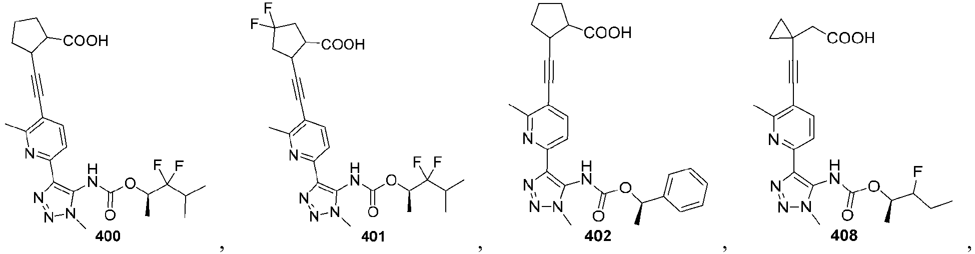

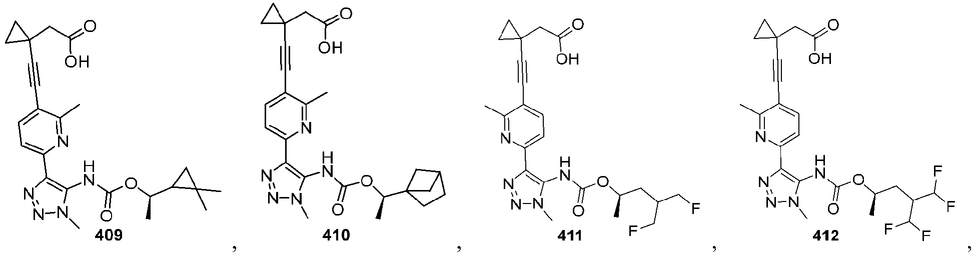

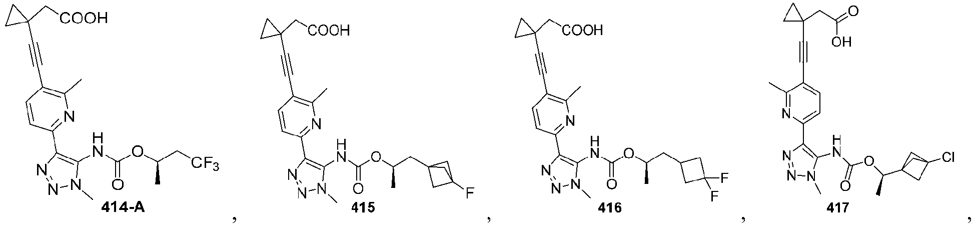

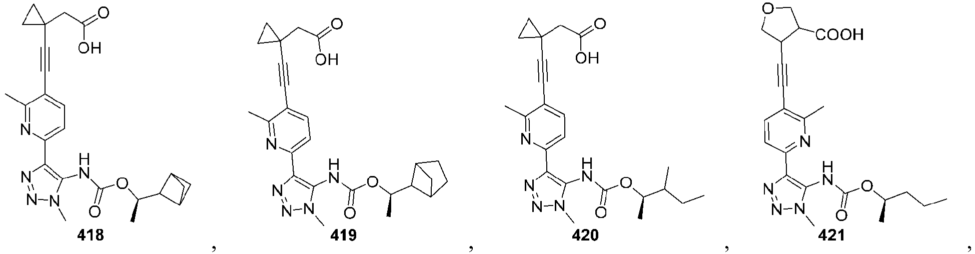

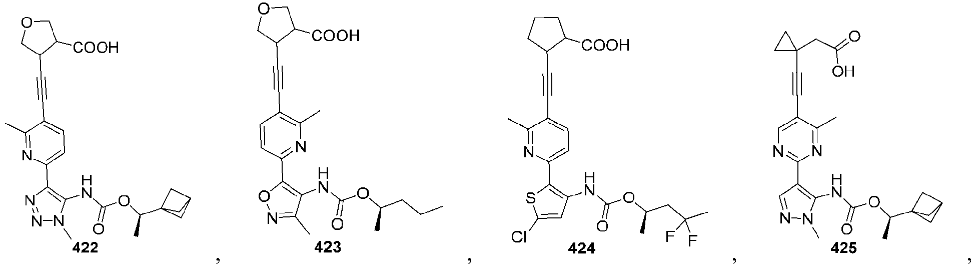

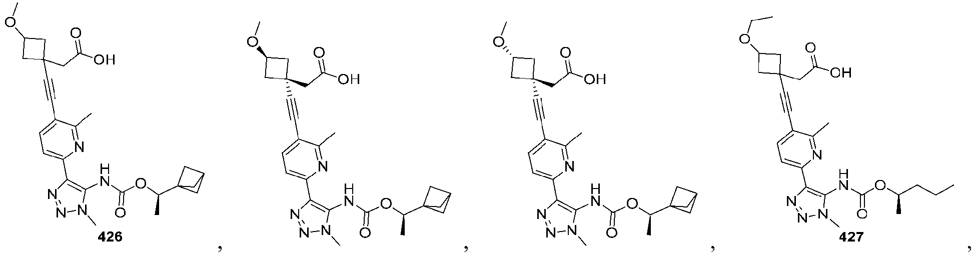

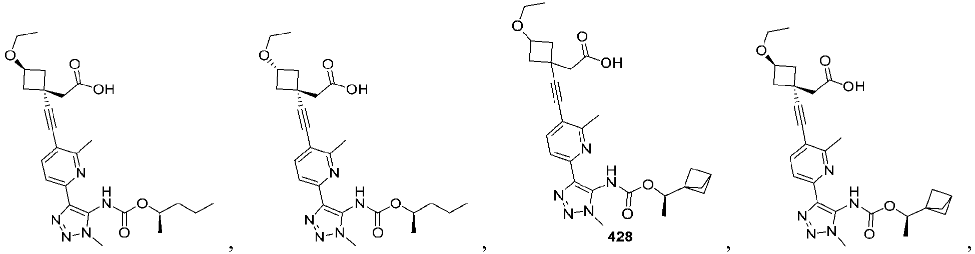

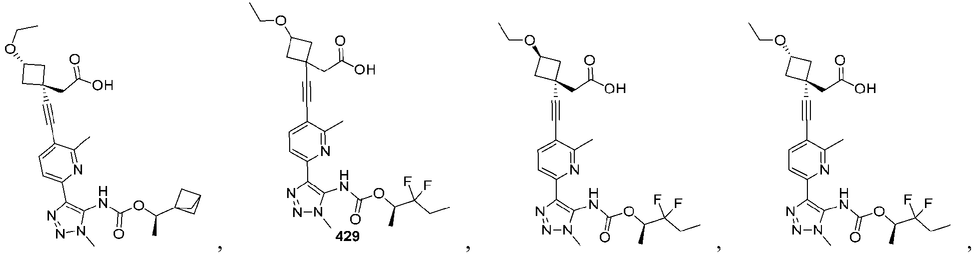

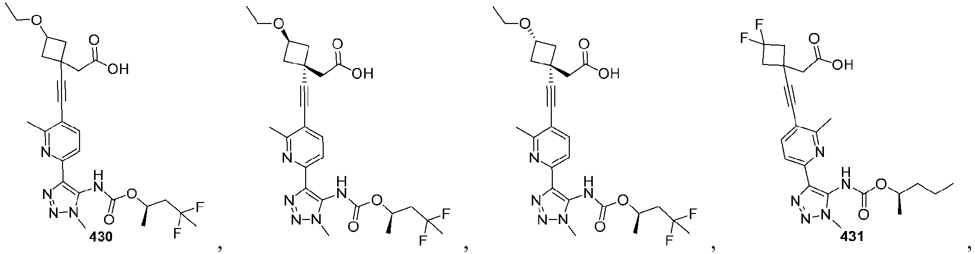

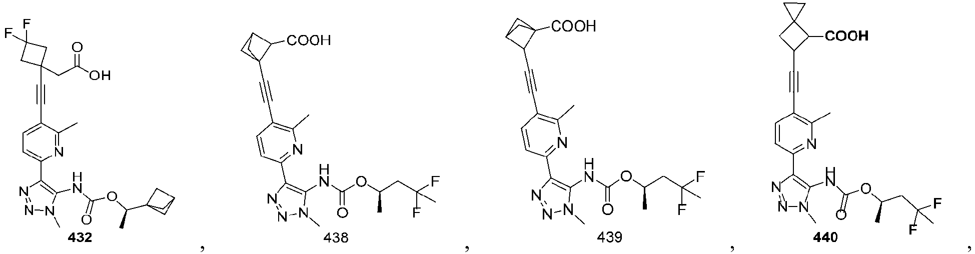

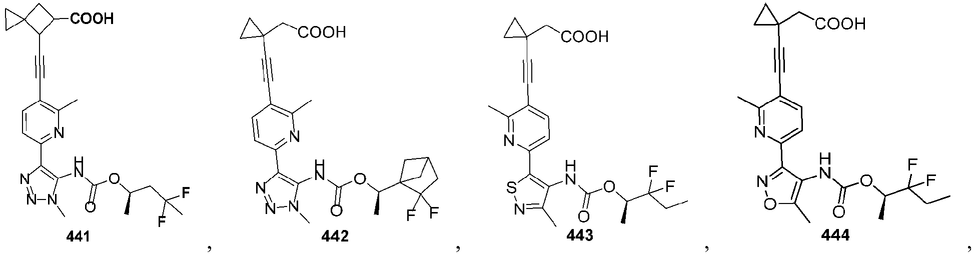

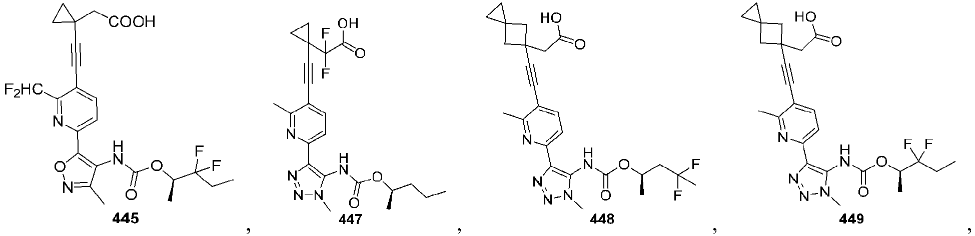

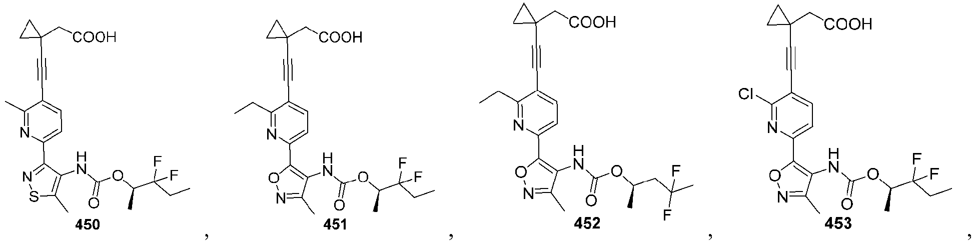

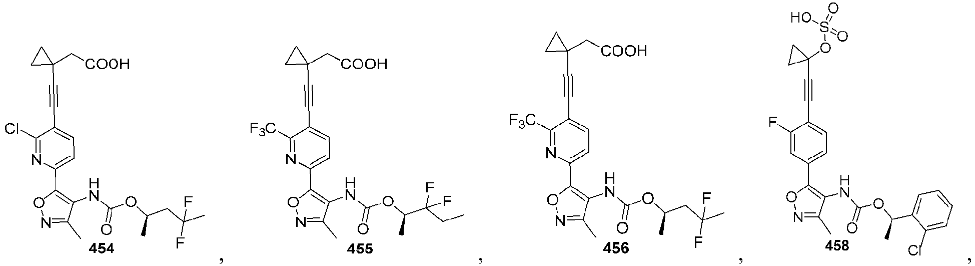

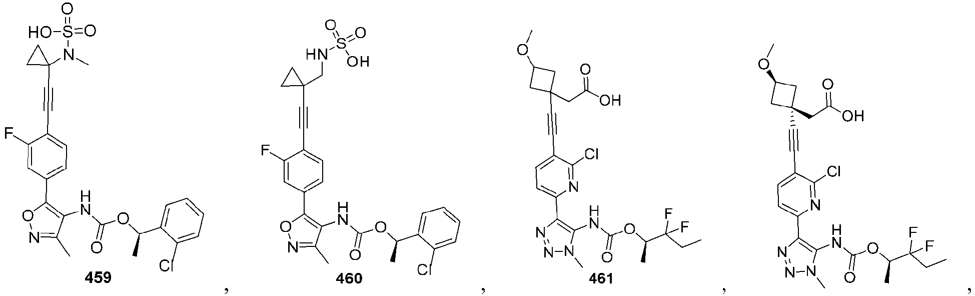

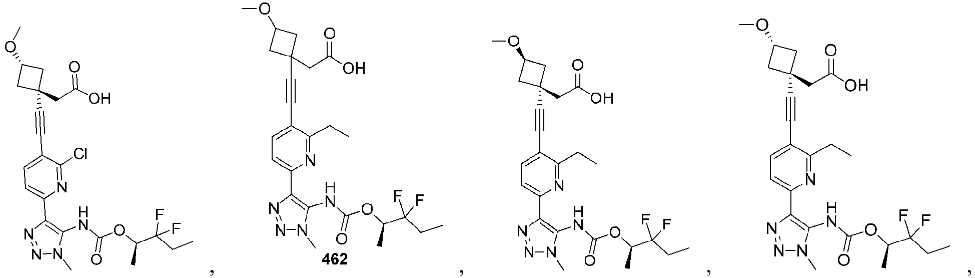

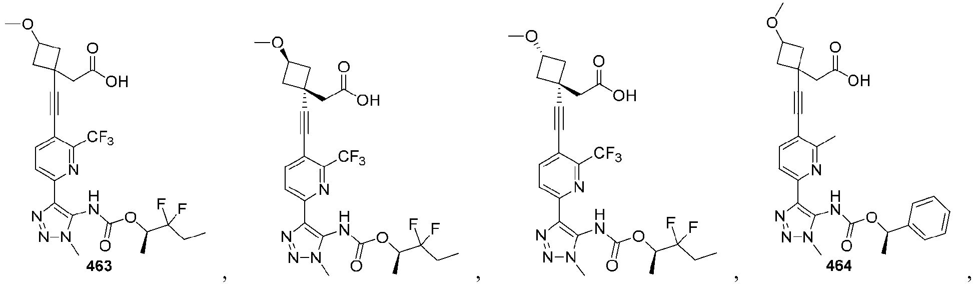

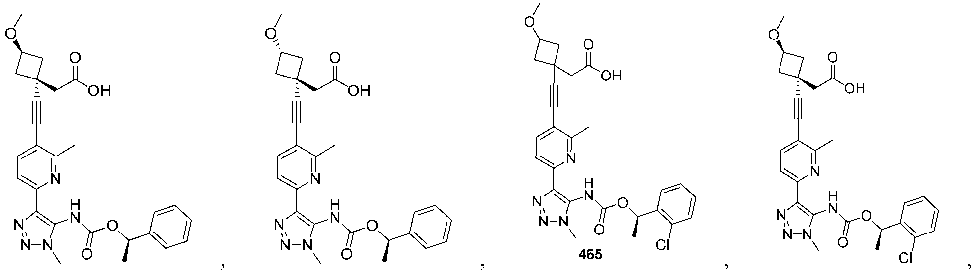

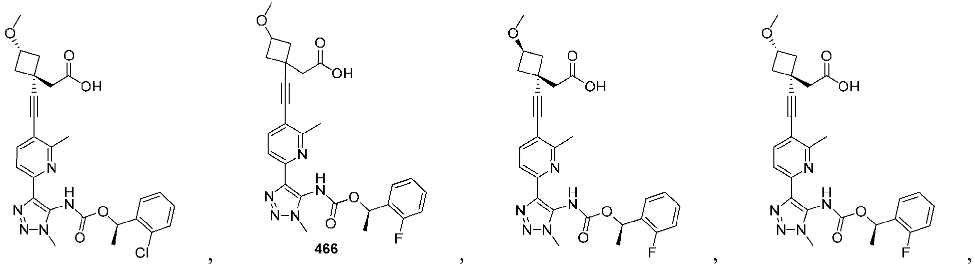

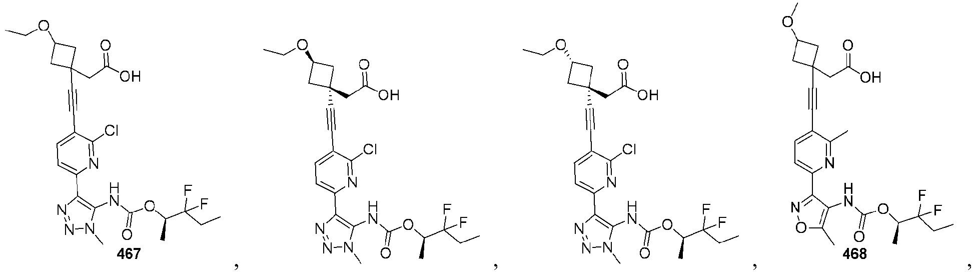

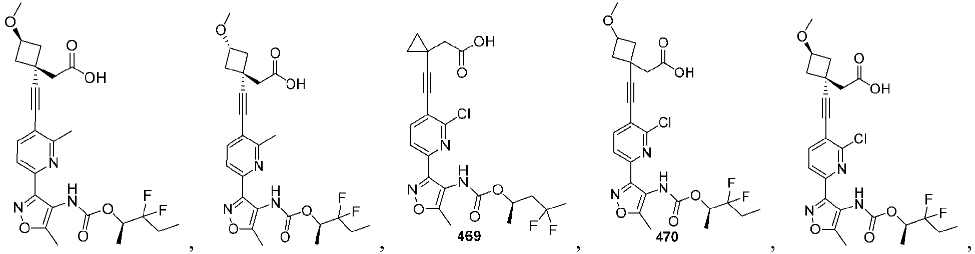

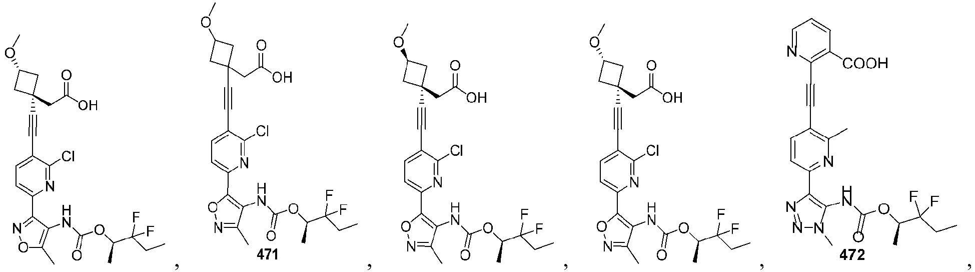

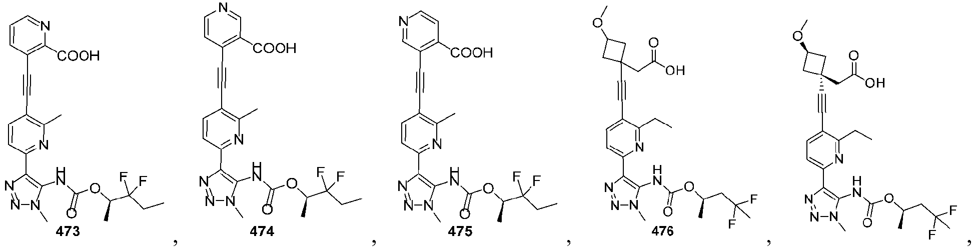

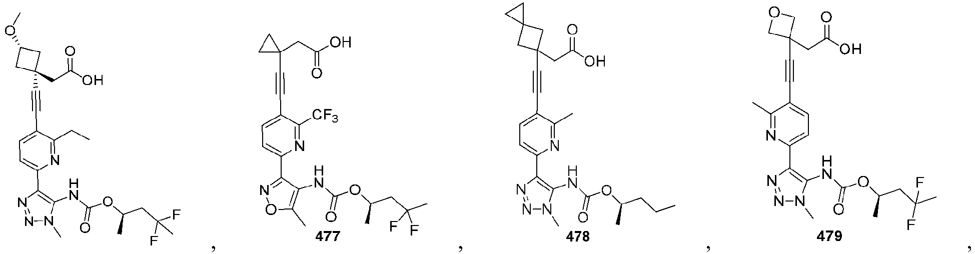

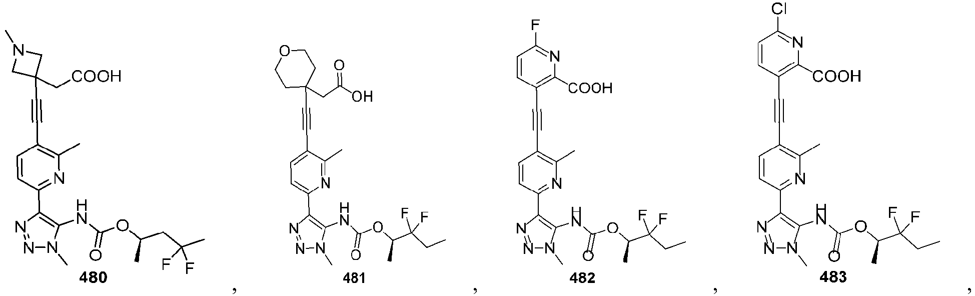

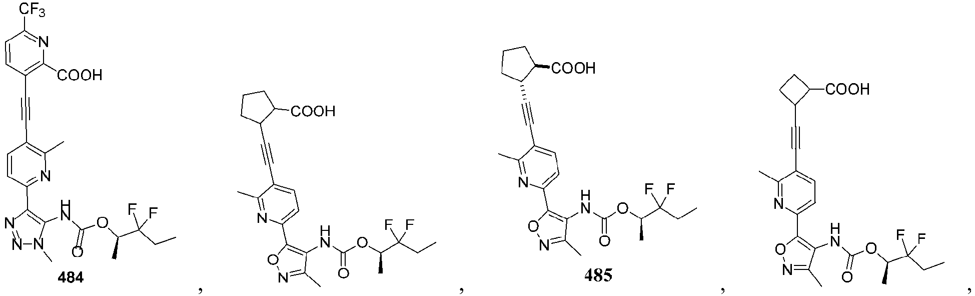

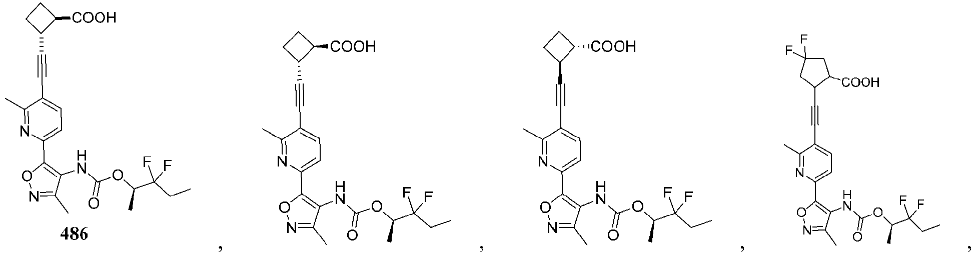

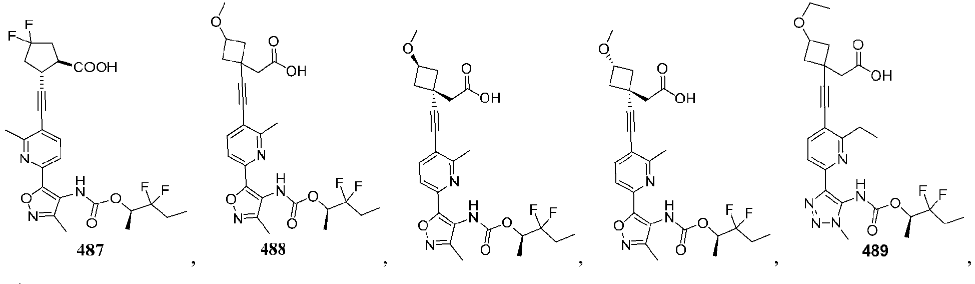

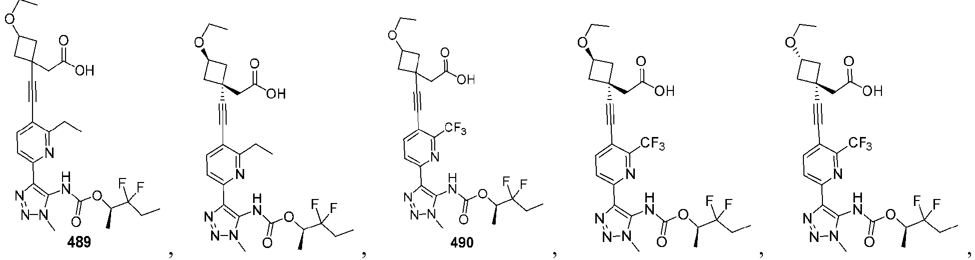

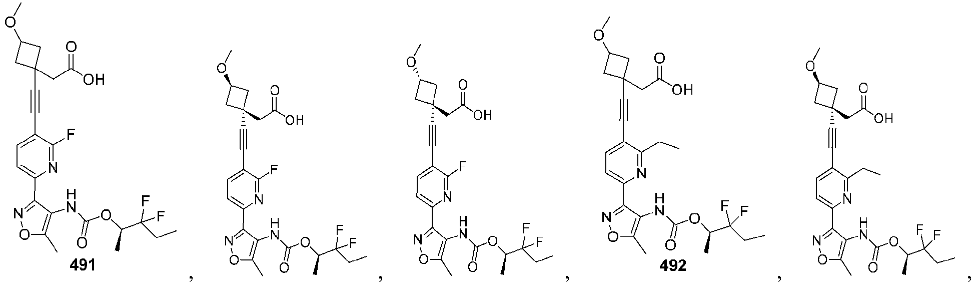

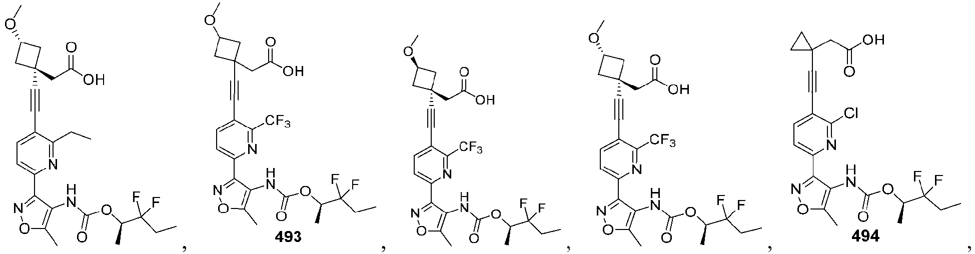

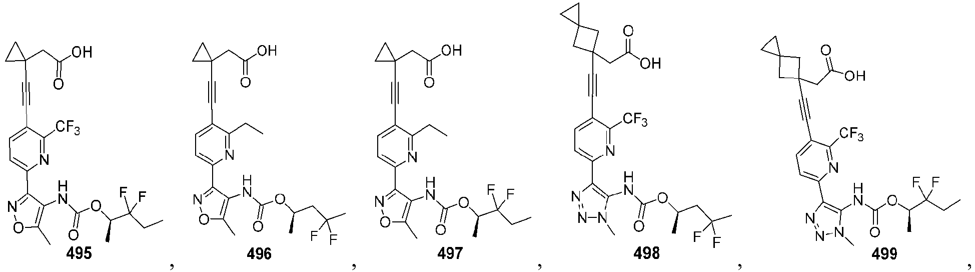

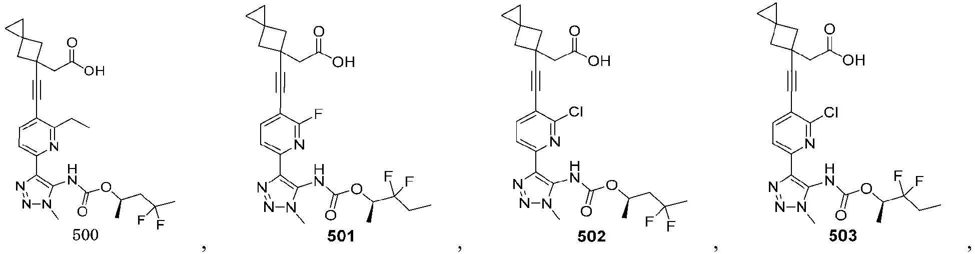

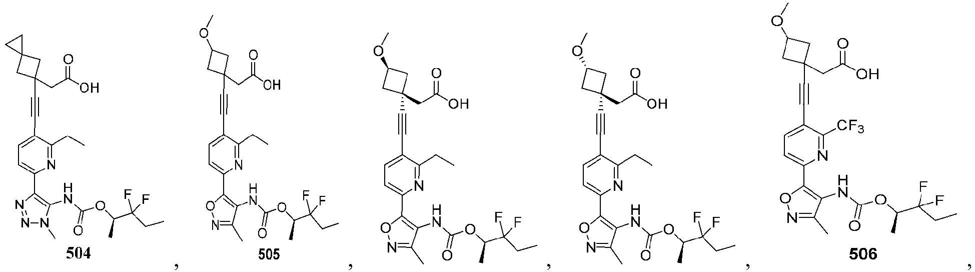

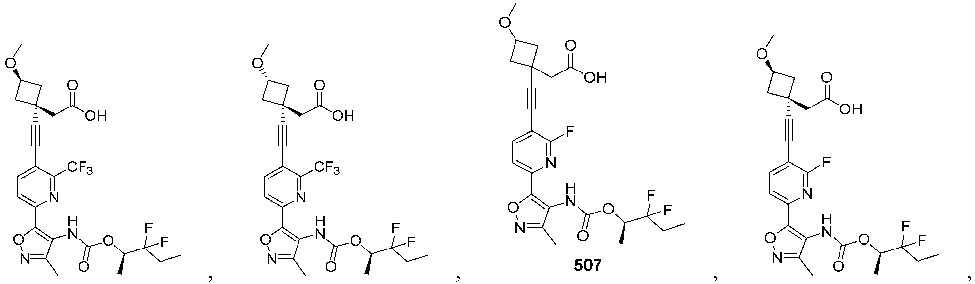

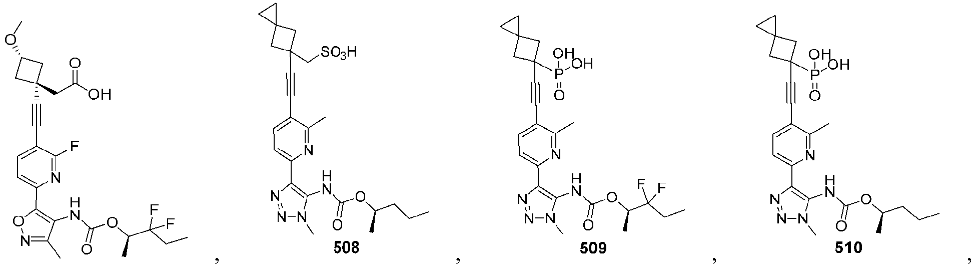

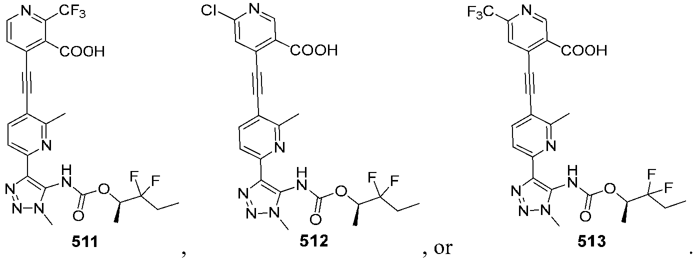

In a preferred embodiment, the compound may also be any one of the following compounds:
the compound 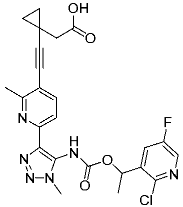 is a compound with a retention time of 13.097 min or 20.769 min under the following conditions:
chiral column: CHIRAL ART Amylose-C NEO, AD-H column, 30 × 250 mm; mobile phase A: *n-*hexane; mobile phase B: ethanol; volume ratio of mobile phases A and B: 83:17; isocratic elution; flow rate: 30 mL/min; column temperature: 25 to 28°C; detection wavelength: 220 nm;
the compound 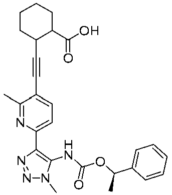 is a compound with a retention time of 5.550 min or 6.336 min under the following conditions:
   chiral column: Daicel Chiralpak IG column, 250 × 30 mm I.D., 5 µm; mobile phase A: supercritical carbon dioxide; mobile phase B: methanol and diethanolamine, with the volume percentage of diethanolamine in mobile phase B being 0.05%; volume ratio of mobile phases A and B: 80:20; isocratic elution; flow rate: 55 g/min; column temperature: 25 to 28°C; detection wavelength: 220 nm;
   the compound 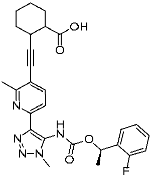 is a compound with a retention time of 4.963 or 5.364 under the following conditions:
      chiral column: Daicel Chiralpak IG column, 250 × 30 mm I.D., 5 µm; mobile phase A: supercritical carbon dioxide; mobile phase B: methanol and diethanolamine, with the volume percentage of diethanolamine in mobile phase B being 0.05%; volume ratio of mobile phases A and B: 80:20; isocratic elution; flow rate: 55 g/min; column temperature: 25 to 28°C; detection wavelength: 220 nm;
      the compound 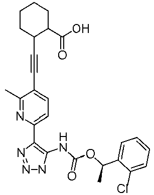 is a compound with a retention time of 8.410 or 7.53 under the following conditions:
         chiral column: CHIRALART Cellulose-SC, IC column, 30 × 250 mm; mobile phase A: n-hexane and diethylamine, with the volume percentage of ethylenediamine in mobile phase A being 0.1%; mobile phase B: ethanol; volume ratio of mobile phases A and B: 7:3; isocratic elution; flow rate: 30 mL/min; column temperature: 25 to 28°C; detection wavelength: 220 nm;
         the compound 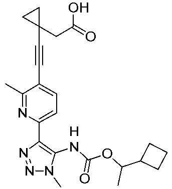 is a compound with a retention time of 3.241 or 3.424 under the following conditions:
            chiral column: CHIRALART Amylose-CNEO, AD-H column, 30 × 250 mm; mobile phase A: supercritical carbon dioxide; mobile phase B: 80% n-hexane and 20% ethanol by volume, volume ratio of mobile phases A and B: 80:20; isocratic elution; flow rate: 55 g/min; column temperature: 25 to 28°C; detection wavelength: 220 nm;
            the compound among 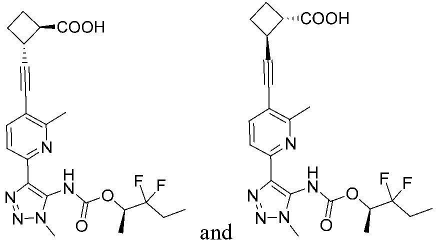 that elutes first under the following HPLC conditions: chiral column: Daicel Chiralpak IG column, 250 × 30 mm I.D., 5 µm; mobile phase A: supercritical carbon dioxide; mobile phase B: isopropanol (IPA) and NH₃H₂O, with NH₃H₂O accounting for 0.1 % by volume of mobile phase B, volume ratio of mobile phases A and B: 80:20; isocratic elution; flow rate: 60 g/min; preferably, column temperature: 25 to 28°C; detection wavelength: 220 nm; preferably, the retention time of the first-eluting compound is 9.353 min;
            the compound among 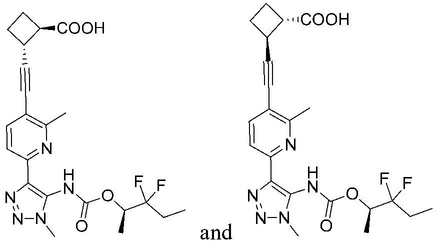 that elutes later under the following HPLC conditions: chiral column: Daicel Chiralpak IG column, 250 × 30 mm I.D., 5 µm; mobile phase A: supercritical carbon dioxide; mobile phase B: isopropanol (IPA) and NH₃H₂O, with NH₃H₂O accounting for 0.1 % by volume of mobile phase B, volume ratio of mobile phases A and B: 80:20; isocratic elution; flow rate: 60 g/min; preferably, column temperature: 25 to 28°C; detection wavelength: 220 nm; preferably, the retention time of the later-eluting compound is 10.778 min;
            the compound among 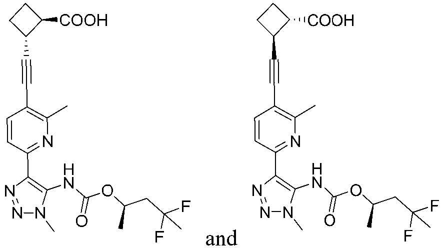 that elutes first under the following HPLC conditions: chiral column: Daicel Chiralpak IG column, 250 × 30 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 85:15; isocratic elution; flow rate: 55 g/min; preferably, column temperature: 25 to 28°C; detection wavelength: 220 nm; preferably, the retention time of the first-eluting compound is 11.977 min;
            the compound among 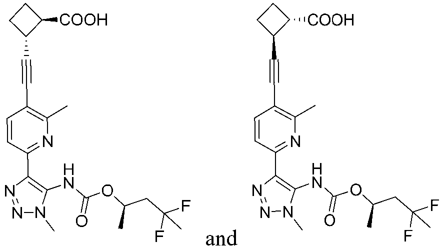 that elutes later under the following HPLC conditions: chiral column: Daicel Chiralpak IG column, 250 × 30 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 85:15; isocratic elution; flow rate: 55 g/min; preferably, column temperature: 25 to 28°C; detection wavelength: 220 nm; preferably, the retention time of the later-eluting compound is 14.219 min;
            the compound among 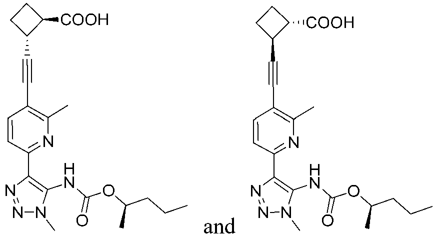 that elutes first under the following HPLC conditions: chiral column: Daicel Chiralpak IG column, 250 × 25 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 85:15; isocratic elution; flow rate: 55 g/min; preferably, column temperature: 25 to 28°C; detection wavelength: 220 nm; preferably, the retention time of the first-eluting compound is 9.181 min;
            the compound among 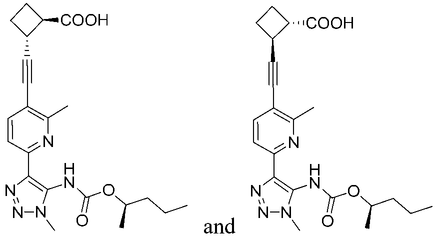 that elutes later under the following HPLC conditions: chiral column: Daicel Chiralpak IG column, 250 × 25 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 85:15; isocratic elution; flow rate: 55 g/min; preferably, column temperature: 25 to 28°C; detection wavelength: 220 nm; preferably, the retention time of the later-eluting compound is 11.647 min;
            the compound among 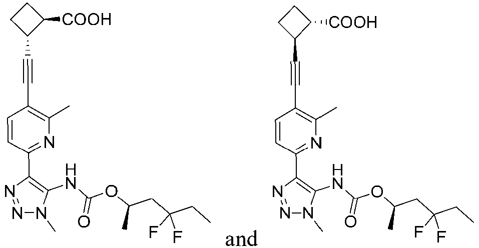 that elutes first under the following HPLC conditions: chiral column: Daicel Chiralpak IG column, 250 × 30 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 85:15; isocratic elution; flow rate: 55 g/min; preferably, column temperature: 25 to 28°C; detection wavelength: 220 nm; preferably, the retention time of the first-eluting compound is 10.806 min;
            the compound among 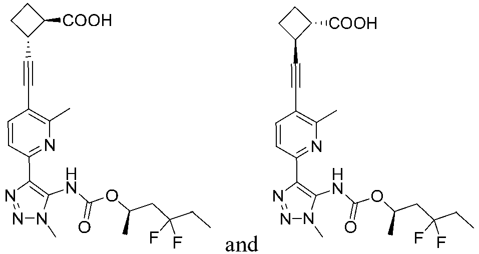 that elutes later under the following HPLC conditions: chiral column: Daicel Chiralpak IG column, 250 × 30 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 85:15; isocratic elution; flow rate: 55 g/min; preferably, column temperature: 25 to 28°C; detection wavelength: 220 nm; preferably, the retention time of the later-eluting compound is 12.817 min;
            the compound among 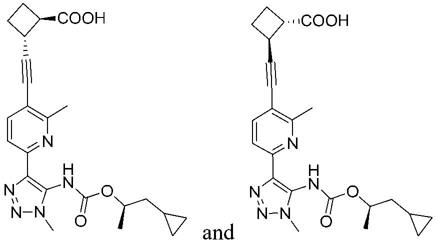 that elutes first under the following HPLC conditions: chiral column: YMC CHIRALART Cellulose-SC, IC column, 30 × 250 mm; mobile phase A: n-hexane; mobile phase B: isopropanol, volume ratio of mobile phases A and B: 33:67; isocratic elution; flow rate: 38 mL/min; preferably, column temperature: 25 to 28°C; detection wavelength: 220 nm; preferably, the retention time of the first-eluting compound is 10.566 min;
            the compound among 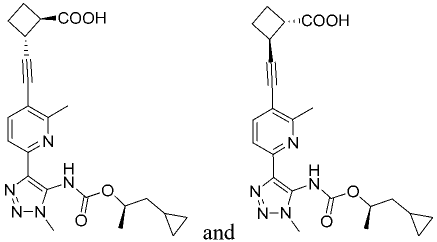 that elutes later under the following HPLC conditions: chiral column: YMC CHIRALART Cellulose-SC, IC column, 30 × 250 mm; mobile phase A: n-hexane; mobile phase B: isopropanol, volume ratio of mobile phases A and B: 33:67; isocratic elution; flow rate: 38 mL/min; preferably, column temperature: 25 to 28°C; detection wavelength: 220 nm; preferably, the retention time of the later-eluting compound is 13.500 min;
            the compound among 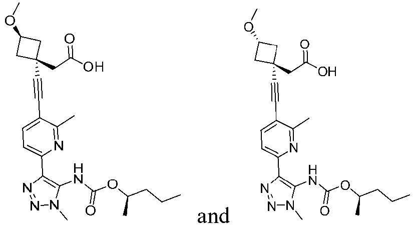 that elutes first under the following HPLC conditions: chiral column: Daicel Chiralpak IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 83:17; isocratic elution; flow rate: 58 g/min; preferably, column temperature: 25 to 28°C; detection wavelength: 220 nm; preferably, the retention time of the first-eluting compound is 7.463 min;
            the compound among 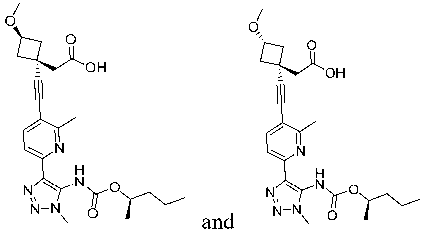 that elutes later under the following HPLC conditions: chiral column: Daicel Chiralpak IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 83:17; isocratic elution; flow rate: 58 g/min; preferably, column temperature: 25 to 28°C; detection wavelength: 220 nm; preferably, the retention time of the later-eluting compound is 8.014 min;
            the compound among 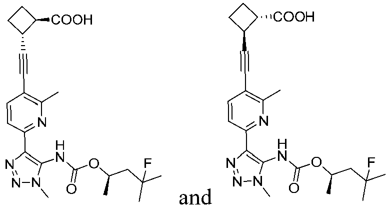 that elutes first under the following HPLC conditions: chiral column: Daicel Chiral IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 80:20; isocratic elution; flow rate: 60 g/min; preferably, column temperature: 25 to 28°C; detection wavelength: 220 nm; preferably, the retention time of the first-eluting compound is 10.152 min;
            the compound among 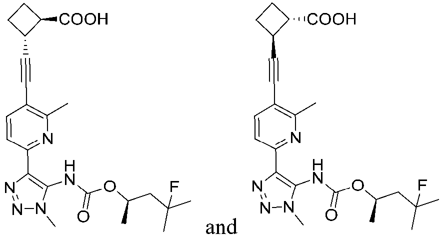 that elutes later under the following HPLC conditions: chiral column: Daicel Chiral IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 80:20; isocratic elution; flow rate: 60 g/min; preferably, column temperature: 25 to 28°C; detection wavelength: 220 nm; preferably, the retention time of the later-eluting compound is 12.037 min;
            the compound among 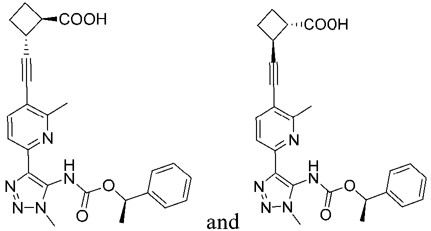 that elutes first under the following HPLC conditions: chiral column: YMC CHIRALART cellulose-SC, 30 × 250 mm; mobile phase A: n-hexane; mobile phase B: isopropanol, volume ratio of mobile phases A and B: 33:67; isocratic elution; flow rate: 30 mL/min; preferably, column temperature: 25 to 28°C; detection wavelength: 220 nm; preferably, the retention time of the first-eluting compound is 13.546 min;
            the compound among 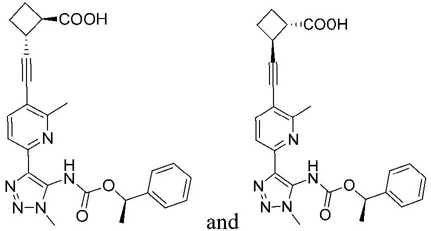 that elutes later under the following HPLC conditions: chiral column: YMC CHIRALART cellulose-SC, 30 × 250 mm; mobile phase A: n-hexane; mobile phase B: isopropanol, volume ratio of mobile phases A and B: 33:67; isocratic elution; flow rate: 30 mL/min; preferably, column temperature: 25 to 28°C; detection wavelength: 220 nm; preferably, the retention time of the later-eluting compound is 15.910 min;
            the compound among 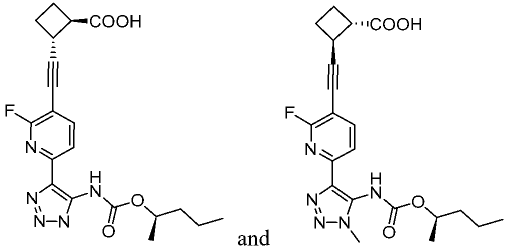 that elutes first under the following HPLC conditions:
               chiral column: Daicel Chiral IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 60:40; isocratic elution; flow rate: 70 g/min; preferably, column temperature: 25 to 28°C; detection wavelength: 220 nm; preferably, the retention time of the first-eluting compound is 12.749 min;
               the compound among 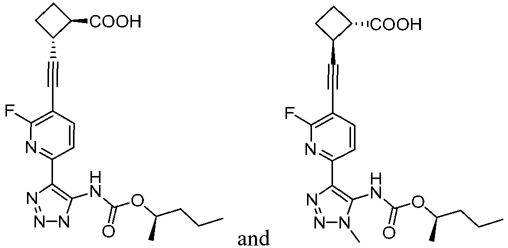 that elutes later under the following HPLC conditions:
                  chiral column: Daicel Chiral IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 60:40; isocratic elution; flow rate: 70 g/min; preferably, column temperature: 25 to 28°C; detection wavelength: 220 nm; preferably, the retention time of the later-eluting compound is 14.022 min;
                  the compound among 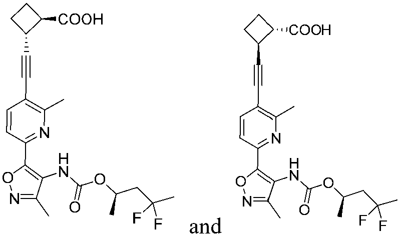 that elutes first under the following HPLC conditions: chiral column: Daicel Chiralpak IK column, 250 × 25 mm I.D., 10 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 82:18; isocratic elution; flow rate: 57 g/min; preferably, column temperature: 25 to 28°C; detection wavelength: 220 nm; preferably, the retention time of the first-eluting compound is 15.825 min;
                  the compound among 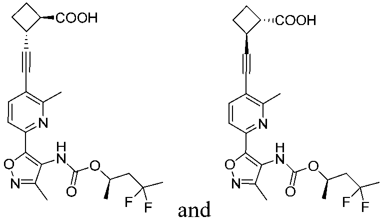 that elutes later under the following HPLC conditions: chiral column: Daicel Chiralpak IK column, 250 × 25 mm I.D., 10 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 82:18; isocratic elution; flow rate: 57 g/min; preferably, column temperature: 25 to 28°C; detection wavelength: 220 nm; preferably, the retention time of the later-eluting compound is 16.274 min;
                  the compound among 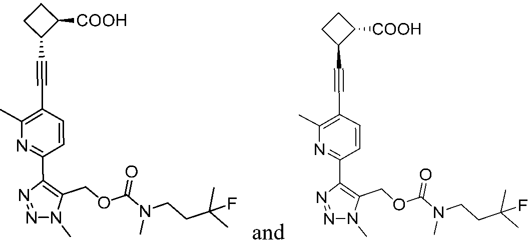 that elutes first under the following HPLC conditions: chiral column: Daicel Chiralpak OX column, 250 × 25 mm I.D., 10 µm; mobile phase A: supercritical carbon dioxide; mobile phase B: EtOH and NH₃H₂O, with NH₃H₂O accounting for 0.1% by volume of mobile phase B, volume ratio of mobile phases A and B: 80:20; isocratic elution; flow rate: 55 g/min; preferably, column temperature: 25 to 28°C; detection wavelength: 220 nm; preferably, the retention time of the first-eluting compound is 16.445 min;
                  the compound among 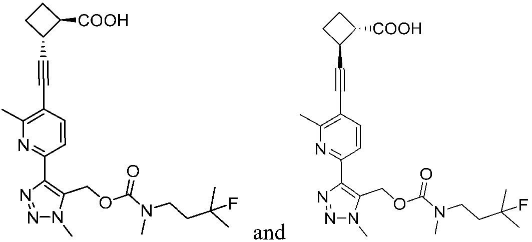 that elutes later under the following HPLC conditions: chiral column: Daicel Chiralpak OX column, 250 × 25 mm I.D., 10 µm; mobile phase A: supercritical carbon dioxide; mobile phase B: EtOH and NH₃H₂O, with NH₃H₂O accounting for 0.1% by volume of mobile phase B, volume ratio of mobile phases A and B: 80:20; isocratic elution; flow rate: 55 g/min; preferably, column temperature: 25 to 28°C; detection wavelength: 220 nm; preferably, the retention time of the later-eluting compound is 18.880 min;
                  the compound among 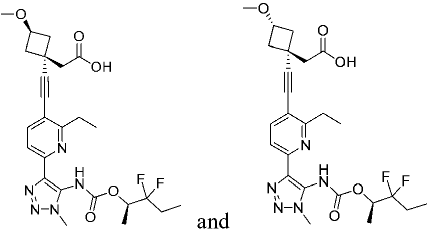 that elutes first under the following HPLC conditions: chiral column: Daicel Chiralpak IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 88:12; isocratic elution; flow rate: 55 g/min; preferably, column temperature: 25 to 28°C; detection wavelength: 220 nm; preferably, the retention time of the first-eluting compound is 13.164 min;
                  the compound among 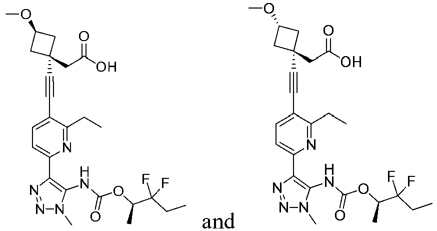 that elutes later under the following HPLC conditions: chiral column: Daicel Chiralpak IG column, 250 × 30 mm I.D., 5 µm; mobile phase A: supercritical carbon dioxide; mobile phase B: isopropanol and NH₃H₂O, with NH₃H₂O accounting for 0.1% by volume of mobile phase B, volume ratio of mobile phases A and B: 88:12; isocratic elution; flow rate: 55 g/min; preferably, column temperature: 25 to 28°C; detection wavelength: 220 nm; preferably, the retention time of the later-eluting compound is 13.847 min;
                  the compound among 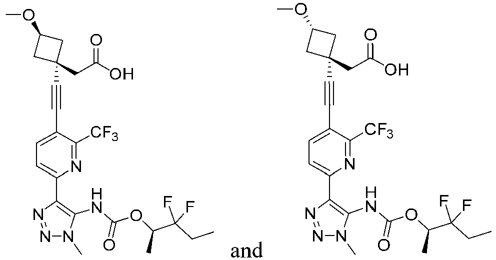 that elutes first under the following HPLC conditions: chiral column: Daicel Chiralpak OD column, 250 × 30 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 88:12; isocratic elution; flow rate: 53 g/min; preferably, column temperature: 25 to 28°C; detection wavelength: 220 nm; preferably, the retention time of the first-eluting compound is 9.828 min;
                  the compound among 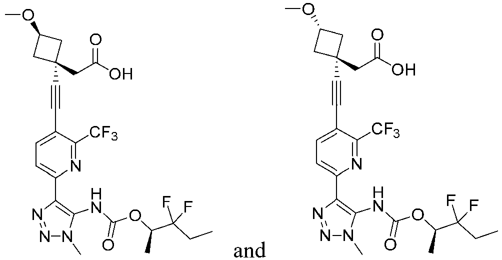 that elutes later under the following HPLC conditions: chiral column: Daicel Chiralpak OD column, 250 × 30 mm I.D., 5 µm; mobile phase A: supercritical carbon dioxide; mobile phase B: EtOH and NH₃H₂O, with NH₃H₂O accounting for 0.1% by volume of mobile phase B, volume ratio of mobile phases A and B: 88:12; isocratic elution; flow rate: 53 g/min; preferably, column temperature: 25 to 28°C; detection wavelength: 220 nm; preferably, the retention time of the later-eluting compound is 10.576 min.

The present disclosure also provides a preparation method for a compound of formula (I), wherein the method is the following method 1, method 2, method 3, or method 4:
method 1: subjecting a compound of formula I'-1A to an ester hydrolysis reaction in a solvent in the presence of a base to obtain the compound of formula (I); 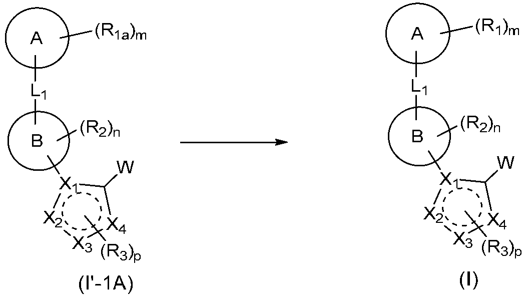
each R₁ₐ is C₁₋₆ alkyl substituted with 1, 2, or more R₁ₐ₋₁ substituents or -C(=O)O-C₁₋₆ alkyl; each R₁ₐ₋₁ is -C(=O)O-C₁₋₆ alkyl; each R₁ is C₁₋₆ alkyl substituted with 1, 2, or more COOH substituents or COOH; m is 1, 2, 3, 4, or 5;
ring A, ring B, Li, R₂, X₁, X₂, X₃, X₄, R₃, W, m, n, and p are as defined in any one embodiment of the present disclosure;
method 2: subjecting a compound of formula I'-1B and a compound of formula I'-1C to a reaction as shown in the following formula in a solvent in the presence of a base, phosphine ligand, and catalyst to obtain the compound of formula (I); 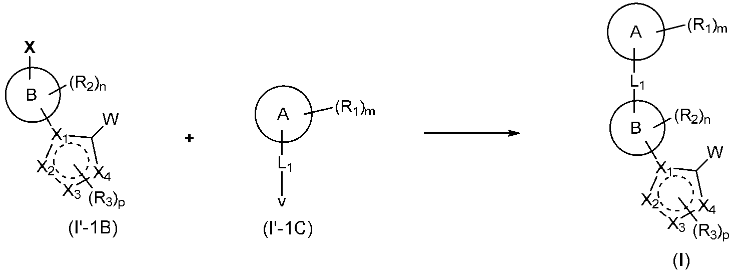
X is halogen (e.g., iodine), ring A, ring B, R₁, L₁, R₂, X₁, X₂, X₃, X₄, R₃, W, m, n, and p are as defined in any one embodiment of the present disclosure; V is H, -TMS, or TBDMS;
method 3: subjecting a compound of formula I'-1D to a reaction in a solvent in the presence of a cyanide and catalyst to obtain the compound of formula (I); 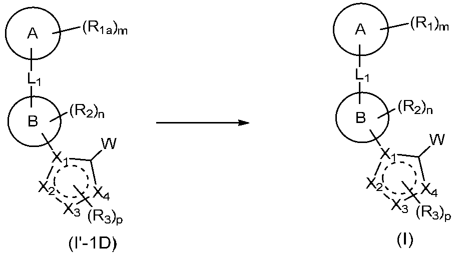
the cyanide is trimethylsilyl cyanide, potassium cyanide, or sodium cyanide;
each R₁ₐ is C₁₋₆ alkyl substituted with 1, 2, or more -OM substituents; each R₁ is C₁₋₆ alkyl substituted with 1, 2, or more cyano substituents or COOH; M is methanesulfonyl (Ms), p-toluenesulfonyl (Ts), or *p-*nitrobenzenesulfonyl (Ns);
ring A, ring B, Li, R₂, X₁, X₂, X₃, X₄, R₃, W, m, n, and p are as defined in any one embodiment of the present disclosure;
method 4:
   step 1) subjecting a compound of formula I'-1E with a compound of formula I'-1F or a salt thereof to a reaction in the presence of an acylation reagent to obtain a compound of formula I'-1G;
   step 2) subjecting the compound of formula I'-1G to an ester hydrolysis reaction in a solvent in the presence of a base to obtain a compound of formula (I-I'); 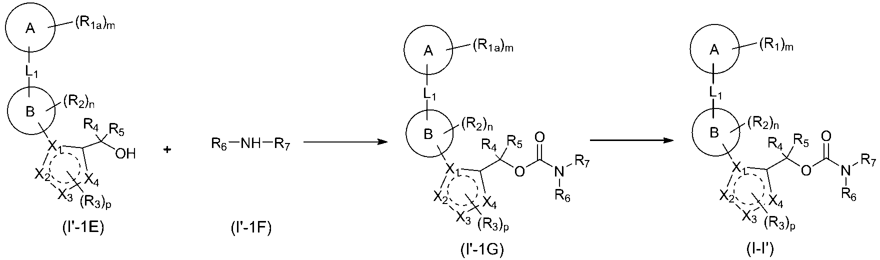
   each R₁ₐ is C₁₋₆ alkyl substituted with 1, 2, or more R₁ₐ₋₁ substituents or -C(=O)O-C₁₋₆ alkyl; each R₁ₐ₋₁ is -C(=O)O-C₁₋₆ alkyl; each R₁ is C₁₋₆ alkyl substituted with 1, 2, or more COOH substituents or COOH; m is 1, 2, 3, 4, or 5;
   ring A, ring B, Li, R₂, X₁, X₂, X₃, X₄, R₃, R₄, R₅, R₆, R₇, m, n, and p are as defined in any one embodiment of the present disclosure.

In a preferred embodiment, in method 1 or method 4, the solvent is an alcohol solvent and water, or a mixed solvent of an alcohol solvent and tetrahydrofuran; for example, a mixed solvent of methanol and water, for another example, a mixed solvent with a methanol-to-water volume ratio of 5:2, 4:1, 3:1, 2:1, or 1:1; or for example, a mixed solvent of methanol and tetrahydrofuran, for another example, a mixed solvent with a methanol-to-water volume ratio of 1:1 or 2:1.

In a preferred embodiment, in method 1 or method 4, the base is an inorganic base, for example, lithium hydroxide monohydrate and/or lithium hydroxide, for another example, lithium hydroxide monohydrate.

In a preferred embodiment, in method 1 or method 4, the temperature for the ester hydrolysis reaction is 20 to 70°C, for example, 25°C, 40°C, 50°C, or 60°C.

In a preferred embodiment, in method 1 or method 4, the duration of the ester hydrolysis reaction is 0.5 to 20 hours, for example, 1 hour, 2 hours, 4 hours, or 16 hours.

In a preferred embodiment, in method 1 or method 4, after the ester hydrolysis reaction is completed, the following post-treatment is further included: adjusting the pH to acidic or neutral, for example, adjusting the pH to 3, 5, 6, or 7 with dilute hydrochloric acid (dilute hydrochloric acid (1 M)).

In a preferred embodiment, in method 2, the solvent is an ether solvent, for example, 1,4-dioxane.

In a preferred embodiment, in method 2, the base is potassium carbonate and/or triethylamine.

In a preferred embodiment, in method 2, the phosphine ligand is bis(triphenylphosphine)palladium(II) chloride.

In a preferred embodiment, in method 2, the catalyst is copper iodide and/or cesium fluoride.

In a preferred embodiment, in method 2, the reaction is carried out under an inert atmosphere, for example, a nitrogen atmosphere.

In a preferred embodiment, in method 2, the reaction temperature is 50 to 100°C, for example, 50°C, 70°C, 80°C, 90°C, or 100°C.

In a preferred embodiment, in method 2, the reaction time is 0.5 to 6 hours, preferably 0.5 to 5 hours, for example, 1 hour, 2 hours, 3 hours, 4 hours, 5 hours, or 6 hours.

In a preferred embodiment, in method 3, the solvent is a nitrile solvent, for example, acetonitrile.

In a preferred embodiment, in method 3, the catalyst is an ammonium catalyst, for example, tetrabutylammonium fluoride.

In a preferred embodiment, in method 3, the reaction temperature is 50 to 100°C, for example, 80°C.

In a preferred embodiment, in method 3, the reaction time is 1 to 3 hours, for example, 1 hour or 2 hours.

In a preferred embodiment, in method 4, the acylation reagent connects the -OH group of the compound of formula I'-1E and the -NH- group of the compound of formula I'-1F via a carbonyl group in the reaction system to form the compound of formula I'-1G; preferably, the acylation reagent is*p*-nitrophenyl chloroformate, *N,N'*-disuccinimidyl carbonate, *p*-nitrophenyl chloroformate, bis(*p*-nitrophenyl) carbonate, 2,2,2-trichloroethyl chloroformate, isopropenyl chloroformate, phenyl chloroformate, dimethyl carbonate, triphosgene, or *N,N'*-carbonyldiimidazole.

In a preferred embodiment, in step 1) of method 4, the acylation reagent forms an active ester with the compound of formula I'-1E, which then reacts with the compound of formula I'-1F to form the compound of formula I'-1G.

In a preferred embodiment, in method 4, the salt of the compound of formula I'-1F is a hydrochloride salt of the compound of formula I'-1F.

The compound of formula (I), the racemate thereof, the stereoisomer thereof, the tautomer thereof, the N-oxide thereof, or the pharmaceutically acceptable salt or prodrug of any one of the foregoing as described in the present disclosure, may also be synthesized by methods analogous to those known in the chemical field, with reference to the steps and conditions of similar reactions in the art, particularly according to the descriptions provided herein. Starting materials are generally obtained from commercial sources or can be readily prepared using methods well-known to those skilled in the art (accessible via SciFinder or Reaxys online databases).

The present disclosure provides a pharmaceutical composition comprising (a therapeutically effective amount of) substance U and a pharmaceutical excipient, wherein substance U is the compound of formula (I), the racemate thereof, the stereoisomer thereof, the tautomer thereof, the N-oxide thereof, or the pharmaceutically acceptable salt of any one of the foregoing as described in any one embodiment of the present disclosure.

According to the present disclosure, the pharmaceutical composition can be formulated into dosage forms suitable for administration by methods known in the art.

The present disclosure further provides a use of the above substance U or the above pharmaceutical composition in the manufacture of an LPAR1 antagonist; the LPAR1 antagonist can be used in mammalian organisms; the LPAR1 antagonist can also be used *in vitro,* for example for experimental purposes, or for example as a standard or control sample for comparison, or prepared into a kit according to conventional methods in the art to provide rapid detection of LPAR1 antagonist effects.

The present disclosure further provides a use of the above substance U or the above pharmaceutical composition in the manufacture of a medicament.

In a preferred embodiment, the medicament is a medicament for diagnosing, preventing, and/or treating a disease or disorder mediated by an LPAR receptor.

In a preferred embodiment, the medicament is a medicament for diagnosing, preventing, and/or treating a disease or disorder mediated by an LPAR1 receptor.

In a preferred embodiment, the medicament is an LPAR1 antagonist.

In a preferred embodiment, the disease or disorder is a fibrotic disease, a respiratory disease, pain, a neurological disease, a cardiovascular or cerebrovascular disease, an inflammatory disease, a kidney disease, a liver disease, an ocular disease, a cancer, a gastrointestinal disease, a urinary system disease, a metabolic disease, or transplant rejection.

In a preferred embodiment, the fibrotic disease includes but is not limited to: pulmonary fibrosis (particularly idiopathic pulmonary fibrosis, progressive pulmonary fibrosis), renal fibrosis, hepatic fibrosis, skin fibrosis, intestinal fibrosis, ocular fibrosis, cardiac fibrosis, or pancreatic fibrosis.

In a preferred embodiment, the respiratory disease includes but is not limited to: interstitial lung disease (ILD), idiopathic interstitial pneumonia (IIP), asthma, chronic obstructive pulmonary disease (COPD), bronchospasm, cough, chronic cough, respiratory failure, silicosis, acute lung injury, or acute respiratory distress.

In a preferred embodiment, the kidney disease includes but is not limited to: acute kidney injury, chronic kidney disease, or diabetic nephropathy.

In a preferred embodiment, the hepatic disease includes but is not limited to: alcoholic steatohepatitis, non-alcoholic fatty liver disease (NAFLD), acute hepatitis, chronic hepatitis, cirrhosis, hepatic insufficiency, primary biliary cirrhosis, and other hepatic diseases; the non-alcoholic fatty liver disease (NAFLD) may be non-alcoholic steatohepatitis (NASH).

In a preferred embodiment, the inflammatory disease includes but is not limited to: autoimmune disease, inflammation, arthritis, rheumatoid arthritis, scleroderma, Raynaud's phenomenon, chronic pruritus, lupus, cryptogenic fibrosing alveolitis, psoriasis, systemic sclerosis, or collagen vascular disease.

In a preferred embodiment, the neurological disease includes but is not limited to: Alzheimer's disease, Parkinson's disease, neurodegenerative disease, traumatic brain injury, epilepsy, mental illness, or sleep disorder.

In a preferred embodiment, the cardiovascular and cerebrovascular disease includes but is not limited to: collagen vascular disease, myocardial infarction, stroke, thrombosis, atherosclerosis, heart failure, or hypertension.

In a preferred embodiment, the gastrointestinal disease includes but is not limited to: irritable bowel syndrome, inflammatory bowel disease, digestive tract disease, or gastrointestinal dysfunction.

In a preferred embodiment, the pain includes but is not limited to: cancer pain, neuropathic pain, inflammatory pain, surgical pain, visceral pain, toothache, premenstrual pain, central pain, pain caused by burns, migraine, cluster headache, or chronic pain.

In a preferred embodiment, the urinary system disease includes urinary incontinence, dysuria, cystitis, prostatic hyperplasia, urinary disturbance associated with prostatic hyperplasia, bladder neck sclerosis, and underactive bladder.

In a preferred embodiment, the ocular disease includes macular degeneration and diabetic retinopathy.

In a preferred embodiment, the cancer includes but is not limited to: breast cancer, pancreatic cancer, ovarian cancer, prostate cancer, glioblastoma, bone cancer, colon cancer, intestinal cancer, liver cancer, head and neck cancer, melanoma, multiple myeloma, chronic lymphocytic leukemia, and tumor metastasis.

In a preferred embodiment, the metabolic disease includes but is not limited to: osteoporosis.

In a preferred embodiment, the disease or disorder is interstitial lung disease, pulmonary fibrosis (particularly idiopathic pulmonary fibrosis), hepatic fibrosis, renal fibrosis, non-alcoholic fatty liver disease (e.g., non-alcoholic steatohepatitis), psoriasis, or scleroderma.

The present disclosure also provides a method for diagnosing, preventing, and/or treating a disease or condition, comprising administering to a patient in need thereof a therapeutically effective amount of at least one compound of formula (I), the racemate thereof, the stereoisomer thereof, the tautomer thereof, the N-oxide thereof, or the pharmaceutically acceptable salt of any one of the foregoing or the above pharmaceutical composition as described in any one embodiment of the present disclosure alone or optionally in combination with another compound of the present disclosure and/or at least one other type of therapeutic agent.

The disease or disorder is as described in any one of the embodiments of the present disclosure.

The compounds of the present disclosure may be used in combination with additional therapeutic agents.

### Definition and explanation of terms

Unless otherwise specified, the definitions of groups and terms recorded in the specification and claims of the present disclosure, including their definitions as examples, exemplary definitions, preferred definitions, definitions recorded in tables, definitions of specific compounds in examples, *etc.,* may be arbitrarily combined and linked with each other. Such combined and linked group definitions and compound structures shall be understood as falling within the scope recorded in the specification and/or claims of the present disclosure.

The term "optionally" means that it may be substituted or unsubstituted. Unless otherwise specified, the type and number of substituents may be arbitrary on the basis of chemical feasibility. For example, the C₁₋₆ alkyl optionally substituted with 1, 2, or more R_{c} substituents represents unsubstituted C₁₋₆ alkyl or C₁₋₆ alkyl substituted with 1, 2, or more R_{c} substituents.

When any variable (*e.g.,* Rₐ, C₁₋₆ alkyl) appears multiple times in the definition of a compound, the definition of the variable at each occurrence is independent of its definition at other occurrences, and their meanings are independent of each other and do not affect each other. Therefore, if a group is substituted with 1, 2, or more Rₐ substituents, that is to say, the group may be substituted with 3 or more Rₐ substituents, the definition of Rₐ at this position is independent of the definition of Rₐ at other positions. Additionally, combinations of substituents and/or variables are permitted only when such combinations result in stable compounds.

When a substituent appears multiple times in the definition of a compound, these repeatedly occurring identical substituents are collectively referred to as "each" in the unified definition. For example, if C₁₋₆ alkyl appears multiple times in the definition of a compound, these repeatedly occurring C₁₋₆ alkyl groups are collectively referred to as "each C₁₋₆ alkyl" in the unified definition, and their meanings are independent of each other and do not affect each other.

Those skilled in the art will understand that, according to the conventions used in the art, the " 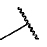 " in the structural formulas of groups described in the present disclosure indicates that the corresponding group is connected to other moieties or groups in the compound through this site.

The "-" at the end of a group indicates that the group is connected to other moieties in the molecule through this site. For example, -C₂₋₃ alkylene-COOH means that the C₂₋₃ alkylene therein is connected to other moieties in the molecule, not the COOH is connected to other moieties in the molecule.

Unless otherwise specified, a solid wedge bond ( 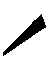 ) and a dashed wedge bond ( 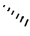 ) indicate the absolute configuration of a stereocenter, while a straight solid bond ( 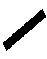 ) and a straight dashed bond ( 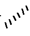 ) indicate the relative configuration of a stereocenter.

Unless otherwise specified, the term "cis-trans isomer" refers to an isomer resulting from the inability of a double bond or a single bond of a ring-forming carbon atom to rotate freely.

The "room temperature" described in the specification of the present disclosure shall be understood as the ambient temperature during the experiment, for example, 10 to 35°C, preferably 25°C ± 5°C. The "room temperature" described in the specification of the present disclosure is denoted as "r.t." in reaction flowcharts.

In the specification of the present disclosure, "more" means three or more, such as 3, 4, 5, or 6.

In the claims of the present disclosure, "more" in "satisfying one or more of the following conditions" refers to 2, 3, 4, or more, where the maximum value of "more" is the largest numerical value of conditions specified in each claim. For example, if a claim specifies 8 conditions, "one or more" in "satisfying one or more of the following conditions" in that claim refers to any integer from 1 to 8, such as 1, 2, 3, 4, 5, 6, 7, or 8.

The term "Cₙ₋ₘ" and "Cₙ-Cₘ", where n and m are integers, represent groups containing n to m carbon atoms. Examples include C₁₋₆, C₁₋₃, and the like. This term is intended to explicitly disclose each member within this range, namely Cₙ, Cₙ₊₁, Cₙ₊₂ ......Cₘ₋₂, Cₘ₋₁, and Cₘ. For example, C₁₋₆ is intended to disclose C₁, C₂, C₃, C₄, C₅, and C₆. The term "Cₙ₋ₘ" has the same meaning as "Cₙ-Cₘ".

The term "n-membered", where n is an integer, typically describes that the number of ring-forming atoms is n.

The term "n- to m-membered", where n and m are integers, describes that the number of ring-forming atoms is in the range of n to m. For example, piperidinyl is an example of a 6-membered heterocycloalkyl ring, pyrazolyl is an example of a 5-membered heteroaryl ring, and pyridinyl is an example of a 6-membered heteroaryl ring.

Unless otherwise specified, the numerical ranges recited in this specification and claims are equivalent to explicitly reciting each and every specific integer value therein. For example, the numerical range "1 to 10" is equivalent to explicitly reciting each integer value within the range "1 to 10," namely 1, 2, 3, 4, 5, 6, 7, 8, 9, and 10.

The term "oxo (=O)" refers to the substitution of hydrogen or lone pair electrons on a non-oxygen atom with oxygen, for example, 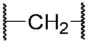 after oxo substitution becomes 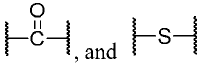 after oxo substitution becomes

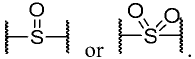

The term "alkyl" refers to a monovalent straight or branched saturated hydrocarbon group. The alkyl includes C₁₋₆ alkyl, C₁₋₃ alkyl, C₁₋₄ alkyl, C₃₋₄ alkyl, C₄₋₆ alkyl, and the like. Examples of the alkyl include, but are not limited to, methyl, ethyl, *n*-propyl, isopropyl, *n*-butyl, *n*-pentyl, *n*-hexyl, isobutyl, *sec-*butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof.

The term "alkylene" refers to a substituent formed by removing two hydrogen atoms from a straight or branched alkane. The two hydrogen atoms removed may be from the same carbon atom or from different carbon atoms (*e.g.,* the two hydrogen atoms removed are from the terminal carbon atoms, respectively). The alkylene includes C₁₋₆ alkylene, C₁₋₃ alkylene, C₁₋₄ alkylene, C₃₋₄ alkylene, C₄₋₆ alkylene, C₂₋₃ alkylene, and the like. Thus, C₁ alkylene (*i.e.,* methylene) refers to -CH₂-, C₂ alkylene (*i.e.,* ethylene) refers to -CH₂-CH₂- or -CH(CH₃)-, and C₃ alkylene (*i.e.,* propylene) refers to -CH₂-CH₂-CH₂-, -CH(CH₂-CH₃)-, -C(CH₃)₂-, or - CH₂-CH(CH₃)-.

The term "alkoxy" refers to the group -O-R^{X}, wherein R^{X} is the alkyl as defined above.

The term "C₂₋₆ alkynylene" refers to a substituent formed by removing two hydrogen atoms from a straight or branched alkyne having 2 to 6 carbon atoms and containing at least one carbon-carbon triple bond. The one or more carbon-carbon triple bonds may be internal or terminal, and the alkynyl includes but is not limited to ethynylene, 1-propynylene, 2-propynylene, 2-pentynylene, 3-butynylene, 1-butynylene, and the like.

The term "cycloalkyl" refers to a cyclic alkyl group having a specified number of ring atoms, which includes monocyclic, bicyclic, or tricyclic alkyl groups, wherein the bicyclic and tricyclic alkyl groups comprise fused rings, bridged rings, or spiro rings. The cycloalkyl includes C₃₋₁₀ cycloalkyl, C₃₋₈ cycloalkyl, C₄₋₈ cycloalkyl, C₃₋₆ cycloalkyl, C₃₋₅ cycloalkyl, C₄₋₆ cycloalkyl, C₃₋₄ cycloalkyl, C₅₋₆ cycloalkyl, and the like. Examples of the cycloalkyl include, but are not limited to, cyclopropyl, cyclobutyl, cyclopentyl, cyclohexyl, cycloheptyl, cyclooctyl, bicyclo[1.1.1]pentyl, spiro[2.3]hexyl, bicyclo[2.1.1]hexyl, bicyclo[3.1.0]hexyl, bicyclo[3.2.0]heptyl, spiro[2.4]heptyl, bicyclo[2.2.2]octyl, and the like.

The term "heterocycloalkyl" refers to a saturated ring or ring system having a specified number of ring atoms (*e.g.,* 3- to 10-membered, 3- to 8-membered) wherein the ring atoms include 1, 2, or 3 heteroatoms selected from O, S, and N, and the remaining ring atoms are carbon atoms, wherein N and S may optionally be oxidized to various oxidation states to form N-oxides, -S(=O)-, or -S(=O)₂-. The 3- to 10-membered heterocycloalkyl may be a 3-, 4-, 5-, 6-, or 7-membered monocyclic ring, a 5-, 6-, 7-, 8-, 9-, or 10-membered bicyclic ring system, or a 10-membered tricyclic ring system, wherein the bicyclic and tricyclic systems include fused rings, bridged rings, or spiro rings. The heterocycloalkyl includes 3- to 10-membered heterocycloalkyl, 3- to 6-membered heterocycloalkyl, 3- to 8-membered heterocycloalkyl, 4- to 5-membered heterocycloalkyl, 4- to 8-membered heterocycloalkyl, 4- to 6-membered heterocycloalkyl, 5- to 6-membered heterocycloalkyl, and the like. Examples of the heterocycloalkyl include, but are not limited to, azetidinyl, oxetanyl, tetrahydrofuranyl (oxolanyl), pyrrolidinyl (azolidinyl), imidazolidinyl, pyrazolidinyl, tetrahydropyranyl (oxanyl), piperidinyl (azinanyl), oxiranyl, oxepanyl, oxocanyl, aziridinyl, azepanyl, or azocanyl, and the like.

The term "C₆₋₁₀ aryl" refers to an aromatic monocyclic, bicyclic, or tricyclic cyclic group having 6, 7, 8, 9, or 10 carbon atoms, wherein the bicyclic and tricyclic hydrocarbon rings may be fused rings. Among them, the ring having 6 carbon atoms ("C₆ aryl"), such as phenyl; or the ring having 9 carbon atoms ("C₉ aryl"), such as indenyl; or the ring having 10 carbon atoms ("C₁₀ aryl"), such as naphthyl. When the C₆₋₁₀ aryl is substituted, it may be monosubstituted or polysubstituted. Furthermore, there are no restrictions on the substitution positions, and for example, it may be ortho-, para-, or meta-substitution.

The term "C₆₋₁₀ arylene" refers to a group formed by removing two hydrogen atoms from an aromatic monocyclic, bicyclic, or tricyclic hydrocarbon ring having 6, 7, 8, 9, or 10 carbon atoms, wherein the bicyclic and tricyclic aromatic hydrocarbon rings may be fused rings. Among them, the ring having 6 carbon atoms ("C₆ arylene"), such as phenylene; or the ring having 9 carbon atoms ("C₉ arylene"), such as indenylene, or the ring having 10 carbon atoms ("C₁₀ arylene"), such as naphthylene. When the C₆₋₁₀ arylene is substituted, it may be monosubstituted or polysubstituted. Furthermore, there are no restrictions on the substitution positions, and for example, it may be ortho-, para-, or meta-substitution.

The term "5- to 10-membered heteroaryl" refers to a monocyclic, bicyclic, or tricyclic aromatic group having 5 to 10 ring atoms, wherein the ring atoms include 1 to 3 heteroatoms independently selected from N, O, and S, with the remaining ring atoms being carbon atoms, and the bicyclic and tricyclic aromatic groups may be fused rings. When the group is a bicyclic or tricyclic aromatic group, at least one ring is an aromatic ring. The 5- to 10-membered heteroaryl preferably contains 1 to 2 heteroatoms. The 5- to 10-membered heteroaryl includes 4- to 9-membered heteroaryl, 5- to 9-membered heteroaryl, 6- to 9-membered heteroaryl, 5- to 7-membered heteroaryl, 8- to 10-membered heteroaryl, 5- to 6-membered heteroaryl, 6-membered heteroaryl, and the like. Examples of the heteroaryl include but are not limited to oxazolyl, pyrazolyl, triazolyl, thienyl, pyridinyl, pyrimidinyl, pyrazinyl, pyridazinyl, imidazolyl, tetrazolyl, isoxazolyl, thiazolyl, isothiazolyl, oxadiazolyl, furazanyl, thiadiazolyl, oxathiazolyl, furanyl, pyrrolyl, and the like, as well as their benzo derivatives. When the 5- to 10-membered heteroaryl is substituted, it may be monosubstituted or polysubstituted. Moreover, there is no limitation on the substitution sites, for example, the hydrogen attached to a carbon atom on the heteroaryl ring may be substituted, or the hydrogen attached to a heteroatom on the heteroaryl ring may be substituted.

Optionally, the bicyclic and tricyclic aromatic groups may independently be benzo-fused, for example but not limited to benzopiperidinyl, quinolinyl, quinazolinyl, isoquinolinyl, cinnolinyl, phthalazinyl, quinazolinyl, quinoxalinyl, and the like. The "5- to 10-membered heteroaryl" may be bicyclic, for example, but not limited to: 5,5-membered rings, such as 4,6-dihydro-1*H*-furo[3,4-*c*]pyrazolyl, 2,6-dihydro-4*H-*furo[3,4-*c*]pyrazolyl, 5,6-dihydro-1*H*-furo[3,2-*c*]pyrazolyl, 5,6-dihydro-2*H*-furo[3,2-*c*]pyrazolyl, 2,3-dihydropyrazolo[5,1-*b*]oxazolyl, 5,6-dihydro-4*H*-pyrrolo[1,2-*b*]pyrazolyl, and 2,4,5,6-tetracyclopenta[*c*]pyrazolyl; 5,6-membered rings, such as 6,7-dihydro-5*H*-cyclopenta[*d*]pyrimidinyl.

The term "heteroarylene" refers to a group formed by removing two hydrogen atoms from a monocyclic, bicyclic, or tricyclic heteroaromatic hydrocarbon having a specified number of ring atoms (*e.g.,* 5- to 7-membered, 5- to 6-membered, 8- to 10-membered), wherein the ring atoms include 1, 2, or 3 heteroatoms independently selected from N, O, and S, with the remaining ring atoms being carbon atoms, and the bicyclic and tricyclic heteroarylene may be fused rings. In the bicyclic and tricyclic heteroarylene, at least one ring is an aromatic ring. The heteroarylene includes 5- to 7-membered heteroarylene, 5- to 6-membered heteroarylene, 6-membered heteroarylene, 8- to 10-membered heteroarylene, and the like. Examples of the heteroarylene include, but are not limited to, oxazolylene, pyrazolylene, triazolylene, thienylene, pyridinylene, pyrimidinylene, pyrazinylene, pyridazinylene, imidazolylene, tetrazolylene, isoxazolylene, thiazolylene, isothiazolylene, oxadiazolylene, furazanylene, thiadiazolylene, oxathiazolylene, furanylene, pyrrolylene, triazinylene, and the like, as well as their benzo derivatives.

The term "heterocyclyl" refers to a saturated or partially unsaturated non-aromatic ring or ring system having a specified number of ring atoms (*e.g.,* 3- to 10-membered, 3- to 8-membered, 3- to 6-membered), wherein the ring atoms include 1 to 5 heteroatoms independently selected from O, S, and N, with the remaining ring atoms being carbon atoms, and wherein N and S may optionally be oxidized to various oxidation states to form N-oxides, -S(=O)-, or -S(=O)₂-. The heterocyclyl may be a 3-, 4-, 5-, 6-, or 7-membered monocyclic ring system, a 5-, 6-, 7-, 8-, 9-, or 10-membered bicyclic ring system, or a 10-membered tricyclic ring system, wherein the bicyclic and tricyclic ring systems include fused rings, bridged rings, or spiro rings. The 3- to 10-membered heterocyclyl includes 3- to 6-membered heterocyclyl, 4- to 8-membered heterocyclyl, 4- to 6-membered heterocyclyl, 4- to 5-membered heterocyclyl, and the like. The heterocyclyl may be attached to the rest of the molecule through any one carbon atom or nitrogen atom (if present) on its ring. Examples of the heterocyclyl include, but are not limited to: 4-membered rings such as azetidinyl, oxetanyl; 5-membered rings such as tetrahydrofuranyl, dihydrofuranyl, dioxolyl, 2,3-dihydro-1*H-*imidazolyl, pyrrolidinyl, imidazolidinyl, pyrazolidinyl, pyrrolinyl; or 6-membered rings such as dihydropyranyl, tetrahydropyranyl, piperidinyl, morpholinyl, dithianyl, thiomorpholinyl, piperazinyl, or trithianyl.

The term "spiro ring" refers to a ring system in which two rings share one ring-forming atom.

The term "fused ring" refers to a ring system in which two rings share two ring-forming atoms.

The term "bridged ring" refers to a ring system in which two rings share three or more ring-forming atoms.

The term "halogen" refers to fluorine, chlorine, bromine, and iodine.

Those skilled in the art will understand that the compound of formula (I) may exist in the form of various pharmaceutically acceptable salts. If these compounds have a basic center, they may form acid addition salts; if these compounds have an acidic center, they may form base addition salts; if these compounds contain both an acidic center (*e.g.,* carboxyl) and a basic center (*e.g.,* amino), they may also form inner salts.

Depending on their molecular structure, the compounds of the present disclosure may be chiral (having one or more stereocenters), and thus may exist in various enantiomeric or diastereomeric forms. Consequently, these compounds may exist in racemic form or optically active form. The compounds of the present disclosure encompass isomers or mixtures thereof with each chiral carbon in R or S configuration, as well as racemates. The compounds of the present disclosure or intermediates thereof may be separated into enantiomeric compounds by chemical or physical methods known to those skilled in the art, or used in this form for synthesis. In the case of racemic amines, diastereomers are prepared from the mixture by reaction with optically active resolving agents.

In some embodiments, the compounds of the present disclosure have (*R*)-configuration. In other embodiments, the compounds have (*S*)-configuration. In compounds having more than one chiral center, unless otherwise specified, each chiral center in the compound may independently be (*R*) or *(S*). In some embodiments, the compounds of the present disclosure are trans isomers. In other embodiments, the compounds of the present disclosure are cis isomers.

The compounds described herein may also include all isotopes of atoms present in the intermediates or final compounds. Isotopes include those atoms having the same atomic number but different mass numbers. For example, the isotopes of hydrogen include tritium and deuterium.

The term "patient" refers to any animal including mammals, preferably mice, rats, other rodents, rabbits, dogs, cats, pigs, cattle, sheep, horses, or primates, most preferably humans.

The term "therapeutically effective amount" refers to the amount of an active compound or medicament that a researcher, veterinarian, physician, or other clinician is seeking to elicit a biological or medical response in a tissue, system, animal, individual, or human, which includes one or more of the following: (1) preventing a disease: for example, preventing a disease, disorder, or condition in an individual who is susceptible to the disease, disorder, or condition but has not yet experienced or exhibited the pathology or symptoms of the disease; (2) inhibiting a disease: for example, inhibiting a disease, disorder, or condition in an individual who is experiencing or exhibiting the pathology or symptoms of the disease, disorder, or condition (*i.e.,* arresting further development of the pathology and/or symptoms); (3) alleviating a disease: for example, alleviating a disease, disorder, or condition in an individual who is experiencing or exhibiting the pathology or symptoms of the disease, disorder, or condition (*i.e.,* reversing the pathology and/or symptoms).

The term "pharmaceutical excipient" refers to the excipients and additives used in medicament manufacturing and prescription formulation, encompassing all substances included in pharmaceutical preparations other than the active ingredients.

Without departing from the common knowledge in the art, the above preferred conditions may be arbitrarily combined to obtain various preferred embodiments of the present disclosure.

All reagents and raw materials used in the present disclosure are commercially available.

The positive and progressive effects of the present disclosure are as follows: The present disclosure provides a class of LPAR1 antagonist compounds of formula (I), which exhibit good LPAR1 antagonistic activity and selectivity, as well as superior pharmacokinetic and pharmacodynamic properties, demonstrating promising potential for drug development and applicability in the prevention or treatment of diseases or conditions associated with LPAR1.

### DETAILED DESCRIPTION OF THE PREFERRED EMBODIMENT

The following will provide a more detailed explanation of the general formula compounds of the present disclosure, their preparation methods and uses in conjunction with specific examples. The following examples are provided merely for purposes of illustration and explanation of the present disclosure and should not be construed as limiting the scope of protection of the present disclosure. All technologies realized based on the above contents of the present disclosure are encompassed within the scope intended to be protected by the present disclosure.

Unless otherwise specified, the raw materials and reagents used in the following examples are commercially available products or can be prepared by known methods.

### Method summary:

The chiral purity of the compounds of the present disclosure was tested using the following HPLC method:
HPLC Method A: CHIRALPAK CHIRALPAK AD-H, 4.6 × 250 mm 5 µm, 30% isopropanol/*n-*hexane, 0.8 mL/min, 30°C.

### Nouns and representative reagents

Unless otherwise specified, the nouns used in the following specific experimental descriptions represent the following reagents:
TMS: tetramethylsilane; DPPA: diphenylphosphoryl azide; DBU: 1,8-diazabicyclo[5.4.0]undec-7-ene; DHP: 3,4-dihydro-2*H*-pyran; DIPEA: *N,N*-diisopropylethylamine; DCM: dichloromethane; THF: tetrahydrofuran; T₃P: propylphosphonic anhydride; Et₃N or TEA: triethylamine; TMSN₃: azidotrimethylsilane; AgNTf₂: silver bis(trifluoromethanesulfonyl)imide; TsNHNH₂: 4-toluenesulfonylhydrazide; MeI: iodomethane; KI: potassium iodide; CuI: copper(I) iodide; NaI: sodium iodide; NaH: sodium hydride; CsF: cesium fluoride; TBHP: *tert*-butyl hydroperoxide; *n*-BuLi: *n*-butyllithium; BF₃-Et₂O: boron trifluoride diethyl etherate; PPTS: pyridinium *p*-toluenesulfonate; MeONa: sodium methoxide; AcONa: sodium acetate; MeOH: methanol; MeCN: acetonitrile; AcOH: acetic acid; PPh₃: triphenylphosphine; BnBr: benzyl bromide; DMSO: dimethyl sulfoxide; Pd(PPh₃)₂Cl₂: bis(triphenylphosphine)palladium(II) chloride; Ru(PPh₃)₂(CP)₂Cl: pentamethylcyclopentadienylbis(triphenylphosphine)ruthenium(II) chloride; DAST: diethylaminosulfur trifluoride; NHBn₂: dibenzylamine; HATU: 2-(7-azabenzotriazol-1-yl)-*N,N,N',N*'-tetramethyluronium hexafluorophosphate; PIDA: iodobenzene diacetate; TEMPO: 2,2,6,6-tetramethylpiperidine 1-oxyl; TMSCHN₂: trimethylsilyldiazomethane; TMSCH₂N₃: trimethylsilylmethyl azide; TBAF: tetrabutylammonium fluoride; TBAI: tetrabutylammonium iodide; (HCHO)ₙ: paraformaldehyde; (COCl)₂: oxalyl chloride; MsCl: methanesulfonyl chloride; TMSCN: trimethylsilyl cyanide; NaBH₄: sodium borohydride; MeMgBr: methylmagnesium bromide; DEA: diethanolamine; TBDMSCl: *tert-*butyldimethylsilyl chloride; NaHCO₃: sodium bicarbonate; K₂CO₃: potassium carbonate; Py: pyridine; BzCl: benzoyl chloride; DMP: 1,1,1-triacetoxy-1,1-dihydro-1,2-benziodoxol-3(1*H*)-one; BAST: bis(2-methoxyethyl)aminosulfur trifluoride; LAH: lithium aluminum hydride; DMAP: 4-dimethylaminopyridine; BnOH: benzyl alcohol; EDCl: 1-(3-dimethylaminopropyl)-3-ethylcarbodiimide hydrochloride; EtMgBr: ethylmagnesium bromide; BH₃-Me₂S: borane-dimethyl sulfide complex; Et₂Zn: diethylzinc; TFA: trifluoroacetic acid; MeI₂: diiodomethane; CbzCl: benzyl chloroformate; IPA: isopropanol; EtOH: ethyl acetate; TBDPSCl: *tert*-butyldiphenylsilyl chloride.

### Preparation examples

### Preparation Example 1: Preparation of methyl 2-(1-((trimethylsilyl)ethynyl)cyclopropyl)acetate (M001) and methyl 2,2-difluoro-2-(1-((trimethylsilyl)ethynyl)cyclopropyl)acetate (M001')

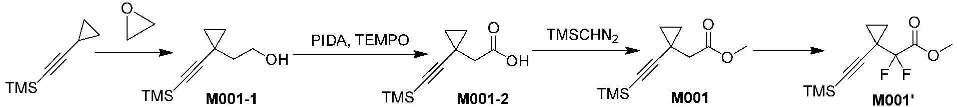

(1) At room temperature, (cyclopropylethynyl)trimethylsilane (20 g, 140 mmol) was dissolved in diethyl ether (250 mL). The reaction mixture was cooled to -78°C, and *n*-butyllithium solution (58 mL, 145 mmol, 2.5 M in *n*-hexane) was added dropwise. After the dropwise addition was completed, the reaction mixture was stirred at -78°C for 1 hour and then warmed to room temperature and stirred for 16 hours. The reaction mixture was then cooled to -78°C, and ethylene oxide (47 mL, 180 mmol, 4 M in tetrahydrofuran) was added dropwise. The reaction mixture was stirred at -78°C for 0.5 hours, then warmed to room temperature, and stirred for an additional 1 hour. The reaction mixture was poured into saturated ammonium chloride solution (200 mL) to quench and extracted with diethyl ether (50 mL). The combined organic phases were washed with saturated brine (200 mL), dried over anhydrous sodium sulfate, filtered, and the filtrate was concentrated under reduced pressure. The resulting residue was purified by silica gel column chromatography (petroleum ether/ethyl acetate = 10/1) to obtain compound M001-1 (10.6 g).
(2) At room temperature, compound M001-1 (12.7 g, 69.6 mmol) was dissolved in acetonitrile (130 mL) and water (130 mL). After cooling the reaction mixture to 0°C, PIDA (56.1 g, 174.1 mmol) and 2,2,6,6-tetramethylpiperidine 1-oxyl (2.2 g, 13.9 mmol) were slowly added in portions. The reaction mixture was heated to 40°C and stirred for an additional 4 hours. The resulting reaction mixture was poured into an aqueous sodium sulfite solution (200 mL) to quench, followed by extraction with ethyl acetate (100 mL), and the organic phases were combined. The resulting organic phase was sequentially washed with brine (100 mL), dried over anhydrous sodium sulfate, filtered, and the filtrate was concentrated under reduced pressure. The resulting residue was purified by silica gel column chromatography (petroleum ether/ethyl acetate = 5/1) to obtain compound M001-2 (5.6 g).
(3) At room temperature, compound M001-2 (5.6 g, 28.5 mmol) was dissolved in methanol (50 mL) and dichloromethane (100 mL). Under a nitrogen atmosphere, the reaction mixture was cooled to 0°C, and a solution of trimethylsilyldiazomethane (28 mL, 57.0 mmol, 2 M in *n*-hexane) was added dropwise. After the dropwise addition was completed, the reaction mixture was stirred at this temperature for an additional 0.5 hours. The reaction mixture was then warmed to room temperature and stirred for an additional 1 hour. The resulting reaction mixture was diluted with water (200 mL), extracted with dichloromethane (100 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 5/1) to obtain compound M001 (5.1 g). LC-MS [M+H]⁺=211.10.
   ¹H NMR (400 MHz, *CD₃OD):* δ 3.71 (s, 3H), 2.38 (s, 2H), 1.05 - 1.02 (m, 2H), 0.80 - 0.77 (m, 2H), 0.11 (s, 9H).
(4) At room temperature, compound M001 (1 g, 4.75 mmol) was dissolved in anhydrous tetrahydrofuran (20 mL). Under a nitrogen atmosphere, the reaction mixture was cooled to -78°C (dry ice-ethyl acetate bath), and then lithium bis(trimethylsilyl)amide (5.7 mL, 5.7 mmol, 1 M in tetrahydrofuran) was added dropwise. The reaction mixture was stirred at this temperature for 0.5 hours, followed by the dropwise addition of a solution of *N*-fluorodibenzenesulfonimide (1647.65 mg, 5.23 mmol) in tetrahydrofuran (10 mL). The reaction mixture was warmed to room temperature and then stirred for an additional 1 hour. The reaction mixture was then cooled again to -78°C (dry ice-ethyl acetate bath), and lithium bis(trimethylsilyl)amide (5.7 mL, 5.7 mmol, 1 M in tetrahydrofuran) was added dropwise. The reaction mixture was stirred at this temperature for 0.5 hours, followed by the dropwise addition of a solution of *N*-fluorodibenzenesulfonimide (1647.65 mg, 5.23 mmol) in tetrahydrofuran (10 mL). The reaction mixture was warmed to room temperature and then stirred for 16 hours. The reaction mixture was poured into saturated ammonium chloride solution (40 mL) to quench and extracted with ethyl acetate (30 mL × 2). The organic phase was dried over anhydrous sodium sulfate, filtered, and the filtrate was concentrated under reduced pressure. The residue was purified by silica gel column chromatography (petroleum ether/ethyl acetate = 20/1) to obtain compound M001' (320 mg, crude). LC-MS: [M+H]+ =247.09.

### Preparation Example 2: Preparation of 3-bromo-2-methyl-6-(1-methyl-5-(((tetrahydro-2H-pyran-2-yl)oxy)methyl)-1H-1,2,3-triazol-4-yl)pyridine (M003)

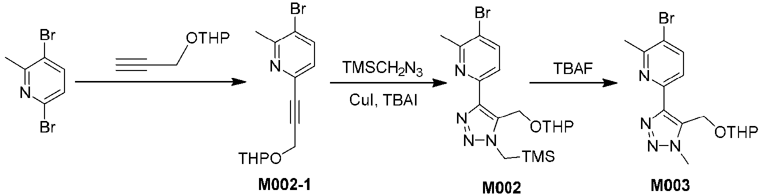

(1) At room temperature, 3,6-dibromo-2-methylpyridine (50 g, 199.27 mmol) and 2-(prop-2-yn-1-yloxy)tetrahydro-2*H*-pyran (41.9 g, 298.9 mmol) were dissolved in acetonitrile (500 mL). Bis(triphenylphosphine)palladium(II) chloride (7.0 g, 9.96 mmol), copper(I) iodide (2.0 g, 9.96 mmol), and triethylamine (60.0 g, 597.8 mmol) were added thereto. The reaction was heated to 50°C and stirred under a nitrogen atmosphere for 1.5 hours. The resulting reaction mixture was filtered through diatomite, and the filtrate was concentrated under reduced pressure. The residue was purified by silica gel column chromatography (petroleum ether/ethyl acetate = 5/1) to obtain compound M002-1 (60.0 g). LC-MS: [M+H]⁺ =311.95.
(2) At room temperature, compound M002-1 (60.0 g, 0.19 mol) and trimethylsilylmethyl azide (50 g, 0.38 mol) were dissolved in anhydrous tetrahydrofuran (600 mL), followed by the addition of pentamethylcyclopentadienylbis(triphenylphosphine)ruthenium(II) chloride (15 g, 0.019 mol), tetrabutylammonium iodide (7.0 g, 0.019 mol), and copper(I) iodide (3.6 g, 0.019 mol). Under a nitrogen atmosphere, the reaction mixture was heated to 40°C and stirred for 16 hours. After cooling to room temperature, the resulting reaction mixture was directly concentrated under reduced pressure. The resulting residue was purified by silica gel column chromatography (petroleum ether/ethyl acetate = 5/1) to obtain compound M002 (68.0 g). LC-MS: [M+H]⁺ =439.00.
(3) At room temperature, compound M002 (68.0 g, 155.21 mmol) was dissolved in tetrahydrofuran (600 mL), and tetrabutylammonium fluoride (48.7 g, 186.26 mmol) was added in portions. The reaction mixture was stirred at room temperature for 1 hour. The resulting reaction mixture was directly concentrated under reduced pressure. The residue was diluted with water (120 mL) and extracted with ethyl acetate (60 mL). The combined organic phases were sequentially washed with saturated brine (150 mL), dried over anhydrous sodium sulfate, and filtered. The resulting filtrate was concentrated under reduced pressure. The residue was purified by silica gel column chromatography (petroleum ether/ethyl acetate = 2/1) to obtain compound M003 (42.2 g). LC-MS: [M+H]⁺ =367.00.

### Preparation Example 3: Preparation of 4-(5-iodo-6-methylpyridin-2-yl)-1-methyl-1H-1,2,3-triazol-5-yl)methanol (M004) and 4-(5-iodo-6-methylpyridin-2-yl)-1-methyl-1H-1,2,3-triazole-5-carboxylic acid (M005)

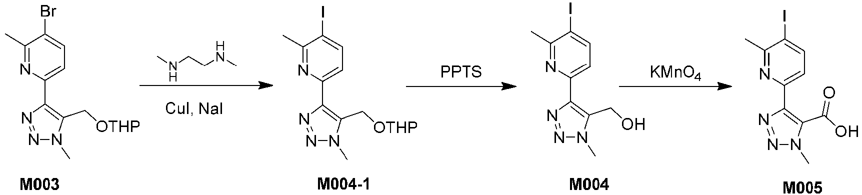

(1) At room temperature, M003 (5 g, 13.6 mmol) and *N,N'*-dimethylethylenediamine (720 mg, 8.2 mmol) were dissolved in 1,4-dioxane (50 mL), followed by the addition of sodium iodide (10.3 g, 68.7 mmol) and copper(I) iodide (260 mg, 1.4 mmol). Under a nitrogen atmosphere, the reaction mixture was heated to 100°C and stirred for an additional 24 hours. After cooling to room temperature, the resulting reaction mixture was concentrated under reduced pressure. The resulting residue was purified by silica gel column chromatography (petroleum ether/ethyl acetate = 5/1) to obtain compound M004-1 (5.5 g). LC-MS: [M+H]⁺ =415.00.
(2) At room temperature, compound M004-1 (5.5 g, 13.3 mmol) was dissolved in methanol (60 mL), and pyridinium *p*-toluenesulfonate (3.3 g, 13.3 mmol) was added. The reaction mixture was heated to 60°C and stirred for an additional 16 hours. The resulting reaction mixture was cooled to room temperature and concentrated under reduced pressure. The resulting residue was purified by silica gel column chromatography (petroleum ether/ethyl acetate = 2/1) to obtain compound M004 (4.5 g). LC-MS: [M+H]⁺ =330.95.
(3) At room temperature, compound M004 (4.5 g, 13.6 mmol) was dissolved in acetonitrile (50 mL) and water (35 mL), and potassium permanganate (4.3 g, 27.2 mmol) was added. The reaction mixture was stirred at room temperature for 16 hours. An aqueous sodium hydroxide solution (2 M, 50 mL) was added to the resulting reaction mixture, and the mixture was filtered under reduced pressure. The filtrate was extracted with ethyl acetate (50 mL). The combined aqueous phases were adjusted to pH = 3 with dilute hydrochloric acid (1 M), filtered under reduced pressure, and the resulting filter cake was washed with pure water (30 mL) and dried under reduced pressure to obtain compound M005 (2.8 g). LC-MS: [M+H]⁺ =344.95.

### Preparation Example 4: Preparation of methyl 2-(1-ethynylcyclopropyl)acetate (M006)

(1) At room temperature, 5-oxaspiro[2.4]heptan-6-one (2.24 g, 20 mmol) was dissolved in methanol (20 mL), and the reaction mixture was cooled to 0°C. A solution of sodium methoxide in methanol (4.8 mL, 5 M) was added, and the reaction mixture was stirred at 0°C for 15 minutes, followed by the addition of ammonium chloride (2.0 g, 37.4 mmol). The reaction mixture was stirred at room temperature for an additional 30 minutes. The resulting reaction mixture was directly concentrated under reduced pressure. The residue was diluted with water (30 mL) and extracted with dichloromethane (30 mL). The organic phases were combined. The resulting organic phase was dried over anhydrous sodium sulfate, filtered, and the filtrate was concentrated under reduced pressure to obtain compound M006-1 (2.88 g, crude).
   ¹H NMR (400 MHz, *CDCl₃*): δ 3.66 (s, 3H), 3.46 (s, 2H), 2.73 (s, 1H), 2.41(s, 2H), 0.55 - 0.52 (m, 2H), 0.49 - 0.42 (m, 2H).
(2) At room temperature, oxalyl chloride (5.08 g, 40 mmol) was dissolved in dichloromethane (30 mL), and the reaction mixture was cooled to -78°C. A solution of dimethyl sulfoxide (6.24 g, 80 mmol) in dichloromethane (10 mL) was then added dropwise to the reaction mixture, maintaining the internal temperature did not exceed -70°C. After the dropwise addition was completed, the reaction mixture was stirred at -78°C for 0.5 hours. Then, a solution of M006-1 (2.88 g, 20 mmol) in dichloromethane (10 mL) was slowly added dropwise to the reaction mixture, maintaining the internal temperature did not exceed - 70°C. The reaction mixture was stirred at -78°C for an additional 0.5 hours. At this point, triethylamine (16.16 g, 160 mmol) was added dropwise, maintaining the internal temperature did not exceed -65°C. After the dropwise addition was completed, the reaction mixture was stirred at -78°C for 0.5 hours, then warmed to room temperature, and stirred for an additional 0.5 hours. The resulting reaction mixture was poured into dichloromethane (200 mL) and washed with water (200 mL). The resulting organic phase was sequentially washed with saturated brine (200 mL), dried over anhydrous sodium sulfate, and filtered. The resulting filtrate was concentrated under reduced pressure to obtain compound M006-2 (2.4 g, crude).
   ¹H NMR (400 MHz, *CDCl₃*): δ 8.66 (s, 1H), 3.64 (s, 3H), 3.46 (s, 2H), 1.29 - 1.25 (m, 2H), 1.07 - 1.03 (m, 2H).
(3) At room temperature, M006-2 (2.4 g, 16.9 mmol) was dissolved in methanol (90 mL). After cooling the reaction mixture to 0°C, potassium carbonate (4.7 g, 33.8 mmol) and dimethyl (1-diazo-2-oxopropyl)phosphonate (4.9 g, 25.4 mmol) were added. The reaction mixture was warmed to room temperature and stirred for 3 hours. The resulting reaction mixture was directly concentrated under reduced pressure, and the residue was purified by silica gel column chromatography (petroleum ether/ethyl acetate = 20/1) to obtain compound M006 (1.3 g).

¹H NMR (400 MHz, *CDCl₃*): δ 3.70 (s, 3H), 2.37 (s, 2H), 1.87 (s, 1H), 1.04 - 1.01 (m, 2H), 0.77 - 0.74 (m, 2H).

### Preparation Example 5: Preparation of methyl 2-(1-ethynylcyclobutyl)acetate (M007)

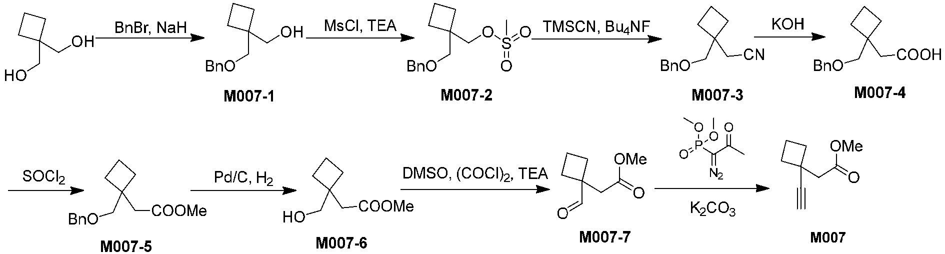

(1) At room temperature, 1,1-cyclobutanedimethanol (4.0 g, 34.44 mmol) was dissolved in anhydrous tetrahydrofuran (20 mL) and *N,N*-dimethylformamide (20 mL). The reaction mixture was cooled to 0°C, and sodium hydride (1.4 g, 34.44 mmol, 60% dispersed in mineral oil) was added in portions. The reaction mixture was warmed to room temperature and stirred for 2 hours, then benzyl bromide (3.5 g, 34.44 mmol) was added. The reaction mixture was stirred at room temperature for an additional 1 hour. The resulting reaction mixture was poured into saturated aqueous ammonium chloride solution (50 mL) to quench and extracted with ethyl acetate (50 mL). The organic phases were combined. The resulting organic phase was dried over anhydrous sodium sulfate, filtered, and the filtrate was concentrated under reduced pressure. The resulting residue was purified by silica gel column chromatography (ethyl acetate/petroleum ether = 5/1) to obtain compound M007-1 (5.3 g). LC-MS [M+H]⁺=207.15.
(2) At room temperature, compound M007-1 (5.3 g, 25.69 mmol) was dissolved in dichloromethane (60 mL), and the reaction mixture was cooled to 0°C, followed by the addition of triethylamine (4.3 mL, 30.83 mmol) and methanesulfonyl chloride (2.2 mL, 28.26 mmol). The reaction mixture was stirred at 0°C for 2 hours. The resulting reaction mixture was poured into water (50 mL) to quench and extracted with dichloromethane (50 mL). The organic phases were combined. The resulting organic phase was dried over anhydrous sodium sulfate, filtered, and the filtrate was concentrated under reduced pressure to obtain compound M007-2 (7.3 g, crude).
(3) At room temperature, compound M007-2 (7.3 g, 25.67 mmol) was dissolved in *N,N-*dimethylformamide (80 mL). Trimethylsilyl cyanide (5.1 g, 51.34 mmol) and tetrabutylammonium fluoride (13.4 g, 51.34 mmol) were added. The reaction was heated to 130°C and stirred for 2 hours. The resulting reaction mixture was diluted with water (100 mL) and extracted with ethyl acetate (100 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 10/1) to obtain compound M007-3 (4.7 g). LC-MS [M+H]⁺=216.00.
(4) At room temperature, compound M007-3 (2.7 g, 12.54 mmol) was dissolved in a mixed solvent of ethanol (30 mL) and water (30 mL), followed by the addition of potassium hydroxide (2.8 g, 50.16 mmol). The reaction mixture was heated to 100°C and stirred for an additional 7 hours. After cooling to room temperature, the resulting reaction mixture was directly concentrated under reduced pressure to remove ethanol. The resulting residue was washed with dichloromethane (30 mL), and the aqueous phases were combined. The resulting aqueous phase was adjusted to pH = 3 to 4 with dilute hydrochloric acid (1 M), then extracted with ethyl acetate (30 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 1/1) to obtain compound M007-4 (1.9 g). LC-MS [M+H]⁺=235.10.
(5) At room temperature, compound M007-4 (3.4 g, 14.51 mmol) was dissolved in methanol (100 mL) and cooled to 0°C. Thionyl chloride (5.3 mL, 72.56 mmol) was added dropwise. After the dropwise addition was completed, the reaction mixture was heated to 80°C and stirred for 1 hour, then cooled to room temperature and concentrated under reduced pressure. The resulting residue was purified by silica gel column chromatography (petroleum ether/ethyl acetate = 3/1) to obtain compound M007-5 (3.4 g). LC-MS [M+H]⁺=249.15.
(6) At room temperature, compound M007-5 (3.4 g, 13.69 mmol) was dissolved in methanol (100 mL), and wet palladium on carbon (500 mg, 10%) was added. Under a hydrogen atmosphere, the reaction mixture was stirred at room temperature for 3 hours. The resulting reaction mixture was filtered through diatomite, and the filtrate was concentrated under reduced pressure. The resulting residue was purified by silica gel column chromatography (petroleum ether/ethyl acetate = 1/1) to obtain compound M007-6 (2.0 g). LC-MS [M+H]⁺=139.16.
(7) At room temperature, oxalyl chloride (0.2 mL, 2.53 mmol) was dissolved in anhydrous dichloromethane (10 mL), and the reaction mixture was cooled to -78°C. Dimethyl sulfoxide (0.4 mL, 5.06 mmol) was slowly added dropwise, maintaining the internal temperature below -70°C. After the dropwise addition was completed, the reaction mixture was stirred at -78°C for 0.5 hours. Then, a solution of compound M007-6 (200 mg, 1.26 mmol) in dichloromethane (4 mL) was slowly added dropwise, maintaining the internal temperature below -70°C. After the dropwise addition was completed, the reaction mixture was stirred at -78°C for an additional 0.5 hours. Triethylamine (1.4 mL, 10.11 mmol) was slowly added dropwise to quench the reaction, while maintaining the internal temperature below -70°C. After the dropwise addition was completed, the reaction mixture was stirred at -70°C for an additional 0.5 hours. The resulting reaction mixture was poured into water (20 mL), extracted with dichloromethane (10 mL), and the organic phases were combined. The resulting organic phase was dried over anhydrous sodium sulfate, filtered, and the filtrate was concentrated under reduced pressure to obtain compound M007-7 (190 mg, crude).
(8) At room temperature, compound M007-7 (190 mg, 1.22 mmol) was dissolved in methanol (5 mL). The reaction mixture was cooled to 0°C, followed by the addition of dimethyl (1-diazo-2-oxopropyl)phosphonate (351 mg, 1.82 mmol) and potassium carbonate (420 mg, 3.04 mmol). The reaction mixture was warmed to room temperature and stirred for an additional 16 hours. The resulting reaction mixture was poured into water (10 mL), extracted with dichloromethane (10 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 20/1) to obtain compound M007 (100 mg). LC-MS: [M+H]⁺ =153.15.

### Preparation Example 6: Preparation of (1-ethynylcyclopropyl)methanol (M008)

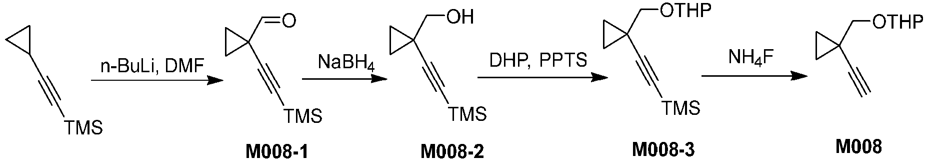

(1) At room temperature, (cyclopropylethynyl)trimethylsilane (5.0 g, 36.16 mmol) was dissolved in anhydrous tetrahydrofuran (50 mL). The reaction mixture was purged with nitrogen and cooled to -78°C under a nitrogen atmosphere, followed by the dropwise addition of *n*-butyllithium (21.7 mL, 54.23 mmol, 2.5 M). After the dropwise addition was completed, the reaction mixture was warmed to room temperature and stirred for 16 hours. The reaction mixture was then cooled to 0°C, and *N,N*-dimethylformamide (5.3 g, 72.31 mmol) was added dropwise at this temperature. After the dropwise addition was completed, the reaction mixture was warmed to room temperature and stirred for 2 hours. The resulting reaction mixture was poured into saturated aqueous ammonium chloride solution (50 mL) to quench and extracted with ethyl acetate (50 mL). The organic phases were combined. The resulting organic phase was dried over anhydrous sodium sulfate, filtered, and the filtrate was concentrated under reduced pressure. The resulting residue was purified by silica gel column chromatography (pure petroleum ether) to obtain compound M008-1 (4.6 g, crude).
(2) At room temperature, compound M008-1 (4.6 g, 27.66 mmol) was dissolved in methanol (50 mL), and the reaction mixture was cooled to 0°C, and sodium borohydride (2.1 g, 55.32 mmol) was added in portions. The reaction mixture was stirred at 0°C for 1 hour. The resulting reaction mixture was poured into water (50 mL), extracted with dichloromethane (50 mL), and the organic phases were combined. The resulting organic phase was dried over anhydrous sodium sulfate, filtered, and the filtrate was concentrated under reduced pressure to obtain compound M008-2 (3.8 g).
(3) At room temperature, compound M008-2 (1.8 g, 10.69 mmol) was dissolved in dichloromethane (30 mL). Pyridinium *p*-toluenesulfonate (269 mg, 1.07 mmol) and 3,4-dihydro-2*H*-pyran (1.2 g, 13.90 mmol) were added. The reaction mixture was stirred at room temperature for 16 hours. The resulting reaction mixture was diluted with water (50 mL), extracted with dichloromethane (50 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 10/1) to obtain compound M008-3 (2.6 g). LC-MS [M+H]⁺=253.42.
(4) At room temperature, compound M008-3 (2.6 g, 10.31 mmol) was dissolved in a mixed solvent of methanol (30 mL) and water (10 mL), followed by the addition of ammonium fluoride (1.1 g, 30.93 mmol). The reaction mixture was heated to 50°C and stirred for an additional 16 hours. The resulting reaction mixture was cooled to room temperature, diluted with water (100 mL), and extracted with dichloromethane (50 mL). The combined organic phases were dried over anhydrous sodium sulfate, filtered, and the filtrate was concentrated under reduced pressure. The resulting residue was purified by silica gel column chromatography (petroleum ether/ethyl acetate = 10/1) to obtain compound M008 (1.6 g). LC-MS [M+H]⁺=181.24.

### Preparation Example 7: Preparation of (4-(5-bromopyridin-2-yl)-1-methyl-1H-1,2,3-triazol-5-yl)methanol (M009) and 4-(5-bromopyridin-2-yl)-1-methyl-1H-1,2,3-triazole-5-carboxylic acid (M009')

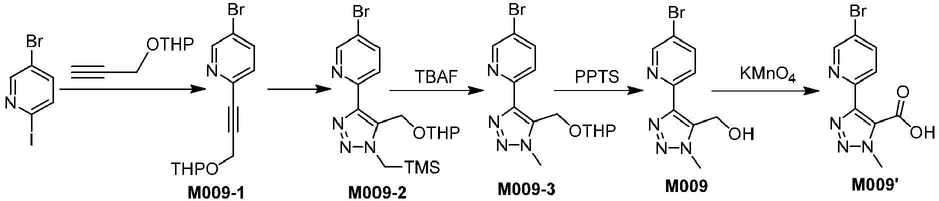

The preparation of compounds M009 and M009' was referred to the methods for preparing compounds M004 and M005 in Preparation Example 2 and Preparation Example 3, wherein the starting material was replaced from 3,6-dibromo-2-methylpyridine to 5-bromo-2-iodopyridine, and compounds M009 and M009' were obtained. LC-MS of compound M009: [M+H]⁺ = 270.95, LC-MS of compound M009': [M+H]⁺ = 284.93. The detailed preparation steps are as follows:
(1) At room temperature, 5-bromo-2-iodopyridine (10.5 g, 37.1 mmol) and 2-(2-propynyloxy)tetrahydropyran (5.98 g, 42.66 mmol) were dissolved in acetonitrile (100 mL), followed by the addition of bis(triphenylphosphine)palladium(II) chloride (2.6 g, 3.71 mmol), copper(I) iodide (703 mg, 3.70 mmol), and triethylamine (11.2 g, 111.3 mmol). Under a nitrogen atmosphere, the reaction mixture was heated to 35°C and stirred for 2 hours. The reaction mixture was filtered through diatomite, and the filtrate was concentrated under reduced pressure. The residue was purified by silica gel column chromatography (petroleum ether/ethyl acetate = 5/1) to obtain compound M009-1 (11.2 g). LC-MS: [M+H]⁺ =297.95.
(2) At room temperature, compound M009-1 (11.2 g, 38.2 mmol) and trimethylsilylmethyl azide (9.8 g, 76.4 mmol) were dissolved in anhydrous tetrahydrofuran (100 mL), followed by the addition of pentamethylcyclopentadienylbis(triphenylphosphine)ruthenium(II) chloride (3.04 g, 3.82 mmol), tetrabutylammonium iodide (1.4 g, 3.82 mmol), and copper(I) iodide (725 mg, 3.82 mmol). Under a nitrogen atmosphere, the reaction was heated to 40°C and stirred for 16 hours. The reaction mixture was cooled to room temperature and then filtered through diatomite. The filtrate was diluted with water (200 mL) and extracted with ethyl acetate (125 mL × 3). The combined organic phases were washed with saturated brine (200 mL × 2). The organic phase was dried over anhydrous sodium sulfate, filtered, and the filtrate was concentrated under reduced pressure. The residue was purified by silica gel column chromatography (petroleum ether/ethyl acetate = 5/1) to obtain compound M009-2 (10.0 g). LC-MS: [M+H]⁺ =427.05.
(3) At room temperature, compound M009-2 (10.0 g, 23.5 mmol) was dissolved in tetrahydrofuran (100 mL), and tetrabutylammonium fluoride (9.20 g, 35.2 mmol) was added in portions. The reaction mixture was stirred at room temperature for 1 hour. The reaction mixture was concentrated under reduced pressure, and the residue was diluted with water (120 mL) and extracted with ethyl acetate (60 mL × 3). The combined organic phases were washed with saturated brine (150 mL × 2). The organic phase was dried over anhydrous sodium sulfate and filtered. The filtrate was concentrated under reduced pressure. The residue was purified by silica gel column chromatography (petroleum ether/ethyl acetate = 3/1) to obtain compound M009-3 (6.8 g). LC-MS: [M+H]⁺ =353.00.
(4) At room temperature, compound M009-3 (6.8 g, 19.31 mmol) was dissolved in methanol (70 mL), and pyridinium *p*-toluenesulfonate (4.84 g, 19.31 mmol) was added. The reaction was heated to 60°C and stirred for 1 hour. After cooling to room temperature, the reaction mixture was directly concentrated under reduced pressure. The residue was diluted with water (60 mL) and extracted with ethyl acetate (40 mL × 2). The combined organic phases were washed with saturated aqueous sodium bicarbonate solution (60 mL) and saturated brine (60 mL). The organic phase was dried over anhydrous sodium sulfate, filtered, and the filtrate was concentrated under reduced pressure. The residue was purified by silica gel column chromatography (petroleum ether/ethyl acetate = 2/1) to obtain compound M009 (4.3 g). LC-MS: [M+H]⁺ =270.95.
(5) At room temperature, compound M009 (2.2 g, 8.18 mmol) was dissolved in acetonitrile (15 mL) and water (15 mL), followed by the addition of potassium permanganate (2.5 g, 16.36 mmol). The reaction mixture was stirred at room temperature for 16 hours. Aqueous sodium hydroxide solution (10 mL, 2 M) was added to the reaction mixture to adjust the pH to 10 to 11, followed by the addition of sodium bisulfite solution (1 M, 20 mL) and stirring for 10 minutes. The mixture was filtered, and the filter cake was washed with water (30 mL). The filtrate was adjusted to pH = 5-6 with dilute hydrochloric acid (1 M), filtered, and the filter cake was washed with water (20 mL), ethanol (20 mL), and then petroleum ether (20 mL). The filter cake was concentrated under reduced pressure to obtain compound M009' (2.0 g). LC-MS: [M+H]+ = 284.93.

Referring to the method of Preparation Example 7, compounds 1 and 2 in the following table were prepared by replacing the starting material 5-bromo-2-iodopyridine with the corresponding reactants listed in the following table:

| Compound 1 | | | Compound 2 | | | Reactant |
|---|---|---|---|---|---|---|
| No. | Structure | LC-MS: [M+H]⁺ | No. | Structure | LC-MS: [M+H]⁺ | |
| 100-5 | 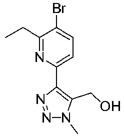 | 298.95 | 229-1 | 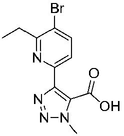 | 310.95 | 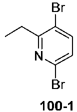 Commercially available, or prepared by common methods in the art |
| 105-5 | 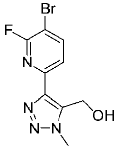 | 286.90 | 237-1 | 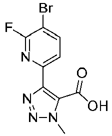 | 300.90 | 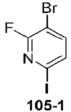 Commercially available, or prepared by referring to the preparation method of compound 209-1 |
| 218-9 | 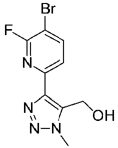 | 308.97 | 258-1 | 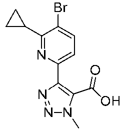 | 322.90 | 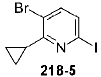 Prepared according to the method in Example 56 |
| 515-5 | 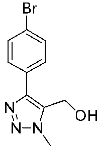 | 268.9 | 515-6 | 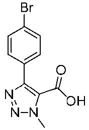 | 282.9 | 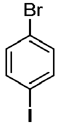 |

### Preparation Example 8: Preparation of methyl 2-(1-((tert-butyldimethylsilyl)ethynyl)cyclopropyl)acetate (M010)

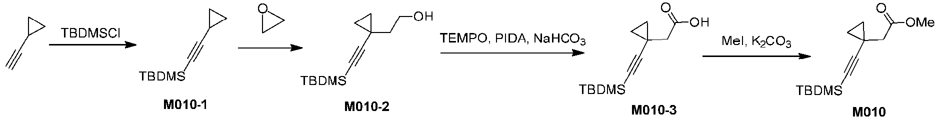

(1) At room temperature, cyclopropylethyne (60 g, 907.7 mmol) was dissolved in anhydrous tetrahydrofuran (240 mL). The reaction mixture was cooled to -78°C (dry ice-ethyl acetate bath), followed by the dropwise addition of n-butyllithium (399.4 mL, 998.5 mmol, 2.5 M in n-hexane). After the dropwise addition was completed, the reaction mixture was stirred at -78°C for 1 hour. Then, a solution of *tert-*butyldimethylchlorosilane (136.81 g, 907.7 mmol) in tetrahydrofuran (300 mL) was added dropwise. The reaction mixture was stirred at -78°C for 0.5 hours, then naturally warmed to room temperature, and stirred for an additional 1 hour. After cooling the reaction mixture to 0°C, saturated ammonium chloride solution (480 mL) and water (120 mL) were added to quench the reaction. The organic phase was separated, and the aqueous phase was extracted with ethyl acetate (300 mL × 2). The combined organic phases were washed with saturated brine (300 mL), dried over anhydrous sodium sulfate, filtered, and the filtrate was concentrated under reduced pressure. The residue was purified by silica gel column chromatography (petroleum ether/ethyl acetate = 1/0) to obtain compound M010-1 (308 g), LC-MS: [M+H]⁺ =181.36.
(2) At room temperature, M010-1 (150 g, 831.7 mmol) was dissolved in methyl *tert*-butyl ether (1500 mL). The reaction mixture was cooled to -78°C (dry ice-ethyl acetate bath), followed by the dropwise addition of *n*-butyllithium (366 mL, 914.8 mmol, 2.5 M in *n*-hexane). After the dropwise addition was completed, the reaction mixture was stirred at -78°C for 1 hour. Subsequently, the reaction mixture was naturally warmed to room temperature and stirred for an additional 3 hours. The reaction mixture was then cooled again to 0°C, and a solution of ethylene oxide (360.4 mL, 1.08 mol, 3 M in diethyl ether) was added dropwise. The reaction mixture was stirred at this temperature for 1 hour. Saturated ammonium chloride solution (1200 mL) and water (300 mL) were added to the reaction mixture to quench the reaction. The organic phase was separated, and the aqueous phase was extracted twice with ethyl acetate (750 mL × 2). All organic phases were combined, washed with brine (500 mL), dried over anhydrous sodium sulfate, filtered, and the filtrate was concentrated under reduced pressure. The residue was purified by silica gel column chromatography (petroleum ether/ethyl acetate = 10/1) to obtain compound M010-2 (104 g), LC-MS: [M+H]⁺ =225.1.
(3) At room temperature, M010-2 (84.0 g, 374.3 mmol) was dissolved in acetonitrile (840 mL) and water (840 mL), followed by the addition of sodium bicarbonate (94.34 g, 1122.9 mmol) and 2,2,6,6-tetramethylpiperidine 1-oxyl (11.7 g, 74.9 mmol). Iodosobenzene diacetate (301.4 g, 935.8 mmol) was then added in portions. The reaction mixture was stirred at room temperature for 2 hours. The reaction was quenched by adding saturated sodium sulfite solution (420 mL) to the reaction mixture. The aqueous phase was extracted with ethyl acetate (600 mL × 3), and the combined organic phases were washed with saturated brine (500 mL), dried over anhydrous sodium sulfate, filtered, and the filtrate was concentrated under reduced pressure. The residue was purified by silica gel column chromatography (petroleum ether/ethyl acetate = 10/1) to obtain compound M010-3 (62 g), LC-MS: [M+H]⁺ =239.40.
(4) At room temperature, M010-3 (67 g, 281.1 mmol) was dissolved in *N,N*-dimethylformamide (420 mL), followed by the addition of iodomethane (48.3 g, 340.1 mmol). The reaction mixture was cooled to 0 to 5°C, and powdered potassium carbonate (42.7 g, 309.0 mmol) was added. The reaction mixture was stirred at this temperature for 0.5 hours, then warmed to room temperature, and stirred for an additional 2 hours. The reaction mixture was poured into water (1.1 L) and extracted twice with ethyl acetate (670 mL × 2). The combined organic phases were washed twice with an aqueous solution (670 mL × 2) and then washed with saturated brine (670 mL). The organic phase was dried over anhydrous sodium sulfate, filtered, and the filtrate was concentrated under reduced pressure. The residue was purified by silica gel column chromatography (petroleum ether: ethyl acetate = 1:40) to obtain compound M010 (129 g). LC-MS: [M+H]⁺ = 253.42.

### Preparation Example 9: Preparation of (R)-4,4-difluoropentan-2-ol (M011)

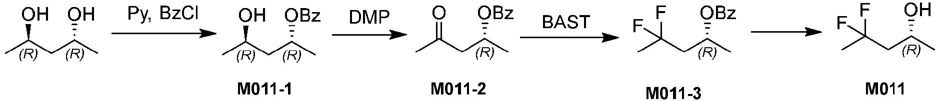

(1) At room temperature, (2R,4R)-2,4-pentanediol (10 g, 95.4 mmol) was dissolved in dichloromethane (300 mL), and pyridine (15.1 g, 190.8 mmol) was added. The reaction mixture was cooled to 0°C, and benzoyl chloride (12.7 g, 90.6 mmol) was slowly added dropwise. The reaction mixture was stirred at 0°C for 0.5 hours, then warmed to room temperature, and stirred for an additional 2 hours. The reaction mixture was poured into dilute hydrochloric acid (200 mL, 1 M) to quench and extracted twice with dichloromethane (100 mL × 2). The combined organic phases were washed with saturated brine (200 mL), dried over anhydrous sodium sulfate, filtered, and the filtrate was concentrated under reduced pressure. The residue was purified by silica gel column chromatography (petroleum ether/ethyl acetate = 5/1) to obtain compound M011-1 (13.8 g). LC-MS: [M+H]⁺= 209.10.
(2) At room temperature, M011-1 (6.8 g, 32.7 mmol) was dissolved in dichloromethane (140 mL), and sodium bicarbonate (13.7 g, 163 mmol) was added. The reaction mixture was cooled to 0°C, followed by the addition of 1,1,1-triacetoxy-1,1-dihydro-1,2-benziodoxol-3(1*H*)-one (15.3 g, 36.0 mmol). After warming to room temperature, the reaction mixture was stirred for an additional 3 hours. The reaction mixture was diluted with dichloromethane (30 mL), filtered under reduced pressure, and the filter cake was washed with dichloromethane (60 mL). The filtrate was washed with saturated aqueous sodium bicarbonate solution (100 mL) and saturated brine (100 mL), dried over anhydrous sodium sulfate, filtered, and the filtrate was concentrated under reduced pressure. The residue was purified by silica gel column chromatography (petroleum ether/ethyl acetate = 5/1) to obtain compound M011-2 (5.98 g). LC-MS: [M+H]⁺ =207.10.
(3) At room temperature, M011-2 (5.9 g, 28.6 mmol) was dissolved in bis(2-methoxyethyl)aminosulfur trifluoride (32 g, 143.2 mmol), and the reaction mixture was heated to 50°C and stirred for an additional 24 hours. After cooling to room temperature, the reaction mixture was slowly added dropwise to cold aqueous sodium bicarbonate solution (100 mL) to quench. The mixture was extracted with ethyl acetate (50 mL × 3). The combined organic phases were washed with saturated aqueous ammonium chloride solution (100 mL), then washed with saturated brine (100 mL), dried over anhydrous sodium sulfate, filtered, and the filtrate was concentrated under reduced pressure. The residue was purified by silica gel column chromatography (petroleum ether/ethyl acetate = 15/1) to obtain compound M011-3 (4.6 g). LC-MS: [M+H]⁺ =229.10.
(4) At room temperature, M011-3 (4.0 g, 17.53 mmol) was dissolved in tetrahydrofuran (30 mL) and methanol (30 mL), followed by the addition of a solution of lithium hydroxide monohydrate (1.84 g, 43.83 mmol) in water (10 mL). The reaction mixture was stirred at room temperature for 1 hour. After adding water (15 mL) to the reaction mixture, methanol and tetrahydrofuran were directly removed by concentration under reduced pressure. The aqueous phase was extracted with ethyl acetate (50 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 5:1) to obtain compound M011 (3.4 g, crude). LC-MS: [M+H]⁺ = 125.04.

### Preparation Example 10: Preparation of methyl trans-2-ethynylcyclopentane-1-carboxylate (M012)

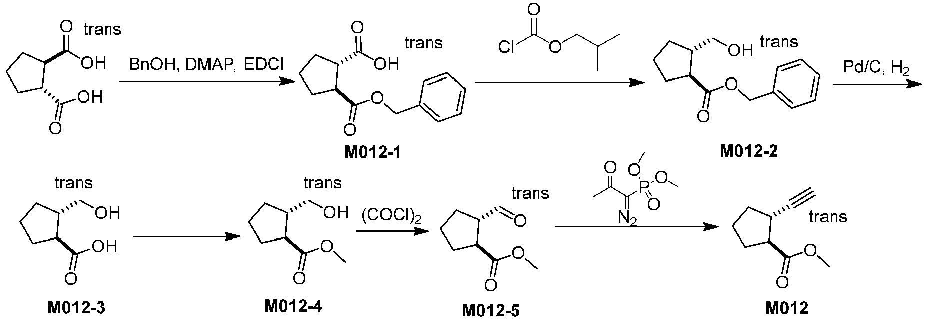

(1) At room temperature, *trans*-(15,25)-cyclopentane-1,2-dicarboxylic acid (3.7 g, 23.4 mmol, containing a pair of trans configurations) was dissolved in dichloromethane (60 mL), and the reaction mixture was cooled to 0°C. Then, 4-dimethylaminopyridine (1.42 g, 11.7 mmol) and benzyl alcohol (2.5 g, 23.4 mmol) were added, and 1-(3-dimethylaminopropyl)-3-ethylcarbodiimide hydrochloride (4.5 g, 23.4 mmol) was added in portions. The reaction mixture was stirred at 0°C for 2 hours. The reaction mixture was diluted with water (60 mL) and extracted twice with dichloromethane (60 mL × 2). The combined organic phases were dried over anhydrous sodium sulfate, filtered, and the filtrate was concentrated under reduced pressure. The residue was purified by silica gel column chromatography (petroleum ether/ethyl acetate = 20/1 to 10/1) to obtain compound M012-1 (3.2 g, containing a pair of trans configurations). LC-MS: [M+H]⁺ = 249.10.
(2) At room temperature, M012-1 (2.7 g, 11.6 mmol) was dissolved in anhydrous tetrahydrofuran (10 mL), and the reaction mixture was cooled to 0°C (ice bath). Then, triethylamine (2.34 g, 23.2 mmol) and isobutyl chloroformate (1.9 g, 13.9 mmol) were added. The reaction mixture was stirred at this temperature for 1 hour. Then, a suspension of sodium borohydride (1.3 g, 34.8 mmol) in water (10 mL) was slowly added. After warming to room temperature, the reaction mixture was stirred for an additional 0.5 hours. The reaction mixture was diluted with water (30 mL), adjusted to pH = 4 with dilute hydrochloric acid (1 M), and then extracted with ethyl acetate (30 mL × 3). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 15/1) to obtain compound M012-2 (1.7 g, containing a pair of trans configurations). LC-MS: [M+H]⁺ =235.10.
(3) At room temperature, M012-2 (700 mg, 2.99 mmol) was dissolved in methanol (15 mL), and after nitrogen purging, wet palladium on carbon (70 mg, 10%) was added. Under a hydrogen atmosphere, the reaction mixture was stirred at room temperature for 3 hours. The reaction mixture was filtered through diatomite, and the filter cake was washed with methanol (30 mL). The filtrate was directly concentrated under reduced pressure to obtain compound M012-3 (600 mg, crude, containing a pair of trans configurations). LCMS: [M+H]⁺ = 144.17.
(4) At room temperature, M012-3 (600 mg, crude) was dissolved in dichloromethane (6 mL) and methanol (3 mL). Under a nitrogen atmosphere, the reaction mixture was cooled to 0°C (ice bath), and trimethylsilyldiazomethane (2.0 mL, 5.0 mmol) was slowly added dropwise. The reaction was stirred at this temperature for an additional 1 hour. The reaction mixture was diluted with water (10 mL) and extracted twice with dichloromethane (10 mL × 2). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 5/1) to obtain compound M012-4 (360 mg, containing a pair of trans configurations). LC-MS: [M+H]⁺ = 159.15.
(5) At room temperature, oxalyl chloride (546 mg, 4.3 mmol) was dissolved in dichloromethane, and the reaction mixture was cooled to -78°C (dry ice-ethyl acetate bath). A solution of dimethyl sulfoxide (672 mg, 8.6 mmol) in dichloromethane (5 mL) was slowly added dropwise. The internal temperature of the reaction mixture was maintained at -70°C or less. After the dropwise addition was completed, the reaction mixture was stirred at this temperature for an additional 0.5 hours. Then, a solution of M012-4 (340 mg, 2.15 mmol) in dichloromethane (5 mL) was added dropwise, and the reaction mixture was stirred at this temperature for an additional 0.5 hours. Finally, triethylamine (1.74 g, 17.2 mmol) was slowly added dropwise, maintaining the internal temperature did not exceed -65°C. After the dropwise addition was completed, the reaction mixture was gradually warmed to room temperature and stirred for an additional 0.5 hours. The reaction mixture was quenched with water (20 mL) and extracted with dichloromethane (10 mL × 3). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 10/1) to obtain compound M012-5 (230 mg, containing a pair of trans configurations). LC-MS: [M+H]⁺ =156.18.
(6) At room temperature, potassium carbonate was added to methanol (2.5 mL). Under a nitrogen atmosphere, the reaction mixture was cooled to 0°C, and dimethyl (1-diazo-2-oxopropyl)phosphonate (135 mg, 0.71 mmol) was added. The reaction mixture was stirred at this temperature for 10 minutes, followed by the dropwise addition of a solution of M012-5 (130 mg, 0.64 mmol) in methanol (1.5 mL). After warming to room temperature, the reaction mixture was stirred for an additional 2 hours. Petroleum ether (10 mL) and water (10 mL) were added to the reaction mixture, and the organic layer was separated. The aqueous phase was extracted with petroleum ether (10 mL). The combined organic phases were washed with saturated brine (10 mL), dried over anhydrous sodium sulfate, filtered, and the filtrate was concentrated under reduced pressure at low temperature (about 15°C) to obtain compound M012 (80 mg, containing a pair of trans configurations). LC-MS: [M+H]⁺ = 152.19.

### Preparation Example 11: Preparation of methyl trans-2-ethynyl-4,4-difluorocyclopentane-1-carboxylate (M013)

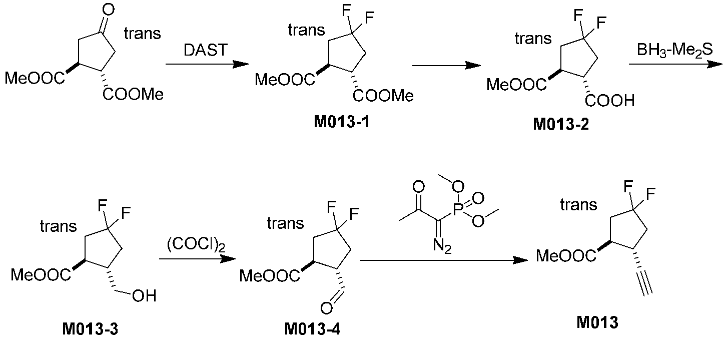

(1) At room temperature, dimethyl *trans*-(1*S*,2*S*)-oxocyclopentyl-1,2-dicarboxylate (5 g, 25.0 mmol) was dissolved in dichloromethane (100 mL), and diethylaminosulfur trifluoride (8.1 g, 50.0 mmol) was added. The reaction mixture was heated to 40°C and stirred for an additional 18 hours. After cooling to room temperature, the reaction mixture was poured into ice water (100 mL) and extracted with dichloromethane (50 mL × 2). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 5/1) to obtain compound M013-1 (4.8 g, containing a pair of trans configurations). LC-MS: [M+H]⁺ =223.19.
(2) At room temperature, M013-1 (4.8 g, 21.6 mmol) was dissolved in methanol (50 mL) and water (10 mL), followed by the addition of sodium hydroxide (0.86 g, 21.6 mmol). The reaction mixture was stirred at room temperature for 18 hours. The reaction mixture was diluted with water (50 mL) and extracted with ethyl acetate (50 mL × 2). The aqueous phase was cooled to 0°C, adjusted to pH = 3 to 4 with dilute hydrochloric acid (1 M), and then extracted with ethyl acetate (50 mL × 3). The combined organic phases were dried over anhydrous sodium sulfate, filtered, and the filtrate was concentrated under reduced pressure to obtain compound M013-2 (3.3 g, containing a pair of trans configurations).
   LC-MS: [M+H]⁺ =209.16.
(3) At room temperature, M013-2 (3.3 g, 15.9 mmol) was dissolved in anhydrous tetrahydrofuran (30 mL). Under a nitrogen atmosphere, the reaction mixture was cooled to 0°C, and borane-dimethyl sulfide complex (3.2 mL, 31.8 mmol, 10 M) was added dropwise. The reaction mixture was stirred at this temperature for an additional 2 hours. The reaction mixture was quenched with water (50 mL) and extracted twice with ethyl acetate (30 mL × 2). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 3/1) to obtain compound M013-3 (2.2 g, containing a pair of trans configurations). LC-MS: [M+H]⁺ =195.18.
(4) At room temperature, dimethyl sulfoxide (1.8 mL, 24.1 mmol) was dissolved in anhydrous dichloromethane (20 mL). Under a nitrogen atmosphere, the reaction mixture was cooled to -78°C (dry ice-ethyl acetate bath), and a solution of oxalyl chloride (1.7 mL, 20.1 mmol) in dichloromethane (2 mL) was slowly added dropwise, maintaining the internal temperature below -70°C. At this temperature, the reaction was stirred for an additional 0.5 hours, followed by the dropwise addition of a solution of M013-3 (2.6 g, 13.4 mmol) in dichloromethane (5 mL), maintaining the internal temperature below -70°C. The reaction mixture was stirred at this temperature for an additional 1 hour. Finally, triethylamine (5.7 mL, 33.5 mmol) was slowly added dropwise, maintaining the internal temperature at -65°C. After the dropwise addition was completed, the reaction mixture was slowly warmed to room temperature and stirred for an additional 1 hour. The reaction mixture was diluted with water (50 mL) and extracted with dichloromethane (50 mL × 2). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 5/1) to obtain compound M013-4 (1.2 g, containing a pair of trans configurations). LC-MS: [M+H]⁺ =193.16.
(5) At room temperature, M013-4 (1.4 g, 7.3 mmol) was dissolved in methanol (20 mL), and the reaction mixture was cooled to 0°C. Potassium carbonate (1.5 g, 10.9 mmol) was added, followed by the slow addition of dimethyl (1-diazo-2-oxopropyl)phosphonate (3.5 g, 18.2 mmol). After warming to room temperature, the reaction mixture was stirred for an additional 2 hours. The reaction mixture was slowly added with water (30 mL) and extracted with ethyl acetate (50 mL). The organic phase was dried over anhydrous sodium sulfate, filtered, and the filtrate was concentrated under reduced pressure. The residue was purified by silica gel column chromatography (petroleum ether/ethyl acetate = 10/1) to obtain compound M013 (0.6 g, containing a pair of trans configurations). LC-MS: [M+H]⁺ =189.17

### Preparation Example 12: Preparation of methyl trans-2-ethynylcyclopropane-1-carboxylate (compound M014)

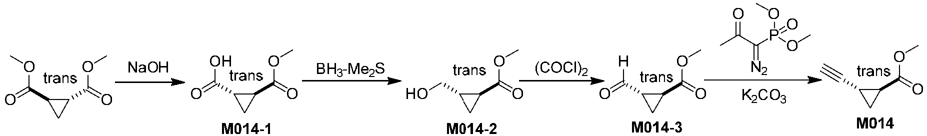

The preparation of compound M014 was referred to the method for preparing M013 in Preparation Example 11, wherein compound M013-1 was replaced with dimethyl *trans*-1,2-cyclopropanedicarboxylate, and compound M014 (1.5 g, containing a pair of trans isomers) was obtained.

LC-MS: [M+H]⁺ = 125.05.

### Preparation Example 13: Preparation of methyl 2-(1-ethynyl-3-methoxycyclobutyl)acetate (compound M015)

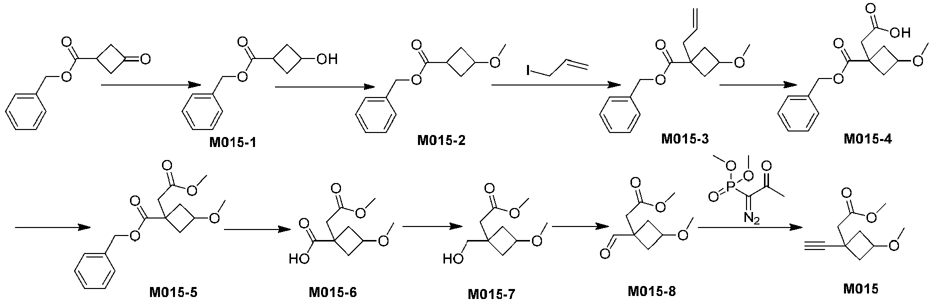

(1) At room temperature, benzyl 3-oxocyclobutanecarboxylate (25 g, 122.42 mmol) was dissolved in anhydrous tetrahydrofuran (250 mL), and the reaction mixture was cooled to 0°C, followed by the slow addition of a solution of sodium borohydride (5.56 g, 146.90 mmol) in water (60 mL). The reaction mixture was stirred at room temperature for 0.5 hours. The reaction mixture was quenched with water (200 mL) and extracted with ethyl acetate (200 mL × 2). The combined organic phases were dried over anhydrous sodium sulfate, filtered, and the filtrate was concentrated under reduced pressure to obtain compound M015-1 (22 g). LC-MS [M+H]⁺=207.05.
(2) At room temperature, compound M015-1 (18 g, 87.3 mmol) was dissolved in *N,N-*dimethylformamide (200 mL), followed by the addition of silver oxide (40.5 g, 174.6 mmol) and iodomethane (105.3 g, 741.9 mmol). The reaction mixture was heated to 45°C and stirred for 16 hours. After cooling to room temperature, the reaction mixture was quenched with saturated ammonium chloride solution (200 mL), filtered, and the filtrate was extracted twice with ethyl acetate (200 mL × 2). The combined organic phases were washed with saturated brine (100 mL × 3). The organic phase was dried over anhydrous sodium sulfate, filtered, and the filtrate was concentrated under reduced pressure. The residue was purified by silica gel column chromatography (petroleum ether/ethyl acetate = 10/1) to obtain compound M015-2 (15 g). LC-MS [M+H]⁺=221.05.
(3) At room temperature, compound M015-2 (15.0 g, 68.1 mmol) was dissolved in anhydrous tetrahydrofuran (500 mL). Under a nitrogen atmosphere, the reaction mixture was cooled to -78°C, and lithium bis(trimethylsilyl)amide (17.1 g, 102.2 mmol, 1 M in tetrahydrofuran) was added dropwise. The reaction mixture was stirred at this temperature for 1 hour, followed by the dropwise addition of allyl iodide (14.9 g, 88.5 mmol). The reaction mixture was warmed to room temperature and stirred for an additional 3 hours. The reaction mixture was poured into saturated ammonium chloride solution (300 mL) to quench and extracted twice with ethyl acetate (300 mL × 2). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M015-3 (7.3 g, containing cis/trans isomers). LC-MS [M+H]⁺=261.10.
(4) At room temperature, compound M015-3 (7.3 g, 28.1 mmol) was dissolved in acetonitrile (100 mL) and water (100 mL), followed by the addition of sodium periodate (24 g, 112.2 mmol) and ruthenium trichloride (174.5 mg, 84.1 mmol). The reaction mixture was stirred at room temperature for 2 hours. Potassium permanganate (13.3 g, 84.1 mmol) was added to the reaction mixture, and the reaction mixture was stirred at room temperature for an additional 2 hours. The reaction mixture was filtered, and the filtrate was adjusted to pH = 9 to 10 with 10% sodium hydroxide solution, diluted with water (200 mL), and extracted with ethyl acetate (200 mL). The resulting aqueous phase was adjusted to pH = 3 to 4 with dilute hydrochloric acid (1 M) and then extracted with ethyl acetate (200 mL × 3). The combined organic phases were concentrated under reduced pressure. The residue was purified by silica gel column chromatography (petroleum ether/ethyl acetate = 1/1) to obtain compound M015-4 (5.2 g, containing cis/trans isomers). LC-MS [M+H]⁺=279.05.
(5) At room temperature, compound M015-4 (5.2 g, 18.68 mmol) was dissolved in methanol (100 mL), and thionyl chloride (6.67 g, 56.04 mmol) was added slowly. The reaction mixture was refluxed (65°C) for 2 hours. After cooling to room temperature, the reaction mixture was directly concentrated under reduced pressure. The residue was purified by silica gel column chromatography (petroleum ether/ethyl acetate = 5/1) to obtain compound M015-5 (4.4 g, containing cis/trans isomers). LC-MS [M+H]⁺=293.05.
(6) At room temperature, compound M015-5 (4 g, 13.7 mmol) was dissolved in methanol (40 mL), followed by the addition of wet palladium on carbon (0.73 g, 0.68 mmol, 10wt.%) and wet palladium hydroxide on carbon (0.48 g, 0.68 mmol, 20 wt.%). Under a hydrogen atmosphere, the reaction mixture was heated to 30°C and stirred for an additional 18 hours. The reaction mixture was filtered through diatomite, and the filtrate was concentrated under reduced pressure to obtain compound M015-6 (2.7 g, containing cis-trans isomers). LC-MS: [M+H]⁺ =203.21.
(7) At room temperature, compound M015-6 (2.7 g, 13.4 mmol) was dissolved in anhydrous tetrahydrofuran (30 mL). Under a nitrogen atmosphere, the reaction mixture was cooled to 0°C, and borane-dimethyl sulfide complex (2.67 mL, 26.7 mmol, 10 M) was added dropwise. The reaction mixture was warmed to room temperature and stirred for an additional 2 hours. The reaction mixture was quenched with water (40 mL) and extracted with ethyl acetate (40 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound M015-7 (2.3 g, containing cis/trans isomers). LC-MS: [M+H]⁺ =189.22.
(8) At room temperature, oxalyl chloride (3.1 g, 24.5 mmol) was dissolved in anhydrous dichloromethane (50 mL). Under a nitrogen atmosphere, the reaction mixture was cooled to -78°C (dry ice-ethyl acetate bath), and a solution of dimethyl sulfoxide (3.8 g, 48.9 mmol) in dichloromethane (20 mL) was slowly added dropwise, maintaining the internal temperature below -70°C. At this temperature, the reaction mixture was stirred for an additional 0.5 hours, followed by the dropwise addition of a solution of compound M015-7 (2.3 g, 12.2 mmol) in dichloromethane (20 mL), maintaining the internal temperature below -70°C. The reaction mixture was further reacted at -78°C for 1 hour. Finally, triethylamine (9.9 g, 97.8 mmol) was slowly added dropwise, maintaining the internal temperature below -65°C. After the dropwise addition was completed, the reaction mixture was slowly warmed to room temperature and stirred for an additional 1 hour. The reaction mixture was diluted with water (200 mL) and extracted with dichloromethane (100 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound M015-8 (2.0 g, containing cis/trans isomers). LC-MS: [M+H]⁺ =187.21.
(9) At room temperature, compound M015-8 (2 g, 10.8 mmol) was dissolved in methanol (20 mL), and the reaction mixture was cooled to 0°C. Potassium carbonate (2.2 g, 16.1 mmol) was added, followed by the slow addition of dimethyl (1-diazo-2-oxopropyl)phosphonate (5.2 g, 26.9 mmol). After warming to room temperature, the reaction mixture was stirred for an additional 1 hour. Water (20 mL) was slowly added to the reaction mixture, and the mixture was extracted with ethyl acetate (20 mL). The organic phase was dried over anhydrous sodium sulfate, filtered, and the filtrate was concentrated under reduced pressure. The residue was purified by silica gel column chromatography (petroleum ether/ethyl acetate = 5/1) to obtain compound M015 (1.5 g, containing cis/trans isomers). LC-MS: [M+H]⁺ =183.22.

### Preparation Example 14: Preparation of 1-ethynyl-1-((methylsulfonyl)methyl)cyclopropane (compound M016)

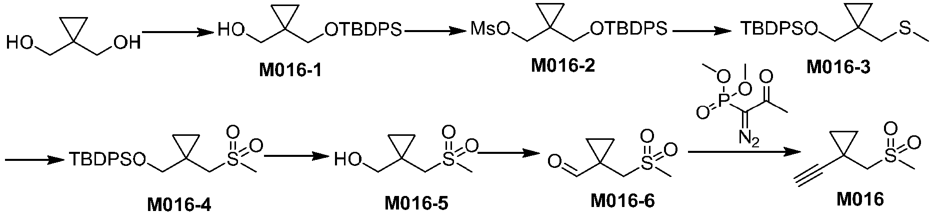

(1) At room temperature, 1,1-cyclopropanedimethanol (5 g, 48.96 mmol) was dissolved in anhydrous tetrahydrofuran (50 mL), and the reaction mixture was cooled to 0°C. Sodium hydride (2.35 g, 97.92 mmol, 60% dispersed in mineral oil) was added in portions. After the addition was completed, the reaction mixture was stirred at 0°C for 0.5 hours, followed by the addition of *tert*-butyldiphenylsilyl chloride (13.46 g, 48.96 mmol). After warming to room temperature, the reaction mixture was stirred for an additional 2 hours. The reaction mixture was quenched with water (20 mL) and extracted twice with ethyl acetate (50 mL × 2). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M016-1 (16 g). LC-MS: [M+H]⁺=341.15.
(2) At room temperature, compound M016-1 (10 g, 29.37 mmol) was dissolved in dichloromethane (100 mL), and the reaction mixture was cooled to 0°C. At this temperature, *N,N*-diisopropylethylamine (7.6 g, 58.74 mmol) and methanesulfonyl chloride (4 g, 35.24 mmol) were added. The reaction mixture was stirred at room temperature for 3 hours. The reaction mixture was quenched with water (50 mL) and extracted with dichloromethane (50 mL × 3). The combined organic phases were dried over anhydrous sodium sulfate, filtered, and the filtrate was concentrated under reduced pressure to obtain compound M016-2 (11 g, crude). LC-MS: [M+H]⁺=419.62.
(3) At room temperature, compound M016-2 (11 g, crude) was dissolved in *N,N-*dimethylformamide (100 mL), and sodium methanethiolate (3.68 g, 52.56 mmol) was added. The reaction mixture was stirred at room temperature for 48 hours. The reaction mixture was diluted with water (50 mL) and extracted with ethyl acetate (50 mL × 3). The organic phases were combined, washed twice with saturated sodium chloride (50 mL × 2), dried over anhydrous sodium sulfate, filtered, and the filtrate was concentrated under reduced pressure. The residue was purified by silica gel column chromatography (petroleum ether/ethyl acetate = 20/1) to obtain compound M016-3 (3.5 g). LC-MS: [M+H]⁺=371.
(4) At room temperature, compound M016-3 (3.5 g, 9.44 mmol) was dissolved in dichloromethane (30 mL), and the reaction mixture was cooled to 0°C, followed by the slow addition of *m-*chloroperoxybenzoic acid (4 g, 23.60 mmol, purity: 85%). After the addition was completed, the reaction mixture was warmed to room temperature and stirred for an additional 2 hours. The reaction mixture was quenched with saturated sodium bicarbonate (20 mL) and extracted with ethyl acetate (30 mL × 3). The organic phases were combined, washed twice with saturated sodium bicarbonate (20 mL × 2), dried over anhydrous sodium sulfate, filtered, and the filtrate was concentrated under reduced pressure. The residue was purified by silica gel column chromatography (petroleum ether/ethyl acetate = 10/1) to obtain compound M016-4 (2.8 g). LC-MS: [M+H]⁺ =403.20.
(5) At room temperature, compound M016-4 (3 g, 7.45 mmol) was dissolved in anhydrous tetrahydrofuran (30 mL), and the reaction mixture was cooled to 0°C, followed by the slow addition of tetrabutylammonium fluoride (2.3 g, 8.94 mmol). After the addition was completed, the reaction mixture was stirred at room temperature for 1 hour. The reaction mixture was diluted with water (30 mL) and extracted with ethyl acetate (50 mL × 3). The organic phases were combined, washed with saturated brine (40 mL), dried over anhydrous sodium sulfate, filtered, and the filtrate was concentrated under reduced pressure. The residue was purified by silica gel column chromatography (ethyl acetate/petroleum ether = 1/0) to obtain compound M016-5 (600 mg). LC-MS: [M+H]⁺ =165.22.
(6) At room temperature, dimethyl sulfoxide (1.04 mL, 14.60 mmol) was added to dichloromethane (10 mL). Under a nitrogen atmosphere, the reaction mixture was cooled to -78°C, and oxalyl chloride (0.62 mL, 7.30 mmol) was slowly added dropwise. The reaction mixture was stirred at -78°C for 0.5 hours. Then, a solution of compound M016-5 (600 mg, 3.65 mmol) in dichloromethane (2 mL) was added. After the dropwise addition was completed, the reaction mixture was stirred at -78°C for an additional 1 hour. Then, triethylamine (4.06 mL, 29.20 mmol) was added dropwise, maintaining the internal temperature below -65°C. After the dropwise addition was completed, the reaction mixture was stirred at -70°C for an additional 0.5 hours, then naturally warmed to room temperature and stirred for an additional 0.5 hours. The reaction mixture was quenched with water (50 mL) and extracted with dichloromethane (50 mL × 3). The organic phases were combined, washed with saturated brine (50 mL), dried over anhydrous sodium sulfate, filtered, and the filtrate was concentrated under reduced pressure. The residue was purified by silica gel column chromatography (petroleum ether/ethyl acetate = 10/1) to obtain compound M016-6 (470 mg). LC-MS: [M+H]⁺ =163.21.
(7) At room temperature, compound M016-6 (200 mg, 1.23 mmol) was dissolved in methanol (5 mL), and the reaction mixture was cooled to 0°C. Potassium carbonate (204 mg, 1.48 mmol) and dimethyl (1-diazo-2-oxopropyl)phosphonate (260 mg, 1.35 mmol) were added slowly. After warming to room temperature, the reaction mixture was stirred for an additional 2 hours. The reaction mixture was quenched with water (10 mL) and extracted with dichloromethane (10 mL × 3). The organic phases were combined, washed with saturated brine (20 mL), dried over anhydrous sodium sulfate, filtered, and the filtrate was concentrated under reduced pressure to obtain compound M016 (190 mg, crude). LC-MS: [M+H]⁺ =159.22.

### Preparation Example 15: Preparation of 5-(5-bromo-6-methylpyridin-2-yl)-3-methylisoxazole-4-carboxylic acid (compound M017)

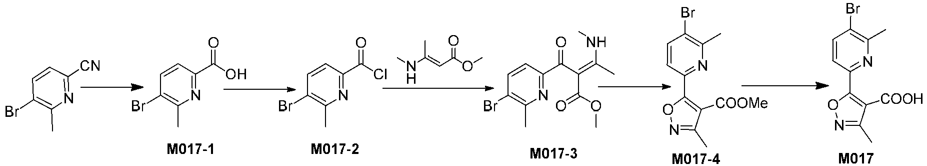

(1) At room temperature, 5-bromo-6-methylpyridine-2-carbonitrile (5.0 g, 25.38 mmol) was dissolved in a mixed solvent of methanol (50 mL) and water (25 mL), and sodium hydroxide (1 g, 25.38 mmol) was added. The reaction mixture was refluxed (85°C) for 5 hours. After cooling to room temperature, the reaction mixture was directly concentrated under reduced pressure. The residue was diluted with water (20 mL), and the pH of the aqueous phase was adjusted to 5 to 6 with concentrated hydrochloric acid (12 M). The mixture was filtered, and the filter cake was washed with water (20 mL). The filter cake was then dissolved in dichloromethane (40 mL). The resulting solution was dried over anhydrous sodium sulfate, filtered, and the filtrate was concentrated under reduced pressure to obtain compound M017-1 (3.0 g). LC-MS: [M+H]⁺ = 215.90.
(2) At room temperature, compound M017-1 (2.5 g, 11.57 mmol) was dissolved in dichloromethane (25 mL), and N,N-dimethylformamide (85 mg, 1.16 mmol) was added. After the reaction mixture was cooled to 0°C, oxalyl chloride (2.9 g, 23.14 mmol) was added dropwise. After the addition was completed, the reaction mixture was warmed to room temperature and stirred for an additional 2 hours. The reaction mixture was directly concentrated to obtain compound M017-2 (2.5 g, crude). LC-MS: [M+H]⁺ = 234.10.
(3) At room temperature, methyl 3-(methylamino)-2-butenoate (4.1 g, 31.98 mmol) was dissolved in anhydrous tetrahydrofuran (30 mL), and pyridine (2.5 g, 31.98 mmol) was added. The reaction mixture was cooled to 0°C, and then compound M017-2 (2.5 g, 10.66 mmol) was added. The reaction mixture was stirred at room temperature for 48 hours. The reaction mixture was diluted with water (10 mL) and extracted with ethyl acetate (20 mL × 3). The organic phases were combined, washed with dilute hydrochloric acid (20 mL, 1 M), dried over anhydrous sodium sulfate, filtered, and the filtrate was concentrated under reduced pressure. The residue was purified by silica gel column chromatography (petroleum ether/ethyl acetate = 2/1) to obtain compound M017-3 (2.5 g, yield: 66.2%). LC-MS: [M+H]⁺ = 328.95.
(4) At room temperature, compound M017-3 (2.5 g, 7.64 mmol) was dispersed in acetic acid (20 mL), and then hydroxylamine hydrochloride (530 mg, 7.64 mmol) was added. The reaction mixture was heated to 80°C and stirred for 2 hours. The reaction mixture was cooled to room temperature, diluted with ethyl acetate (30 mL), and washed with water (20 mL × 3). The organic phase was washed with saturated sodium bicarbonate solution (30 mL), dried over anhydrous sodium sulfate, filtered, and the filtrate was concentrated under reduced pressure to obtain compound M017-4 (1.7 g, crude). LC-MS: [M+H]⁺ = 312.90.
(5) At room temperature, compound M017-4 (1.7 g, crude) was dissolved in a mixed solvent of methanol (20 mL) and water (10 mL), followed by the addition of lithium hydroxide monohydrate (687 mg, 16.38 mmol). The reaction mixture was stirred at room temperature for 2 hours. The reaction mixture was diluted with a small amount of water (10 mL) and then concentrated under reduced pressure. The residue was extracted twice with dichloromethane (10 mL × 2), and the resulting aqueous phase was adjusted to pH = 5 to 6 with dilute hydrochloric acid (1 M), filtered, and the filter cake was washed with water (10 mL). The filter cake was concentrated under reduced pressure to obtain compound M017 (1.2 g). LC-MS: [M+H]⁺ =298.90.

### Preparation Example 16: Preparation of 4-(5-bromo-4-methylpyrimidin-2-yl)-1-methyl-1H-pyrazole-5-carboxylic acid (compound M018)

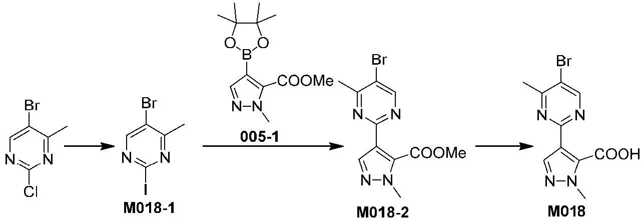

(1) At room temperature, 5-bromo-2-chloro-4-methylpyrimidine (5 g, 24.10 mmol) and sodium iodide (6.14 g, 40.97 mmol) were dissolved in chloroform (100 mL). The reaction mixture was cooled to 0°C, and after adding hydroiodic acid (4.9 g, 21.69 mmol), the reaction mixture was warmed to room temperature and stirred for 16 hours. The reaction mixture was poured into saturated aqueous sodium bicarbonate solution (100 mL) to quench and extracted with dichloromethane (100 mL × 3). The combined organic phases were dried over anhydrous sodium sulfate. After filtration, the filtrate was concentrated under reduced pressure, and the residue was purified by silica gel column chromatography (petroleum ether/ethyl acetate = 5/1) to obtain compound M018-1 (3.3 g). LC-MS: [M+H]⁺ = 298.75.
(2) At room temperature, compound 005-1 (2.4 g, 9.13 mmol) was dissolved in 1,4-dioxane (80 mL) and water (40 mL), followed by the addition of compound M018-1 (3.23 g, 10.96 mmol), [1,1'-bis(diphenylphosphino)ferrocene]dichloropalladium(II) (666 mg, 0.91 mmol), and sodium carbonate (2.9 g, 27.39 mmol). The reaction mixture was heated to 80°C under a nitrogen atmosphere and stirred for an additional 1 hour. After cooling to room temperature, the reaction mixture was poured into water (50 mL) and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound M018-2 (1.3 g). LC-MS: [M+H]⁺ = 312.90.
(3) At room temperature, compound M018-2 (1.3 g, 4.18 mmol) was dissolved in methanol (20 mL) and water (10 mL), followed by the addition of lithium hydroxide monohydrate (0.5 g, 12.54 mmol). The reaction mixture was stirred at room temperature for 1 hour. The reaction mixture was diluted with water (20 mL) and directly concentrated under reduced pressure to remove methanol. The residue was adjusted to pH = 3 to 4 with dilute hydrochloric acid (1 M). The mixture was filtered under reduced pressure, and the filter cake was dried under reduced pressure to obtain compound M018 (700 mg). LCMS: [M+H]⁺ =298.90.

### Preparation Example 17: Preparation of 5-(5-bromo-6-methylpyridin-2-yl)-3-methylisothiazole-4-carboxylic acid (compound M019)

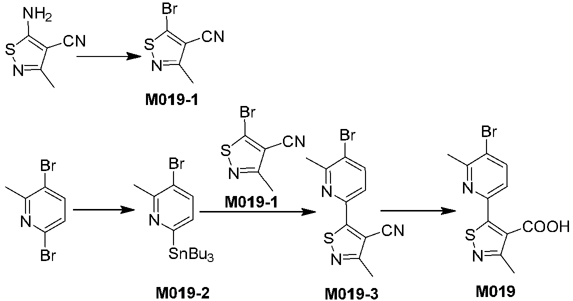

(1) At room temperature, 3-methyl-4-cyano-5-aminoisothiazole (2.0 g, 14.37 mmol) was dissolved in hydrobromic acid solution (46wt.%, 10 mL). The reaction mixture was cooled to 0°C, and a solution of sodium nitrite (1.19 g, 17.24 mmol) in water (10 mL) was added. The reaction mixture was stirred at 0°C for 0.5 hours. Then, a solution of cuprous bromide (3.09 g, 21.55 mmol) in hydrobromic acid (10 mL) was added. The reaction mixture was warmed to room temperature and stirred for 2 hours. The reaction mixture was diluted with water (20 mL), and the pH of the solution was adjusted to 8 to 9 with sodium carbonate solution. Ethyl acetate (30 mL) was then added, and the mixture was stirred to separate layers. The organic phase was separated, and the aqueous phase was extracted with ethyl acetate (50 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 10/1) to obtain compound M019-1 (1.6 g). LC-MS: [M+H]⁺ =302.90.
(2) At room temperature, 2,5-dibromo-6-methylpyridine (2.00 g, 8.00 mmol) was dissolved in toluene (100 mL), and the reaction mixture was cooled to -70°C (dry ice-ethyl acetate bath). *n*-Butyllithium (3.84 mL, 9.61 mmol, 2.5 M in *n*-hexane) was added dropwise to the reaction. After the dropwise addition was completed, the reaction mixture was stirred at -70°C for 2 hours. Then, tributyltin chloride (3.39 g, 10.41 mmol) was added dropwise. The reaction mixture was stirred at a temperature below -70°C for an additional 1 hour. The reaction mixture was warmed to approximately -10°C, and quenched with saturated aqueous ammonium chloride solution (50 mL), and then extracted twice with ethyl acetate (50 mL × 2). The combined organic phases were dried over anhydrous sodium sulfate, filtered, and the filtrate was concentrated under reduced pressure. The residue was purified by silica gel column chromatography (petroleum ether/ethyl acetate = 1/0) to obtain compound M019-2 (1.5 g). LC-MS: [M+H]⁺ =461.00.
(3) At room temperature, compound M019-2 (1.25 g, 2.71 mmol) and compound M019-1 (0.55 g, 2.71 mmol) were dissolved in xylene (12 mL), and bis(triphenylphosphine)palladium(II) chloride (0.19 g, 0.27 mmol) and triphenylphosphine (0.14 g, 0.54 mmol) were added. Under a nitrogen atmosphere, the reaction mixture was heated to 120°C and stirred for 20 hours. After cooling to room temperature, the reaction mixture was concentrated under reduced pressure. The residue was purified by silica gel column chromatography (petroleum ether/ethyl acetate = 10/1) to obtain compound M019-3 (440 mg). LC-MS: [M+H]⁺ =294.00.
(4) At room temperature, compound M019-3 (390 mg, 1.33 mmol) was dissolved in concentrated sulfuric acid (5 mL), and the reaction mixture was heated to 120°C and stirred for 1 hour. After the reaction mixture was cooled to room temperature, a solution of sodium nitrite (275.31 mg, 3.99 mmol) in water (2.5 mL) was added. The reaction mixture was heated to 50°C and stirred for 2 hours. The reaction mixture was cooled to room temperature, then slowly added to ice water (20 mL) dropwise to quench, and washed with ethyl acetate (20 mL × 3). The organic phases were combined, washed with water (30 mL) and saturated brine (30 mL), dried over anhydrous sodium sulfate, filtered, and the filtrate was concentrated under reduced pressure to obtain compound M019 (410 mg). LC-MS: [M+H]⁺ = 312.85.

### Preparation Example 18: Preparation of 1-(bicyclo[1.1.1]pentan-1-yl)-N-methylmethanamine (compound 204-3)

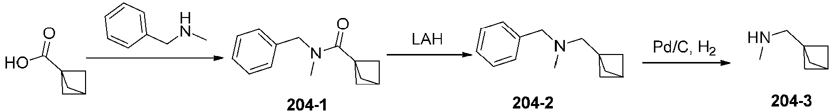

(1) At room temperature, bicyclo[1.1.1]pentane-1-carboxylic acid (0.9 g, 8.0 mmol) and *N-*methylbenzylamine (1.1 g, 9.1 mmol) were dissolved in *N,N*-dimethylformamide (10 mL), followed by the addition of propylphosphonic anhydride (3.8 g, 12.1 mmol, 50% in ethyl acetate) and *N,N-*diisopropylethylamine (4.3 mL, 24.1 mmol). The reaction mixture was stirred at room temperature for 1 hour. The reaction mixture was diluted with water (20 mL) and extracted twice with ethyl acetate (30 mL × 2). The combined organic phases were washed with saturated sodium chloride solution (20 mL × 3). The organic phase was dried over anhydrous sodium sulfate, filtered, and the filtrate was concentrated under reduced pressure. The residue was purified by silica gel column chromatography (ethyl acetate/petroleum ether = 1/8) to obtain compound 204-1 (1.1 g). LC-MS: [M+H]⁺ =216.10.
(2) At room temperature, lithium aluminum hydride (354 mg, 9.2 mmol) was dispersed in anhydrous tetrahydrofuran (20 mL). Under a nitrogen atmosphere, the reaction mixture was cooled to 0°C, and a solution of compound 204-1 (1 g, 4.6 mmol) in tetrahydrofuran (5 mL) was added dropwise. The reaction mixture was heated to 70°C and stirred for an additional 2 hours. The reaction mixture was cooled to 0°C, and then water (2 mL), 15% sodium hydroxide solution (6 mL), tetrahydrofuran (20 mL), water (6 mL), and anhydrous magnesium sulfate (2 g) were sequentially added to the reaction mixture. The reaction mixture was stirred at room temperature for 15 minutes and filtered. The filtrate was separated into layers, and the aqueous phase was extracted with ethyl acetate (20 mL × 2). The combined organic phases were washed with saturated sodium chloride solution (20 mL × 2), dried over anhydrous sodium sulfate, filtered, and the filtrate was concentrated under reduced pressure. The residue was purified by silica gel column chromatography (ethyl acetate/petroleum ether = 1/1) to obtain compound 204-2 (591 mg). LC-MS: [M+H]⁺ =202.15.
(3) At room temperature, compound 204-2 (590 mg, 2.9 mmol) was dissolved in methanol (20 mL), followed by the addition of wet palladium on carbon (60 mg, 10%) and a solution of hydrogen chloride in methanol (3.7 mL, 4 M). Under a hydrogen atmosphere, the reaction mixture was heated to 70°C and stirred for 2 hours. After cooling to room temperature, the reaction mixture was filtered through diatomite, and the filter cake was washed with methanol (10 mL). The filtrate was concentrated under reduced pressure to obtain the crude hydrochloride of compound 204-3 (0.8 g). LC-MS: [M+H]⁺ =112.20.

The preparation of the compounds and their hydrochloride salts in the following table was referred to the preparation method of Preparation Example 18, wherein the starting material was replaced from bicyclo[1.1.1]pentane-1-carboxylic acid to the corresponding starting materials listed in the following table:

| No. | Compound structure | Starting material | LC-MS [M+H]⁺ |
|---|---|---|---|
| 068-3 | 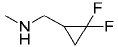 | 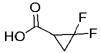 | 122.1 |
| 073-3 | 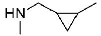 | 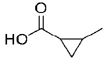 | 100.15 |
| 089-3 | 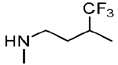 | 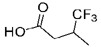 | 156.09 |
| 094-3 | 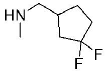 | 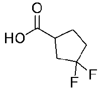 | 150.15 |
| 281-3 | 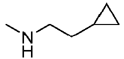 | 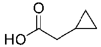 | 100.15 |

### Preparation Example 19: Preparation of 2,2-difluoro-N-methylbutan-1-amine (compound 082-3)

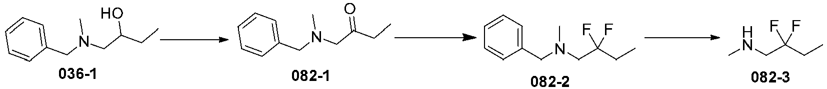

(1) At room temperature, dimethyl sulfoxide (3.7 mL, 51.7 mmol) was dissolved in dichloromethane (50 mL). Under a nitrogen atmosphere, the reaction mixture was cooled to -78°C, and a solution of oxalyl chloride (3.3 mL, 38.8 mmol) in dichloromethane (3 mL) was added dropwise, maintaining the internal temperature below -70°C. After the dropwise addition was completed, the reaction mixture was stirred at -78°C for 0.5 hours. Then, a solution of compound 036-1 (5 g, 25.9 mmol) in dichloromethane (10 mL) was added dropwise. The internal temperature was maintained below -70°C. After the dropwise addition was completed, the reaction mixture was stirred at -78°C for an additional 0.5 hours. Then, triethylamine (10.8 mL, 77.6 mmol) was added dropwise, and the internal temperature was maintained below -65°C. After the dropwise addition was completed, the reaction mixture was warmed to room temperature and stirred for an additional 2 hours. The reaction mixture was diluted with water (20 mL) and extracted with ethyl acetate (30 mL × 2). The combined organic phases were dried over anhydrous sodium sulfate, filtered, and the filtrate was concentrated under reduced pressure. The residue was purified by silica gel column chromatography (ethyl acetate/petroleum ether = 1/10) to obtain compound 082-1 (3.8 g). LC-MS: [M+H]⁺ =192.10.
(2) At room temperature, compound 082-1 (2.8 g, 14.6 mmol) was dissolved in 1,2-dichloroethane (30 mL). Under a nitrogen atmosphere, the reaction mixture was cooled to 0°C, followed by the dropwise addition of bis(2-methoxyethyl)aminosulfur trifluoride (5.4 mL, 29.2 mmol). After the dropwise addition was completed, the reaction mixture was warmed to room temperature and stirred for an additional 18 hours. The reaction mixture was poured into saturated sodium bicarbonate solution (20 mL) and extracted with dichloromethane (30 mL × 2). The combined organic phases were dried over anhydrous sodium sulfate, filtered, and the filtrate was concentrated under reduced pressure. The residue was purified by silica gel column chromatography (ethyl acetate/petroleum ether = 1/10) to obtain compound 082-2 (0.95 g). LC-MS: [M+H]⁺ =214.10.
(3) At room temperature, compound 082-2 (0.95 g, 4.5 mmol) was dissolved in methanol (10 mL), and wet palladium on carbon (100 mg, 10%) was added. Under a hydrogen atmosphere, the reaction mixture was heated to 60°C and stirred for an additional 18 hours. The reaction mixture was filtered through diatomite, and the filter cake was washed with methanol (10 mL). A solution of hydrogen chloride in methanol (10 mL, 4 M) was added to the filtrate, and then the filtrate was concentrated under reduced pressure to obtain the hydrochloride of compound 082-3 (1 g, crude). LC-MS: [M+H]⁺ =124.15.

### Preparation Example 20: Preparation of (2-fluorocyclobutyl)methanamine (compound 098-3)

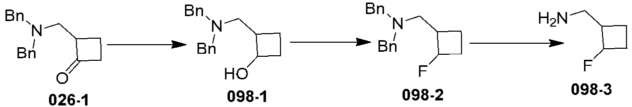

(1) At room temperature, compound 026-1 (1.5 g, 5.54 mmol) was dissolved in methanol (30 mL). The reaction mixture was cooled to 0°C, and sodium borohydride (203 mg, 5.54 mmol) was added. After the addition was completed, the reaction mixture was warmed to room temperature and stirred for an additional 1 hour. The reaction mixture was quenched with aqueous sodium bicarbonate solution (20 mL) and extracted with ethyl acetate (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 5/1) to obtain compound 098-1 (900 mg). LC-MS: [M+H]⁺ = 282.10.
(2) At room temperature, compound 098-1 (800 mg, 2.84 mmol) was dissolved in dichloromethane (10 mL). After the reaction mixture was cooled to 0°C, diethylaminosulfur trifluoride (688 mg, 4.26 mmol) was added dropwise. After the dropwise addition was completed, the reaction mixture was warmed to room temperature and stirred for an additional 2 hours. The reaction mixture was quenched with aqueous sodium bicarbonate solution (20 mL) and extracted with dichloromethane (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 20/1) to obtain compound 098-2 (300 mg). LC-MS: [M+H]⁺ = 284.10.
(3) At room temperature, compound 098-2 (300 mg, 1.06 mmol) was dissolved in methanol (6 mL), followed by the addition of a solution of hydrogen chloride in methanol (4 M, 1.06 mL, 4.23 mmol) and palladium hydroxide on carbon (10%, 300 mg). Under a hydrogen atmosphere (hydrogen balloon), the reaction mixture was heated to 40°C and stirred for 48 hours. After cooling to room temperature, the reaction mixture was directly filtered, and the filtrate was concentrated under reduced pressure to obtain the hydrochloride of compound 098-3 (200 mg, crude). LC-MS: [M+H]⁺ = 104.15.

### Preparation Example 21: Preparation of 1-(3,3-difluorocyclobutyl)ethan-1-ol (compound 029-3)

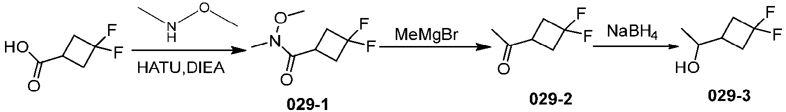

(1) At room temperature, 3,3-difluorocyclobutanecarboxylic acid (4.0 g, 29.39 mmol) was dissolved in dichloromethane (80 mL), followed by the addition of *N,N'*-diisopropylethylamine (11.40 g, 88.17 mmol), dimethylhydroxylamine hydrochloride (3.93 g, 35.27 mmol), and 2-(7-azabenzotriazol-1-yl)-*N,N,N',N'-*tetramethyluronium hexafluorophosphate (16.76 g, 44.09 mmol). The reaction mixture was stirred at room temperature for 2 hours. The resulting reaction mixture was diluted with water (100 mL), extracted with dichloromethane (100 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 029-1 (5.2 g). LC-MS: [M+H]⁺ = 180.10.
(2) At room temperature, compound 029-1 (5.2 g, 29.02 mmol) was dissolved in anhydrous tetrahydrofuran (50 mL). After nitrogen purging, the reaction mixture was cooled to 0°C, and a solution of methylmagnesium bromide in tetrahydrofuran (58 mL, 58.05 mmol, 1 M) was added dropwise to the reaction mixture. The reaction mixture was warmed to room temperature and stirred for an additional 4 hours. The resulting reaction mixture was poured into saturated aqueous ammonium chloride solution (100 mL) to quench and extracted with ethyl acetate (100 mL). The organic phases were combined. The resulting organic phase was dried over anhydrous sodium sulfate, filtered, and the resulting filtrate was distilled to collect the fraction at 82°C to obtain compound 029-2 (4.0 g).
(3) At room temperature, compound 029-2 (4.0 g) was dissolved in ethanol (20 mL), and the reaction mixture was cooled to 0°C, followed by the portionwise addition of sodium borohydride (564 mg, 11.91 mmol). The reaction mixture was warmed to room temperature and stirred for an additional 1 hour. The resulting reaction mixture was diluted with water (20 mL), extracted with dichloromethane (10 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 5/1) to obtain compound 029-3 (440 mg). LC-MS: [M+H]⁺ = 137.14.

The preparation of the compounds in the following table was referred to the preparation method in Preparation Example 21, wherein the starting material was replaced from 3,3-difluorocyclobutanecarboxylic acid to the corresponding starting materials listed in the following table:

| No. | Compound structure | Starting material | LC-MS [M+H]⁺ |
|---|---|---|---|
| 081-3 | 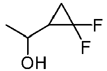 | 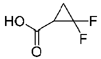 | 123.11 |
| 237-3 | 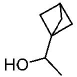 | 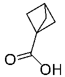 | 113.17 |
| 275-3 | 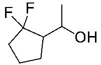 | 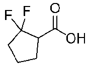 Commercially available, or prepared by methods known in the art | 151.17 |
| 312-3 | 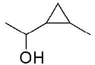 | 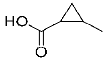 | 101.16 |
| 285-3 | 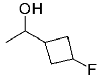 | 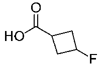 | 119.15 |
| 317-3 | 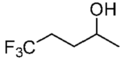 | 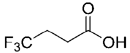 | 143.12 |
| 274-3 | 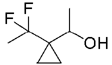 | 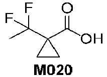 Commercially available, or prepared by Preparation Example 22 | 151.05 |

### Preparation Example 22: Preparation of 1-(1,1-difluoroethyl)cyclopropane-1-carboxylic acid (compound M020)

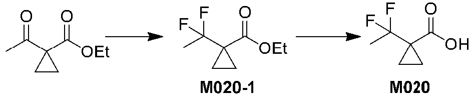

(1) At room temperature, ethyl 1-acetylcyclopropanecarboxylate (5 g, 32.01 mmol) was added to bis(2-methoxyethyl)aminosulfur trifluoride (28.33 g, 128.04 mmol). The reaction mixture was heated to 60°C and stirred for an additional 16 hours. After cooling to room temperature, the reaction mixture was quenched with aqueous sodium carbonate solution (200 mL) and extracted with dichloromethane (100 mL × 3). The organic phases were combined, dried over anhydrous sodium sulfate, filtered, and the filtrate was concentrated under reduced pressure to obtain compound M020-1 (6 g, crude). LC-MS: [M+H]⁺= 179.05.
(2) At room temperature, compound M020-1 (6 g, crude) was dissolved in methanol (90 mL) and water (30 mL), followed by the addition of lithium hydroxide monohydrate (6.71 g, 159.95 mmol). The reaction mixture was stirred at room temperature for 2 hours. The reaction mixture was concentrated, and the residue was adjusted to pH = 5 to 6 with dilute hydrochloric acid (1 M). The mixture was extracted with ethyl acetate (100 mL × 3). The combined organic phases were washed with water (100 mL) and saturated brine (100 mL), dried over anhydrous magnesium sulfate, filtered, and the filtrate was directly concentrated under reduced pressure. The residue was purified by silica gel column chromatography (petroleum ether/ethyl acetate = 3/1) to obtain compound M020 (4.0 g). LC-MS: [M+H]⁺ =151.05.

### Preparation Example 23: Preparation of 1-(2,5-difluoropyridin-3-yl)ethanol (compound M021)

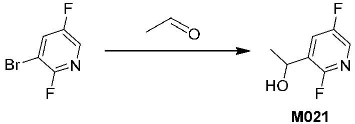

At room temperature, 2,5-difluoro-3-bromopyridine (1.8 g, 9.32 mmol) was dissolved in tetrahydrofuran (35 mL). The reaction mixture was cooled to 0°C, followed by the addition of isopropylmagnesium chloride-lithium chloride solution (1.3 M, 10.7 mL, 13.9 mmol). After the addition was completed, the reaction mixture was warmed to room temperature and stirred for an additional 0.5 hours. The reaction mixture was cooled back to 0°C, and acetaldehyde (2.2 mL, 11.0 mmol, 5 M in tetrahydrofuran) was added. The reaction mixture was warmed to room temperature and stirred for 1 hour. The reaction mixture was quenched with saturated ammonium chloride solution (20 mL), diluted with water (30 mL), and extracted with ethyl acetate (30 mL × 3). The combined organic phases were washed with saturated brine (60 mL), dried over anhydrous sodium sulfate, filtered, and the filtrate was concentrated under reduced pressure. The residue was purified by silica gel column chromatography (petroleum ether/ethyl acetate = 10/1 to 4/1) to obtain compound M021 (730 mg). LC-MS: [M+H]⁺ = 160.05.

### Preparation Example 24: Preparation of 1-(5-fluoro-2-methylpyridin-3-yl)ethanol (compound M022)

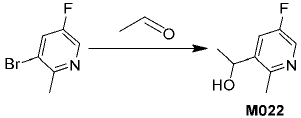

The preparation of compound M022 was referred to the method for preparing compound M021 in Preparation Example 23, wherein the starting material was replaced from 2,5-difluoro-3-bromopyridine to 3-bromo-5-fluoro-2-methylpyridine, and compound M022 (1.0 g) was obtained. LC-MS: [M+H]⁺ =156.10.

### Preparation Example 25: Preparation of (R)-4,4-difluorohexan-2-ol (compound M023)

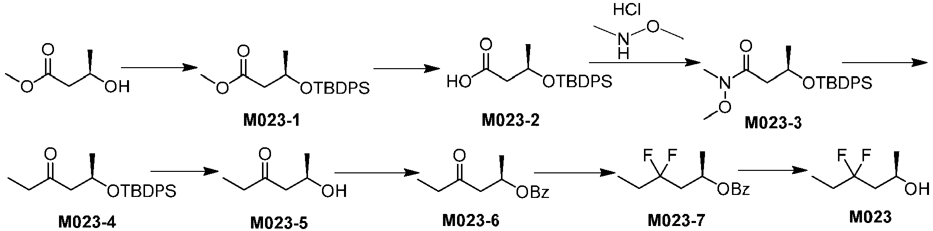

(1) At room temperature, methyl (*R*)-3-hydroxybutyrate (6 g, 50.8 mmol) was dissolved in dichloromethane (90 mL). Under a nitrogen atmosphere, the reaction mixture was cooled to 0°C, followed by the addition of imidazole (6 g, 88.2 mmol) and stirring for 10 minutes. Then, *tert*-butyldiphenylsilyl chloride (15.4 g, 56.0 mmol) was added dropwise. After the dropwise addition was completed, the reaction mixture was warmed to room temperature and stirred for an additional 4 hours. The reaction mixture was quenched with saturated ammonium chloride solution (50 mL), diluted with water (50 mL), and extracted with dichloromethane (50 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 20/1) to obtain compound M023-1 (18 g). LC-MS: [M+H]⁺ =357.56.
(2) At room temperature, compound M023-1 (18 g, 50.6 mmol) was dissolved in methanol (120 mL) and water (40 mL), followed by the addition of lithium hydroxide (6.0 g, 250.0 mmol). The reaction mixture was stirred at room temperature for 1 hour. The reaction mixture was diluted with water (80 mL) and then directly concentrated under reduced pressure to remove methanol. The residue was neutralized with dilute hydrochloric acid (1 M) (pH = 7) and then extracted twice with ethyl acetate (60 mL × 2). The organic phase was dried over anhydrous sodium sulfate, filtered, and the filtrate was concentrated under reduced pressure. The residue was purified by silica gel column chromatography (petroleum ether/ethyl acetate = 5/1) to obtain compound M023-2 (14.7 g). LC-MS: [M+H]⁺ =343.51.
(3) At room temperature, compound M023-2 (14.7 g, 43 mmol) and dimethylhydroxylamine hydrochloride (12.5 g, 129 mmol) were dissolved in anhydrous tetrahydrofuran (250 mL), followed by the addition of *N,N,N',N*'-tetramethyl-O-(7-azabenzotriazol-1-yl)uronium hexafluorophosphate (49 g, 129 mmol) and *N,N*-diisopropylethylamine (47.1 g, 365.5 mmol). The reaction mixture was stirred at room temperature for an additional 2 hours. The reaction mixture was diluted with water (100 mL) and extracted twice with ethyl acetate (100 mL × 2). The organic phase was washed with saturated brine (100 mL), dried over anhydrous sodium sulfate, filtered, and the filtrate was concentrated under reduced pressure. The residue was purified by silica gel column chromatography (petroleum ether/ethyl acetate = 10/1) to obtain compound M023-3 (13.9 g). LC-MS: [M+H]⁺ =386.58.
(4) At room temperature, compound M023-3 (6 g, 15.58 mmol) was dissolved in anhydrous tetrahydrofuran (100 mL). Under a nitrogen atmosphere, the reaction mixture was cooled to -78°C, followed by the dropwise addition of ethylmagnesium bromide (39 mL, 77.9 mmol, 3.0 M in diethyl ether). After the dropwise addition was completed, the reaction mixture was slowly warmed to room temperature and stirred for an additional 1 hour. The reaction mixture was quenched by slowly adding saturated ammonium chloride solution (50 mL) dropwise, then diluted with water (100 mL), and extracted with ethyl acetate (100 mL × 3). The organic phases were combined, washed with saturated brine (100 mL), dried over anhydrous sodium sulfate, filtered, and the filtrate was concentrated under reduced pressure. The residue was purified by silica gel column chromatography (petroleum ether/ethyl acetate = 20/1) to obtain compound M023-4 (5.5 g). LC-MS: [M+1]⁺ =355.15.
(5) At room temperature, compound M023-4 (5 g, 14.10 mmol) was dissolved in anhydrous tetrahydrofuran (25 mL), and tetrabutylammonium fluoride (42.3 mL, 42.3 mmol, 1 M in tetrahydrofuran) was added. The reaction mixture was stirred at room temperature for 3 hours. The reaction mixture was concentrated under reduced pressure. The residue was diluted with water (40 mL) and extracted with dichloromethane (30 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 2/1 to 1/1) to obtain compound M023-5 (1.4 g). LC-MS:[M+1]⁺=117.05.
(6) At room temperature, compound M023-5 (1.4 g, 12.05 mmol) was dissolved in dichloromethane (15 mL), and the reaction mixture was cooled to 0°C. Pyridine (2.86 g, 36.15 mmol) and benzoyl chloride (2.54 g, 18.08 mmol) were added. After slowly warming to room temperature, the reaction was stirred for an additional 2 hours. The reaction mixture was quenched with water (30 mL) and extracted twice with dichloromethane (15 mL × 2). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 4/1) to obtain a crude product (2.5 g), which was further purified by high-performance liquid chromatography (0.1% trifluoroacetic acid system) to obtain compound M023-6 (1.4 g). LC-MS: [M+1]⁺ =221.05.
(7) At room temperature, compound M023-6 (830 mg, 3.77 mmol) was added to bis(2-methoxyethyl)aminosulfur trifluoride (3.33 g, 15.08 mmol). The reaction mixture was heated to 60°C and stirred for 16 hours. After cooling to room temperature, the reaction mixture was slowly poured into saturated aqueous sodium bicarbonate solution (30 mL) pre-cooled to 0°C to quench, and then extracted with dichloromethane (30 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 20/1 to 5/1) to obtain compound M023-7 (400 mg). LC-MS: [M+1]⁺ =243.05.
(8) At room temperature, compound M023-7 (400 mg, 1.65 mmol) was dissolved in methanol (2 mL), tetrahydrofuran (2 mL), and water (1 mL). Lithium hydroxide monohydrate (338.5 mg, 8.25 mmol) was added. The reaction mixture was stirred at room temperature for 2 hours. The reaction mixture was diluted with water (10 mL) and then concentrated under reduced pressure. The residue was extracted with dichloromethane (8 mL × 3). The combined organic phases were washed with saturated brine (15 mL), dried over anhydrous sodium sulfate, filtered, and the filtrate was concentrated under reduced pressure. The residue was purified by silica gel column chromatography (petroleum ether/ethyl acetate = 10/1 to 5/1) to obtain compound M023 (100 mg). LC-MS: [M+1]⁺ =139.16.

### Preparation Example 26: Preparation of (R)-3,3-difluoro-4-methylpentan-2-ol (compound M024)

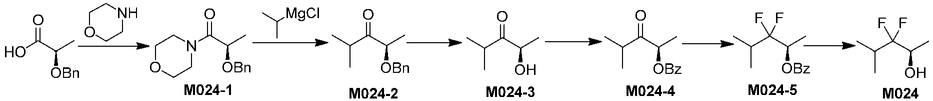

(1) At room temperature, (*R*)-2-(benzyloxy)propanoic acid (10.0 g, 55.5 mmol) was dissolved in *N,N*-dimethylformamide (100 mL), and *N,N*-diisopropylethylamine (21.5 g, 166 mmol) and propylphosphonic anhydride (21.2 g, 66.6 mmol, 50% in ethyl acetate) were added, followed by the addition of morpholine (5.80 g, 66.6 mmol). The reaction mixture was stirred at room temperature for 1 hour. The reaction mixture was diluted with water (100 mL) and extracted with ethyl acetate (50 mL × 4). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 3/1) to obtain compound M024-1 (12.6 g). LC-MS: [M+H]⁺=250.05.
(2) At room temperature, compound M024-1 (12.6 g, 50.5 mmol) was dissolved in anhydrous tetrahydrofuran (200 mL), and the reaction mixture was cooled to -78°C (dry ice-ethyl acetate bath), followed by the dropwise addition of isopropylmagnesium chloride (15.6 g, 151.6 mmol). After the dropwise addition was completed, the reaction mixture was stirred at this temperature for 0.5 hours, then warmed to room temperature, and stirred for an additional 1 hour. The reaction mixture was poured into aqueous ammonium chloride solution (100 mL) to quench. The mixture was extracted with ethyl acetate (50 mL × 3). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 20/1) to obtain product M024-2 (11.3 g). LC-MS:[M+H]⁺=207.28.
(3) At room temperature, compound M024-2 (11.3 g, 50.1 mmol) was dissolved in methanol (110 mL), followed by the addition of wet palladium on carbon (1.60 g, 15.0 mmol, 10%). Under a hydrogen atmosphere, the reaction mixture was heated to 60°C and stirred for 3 hours. After cooling to room temperature, the reaction mixture was filtered through diatomite, and the filter cake was washed twice with methanol (30 mL × 2). The filtrate was concentrated under reduced pressure. The residue was purified by silica gel column chromatography (petroleum ether/ethyl acetate = 4/1) to obtain compound M024-3 (5.30 g). LC-MS:[M+H]⁺=117.16.
(4) At room temperature, compound M024-3 (5.30 g, 45.6 mmol) was dissolved in dichloromethane (50 mL). After cooling the reaction mixture to 0°C, pyridine (3.61 g, 45.6 mmol) was added, followed by the slow dropwise addition of benzoyl chloride (6.41 g, 45.6 mmol). The reaction mixture was stirred at 0°C for 30 minutes, then warmed to room temperature, and stirred for an additional 1 hour. The reaction mixture was poured into dilute hydrochloric acid (50 mL, 1 M) to quench. The mixture was extracted with dichloromethane (30 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 8/1) to obtain compound M024-4 (6.60 g). LC-MS: [M+1]⁺ =221.05.
(5) At room temperature, compound M024-4 (6.50 g, 29.5 mmol) was added to bis(2-methoxyethyl)aminosulfur trifluoride (32.6 g, 147 mmol). The reaction mixture was stirred at room temperature for 0.5 hours, then heated to 60°C, and stirred for an additional 16 hours. After cooling to room temperature, the reaction mixture was added dropwise to a cold aqueous solution of sodium bicarbonate (300 mL) to quench, extracted with ethyl acetate (100 mL × 3), and the combined organic phases were washed with saturated brine (300 mL), dried over anhydrous sodium sulfate, filtered, and the filtrate was concentrated under reduced pressure. The residue was purified by silica gel column chromatography (petroleum ether/ethyl acetate = 10/1) to obtain compound M024-5 (3.50 g). LC-MS: [M+1]⁺ =243.05.
(6) At room temperature, compound M024-5 (3.5 g, 14.5 mmol) was dissolved in a mixed solvent of methanol (15 mL) and tetrahydrofuran (15 mL). A solution of lithium hydroxide monohydrate (3.03 g, 72.3 mmol) in water (5 mL) was added. The reaction mixture was stirred at room temperature for 1 hour. Ethyl acetate (20 mL) and saturated brine (20 mL) were added to the reaction mixture, followed by phase separation. The aqueous phase was extracted with ethyl acetate (20 mL × 2). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 5/1) to obtain compound M024 (2.60 g, crude). LC-MS: [M+1]⁺ =139.16.

### Preparation Example 27: Preparation of (R)-5,5-difluorohexan-2-ol (compound M025)

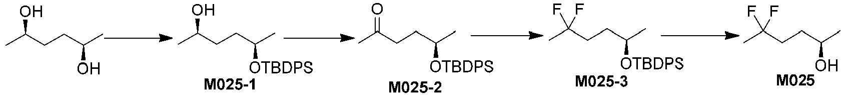

(1) At room temperature, (2*R*,5*R*)-2,5-hexanediol (4.5 g, 38.1 mmol) was dissolved in tetrahydrofuran (150 mL), and imidazole (2.6 g, 38.1 mmol) and *tert*-butyldiphenylsilyl chloride (9.9 g, 36.2 mmol) were added under an ice-water bath. After the addition was completed, the reaction mixture was slowly warmed to room temperature and stirred for an additional 2 hours. The reaction mixture was diluted with aqueous ammonium chloride solution (100 mL) and extracted with ethyl acetate (50 mL × 3). The combined organic phases were washed with saturated brine (200 mL), dried over anhydrous sodium sulfate, filtered, and the filtrate was concentrated under reduced pressure. The residue was purified by silica gel column chromatography (petroleum ether/ethyl acetate = 5/1) to obtain compound M025-1 (4.8 g). LC-MS: [M+H]⁺=357.10.
(2) At room temperature, compound M025-1 (4.8 g, 13.5 mmol) was dissolved in dichloromethane (100 mL), then sodium bicarbonate (5.7 g, 67.5 mmol) was added, and 1,1,1-triacetoxy-1,1-dihydro-1,2-benziodoxol-3(1*H*)-one (6.3 g, 14.8 mmol) was added under an ice-water bath. After warming to room temperature, the reaction mixture was stirred for an additional 3 hours. The reaction mixture was directly filtered, and the filter cake was washed with dichloromethane (100 mL). The filtrate was added to aqueous sodium carbonate solution (100 mL) and stirred for 10 minutes. The organic phase was separated, and the aqueous phase was extracted twice with dichloromethane (50 mL × 2). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 5/1) to obtain compound M025-2 (4.2 g). LC-MS: [M+H]⁺ =355.15.
(3) At room temperature, compound M025-2 (1.5 g, 4.2 mmol) was dissolved in bis(2-methoxyethyl)aminosulfur trifluoride (4.7 g, 21.2 mmol). The reaction mixture was heated to 50°C and stirred for an additional 16 hours. The reaction mixture was cooled to room temperature and then added dropwise to a cold aqueous solution of sodium bicarbonate (40 mL). The resulting mixture was extracted with ethyl acetate (50 mL × 3). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 10/1) to obtain compound M025-3 (1.36 g). LC-MS: [M+H]⁺ =377.20.
(4) At room temperature, compound M025-3 (1.36 g, 3.6 mmol) was dissolved in a solution of tetrabutylammonium fluoride in tetrahydrofuran (1 M, 18 mL). The reaction mixture was stirred at room temperature for 16 hours. The reaction mixture was diluted with water (30 mL) and extracted with ethyl acetate (30 mL × 3). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 4/1) to obtain compound M025 (600 mg, crude). LC-MS: [M+H]⁺= 139.09.

### Preparation Example 28: Preparation of (2R)-4-fluoropentan-2-ol (compound M026)

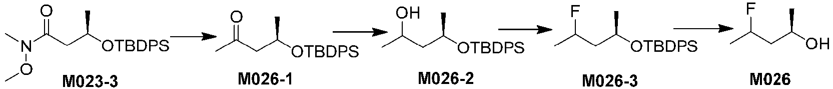

(1) At room temperature, compound M023-3 (13.9 g, 36.1 mmol) was dissolved in anhydrous tetrahydrofuran (200 mL). Under a nitrogen atmosphere, the reaction mixture was cooled to -78°C, followed by the dropwise addition of methylmagnesium bromide (60 mL, 3.0 M in diethyl ether). After the dropwise addition was completed, the reaction mixture was slowly warmed to room temperature and stirred for an additional 3 hours. The reaction mixture was quenched with saturated ammonium chloride solution (50 mL), then diluted with water (100 mL), and extracted with ethyl acetate (100 mL × 3). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 10/1) to obtain compound M026-1 (11.3 g). LC-MS: [M+H]⁺ =341.56.
(2) At room temperature, compound M026-1 (5 g, 14.7 mmol) was dissolved in methanol (50 mL). The reaction mixture was cooled to 0°C, and sodium borohydride (1.7 g, 44 mmol) was slowly added in portions. After the addition was completed, the reaction mixture was warmed to room temperature and stirred for an additional 1 hour. The reaction mixture was quenched by slowly adding water (50 mL), adjusted to pH = 2 to 3 with dilute hydrochloric acid (1 M), and then extracted twice with ethyl acetate (50 mL × 2). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M026-2 (4.7 g). LC-MS: [M+H]⁺ =343.55.
(3) At room temperature, compound M026-2 (4.7 g, 13.7 mmol) was dissolved in dichloromethane (90 mL). Under a nitrogen atmosphere, the reaction mixture was cooled to 0°C, and diethylaminosulfur trifluoride (11 g, 68.6 mmol) was added dropwise. After the dropwise addition was completed, the reaction mixture was warmed to room temperature and stirred for an additional 18 hours. The reaction mixture was poured into ice water (100 mL) to quench and extracted with dichloromethane (100 mL). The organic phase was washed with saturated sodium carbonate solution (50 mL) and saturated brine (50 mL). The combined organic phases were dried over anhydrous sodium sulfate, filtered, and the filtrate was concentrated under reduced pressure. The residue was purified by silica gel column chromatography (petroleum ether) to obtain compound M026-3 (2.15 g). LC-MS: [M+H]⁺ =345.52.
(4) At room temperature, compound M026-3 (1 g, 2.9 mmol) was dissolved in anhydrous tetrahydrofuran (10 mL), and tetrabutylammonium fluoride (1.1 g, 4.2 mmol) was added. The reaction mixture was heated to 60°C and stirred for an additional 3 hours. After cooling to room temperature, the reaction mixture was diluted with water (20 mL) and extracted twice with ethyl acetate (20 mL × 2). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 5/1) to obtain compound M026 (310 mg). LC-MS: [M+H]⁺ =107.1.

The preparation of the compounds in the following table was referred to the preparation method of Preparation Example 28, wherein compound M026-1 was replaced with the corresponding reactants listed in the following table:

| No. | Compound structure | Reactant | LC-MS [M+H]⁺ |
|---|---|---|---|
| M027 | 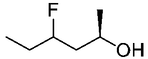 | 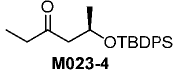 | 121.17 |
| M028 | 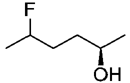 | 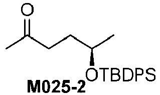 | 121.09 |

### Preparation Example 29: Preparation of 1-fluoropentan-2-ol (compound M029)

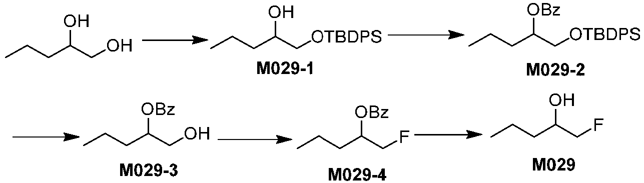

(1) At room temperature, 1,2-pentanediol (3.0 g, 28.80 mmol) and imidazole (2.94 g, 43.21 mmol) were dissolved in dichloromethane (30 mL), followed by the addition of a solution of tert-butyldiphenylsilyl chloride (7.92 g, 28.80 mmol) in dichloromethane (30 mL). The reaction mixture was stirred at room temperature for 16 hours. The reaction mixture was diluted with water (100 mL) and extracted with dichloromethane (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 5/1) to obtain compound M029-1 (5.8 g). LC-MS: [M+H-78]⁺ = 265.10.
(2) At room temperature, compound M029-1 (2.3 g, 6.71 mmol), 4-dimethylaminopyridine (82 mg, 0.67 mmol), and pyridine (5.31 g, 67.14 mmol) were dissolved in dichloromethane (30 mL), followed by the dropwise addition of benzoyl chloride (1.42 g, 10.07 mmol). After the addition was completed, the reaction mixture was stirred at room temperature for 16 hours. The reaction mixture was diluted with water (50 mL) and extracted with dichloromethane (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 20/1) to obtain compound M029-2 (2.8 g). LC-MS: [M+H]⁺ = 447.10.
(3) At room temperature, compound M029-2 (2.6 g, 5.82 mmol) was dissolved in anhydrous tetrahydrofuran (30 mL), and tetrabutylammonium fluoride (1.83 g, 6.99 mmol) was added. The reaction mixture was stirred at room temperature for 6 hours. The reaction mixture was diluted with water (30 mL) and extracted with ethyl acetate (30 mL × 3). The combined organic phases were washed with water (30 mL) and saturated brine (30 mL), dried over anhydrous sodium sulfate, filtered, and the filtrate was concentrated under reduced pressure. The residue was purified by silica gel column chromatography (petroleum ether/ethyl acetate = 2/1) to obtain compound M029-3 (1.2 g). LC-MS: [M+H]⁺ = 209.10.
(4) At room temperature, compound M029-3 (900 mg, 4.32 mmol) was dissolved in dichloromethane (10 mL), and the reaction mixture was cooled to 0°C, followed by the dropwise addition of diethylaminosulfur trifluoride (1.04 g, 6.48 mmol). After warming to room temperature, the reaction mixture was stirred for an additional 3 hours. The reaction mixture was quenched by adding dropwise to saturated sodium bicarbonate solution (50 mL) and extracted with dichloromethane (50 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound M029-4 (700 mg). LC-MS: [M+H]⁺ = 211.10.
(5) At room temperature, compound M029-4 (0.6 g, 2.85 mmol) was dissolved in methanol (4.5 mL) and water (1.5 mL), followed by the addition of lithium hydroxide monohydrate (360 mg, 8.56 mmol). The reaction mixture was stirred at room temperature for 1 hour. The reaction mixture was diluted with water (20 mL) and extracted with ethyl acetate (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 5/1) to obtain compound M029 (250 mg).

### Preparation Example 30: Preparation of 4-(5-bromopyridin-2-yl)-1-methyl-1H-pyrazole-5-carboxylic acid (M030)

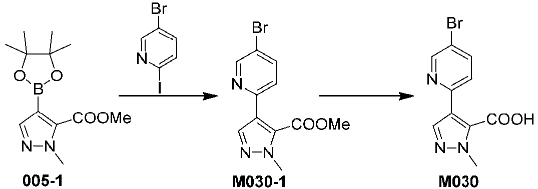

The preparation of compound M030 was referred to the method for preparing compound M018 in Preparation Example 16, wherein compound M018-1 was replaced with 5-bromo-2-iodopyridine, and compound M030 (450 mg) was obtained. LC-MS: [M+H]⁺ = 283.9.

### Preparation Example 31: Preparation of (R)-1-(bicyclo[1.1.1]pentan-1-yl)ethan-1-ol (compound 237-3A)

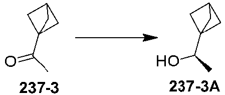

At room temperature, compound 237-3 (21 g, crude) was dissolved in dichloromethane (210 mL), followed by the addition of (*S*)-2-methyl-CBS-oxazaborolidine (53 g, 190.65 mmol). The reaction mixture was cooled to 0°C, and borane-dimethyl sulfide complex (95 mL, 2 M in THF) was slowly added dropwise. After the dropwise addition was completed, the reaction mixture was warmed to room temperature and stirred for an additional 2 hours. The reaction mixture was quenched with 1 M dilute hydrochloric acid (100 mL), filtered, and the filtrate was extracted twice with dichloromethane (200 mL). The combined organic phases were dried over anhydrous sodium sulfate, filtered, and the filtrate was concentrated under reduced pressure at room temperature. The residue was added to diethyl ether (100 mL), and the mixture was filtered. The filtrate was concentrated under reduced pressure. The operation with diethyl ether was repeated twice. The residue was purified by silica gel column chromatography (petroleum ether/ethyl acetate = 10/1) to obtain compound 237-3A (21 g). LC-MS: [M+H]⁺ =113.17.

### Preparation Example 32: Preparation of 2-chloro-4-(3,3-difluorobutyl)pyrimidine (compound 255-2)

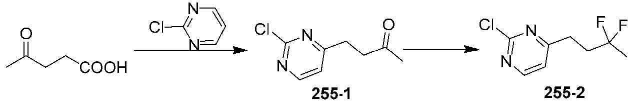

(1) At room temperature, 2-chloropyrimidine (4.0 g, 34.92 mmol) was dissolved in dichloromethane (60 mL) and water (60 mL), followed by the addition of levulinic acid (8.11 g, 69.85 mmol), ammonium persulfate (24 g, 104.77 mmol), silver nitrate (593 mg, 3.50 mmol), and trifluoroacetic acid (7.96 g, 69.85 mmol) under stirring. The reaction mixture was heated to 45°C and stirred for an additional 16 hours. After cooling to room temperature, the reaction mixture was quenched with aqueous sodium bicarbonate solution (60 mL), filtered through diatomite, and the filtrate was washed with dichloromethane (180 mL). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 255-1 (600 mg). LC-MS: [M+H]⁺ = 185.00.
(2) At room temperature, compound 255-1 (550 mg, 2.98 mmol) was dissolved in dichloromethane (10 mL), and the reaction mixture was cooled to 0°C, followed by the slow dropwise addition of diethylaminosulfur trifluoride (1.44 g, 8.94 mmol). The reaction mixture was heated to 40°C and stirred for 6 hours. After cooling to room temperature, the reaction mixture was quenched with aqueous sodium bicarbonate solution (20 mL) and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 255-2 (400 mg). LC-MS: [M+H]⁺ = 207.00.

### Preparation Example 33: Preparation of 3-(5-bromo-6-methylpyridin-2-yl)-5-methylisoxazole-4-carboxylic acid (M031)

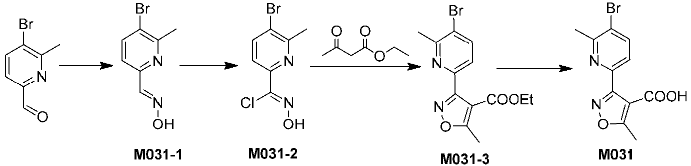

(1) At room temperature, 5-bromo-6-methylpyridine-2-carbaldehyde (4 g, 20.0 mmol) was dissolved in ethanol (60 mL) and water (30 mL), followed by the addition of hydroxylamine hydrochloride (1.81 g, 26.0 mmol) and sodium acetate (2.46 g, 30.0 mmol). The reaction mixture was stirred at room temperature for 0.5 hours. The reaction mixture was directly filtered, and the filtrate was concentrated under reduced pressure to obtain compound M031-1 (2.8 g). LC-MS: [M+H]⁺ =214.95.
(2) At room temperature, compound M031-1 (2.8 g, 15.4 mmol) was dissolved in *N,N-*dimethylformamide (15 mL), and *N*-chlorosuccinimide (2.43 g, 18.23 mmol) was added. The reaction mixture was stirred at room temperature for 0.5 hours. Ice water (35 mL) was added to the reaction mixture, and the mixture was filtered. The filter cake was concentrated under reduced pressure to obtain compound M031-2 (1.2 g). LC-MS: [M+H]⁺ =250.85.
(3) Ethyl acetoacetate (813.7 mg, 18.14 mmol) was dissolved in anhydrous methanol (30 mL) at room temperature, followed by the addition of compound M031-2 (1.2 g, 4.81 mmol). The reaction mixture was cooled to 0°C, and sodium methoxide (0.98 mL, 5.29 mmol, 30% in methanol) was added. The reaction mixture was stirred at 0°C for 0.25 hours, then additional sodium methoxide (0.98 mL, 5.29 mmol, 30% in methanol) was added. After warming to room temperature, the reaction mixture was stirred for an additional 0.5 hours. The reaction mixture was diluted with water (40 mL) and extracted twice with ethyl acetate (30 mL × 2). The combined organic phases were washed with water (30 mL) and saturated brine (50 mL), dried over anhydrous sodium sulfate, filtered, and the filtrate was concentrated under reduced pressure. The residue was purified by silica gel column chromatography (petroleum ether/ethyl acetate = 10/1) to obtain compound M031-3 (950 mg). LC-MS: [M +H]⁺ = 324.95.
(4) At room temperature, compound M031-3 (750 mg, 2.31 mmol) was dissolved in methanol (7 mL) and water (3.5 mL), followed by the addition of lithium hydroxide monohydrate (379.2 mg, 9.24 mmol). The reaction mixture was stirred at room temperature for 0.5 hours. The reaction mixture was concentrated under reduced pressure to remove methanol, and the residue was diluted with water (20 mL). The aqueous phase was adjusted to pH = 2 to 3 with dilute hydrochloric acid (1 M) and extracted twice with ethyl acetate (30 mL × 2). The organic phases were combined and washed with saturated brine (30 mL). The organic phase was dried over anhydrous sodium sulfate, filtered, and the filtrate was concentrated under reduced pressure to obtain compound M031 (560 mg). LC-MS: [M+H]⁺ = 296.90.

### Preparation Example 34: Preparation of 3-(5-bromo-6-chloropyridin-2-yl)-5-methylisoxazole-4-carboxylic acid (M032)

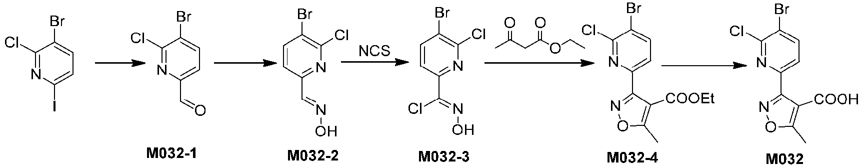

The preparation of compound M032 was referred to the method for preparing compound M031 in Preparation Example 33, wherein the starting material 5-bromo-6-methylpyridine-2-carbaldehyde was replaced with compound M032-1, and compound M032 (450 mg) was obtained. LC-MS: [M+H]⁺ =318.85.

The preparation method for compound M032-1 is as follows:
At room temperature, 3-bromo-2-chloro-6-iodopyridine (4.8 g, 15.0 mmol) was dissolved in anhydrous tetrahydrofuran (60 mL). Under a nitrogen atmosphere, the reaction mixture was cooled to - 15°C, and isopropylmagnesium chloride (2 M in tetrahydrofuran, 8.3 mL, 16.6 mmol) was added. The reaction mixture was stirred at -15°C for 1 hour, and then N,N-dimethylformamide (1.65 g, 22.62 mmol) was added. The reaction mixture was stirred for 1 hour, then warmed to room temperature, and stirred for an additional 1 hour. The reaction mixture was cooled to 0°C, and the reaction was quenched with dilute hydrochloric acid (10 mL, 3 M). The pH of the solution was adjusted to 9 to 10 with dilute aqueous sodium hydroxide solution (15%), followed by dilution with water (100 mL). The mixture was extracted twice with ethyl acetate (70 mL × 2). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 15/1) to obtain compound M032-1 (1.6 g). LCMS:[M+1]⁺ = 221.85.

Referring to the method of Preparation Example 34, the compounds in the following table were prepared by replacing the starting material 3-bromo-2-chloro-6-iodopyridine with the corresponding reactants listed in the following table:

| No. | Structure | LC-MS: [M+H]⁺ | Reactant |
|---|---|---|---|
| 491-5 | 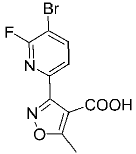 | 300.85 | 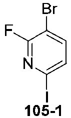 |
| 496-7 | 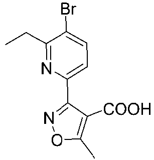 | 312.90 | 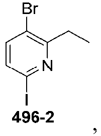 its preparation refers to the method for preparing compound 218-5 in Example 56, with 218-3 replaced with 6-ethylpyridin-2-amine. |
| 477-5 | 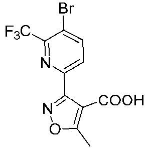 | 350.85 | 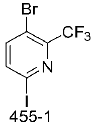 |

### Preparation Example 35: Preparation of 5-(5-bromopyridin-2-yl)-3-methylisoxazole-4-carboxylic acid (M033)

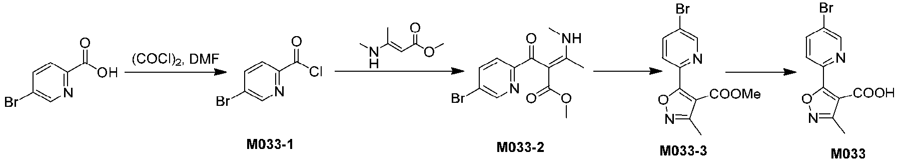

The preparation of compound M033 was referred to the method for preparing compound M017 in Preparation Example 15, wherein compound M017-1 was replaced with the starting material 5-bromo-2-pyridinecarboxylic acid, and compound M033 (600 mg) was obtained. LC-MS: [M+H]⁺=284.85.

### Preparation Example 36: Preparation of 5-(5-bromo-6-chloropyridin-2-yl)-3-methylisoxazole-4-carboxylic acid (M034)

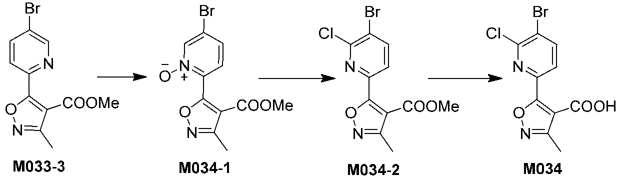

(1) At room temperature, compound M033-3 (2 g, 6.73 mmol) was dissolved in dichloromethane (20 mL), and after cooling the reaction mixture to 0°C, *m*-chloroperoxybenzoic acid (7.00 g, 33.65 mmol, 85%) was added in portions. The reaction mixture was stirred at room temperature for 6 hours. The reaction mixture was quenched with saturated sodium sulfite (50 mL). The mixture was extracted with ethyl acetate (30 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/2) to obtain compound M034-1 (1.6 g). LC-MS: [M+H]⁺ =312.95.
(2) At room temperature, compound M034-1 (1.6 g, 5.11 mmol) was dissolved in 1,2-dichloroethane (16 mL), and the reaction mixture was cooled to 0°C, followed by the dropwise addition of phosphorus oxychloride (4.70 g, 30.66 mmol). The reaction was slowly warmed to room temperature, stirred for 0.5 hours, then heated to 50°C, and stirred for an additional 12 hours. After cooling to room temperature, the reaction mixture was quenched with saturated aqueous sodium sulfite solution (30 mL), diluted with saturated brine (30 mL), and extracted twice with ethyl acetate (25 mL × 2). The organic phases were combined, dried over anhydrous sodium sulfate, filtered, and the filtrate was concentrated under reduced pressure. The residue was purified by silica gel column chromatography (petroleum ether/ethyl acetate = 1/2) to obtain compound M034-2 (0.9 g).
   LC-MS: [M+H]⁺ =332.90.
(3) At room temperature, compound M034-2 (0.9 g, 2.71 mmol) was dissolved in methanol (9 mL) and tetrahydrofuran (9 mL), followed by the addition of a solution of lithium hydroxide (0.33 g, 13.55 mmol) in water (3 mL). The reaction mixture was stirred at room temperature for 1 hour. The reaction mixture was diluted with water (30 mL), and methanol and tetrahydrofuran were removed by concentration under reduced pressure. The residue was extracted with ethyl acetate (15 mL), and the aqueous phase was adjusted to pH = 5 with dilute hydrochloric acid (1 M), then extracted with ethyl acetate (30 mL × 3). The combined organic phases were washed with saturated brine (30 mL), dried over anhydrous sodium sulfate, filtered, and the filtrate was concentrated under reduced pressure to obtain compound M034 (850 mg). LC-MS: [M+H]⁺ =318.90.

### Preparation Example 37: Preparation of compound M035

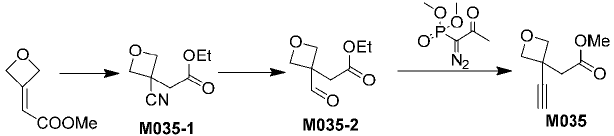

(1) At room temperature, trimethylsilyl cyanide (5.6 g, 56.28 mmol) was added to tetrabutylammonium fluoride (14.7 g, 56.28 mmol). The reaction mixture was stirred at room temperature for 0.5 hours, followed by the addition of a solution of methyl 2-(oxetan-3-ylidene)acetate (4.0 g, 28.14 mmol) in tetrahydrofuran (25 mL). The reaction mixture was heated to 50°C and stirred for an additional 3 hours. After cooling to room temperature, the reaction mixture was poured into water (50 mL) to dilute and extracted with ethyl acetate (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 5/1) to obtain compound M035-1 (4.4 g). LC-MS: [M+H]⁺ =170.07.
(2) At room temperature, compound M035-1 (500 mg, 2.96 mmol) was dissolved in pyridine (6 mL), glacial acetic acid (2 mL), and water (2 mL), followed by the addition of Raney nickel (174 mg, 2.96 mmol). The reaction mixture was heated to 60°C and stirred for 16 hours. After cooling to room temperature, the reaction mixture was diluted with ethyl acetate (30 mL), washed with dilute hydrochloric acid (30 mL × 3), and then washed with saturated aqueous sodium bicarbonate solution (30 mL × 3). The organic phase was dried over anhydrous sodium sulfate, filtered, and the filtrate was concentrated under reduced pressure to obtain compound M035-2 (380 mg). LC-MS: [M+H]⁺ =173.07.
(3) At room temperature, dimethyl (1-diazo-2-oxopropyl)phosphonate (637 mg, 3.31 mmol) was dissolved in methanol (10 mL). After cooling the reaction mixture to 0°C, potassium carbonate (764 mg, 5.53 mmol) was added, and the reaction mixture was stirred at room temperature for 0.5 hours. Then, compound M035-2 (380 mg, 2.21 mmol) was added. The reaction mixture was stirred at room temperature for an additional 16 hours. The reaction mixture was poured into water (30 mL) to dilute, extracted with dichloromethane (30 mL × 3), and the combined organic phases were dried over anhydrous sodium sulfate, filtered, and the filtrate was concentrated under reduced pressure. The residue was purified by silica gel column chromatography (petroleum ether/ethyl acetate = 5/1) to obtain compound M035 (130 mg). LC-MS: [M+H]⁺ =155.06.

### Preparation Example 38: Preparation of methyl 2-(3-ethoxy-1-ethynylcyclobutyl)acetate (compound M036)

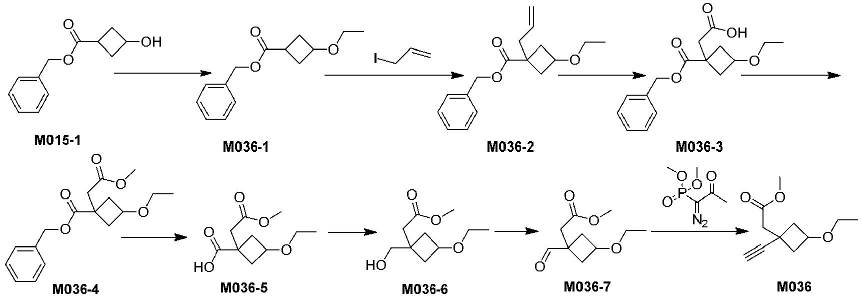

(1) At room temperature, compound M015-1 (15 g, 72.73 mmol) was dissolved in *N,N-*dimethylformamide (150 mL). The reaction mixture was cooled to 0°C, and sodium hydride (4.36 g, 109.09 mmol, 60% dispersed in mineral oil) was added in portions. The reaction mixture was stirred at 0°C for 0.5 hours, and then iodoethane (22.69 g, 145.46 mmol) was added. The reaction mixture was warmed to room temperature and stirred for an additional 2 hours. The reaction mixture was slowly added dropwise to dilute hydrochloric acid (100 mL, 1 M) to quench and then extracted with ethyl acetate (100 mL × 3). The combined organic phases were dried over anhydrous sodium sulfate, filtered, and the filtrate was concentrated under reduced pressure. The residue was purified by silica gel column chromatography (petroleum ether/ethyl acetate = 20/1) to obtain compound M036-1 (8.1 g). LC-MS: [M+H]⁺=235.05.
(2) At room temperature, compound M036-1 (8.1 g, 34.57 mmol) was dissolved in ultra-dry tetrahydrofuran (400 mL), followed by the addition of allyl iodide (14.52 g, 86.42 mmol). The reaction mixture was cooled to -78°C (dry ice-ethyl acetate bath), and lithium bis(trimethylsilyl)amide (69.14 mL, 69.14 mmol, 1 M in tetrahydrofuran) was slowly added. The reaction mixture was slowly warmed to room temperature and stirred for an additional 2 hours. The reaction mixture was poured into saturated ammonium chloride (150 mL) to quench and extracted with ethyl acetate (200 mL × 3). The combined organic phases were dried over anhydrous sodium sulfate, filtered, and the filtrate was concentrated under reduced pressure. The residue was purified by silica gel column chromatography (petroleum ether/ethyl acetate = 20/1) to obtain compound M036-2 (7.5 g, containing cis/trans isomers). LC-MS: [M+H]⁺=275.10.
(3) At room temperature, compound M036-2 (5.5 g, 20.05 mmol) was dissolved in acetonitrile (50 mL) and water (50 mL), followed by the addition of sodium periodate (17.15 g, 80.2 mmol) and ruthenium trichloride (420 mg, 2.01 mmol). The reaction mixture was stirred at room temperature for 2 hours, then potassium permanganate (3.17 g, 20.05 mmol) was added, and the reaction mixture was stirred at room temperature for an additional 1 hour. The reaction mixture was quenched with saturated sodium bicarbonate solution (30 mL), filtered, and saturated sodium sulfite (20 mL) was added to the filtrate. The mixture was stirred for 15 minutes and filtered again. The filtrate was adjusted to pH = 5 to 6 with dilute aqueous hydrochloric acid (1 M) solution, extracted with ethyl acetate (50 mL × 3), and the combined organic phases were dried over anhydrous sodium sulfate, filtered, and the filtrate was concentrated under reduced pressure. The residue was purified by silica gel column chromatography (petroleum ether/ethyl acetate = 2/1) to obtain compound M036-3 (3.5 g, containing cis/trans isomers). LC-MS: [M+H]⁺ =293.05.
(4) At room temperature, compound M036-3 (3.5 g, 11.97 mmol) was dissolved in methanol (30 mL), and thionyl chloride (4.27 g, 35.91 mmol) was added slowly. The reaction mixture was refluxed (65°C) for 2 hours. After cooling to room temperature, the reaction mixture was concentrated under reduced pressure. The residue was purified by silica gel column chromatography (petroleum ether/ethyl acetate = 10/1) to obtain compound M036-4 (3.5 g, containing cis/trans isomers). LC-MS: [M+H]⁺ =307.10.
(5) At room temperature, compound M036-4 (3.5 g, 11.42 mmol) was dissolved in tetrahydrofuran (35 mL), and wet palladium on carbon (350 mg, 3.31 mmol, 10%) and wet palladium hydroxide on carbon (350 mg, 2.51 mmol, 20%) were added. Under a hydrogen atmosphere, the reaction mixture was heated to 40°C and stirred for 2 hours. The reaction mixture was filtered through diatomite, and the filtrate was concentrated under reduced pressure to obtain compound M036-5 (2.3 g, crude, containing cis-trans isomers). LC-MS: [M+H]⁺ =217.05.
(6) At room temperature, compound M036-5 (2.3 g, crude) was dissolved in ultra-dry tetrahydrofuran (25 mL). Under a nitrogen atmosphere, the reaction mixture was cooled to 0°C, and borane-dimethyl sulfide complex (2.13 mL, 21.23 mmol, 10 M in tetrahydrofuran) was added dropwise. The reaction mixture was warmed to room temperature and stirred for 2 hours. The reaction mixture was quenched with water (20 mL) and extracted with ethyl acetate (30 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound M036-6 (1.3 g, containing cis/trans isomers). LC-MS: [M+H]⁺ =203.05.
(7) At room temperature, oxalyl chloride (1.34 mL, 15.82 mmol) was dissolved in anhydrous dichloromethane (10 mL). Under a nitrogen atmosphere, the reaction mixture was cooled to -78°C (dry ice-ethyl acetate bath), and dimethyl sulfoxide (2.25 mL, 31.64 mmol) was slowly added dropwise, maintaining the internal temperature below -70°C. At this temperature, the reaction mixture was stirred for 0.5 hours, followed by the dropwise addition of a solution of compound M036-6 (1.6 g, 7.91 mmol) in dichloromethane (10 mL), maintaining the internal temperature below -70°C. The reaction mixture was further reacted at - 78°C for 1 hour. Finally, triethylamine (8.80 mL, 63.28 mmol) was slowly added dropwise, maintaining the internal temperature below -65°C. After the dropwise addition was completed, the reaction mixture was slowly warmed to room temperature and stirred for 1 hour. The reaction mixture was diluted with water (50 mL) and extracted with dichloromethane (30 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M036-7 (1.5 g, containing cis/trans isomers). LC-MS: [M+H]⁺ =201.05.
(8) At room temperature, compound M036-7 (1.5 g, 7.49 mmol) was dissolved in methanol (15 mL), and the reaction mixture was cooled to 0°C. Potassium carbonate (1.24 g, 8.99 mmol) was added, followed by the slow addition of dimethyl (1-diazo-2-oxopropyl)phosphonate (1.58 g, 8.24 mmol). After warming to room temperature, the reaction mixture was stirred for 2 hours. Water (15 mL) was slowly added to the reaction mixture, and the mixture was extracted with dichloromethane (20 mL × 3). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 5/1) to obtain compound M036 (1.3 g, containing cis/trans isomers). LC-MS: [M+H]⁺ =197.05

### Preparation Example 39: Preparation of methyl 2-(1-ethynyl-3,3-difluorocyclobutyl)acetate (compound M037)

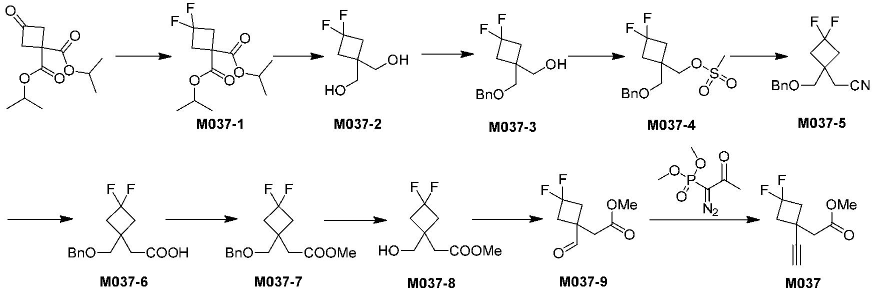

(1) At room temperature, diisopropyl 3-oxocyclobutane-1,1-dicarboxylate (20 g, 82.55 mmol) was dissolved in dichloromethane (300 mL). The reaction mixture was cooled to 0°C, and diethylaminosulfur trifluoride (33.3 g, 206.4 mmol) was slowly added. The reaction mixture was slowly warmed to room temperature and stirred for an additional 24 hours. The reaction mixture was slowly poured into cold (0°C) saturated sodium carbonate solution (200 mL) to quench, extracted with dichloromethane (200 mL × 2), and the combined organic phases were dried over anhydrous sodium sulfate, filtered, and the filtrate was concentrated under reduced pressure. The residue was purified by silica gel column chromatography (petroleum ether/ethyl acetate = 20/1) to obtain compound M037-1 (18.6 g). LC-MS [M+H]⁺ = 265.12.
(2) At room temperature, lithium aluminum hydride (4.0 g, 105.6 mmol) was dissolved in anhydrous tetrahydrofuran (190 mL), and under a nitrogen atmosphere, the reaction mixture was cooled to 0°C, followed by the slow addition of compound M037-1 (18.6 g, 70.4 mmol). The reaction mixture was heated to 25°C and stirred for an additional 16 hours. Water (50 mL), 50% sodium hydroxide solution (20 mL), and water (100 mL) were sequentially added to the reaction mixture to quench the reaction. The reaction mixture was filtered under reduced pressure, and the filter cake was washed with ethyl acetate (100 mL). The filtrate was extracted with ethyl acetate (200 mL × 2), and the combined organic phases were dried over anhydrous sodium sulfate, filtered, and the filtrate was concentrated under reduced pressure. The residue was purified by silica gel column chromatography (ethyl acetate/petroleum ether = 1/0) to obtain compound M037-2 (6.8 g). LC-MS [M+H]⁺=153.06.
(3) At room temperature, compound M037-2 (6.8 g, 44.7 mmol) was dissolved in anhydrous tetrahydrofuran (70 mL) and *N*,*N*-dimethylformamide (70 mL). The reaction mixture was cooled to 0°C, and sodium hydride (1.8 g, 44.7 mmol, 60% dispersed in mineral oil) was added slowly in portions. The reaction mixture was stirred at 0°C for 2 hours, followed by the dropwise addition of benzyl bromide (7.65 g, 44.7 mmol). After warming to room temperature, the reaction mixture was stirred for an additional 1 hour. The reaction mixture was quenched with saturated ammonium chloride solution (100 mL) and extracted with ethyl acetate (80 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound M037-3 (5 g). LC-MS [M+H]⁺=243.11.
(4) At room temperature, compound M037-3 (5 g, 20.7 mmol) was dissolved in dichloromethane (50 mL), and the reaction mixture was cooled to 0°C. Triethylamine (2.5 g, 24.8 mmol) and methanesulfonyl chloride (2.6 g, 22.7 mmol) were added, and the reaction mixture was stirred at 0°C for 2 hours. The reaction mixture was quenched with water (100 mL) and extracted with dichloromethane (100 mL × 2). The combined organic phases were dried over anhydrous sodium sulfate, filtered, and the filtrate was concentrated under reduced pressure to obtain compound M037-4 (6 g). LC-MS [M+H]⁺=321.09.
(5) At room temperature, compound M037-4 (6 g, 18.7 mmol) was dissolved in *N,N-*dimethylformamide (80 mL), followed by the addition of trimethylsilyl cyanide (3.72 g, 37.5 mmol) and tetrabutylammonium fluoride (9.8 g, 37.5 mmol). The reaction mixture was heated to 130°C and stirred for 2 hours. The reaction mixture was cooled to room temperature, diluted with water (50 mL), and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M037-5 (3.8 g). LC-MS [M+H]⁺=252.11.
(6) At room temperature, compound M037-5 (3.8 g, 15.2 mmol) was dissolved in ethanol (50 mL) and water (50 mL), followed by the addition of potassium hydroxide (5.1 g, 90.8 mmol). The reaction mixture was heated to 100°C and stirred for 4 days. Additional potassium hydroxide (1.5 g, 26.8 mmol) was added, and the reaction mixture was stirred at 100°C for an additional 16 hours. After cooling to room temperature, the reaction mixture was concentrated under reduced pressure to remove ethanol. The residue was washed with ethyl acetate (60 mL × 3). The aqueous phase was adjusted to pH = 3 to 4 with dilute hydrochloric acid (1 M) and then extracted with ethyl acetate (60 mL × 3). The combined organic phases were dried over anhydrous sodium sulfate, filtered, and the filtrate was concentrated under reduced pressure to obtain compound M037-6 (4 g). LC-MS [M+H]⁺=271.11.
(7) At room temperature, compound M037-6 (4 g, 14.8 mmol) was dissolved in methanol (90 mL), and the reaction mixture was cooled to 0°C. Thionyl chloride (8.7 g, 73.1 mmol) was added slowly, and the reaction mixture was heated to 80°C and stirred for an additional 1 hour. The reaction mixture was cooled to room temperature and concentrated under reduced pressure. The residue was purified by silica gel column chromatography (petroleum ether/ethyl acetate = 2/1) to obtain compound M037-7 (3.7 g). LC-MS [M+H]⁺=285.12.
(8) At room temperature, compound M037-7 (3.7 g, 13.0 mmol) was dissolved in methanol (40 mL), and wet palladium on carbon (0.69 g, 0.65 mmol, 10%) was added. Under a hydrogen atmosphere, the reaction mixture was heated to 30°C and stirred for an additional 48 hours. The reaction mixture was filtered under reduced pressure through diatomite, and the filter cake was washed with methanol (40 mL). The filtrate was concentrated under reduced pressure to obtain compound M037-8 (2.8 g, crude). LC-MS: [M+H]⁺ =195.08.
(9) At room temperature, oxalyl chloride (1.3 g, 10.3 mmol) was dissolved in anhydrous dichloromethane (20 mL). Under a nitrogen atmosphere, the reaction mixture was cooled to -78°C (dry ice-ethyl acetate bath), and a solution of dimethyl sulfoxide (1.6 g, 20.6 mmol) in dichloromethane (2 mL) was slowly added dropwise, maintaining the internal temperature below -70°C. At this temperature, the reaction was stirred for an additional 0.5 hours, followed by the dropwise addition of a solution of compound M037-8 (1.0 g, 5.15 mmol) in dichloromethane (2 mL), maintaining the internal temperature below -70°C. The reaction mixture was stirred at this temperature for an additional 1 hour. Triethylamine (4.2 g, 4.12 mmol) was then slowly added dropwise, maintaining the internal temperature below -65°C. After the dropwise addition was completed, the reaction mixture was slowly warmed to room temperature and stirred for an additional 1 hour. The reaction mixture was diluted with water (30 mL) and extracted with dichloromethane (30 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound M037-9 (0.72 g). LC-MS: [M+H]⁺ =193.06.
(10) At room temperature, compound M037-9 (0.72 g, 3.75 mmol) was dissolved in methanol (15 mL), and the reaction mixture was cooled to 0°C. Potassium carbonate (0.78 g, 5.63 mmol) was added, followed by the slow addition of dimethyl (1-diazo-2-oxopropyl)phosphonate (1.8 g, 9.4 mmol). After warming to room temperature, the reaction mixture was stirred for an additional 1 hour. The reaction mixture was diluted by adding water (20 mL) slowly and extracted with ethyl acetate (20 mL × 2). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound M037 (0.45 g). LC-MS: [M+H]⁺ =189.06.

### Preparation Example 40: Preparation of diethyl (5-ethynylspiro[2.3]hexan-5-yl)phosphonate (compound M038)

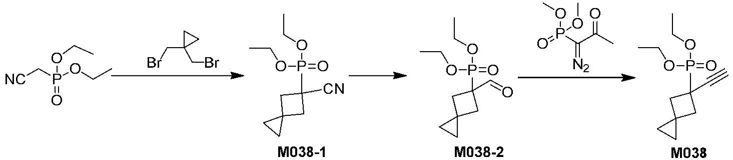

(1) At room temperature, diethyl cyanomethylphosphonate (1.0 g, 5.60 mmol) was dissolved in acetonitrile (10 mL), followed by the addition of 1,1-bis(bromomethyl)cyclopropane (1.23 g, 5.6 mmol), benzyltriethylammonium chloride (130 mg, 0.56 mmol, TBAC), and potassium carbonate (2.3 g, 16.80 mmol). The reaction mixture was heated to 80°C and stirred for 16 hours. After cooling to room temperature, the reaction mixture was poured into water (30 mL) to dilute and extracted with ethyl acetate (30 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1) to obtain compound M038-1 (0.9 g). LCMS: [M+H]⁺ =244.05.
(2) At room temperature, M038-1 (1.6 g, 6.58 mmol) was dissolved in pyridine (20 mL), glacial acetic acid (10 mL), and water (10 mL), followed by the addition of Raney nickel (386 mg, 6.58 mmol). The reaction mixture was heated to 60°C and stirred for 4 hours. The reaction mixture was cooled to room temperature, diluted with ethyl acetate (50 mL), washed with dilute hydrochloric acid (50 mL × 3, 1 M), and then washed with saturated aqueous sodium bicarbonate solution (50 mL × 3). The organic phase was dried over anhydrous sodium sulfate, filtered, and the filtrate was concentrated under reduced pressure to obtain compound M038-2 (1.6 g, crude). LCMS: [M+H]⁺ =247.10.
(3) At room temperature, dimethyl (1-diazo-2-oxopropyl)phosphonate (1.3 g, 6.71 mmol) was dissolved in methanol (30 mL), and the reaction mixture was cooled to 0°C, followed by the addition of potassium carbonate (1.5 g, 11.17 mmol). The reaction mixture was warmed to room temperature and stirred for an additional 0.5 hours. Then, compound M038-2 (1.1 g, 4.47 mmol) was added. The reaction mixture was stirred at room temperature for an additional 1 hour. The reaction mixture was poured into water (50 mL) to quench, extracted with dichloromethane (50 mL × 3), and the combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M038 (800 mg). LCMS: [M+H]⁺ =243.11.

### Preparation Example 41: Preparation of methyl 2-(5-ethynylspiro[2.3]hexan-5-yl)acetate (compound M039)

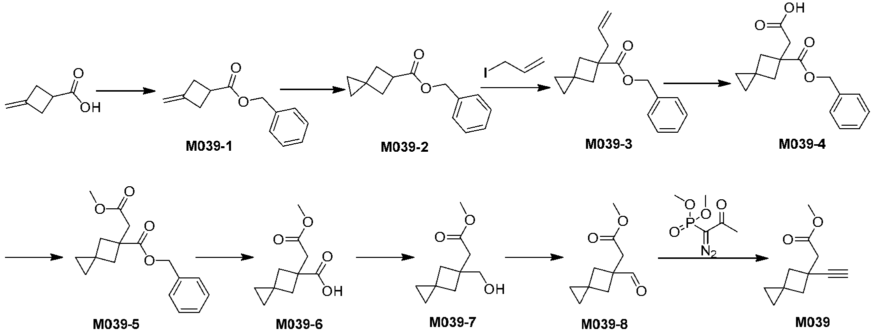

The preparation of compound M039 was referred to the method for preparing compound M015 in Preparation Example 13, wherein the intermediate M015-2 was replaced with intermediate M039-2, and compound M039 (1 g) was obtained. LC-MS: [M+H]⁺ =179.05.

The preparation method for intermediate M039-2 is as follows:
(1) At room temperature, 3-methylenecyclobutanecarboxylic acid (5 g, 44.59 mmol) was dissolved in N,N-dimethylformamide (50 mL), followed by the addition of benzyl bromide (9 g, 53.51 mmol) and potassium carbonate (9 g, 66.89 mmol). The reaction mixture was stirred at room temperature for 24 hours. The reaction mixture was diluted with water (50 mL) and extracted with ethyl acetate (100 mL × 3). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 10/1) to obtain compound M039-1 (9 g). LC-MS: [M+H]⁺=203.10.
(2) At room temperature, diethylzinc (11 g, 89.00 mmol) was dispersed in dichloromethane (100 mL). Under a nitrogen atmosphere, the reaction mixture was cooled to 0°C, followed by the dropwise addition of trifluoroacetic acid (3.3 mL, 44.50 mmol). The reaction mixture was stirred at 0°C for 15 minutes, then diiodomethane (48 g, 178.00 mmol) was added, and the reaction mixture was stirred at 0°C for an additional 30 minutes. Finally, a solution of M039-1 (9 g, 44.50 mmol) in dichloromethane was added. The reaction mixture was stirred at room temperature for 4 hours. The reaction mixture was quenched with saturated sodium bicarbonate solution (50 mL) and then extracted with dichloromethane (60 mL × 3). The combined organic phases were dried over anhydrous sodium sulfate, filtered, and the filtrate was concentrated under reduced pressure. The residue was purified by silica gel column chromatography (petroleum ether/ethyl acetate = 20/1) to obtain compound M039-2 (8 g). LC-MS: [M+H]⁺=217.05.

### Preparation Example 42: Preparation of 5-(5-bromo-6-(fluoromethyl)pyridin-2-yl)-3-methylisoxazole-4-carboxylic acid (compound M040)

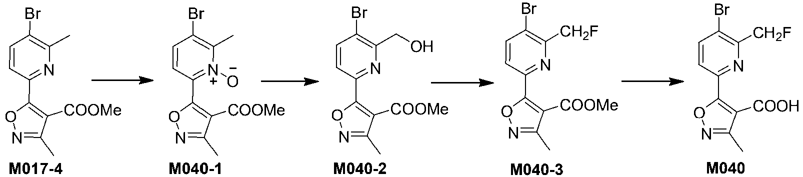

(1) At room temperature, compound M017-4 (2.4 g, 7.71 mmol) was dissolved in dichloromethane (50 mL). The reaction mixture was cooled to 0°C, and m-chloroperoxybenzoic acid (4.0 g, 23.13 mmol, purity: 85%) was added. The reaction mixture was then warmed to room temperature and stirred for 16 hours. The reaction mixture was diluted with dichloromethane (50 mL) and washed with saturated aqueous sodium bicarbonate solution (30 mL × 3). The organic phase was dried over anhydrous sodium sulfate, filtered, and the filtrate was concentrated under reduced pressure. The residue was purified by silica gel column chromatography (petroleum ether/ethyl acetate = 10/1) to obtain compound M040-1 (1.7 g). LCMS: [M+H]⁺ =328.90.
(2) At room temperature, compound M040-1 (1.7 g, 5.20 mmol) was dissolved in dichloromethane (50 mL), and trifluoroacetic anhydride (5.5 g, 26 mmol) was added. The reaction mixture was stirred at room temperature for 16 hours. The reaction mixture was concentrated under reduced pressure, and the residue was diluted with ethyl acetate (30 mL). Saturated sodium bicarbonate solution (100 mL) was then added, and the mixture was stirred at room temperature for 16 hours. The phases were then separated, and the organic phase was dried over anhydrous sodium sulfate, filtered, and the filtrate was concentrated under reduced pressure. The residue was purified by silica gel column chromatography (petroleum ether/ethyl acetate = 5/1) to obtain compound M040-2 (1.5 g). LCMS: [M+H]⁺ =326.85.
(3) At room temperature, compound M040-2 (500 mg, 1.53 mmol) was dissolved in dichloromethane (5 mL), and the reaction mixture was cooled to 0°C. Diethylaminosulfur trifluoride (247 mg, 1.53 mmol) was added. The reaction mixture was stirred at 0°C for 1 hour. The reaction mixture was poured into saturated aqueous sodium bicarbonate solution (30 mL) to quench and extracted with dichloromethane (30 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M040-3 (250 mg). LCMS: [M+H]⁺ =328.85.
(4) At room temperature, compound M040-3 (250 mg, 0.76 mmol) was dissolved in tetrahydrofuran (3 mL) and water (3 mL), followed by the addition of lithium hydroxide monohydrate (96 mg, 2.28 mmol). The reaction mixture was stirred at room temperature for 1 hour. The reaction mixture was diluted with water (10 mL) and then concentrated under reduced pressure to remove methanol. The residue was washed with dichloromethane (10 mL × 3), and the aqueous phase was adjusted to pH = 3 to 4 with dilute hydrochloric acid (1 M). The mixture was filtered under reduced pressure, and the filter cake was dried under reduced pressure to obtain compound M040 (230 mg). LCMS: [M+H]⁺ =316.90.

### Preparation Example 43: Preparation of 4-(5-bromo-6-(difluoromethyl)pyridin-2-yl)-1-methyl-1H-1,2,3-triazole-5-carboxylic acid (compound M041)

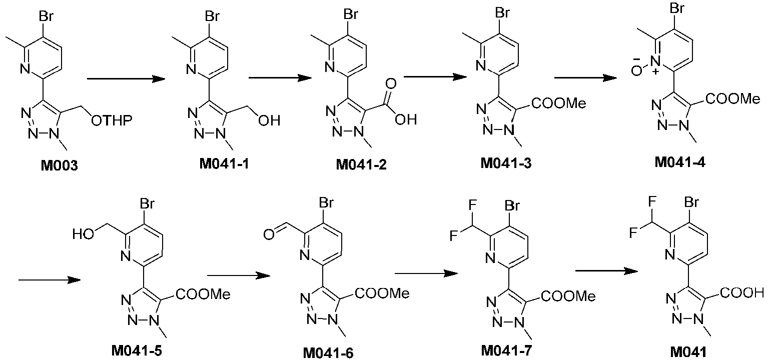

(1) At room temperature, compound M003 (6.0 g, 16.34 mmol) was dissolved in methanol (60 mL), and pyridinium *p*-toluenesulfonate (4.1 g, 16.34 mmol) was added. The reaction mixture was heated to 60°C and stirred for 1 hour. After cooling to room temperature, the reaction mixture was concentrated under reduced pressure. The residue was diluted with water (60 mL) and extracted with ethyl acetate (40 mL × 2). The combined organic phases were washed with saturated aqueous sodium bicarbonate solution (60 mL) and saturated brine (60 mL). The organic phase was dried over anhydrous sodium sulfate, filtered, and the filtrate was concentrated under reduced pressure. The residue was purified by silica gel column chromatography (petroleum ether/ethyl acetate = 2/1) to obtain compound M041-1 (4.5 g). LC-MS: [M+H]⁺ = 282.95.
(2) At room temperature, compound M041-1 (600 mg, 2.1 mmol) was dissolved in acetonitrile (6 mL) and water (4.5 mL), followed by the addition of potassium permanganate (664 mg, 4.2 mmol). The reaction mixture was stirred at room temperature for 22 hours. The reaction mixture was added with aqueous sodium hydroxide solution (10 mL, 2 M) and extracted with ethyl acetate (10 mL × 3). The aqueous phase was adjusted to pH = 2 to 3 with dilute hydrochloric acid (1 M), filtered, and the filter cake was washed with water (10 mL), then concentrated under reduced pressure to obtain compound M041-2 (476 mg, crude). LC-MS: [M+H]+ = 298.85.
(3) At room temperature, compound M041-2 (1.0 g, 3.37 mmol) was dissolved in methanol (5 mL) and dichloromethane (5 mL), and a solution of trimethylsilyldiazomethane in *n*-hexane (3.37 mL, 6.74 mmol, 2 M) was slowly added dropwise. The reaction mixture was stirred at room temperature for 2 hours. The reaction mixture was concentrated under reduced pressure, and the residue was purified by silica gel column chromatography (petroleum ether/ethyl acetate = 5/1) to obtain compound M041-3 (800 mg). LC-MS: [M+H]⁺ =310.90.
(4) At room temperature, compound M041-3 (800 mg, 2.57 mmol) was dissolved in dichloromethane (10 mL), and m-chloroperoxybenzoic acid (443.5 mg, 2.57 mmol, purity: 85%) was slowly added. The reaction mixture was stirred at room temperature for 16 hours. The reaction mixture was quenched with sodium carbonate solution (20 mL), washed with dichloromethane (20 mL × 3), and the combined organic phases were washed with water (20 mL), dried over anhydrous sodium sulfate, filtered, and the filtrate was concentrated under reduced pressure. The residue was purified by silica gel column chromatography (petroleum ether/ethyl acetate = 1/1) to obtain compound M041-4 (700 mg). LC-MS: [M+H]⁺ =326.95.
(5) At room temperature, compound M041-4 (600 mg, 1.83 mmol) was dissolved in dichloromethane (18 mL), and trifluoroacetic anhydride (1921.77 mg, 9.15 mmol) was slowly added dropwise. The reaction mixture was stirred at room temperature for 16 hours. Methanol (10 mL) and saturated sodium bicarbonate solution (30 mL) were added to the reaction mixture, and the mixture was stirred at room temperature for 2 hours and then extracted with dichloromethane (20 mL × 3). The combined organic phases were washed with water (30 mL), dried over anhydrous sodium sulfate, filtered, and the filtrate was concentrated under reduced pressure. The residue was purified by silica gel column chromatography (petroleum ether/ethyl acetate = 1/1) to obtain compound M041-5 (470 mg). LC-MS: [M+H]⁺ =326.90.
(6) At room temperature, compound M041-5 (420 mg, 1.28 mmol) was dissolved in dichloromethane (5 mL), followed by the slow addition of Dess-Martin periodinane (651 mg, 1.54 mmol). The reaction mixture was stirred at room temperature for 6 hours. Saturated sodium bicarbonate solution (20 mL) was added to the reaction mixture, and the mixture was stirred at room temperature for 2 hours, followed by extraction with dichloromethane (20 mL × 3). The combined organic phases were washed with water (30 mL), dried over anhydrous sodium sulfate, filtered, and the filtrate was concentrated under reduced pressure. The residue was purified by silica gel column chromatography (petroleum ether/ethyl acetate = 2/1) to obtain compound M041-6 (330 mg). LC-MS: [M+H]⁺ =324.90.
(7) At room temperature, compound M041-6 (280 mg, 0.86 mmol) was dissolved in dichloromethane (6 mL), and diethylaminosulfur trifluoride (555 mg, 3.44 mmol) was slowly added dropwise. The reaction mixture was stirred at room temperature for 2 hours. The reaction mixture was quenched with saturated sodium bicarbonate solution (10 mL). Then, the mixture was extracted with dichloromethane (10 mL × 3). The combined organic phases were washed with water (10 mL), dried over anhydrous sodium sulfate, filtered, and the filtrate was concentrated under reduced pressure. The residue was purified by silica gel column chromatography (petroleum ether/ethyl acetate = 4/1) to obtain compound M041-7 (260 mg). LC-MS: [M+H]⁺ =346.90.
(8) At room temperature, compound M041-7 (260 mg, 0.75 mmol) was dissolved in tetrahydrofuran (3 mL) and water (3 mL), and lithium hydroxide monohydrate (157 mg, 3.75 mmol) was added. The reaction mixture was stirred at room temperature for 4 hours. The reaction mixture was diluted with water (5 mL), concentrated under reduced pressure, and the pH of the residual solution was adjusted to 5 to 6 with dilute hydrochloric acid (1 M). The mixture was filtered under reduced pressure, and the filter cake was washed with water (10 mL). The filter cake was dried under reduced pressure to obtain compound M041 (220 mg). LC-MS: [M+H]⁺ =332.90.

### Preparation Example 44: Preparation of 3-(5-bromo-6-methylpyridin-2-yl)-5-methylisothiazole-4-carboxylic acid (compound M042)

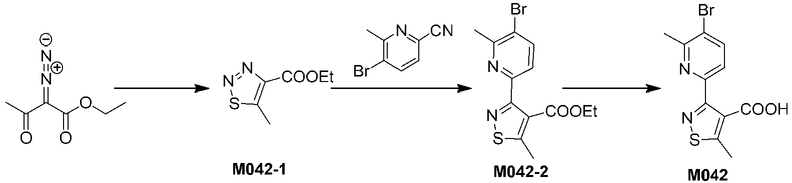

(1) At room temperature, ethyl 2-diazo-3-oxobutanoate (1 g, 6.4 mmol) was dissolved in toluene (15 mL), followed by the addition of Lawesson's reagent (3.11 g, 7.68 mmol). Under a nitrogen atmosphere, the reaction mixture was heated to 100°C and stirred for 16 hours. After cooling to room temperature, the reaction mixture was poured into water (50 mL) and extracted with ethyl acetate (30 mL × 2). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 10/1 to 5/1) to obtain compound M042-1 (0.82 g). LC-MS: [M+H]⁺=172.95.
(2) At room temperature, compound M042-1 (600 mg, 3.48 mmol), 5-bromo-6-methyl-2-pyridinecarbonitrile (686 mg, 3.48 mmol), dichloro(cycloocta-1,5-diene)ruthenium(II) (24.4 mg, 0.0870 mmol), and 1,1'-bis(diphenylphosphino)ferrocene (116 mg, 0.210 mmol) were added to chlorobenzene (9 mL). Under a nitrogen atmosphere, the reaction mixture was heated to 130°C and stirred for 1 hour. After cooling to room temperature, the reaction mixture was diluted with saturated brine (15 mL) and extracted with ethyl acetate (20 mL × 3). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 8/1) to obtain compound M042-2 (1.08 g). LC-MS: [M+H]⁺=342.90.
(3) At room temperature, compound M042-2 (800 mg, 2.34 mmol) was dissolved in methanol (5 mL) and tetrahydrofuran (5 mL), followed by the addition of a solution of lithium hydroxide monohydrate (295 mg, 7.02 mmol) in water (2.5 mL). The reaction mixture was stirred at room temperature for 16 hours. The reaction mixture was diluted with water (10 mL) and then concentrated under reduced pressure. The residue was extracted with ethyl acetate (10 mL). The aqueous phase was adjusted to pH = 5 with 1 M hydrochloric acid, filtered under reduced pressure, and the filter cake was sequentially washed with water (5 mL × 2), ethanol (5 mL × 2), and petroleum ether (5 mL × 2). The filter cake was dried under reduced pressure to obtain compound M042 (560 mg). LC-MS: [M+H]⁺=314.85.

### Preparation Example 45: Preparation of 5-(5-bromo-6-ethylpyridin-2-yl)-3-methylisoxazole-4-carboxylic acid (compound M043)

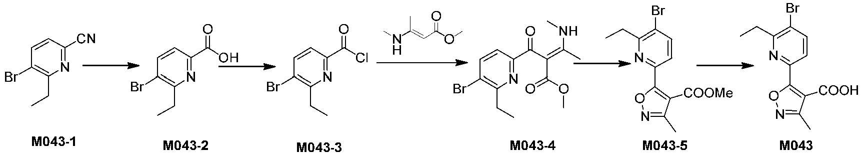

The preparation of compound M043 was referred to the method for preparing compound M017 in Preparation Example 15, wherein the starting material 5-bromo-6-methyl-2-pyridinecarbonitrile was replaced with M043-1, and compound M043 (500 mg) was obtained. LC-MS: [M+H]⁺=310.95.

The preparation method for compound M043-1 is as follows:

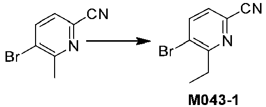

At room temperature, 5-bromo-6-methyl-2-pyridinecarbonitrile (5.00 g, 25.4 mmol) was dissolved in anhydrous tetrahydrofuran (100 mL), cooled to -50°C under a nitrogen atmosphere, and lithium bis(trimethylsilyl)amide (8.37 g, 50.0 mmol, 1 M in tetrahydrofuran) was slowly added dropwise. The reaction mixture was stirred at this temperature (-50°C) for 0.5 hours. The reaction mixture was cooled to -70°C and stirred for an additional 1 hour, followed by the dropwise addition of iodomethane (7.20 g, 50.8 mmol). The reaction mixture was stirred at this temperature (-70°C) for an additional 1 hour. The reaction mixture was slowly warmed to 0°C, and saturated aqueous ammonium chloride solution (100 mL) was added to quench the reaction. The mixture was extracted with ethyl acetate (100 mL × 2). The combined organic phases were washed with saturated brine (200 mL), dried over anhydrous sodium sulfate, filtered, and the filtrate was concentrated under reduced pressure. The residue was purified by silica gel column chromatography (petroleum ether/ethyl acetate = 10/1) and then purified by preparative high-performance liquid chromatography (0.1% ammonia in water conditions) to obtain compound M043-1 (1.09 g). LC-MS: [M+H]⁺=212.90.

### Preparation Example 46: Preparation of 5-(5-bromo-6-(trifluoromethyl)pyridin-2-yl)-3-methylisoxazole-4-carboxylic acid (compound M044)

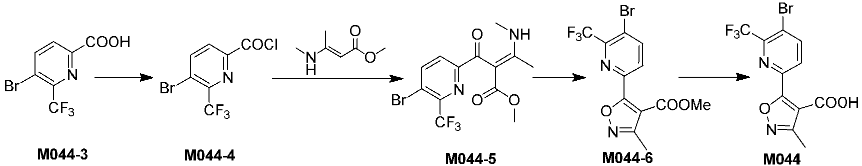

The preparation of compound M044 was referred to the method for preparing compound M017 in Preparation Example 15, wherein compound M017-1 was replaced with compound M044-3, and compound M044 (400 mg) was obtained. LC-MS: [M+H]⁺ = 352.90.

The preparation method for compound M044-3 is as follows: 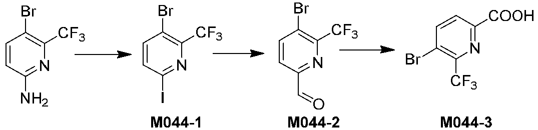
(1) At room temperature, 5-bromo-6-(trifluoromethyl)pyridin-2-amine (20 g, 83.1 mmol) was dissolved in tetrahydrofuran (200 mL), followed by the addition of copper(I) iodide (23.7 g, 124.2 mmol), diiodomethane (177.3 g, 53.3 mmol), and tert-butyl nitrite (34.1 g, 331.2 mmol). The reaction mixture was heated to 80°C and stirred for 1 hour. The reaction mixture was cooled to room temperature, filtered, and the filtrate was diluted with ethyl acetate (400 mL) and washed with water (200 mL). The organic phase was dried over anhydrous sodium sulfate, filtered, and the filtrate was concentrated under reduced pressure. The residue was purified by silica gel column chromatography (petroleum ether/ethyl acetate = 1/0) to obtain compound M044-1 (9 g). LC-MS: [M+H]⁺ = 351.84.
(2) At room temperature, compound M044-1 (8 g, 22.8 mmol) was dissolved in tetrahydrofuran (100 mL), and the reaction mixture was cooled to -15°C (ice salt bath) under a nitrogen atmosphere, followed by the dropwise addition of isopropylmagnesium chloride (25 mL, 25.0 mmol, 1 M in tetrahydrofuran). The reaction mixture was stirred at this temperature for 1 hour, and then N,N-dimethylformamide (2.5 g, 34.1 mmol) was added dropwise. After stirring for 1 hour, the reaction mixture was warmed to room temperature and stirred for an additional 1 hour. The reaction mixture was quenched with dilute hydrochloric acid (20 mL, 3 M), adjusted to pH = 9 with sodium hydroxide solution (15%), diluted with water (200 mL), and extracted with ethyl acetate (200 mL). The organic phase was dried over anhydrous sodium sulfate, filtered, and the filtrate was concentrated under reduced pressure. The residue was purified by silica gel column chromatography (petroleum ether/ethyl acetate = 20/1) to obtain compound M044-2 (4.1 g). LC-MS: [M+H]⁺=253.94.
(3) At room temperature, compound M044-2 (1.40 g, 5.51 mmol) was dissolved in acetonitrile (14 mL) and water (14 mL), followed by the addition of potassium permanganate (1.74 g, 11.0 mmol). The reaction mixture was stirred at room temperature for 4 hours. Ethyl acetate (20 mL) was added to the reaction mixture, followed by the dropwise addition of saturated sodium sulfite solution until the purple-red color disappeared. Then, 15% aqueous sodium hydroxide solution (4 mL) was added dropwise, and the mixture was vigorously stirred for 10 minutes. The reaction mixture was filtered under reduced pressure, and the filter cake was washed with water (50 mL × 2). The phases of the filtrate were separated, and the aqueous phase was adjusted to pH = 5 with dilute hydrochloric acid (1 M) and then extracted with ethyl acetate (50 mL × 3). The combined organic phases were washed with saturated brine (20 mL), dried over anhydrous sodium sulfate, filtered, and the filtrate was concentrated under reduced pressure to obtain compound M044-3 (1.26 g). LC-MS: [M+H]⁺=270.00.

### Preparation Example 47: Preparation of 3,3-difluoropentan-2-ol (compound M045)

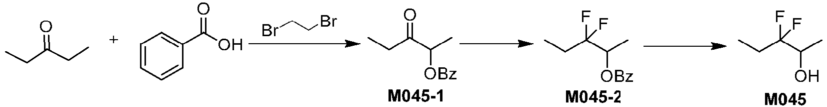

(1) At room temperature, 3-pentanone (125 mL, 1.18 mol), benzoic acid (6.0 g, 49.13 mmol), and 1,2-dibromoethane (13.84 g, 73.70 mmol) were added to a reaction flask, followed by the addition of potassium iodide (1.63 g, 9.83 mmol) and potassium carbonate (10.19 g, 73.70 mmol). Under an oxygen atmosphere, the reaction mixture was heated to 60°C and stirred for an additional 20 hours. The reaction mixture was cooled to room temperature, diluted with water (100 mL), and extracted with ethyl acetate (100 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound M045-1 (2.9 g). LC-MS: [M+H]⁺ = 207.10.
(2) At room temperature, compound M045-1 (2.9 g, 14.06 mmol) was dissolved in dichloromethane (30 mL), and the reaction mixture was cooled to 0°C, followed by the dropwise addition of diethylaminosulfur trifluoride (6.8 g, 42.18 mmol). After the dropwise addition was completed, the reaction mixture was heated to 40°C and stirred for 16 hours. The reaction mixture was cooled to room temperature and poured into saturated sodium bicarbonate solution (50 mL) to quench. The mixture was extracted with dichloromethane (50 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound M045-2 (2.4 g). LC-MS: [M+H]⁺ = 229.06.
(3) At room temperature, compound M045-2 (1.0 g, 4.38) was dissolved in methanol (7 mL) and water (3 mL), followed by the addition of lithium hydroxide monohydrate (0.92 g, 21.91 mmol). The reaction mixture was stirred at room temperature for 1 hour. The reaction mixture was diluted with water (20 mL) and extracted with ethyl acetate (20 mL × 3). The combined organic phases were dried over anhydrous sodium sulfate, filtered, and the filtrate was concentrated under reduced pressure at room temperature to obtain compound M045 (335 mg). C-MS: [M+H]⁺ = 125.07.

### Preparation Example 48: Preparation of the hydrochloride of 4-fluoro-N,4-dimethylpentan-2-amine (compound M046)

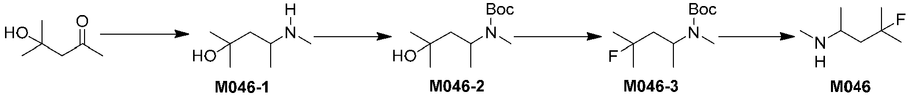

(1) At room temperature, diacetone alcohol (5 g, 43.04 mmol) and methylamine hydrochloride (7.56 g, 111.90 mmol) were dissolved in dichloromethane (100 mL). Triethylamine (11.32 g, 111.90 mmol) was added to the reaction mixture under stirring, followed by the addition of glacial acetic acid (5.17 g, 86.08 mmol). The reaction mixture was stirred at room temperature for 30 minutes, then sodium triacetoxyborohydride (25.54 g, 120.51 mmol) was added, and the reaction mixture was stirred at room temperature for 16 hours. The reaction mixture was poured into saturated sodium bicarbonate solution (100 mL) to quench, extracted with dichloromethane (100 mL × 3), and the combined organic phases were sequentially washed with water (100 mL) and saturated brine (100 mL), dried over anhydrous sodium sulfate, filtered, and the filtrate was concentrated under reduced pressure to obtain compound M046-1 (2 g, crude). LC-MS: [M+H]⁺ =132.10.
(2) At room temperature, compound M046-1 (2 g, 15.24 mmol) and triethylamine (2.31 g, 22.86 mmol) were dissolved in dichloromethane (20 mL), and the reaction mixture was cooled to 0°C. Under stirring, di-tert-butyl dicarbonate (3.99 g, 18.29 mmol) was added dropwise, and the reaction mixture was stirred at room temperature for 1 hour. The reaction mixture was quenched with water (50 mL) and extracted with dichloromethane (20 mL × 3). The combined organic phases were washed with water (30 mL) and saturated brine (30 mL), dried over anhydrous sodium sulfate, filtered, and the filtrate was concentrated under reduced pressure. The residue was purified by silica gel column chromatography (petroleum ether/ethyl acetate = 5/1) to obtain compound M046-2 (1.3 g). LC-MS: [M+H]⁺ = 232.15.
(3) At room temperature, compound M046-2 (300 mg, 1.3 mmol) was dissolved in dichloromethane (2 mL), and the reaction mixture was cooled to -78°C (dry ice-ethyl acetate bath), followed by the dropwise addition of diethylaminosulfur trifluoride (314 mg, 1.95 mmol). After the dropwise addition was completed, the reaction mixture was stirred at -78°C for 1 hour. The reaction mixture was quenched with saturated aqueous sodium bicarbonate solution (20 mL) and extracted with dichloromethane (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 10/1) to obtain compound M046-3 (400 mg). LC-MS: [M+H]⁺ = 234.10.
(4) At room temperature, compound M046-3 (400 mg, 1.71 mmol) was dissolved in dichloromethane (5 mL), and a solution of hydrogen chloride in ethyl acetate (5 mL, 4 M) was added. The reaction mixture was stirred at room temperature for 2 hours. The reaction mixture was concentrated under reduced pressure to obtain the hydrochloride of compound M046 (200 mg, crude). LC-MS: [M+H]⁺ = 134.15.

### Example 1: Preparation of 2-(1-((6-(5-((4-(cyclopropylmethyl)-1H-1,2,3-triazol-1-yl)methyl)-1-methyl-1H-1,2,3-triazol-4-yl)-2-methylpyridin-3-yl)ethynyl)cyclopropyl)acetic acid (compound 001)

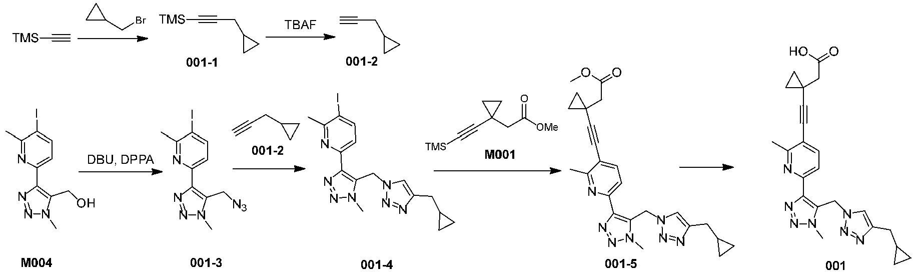

(1) At room temperature, trimethylsilylacetylene (3.70 g, 37.75 mmol) was dissolved in anhydrous tetrahydrofuran (20 mL). The reaction mixture was purged with nitrogen, cooled to -78°C under a nitrogen atmosphere, and then n-butyllithium (16.4 mL, 40.90 mmol, 2.5 M) was added dropwise. After the dropwise addition was completed, the reaction mixture was stirred at -78°C for an additional 1 hour. At this temperature, a solution of bromomethylcyclopropane (4.25 g, 31.46 mmol) and hexamethylphosphoramide (6.20 g, 34.61 mmol) in tetrahydrofuran (20 mL) was added dropwise. The internal temperature was maintained below -70°C. After the dropwise addition was completed, the reaction mixture was stirred at - 78°C for 1 hour, and then warmed to room temperature, and stirred for an additional 16 hours. The resulting reaction mixture was quenched with saturated aqueous ammonium chloride solution (30 mL), diluted with water (10 mL), and extracted with diethyl ether (40 mL). The organic phases were combined. The resulting organic phase was washed with saturated aqueous sodium chloride solution (50 mL) and water (40 mL), dried over anhydrous sodium sulfate, filtered, and the filtrate was concentrated under reduced pressure. The resulting residue was purified by silica gel column chromatography (pure petroleum ether) to obtain compound 001-1 (3.4 g, crude).
(2) At room temperature, compound 001-1 (2.90 g, 19.08 mmol) was dissolved in anhydrous tetrahydrofuran (30 mL), and tetrabutylammonium fluoride (8.8 g, 28.62 mmol) was added. The reaction mixture was stirred at room temperature for 4 hours. The resulting reaction mixture was washed with saturated aqueous ammonium chloride solution (30 mL), and the resulting organic phase was dried over anhydrous sodium sulfate and filtered. The resulting filtrate was concentrated under reduced pressure to obtain a solution of compound 001-2 in tetrahydrofuran (30 mL).
(3) At room temperature, compound M004 (980 mg, 2.97 mmol) was dissolved in anhydrous tetrahydrofuran (15 mL). After cooling the reaction mixture to 0°C, diphenylphosphoryl azide (1.22 g, 4.45 mmol) and 1,8-diazabicyclo[5.4.0]undec-7-ene (677 mg, 4.45 mmol) were sequentially added. The reaction mixture was warmed to room temperature and stirred for an additional 16 hours. The resulting reaction mixture was diluted with water (20 mL) and extracted with ethyl acetate (20 mL). The organic phases were combined. The resulting organic phase was washed with saturated aqueous sodium chloride solution (30 mL), dried over anhydrous sodium sulfate, filtered, and the filtrate was concentrated under reduced pressure. The resulting residue was purified by silica gel column chromatography (petroleum ether/ethyl acetate = 3/1) to obtain compound 001-3 (1.00 g). LC-MS [M+H]⁺=355.95.
(4) At room temperature, compound 001-3 (1.00 g, 2.82 mmol) was dissolved in a mixed solvent of tert-butanol (10 mL), tetrahydrofuran (10 mL), and water (10 mL), followed by the addition of compound 001-2 (the solution of compound 001-2 in tetrahydrofuran obtained in the second step). The reaction mixture was stirred at room temperature for 48 hours. The resulting reaction mixture was directly concentrated under reduced pressure, and the residue was diluted with water (20 mL) and extracted with ethyl acetate (20 mL). The organic phases were combined. The resulting organic phase was washed with saturated aqueous sodium chloride solution (30 mL), dried over anhydrous sodium sulfate, filtered, and the filtrate was concentrated under reduced pressure. The resulting residue was purified by silica gel column chromatography (petroleum ether/ethyl acetate = 3/1) to obtain compound 001-4 (110 mg). LC-MS [M+H]⁺ = 436.05.
(5) At room temperature, compound 001-4 (110 mg, 0.25 mmol) was dissolved in 1,4-dioxane (3 mL), followed by the addition of M001 (212 mg, 1.01 mmol), bis(triphenylphosphine)palladium(II) chloride (18 mg, 0.03 mmol), potassium carbonate (70 mg, 0.56 mmol), triethylamine (77 mg, 0.76 mmol), copper(I) iodide (10 mg, 0.05 mmol), and cesium fluoride (154 mg, 1.01 mmol). The reaction mixture was purged with nitrogen, heated to 90°C under a nitrogen atmosphere, and stirred for 5 hours. The reaction mixture was cooled to room temperature, then diluted with water (10 mL), and extracted with ethyl acetate (10 mL). The organic phases were combined. The resulting organic phase was washed with saturated aqueous sodium chloride solution (10 mL), dried over anhydrous sodium sulfate, filtered, and the filtrate was concentrated under reduced pressure. The resulting residue was purified by silica gel column chromatography (petroleum ether/ethyl acetate = 3/1) to obtain compound 001-5 (100 mg). LC-MS [M+H]⁺=446.15.
(6) At room temperature, compound 001-5 (100 mg, 0.23 mmol) was dissolved in a mixed solvent of methanol (3 mL) and water (1 mL), and lithium hydroxide monohydrate (50 mg, 1.13 mmol) was added. The reaction mixture was heated to 50°C and stirred for 1 hour. The resulting reaction mixture was cooled to room temperature, adjusted to pH = 6 to 7 with dilute hydrochloric acid (1 M), and then concentrated under reduced pressure. The resulting residue was purified by high-performance liquid chromatography (0.1% hydrochloric acid) and lyophilized to obtain compound 001 (7.5 mg). LC-MS: [M+H]⁺ = 432.15.

¹H NMR (400 MHz, DMSO-*d*₆) δ 8.02 (s, 1H), 7.86 (d, *J* = 8.0 Hz, 1H), 7.75 (d, *J* = 8.0 Hz, 1H), 6.18 (s, 2H), 4.16 (s, 3H), 2.62 (s, 3H), 2.50 (s, 2H), 2.38 (s, 2H), 1.01 (s, 2H), 0.95 (s, 3H), 0.41 (d, *J* = 7.2 Hz, 2H), 0.12 (d, *J* = 4.4 Hz, 2H).

### Example 2: Preparation of (R)-2-(1-((6-(5-((((1-cyclopropylethyl)(methyl)carbamoyl)oxy)methyl)-1-methyl-1H-1,2,3-triazol-4-yl)pyridin-3-yl)ethynyl)cyclopropyl)acetic acid (compound 002)

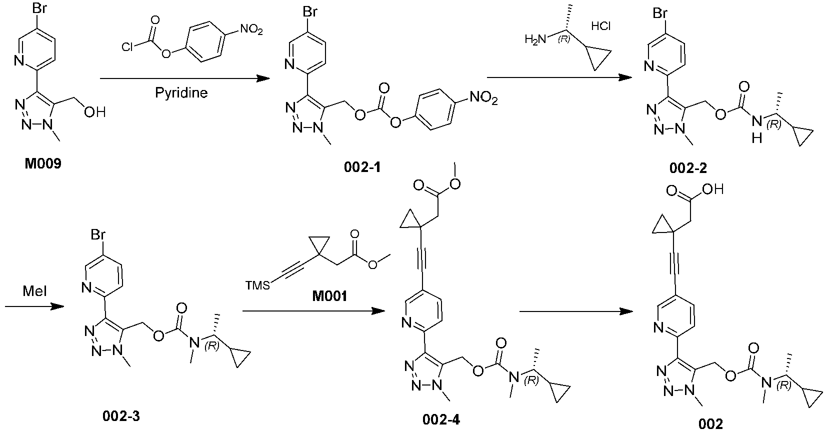

(1) At room temperature, compound M009 (2.0 g, 7.5 mmol) was dissolved in dichloromethane (20 mL), and the resulting solution was cooled to 0°C, followed by the addition of 4-nitrophenyl chloroformate (3.0 g, 14.9 mmol) and pyridine (1.2 g, 14.9 mmol). The reaction mixture was warmed to room temperature and stirred for 2 hours. The resulting reaction mixture was diluted with water (20 mL), extracted with dichloromethane (20 mL), and the organic phases were combined. The resulting organic phase was washed with saturated brine (40 mL), dried over anhydrous sodium sulfate, filtered, and the resulting filtrate was concentrated under reduced pressure to obtain compound 002-1 (3.8 g). LC-MS: [M+H]⁺ = 433.95.
(2) At room temperature, compound 002-1 (400 mg, 0.92 mmol) was dissolved in dichloromethane (2 mL), and *N*,*N*-diisopropylethylamine (178 mg, 1.38 mmol) and (R)-1-cyclopropylethylamine hydrochloride (140 mg, 1.15 mmol) were added, and then the reaction mixture was stirred at room temperature for 1 hour. The resulting reaction mixture was diluted with water (5 mL), extracted with dichloromethane (5 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 002-2 (290 mg) as a powder. LC-MS: [M+H]⁺ = 382.00.
(3) At room temperature, compound 002-2 (290 mg, 0.77 mmol) was dissolved in acetonitrile (3 mL), followed by the addition of potassium hydroxide (129 mg, 2.3 mmol) and iodomethane (327 mg, 2.3 mmol). The reaction mixture was stirred at room temperature for 3 hours. The resulting reaction mixture was filtered under reduced pressure, and the filter cake was washed with acetonitrile (15 mL). The resulting filtrate was concentrated under reduced pressure. The residue was purified by silica gel column chromatography (petroleum ether/ethyl acetate = 2/1) to obtain compound 002-3 (190 mg). LC-MS: [M+H]⁺ = 394.00.
(4) At room temperature, compound 002-3 (190 mg, 0.48 mmol) was dissolved in 1,4-dioxane (3 mL), followed by the addition of bis(triphenylphosphine)palladium(II) chloride (34 mg, 0.048 mmol), copper(I) iodide (9 mg, 0.048 mmol), M001 (80 mg, 0.58 mmol), and triethylamine (145 mg, 1.44 mmol). The reaction mixture was purged with nitrogen, heated to 80°C under a nitrogen atmosphere, and stirred for 1 hour. The resulting reaction mixture was cooled to room temperature, diluted with water (10 mL), and extracted twice with ethyl acetate (10 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 002-4 (170 mg). LC-MS: [M+H]⁺ = 452.15.
(5) At room temperature, compound 002-4 (260 mg, 0.58 mmol) was dissolved in a mixed solvent of methanol (2 mL) and tetrahydrofuran (2 mL), followed by the addition of a solution of lithium hydroxide monohydrate (73 mg, 1.73 mmol) in water (0.5 mL). The reaction mixture was heated to 40°C and stirred for 2 hours. The resulting reaction mixture was cooled to room temperature, adjusted to pH = 7 with dilute hydrochloric acid (1 M), and then concentrated under reduced pressure. The resulting residue was diluted with water (5 mL), and the solution was adjusted to pH = 5 with dilute hydrochloric acid (1 M). The mixture was then extracted with ethyl acetate (5 mL). The combined organic phases were dried over anhydrous sodium sulfate, filtered, and the filtrate was concentrated under reduced pressure. The residue was purified by preparative high-performance liquid chromatography (0.1% hydrochloric acid) and lyophilized to obtain compound 002 (65 mg). LC-MS: [M+H]⁺ = 438.15.

¹H NMR (400 MHz, DMSO-*d*₆) δ 8.55 (d, *J* = 1.6 Hz, 1H), 8.02 (d, *J* = 8.4 Hz, 1H), 7.84 (m, *J* = 8.4 1H), 5.64 - 5.59 (m, 2H), 4.10 (s, 3H), 3.11 (s, 1H), 2.74 - 2.71 (m, 3H), 2.45 (s, 2H), 1.06 (m, *J* = 6.4 5H), 0.94 (m, *J* = 6.8 3H), 0.53 - 0.15 (m, 4H).

### Example 3: Preparation of 2-(1-((6-(5-(((isobutyl(methyl)carbamoyl)oxy)methyl)-1-methyl-1H-1,2,3-triazol-4-yl)-2-methylpyridin-3-yl)ethynyl)cyclopropyl)acetic acid (compound 003)

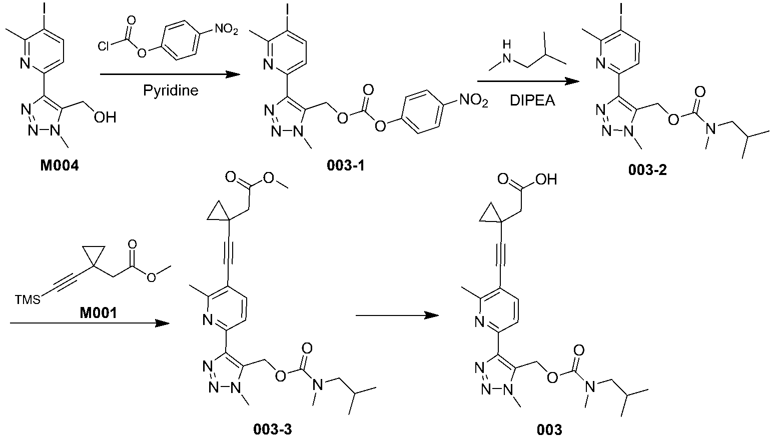

(1) At room temperature, M004 (1.17 g, 3.54 mmol) was dissolved in dichloromethane (10 mL). After cooling the resulting solution to 0°C, 4-nitrophenyl chloroformate (1.4 g, 7.08 mmol) and pyridine (560 mg, 7.08 mmol) were added. The reaction mixture was warmed to room temperature and stirred for 2 hours. The resulting reaction mixture was diluted with water (15 mL), extracted with dichloromethane (20 mL), and the organic phases were combined. The resulting organic phase was washed with saturated brine (30 mL), dried over anhydrous sodium sulfate, filtered, and the filtrate was concentrated under reduced pressure. The resulting residue was purified by silica gel column chromatography (petroleum ether/ethyl acetate = 1/1) to obtain compound 003-1 (1.4 g). LC-MS: [M+H]⁺ = 496.00.
(2) At room temperature, compound 003-1 (441 mg, 0.89 mmol), N-methylisobutylamine (93 mg, 1.07 mmol), and N,N-diisopropylethylamine (344 mg, 2.67 mmol) were dissolved in dichloromethane (8 mL). The reaction mixture was stirred at room temperature for 1 hour. The resulting reaction mixture was diluted with water (15 mL), extracted with dichloromethane (20 mL), and the organic phases were combined. The resulting organic phase was washed with saturated brine (30 mL), dried over anhydrous sodium sulfate, filtered, and the resulting filtrate was concentrated under reduced pressure to obtain compound 003-2 (350 mg, crude). LC-MS: [M+H]⁺ = 444.08.
(3) At room temperature, compound 003-2 (226 mg, 0.51 mmol), M001 (214 mg, 1.02 mmol), triethylamine (154 mg, 1.53 mmol), potassium carbonate (141 mg, 1.02 mmol), cesium fluoride (310 mg, 2.04 mmol), bis(triphenylphosphine)palladium(II) chloride (36 mg, 0.051 mmol), and copper(I) iodide (19 mg, 0.102 mmol) were dissolved in 1,4-dioxane (6 mL). Under a nitrogen atmosphere, the reaction mixture was heated to 90°C and stirred for an additional 4 hours. The resulting reaction mixture was added with water (10 mL) and extracted with ethyl acetate (20 mL). The organic phases were combined. The resulting organic phase was washed with saturated brine (30 mL), dried over anhydrous sodium sulfate, filtered, and the filtrate was concentrated under reduced pressure. The resulting residue was purified by silica gel column chromatography (petroleum ether/ethyl acetate = 1/1) to obtain compound 003-3 (150 mg). LC-MS: [M+H]⁺ = 454.20.
(4) At room temperature, compound 003-3 (150 mg, 0.33 mmol) was dissolved in a mixed solvent of methanol (6 mL) and water (2 mL), and lithium hydroxide monohydrate (42 mg, 0.99 mmol) was added. The reaction mixture was stirred at room temperature for 16 hours. The resulting reaction mixture was adjusted to pH = 5 to 6 with dilute hydrochloric acid (1 M) and then concentrated under reduced pressure. The resulting residue was purified by high-performance liquid chromatography (0.1% ammonia in water) and then lyophilized to obtain compound 003 (36.3 mg). LC-MS [M+H]⁺ = 440.15.

¹H NMR (400 MHz, DMSO-*d*₆) δ 12.18 (s, 1H), 7.84 (s, 1H), 7.73 (d, *J* = 4.0 Hz, 1H), 5.64 (d, *J* = 16.0 Hz, 2H), 4.11 (s, 3H), 3.01 - 3.00 (m, 1H), 2.90 - 2.89 (m, 1H), 2.76 (d, *J* = 20.0 Hz, 3H), 2.54 (s, 3H), 2.46 (s, 2H), 1.85 - 1.69 (m, 1H), 1.06 (s, 2H), 0.95 (s, 2H), 0.85 - 0.79 (m, 3H), 0.63 - 0.62 (m, 3H).

### Example 4: Preparation of 2-(1-((6-(5-(((butyl(methyl)carbamoyl)oxy)methyl)-1-methyl-1H-1,2,3-triazol-4-yl)-2-methylpyridin-3-yl)ethynyl)cyclopropyl)acetic acid (compound 004)

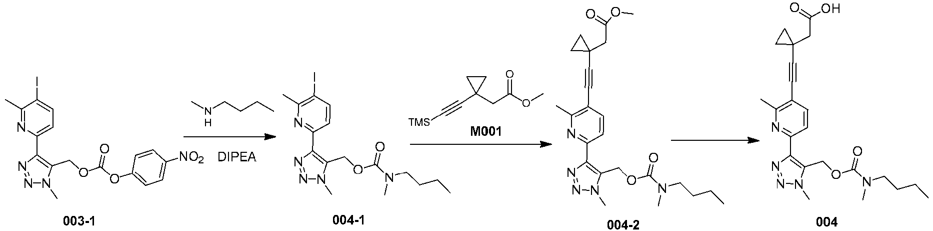

(1) At room temperature, compound 003-1 (555 mg, 1.12 mmol), N-methylbutylamine (107 mg, 1.23 mmol), and *N*,*N*-diisopropylethylamine (433 mg, 3.36 mmol) were dissolved in dichloromethane (10 mL). The reaction mixture was stirred at room temperature for 1 hour. The resulting reaction mixture was diluted with water (15 mL), extracted with dichloromethane (20 mL), and the organic phases were combined. The resulting organic phase was washed with saturated brine (30 mL), dried over anhydrous sodium sulfate, filtered, and the filtrate was concentrated under reduced pressure to obtain compound 004-1 (600 mg, crude). LC-MS: [M+H]⁺ =444.08.
(2) At room temperature, compound 004-1 (111 mg, 0.25 mmol), M001 (105 mg, 0.50 mmol), triethylamine (76 mg, 0.75 mmol), potassium carbonate (69 mg, 0.50 mmol), cesium fluoride (152 mg, 1.00 mmol), bis(triphenylphosphine)palladium(II) chloride (17 mg, 0.025 mmol), and copper(I) iodide (9.5 mg, 0.05 mmol) were dissolved in 1,4-dioxane (4 mL). Under a nitrogen atmosphere, the reaction mixture was heated to 90°C and stirred for 4 hours. The resulting reaction mixture was diluted with water (10 mL) and extracted with ethyl acetate (10 mL). The organic phases were combined, washed with saturated brine (30 mL), dried over anhydrous sodium sulfate, filtered, and the filtrate was concentrated under reduced pressure. The resulting residue was purified by silica gel column chromatography (petroleum ether/ethyl acetate = 1/1) to obtain compound 004-2 (100 mg). LC-MS: [M+H]⁺ = 454.15.
(3) At room temperature, compound 004-2 (100 mg, 0.22 mmol) was dissolved in a mixed solvent of methanol (4 mL) and water (1 mL), and lithium hydroxide monohydrate (28 mg, 0.66 mmol) was added. The reaction mixture was stirred at room temperature for 16 hours. The resulting reaction mixture was acidified with dilute hydrochloric acid (1 M) to pH = 6 to 7 and then concentrated under reduced pressure. The resulting residue was purified by high-performance liquid chromatography (0.1% ammonia in water conditions) and lyophilized to obtain compound 004 (10.6 mg). LC-MS [M+H]⁺ = 440.15.

¹H NMR (400 MHz, DMSO-*d*₆) δ 7.80 (d, *J* = 4.0 Hz, 1H), 7.69 (d, *J* = 4.0 Hz, 1H), 5.60 (d, *J* = 16.0 Hz, 2H), 4.08 (s, 3H), 3.14 (s, 1H), 3.02 (s, 1H), 2.71 (d, *J* = 20.0 Hz, 3H), 2.46 (s, 3H), 2.41 (s, 2H), 1.38 - 1.37 (m, 1H), 1.19 - 1.18 (m, 2H), 1.03 - 0.97 (m, 3H), 0.91 - 0.90 (m, 2H), 0.84 - 0.83 (m, 1H), 0.63 - 0.60 (m, 2H).

### Example 5: Preparation of (R)-2-(1-((6-(5-(((1-(2-chlorophenyl)ethoxy)carbonyl)amino)-1-methyl-1H-pyrazol-4-yl)-2-methylpyridin-3-yl)ethynyl)cyclopropyl)acetic acid (compound 005)

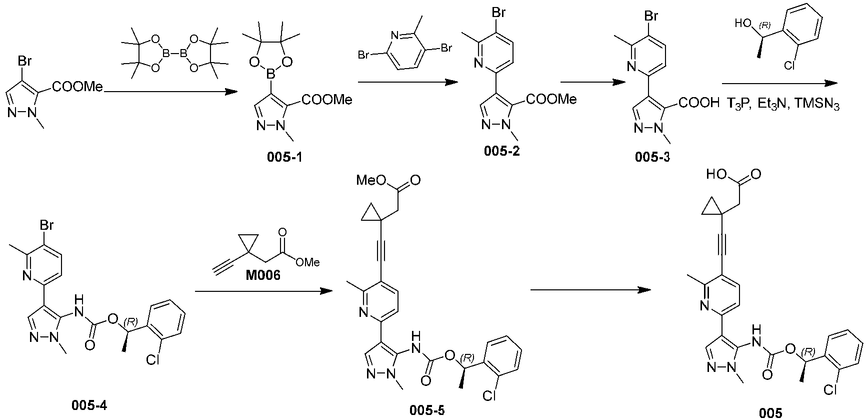

(1) At room temperature, methyl 4-bromo-1-methylpyrazole-5-carboxylate (2.2 g, 10 mmol) and bis(pinacolato)diboron (6.8 g, 27 mmol) were dissolved in 1,4-dioxane (60 mL), followed by the addition of potassium acetate (4.9 g, 50 mmol) and [1,1'-bis(diphenylphosphino)ferrocene]dichloropalladium(II) (1464 mg, 2 mmol). The reaction mixture was purged with nitrogen, heated to 90°C under a nitrogen atmosphere, and stirred for 4 hours. The resulting reaction mixture was then cooled to room temperature and concentrated under reduced pressure to obtain compound 005-1 (16 g, crude). LC-MS: [M+H]⁺ = 267.00.
(2) At room temperature, compound 005-1 (16 g, crude) and 2,5-dibromo-6-methylpyridine (5.0 g, 20 mmol) were dissolved in a mixed solvent of 1,4-dioxane (60 mL) and water (15 mL). Sodium carbonate (3.2 g, 30 mmol) and [1,1'-bis(diphenylphosphino)ferrocene]dichloropalladium(II) (732 mg, 1 mmol) were added. The reaction mixture was purged with nitrogen, heated to 100°C under a nitrogen atmosphere, and stirred for 1.5 hours. The reaction mixture was then cooled to room temperature, diluted with water (100 mL), and extracted with ethyl acetate (50 mL). The organic phases were combined. The resulting organic phase was washed with saturated brine (60 mL), dried over anhydrous sodium sulfate, filtered, and the filtrate was concentrated under reduced pressure to obtain compound 005-2 (4.5 g, crude). LC-MS: [M+H]⁺ = 311.90.
(3) At room temperature, compound 005-2 (4.5 g, crude) was dissolved in a mixed solvent of methanol (45 mL) and water (15 mL), followed by the addition of lithium hydroxide monohydrate (2.1 g, 50 mmol). The reaction mixture was heated to 60°C and stirred for 1 hour. The resulting reaction mixture was then cooled to room temperature and concentrated under reduced pressure. The resulting residue was diluted with water (50 mL), extracted with ethyl acetate (50 mL), and the aqueous phases were combined. The resulting aqueous phase was adjusted to pH = 3 with dilute hydrochloric acid (1 M), filtered, and the filter cake was washed with water (20 mL). The resulting filter cake was dried under reduced pressure to obtain compound 005-3 (888 mg). LC-MS: [M+H]⁺ = 295.85.
(4) At room temperature, compound 005-3 (888 mg, 3 mmol) was dissolved in anhydrous tetrahydrofuran (15 mL), followed by the addition of propylphosphonic anhydride (2.9 g, 4.5 mmol, 50% in ethyl acetate), azidotrimethylsilane (518 mg, 4.5 mmol), and triethylamine (606 mg, 6 mmol). The reaction mixture was stirred at room temperature for 0.5 hours, then (*R*)-1-(2-chlorophenyl)ethanol (707 mg, 4.5 mmol) was added. The reaction mixture was heated to 70°C and stirred for an additional 1 hour. The resulting reaction mixture was cooled to room temperature and then concentrated under reduced pressure. The resulting residue was purified by silica gel column chromatography (petroleum ether/ethyl acetate = 1/1) to obtain compound 005-4 (800 mg). LC-MS: [M+H]⁺ =450.75.
(5) At room temperature, compound 005-4 (450 mg, 1 mmol) and M006 (276 mg, 2 mmol) were dissolved in 1,4-dioxane (6 mL), followed by the addition of triethylamine (240 mg, 2.4 mmol), copper(I) iodide (42 mg, 0.2 mmol), bis(triphenylphosphine)palladium(II) chloride (70 mg, 0.1 mmol), and potassium carbonate (276 mg, 2 mmol). The reaction mixture was heated to 100°C and stirred for 16 hours. The resulting reaction mixture was cooled to room temperature and then concentrated under reduced pressure. The resulting residue was purified by silica gel column chromatography (petroleum ether/ethyl acetate = 2/1) to obtain compound 005-5 (102 mg). LC-MS: [M+H]⁺=507.00.
(6) At room temperature, compound 005-5 (102 mg, 0.2 mmol) was dissolved in a mixed solvent of methanol (9 mL) and water (3 mL), and lithium hydroxide monohydrate (42 mg, 1 mmol) was added. The reaction mixture was heated to 60°C and stirred for 1 hour. The resulting reaction mixture was cooled to room temperature, adjusted to pH = 3 with dilute hydrochloric acid (1 M), and then concentrated under reduced pressure. The resulting residue was purified by high-performance liquid chromatography (0.1% hydrochloric acid conditions) and lyophilized to obtain compound 005 (60.1 mg). LC-MS: [M+H]⁺ = 492.95.

¹H NMR (400 MHz, *CD₃OD*) δ 8.08 (d, *J* = 8.0 Hz, 1H), 7.99(s, 1H), 7.62 - 7.57 (m, 1H), 7.50 - 7.25 (m, 4H), 6.06 - 6.01 (m, 1H), 3.76 (s, 3H), 2.70 (s, 3H), 2.50 (s, 2H), 1.49(s, 3H), 1.17 - 1.14 (m, 2H), 1.02 - 0.99 (m, 2H).

### Example 6: Preparation of (R)-2-(1-((6-(5-(((1-(2-chlorophenyl)ethoxy)carbonyl)amino)-4-methyl-1H-1,2,3-triazol-1-yl)-2-methylpyridin-3-yl)ethynyl)cyclopropyl)acetic acid (compound 006)

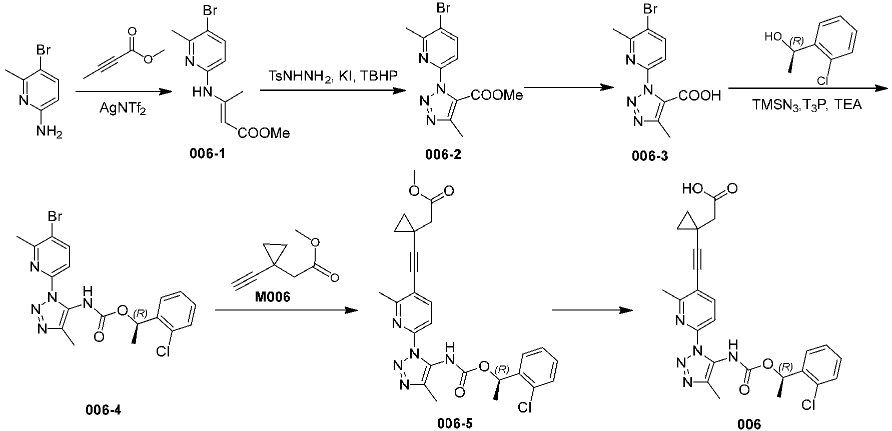

(1) At room temperature, 2-amino-5-bromo-6-methylpyridine (15.0 g, 80.19 mmol) was dissolved in toluene (150 mL), followed by the addition of methyl 2-butynoate (7.9 g, 80.19 mmol) and silver bis(trifluoromethanesulfonyl)imide (3.1 g, 8.02 mmol). The reaction mixture was heated to 70°C and stirred for an additional 20 hours. After cooling to room temperature, the resulting reaction mixture was directly concentrated under reduced pressure. The resulting residue was purified by silica gel column chromatography (petroleum ether/ethyl acetate = 10/1) to obtain compound 006-1 (7.0 g, crude). LC-MS: [M+H]⁺ = 286.90.
(2) At room temperature, compound 006-1 (7.0 g, crude) was dissolved in dimethyl sulfoxide (70 mL), followed by the addition of 4-methylbenzenesulfonhydrazide (5.5 g, 29.46 mmol) and potassium iodide (4.0 g, 24.55 mmol). Then, *tert*-butyl hydroperoxide (6.3 g, 49.10 mmol, 70% in water) was slowly added dropwise. After the dropwise addition was completed, the reaction mixture was heated to 60°C and stirred for an additional 16 hours. The resulting reaction mixture was cooled to room temperature, then diluted with water (100 mL), and extracted with ethyl acetate (100 mL). The organic phases were combined. The resulting organic phase was washed with saturated aqueous sodium chloride solution (100 mL), dried over anhydrous sodium sulfate, filtered, and the filtrate was concentrated under reduced pressure to obtain compound 006-2 (7.5 g, crude). LC-MS: [M+H]⁺ = 310.85.
(3) At room temperature, compound 006-2 (7.5 g, crude) was dissolved in a mixed solvent of methanol (45 mL) and water (15 mL), and lithium hydroxide monohydrate (2.9 g, 120.52 mmol) was added. The reaction mixture was heated to 60°C and stirred for 2 hours. The resulting reaction mixture was then cooled to room temperature and diluted with water (50 mL). The resulting solution was adjusted to pH = 12 with aqueous sodium hydroxide solution (1 M) and then extracted with ethyl acetate (100 mL). The aqueous phases were combined. The resulting aqueous phase was acidified with dilute hydrochloric acid (1 M) (pH = 2 to 3), filtered, and the filter cake was washed with water (50 mL) and dried under reduced pressure to obtain compound 006-3 (2.4 g). LC-MS: [M+H]⁺ = 296.85.
(4) At room temperature, compound 006-3 (2.4 g, 8.08 mmol) was dissolved in THF (20 mL), followed by the addition of propylphosphonic anhydride (7.7 g, 12.12 mmol, 50% in ethyl acetate), triethylamine (1.6 g, 16.16 mmol), and azidotrimethylsilane (1.4 g, 12.12 mmol). The reaction mixture was stirred at room temperature for 0.5 hours, then (R)-1-(2-chlorophenyl)ethanol (6.3 g, 40.40 mmol) was added. The resulting reaction mixture was heated to 90°C and stirred for an additional 1 hour. The reaction mixture was then cooled to room temperature and concentrated under reduced pressure. The resulting residue was purified by silica gel column chromatography (petroleum ether/ethyl acetate = 3/1) to obtain compound 006-4 (2.6 g). LC-MS [M+H]⁺=451.85.
(5) At room temperature, compound 006-4 (250 mg, 0.56 mmol) and M006 (154 mg, 1.12 mmol) were dissolved in 1,4-dioxane (5 mL), followed by the addition of bis(triphenylphosphine)palladium(II) chloride (42 mg, 0.06 mmol), triethylamine (136 mg, 1.35 mmol), potassium carbonate (154 mg, 1.12 mmol), and copper(I) iodide (21 mg, 0.11 mmol). Under a nitrogen atmosphere, the reaction mixture was heated to 90°C and stirred for 16 hours. After cooling to room temperature, the resulting reaction mixture was concentrated under reduced pressure. The resulting residue was purified by silica gel column chromatography (petroleum ether/ethyl acetate = 3/1) to obtain compound 006-5 (90 mg). LC-MS [M+H]⁺ = 508.00.
(6) At room temperature, compound 006-5 (90 mg, 0.18 mmol) was dissolved in a mixed solvent of methanol (3 mL) and water (1 mL), and lithium hydroxide monohydrate (21 mg, 0.89 mmol) was added. The reaction mixture was heated to 60°C and stirred for an additional 1 hour. The resulting reaction mixture was cooled to room temperature, adjusted to pH = 6 to 7 with dilute hydrochloric acid (1 M), and then concentrated under reduced pressure. The resulting residue was purified by high-performance liquid chromatography (0.1% hydrochloric acid conditions) and lyophilized to obtain compound 006 (39.8 mg). LC-MS: [M+H]⁺ = 493.95.

¹H NMR (400 MHz, *CD₃OD*) δ 7.85 (d, *J* = 8.0 Hz, 1H), 7.65 (d, *J* = 8.4 Hz, 1H), 7.42 - 7.32 (m, 4H), 6.13 (q, *J* = 6.4 Hz, 1H), 2.62 (s, 3H), 2.57 (s, 2H), 2.32 (s, 3H), 1.53 (s, 3H), 1.20 (dd, *J* = 6.8, 4.4 Hz, 2H), 1.06 (dd, *J* = 6.8, 4.4 Hz, 2H).

### Example 7: Preparation of ethyl trans-(R)-1-(2-fluorophenyl)(4-(5-((2-hydroxycyclohexyl)ethynyl)-6-methylpyridin-2-yl)-1-methyl-1H-1,2,3-triazol-5-yl)carbamate (compound 007)

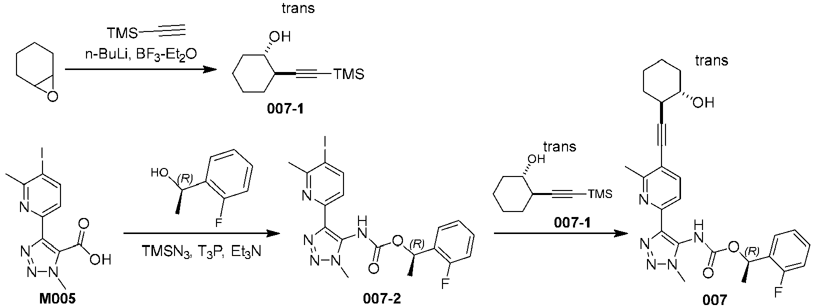

(1) At room temperature, trimethylsilylacetylene (21.5 mL, 152.8 mmol) was dissolved in tetrahydrofuran (200 mL). Under a nitrogen atmosphere, the reaction mixture was cooled to -78°C, and n-butyllithium solution (61.2 mL, 152.8 mmol, 2.5 M) was slowly added dropwise. The resulting reaction mixture was stirred at -78°C for 10 minutes, followed by the dropwise addition of boron trifluoride diethyl etherate (25.2 mL, 204.8 mmol). The reaction mixture was stirred at -78°C for an additional 10 minutes. Then, cyclohexene oxide (10 g, 101.9 mmol) was added dropwise. After the dropwise addition was completed, the reaction mixture was stirred at -78°C for an additional 3 hours. The resulting reaction mixture was poured into saturated ammonium chloride solution (200 mL) to quench, extracted with dichloromethane (100 mL), and the combined organic phases were washed with saturated sodium chloride solution (100 mL), dried over anhydrous sodium sulfate, filtered, and the filtrate was concentrated under reduced pressure. The resulting residue was purified by silica gel column chromatography (petroleum ether/ethyl acetate = 20/1) to obtain compound 007-1 (3.5 g, crude, containing a pair of trans configurations).
(2) At room temperature, M005 (1.4 g, 4.1 mmol) was dissolved in tetrahydrofuran (25 mL), followed by the addition of azidotrimethylsilane (0.8 mL, 6.1 mmol), propylphosphonic anhydride (3.2 mL, 6.1 mmol, 50% in ethyl acetate), and triethylamine (1.2 mL, 8.1 mmol). The reaction mixture was stirred at room temperature for 1 hour. Then, (*R*)-1-(2-fluorophenyl)ethanol (0.85 g, 6.1 mmol) was added. The reaction mixture was heated to 70°C and stirred for an additional 1 hour. The resulting reaction mixture was cooled to room temperature and directly concentrated under reduced pressure. The resulting residue was purified by silica gel column chromatography (petroleum ether/ethyl acetate = 2/1) to obtain compound 007-2 (1.5 g). LC-MS: [M+H]⁺ = 481.95.
(3) At room temperature, compound 007-1 (653 mg, 3.3 mmol) and compound 007-2 (400 mg, 0.83 mmol) were dissolved in 1,4-dioxane (8 mL), followed by the addition of potassium carbonate (230 mg, 1.7 mmol), bis(triphenylphosphine)palladium(II) chloride (58 mg, 83 µmol), copper(I) iodide (16 mg, 83 µmol), triethylamine (421 mg, 4.2 mmol), and cesium fluoride (505 mg, 3.3 mmol). Under a nitrogen atmosphere, the reaction mixture was heated to 80°C and stirred for an additional 1 hour. The resulting reaction mixture was then cooled to room temperature and directly concentrated under reduced pressure. The resulting residue was purified by high-performance liquid chromatography (alkaline conditions) and lyophilized to obtain compound 007 (230 mg, containing a pair of trans configurations). LC-MS: [M+H]⁺ =478.15.

¹H NMR(400 MHz, DMSO-*d*₆) δ 9.71 (s, 1H), 7.74 (s, 2H), 7.35 (s, 1H), 7.18 (s, 2H), 5.93 (s, 1H), 3.88 (s, 3H), 3.52-3.47 (m, 2H), 3.40 (s, 2H), 2.47 (s, 3H), 2.00 (d, *J* = 9.6 Hz, 1H), 1.87 (d, *J* = 8.8 Hz, 1H), 1.66 - 1.61 (m, 3H), 1.51 - 1.40 (m, 2H), 1.32 - 1.23 (m, 4H).

### Example 8: Preparation of 2-(1-((6-(5-(((1-cyclopropylethoxy)carbonyl)amino)-1-methyl-1H-1,2,3-triazol-4-yl)-2-methylpyridin-3-yl)ethynyl)cyclopropyl)acetic acid (compound 008)

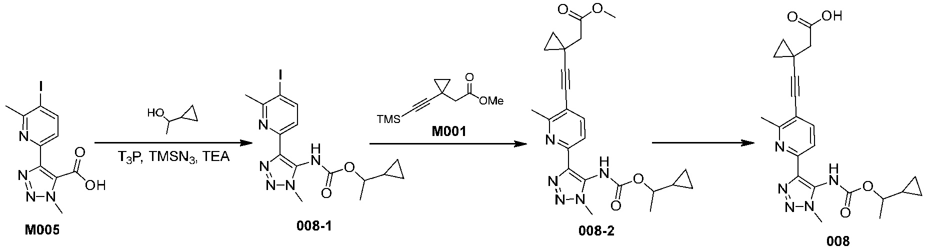

(1) At room temperature, M005 (1.27 g, 3.7 mmol) was dissolved in 1,4-dioxane (20 mL), followed by the addition of propylphosphonic anhydride (4.7 g, 7.4 mmol, 50% in ethyl acetate), triethylamine (1.1 g, 11.1 mmol), and azidotrimethylsilane (1.3 g, 11.1 mmol). The reaction mixture was heated to 60°C and stirred for 1 hour. The reaction mixture was cooled to room temperature, and 1-cyclopropylethanol (636 mg, 7.4 mmol) was added. The reaction mixture was then reheated to 70°C and stirred for an additional 1 hour. The resulting reaction mixture was cooled to room temperature, diluted with water (20 mL), and extracted with ethyl acetate (10 mL). The organic phases were combined. The resulting organic phase was washed with saturated brine (20 mL), dried over anhydrous sodium sulfate, filtered, and the filtrate was concentrated under reduced pressure. The resulting residue was purified by silica gel column chromatography (petroleum ether/ethyl acetate = 5/1) to obtain compound 008-1 (382 mg). LC-MS: [M+H]⁺ = 428.05.
(2) At room temperature, compound 008-1 (363 mg, 0.85 mmol) was dissolved in 1,4-dioxane (6 mL), followed by the addition of bis(triphenylphosphine)palladium(II) chloride (60 mg, 0.085 mmol), copper(I) iodide (16 mg, 0.085 mmol), M001 (210 mg, 1.0 mmol), cesium fluoride (517 mg, 3.4 mmol), and triethylamine (258 mg, 2.55 mmol). Under a nitrogen atmosphere, the reaction mixture was heated to 90°C and stirred for 3 hours. After cooling to room temperature, the reaction mixture was directly filtered under reduced pressure, and the filtrate was concentrated under reduced pressure. The resulting residue was purified by silica gel column chromatography (petroleum ether/ethyl acetate = 2/1) to obtain compound 008-2 (240 mg). LC-MS: [M+H]⁺ = 438.10.
(3) At room temperature, compound 008-2 (240 mg, 0.55 mmol) was dissolved in a mixed solvent of methanol (2 mL) and tetrahydrofuran (2 mL), followed by the addition of a solution of lithium hydroxide monohydrate (69 mg, 1.65 mmol) in water (0.5 mL). The reaction mixture was heated to 40°C and stirred for 2 hours. The resulting reaction mixture was adjusted to pH = 7 with dilute hydrochloric acid (1 M) and directly concentrated under reduced pressure. The resulting residue was diluted with ethyl acetate (10 mL), washed with saturated aqueous sodium carbonate solution (5 mL), and the aqueous phases were combined. The resulting aqueous phase was adjusted to pH = 5 with dilute hydrochloric acid (1 M), then extracted with ethyl acetate (10 mL), and the organic phases were combined. The resulting organic phase was washed with saturated brine (10 mL), dried over anhydrous sodium sulfate, filtered, and the filtrate was concentrated under reduced pressure. The resulting residue was purified by high-performance liquid chromatography (neutral conditions with ammonium bicarbonate) and lyophilized to obtain compound 008 (110 mg). LC-MS: [M+H]⁺ = 424.15.

¹H NMR (400 MHz, DMSO-*d*₆) δ 9.47 (s, 1H), 7.72 - 7.67 (m, 3H), 4.26 - 4.06 (m, 2H), 3.88 (s, 5H), 2.52 (s, 4H), 2.29 (s, 3H), 1.21 (s, 4H), 0.94 (s, 8H), 0.46 (s, 3H), 0.26 (s, 2H).

### Example 9: Preparation of 2-(1-((6-(5-(((((1-fluorocyclobutyl)methyl)(methyl)carbamoyl)oxy)methyl)-1-methyl-1H-1,2,3-triazol-4-yl)-2-methylpyridin-3-yl)ethynyl)cyclopropyl)acetic acid (compound 009)

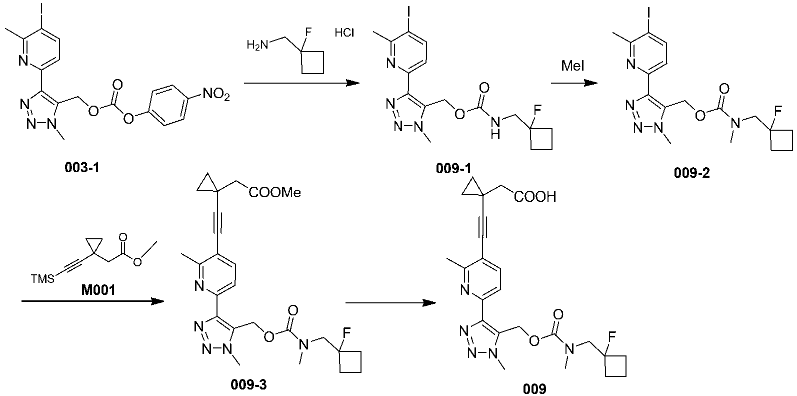

(1) At room temperature, compound 003-1 (555 mg, 1.12 mmol) was dissolved in dichloromethane (5 mL), followed by the addition of *N*,*N*-diisopropylethylamine (720 mg, 5.58 mmol) and (1-fluorocyclobutyl)methylamine hydrochloride (185 mg, 1.34 mmol). The reaction mixture was stirred at room temperature for 1 hour. The reaction mixture was diluted with water (5 mL), extracted with dichloromethane (5 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 5/1) to obtain compound 009-1 (450 mg). LC-MS: [M+H]⁺ = 460.06.
(2) At room temperature, compound 009-1 (445 mg, 0.97 mmol) was dissolved in anhydrous tetrahydrofuran (8 mL). After cooling the reaction mixture to 0°C, sodium hydride (77.61 mg, 1.94 mmol, 60% dispersed in mineral oil) was added. The resulting reaction mixture was warmed to room temperature and stirred for 0.5 hours. Iodomethane (413 mg, 2.91 mmol) was added. The resulting reaction mixture was heated to 40°C and stirred for 2 hours. After cooling the resulting reaction mixture to room temperature, saturated ammonium chloride solution (10 mL) was added to quench, followed by extraction with ethyl acetate (10 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 009-2 (400 mg). LC-MS: [M+H]⁺ = 474.07.
(3) At room temperature, compound 009-2 (332 mg, 0.70 mmol) was dissolved in 1,4-dioxane (5 mL), followed by the addition of bis(triphenylphosphine)palladium(II) chloride (50 mg, 0.070 mmol), copper(I) iodide (27 mg, 0.14 mmol), M001 (300 mg, 1.41 mmol), cesium fluoride (213 mg, 1.41 mmol), potassium carbonate (194 mg, 1.41 mmol), and triethylamine (178 mg, 1.76 mmol). The reaction mixture was purged with nitrogen, heated to 90°C under a nitrogen atmosphere, and stirred for 5 hours. The reaction mixture was then cooled to room temperature, diluted with water (10 mL), and extracted twice with ethyl acetate (10 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 009-3 (200 mg). LC-MS: [M+H]⁺ =484.20.
(4) At room temperature, compound 009-3 (180 mg, 0.37 mmol) was dissolved in a mixed solvent of methanol (3 mL) and water (1 mL), and lithium hydroxide monohydrate (80 mg, 1.86 mmol) was added. The reaction mixture was stirred at room temperature for 2 hours. The resulting reaction mixture was adjusted to pH = 7 with dilute hydrochloric acid (1 M) and then concentrated under reduced pressure. The resulting residue was purified by preparative high-performance liquid chromatography (acetonitrile/water = 2/5) and lyophilized to obtain compound 009 (61.5 mg). LC-MS: [M+H]⁺ =470.10.

¹H NMR (400 MHz, DMSO-*d*₆)δ 7.81 (s, 1H), 7.70 (d, *J* = 8.0 Hz, 1H), 5.68 (d, *J* = 10.0 Hz, 2H), 4.12 (s, 3H), 3.60 - 3.42 (m, 2H), 2.82 (d, *J* = 23.2 Hz, 3H), 2.53 (s, 3H), 2.26 (s, 2H), 2.16 - 1.82 (m, 4H), 1.50 - 1.39 (m, 1H), 0.98 - 0.89 (m, 5H).

### Example 10: Preparation of 2-(1-((2-methyl-6-(1-methyl-5-(((pentan-2-yloxy)carbonyl)amino)-1H-1,2,3-triazol-4-yl)pyridin-3-yl)ethynyl)cyclopropyl)acetic acid (compound 010)

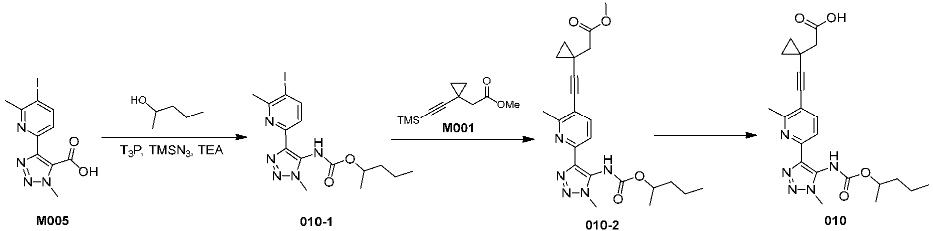

(1) At room temperature, M005 (0.4 g, 1.16 mmol) was dissolved in tetrahydrofuran (8 mL), followed by the addition of azidotrimethylsilane (268 mg, 2.3 mmol), propylphosphonic anhydride (1 mL, 3.5 mmol, 50% in ethyl acetate), and triethylamine (353 mg, 3.5 mmol). The reaction mixture was stirred at room temperature for 1 hour. Then, 2-pentanol (512 mg, 5.8 mmol) was added, and the reaction mixture was heated to 70°C and stirred for an additional 2 hours. After cooling to room temperature, the resulting reaction mixture was directly concentrated under reduced pressure. The resulting residue was purified by silica gel column chromatography (petroleum ether/ethyl acetate = 1/1) to obtain compound 010-1 (489 mg). LC-MS: [M+H]⁺ =430.00.
(2) At room temperature, compound 010-1 (430 mg, 1.0 mmol) was dissolved in 1,4-dioxane (8 mL), followed by the addition of M001 (422 mg, 2.0 mmol), potassium carbonate (277 mg, 2.0 mmol), copper(I) iodide (20 mg, 0.1 mmol), triethylamine (507 mg, 5.0 mmol), cesium fluoride (609 mg, 4.0 mmol), and bis(triphenylphosphine)palladium(II) chloride (71 mg, 0.1 mmol). After nitrogen purging, the reaction mixture was heated to 80°C under a nitrogen atmosphere and stirred for an additional 4 hours. After cooling to room temperature, the resulting reaction mixture was directly concentrated under reduced pressure, and the residue was purified by silica gel column chromatography (petroleum ether/ethyl acetate = 3/1) to obtain compound 010-2 (440 mg). LC-MS: [M+H]⁺ =440.15.
(3) At room temperature, compound 010-2 (420 mg, 0.96 mmol) was dissolved in a mixed solvent of methanol (6 mL) and water (2 mL), followed by the addition of lithium hydroxide monohydrate (200 mg, 8.3 mmol). The reaction mixture was stirred at room temperature for 2 hours. The resulting reaction mixture was adjusted to pH = 6 to 7 with dilute hydrochloric acid (1 M) and then directly concentrated under reduced pressure. The resulting residue was purified by high-performance liquid chromatography (alkaline conditions) and lyophilized to obtain compound 010 (80.3 mg). LC-MS: [M+H]⁺ =426.10.

¹H NMR (400 MHz, DMSO-*d*₆) δ12.21 (s, 1H), 9.37 (s, 1H), 7.73 - 7.67 (m, 2H), 4.69 (s, 1H), 3.86 (s, 3H), 3.32 (s, 3H), 2.49 (s, 1H), 2.43 (s, 2H), 1.59 - 1.09 (m, 6H), 1.03 (s, 2H), 0.94 (s, 2H), 0.82 (s, 3H).

### Example 11: Preparation of (R)-2-(1-((6-(5-((sec-butoxycarbonyl)amino)-1-methyl-1H-1,2,3-triazol-4-yl)-2-methylpyridin-3-yl)ethynyl)cyclopropyl)acetic acid (compound 011)

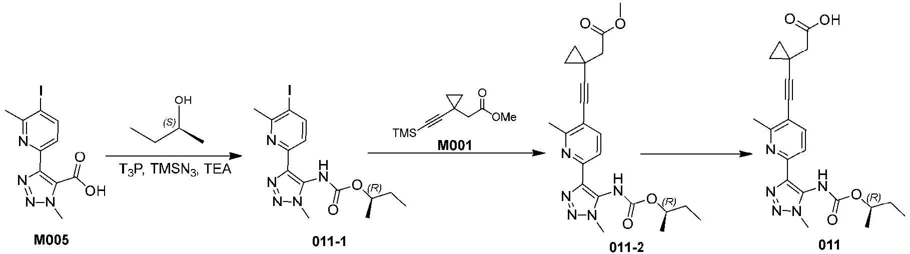

The preparation of compound 011 was referred to the method for preparing compound 008 in Example 8, wherein the starting material was replaced from 1-cyclopropylethanol to (*S*)-(+)-2-butanol, and compound 011 (78.2 mg) was obtained.

LC-MS: [M+H]⁺ = 412.15.

¹H NMR (400 MHz, DMSO-*d*₆) δ 9.41 (s, 1H), 7.75 - 7.68 (m, 3H), 4.63 (s, 1H), 3.88 (s, 4H), 2.51 (s, 3H), 2.44 (s, 2H), 1.51 (s, 1H), 1.15 (s, 1H), 1.05 (m, 2H), 1.04 - 0.97 (s, 6H), 0.96 - 0.95 (s, 3H), 0.83 (s, 2H).

### Example 12: Preparation of 2-(1-((6-(5-(((ethyl(propyl)carbamoyl)oxy)methyl)-1-methyl-1H-1,2,3-triazol-4-yl)-2-methylpyridin-3-yl)ethynyl)cyclopropyl)acetic acid (compound 012)

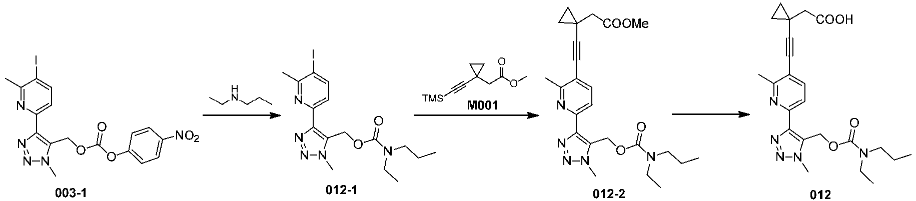

The preparation of compound 012 was referred to the method for preparing compound 004 in Example 4, wherein the starting material was replaced from *N*-methylbutylamine to N-ethyl-n-propylamine, and compound 012 (67.1 mg) was obtained. LC-MS: [M+H]⁺=440.15.

¹H NMR (400 MHz, DMSO-*d*₆) δ 7.81 (d, *J* = 8.0 Hz, 1H), 7.69 (d, *J* = 8.0 Hz, 1H), 5.60 (s, 2H), 4.09 (s, 3H), 3.15 - 2.98 m, 4H), 2.51 (s, 3H), 2.39 (s, 2H), 1.42 - 1.27 (m, 2H), 1.03 - 0.57 (m, 10H).

### Example 13: Preparation of (R)-2-(1-((6-(5-(((1-(2-chlorophenyl)ethoxy)carbonyl)amino)-1-methyl-1H-1,2,3-triazol-4-yl)-2-methylpyridin-3-yl)ethynyl)cyclopropyl)acetic acid (compound 013)

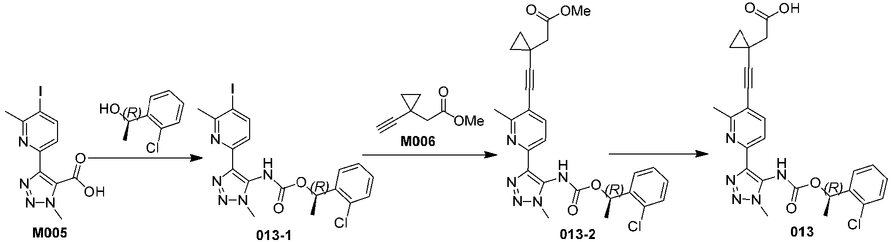

(1) At room temperature, M005 (900 mg, 2.62 mmol) was dissolved in anhydrous tetrahydrofuran (20 mL), followed by the addition of propylphosphonic anhydride (2.5 g, 3.92 mmol, 50% in ethyl acetate), azidotrimethylsilane (1.5 g, 13.08 mmol), and triethylamine (529 mg, 6 mmol). The reaction mixture was stirred at room temperature for 0.5 hours, then (*R*)-1-(2-chlorophenyl)ethan-1-ol (1.6 g, 10.46 mmol) was added. The reaction mixture was heated to 70°C and stirred for an additional 1 hour. After cooling to room temperature, the resulting reaction mixture was poured into water (50 mL) to dilute and extracted with ethyl acetate (50 mL). The combined organic phases were dried over anhydrous sodium sulfate, filtered, and the filtrate was concentrated under reduced pressure. The resulting residue was purified by silica gel column chromatography (system: petroleum ether/ethyl acetate = 1/1) to obtain compound 013-1 (1.1 g). LC-MS: [M+H]⁺ =498.01.
(2) At room temperature, compound 013-1 (249 mg, 0.5 mmol) and M006 (138 mg, 1 mmol) were dissolved in 1,4-dioxane (6 mL), followed by the addition of triethylamine (120 mg, 1.2 mmol), bis(triphenylphosphine)palladium(II) chloride (35 mg, 0.05 mmol), potassium carbonate (138 mg, 1 mmol), and copper(I) iodide (10 mg, 0.085 mmol). Under a nitrogen atmosphere, the reaction mixture was heated to 100°C and stirred for 16 hours. After cooling to room temperature, the resulting reaction mixture was directly concentrated under reduced pressure. The residue was purified by silica gel column chromatography (petroleum ether/ethyl acetate = 2/1) to obtain compound 013-2 (102 mg). LC-MS: [M+H]⁺=507.95.
(3) At room temperature, compound 013-2 (102 mg, 0.2 mmol) was dissolved in a mixed solvent of methanol (9 mL) and water (3 mL), and lithium hydroxide monohydrate (42 mg, 1 mmol) was added. The reaction mixture was heated to 60°C and stirred for 1 hour. After the resulting reaction mixture cooled to room temperature, the pH was adjusted to 3 with dilute hydrochloric acid (1 M). The resulting reaction mixture was directly concentrated under reduced pressure, and the residue was purified by high-performance liquid chromatography (0.1% hydrochloric acid conditions) and lyophilized to obtain compound 013 (20.4 mg). LC-MS: [M+H]⁺ = 494.00.

¹H NMR (400 MHz, *CD₃OD*): δ 8.01 (d, *J* = 8.0 Hz, 1H), 7.72 (d, *J* = 8.0 Hz, 1H), 7.54 - 7.30 (m, 4H), 6.18 - 6.13 (m, 1H), 4.01 (s, 3H), 2.74 (s, 3H), 2.55 (s, 2H), 1.57 (s, 3H), 1.21 - 1.18 (m, 2H), 1.06 - 1.03 (m, 2H).

### Example 14: Preparation of (R)-2-(1-((6-(5-(((1-(2-fluorophenyl)ethoxy)carbonyl)amino)-1-methyl-1H-1,2,3-triazol-4-yl)-2-methylpyridin-3-yl)ethynyl)cyclopropyl)acetic acid (compound 014)

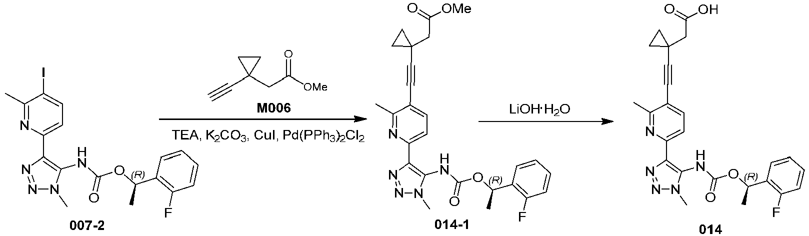

The preparation of compound 014 was referred to the method for preparing compound 013 in Example 13, wherein compound 013-1 was replaced with compound 007-2, and compound 014 (60.0 mg) was obtained. LC-MS: [M+H]⁺ =478.10.

¹H NMR(400 MHz, DMSO-*d*₆) δ 7.70 - 7.64(m, 2H), 7.31(d, *J* = 8.0 Hz, 2H), 7.12 - 7.04(m, 2H), 6.07(d, *J* = 6.4 Hz, 1H), 3.97(s, 3H), 2.55(s, 3H), 2.51(s, 2H), 1.56(s, 3H), 1.14 - 1.01(m, 2H), 0.99 - 0.97(m, 2H).

### Example 15: Preparation of 2-(1-((6-(5-(((1-(2-chloropyridin-3-yl)ethoxy)carbonyl)amino)-1-methyl-1H-1,2,3-triazol-4-yl)-2-methylpyridin-3-yl)ethynyl)cyclopropyl)acetic acid (compound 015)

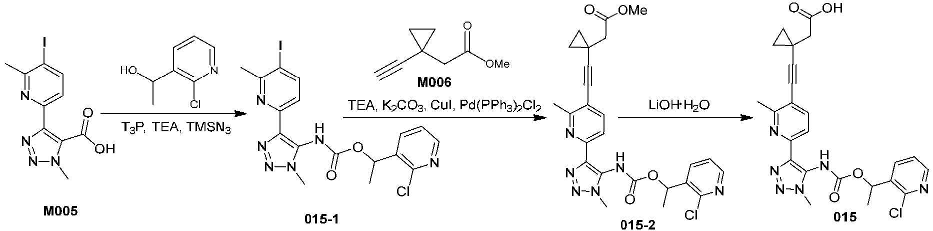

The preparation of compound 015 was referred to the method for preparing compound 013 in Example 13, wherein the starting material (*R*)-1-(2-chlorophenyl)ethan-1-ol was replaced with 1-(2-chloropyridin-3-yl)ethan-1-ol, and compound 015 (46.0 mg) was obtained. LC-MS: [M+H]⁺ =495.05.

¹H NMR (400 MHz, DMSO-*d*₆) δ 12.28 (s, 1H), 9.79 (s, 1H), 8.36 (s, 1H), 8.01 (s, 1H), 7.74 (d, *J* = 8.0 Hz, 1H), 7.66 (d, *J* = 8.0 Hz, 1H), 7.49 (s, 1H), 5.91 (s, 1H), 3.89 (s, 3H), 2.44 (d, *J* = 9.6 Hz, 5H), 1.56 (s, 3H), 1.09 - 1.04 (m, 2H), 1.02 - 0.87 (m, 2H).

### Example 16: Preparation of 2-(1-((2-methyl-6-(1-methyl-5-(((methyl(propyl)carbamoyl)oxy)methyl)-1H-1,2,3-triazol-4-yl)pyridin-3-yl)ethynyl)cyclopropyl)acetic acid (compound 016)

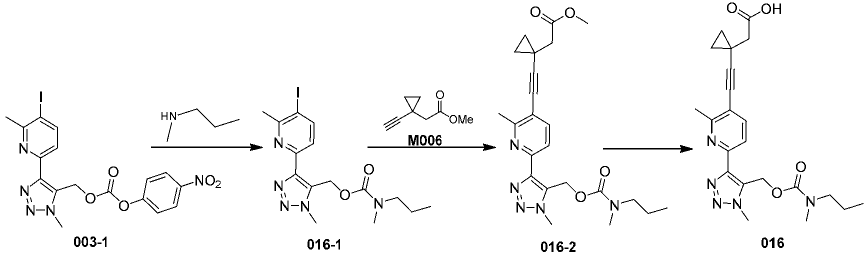

(1) At room temperature, compound 003-1 (644 mg, 1.3 mmol), *N*-methylpropan-1-amine (196 mg, 2.7 mmol), and *N*,*N*'-diisopropylethylamine (865 mg, 6.7 mmol) were dissolved in tetrahydrofuran (15 mL). The reaction mixture was stirred at room temperature for 1 hour. The resulting reaction mixture was directly concentrated under reduced pressure, and the residue was purified by silica gel column chromatography (petroleum ether/ethyl acetate = 1/1) to obtain compound 016-1 (456 mg, crude). LC-MS: [M+H]⁺ = 430.07.
(2) At room temperature, compound 016-1 (390 mg, 0.9 mmol) and M006 (188 mg, 1.36 mmol) were dissolved in 1,4-dioxane (5 mL), followed by the addition of potassium carbonate (251 mg, 1.8 mmol), bis(triphenylphosphine)palladium(II) chloride (64 mg, 0.091 mmol), copper(I) iodide (18 mg, 0.091 mmol), and triethylamine (460 mg, 4.5 mmol). Under a nitrogen atmosphere, the reaction mixture was heated to 100°C and stirred for 1 hour. After cooling to room temperature, the resulting reaction mixture was directly concentrated under reduced pressure. The resulting residue was purified by silica gel column chromatography to obtain compound 016-2 (404 mg, crude). LC-MS: [M+H]⁺ =440.22.
(3) At room temperature, compound 016-2 (404 mg, 0.92 mmol) was dissolved in methanol (6 mL) and water (2 mL), followed by the addition of lithium hydroxide monohydrate (110 mg, 4.6 mmol). The reaction mixture was heated to 60°C and stirred for 1 hour. After cooling to room temperature, the resulting reaction mixture was directly concentrated under reduced pressure. The resulting residue was diluted with water (10 mL) and extracted twice with ethyl acetate (10 mL). The aqueous phases were combined. The resulting aqueous phase was adjusted to pH = 2 to 3 with dilute hydrochloric acid (1 M), then extracted twice with ethyl acetate (10 mL), and the organic phases were combined. The resulting organic phase was dried over anhydrous sodium sulfate, filtered, and the filtrate was concentrated under reduced pressure. The resulting residue was purified by high-performance liquid chromatography (alkaline conditions) and lyophilized to obtain compound 016 (108.9 mg). LC-MS: [M+H]⁺ =426.15.

¹H NMR (400 MHz, DMSO-*d*₆) δ7.83 (d, *J* = 8.0 Hz, 1H), 7.72 (d, *J* = 8.0 Hz, 1H), 5.64 (d, *J* = 12.0 Hz, 2H), 4.11 (s, 3H), 3.14 (s, 1H), 3.03 (s, 1H), 2.75 (d, *J* = 20.0 Hz, 3H), 2.54 (s, 3H), 2.42 (s, 2H), 1.46 - 1.44 (m, 1H), 1.31 - 1.30 (m, 1H), 1.03 (t, *J* = 12.0 Hz, 2H), 1.00 - 0.91 (m, 2H), 0.81 - 0.61 (m, 3H).

### Example 17: Preparation of 2-(1-((6-(5-(((cyclobutylmethyl)(methyl)carbamoyl)oxy)methyl)-1-methyl-1H-1,2,3-triazol-4-yl)-2-methylpyridin-3-yl)ethynyl)cyclopropyl)acetic acid (compound 017)

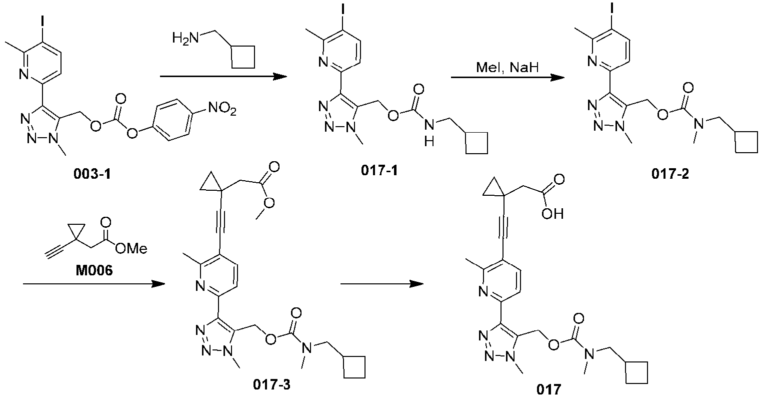

(1) At room temperature, compound 003-1 (743 mg, 1.5 mmol) and cyclobutylmethylamine (270 mg, 3.2 mmol) were dissolved in anhydrous tetrahydrofuran (18 mL), followed by the addition of *N,N-*diisopropylethylamine (1.4 mL, 7.4 mmol). The reaction mixture was stirred at room temperature for 1 hour. The resulting reaction mixture was directly concentrated under reduced pressure. The residue was purified by silica gel column chromatography (petroleum ether/ethyl acetate = 2/1) to obtain compound 017-1 (594 mg). LC-MS: [M+H]⁺ =442.07.
(2) At room temperature, compound 017-1 (618 mg, 1.4 mmol) was dissolved in anhydrous tetrahydrofuran (6 mL). After cooling the reaction mixture to 0°C, sodium hydride (100 mg, 4.2 mmol, 60% dispersed in mineral oil) was added. The reaction mixture was warmed to room temperature and stirred for 0.5 hours, followed by the addition of iodomethane (0.7 mL, 11.2 mmol). The reaction mixture was further heated to 45°C and stirred for 2 hours. The resulting reaction mixture was cooled to room temperature, poured into water (10 mL) to quench, and extracted twice with ethyl acetate (10 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 017-2 (460 mg). LC-MS: [M+H]⁺ =456.08.
(3) At room temperature, compound 017-2 (319 mg, 0.7 mmol) and M006 (150 mg, 1.1 mmol) were dissolved in 1,4-dioxane (5 mL), followed by the addition of potassium carbonate (200 mg, 1.5 mmol), bis(triphenylphosphine)palladium(II) chloride (51 mg, 0.072 mmol), copper(I) iodide (14 mg, 0.072 mmol), and triethylamine (367 mg, 3.6 mmol). Under a nitrogen atmosphere, the reaction mixture was heated to 100°C and stirred for 3 hours. After cooling to room temperature, the resulting reaction mixture was directly concentrated under reduced pressure. The resulting residue was purified by silica gel column chromatography (petroleum ether/ethyl acetate = 1/1) to obtain compound 017-3 (288 mg, crude). LC-MS: [M+H]⁺ =466.20.
(4) At room temperature, compound 017-3 (270 mg, 0.58 mmol) was dissolved in a mixed solvent of methanol (3 mL) and water (1 mL), and lithium hydroxide monohydrate (70 mg, 2.9 mmol) was added. The reaction mixture was heated to 60°C and stirred for 0.5 hours. After cooling to room temperature, the resulting reaction mixture was directly concentrated under reduced pressure. The resulting residue was diluted with water (10 mL) and extracted twice with ethyl acetate (10 mL). The aqueous phases were combined. The resulting aqueous phase was adjusted to pH = 3 to 4 with dilute hydrochloric acid (1 M) and then extracted twice with ethyl acetate (10 mL). The organic phases were combined. The resulting organic phase was dried over anhydrous sodium sulfate, filtered, and the filtrate was concentrated under reduced pressure. The resulting residue was purified by high-performance liquid chromatography (alkaline conditions) and lyophilized to obtain compound 017 (73.3 mg). LC-MS: [M+H]⁺ =452.15.

¹H NMR (400 MHz, DMSO-*d*₆) δ 7.83 (d, *J* = 8.0 Hz, 1H), 7.72 (d, *J* = 8.0 Hz, 1H), 5.65 - 5.62 (m, 2H), 4.11 (s, 3H), 3.23 (d, *J* = 8.0 Hz, 1H), 3.10 (d, *J* = 8.0 Hz, 1H), 2.76 - 2.71 (m, 3H), 2.54 (s, 3H), 2.44 (s, 2H), 2.39 - 2.21 (m, 1H), 1.92 (s, 1H), 1.77 (s, 2H), 1.70 - 1.51 (m, 2H), 1.49 - 1.35 (m, 1H), 1.05 (d, *J* = 8.0 Hz, 2H), 1.01 - 0.91 (m, 2H).

### Example 18: Preparation of 2-(1-((6-(5-(((cyclopentyl(methyl)carbamoyl)oxy)methyl)-1-methyl-1H-1,2,3-triazol-4-yl)-2-methylpyridin-3-yl)ethynyl)cyclopropyl)acetic acid (compound 018)

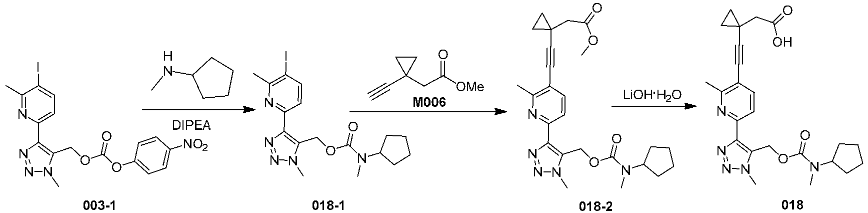

The preparation of compound 018 was referred to the method for preparing compound 016 in Example 16, wherein the starting material was replaced from *N*-methylpropan-1-amine to cyclopentylmethylamine, and compound 018 (113.1 mg) was obtained. LC-MS: [M+H]⁺ =452.10.

¹H NMR (400 MHz, DMSO-*d*₆) δ 7.83 (d, *J* = 8.0 Hz, 1H), 7.72 (d, *J* = 8.0 Hz, 1H), 5.64 (s, 2H), 4.59 - 4.15 (m, 1H), 4.11 (s, 3H), 2.64 (s, 3H), 2.53 (s, 3H), 2.42 (s, 2H), 1.57 (s, 4H), 1.43 (s, 4H), 1.03 (dd, *J* = 4.0, 4.0 Hz, 2H), 0.99 - 0.92 (m, 2H).

### Example 19: Preparation of 2-(1-((6-(5-(((4-isopropylpyrimidin-2-yl)amino)methyl)-1-methyl-1H-1,2,3-triazol-4-yl)-2-methylpyridin-3-yl)ethynyl)cyclopropyl)acetic acid (compound 019)

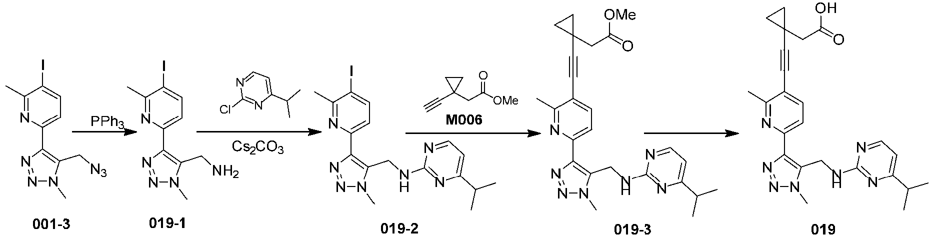

(1) At room temperature, compound 001-3 (1.07 g, 3.0 mmol) was dissolved in a mixed solvent of tetrahydrofuran (20 mL) and water (4 mL), followed by the addition of triphenylphosphine (1.6 g, 6.0 mmol). The reaction mixture was heated to 40°C and stirred for 3 hours. The resulting reaction mixture was cooled to room temperature, poured into dilute aqueous hydrochloric acid solution (20 mL), extracted twice with ethyl acetate (20 mL), and the aqueous phases were combined. The resulting aqueous phase was adjusted to pH = 10 with saturated sodium carbonate solution, then extracted with ethyl acetate (30 mL), and the organic phases were combined. The resulting organic phase was sequentially washed twice with saturated brine (60 mL), dried over anhydrous sodium sulfate, filtered, and the filtrate was concentrated under reduced pressure to obtain compound 019-1 (580 mg, crude). LC-MS: [M+H]⁺ =330.01.
(2) At room temperature, compound 019-1 (560 mg, 1.7 mmol) and 2-chloro-4-isopropylpyrimidine (526 mg, 3.37 mmol) were dissolved in *N,N-*dimethylformamide (20 mL), followed by the addition of cesium carbonate (1.85 g, 5.68 mmol). The reaction mixture was heated to 120°C and stirred for 5 hours. After cooling to room temperature, the resulting reaction mixture was diluted with water (100 mL) and extracted with ethyl acetate (30 mL). The combined organic phases were sequentially washed twice with saturated brine (60 mL), dried over anhydrous sodium sulfate, filtered, and the filtrate was concentrated under reduced pressure. The resulting residue was purified by silica gel column chromatography (ethyl acetate/petroleum ether = 1/2) to obtain compound 019-2 (400 mg). LC-MS: [M+H]⁺ =450.08.
(3) At room temperature, compound 019-2 (445 mg, 0.99 mmol) and M006 (205.8 mg, 1.48 mmol) were dissolved in 1,4-dioxane (10 mL), followed by the addition of potassium carbonate (272 mg, 1.98 mmol), triethylamine (248 mg, 2.48 mmol), bis(triphenylphosphine)palladium(II) chloride (70 mg, 0.1 mmol), and copper(I) iodide (18.4 mg, 0.1 mmol). Under a nitrogen atmosphere, the reaction mixture was heated to 90°C and stirred for 3 hours. The resulting reaction mixture was cooled to room temperature, diluted with water (20 mL), and extracted with ethyl acetate (20 mL). The organic phases were combined. The resulting organic phase was sequentially washed twice with saturated brine (30 mL), dried over anhydrous sodium sulfate, filtered, and the filtrate was concentrated under reduced pressure. The resulting residue was purified by silica gel column chromatography (ethyl acetate/petroleum ether = 1/2) to obtain compound 019-3 (100 mg). LC-MS: [M+H]⁺ = 460.15.
(4) At room temperature, compound 019-3 (100 mg, 0.21 mmol) was dissolved in a mixed solvent of methanol (2 mL) and water (1 mL), followed by the addition of lithium hydroxide monohydrate (42 mg, 1.0 mmol). The reaction mixture was heated to 50°C and stirred for 1 hour. After cooling to room temperature, the resulting reaction mixture was directly concentrated under reduced pressure. The resulting residue was diluted with water (3 mL) and extracted with ethyl acetate (5 mL). The organic phase was washed twice with lithium hydroxide solution (1 mL, 2 M), and the combined aqueous phases were adjusted to pH = 5 to 6 with dilute hydrochloric acid, then directly purified by high-performance liquid chromatography (0.1% hydrochloric acid conditions). The resulting solution was adjusted to pH = 5 to 6 with saturated sodium bicarbonate, then extracted twice with ethyl acetate (60 mL), and the organic phases were combined. The resulting organic phase was sequentially washed with saturated brine (60 mL), dried over anhydrous sodium sulfate, filtered, and the filtrate was concentrated under reduced pressure. The resulting residue was added with deionized water (10 mL) and acetonitrile (2 mL), then lyophilized to obtain compound 019 (22.2 mg). LC-MS: [M+H]⁺ =446.15.

¹H NMR (400 MHz, DMSO-*d*₆) δ 12.24 (s, 1H), 8.17 (d, *J* = 5.2 Hz, 1H), 7.84 (d, *J* = 8.2 Hz, 1H), 7.73 (d, *J* = 8.2 Hz, 1H), 7.40 (t, *J* = 5.6 Hz, 1H), 6.52 (d, *J* = 5.2 Hz, 1H), 5.00 (d, *J* = 5.6 Hz, 2H), 4.15 (s, 3H), 2.64 (m 1H), 2.57 (s, 3H), 2.46 (s, 2H), 1.07 (m, 7H), 0.96 (m,2H).

### Example 20: Preparation of (R)-2-(1-((6-(5-(((1-(2-chlorophenyl)ethoxy)carbonyl)amino)-1-methyl-1H-1,2,3-triazol-4-yl)-2-methylpyridin-3-yl)ethynyl)cyclobutyl)acetic acid (compound 020)

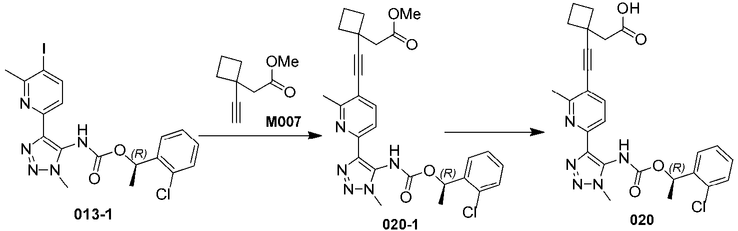

The preparation of compound 020 was referred to the method for preparing compound 013 in Example 13, wherein the starting material was replaced from compound M006 to compound M007, and compound 020 (7.9 mg) was obtained. LC-MS: [M+H]⁺=508.10.

¹H NMR (400 MHz, DMSO-*d*₆) δ 7.70 (d, *J* = 8.4 Hz, 1H), 7.59 (d, *J* = 8.0 Hz, 1H), 7.55 - 7.36 (m, 2H), 7.31 - 7.29 (m, 2H), 5.96 (q, *J* = 6.4 Hz, 1H), 3.80 (s, 3H), 2.51 - 2.50 (m, 3H), 2.39 (s, 2H), 2.36 - 2.30 (m, 2H), 2.27 - 2.20 (m, 2H), 2.03 - 1.85 (m, 2H), 1.38 (s, 3H).

### Example 21: Preparation of (R)-1-(2-chlorophenyl)ethyl (4-(5-((1-cyanomethyl)cyclopropyl)ethynyl)-6-methylpyridin-2-yl)-1-methyl-1H 1,2,3-triazol-5-yl)carbamate (compound 021)

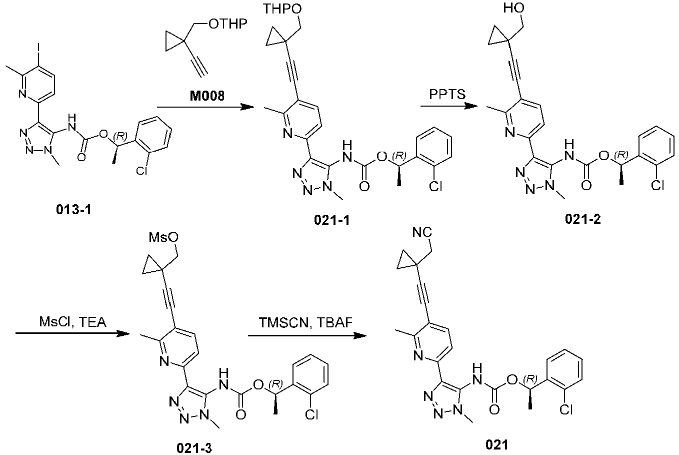

(1) At room temperature, compound M008 (543 mg, 3.01 mmol) and compound 013-1 (500 mg, 1.00 mmol) were dissolved in 1,4-dioxane (15 mL), followed by the addition of triethylamine (203 mg, 2.01 mmol), copper(I) iodide (38 mg, 0.20 mmol), bis(triphenylphosphine)palladium(II) chloride (71 mg, 0.10 mmol), and potassium carbonate (278 mg, 2.01 mmol). After nitrogen purging, the reaction mixture was heated to 80°C under a nitrogen atmosphere and stirred for an additional 1 hour. After cooling to room temperature, the reaction mixture was poured into water (20 mL) to dilute and extracted with ethyl acetate (30 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 1/1) to obtain compound 021-1 (500 mg, crude). LC-MS: [M+H]⁺=550.15.
(2) At room temperature, compound 021-1 (580 mg, 1.06 mmol) was dissolved in methanol (10 mL), followed by the addition of pyridinium *p*-toluenesulfonate (796 mg, 1.06 mmol). The reaction mixture was heated to 60°C and stirred for 1 hour. The resulting reaction mixture was cooled to room temperature, poured into water (20 mL), then extracted with dichloromethane (30 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 1/1) to obtain compound 021-2 (280 mg). LC-MS: [M+H]⁺=466.10.
(3) At room temperature, compound 021-2 (150 mg, 0.32 mmol) was dissolved in dichloromethane (5 mL), and the reaction mixture was cooled to 0°C, followed by the addition of triethylamine (65 mg, 0.64 mmol) and methanesulfonyl chloride (44 mg, 0.39 mmol). The reaction mixture was stirred at 0°C for 2 hours. The resulting reaction mixture was poured into water (20 mL) to dilute, extracted with dichloromethane (20 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 3/1) to obtain compound 021-3 (200 mg, crude). LC-MS: [M+H]⁺=544.05.
(4) At room temperature, compound 021-3 (190 mg, 0.35 mmol) was dissolved in acetonitrile (10 mL), followed by the addition of trimethylsilyl cyanide (104 mg, 1.05 mmol) and tetrabutylammonium fluoride (274 mg, 1.05 mmol). The reaction mixture was heated to 80°C and stirred for 1 hour. After cooling to room temperature, the resulting reaction mixture was poured into water (10 mL) and extracted with ethyl acetate (20 mL). The organic phases were combined. The resulting organic phase was dried over anhydrous sodium sulfate, filtered, and the filtrate was concentrated under reduced pressure. The resulting residue was purified by high-performance liquid chromatography (0.1% HCl system) and lyophilized to obtain compound 021 (12.1 mg). LC-MS: [M+H]⁺=475.10.

¹H NMR (400 MHz, DMSO-*d*₆) δ 9.79 (s, 1H), 7.76 - 7.70 (m, 3H), 7.42 - 7.31 (m, 4H), 5.98 (s, 1H), 3.88 (s, 3H), 2.87 (s, 3H), 2.52 - 2.49 (m, 2H), 1.51 (s, 3H), 1.14 - 1.12 (m, 2H), 1.05 - 1.02 (m, 2H).

### Example 22: Preparation of (R)-2-(1-((2-methyl-6-(1-methyl-5-(((1-phenylethoxy)carbonyl)amino)-1H-1,2,3-triazol-4-yl)pyridin-3-yl)ethynyl)cyclopropyl)acetic acid (compound 022)

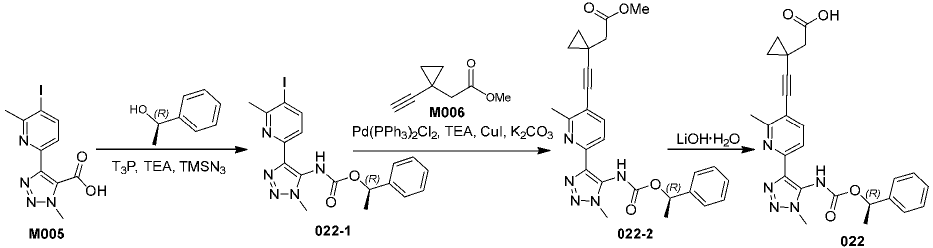

The preparation of compound 022 was referred to the method for preparing compound 013 in Example 13, wherein the starting material was replaced from (R)-1-(2-chlorophenyl)ethan-1-ol to (*R*)-1-phenylethan-1-ol, and compound 022 (18.2 mg) was obtained. LC-MS: [M+H]⁺ =460.15.

¹H NMR (400 MHz, *CD₃OD*) δ 7.70 - 7.64 (m, 2H), 7.32 (s, 5H), 5.81 (d, *J* = 6.4 Hz, 1H), 3.95 (s, 3H), 2.55 (s, 3H), 2.48 (s, 2H), 1.54 (s, 3H), 1.11 (dd, *J* = 6.8, 4.4 Hz, 2H), 0.98 (dd, *J* = 6.8, 4.4 Hz, 2H).

### Example 23: Preparation of 2-(1-((6-(5-(((1-(2-chloro-5-fluoropyridin-3-yl)ethoxy)carbonyl)amino)-1-methyl-1H-1,2,3-triazol-4-yl)-2-methylpyridin-3-yl)ethynyl)cyclopropyl)acetic acid (compound 023), compound 023-A, and compound 023-B

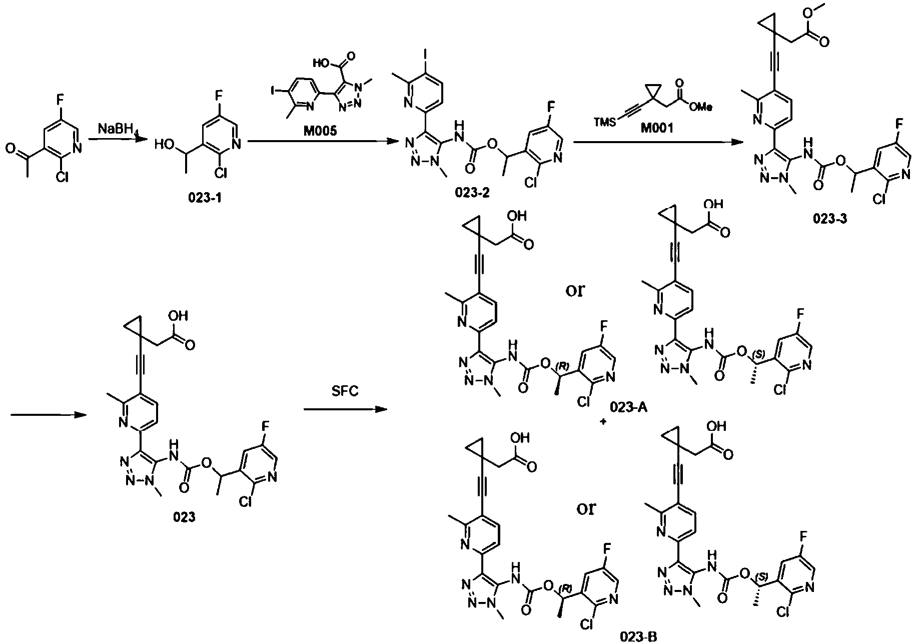

(1) At room temperature, 1-(2-chloro-5-fluoropyridin-3-yl)ethan-1-one (2.0 g, 11.52 mmol) was dissolved in methanol (20 mL). After cooling the reaction mixture to 0°C, sodium borohydride (436 mg, 11.52 mmol) was added. The reaction mixture was warmed to room temperature and stirred for an additional 1 hour. The resulting reaction mixture was diluted with water (10 mL), extracted with dichloromethane (10 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 023-1 (1.7 g). LC-MS: [M+H]⁺ = 176.00.
(2) At room temperature, compound M005 (578 mg, 1.68 mmol), triethylamine (613 mg, 6.06 mmol), azidotrimethylsilane (312 mg, 2.52 mmol), and propylphosphonic anhydride (2.68 g, 4.21 mmol, 50% in ethyl acetate) were dissolved in anhydrous tetrahydrofuran (5 mL). The reaction mixture was heated to 60°C and stirred for 1 hour. The reaction mixture was cooled to room temperature, and compound 023-1 (592 mg, 3.37 mmol) was added. The reaction mixture was heated to 70°C and stirred for an additional 1 hour. The resulting reaction mixture was diluted with water (10 mL), extracted with dichloromethane (10 mL), and the organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 023-2 (700 mg). LC-MS: [M+H]⁺ = 517.00.
(3) At room temperature, compound 023-2 (222 mg, 0.43 mmol) was dissolved in 1,4-dioxane (3 mL), followed by the addition of bis(triphenylphosphine)palladium(II) chloride (30 mg, 0.042 mmol), copper(I) iodide (17 mg, 0.085 mmol), M001 (358 mg, 1.70 mmol), cesium fluoride (129 mg, 0.85 mmol), potassium carbonate (117 mg, 0.85 mmol), and triethylamine (165 mg, 1.28 mmol). The reaction mixture was purged with nitrogen, heated to 90°C under a nitrogen atmosphere, and stirred for 3 hours. The resulting reaction mixture was cooled to room temperature, diluted with water (10 mL), and extracted with ethyl acetate (10 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 2/1) to obtain compound 023-3 (100 mg). LC-MS: [M+H]⁺ = 527.10.
(4) At room temperature, compound 023-3 (50 mg, 0.095 mmol) was dissolved in a mixed solvent of methanol (2 mL) and water (0.8 mL), and lithium hydroxide monohydrate (20 mg, 0.47 mmol) was added. The reaction mixture was heated to 40°C and stirred for 2 hours. The resulting reaction mixture was cooled to room temperature and adjusted to pH = 7 with dilute hydrochloric acid (1 M). The resulting reaction mixture was concentrated under reduced pressure. The residue was diluted with water (5 mL), and the resulting solution was acidified to pH = 5 to 6 with dilute hydrochloric acid (1 M). The resulting solution was extracted with ethyl acetate (5 mL). The combined organic phases were dried over anhydrous sodium sulfate, filtered, and the filtrate was concentrated under reduced pressure. The resulting residue was purified by preparative high-performance liquid chromatography (0.1% hydrochloric acid conditions) and lyophilized to obtain compound 023 (8.8 mg). LC-MS: [M+H]⁺ =513.10.
   ¹H NMR (400 MHz, DMSO-*d*₆) δ 12.25 (s, 1H), 9.84 (s, 1H), 8.41 (s, 1H), 7.74 (d, *J* = 8.0 Hz, 1H), 7.65 (s, 1H), 5.86 (s, 1H), 3.90 (s, 3H), 2.45 (s, 3H), 2.40 (s, 2H), 1.66 - 1.18 (m, 3H), 1.09 - 1.02 (m, 2H), 0.99 - 0.91 (m, 2H).
(5) Compound 023 (200 mg) was subjected to chiral separation (CHIRAL ART Amylose-C NEO, AD-H column, 30 × 250 mm; mobile phase A: *n*-hexane; mobile phase B: ethanol, volume ratio of mobile phases A and B: 83:17; isocratic elution; flow rate: 30 mL/min, column temperature: room temperature (25 to 28°C), injection volume: 0.4 mL), detection wavelength: 220 nm; to obtain compound 023-A (retention time Rt = 13.097 min, 31.8 mg) and compound 023-B (retention time Rt = 20.769 min, 38.7 mg).

Compound 023-A: ¹H NMR (400 MHz, DMSO-*d*₆)6 8.41 (s, 1H), 7.75 (d, *J* = 8.0 Hz, 1H), 7.66 (s, 1H), 5.86 (s, 1H), 3.91 (s, 3H), 2.52 (s, 2H), 2.45-2.41 (m, 3H), 1.62 - 1.20 (m, 3H), 1.10 - 1.02 (m, 2H), 0.98 - 0.91 (m, 2H).

Compound 023-B: ¹H NMR (400 MHz, DMSO-*d*₆)6 8.42 (s, 1H), 7.75 (d, *J* = 8.0 Hz, 1H), 7.66 (s, 1H), 5.86 (s, 1H), 3.91 (s, 3H), 2.52 (s, 2H), 2.47 - 2.38 (m, 3H), 1.58 - 1.20 (m, 3H), 1.09 - 1.02 (m, 2H), 1.00 - 0.93 (m, 2H).

### Example 24: Preparation of 2-(1-((6-(5-((((cyclopropylmethyl)(methyl)carbamoyl)oxy)methyl)-1-methyl-1H-1,2,3-triazol-4-yl)-2-methylpyridin-3-yl)ethynyl)cyclopropyl)acetic acid (compound 024)

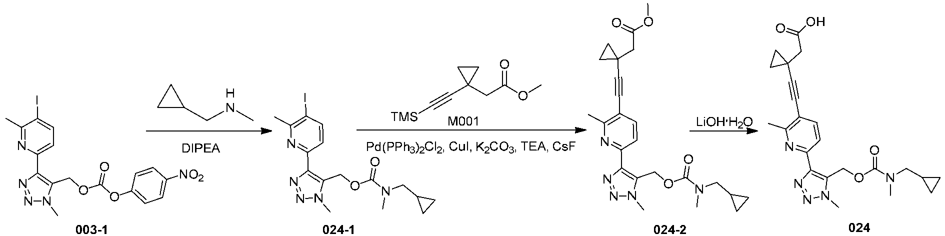

The preparation of compound 024 was referred to the method for preparing compound 004 in Example 4, wherein the starting material was replaced from *N*-methylbutylamine to (cyclopropylmethyl)methylamine, and compound 024 (33.5 mg) was obtained. LC-MS [M+H]⁺ = 438.15.

¹H NMR (400 MHz, DMSO-*d*₆) δ 7.85 (d, *J* = 4.0 Hz, 1H), 7.76 (s, 1H), 5.65 (d, *J* = 16.0 Hz, 2H), 4.11 (s, 3H), 3.07 - 2.95 (m, 2H), 2.81 (d, *J* = 16.0 Hz, 3H), 2.55 (s, 3H), 2.46 (s, 2H), 1.09 - 1.03 (m, 2H), 0.98 - 0.94 (m, 2H), 0.93 - 0.70 (m, 1H), 0.41 - 0.40 (m, 1H), 0.31 - 0.12 (m, 2H), 0.01 (s, 1H).

### Example 25: Preparation of 2-(1-((6-(5-((((cyclobutylmethyl)(methyl)carbamoyl)oxy)methyl)-1-methyl-1H-1,2,3-triazol-4-yl)pyridin-3-yl)ethynyl)cyclopropyl)acetic acid (compound 025)

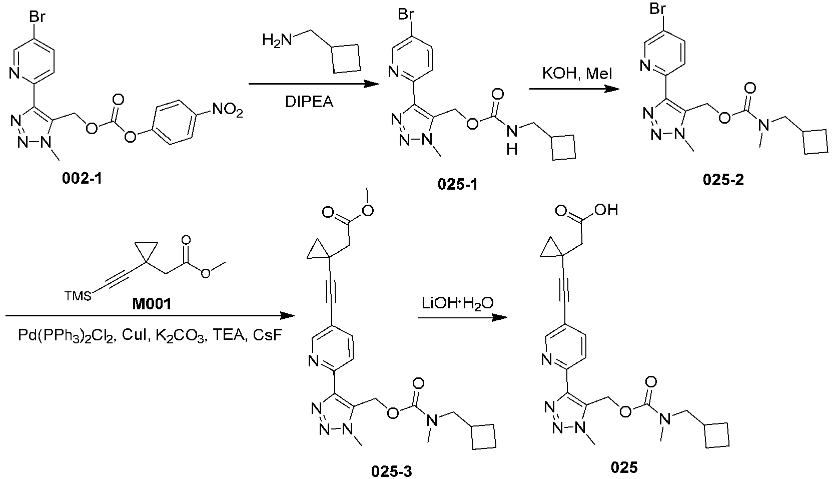

The preparation of compound 025 was referred to the method for preparing compound 002 in Example 2, wherein the starting material was replaced from (*R*)-1-cyclopropylethylamine hydrochloride to cyclobutylmethylamine, and compound 025 (9.5 mg) was obtained. LC-MS: [M+H]⁺ = 438.15.

¹H NMR (400 MHz, DMSO-*d*₆) δ 12.04 (s, 1H), 8.55 (s, 2H), 8.02 (d, *J* = 8.0 Hz, 2H), 7.84 (m, 2H), 5.62 (d, *J* = 12.4 Hz, 4H), 4.12 (s, 6H), 3.30 (s, 5H), 3.22 (d, *J* = 6.0 Hz, 2H), 3.11 (d, *J* = 6.4 Hz, 2H), 2.73 (d, *J* = 13.2 Hz, 6H), 2.45 (s, 4H), 1.93 (s, 2H), 1.79 (s, 4H), 1.66 (d, *J* = 8.4 Hz, 2H), 1.46 (s, 2H), 1.06 (t, *J* = 5.2 Hz, 4H), 0.97 - 0.92 (m, 4H).

### Example 26: Preparation of 2-(1-((6-(5-(((((2,2-difluorocyclobutyl)methyl)(methyl)carbamoyl)oxy)methyl)-1-methyl-1H-1,2,3-triazol-4-yl)-2-methylpyridin-3-yl)ethynyl)cyclopropyl)acetic acid (compound 026)

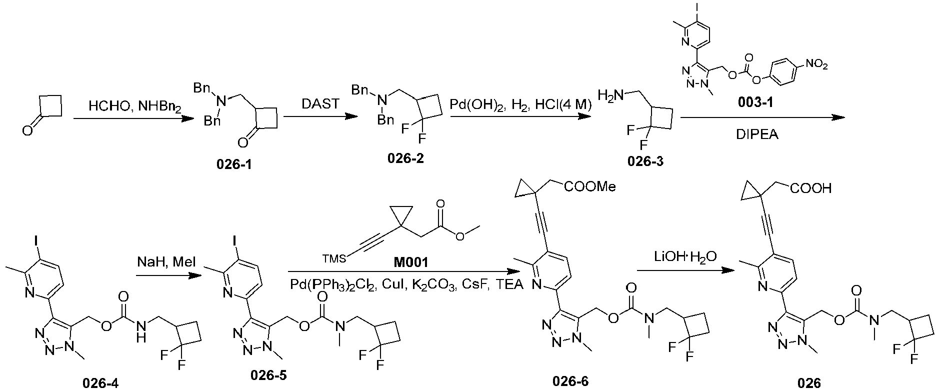

The preparation of compound 026 was referred to the method for preparing compound 009 in Example 9, wherein the starting material was replaced from (1-fluorocyclobutyl)methylamine hydrochloride to the hydrochloride of compound 026-3, and compound 026 (74.9 mg) was obtained. LC-MS: [M+H]⁺ =488.10.

¹H NMR (400 MHz, *CD₃OD*) δ 7.80 (d, *J* = 7.8 Hz, 1H), 7.73 (d, *J* = 7.8 Hz, 1H), 5.78 - 5.64 (m, 2H), 4.18 (d, *J* = 5.6 Hz, 3H), 3.29 - 3.19 (m, 1H), 2.86 (d, *J* = 20.2Hz, 3H), 2.62 (s, 3H), 2.43 (s, 3H), 2.36 - 2.19 (m, 1H), 2.04 - 1.84 (m, 1H), 1.71 - 1.57 (m, 1H), 1.54 - 1.42 (m, 1H), 1.32 - 1.19 (m, 1H) , 1.12 - 1.04 (m, 2H), 1.00 - 0.95 (m, 2H).

The hydrochloride of the starting material compound 026-3 was commercially available, and could also be prepared by the following method:
(1) At room temperature, cyclobutanone (5.0 g, 71.34 mmol) was dissolved in toluene (50 mL), followed by the addition of formaldehyde (40%, 5.35 g, 71.34 mmol), dibenzylamine (14 g, 71.34 mmol), sodium acetate (1.17 g, 14.27 mmol), and glacial acetic acid (857 mg, 14.27 mmol). The reaction mixture was stirred at 110°C for 16 hours. The resulting reaction mixture was quenched with aqueous sodium bicarbonate solution (50 mL) and extracted with ethyl acetate (50 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 20/1) to obtain compound 026-1 (9.0 g). LC-MS: [M+H]⁺ = 280.10.
(2) At room temperature, compound 026-1 (1.0 g, 3.58 mmol) was dissolved in dichloromethane (10 mL), and diethylaminosulfur trifluoride (1.73 mg, 10.74 mmol) was added dropwise to the reaction mixture at 0°C. The reaction mixture was stirred at room temperature for 16 hours. The resulting reaction mixture was quenched with water (20 mL) and extracted with dichloromethane (20 mL). The organic phases were combined. The resulting organic phase was dried over anhydrous sodium sulfate, filtered, and the filtrate was concentrated under reduced pressure. The resulting residue was purified by silica gel column chromatography (petroleum ether/ethyl acetate = 20/1) to obtain compound 026-2 (350 mg). LC-MS: [M+H]⁺ = 302.10.
(3) At room temperature, compound 026-2 (350 mg, 1.16 mmol) was dissolved in methanol (7 mL), followed by the addition of a solution of hydrogen chloride in methanol (4 M, 1.16 mL, 4.65 mmol) and palladium hydroxide on carbon (10%, 350 mg). The reaction mixture was stirred under a hydrogen atmosphere at 40°C for 48 hours. The reaction mixture was filtered, and the filtrate was concentrated under reduced pressure to obtain the crude hydrochloride of compound 026-3 (250 mg). LC-MS: [M+H]⁺ = 122.15.

### Example 27: Preparation of trans-2-((2-methyl-6-(1-methyl-5-((((R)-1-phenylethoxy)carbonyl)amino)-1H-1,2,3-triazol-4-yl)pyridin-3-yl)ethynyl)cyclohexane-1-carboxylic acid (compound 027), compound 027-A, and compound 027-B

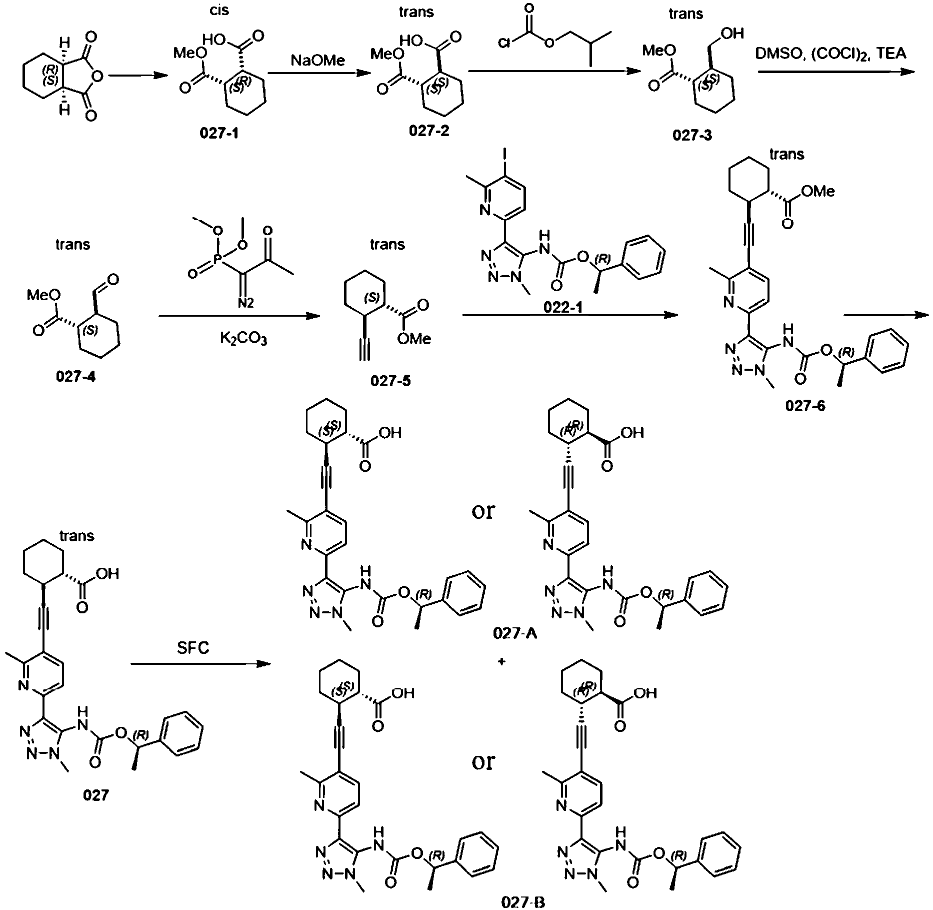

(1) At room temperature, (3aR,7aS)-hexahydroisobenzofuran-1,3-dione (10.0 g, 64.9 mmol) was dissolved in a sealed container containing methanol (3.1 g, 97.4 mmol). The reaction mixture was heated to 100°C and stirred for 1 hour. After cooling to room temperature, the resulting reaction mixture was transferred to a single-neck flask and concentrated directly under reduced pressure to obtain compound 027-1 (11.0 g, crude), containing a pair of cis configurations. LC-MS: [M+H]⁺=187.10.
   ¹H NMR (400 MHz, *CDCl₃)* δ 9.91 (s, 1H), 3.67 (s, 3H), 2.84 (s, 2H), 2.12 - 1.94 (m, 2H), 1.77 (dd, *J* = 6.8, 3.8 Hz, 2H), 1.62 - 1.31 (m, 4H).
(2) At room temperature, compound 027-1 (10.0 g, 53.8 mmol) was dissolved in methanol (20 mL), and a solution of sodium methoxide in methanol (150 mL, 2.5 mol/L) was added. The reaction mixture was heated to 70°C and stirred for 2 hours. After cooling to 0°C, the resulting reaction mixture was slowly poured into dilute hydrochloric acid (300 mL, 2 M) to adjust the pH of the solution to 3 to 4. Water (200 mL) was then added to dilute, followed by extraction with ethyl acetate (100 mL). The organic phases were combined. The resulting organic phase was washed with saturated brine (50 mL), dried over anhydrous sodium sulfate, and filtered. The resulting filtrate was concentrated under reduced pressure to obtain compound 027-2 (8.0 g), containing a pair of trans configurations. LC-MS: [M+H]⁺=187.10.
   ¹H NMR (400 MHz, *CDCl₃)* δ 10.05 (s, 1H), 3.67 (s, 3H), 2.73 - 2.46 (s, 2H), 2.18 - 1.99 (m, 2H), 1.88 - 1.71 (m, 2H), 1.37 - 1.26 (m, 4H).
(3) At room temperature, compound 027-2 (3.0 g, 16.1 mmol) and triethylamine (3.2 g, 32.2 mmol) were dissolved in tetrahydrofuran (30 mL). The reaction mixture was cooled to 0°C, and isobutyl chloroformate (2.6 g, 19.3 mmol) was slowly added dropwise. After the dropwise addition was completed, the reaction mixture was warmed to room temperature and stirred for an additional 1 hour. The reaction mixture was cooled to 0°C, and then a solution of sodium borohydride (1.8 g, 48.3 mmol) in water (10 mL) was slowly added. The reaction mixture was warmed to room temperature and stirred for an additional 1 hour. Dilute hydrochloric acid (1 M) was added to the resulting reaction mixture to adjust the pH of the solution to 5 to 6. Water (50 mL) was added to dilute, followed by extraction with ethyl acetate (30 mL). The organic phases were combined. The resulting organic phase was washed with saturated brine (30 mL), dried over anhydrous sodium sulfate, filtered, and the filtrate was concentrated under reduced pressure. The resulting residue was purified by silica gel column chromatography (petroleum ether/ethyl acetate = 3/1) to obtain compound 027-3 (1.9 g). LC-MS: [M+H]+=173.10.
(4) At room temperature, dimethyl sulfoxide (1.2 g, 15.7 mmol) was dissolved in dichloromethane (15 mL), and the reaction mixture was cooled to -78°C, followed by the slow dropwise addition of oxalyl chloride (1.6 g, 13.0 mmol). After the dropwise addition was completed, the reaction mixture was stirred at -78°C for 0.5 hours. Then, compound 027-3 (1.5 g, 8.7 mmol) was added dropwise. The reaction mixture was stirred at -78°C for an additional 0.5 hours. Finally, triethylamine (2.7 g, 26.1 mmol) was slowly added dropwise, maintaining the internal temperature of the reaction system below -70°C. After the dropwise addition was completed, the reaction mixture was warmed to room temperature and stirred for an additional 0.5 hours. The resulting reaction mixture was poured into water (30 mL) to quench and extracted with dichloromethane (30 mL). The organic phases were combined. The resulting organic phase was washed with saturated brine (30 mL), dried over anhydrous sodium sulfate, filtered, and the filtrate was concentrated under reduced pressure to obtain compound 027-4 (1.4 g). LC-MS: [M+H]⁺=171.21.
(5) At room temperature, compound 027-4 (1.36 g, 8.0 mmol) was dissolved in methanol (15 mL), and the reaction mixture was cooled to 0°C, followed by the addition of potassium carbonate (2.76 g, 20.0 mmol) and dimethyl (1-diazo-2-oxopropyl)phosphonate (2.3 g, 12.0 mmol). The reaction was warmed to room temperature and stirred for an additional 16 hours. The resulting reaction mixture was diluted with water (50 mL), extracted with ethyl acetate (20 mL), and the organic phases were combined. The resulting organic phase was washed with saturated brine (20 mL), dried over anhydrous sodium sulfate, filtered, and the filtrate was concentrated under reduced pressure to obtain compound 027-5 (900 mg). LC-MS: [M+H]⁺=167.15.
(6) At room temperature, compound 022-1 (330 mg, 0.7 mmol) and compound 027-5 (356 mg, 2.1 mmol) were dissolved in 1,4-dioxane (6 mL), followed by the addition of potassium carbonate (197 mg, 1.4 mmol), bis(triphenylphosphine)palladium(II) chloride (50 mg, 6 µmol), copper(I) iodide (14 mg, 71 µmol), cesium fluoride (433 mg, 2.9 mmol), and triethylamine (361 mg, 3.6 mmol). After nitrogen purging, the reaction mixture was heated to 80°C under a nitrogen atmosphere and stirred for 1 hour. After cooling to room temperature, the resulting reaction mixture was directly concentrated under reduced pressure. The resulting residue was purified by silica gel column chromatography (petroleum ether/ethyl acetate = 1/1) to obtain compound 027-6 (455 mg, crude). LC-MS: [M+H]⁺ =502.15.
(7) At room temperature, compound 027-6 (400 mg, 0.8 mmol) was dissolved in a mixed solvent of methanol (6 mL) and water (2 mL), and lithium hydroxide monohydrate (192 mg, 8 mmol) was added. The reaction mixture was heated to 40°C and stirred for 2 hours. The resulting reaction mixture was cooled to room temperature, adjusted to pH = 7 with dilute hydrochloric acid, and directly concentrated. The resulting residue was purified by high-performance liquid chromatography (alkaline conditions) and lyophilized to obtain compound 027 (200 mg). LC-MS: [M+H]⁺=488.10.
(8) Compound 027 (100 mg) was subjected to chiral separation (Daicel Chiralpak IG column, 250 × 30 mm I.D., 5 µm; mobile phase A: supercritical carbon dioxide; mobile phase B: methanol and DEA (DEA accounts for 0.05% of the volume of mobile phase B, *i.e.,* the total volume); volume ratio of mobile phase A to B: 80:20; isocratic elution; flow rate: 55 g/min; column temperature: room temperature (25 to 28°C); injection volume: 0.4 mL; detection wavelength: 220 nm) to obtain compound 027-A (retention time Rt = 5.550 min, 21.1 mg, purity: 99.328%) and compound 027-B (retention time Rt = 6.336 min, 18.4 mg, purity: 99.024%). LC-MS: [M+H]⁺ =488.10.

Compound 027-A: ¹H NMR (400 MHz, DMSO-*d₆*) δ 7.71 (dd, *J =* 8.0, 8.0 Hz, 2H), 7.57-6.92(m, 5H), 5.72 (s, 1H), 3.87 (s, 3H), 2.92 - 2.77 (m, 1H), 2.66 - 2.63 (m, 1H) 2.45 (s, 3H), 2.34 - 2.28 (m, 1H), 2.03 - 1.94 (m, 1H), 1.52 - 1.40 (m, 1H), 1.67 (d, *J =* 12.0 Hz, 2H), 1.52 - 1.40 (m, 3H), 1.30 (s, 2H), 1.23 (s, 1H).

Compound 027-B: ¹H NMR (400 MHz, DMSO-*d₆*) δ 7.69 (dd, *J* = 8.0, 4.0 Hz, 2H), 7.62 - 6.80(m, 5H), 5.71 (s, 1H), 3.85 (s, 3H), 2.84 - 2.75 (m, 1H), 2.71 - 2.62 (m, 1H), 2.43 (s, 2H), 2.35 - 2.28 (m, 1H), 1.91 (dd, *J =* 8.0,8.0 Hz, 2H), 1.66 (s, 2H), 1.55 - 1.35 (m, 4H), 1.33 - 1.14 (m, 3H).

### Example 28: Preparation of trans-2-((6-(5-((((R)-1-(2-fluorophenyl)ethoxy)carbonyl)amino)-1-methyl-1H-1,2,3-triazol-4-yl)-2-methylpyridin-3-yl)ethynyl)cyclohexane-1-carboxylic acid (compound 028), compound 028-A, and compound 028-B

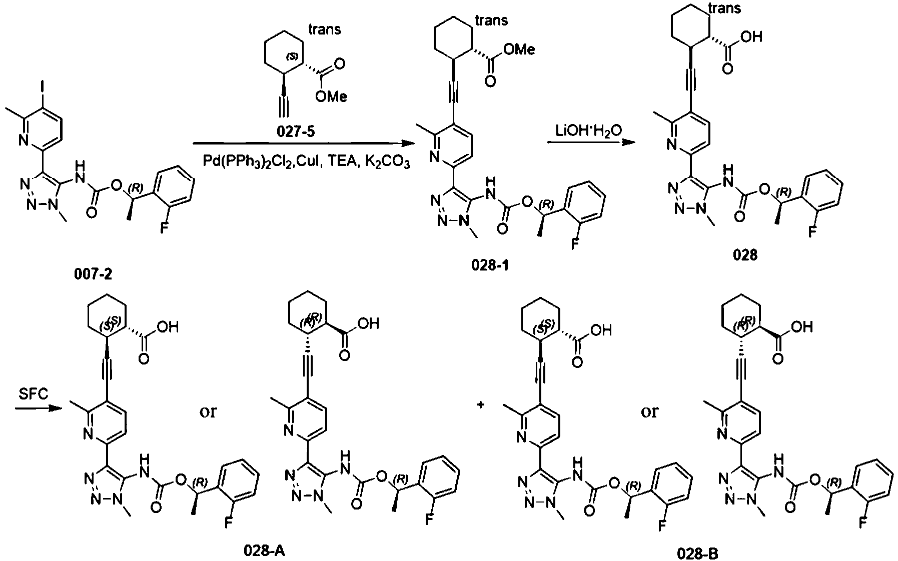

The preparation of compound 028 was referred to the method for preparing compound 027 in Example 27, wherein 027-1 was replaced with compound 007-2, and compound 028 (100 mg) was obtained.

Compound 028 (100 mg) was subjected to chiral separation (Daicel Chiralpak IG column, 250 × 30 mm I.D., 5 µm; mobile phase A: supercritical carbon dioxide; mobile phase B: methanol and DEA, (DEA accounts for 0.05% of the volume of mobile phase B, *i.e.,* the total volume); volume ratio of mobile phase A to B: 80:20; isocratic elution; flow rate: 55 g/min; column temperature: room temperature (25 to 28°C); injection volume: 0.4 mL; detection wavelength: 220 nm; to obtain compound 028-A (retention time Rt = 4.963 min, 12.1 mg, purity: 99.328%) and compound 028-B (retention time Rt = 5.364 min, 12.0 mg, purity: 99.024%).

LC-MS: [M+H]⁺ =506.15.

Compound 028-A: ¹H NMR (400 MHz, DMSO-*d₆*) δ 7.73 (d, *J* = 7.6 Hz, 1H), 7.68 (s, 1H), 7.35 (s, 1H), 7.20 (d, *J =* 7.6 Hz, 2H), 5.93 (s, 1H), 3.87 (s, 3H), 2.83 (s, 1H), 2.67 (s, 2H), 2.43 (s, 1H), 2.33 (d, *J=* 13.6 Hz, 1H), 2.04 - 1.95 (m, 1H), 1.93-1.84 (m, 1H), 1.68 (d, *J* = 12.0 Hz, 1H), 1.57 - 1.37 (m, 4H), 1.31 (d, *J* = 8.0 Hz, 2H), 1.23 (s, 2H).

Compound 028-B: ¹H NMR (400 MHz, DMSO-*d₆*) δ 7.74 - 7.69 (m, 2H), 7.35 (s, 1H), 7.18 (s, 2H), 5.93 (s, 1H), 3.88 (s, 3H), 2.87-2.73 (m, 1H), 2.67 (s, 1H), 2.43 (s, 3H), 2.33 (s, 1H), 1.99 (d, *J =* 16.0 Hz, 1H), 1.89 (d, *J =* 16.0 Hz, 1H), 1.68 (s, 2H), 1.62 - 1.40 (m, 4H), 1.37 - 1.26 (m, 2H), 1.23 (s, 1H).

### Example 29: Preparation of 2-(1-((6-(5-(((1-(3,3-difluorocyclobutyl)ethoxy)carbonyl)amino)-1-methyl-1H-1,2,3-triazol-4-yl)-2-methylpyridin-3-yl)ethynyl)cyclopropyl)acetic acid (compound 029)

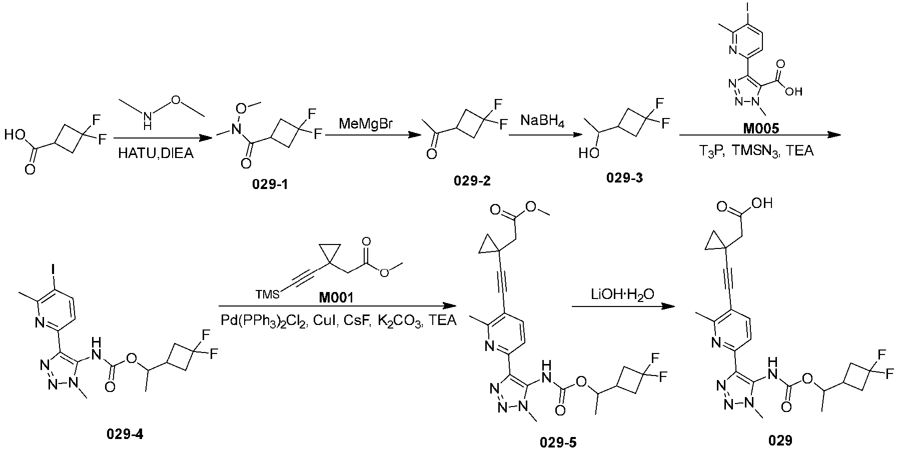

The preparation of compound 029 was referred to the method for preparing compound 008 in Example 8, wherein the starting material 1-cyclopropylethanol was replaced with compound 029-3, and compound 029 (40.7 mg) was obtained. LC-MS: [M+H]⁺=474.10.

¹H NMR (400 MHz, *CD₃OD)* δ 7.76 - 7.69 (m, 2H), 4.92 - 4.87 (m, 1H), 4.00 (s, 3H), 2.62 (s, 3H), 2.59 - 2.47 (m, 4H), 2.43 - 2.23 (m, 3H), 1.31 - 1.16 (m, 3H), 1.12 (dd, *J =* 7.0, 4.6 Hz, 2H), 0.98 (dd, *J =* 7.0, 4.6 Hz, 2H).

Compound 029-3 was commercially available or prepared according to Preparation Example 21.

### Example 30: Preparation of (R)-2-(1-((6-(5-((((1-cyclopropylethyl)(methyl)carbamoyl)oxy)methyl)-1-methyl-1H-1,2,3-triazol-4-yl)-2-methylpyridin-3-yl)ethynyl)cyclopropyl)acetic acid (compound 030)

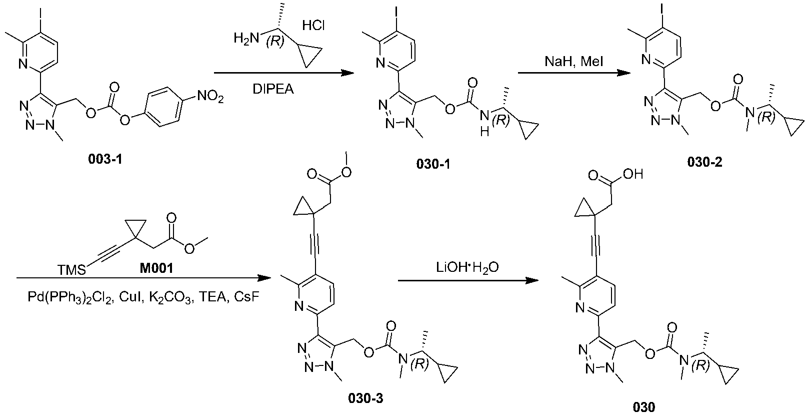

The preparation of compound 030 was referred to the method for preparing compound 009 in Example 9, wherein the starting material was replaced from (1-fluorocyclobutyl)methanamine hydrochloride to (*R*)-1-cyclopropylethan-1-amine hydrochloride, and compound 030 (73.1 mg) was obtained. LC-MS: [M+H]⁺=452.10.

¹H NMR (400 MHz, DMSO-*d*₆) δ 7.84 (d, *J =* 8.0 Hz, 1H), 7.72 (d, *J =* 8.0 Hz, 1H), 5.62 (d, *J =* 25.8 Hz, 2H), 4.10 (s, 3H), 3.09 (s, 1H), 2.54 (s, 3H), 2.45 (s, 2H), 1.23 - 0.81 (m, 8H), 0.54 - -0.04 (m, 4H).

### Example 31: Preparation of trans-2-((6-(5-((((R)-1-(2-chlorophenyl)ethoxy)carbonyl)amino)-1-methyl-1H-1,2,3-triazol-4-yl)-2-methylpyridin-3-yl)ethynyl)cyclohexane-1-carboxylic acid (compound 031), compound 031-A, and compound 031-B

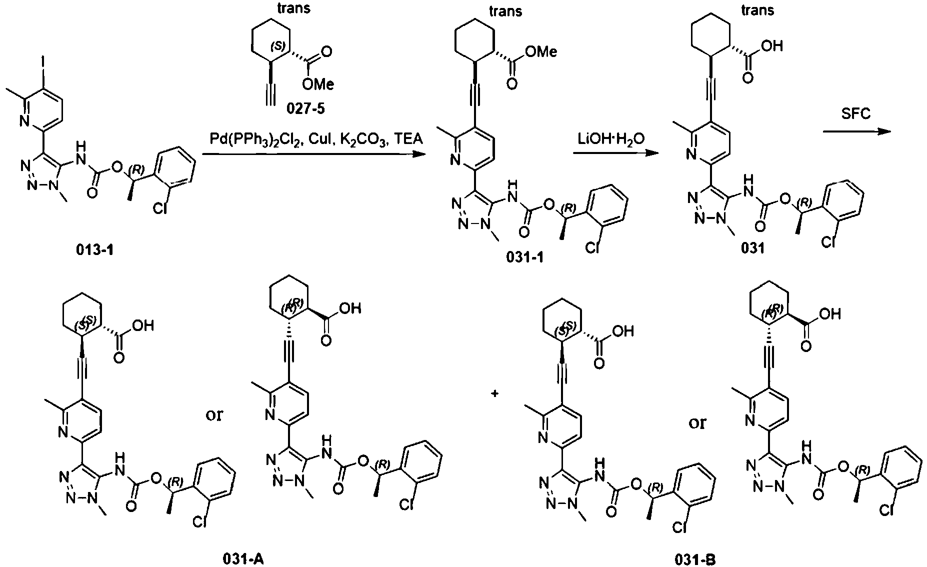

The preparation of compound 031 was referred to the method for preparing compound 027 in Example 27, wherein 027-1 was replaced with compound 013-1, and compound 031 (65 mg) was obtained. Compound 031 was subjected to chiral separation (separation column: CHIRALART Cellulose-SC, IC column, 30 × 250 mm; mobile phase A: n-hexane and diethylamine (diethylamine accounts for 0.1% of the volume of mobile phase A), mobile phase B: ethanol; volume ratio of mobile phase A to B: 7:3; isocratic elution; flow rate: 30 mL/min; column temperature: room temperature (25 to 28°C); injection volume: 1.8 mL; detection wavelength: 220 nm) to obtain compound 031-A (retention time Rt = 8.410 min, 7.4 mg) and compound 031-B (retention time Rt = 7.53 min, 5.2 mg). LC-MS: [M+H]⁺= 522.20.

031-A: ¹H NMR (400 MHz, DMSO-*d*₆) δ 7.71 (d, *J* = 8.0 Hz, 1H), 7.65 (d, *J* = 8.0 Hz, 1H), 7.35-7.33 (m, 3H), 5.96 (s, 1H), 3.86 (s, 3H), 2.79 (t, *J* = 8.4 Hz, 1H), 2.43 (s, 3H), 2.38 - 2.26 (m, 1H), 1.97 (d, *J* = 10.8 Hz, 1H), 1.88 (d, *J =* 11.6 Hz, 1H), 1.66 (s, 2H), 1.56 - 1.14 (m, 7H).

031-B: ¹H NMR (400 MHz, DMSO-*d*₆) δ 12.32 (s, 1H), 9.75 (s, 1H), 7.72 (d, *J* = 8.0 Hz, 1H), 7.66 (s, 1H), 7.35 - 7.33 (m, 3H), 5.97 (s, 1H), 3.87 (s, 3H), 2.79 - 2.76 (m, 1H), 2.43 (s, 3H), 2.40 - 2.30 (m, 1H), 1.99 (d, *J* = 13.2 Hz, 1H), 1.89 (d, *J* = 14.0 Hz, 1H), 1.67 (s, 2H), 1.59 - 1.15 (m, 7H).

### Example 32: Preparation of 2-(1-((6-(5-(((1-cyclobutylethoxy)carbonyl)amino)-1-methyl-1H-1,2,3-triazol-4-yl)-2-methylpyridin-3-yl)ethynyl)cyclopropyl)acetic acid (compound 032), compound 032-A, and compound 032-B

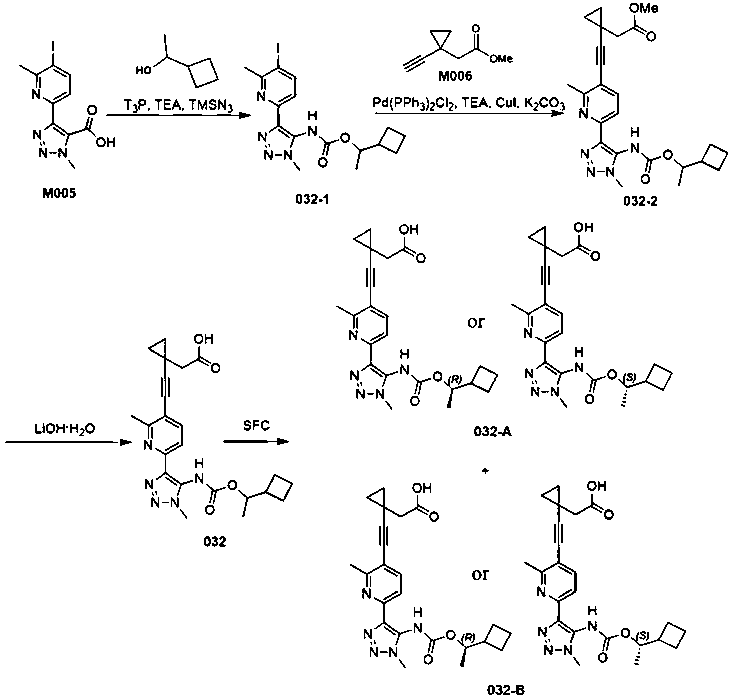

The preparation of compound 032 was referred to the method for preparing compound 013 in Example 13, wherein (R)-1-(2-chlorophenyl)ethan-1-ol was replaced with 1-cyclobutylethan-1-ol, and compound 032 (80.0 mg) was obtained. LC-MS: [M+H]⁺ =438.15.

Compound 032 (128 mg) was subjected to chiral separation (CHIRALART Amylose-CNEO, AD-H column, 30 × 250 mm; mobile phase A: supercritical carbon dioxide; mobile phase B: 80% n-hexane and 20% ethanol by volume; volume ratio of mobile phase A to B: 80:20; isocratic elution; flow rate: 55 g/min, column temperature: room temperature (25 to 28°C); injection volume: 0.3 mL; detection wavelength: 220 nm) to obtain compound 032-A (retention time Rt = 3.241 min, 36.0 mg, purity: 99.7%) and compound 032-B (retention time Rt = 3.424 min, 33.0 mg, purity: 99.1%). LC-MS: [M+H]⁺ =438.40.

032-A: ¹H NMR (400 MHz, *CD₃OD)* δ 7.71 (s, 2H), 4.82 - 4.76 (m, 1H), 4.01 (s, 3H), 2.62 (s, 3H), 2.47 (s, 3H), 1.96 - 1.79 (m, 6H), 1.15 - 1.08 (m, 5H), 1.00 - 0.96 (m, 2H).

032-B: ¹H NMR (400 MHz, *CD₃OD*) δ 7.70 (s, 2H), 4.81- 4.77 (m, 1H), 4.00 (s, 3H), 2.63 (s, 3H), 2.44 (s, 3H), 2.03 - 1.70 (m, 6H), 1.15-1.02 (m, 5H), 0.99-0.95 (m, 2H).

### Example 33: Preparation of trans-2-(6-(5-(((R)-1-(2-chlorophenylethoxy)carbonyl)amino)-1-methyl-1H-1,2,3-triazol-4-yl)-2-methylpyridin-3-yl)ethynyl)cyclobutane-1-carboxylic acid (compound 033)

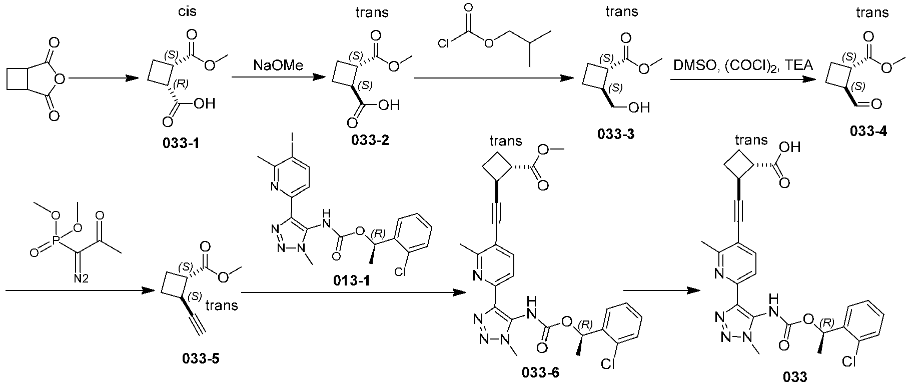

(1) At room temperature, 3-oxabicyclo[3.2.0]heptane-2,4-dione (5.0 g, 39.68 mmol) was dissolved in methanol (2.5 mL, 59.52 mmol). The reaction mixture was heated to 100°C and stirred for 2 hours. After cooling to room temperature, the resulting reaction mixture was directly concentrated under reduced pressure. The residue was purified by silica gel column chromatography (petroleum ether/ethyl acetate = 2/1) to obtain compound 033-1 (5.5 g, containing a pair of cis configurations). LC-MS: [M+H]⁺= 159.10.
(2) At room temperature, compound 033-1 (6.0 g, 37.97 mmol) was dissolved in methanol (10 mL), and a solution of sodium methoxide in methanol (90 mL, 15%) was added. The reaction mixture was heated to 70°C and stirred for 2 hours. After cooling to 0°C, the resulting reaction mixture was slowly poured into dilute hydrochloric acid (300 mL, 2 M), and the pH was adjusted to 5 to 6. The mixture was then diluted with water (200 mL) and extracted with ethyl acetate (100 mL). The combined organic phases were sequentially washed with saturated brine (50 mL), dried over anhydrous sodium sulfate, filtered, and the filtrate was concentrated under reduced pressure to obtain compound 033-2 (4.6 g, crude, containing a pair of trans configurations). LC-MS: [M+H]⁺=159.10.
(3) At room temperature, compound 033-2 (2.6 g, 16.45 mmol) and triethylamine (3.3 g, 32.91 mmol) were dissolved in tetrahydrofuran (30 mL). The reaction mixture was cooled to 0°C, and isobutyl chloroformate (2.7 g, 19.75 mmol) was slowly added dropwise. After the dropwise addition was completed, the reaction mixture was warmed to room temperature and stirred for an additional 1 hour. The reaction mixture was cooled to 0°C, and then a solution of sodium borohydride (1.9 g, 49.35 mmol) in water (10 mL) was slowly added. The reaction mixture was warmed to room temperature and stirred for an additional 1 hour. Dilute hydrochloric acid (1 M) was added to the resulting reaction mixture to adjust the pH to 5 to 6. Water (50 mL) was added to dilute, followed by extraction with ethyl acetate (30 mL). The organic phases were combined. The resulting organic phase was sequentially washed with saturated brine (30 mL), dried over anhydrous sodium sulfate, filtered, and the filtrate was concentrated under reduced pressure. The resulting residue was purified by silica gel column chromatography (petroleum ether/ethyl acetate = 2/1) to obtain compound 033-3 (1.3 g, containing a pair of trans configurations). LC-MS: [M+H]⁺=145.15.
(4) At room temperature, dimethyl sulfoxide (0.98 mL, 13.88 mmol) was dissolved in dichloromethane (10 mL). After cooling the reaction mixture to -78°C, oxalyl chloride (0.58 mL, 6.94 mmol) was slowly added dropwise. After the addition was completed, the reaction mixture was stirred at - 78°C for 0.5 hours. Then, compound 033-3 (500 mg, 3.47 mmol) was added dropwise. The reaction mixture was stirred at -78°C for an additional 0.5 hours. Finally, triethylamine (3.8 mL, 27.76 mmol) was slowly added dropwise, maintaining the internal temperature of the reaction system below -70°C. After the dropwise addition was completed, the reaction mixture was warmed to room temperature and stirred for an additional 0.5 hours. The resulting reaction mixture was poured into water (30 mL) to quench and extracted with dichloromethane (30 mL). The organic phases were combined. The resulting organic phase was washed with saturated brine (30 mL), dried over anhydrous sodium sulfate, filtered, and the filtrate was concentrated under reduced pressure to obtain compound 033-4 (540 mg, crude, containing a pair of trans configurations).
(5) At room temperature, compound 033-4 (400 mg, 2.82 mmol) was dissolved in methanol (6 mL). After cooling the reaction mixture to 0°C, dimethyl (1-diazo-2-oxopropyl)phosphonate (2.3 g, 12.0 mmol) and potassium carbonate (974 mg, 7.05 mmol) were added. The reaction was warmed to room temperature and stirred for an additional 16 hours. The resulting reaction mixture was diluted with water (50 mL), extracted with dichloromethane (20 mL), and the organic phases were combined. The resulting organic phase was washed with saturated brine (20 mL), dried over anhydrous sodium sulfate, filtered, and the filtrate was concentrated under reduced pressure. The residue was purified by silica gel column chromatography (petroleum ether/ethyl acetate = 10/1) to obtain compound 033-5 (120 mg, containing a pair of trans configurations).
(6) At room temperature, compound 033-5 (200 mg, 1.45 mmol) and compound 013-1 (360 mg, 0.73 mmol) were dissolved in 1,4-dioxane (6 mL), followed by the addition of potassium carbonate (200 mg, 1.45 mmol), bis(triphenylphosphine)palladium(II) chloride (51 mg, 0.073 mmol), copper(I) iodide (28 mg, 0.14 mmol), and triethylamine (221 mg, 2.19 mmol). Under a nitrogen atmosphere, the reaction mixture was heated to 100°C and stirred for 3 hours. After cooling to room temperature, the resulting reaction mixture was directly concentrated, diluted with water (30 mL), and extracted with ethyl acetate (20 mL). The organic phases were combined. The resulting organic phase was washed with saturated brine (20 mL), dried over anhydrous sodium sulfate, filtered, and the filtrate was concentrated under reduced pressure. The resulting residue was purified by silica gel column chromatography (petroleum ether/ethyl acetate = 2/1) to obtain compound 033-6 (110 mg, containing a pair of trans configurations). LC-MS: [M+H]⁺ = 508.10.
(7) At room temperature, compound 033-6 (110 mg, 0.22 mmol) was dissolved in a mixed solvent of methanol (2 mL) and water (2 mL), and lithium hydroxide monohydrate (28 mg, 0.66 mmol) was added. The reaction mixture was stirred at room temperature for 16 hours. After adjusting the pH to 7, the resulting reaction mixture was directly concentrated under reduced pressure. The residue was purified by high-performance liquid chromatography (0.1% ammonia in water) and lyophilized to obtain compound 033 (14.3 mg, containing a pair of trans configurations). LC-MS: [M+H]⁺ =494.05.

¹H NMR (400 MHz, DMSO-*d*₆) δ 7.75 (s, 3H), 7.42 - 7.31 (m, 3H), 5.99 (s, 1H), 3.89 (s, 3H), 3.48 - 3.45 (m, 2H), 3.22 - 3.15 (m, 3H), 2.67 (s, 1H), 2.48 (s, 2H), 2.32 - 2.21 (m, 1H), 2.14 - 2.03 (m, 3H).

### Example 34: Preparation of 2-(1-((6-(5-((((4-fluorobutyl)(methyl)carbamoyl)oxy)methyl)-1-methyl-1H-1,2,3-triazol-4-yl)-2-methylpyridin-3-yl)ethynyl)cyclopropyl)acetic acid (compound 034)

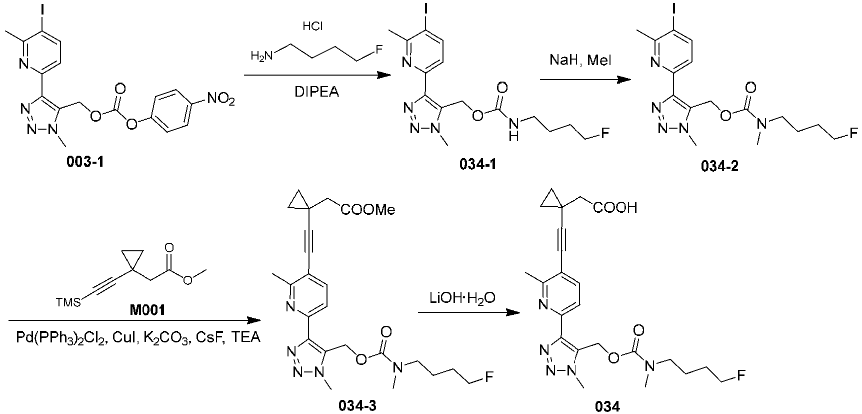

The preparation of compound 034 was referred to the method for preparing compound 009 in Example 9, wherein (1-fluorocyclobutyl)methylamine hydrochloride was replaced with 4-fluorobutan-1-amine hydrochloride, and compound 034 (28.1 mg) was obtained. LC-MS [M+H]⁺= 458.10.

¹H NMR (400 MHz, DMSO-*d₆*) δ 7.84 (d, *J =* 8.0 Hz, 1H), 7.72 (d, *J =* 8.0 Hz, 1H), 5.66 - 5.64 (m, 2H), 4.48 - 4.17 (m, 2H), 4.11 (s, 3H), 3.21 - 3.11 (m, 2H), 2.79 - 2.74 (m, 3H), 2.54 (s, 3H), 2.44 (s, 2H), 1.59 - 1.53 (m, 2H), 1.38 (s, 2H), 1.05 - 1.01 (m, 2H), 0.96 - 0.93 (m, 2H).

### Example 35: Preparation of 2-(1-((6-(5-(((((3,3-difluorocyclobutyl)methyl)(methyl)carbamoyl)oxy)methyl)-1-methyl-1H-1,2,3-triazol-4-yl)-2-methylpyridin-3-yl)ethynyl)cyclopropyl)acetic acid (compound 035)

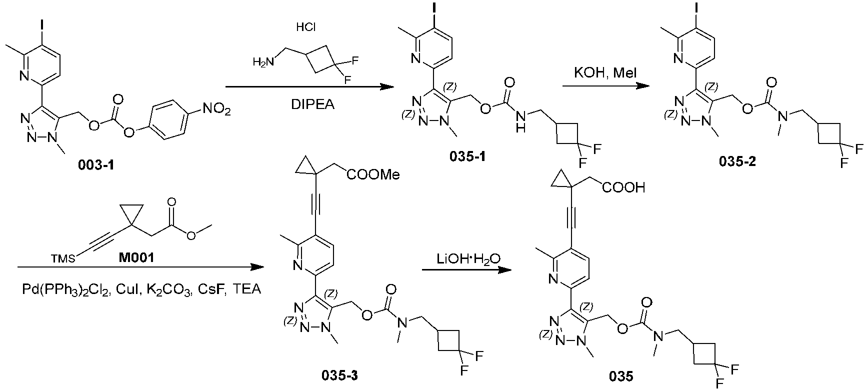

The preparation of compound 035 was referred to the method for preparing compound 009 in Example 9, wherein (1-fluorocyclobutyl)methylamine hydrochloride was replaced with (3,3-difluorocyclobutyl)methylamine hydrochloride, and compound 035 (8.9 mg) was obtained. LC-MS: [M+H]⁺= 488.10.

¹H NMR (400 MHz, DMSO-*d₆*) δ 7.83 (t, *J* = 6.8 Hz, 1H), 7.76 - 7.67 (m, 1H), 5.65 (d, *J* = 11.6 Hz, 2H), 4.11 (s, 3H), 3.25 - 3.20 (m, 2H), 2.77 (d, *J* = 22.8 Hz, 3H), 2.53 (s, 2H), 2.52 (d, *J* = 2.0 Hz, 1H), 2.43 (s, 2H), 2.33 (s, 1H), 2.30 - 2.25 (m, 1H), 2.16 (d, *J =* 12.9 Hz, 1H), 2.15 - 2.04 (m, 1H), 1.04 (t, *J =* 5.3 Hz, 2H), 0.95 (m 2H).

### Example 36: Preparation of 2-(1-((6-(5-((((2-fluorobutyl)(methyl)carbamoyl)oxy)methyl)-1-methyl-1H-1,2,3-triazol-4-yl)-2-methylpyridin-3-yl)ethynyl)cyclopropyl)acetic acid (compound 036)

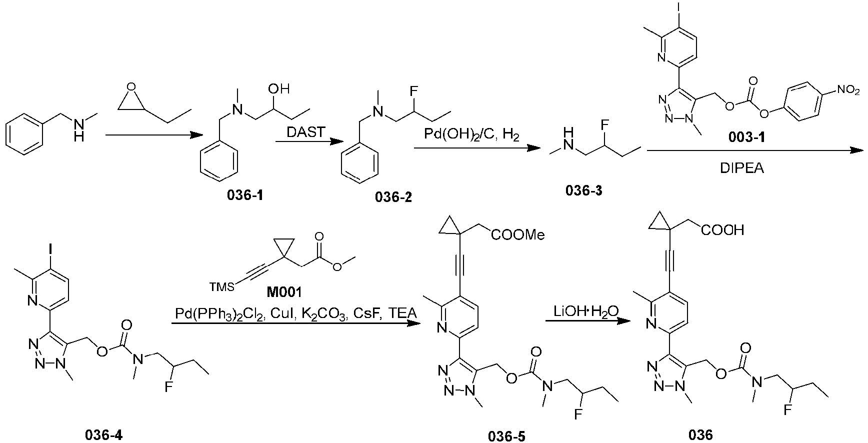

The preparation of compound 036 was referred to the method for preparing compound 003 in Example 3, wherein N-methylisobutylamine was replaced with compound 036-3, and compound 036 (54.3 mg) was obtained. LC-MS: [M+H]⁺= 458.10.

¹H NMR (400 MHz, DMSO-*d₆*) δ7.84 (d, *J =* 8.0 Hz, 1H), 7.73 (d, *J =* 8.0 Hz, 1H), 5.66 (d, *J =* 21.6 Hz, 2H), 4.68-4.27 (m, 2H), 4.11 (s, 3H), 3.45-3.34 (m, 2H), 2.83 (d, *J =* 20.0 Hz, 3H), 2.53 (s, 3H), 2.44 (s, 2H), 1.62 - 1.49 (m, 1H), 1.41 - 1.31 (m, 1H), 1.04 (dd, *J =* 5.2, 3.6 Hz, 2H), 0.96 - 0.93 (m, 3H), 0.70 - 0.67 (m, 2H).

The preparation method for compound 036-3 is as follows:
(1) At room temperature, N-methyl-1-phenylmethanamine (61 mL, 461.2 mmol) and 2-ethyloxirane (10 mL, 0.83 mmol) were dissolved in ethanol (500 mL). The reaction mixture was heated to 90°C and stirred for 16 hours. After cooling to room temperature, the reaction mixture was concentrated under reduced pressure. The resulting residue was purified by silica gel column chromatography (dichloromethane/methanol = 10/1) to obtain compound 036-1 (14.5 g). LC-MS: [M+H]⁺ =194.15.
(2) At room temperature, compound 036-1 (5 g, 25.9 mmol) was dissolved in dichloromethane (30 mL). Under a nitrogen atmosphere, the reaction mixture was cooled to -78°C, and diethylaminosulfur trifluoride (7 mL, 51.8 mmol) was added dropwise. The reaction mixture was stirred at this temperature for 3 hours, then warmed to room temperature, and stirred for an additional 16 hours. The resulting reaction mixture was directly concentrated under reduced pressure. The residue was purified by silica gel column chromatography (dichloromethane/methanol = 20/1) to obtain compound 036-2 (1.2 g). LC-MS: [M+H]⁺ =196.20.
(3) At room temperature, compound 036-2 (0.6 g, 3.1 mmol) was dissolved in methanol (10 mL), followed by the addition of wet palladium hydroxide on carbon (100 mg, 20%). Under a hydrogen atmosphere, the reaction mixture was stirred at room temperature for 1 hour, then heated to 60°C, and reacted for 18 hours. The resulting reaction mixture was filtered through diatomite, and the filtrate was concentrated under reduced pressure to obtain compound 036-3 (4 g, 3 mL methanol solution). LC-MS: [M+H]⁺ =106.20.

### Example 37: Preparation of 2-(1-((6-(5-((((3-fluorobutyl)(methyl)carbamoyl)oxy)methyl)-1-methyl-1H-1,2,3-triazol-4-yl)-2-methylpyridin-3-yl)ethynyl)cyclopropyl)acetic acid (compound 037)

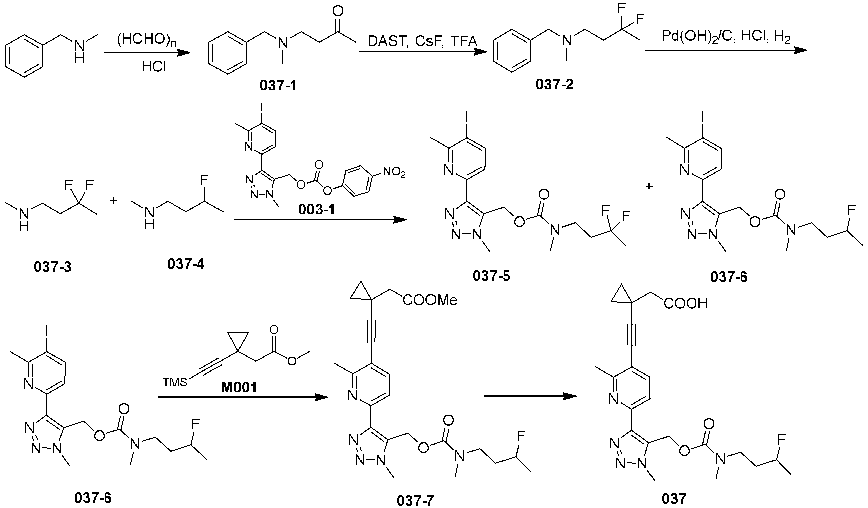

(1) At room temperature, N-methyl-1-phenylmethanamine (10 g, 82.52 mmol) was dissolved in acetone (200 mL). Concentrated hydrochloric acid (6.9 mL, 82.52 mmol, 12 M), paraformaldehyde (11.2 g, 123.78 mmol), and isopropanol (8 mL) were added to the reaction mixture. The reaction mixture was heated to 80°C and stirred for 16 hours. The resulting reaction mixture was concentrated under reduced pressure. The residue was purified by silica gel column chromatography (ethyl acetate/petroleum ether = 10/1) to obtain compound 037-1 (15.7 g). LC-MS [M+H]⁺=192.10.
(2) At room temperature, compound 037-1 (5.0 g, 26.14 mmol) and diethylaminosulfur trifluoride (12.6 g, 78.42 mmol) were dissolved in dichloromethane (50 mL), followed by the addition of cesium fluoride (1.2 g, 7.84 mmol) and trifluoroacetic acid (1 mL). The reaction mixture was stirred at room temperature for 16 hours. The resulting reaction mixture was slowly poured into saturated aqueous sodium bicarbonate solution (100 mL) to quench and extracted with dichloromethane (50 mL). The organic phases were combined. The resulting organic phase was washed with dilute hydrochloric acid (50 mL), and the aqueous phases were combined. The resulting aqueous phase was adjusted to pH = 8 to 9 with saturated sodium carbonate solution and then extracted with dichloromethane (50 mL). The organic phases were combined. The resulting organic phase was dried over anhydrous sodium sulfate, filtered, and the filtrate was concentrated under reduced pressure. The resulting residue was purified by silica gel column chromatography (ethyl acetate/petroleum ether = 10/1) to obtain compound 037-2 (1 g). LC-MS: [M+H]⁺=214.15.
(3) At room temperature, compound 037-2 (1 g, 4.69 mmol) was dissolved in ethanol (50 mL), followed by the addition of wet palladium hydroxide on carbon (200 mg, 20%) and concentrated hydrochloric acid (1 mL, 12 M). Under a hydrogen atmosphere, the reaction mixture was heated to 60°C and stirred for 2 hours. After cooling to room temperature, the resulting reaction mixture was directly filtered under reduced pressure. The resulting filter cake was washed with ethanol (10 mL). The filtrate was concentrated under reduced pressure to obtain a mixture of compound 037-3 hydrochloride and compound 037-4 hydrochloride (800 mg, crude). LC-MS: [M+H]⁺=124.15.
(4) At room temperature, a mixture of compound 037-3 hydrochloride and compound 037-4 hydrochloride (534 mg, crude) was added to dichloromethane (10 mL), followed by the addition of compound 003-1 (555 mg, 1.12 mmol) and N,N-diisopropylethylamine (577 mg, 4.46 mmol). The reaction mixture was stirred at room temperature for 1 hour. The resulting reaction mixture was poured into water (30 mL) to dilute and extracted with dichloromethane (30 mL). The organic phases were combined. The resulting organic phase was dried over anhydrous sodium sulfate, filtered, and the filtrate was concentrated under reduced pressure. The resulting residue was purified by high-performance liquid chromatography (ammonia in water conditions) to obtain compound 037-5 (210 mg) and compound 037-6 (190 mg).
   LC-MS of compound 037-5: [M+H]⁺=480.07.
   LC-MS of compound 037-6: [M+H]⁺=462.07.
(5) At room temperature, compound 037-6 (155 mg, 0.34 mmol) was dissolved in 1,4-dioxane (10 mL), followed by the addition of M001 (142 mg, 0.68 mmol), triethylamine (103 mg, 1.01 mmol), copper(I) iodide (13 mg, 0.07 mmol), bis(triphenylphosphine)palladium(II) chloride (24 mg, 0.03 mmol), potassium carbonate (93 mg, 0.68 mmol), and cesium fluoride (205 mg, 1.35 mmol). The reaction mixture was heated to 90°C under a nitrogen atmosphere and stirred for 16 hours. After cooling to room temperature, the resulting reaction mixture was diluted with water (10 mL) and extracted with ethyl acetate (10 mL). The combined organic phases were dried over anhydrous sodium sulfate, filtered, and the filtrate was concentrated under reduced pressure. The resulting residue was purified by silica gel thin-layer chromatography (petroleum ether/ethyl acetate = 1/1) to obtain compound 037-7 (100 mg, crude). LC-MS: [M+H]⁺=472.15.
(6) At room temperature, compound 037-7 (80 mg, 0.17 mmol) was dissolved in a mixed solvent of methanol (2 mL) and water (2 mL), followed by the addition of lithium hydroxide monohydrate (21 mg, 0.51 mmol). The reaction mixture was stirred at room temperature for 2 hours. The resulting reaction mixture was diluted with water (10 mL) and directly concentrated under reduced pressure. The residue was washed with dichloromethane (10 mL), and the combined aqueous phases were adjusted to pH = 3 to 4 with dilute hydrochloric acid (1 M), then extracted with ethyl acetate (10 mL). The combined organic phases were dried over anhydrous sodium sulfate, filtered, and the filtrate was concentrated under reduced pressure. The resulting residue was purified by high-performance liquid chromatography (0.1% hydrochloric acid system) and lyophilized to obtain compound 037 (21.2 mg). LC-MS: [M+H]⁺=458.15.

¹H NMR (400 MHz, DMSO-*d₆*) δ 7.85 (d, *J =* 8.4 Hz, 1H), 7.75 (d, *J =* 8.4 Hz, 1H), 5.65 (d, *J =* 10.4 Hz, 2H), 4.56 (s, 1H), 4.11 (s, 3H), 3.40 - 3.11 (m, 2H), 2.77 (d, *J =* 19.2 Hz, 3H), 2.54 (s, 3H), 2.46 (s, 2H), 1.84 - 0.88 (m, 9H).

### Example 38: Preparation of 2-(1-((6-(5-((((3,3-difluorobutyl)(methyl)carbamoyl)oxy)methyl)-1-methyl-1H-1,2,3-triazol-4-yl)-2-methylpyridin-3-yl)ethynyl)cyclopropyl)acetic acid (compound 038)

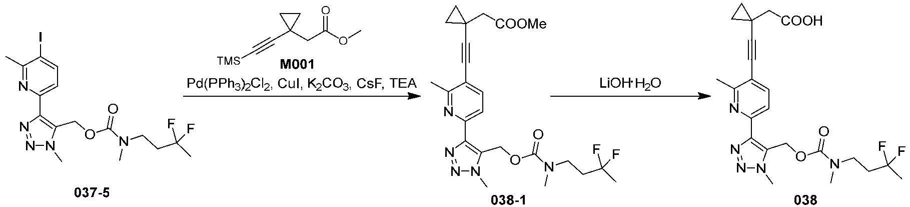

The preparation of compound 038 was referred to the method for preparing compound 037 in Example 37, wherein compound 037-6 was replaced with compound 037-5, and compound 038 (24.3 mg) was obtained. LC-MS: [M+H]⁺= 476.10.

¹H NMR (400 MHz, DMSO-*d₆*) δ 7.83 (d, *J =* 8.0 Hz, 1H), 7.72 (d, *J =* 8.0 Hz, 1H), 5.63 (d, *J =* 12.0 Hz, 2H), 4.09 (s, 3H), 3.37 - 3.20 (m, 2H), 2.75 (d, *J =* 17.6 Hz, 3H), 2.52 (s, 3H), 2.44 (s, 2H), 2.18 - 1.84 (m, 2H), 1.63 - 1.34 (m, 3H), 1.08 - 0.86 (m, 4H).

### Example 39: Preparation of 2-(1-((6-(5-((((cyclopentylmethyl)(methyl)carbamoyl)oxy)methyl)-1-methyl-1H-1,2,3-triazol-4-yl)-2-methylpyridin-3-yl)ethynyl)cyclopropyl)acetic acid (compound 039)

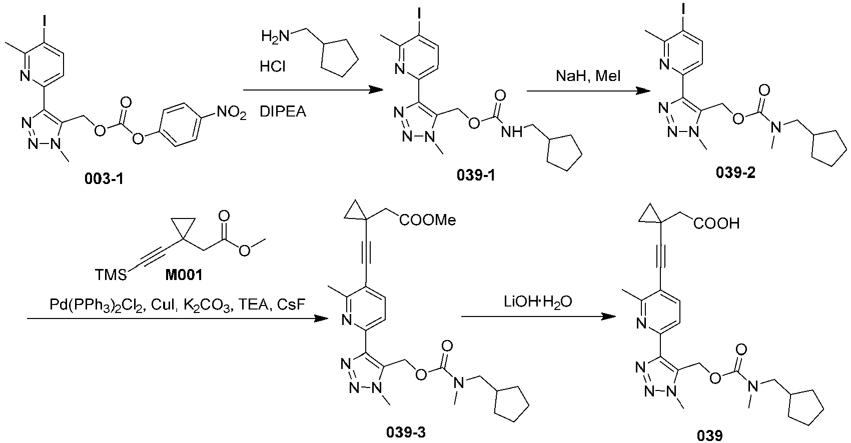

The preparation of compound 039 was referred to the method for preparing compound 009 in Example 9, wherein the starting material (1-fluorocyclobutyl)methylamine hydrochloride was replaced with cyclopentylethylamine hydrochloride, and compound 039 (51.3 mg) was obtained. LC-MS: [M+H]⁺ = 466.15.

¹H NMR (400 MHz, DMSO-*d₆*) δ 7.83 (d, *J =* 8.0 Hz, 1H), 7.72 (d, *J =* 8.0 Hz, 1H), 5.64 (d, *J =* 18.4 Hz, 2H), 4.11 (s, 3H), 3.13 (d, *J* = 7.0 Hz, 1H), 2.99 (d, *J* = 7.0 Hz, 1H), 2.76 (d, *J* = 17.6 Hz, 3H), 2.54 (s, 3H), 2.45 (s, 2H), 2.17 - 1.87 (m, 1H), 1.67 - 1.24 (m, 6H), 1.18 - 0.85 (m, 6H).

### Example 40: Preparation of 2-(1-((6-(5-(((sec-butyl(methyl)carbamoyl)oxy)methyl)-1-methyl-1H-1,2,3-triazol-4-yl)-2-methylpyridin-3-yl)ethynyl)cyclopropyl)acetic acid (compound 040)

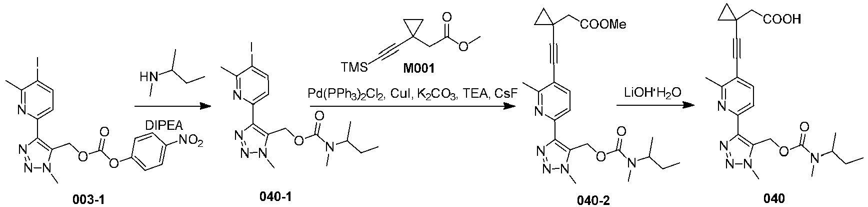

The preparation of compound 040 was referred to the method for preparing compound 003 in Example 3, wherein the starting material N-methylisobutylamine was replaced with N-methylbutan-2-amine, and compound 040 (40.8 mg) was obtained. LC-MS: [M+H]⁺ = 440.15.

¹H NMR (400 MHz, DMSO-*d*₆) δ 7.83 (d, *J =* 8.0 Hz, 1H), 7.72 (d, *J =* 8.0 Hz, 1H), 5.67 - 5.63 (m, 2H), 4.11 (s, 3H), 2.67 - 2.62 (m, 3H), 2.56 (s, 2H), 2.54 (s, 3H), 2.52 (d, *J =* 2.0 Hz, 2H), 2.42 (s, 2H), 1.42 - 1.23 (m, 2H), 1.06 - 0.99 (m, 3H), 0.97 - 0.90 (m, 4H), 0.74 (t, *J=* 7.2 Hz, 2H), 0.59 (d, *J=* 7.2 Hz, 1H.

### Example 41: Preparation of 2-(1-((6-(5-(((1-cyclopentylethoxy)carbonyl)amino)-1-methyl-1H-1,2,3-triazol-4-yl)-2-methylpyridin-3-yl)ethynyl)cyclopropyl)acetic acid (compound 092')

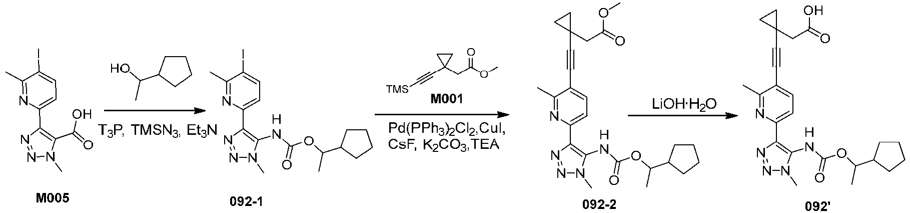

The preparation of compound 092' was referred to the method for preparing compound 008 in Example 8, wherein the starting material 1-cyclopropylethanol was replaced with 2-cyclopentylethanol, and compound 092' (231.5 mg) was obtained.

LC-MS: [M+H]⁺ =452.15.

¹H NMR (400 MHz, CD₃OD) δ 9.41 (s, 1H), 7.75 -7.68 (m, 2H), 4.58 (t, *J =* 6.0 Hz, 1H), 3.88 (s, 3H), 2.52 (s, 3H), 2.45 (s, 2H), 1.95 (s, 1H), 1.79 - 1.20 (m, 8H), 1.26 - 1.10 (m, 3H), 1.09 - 1.03 (m, 2H), 0.98 - 0.92 (m, 2H).

### Example 42: Preparation of 2-(1-((6-(5-(((bicyclo[1.1.1]pentan-1-ylmethyl)(methyl)carbamoyl)oxy)methyl)-1-methyl-1H-1,2,3-triazol-4-yl)-2-methylpyridin-3-yl)ethynyl)cyclopropyl)acetic acid (compound 204)

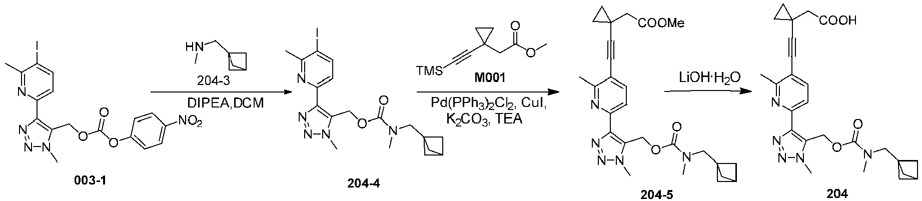

The preparation of compound 204 was referred to the method for preparing compound 003 in Example 3, wherein the starting material was replaced from *N*-methylisobutylamine to the hydrochloride of compound 204-3, and compound 204 (80.9 mg) was obtained.

LC-MS: [M+H]⁺=464.10.

¹H NMR (400 MHz, DMSO-*d₆*) δ 7.84 (d, *J =* 8.0 Hz, 1H), 7.73 (d, *J =* 8.0 Hz, 1H), 5.65 (d, *J =* 8.0 Hz, 2H), 4.11 (s, 3H), 3.21 (s, 2H), 3.11 (s, 1H), 2.78 (d, *J =* 24.0 Hz, 3H), 2.55 (s, 3H), 2.45 (s, 2H), 2.36-2.31 (m, 1H), 1.66 (s, 2H), 1.49 (s, 3H), 1.05 (dd, *J* = 4.0, 4.0 Hz, 2H), 0.95 (dd, *J =* 4.0, 4.0 Hz, 2H).

### Example 43: Preparation of trans-2-((6-(5-(((((R)-1-cyclopropylpropan-2-yl)oxy)carbonyl)amino)-1-methyl-1H-1,2,3-triazol-4-yl)-2-methylpyridin-3-yl)ethynyl)-4,4-difluorocyclopentane-1-carboxylic acid (compound 205)

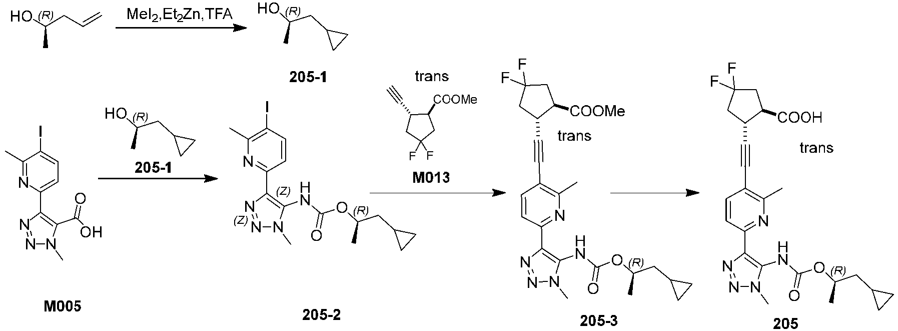

(1) At room temperature, diethylzinc (4.64 mL, 4.64 mmol, 1 M in hexane) was dispersed in dichloromethane (5 mL). After cooling the reaction mixture to 0°C, trifluoroacetic acid (264 mg, 2.32 mmol) was slowly added dropwise. After the dropwise addition was completed, the reaction mixture was stirred at 0°C for 15 minutes, followed by the addition of diiodomethane (2.4 g, 9.28 mmol). The reaction mixture was further stirred at 0°C for 0.5 hours. Then, a solution of (*R*)-4-penten-2-ol (200 mg, 2.32 mmol) in dichloromethane (5 mL) was added dropwise. After warming to room temperature, the reaction mixture was stirred for an additional 5 hours. The reaction mixture was quenched with water (10 mL) and extracted with dichloromethane (20 mL × 3). The combined organic phases were washed with saturated brine (30 mL), dried over anhydrous sodium sulfate, filtered, and the filtrate was concentrated under reduced pressure to obtain compound 205-1 (200 mg, crude).
(2) At room temperature, M005 (345 mg, 1.0 mmol) was dissolved in tetrahydrofuran (5 mL), followed by the addition of azidotrimethylsilane (345 mg, 3.0 mmol), triethylamine (303 mg, 3.0 mmol), and propylphosphonic anhydride (1.2 g, 1.99 mmol, 50% in ethyl acetate). The reaction mixture was stirred at room temperature for 0.5 hours, and then compound 205-1 (200 mg, crude) was added. The reaction mixture was heated to 70°C and stirred for an additional 1 hour. After cooling to room temperature, the reaction mixture was quenched with water (15 mL) and extracted with ethyl acetate (20 mL × 3). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 3/1) to obtain compound 205-2 (300 mg, containing a pair of trans configurations). LC-MS: [M+H]⁺= 441.00.
(3) At room temperature, compound 205-2 (225 mg, 0.51 mmol) and M013 (192 mg, 1.02 mmol, containing a pair of trans configurations) were dissolved in 1,4-dioxane (4 mL), followed by the addition of bis(triphenylphosphine)palladium(II) chloride (35 mg, 0.051 mmol), copper(I) iodide (9 mg, 0.051 mmol), potassium carbonate (141 mg, 1.02 mmol), and triethylamine (155 mg, 1.53 mmol). The reaction mixture was purged with nitrogen, heated to 90°C under a nitrogen atmosphere, and stirred for 3 hours. After cooling to room temperature, the reaction mixture was diluted with water (5 mL) and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 205-3 (350 mg). LC-MS: [M+H]⁺= 502.25.
(4) At room temperature, compound 205-3 (350 mg, 0.70 mmol) was dissolved in methanol (8 mL) and water (4 mL), followed by the addition of lithium hydroxide monohydrate (88 mg, 2.10 mmol). The reaction mixture was stirred at room temperature for 2 hours. The reaction mixture was diluted with a small amount of water (10 mL), and the pH of the solution was adjusted to 5 to 6 with dilute hydrochloric acid (1 M). Then, the mixture was extracted with ethyl acetate (20 mL × 3). The combined organic phases were dried over anhydrous sodium sulfate, filtered, and the filtrate was concentrated under reduced pressure. The residue was purified by preparative high-performance liquid chromatography (0.1% ammonia in water conditions) and lyophilized to obtain compound 205 (166.2 mg, containing a pair of trans configurations). LC-MS: [M+H]⁺ =488.15.

¹H NMR (400 MHz, DMSO-*d*₆) δ 7.81 - 7.72 (m, 2H), 4.76 (s, 1H), 3.88 (s, 3H), 3.49 - 3.43 (m, 1H), 3.07 - 3.01 (m, 1H), 2.73 - 2.51 (m, 5H), 2.39 - 2.21 (m, 2H), 1.36 - 0.63 (m, 6H), 0.35 (s, 2H), 0.00 (s, 2H).

### Example 44: Preparation of (R)-2-(1-((6-(5-((((4,4-difluoropentan-2-yl)oxy)carbonyl)amino)-1-methyl-1H-1,2,3-triazol-4-yl)-2-(trifluoromethyl)pyridin-3-yl)ethynyl)cyclopropyl)acetic acid (compound 206)

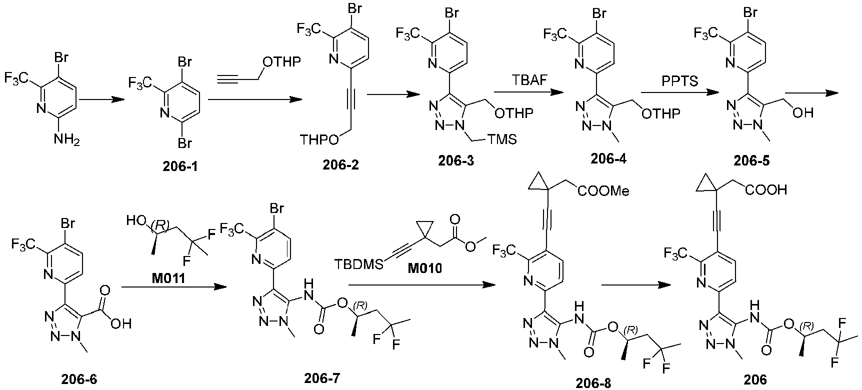

(1) At room temperature, 5-bromo-6-trifluoromethyl-2-aminopyridine (3.5 g, 14.52 mmol) was dispersed in aqueous hydrobromic acid solution (11.7 g, 145.20 mmol, 40%). After cooling the reaction mixture to 0°C, a solution of sodium nitrite (2.5 g, 36.30 mmol) in water (20 mL) was added, followed by the dropwise addition of liquid bromine (2.08 mL, 40.68 mmol). After the addition was completed, the reaction mixture was stirred at 0°C for an additional 1 hour. The reaction mixture was diluted with ethyl acetate (50 mL), washed with water (30 mL), and then washed with saturated brine (60 mL). The organic phase was dried over anhydrous sodium sulfate, filtered, and the filtrate was concentrated under reduced pressure. The residue was purified by silica gel column chromatography (petroleum ether/ethyl acetate = 20/1) to obtain compound 206-1 (2.0 g). LC-MS: [M+H]⁺= 305.75.
(2) At room temperature, compound 206-1 (2.0 g, 6.56 mmol), pyran (1.4 g, 9.84 mmol) were dissolved in acetonitrile (20 mL), followed by the addition of bis(triphenylphosphine)palladium(II) chloride (230 mg, 0.33 mmol), copper(I) iodide (62 mg, 0.33 mmol), and triethylamine (2.0 g, 19.68 mmol). Under a nitrogen atmosphere, the reaction mixture was heated to 50°C and stirred for 3 hours. After cooling to room temperature, the reaction mixture was directly concentrated under reduced pressure. The residue was purified by silica gel column chromatography (petroleum ether/ethyl acetate = 5/1) to obtain compound 206-2 (1.7 g). LC-MS: [M+H]⁺= 363.90.
(3) At room temperature, compound 206-2 (1.7 g, 4.68 mmol) and trimethylsilylmethyl azide (1.2 g, 9.36 mmol) were dissolved in anhydrous tetrahydrofuran (20 mL), followed by the addition of pentamethylcyclopentadienylbis(triphenylphosphine)ruthenium(II) chloride (374 mg, 0.47 mmol, CAS: 92361-49-4), tetrabutylammonium iodide (173 mg, 0.47 mmol), and copper(I) iodide (89 mg, 0.47 mmol). Under a nitrogen atmosphere, the reaction was heated to 40°C and stirred for 16 hours. After cooling to room temperature, the reaction mixture was directly concentrated under reduced pressure. The residue was purified by silica gel column chromatography (petroleum ether/ethyl acetate = 5/1) to obtain compound 206-3 (2.0 g). LC-MS: [M-THP+H]⁺ = 493.39.
(4) At room temperature, compound 206-3 (2.3 g, 4.67 mmol) was dissolved in tetrahydrofuran (25 mL), and tetrabutylammonium fluoride (1.5 g, 56.10 mmol) was added in portions. The reaction mixture was stirred at room temperature for 1 hour. The reaction mixture was diluted with water (20 mL) and extracted with ethyl acetate (40 mL). The combined organic phases were washed with saturated brine (50 mL). The organic phase was dried over anhydrous sodium sulfate, filtered, and the filtrate was concentrated under reduced pressure. The residue was purified by silica gel column chromatography (petroleum ether/ethyl acetate = 5/1) to obtain compound 206-4 (1.4 g). LC-MS: [M+H]⁺ = 423.00.
(5) At room temperature, compound 206-4 (1.4 g, 3.32 mmol) was dissolved in methanol (15 mL), and pyridinium *p*-toluenesulfonate (835 mg, 3.32 mmol) was added. The reaction was heated to 60°C and stirred for 1 hour. The reaction mixture was diluted with saturated aqueous sodium bicarbonate solution (15 mL) and extracted with ethyl acetate (30 mL × 3). The combined organic phases were washed with saturated brine (30 mL). The organic phase was dried over anhydrous sodium sulfate, filtered, and the filtrate was concentrated under reduced pressure to obtain compound 206-5 (1.2 g, crude). LC-MS: [M+H]⁺= 336.90.
(6) At room temperature, compound 206-5 (1.2 g, 3.56 mmol) was dissolved in acetonitrile (4 mL) and water (4 mL), followed by the addition of potassium permanganate (1.13 g, 7.12 mmol). The reaction mixture was heated to 40°C and stirred for 4 hours. The reaction mixture was cooled to room temperature and quenched with saturated aqueous sodium sulfite solution (30 mL). The pH of the solution was adjusted to 11 to 12 using a 15% aqueous sodium hydroxide solution, and then ethyl acetate (100 mL) was added. The mixture was stirred for 15 minutes and filtered through diatomite. The phases of the filtrate were separated, and the pH of the aqueous phase was adjusted to 3 to 4 with dilute hydrochloric acid (4 M). The mixture was then filtered, and the filter cake was concentrated under reduced pressure to obtain compound 206-6 (900 mg). LC-MS: [M+H]⁺ =352.90.
(7) At room temperature, compound 206-6 (200 mg, 0.57 mmol) was dissolved in anhydrous tetrahydrofuran (4 mL), followed by the addition of triethylamine (201.8 mg, 1.99 mmol), azidotrimethylsilane (141.5 mg, 0.85 mmol), and propylphosphonic anhydride (906.8 mg, 1.42 mmol, 50% in ethyl acetate). The reaction mixture was stirred at room temperature for 0.5 hours. M011 (141.5 mg, 1.14 mmol) was added to the reaction mixture. The reaction mixture was heated to 70°C and stirred for an additional 1 hour. The reaction mixture was cooled to room temperature, diluted with water (20 mL), and extracted twice with ethyl acetate (15 mL × 2). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 2/1) to obtain compound 206-7 (250 mg). LC-MS: [M+H]⁺ =472.05.
(8) At room temperature, compound M010 (171.6 mg, 0.68 mmol) was dissolved in 1,4-dioxane (4 mL), and tetrabutylammonium fluoride (0.85 mL, 0.85 mmol, 1.0 M in tetrahydrofuran) was added. The reaction mixture was stirred at room temperature for 0.5 hours. Anhydrous magnesium sulfate (400 mg) was added to the reaction mixture, and the mixture was stirred and dried for 0.5 hours. The reaction mixture was filtered. Compound 206-7 (160 mg, 0.34 mmol), bis(triphenylphosphine)palladium(II) chloride (23.8 mg, 0.034 mmol), copper(I) iodide (6.5 mg, 0.034 mmol), potassium carbonate (93.9 mg, 0.68 mmol), and triethylamine (103.2 mg, 1.02 mmol) were added to the filtrate. The reaction mixture was purged with nitrogen, heated to 70°C under a nitrogen atmosphere, and reacted for 1 hour. The reaction mixture was cooled to room temperature, diluted with water (15 mL), and extracted twice with ethyl acetate (10 mL × 2). The combined organic phases were washed with saturated brine (15 mL), dried over anhydrous sodium sulfate, filtered, and the filtrate was concentrated under reduced pressure. The residue was purified by silica gel column chromatography (petroleum ether/ethyl acetate = 2/1) to obtain compound 206-8 (150 mg). LC-MS: [M+H]⁺ =530.20.
(9) At room temperature, compound 206-8 (150 mg, 0.28 mmol) was dissolved in methanol (2 mL) and water (1 mL), followed by the addition of lithium hydroxide monohydrate (45.9 mg, 1.12 mmol). The reaction mixture was stirred at 35°C for 1 hour. The reaction mixture was concentrated under reduced pressure, and the residue was diluted with water (10 mL). The aqueous phase was adjusted to pH = 6 with dilute hydrochloric acid (1 M). The mixture was extracted twice with ethyl acetate (10 mL × 2). The combined organic phases were washed with saturated brine (10 mL), dried over anhydrous sodium sulfate, filtered, and the filtrate was concentrated under reduced pressure. The residue was purified by preparative high-performance liquid chromatography (0.1% ammonia in water conditions) and lyophilized to obtain compound 206 (29.2 mg). LC-MS: [M+H]⁺ =516.15.

¹H NMR (400 MHz, DMSO-*d₆*) δ 8.16 (d, *J* = 8.4 Hz, 1H), 8.05 (d, *J* = 8.4 Hz, 1H), 4.96 (s, 1H), 3.89 (s, 3H), 2.45 (s, 2H), 2.18 (s, 2H), 1.60 (s, 3H), 1.23 (s, 3H), 1.05 (s, 2H), 0.99 (s, 2H).

### Example 45: Preparation of (R)-2-(1-((6-(5-((((3,3-difluoropentan-2-yl)oxy)carbonyl)amino)-1-methyl-1H-1,2,3-triazol-4-yl)-2-methylpyridin-3-yl)ethynyl)cyclopropyl)acetic acid (compound 207)

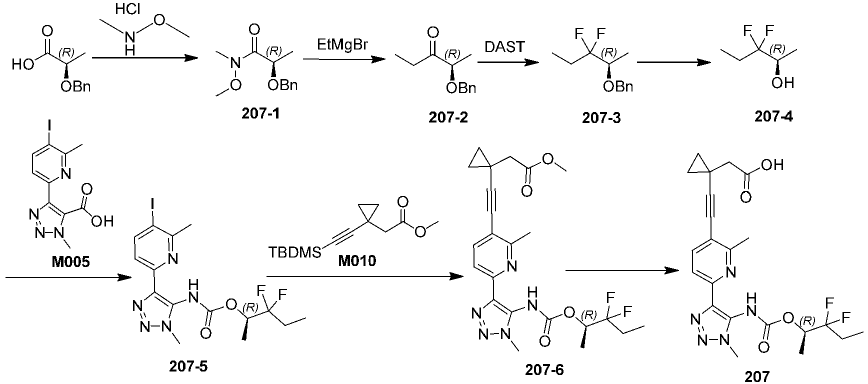

(1) At room temperature, (*R*)-2-(benzyloxy)propanoic acid (50 g, 277.47 mmol) was dissolved in dichloromethane (500 mL), followed by the addition of dimethylhydroxylamine hydrochloride (32.48 g, 332.96 mmol), *N,N*-diisopropylethylamine (107.58 g, 832.41 mmol), and 2-(7-azabenzotriazol-1-yl)-*N,N,N',N'-*tetramethyluronium hexafluorophosphate (158.25 g, 416.21 mmol). The reaction mixture was stirred at room temperature for 2 hours. The reaction mixture was poured into water (500 mL) to quench and extracted with dichloromethane (500 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 207-1 (60 g). LC-MS: [M+H]⁺= 224.05.
(2) At room temperature, compound 207-1 (60 g, 268.73 mmol) was dissolved in anhydrous tetrahydrofuran (600 mL). The reaction mixture was cooled to 0°C, followed by dropwise addition of ethylmagnesium bromide solution (201.55 mL, 403.10 mmol, 2 M in tetrahydrofuran). The reaction mixture was warmed to room temperature and stirred for an additional 2 hours. The reaction mixture was poured into saturated aqueous ammonium chloride solution (500 mL) to quench and extracted with ethyl acetate (500 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 207-2 (45 g). LC-MS: [M+H]⁺ = 193.25.
(3) At room temperature, compound 207-2 (55 g, 286.07 mmol) was dissolved in dichloromethane (550 mL), and the reaction mixture was cooled to 0°C, followed by the dropwise addition of diethylaminosulfur trifluoride (184.44 g, 1.14 mol). The reaction mixture was heated to 30°C and stirred for an additional 48 hours. The reaction mixture was slowly poured into saturated aqueous sodium bicarbonate solution (2000 mL) to quench and extracted with dichloromethane (500 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 207-3 (60 g, crude). The crude product was dissolved in anhydrous tetrahydrofuran (500 mL), and activated carbon (5.5 g) was added. The solution was heated to 50°C and stirred for decolorization for 0.5 hours, filtered, and the filtrate was concentrated under reduced pressure to obtain compound 207-3 (55 g). LC-MS: [M+H]⁺= 215.25.
(4) At room temperature, compound 207-3 (50 g, 233.37 mmol) was dissolved in anhydrous tetrahydrofuran (500 mL), and wet palladium on carbon (5.0 g, 10%) and wet palladium hydroxide on carbon (2.0 g, 20%) were added. Under a hydrogen atmosphere, the reaction mixture was heated to 50°C and stirred for 24 hours. After cooling to room temperature, the reaction mixture was filtered under reduced pressure. The filtrate was dried over molecular sieves, filtered again, and concentrated under reduced pressure to remove a small portion of the solvent to obtain a solution of compound 207-4 in tetrahydrofuran (186 g). LC-MS: [M+H]⁺ =125.13.
(5) At room temperature, compound M005 (16 g, 46.50 mmol), triethylamine (16.94 g, 167.39 mmol), and azidotrimethylsilane (10.71 g, 92.99 mmol) were dissolved in anhydrous tetrahydrofuran (160 mL). Under stirring, propylphosphonic anhydride (44.38 g, 69.74 mmol, 50% in ethyl acetate) was added dropwise to the reaction. The reaction mixture was stirred at room temperature for 0.5 hours. Then, the solution of compound 207-4 in tetrahydrofuran (74 g, 92.99 mmol) was added. The reaction mixture was heated to 80°C and stirred for an additional 2 hours. The reaction mixture was directly concentrated under reduced pressure. The residue was purified by silica gel column chromatography (petroleum ether/ethyl acetate = 2/1) to obtain compound 207-5 (17 g). LC-MS: [M+H]⁺= 466.20.
(6) At room temperature, compound M010 (16.28 g, 64.48 mmol) was dissolved in anhydrous tetrahydrofuran (170 mL), and a solution of tetrabutylammonium fluoride in tetrahydrofuran (1 M, 64.48 mL, 64.48 mmol) was added dropwise. The reaction mixture was stirred at room temperature for 0.5 hours. The reaction mixture was added with anhydrous magnesium sulfate (30 g), dried for 1 hour, then filtered to obtain the filtrate. Compound 207-5 (15 g, 32.24 mmol), bis(triphenylphosphine)palladium(II) chloride (2.26 g, 3.22 mmol), copper(I) iodide (614 mg, 3.22 mmol), and triethylamine (9.79 g, 96.72 mmol) were added to the filtrate. Under a nitrogen atmosphere, the reaction was stirred at room temperature (25°C) for 16 hours. The reaction mixture was directly diluted with water (100 mL) and extracted with ethyl acetate (100 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 207-6 (12 g, crude), which was further purified by preparative high-performance liquid chromatography (0.1% trifluoroacetic acid) to obtain compound 207-6 (10 g). LC-MS: [M+H]⁺= 476.20.
(7) At room temperature, compound 207-6 (10 g, 21.03 mmol) was dissolved in a mixed solvent of methanol (150 mL, analytical grade) and water (50 mL, deionized water), followed by the addition of lithium hydroxide monohydrate (4.41 g, 105.15 mmol, analytical grade). The reaction mixture was stirred at room temperature for 2 hours. The reaction mixture was concentrated under reduced pressure, and the residue was adjusted to pH = 5 to 6 with dilute hydrochloric acid (1 M, prepared with deionized water). The mixture was extracted with ethyl acetate (200 mL × 3, analytical grade). The combined organic phases were washed with water (200 mL, deionized water) and saturated brine (200 mL, prepared with deionized water), dried over anhydrous magnesium sulfate, filtered, and the filtrate was directly concentrated under reduced pressure. After adding water (20 mL, deionized water), the mixture was lyophilized to obtain compound 207 (8419.5 mg). LC-MS: [M+H]⁺ =462.15.

H NMR (400 MHz, DMSO-*d*₆) δ 12.23 (s, 1H), 9.89 (s, 1H), 7.75 (d, *J* = 8.4 Hz, 1H), 7.70 (d, *J =* 8.4 Hz, 1H), 4.90 - 5.10 (m, 1H), 3.90 (s, 3H), 2.51 (s, 3H), 2.46 (s, 2H), 2.14 - 1.77 (m, 2H), 1.52 - 1.13 (m, 3H), 1.10 - 0.78 (m, 7H).

### Example 46: Preparation of (R)-2-(1-((6-(5-((((3,3-difluoropentan-2-yl)oxy)carbonyl)amino)-1-methyl-1H-1,2,3-triazol-4-yl)-2-(trifluoromethyl)pyridin-3-yl)ethynyl)cyclopropyl)acetic acid (compound 208)

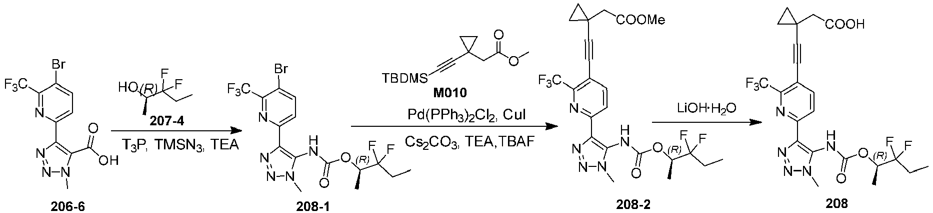

The preparation of compound 208 was referred to the method for preparing compound 206 in Example 44, wherein compound M011 was replaced with compound 207-4, and the condition of high-performance liquid chromatography was changed from 0.1% ammonia in water to 0.1% hydrochloric acid, and the hydrochloride of compound 208 (15.0 mg) was obtained.

LC-MS:[M+H]⁺=516.15.

¹H NMR (400 MHz, DMSO-*d₆*) δ 8.17 (d, *J =* 8.0 Hz, 1H), 8.06 (d, *J =* 8.0 Hz, 1H), 4.95 (s, 1H), 3.92 (s, 3H), 2.46 (s, 2H), 2.09 - 1.80 (m, 3H), 1.30 (s, 2H), 1.24 (s, 3H), 1.06 (s, 2H), 1.00 (s, 2H).

### Example 47: Preparation of (R)-2-(1-((2-chloro-6-(5-((((3,3-difluoropentan-2-yl)oxy)carbonyl)amino)-1-methyl-1H-1,2,3-triazol-4-yl)pyridin-3-yl)ethynyl)cyclopropyl)acetic acid (compound 209)

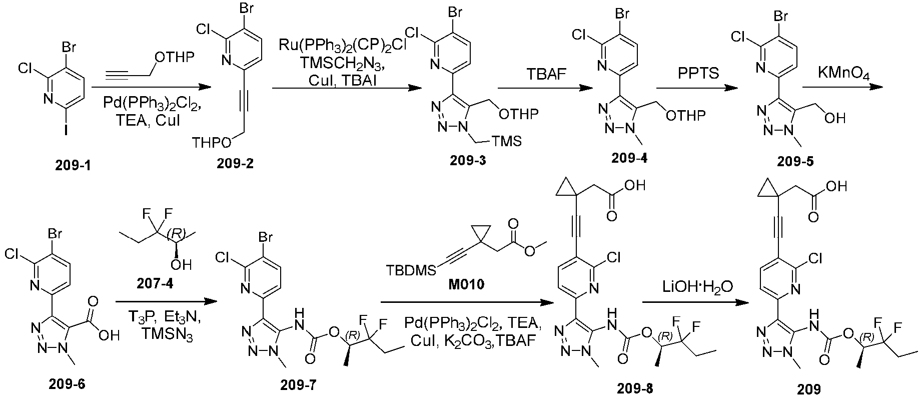

The preparation of compound 209 was referred to the method for preparing compound 206 in Example 44, wherein compound 206-1 was replaced with compound 209-1, compound M011 was replaced with compound 207-4, and the condition of high-performance liquid chromatography was changed from 0.1% ammonia in water to 0.1% hydrochloric acid, and the hydrochloride of compound 209 (2.3 mg) was obtained. LC-MS: [M+H]⁺ =482.10.

The preparation method for compound 209-1 is as follows:

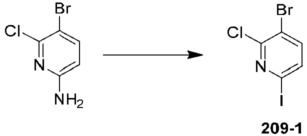

At room temperature, 5-bromo-6-chloro-2-aminopyridine (9 g, 43.38 mmol) and copper(I) iodide (41 g, 216.9 mmol) were dissolved in acetonitrile (360 mL), followed by the addition of *tert*-butyl nitrite (22 g, 216.91 mmol). The reaction mixture was heated to 60°C and stirred for 5 hours. The reaction mixture was cooled to room temperature, and ethyl acetate (300 mL) was added, followed by stirring for 15 minutes. The reaction mixture was filtered, and the filtrate was concentrated under reduced pressure. The residue was purified by silica gel column chromatography (petroleum ether/ethyl acetate = 20/1) to obtain compound 209-1 (4.9 g). LC-MS: [M+H]⁺ =319.75.

### Example 48: Preparation of (R)-2-(1-((2-chloro-6-(5-((((4,4-difluoropentan-2-yl)oxy)carbonyl)amino)-1-methyl-1H-1,2,3-triazol-4-yl)pyridin-3-yl)ethynyl)cyclopropyl)acetic acid (compound 210)

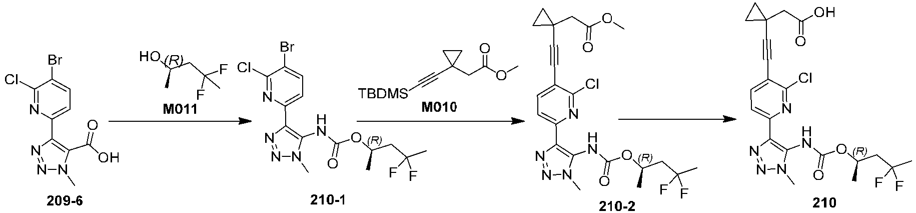

(1) At room temperature, compound 209-6 (390 mg, 1.23 mmol) was dissolved in anhydrous tetrahydrofuran (7 mL), followed by the addition of triethylamine (435.6 mg, 4.3 mmol), azidotrimethylsilane (212.5 mg, 1.84 mmol), and propylphosphonic anhydride (1.95 g, 3.08 mmol, 50% in ethyl acetate). The reaction mixture was stirred at room temperature for 0.5 hours. Compound M011 (305.3 mg, 2.46 mmol) was added to the reaction mixture. The reaction mixture was heated to 70°C and stirred for an additional 1 hour. The reaction mixture was diluted with water (30 mL) and extracted twice with ethyl acetate (20 mL × 2). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 2/1) to obtain compound 210-1 (380 mg). LC-MS: [M+H]⁺ =439.95.
(2) At room temperature, compound M010 (55.5 mg, 0.22 mmol) was dissolved in 1,4-dioxane (1 mL), followed by the addition of tetrabutylammonium fluoride (0.11 mL, 0.11 mmol, 1.0 M in tetrahydrofuran). The reaction mixture was stirred at room temperature for 0.5 hours, added with anhydrous magnesium sulfate (100 mg), stirred for 15 minutes, then filtered to obtain a solution of the intermediate. Compound 210-1 (50 mg, 0.11 mmol), bis(triphenylphosphine)palladium(II) chloride (7.7 mg, 0.011 mmol), copper(I) iodide (2.1 mg, 0.011 mmol), and triethylamine (38.9 mg, 0.39 mmol) were added to the filtrate. After nitrogen purging, the reaction mixture was heated to 30°C under a nitrogen atmosphere and stirred for an additional 16 hours. The reaction mixture was directly concentrated under reduced pressure. The residue was purified by preparative thin-layer chromatography (petroleum ether/ethyl acetate = 3/2) to obtain compound 210-2 (50 mg). LC-MS: [M+H]⁺ =496.20.
(3) At room temperature, compound 210-2 (50 mg, 0.1 mmol) was dissolved in methanol (1 mL) and water (0.5 mL), followed by the addition of lithium hydroxide monohydrate (16.4 mg, 0.4 mmol). The reaction mixture was stirred at room temperature for 1 hour. The reaction mixture was directly concentrated under reduced pressure to remove methanol. The residue was diluted with water (5 mL), and the aqueous phase was adjusted to pH = 5 to 6 with dilute hydrochloric acid (1 M). The mixture was extracted twice with ethyl acetate (5 mL × 2). The combined organic phases were washed with saturated brine (10 mL), dried over anhydrous sodium sulfate, filtered, and the filtrate was concentrated under reduced pressure. The residue was purified by preparative high-performance liquid chromatography (0.1% ammonia in water conditions) and lyophilized to obtain compound 210 (8.7 mg). LC-MS: [M+H]⁺ =482.20.

¹H NMR (400 MHz, DMSO-*d₆*) δ 7.95 - 7.87 (m, 2H), 4.99 (d, *J =* 6.0 Hz, 1H), 3.88 (s, 3H), 2.44 (s, 2H), 2.17 (s, 2H), 1.61 (s, 3H), 1.23 (s, 3H), 1.06 (t, *J =* 5.2 Hz, 2H), 0.98 (t, *J =* 5.6 Hz, 2H).

### Example 49: Preparation of 2-(1-((6-(5-((((1-cyclobutylethyl)(methyl)carbamoyl)oxy)methyl)-1-methyl-1H-1,2,3-triazol-4-yl)-2-methylpyridin-3-yl)ethynyl)cyclopropyl)acetic acid (compound 211)

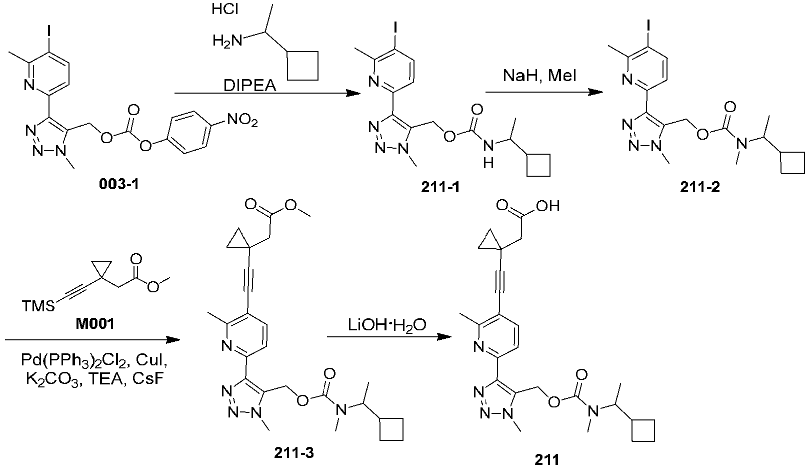

The preparation of compound 211 was referred to the method for preparing compound 009 in Example 9, wherein the starting material was replaced from (1-fluorocyclobutyl)methylamine hydrochloride to 1-cyclobutylethan-1-amine hydrochloride, and compound 211 (21.2 mg) was obtained. LC-MS: [M+H]⁺ = 466.15.

¹H NMR (400 MHz, DMSO-*d*₆) δ7.84 (d, *J* = 8.0 Hz, 1H), 7.73 (d, *J* = 8.0 Hz, 1H), 5.64 (d, *J =* 24.0 Hz, 2H), 4.12 (d, *J* = 16.0 Hz, 3H), 4.06 - 3.71 (m, 1H), 2.57 (s, 1H), 2.55 (d, *J =* 6.4 Hz, 4H), 2.52 (d, *J* = 1.6 Hz, 1H), 2.45 (s, 2H), 2.39 - 2.22 (m, 1H), 2.02 - 1.37 (m, 6H), 1.06 (d, *J =* 6.8 Hz, 2H), 0.95 (m, 2H), 0.86 (m, 3H).

### Example 50: Preparation of trans-2-((6-(5-(((((R)-3,3-difluoropentan-2-yl)oxy)carbonyl)amino)-1-methyl-1H-1,2,3-triazol-4-yl)-2-methylpyridin-3-yl)ethynyl)cyclobutane-1-carboxylic acid (compound 212), compound 212-A, and compound 212-B

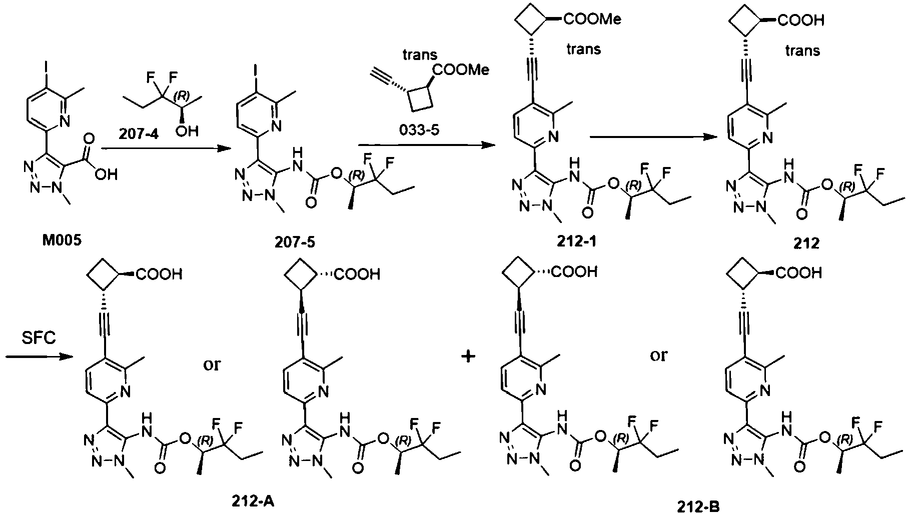

(1) At room temperature, compound M005 (16 g, 46.50 mmol), triethylamine (16.94 g, 167.39 mmol), and azidotrimethylsilane (10.71 g, 92.99 mmol) were dissolved in anhydrous tetrahydrofuran (160 mL). Under stirring, propylphosphonic anhydride (44.38 g, 69.74 mmol, 50% in ethyl acetate) was added dropwise to the reaction. The reaction mixture was stirred at room temperature for 0.5 hours. Then, the solution of compound 207-4 in tetrahydrofuran (74 g, 92.99 mmol, 15.60%) was added. The reaction mixture was heated to 80°C and stirred for an additional 2 hours. The reaction mixture was directly concentrated under reduced pressure. The residue was purified by silica gel column chromatography (petroleum ether/ethyl acetate = 2/1) to obtain compound 207-5 (17 g). LC-MS: [M+H]⁺= 466.20.
(2) At room temperature, compound 207-5 (500 mg, 1.07 mmol) and compound 033-5 (296 mg, 2.14 mmol) were dissolved in 1,4-dioxane (10 mL), followed by the addition of bis(triphenylphosphine)palladium(II) chloride (75.10 mg, 110 µmol), copper(I) iodide (41 mg, 210 µmol), and triethylamine (324 mg, 3.21 mmol). Under a nitrogen atmosphere, the reaction mixture was heated to 90°C and stirred for an additional 2 hours. The reaction mixture was cooled to room temperature, diluted with water (10 mL), and extracted twice with ethyl acetate (20 mL × 2). The combined organic phases were dried over anhydrous sodium sulfate, filtered, and the filtrate was concentrated under reduced pressure. The residue was purified by silica gel column chromatography (ethyl acetate/petroleum ether = 1/2) to obtain compound 212-1 (450 mg, containing a pair of trans configurations). LC-MS: [M+H]⁺ =476.10.
(3) At room temperature, 212-1 (450 mg, 0.76 mmol) was dissolved in methanol (3 mL) and water (1 mL), followed by the addition of lithium hydroxide monohydrate (160 mg, 3.8 mmol). The reaction mixture was stirred at room temperature for 2 hours. After the reaction mixture was diluted with water (10 mL), methanol was removed by concentration under reduced pressure. The residue was extracted with ethyl acetate (5 mL × 3), and the aqueous phase was adjusted to pH = 5 to 6 with dilute hydrochloric acid (1 M), followed by extraction with ethyl acetate (10 mL × 3). The combined organic phases were dried over anhydrous sodium sulfate, filtered, and the filtrate was concentrated under reduced pressure. The residue was purified by high-performance liquid chromatography (0.1% ammonia in water conditions) and lyophilized to obtain compound 212 (270 mg, containing a pair of trans configurations). LC-MS: [M+H]⁺ =462.10.
(4) At room temperature, compound 212 (270 mg, 0.59 µmol) was subjected to chiral separation (Waters 80Q Preparative SFC system, Daicel Chiralpak IG column, 250 × 30 mm I.D., 5 µm; column temperature: room temperature; mobile phase A: supercritical carbon dioxide; mobile phase B: IPA and NH₃H₂O (NH₃H₂O accounts for 0.1% of the volume of mobile phase B); volume ratio of mobile phase A to B: 80:20; flow rate: 60 g/min; isocratic elution; injection volume: 4 µL, detection wavelength: 220 nm) to obtain compound 212-A (retention time Rt = 9.353 min, 76.0 mg) and compound 212-B (retention time Rt = 10.778 min, 52.5 mg).

LCMS: [M+H]⁺= 462.10.

Compound 212-A: ¹H NMR (400 MHz, DMSO-*d*₆) δ 9.92 (s, 1H), 7.78 (q, *J =* 8.0 Hz, 2H), 4.98 (s, 1H), 3.89 (s, 3H), 3.43 (dd, *J =* 17.2, 8.8 Hz, 1H), 3.12 (dd, *J =* 16.6, 7.8 Hz, 1H), 2.55 (s, 3H), 2.25 - 1.79 (m, 6H), 1.37 - 1.10 (m, 3H), 0.93 (s, 3H).

Compound 212-B: ¹H NMR (400 MHz, DMSO-*d*₆) δ 9.91 (s, 1H), 7.79 (q, *J =* 8.0 Hz, 2H), 4.99 (s, 1H), 3.90 (s, 3H), 3.45 (dd, *J =* 17.2, 8.8 Hz, 1H), 3.19 (dd, *J =* 18.0, 9.2 Hz, 1H), 2.55 (s, 3H), 2.28 - 1.84 (m, 6H), 1.43 - 1.15 (m, 3H), 0.95 (s, 3H).

### Example 51: Preparation of trans-2-((6-(5-(((((R)-4,4-difluoropentan-2-yl)oxy)carbonyl)amino)-1-methyl-1H-1,2,3-triazol-4-yl)-2-methylpyridin-3-yl)ethynyl)cyclobutane-1-carboxylic acid (compound 213), compound 213-A, and compound 213-B

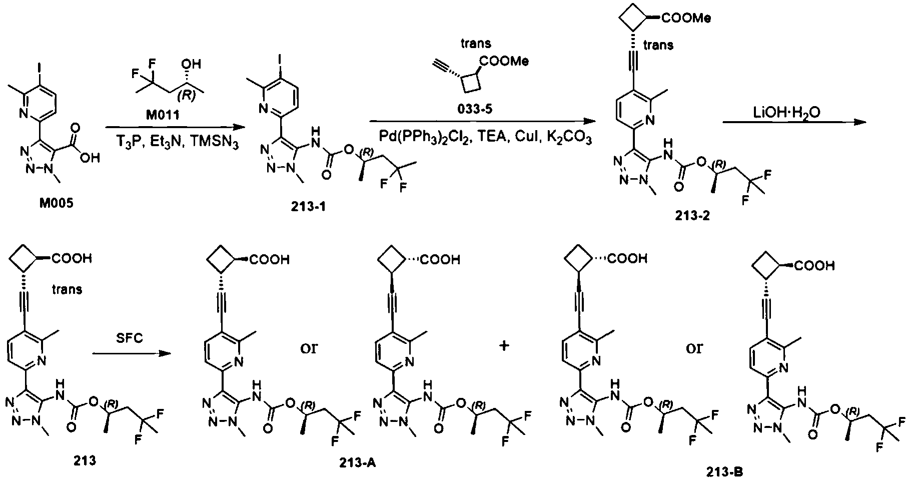

The preparation of compound 213 was referred to the method for preparing compound 212 in Example 50, wherein compound 207-4 was replaced with compound M011, and compound 213 (84.2 mg, containing a pair of trans configurations) was obtained. LC-MS: [M+H]⁺= 462.10.

¹H NMR (400 MHz, DMSO-*d₆*) δ 7.80 (d, *J =* 8.2 Hz, 1H), 7.76 (d, *J =* 8.2 Hz, 1H), 4.99 (s, 1H), 3.87 (s, 3H), 3.44 (m, 1H), 3.22 - 3.14 (m, 1H), 2.55 (s, 3H), 2.23 (d, *J* = 9.2 Hz, 2H), 2.16 - 1.96 (m, 4H), 1.68 (d, *J* = 57.6 Hz, 3H), 1.23 (s, 3H).

At room temperature, compound 213 (70 mg) was subjected to chiral separation (Daicel Chiralpak IG column, 250 × 30 mm I.D., 5 µm, mobile phase A: supercritical carbon dioxide; mobile phase B: EtOH and NH₃H₂O (NH₃H₂O accounts for 0.1% of the volume of mobile phase B); volume ratio of mobile phase A to B: 85:15; flow rate: 55 g/min; column temperature: room temperature; isocratic elution; injection volume: 2 µL; detection wavelength: 220 nm) to obtain compound 213-A (retention time Rt = 11.977 min, 25.6 mg) and compound 213-B (retention time Rt = 14.219 min, 24.7 mg).

LC-MS: [M+H]⁺= 462.15.

Compound 213-A: ¹H NMR (400 MHz, DMSO-*d₆*) δ 9.53 (s, 1H), 7.80 (d, *J =* 8.2 Hz, 1H), 7.76 (d, *J* = 8.2 Hz, 1H), 4.99 (s, 1H), 3.88 (s, 3H), 3.49 - 3.43 (m, 1H), 3.21 (m, 1H), 2.55 (s, 3H), 2.31 - 2.17 (m, 2H), 2.17 - 1.98 (m, 4H), 1.60 (s, 3H), 1.23 (s, 3H).

Compound 213-B: ¹H NMR (400 MHz, DMSO-*d₆*) δ 9.45 (s, 1H), 7.80 (d, *J* = 8.2 Hz, 1H), 7.76 (d, *J =* 8.2 Hz, 1H), 4.99 (s, 1H), 3.88 (s, 3H), 3.45 (m, 1H), 3.20 (m, 1H), 2.55 (s, 3H), 2.23 (t, *J=* 8.8 Hz, 2H), 2.16 - 1.99 (m, 4H), 1.60 (s, 3H), 1.23 (s, 3H).

### Example 52: Preparation of 2-(1-((6-(5-(((4-cyclobutylpyrimidin-2-yl)amino)methyl)-1-methyl-1H-1,2,3-triazol-4-yl)-2-methylpyridin-3-yl)ethynyl)cyclopropyl)acetic acid (compound 214)

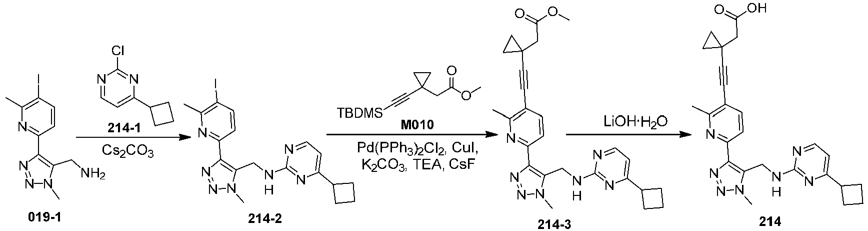

The preparation of compound 214 was referred to the method for preparing compound 019 in Example 19, wherein the starting material 2-chloro-4-isopropylpyrimidine was replaced with compound 214-1, and compound M006 was replaced with M010, and compound 214 (210.6 mg) was obtained. LC-MS: [M+H]⁺= 458.10.

¹H NMR (400 MHz, DMSO-*d₆*) δ 8.15 (d, *J* = 4.9 Hz, 1H), 7.85 (d, *J =* 8.2 Hz, 1H), 7.73 (d, *J =* 8.2 Hz, 1H), 7.42 (t, *J =* 5.6 Hz, 1H), 6.49 (d, *J =* 5.0 Hz, 1H), 5.01 (d, *J =* 5.6 Hz, 2H), 4.15 (s, 3H), 3.23 (s, 1H), 2.57 (s, 3H), 2.45 (s, 2H), 2.10 (t, *J =* 8.2 Hz, 4H), 1.90 (m, 1H), 1.68 (m, 1H), 1.06 (m, 2H), 0.95 (m, 2H).

The preparation method for compound 214-1 is as follows:

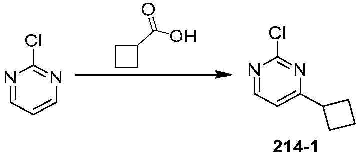

At room temperature, 2-chloropyrimidine (3.0 g, 26.2 mmol), silver nitrate (0.89 g, 5.2 mmol), and ammonium persulfate (6.0 g, 26.2 mmol) were added to a reaction flask, followed by the addition of dichloromethane (15 mL) and water (15 mL). After cooling the reaction mixture to 0°C, cyclobutanecarboxylic acid (2.3 mL, 23.6 mmol) was added. After warming to room temperature, the reaction mixture was stirred for an additional 16 hours. Dichloromethane (40 mL) was added to the reaction mixture, followed by sequential washing with water (40 mL) and saturated brine (40 mL). The organic phase was dried over anhydrous sodium sulfate, filtered, and the filtrate was concentrated under reduced pressure. The residue was purified by silica gel column chromatography (petroleum ether/ethyl acetate = 10/1) to obtain compound 214-1 (2.9 g). LC-MS: [M+H]⁺ =169.10.

### Example 53: Preparation of 2-(1-((6-(5-(((benzyloxy)carbonyl)amino)methyl)-1-methyl-1H- 1,2,3-triazol-4-yl)-2-methylpyridin-3-yl)ethynyl)cyclopropyl)acetic acid (compound 215)

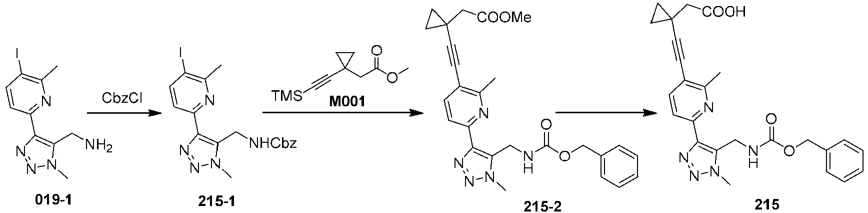

(1) At room temperature, compound 019-1 (587.42 mg, 1.78 mmol) was dissolved in ethyl acetate (10 mL) and water (10 mL), followed by the addition of sodium bicarbonate (600 mg, 7.1 mmol) and benzyl chloroformate (460 mg, 2.67 mmol). The reaction mixture was stirred at room temperature for 1 hour. The reaction mixture was diluted with water (20 mL) and extracted with ethyl acetate (20 mL × 3). The combined organic phases were washed with saturated brine (40 mL), dried over anhydrous sodium sulfate, filtered, and the filtrate was concentrated under reduced pressure. The residue was purified by silica gel column chromatography (petroleum ether/ethyl acetate = 3/1) to obtain compound 215-1 (540 mg). LC-MS: [M+H]⁺ =464.95.
(2) At room temperature, compound 215-1 (270 mg, 0.58 mmol) and compound M001 (183 mg, 0.87 mmol) were dissolved in 1,4-dioxane (4 mL), followed by the addition of bis(triphenylphosphine)palladium(II) chloride (41 mg, 0.058 mmol), copper(I) iodide (11 mg, 0.058 mmol), cesium fluoride (353 mg, 2.32 mmol), potassium carbonate (160 mg, 1.16 mmol), and triethylamine (176 mg, 1.74 mmol). Under a nitrogen atmosphere, the reaction mixture was heated to 90°C and stirred for 3 hours. After cooling to room temperature, the reaction mixture was diluted with ethyl acetate (20 mL), filtered, and the filter cake was washed with ethyl acetate (15 mL). The filtrate was sequentially washed with water (10 mL) and saturated brine (10 mL). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 215-2 (200 mg). LC-MS: [M+H]⁺ =474.10.
(3) At room temperature, compound 215-2 (200 mg, 0.42 mmol) was dissolved in tetrahydrofuran (3 mL), and a solution of lithium hydroxide monohydrate (54 mg, 1.27 mmol) in water (1 mL) was added. The reaction mixture was stirred at room temperature for 4 hours. The reaction mixture was diluted with ethyl acetate (5 mL) and washed with water (10 mL). The aqueous phase was adjusted to pH = 5 with dilute hydrochloric acid (1 M) and then extracted with ethyl acetate (5 mL × 3). The combined organic phases were washed with saturated brine (10 mL), dried over anhydrous sodium sulfate, filtered, and the filtrate was concentrated under reduced pressure. The residue was purified by preparative high-performance liquid chromatography (0.1% ammonia in water conditions) and lyophilized to obtain compound 215 (17.6 mg). LC-MS: [M+H]⁺= 460.10.

¹H NMR (400 MHz, DMSO-*d*₆) δ 7.82 (d, *J* = 8.2 Hz, 1H), 7.74 - 7.65 (m, 2H), 7.32 (m, 4H), 5.01 (s, 2H), 4.84 (d, *J =* 5.2 Hz, 2H), 4.06 (s, 3H), 2.55 (s, 3H), 2.44 (s, 2H), 1.04 (m, 2H), 0.95 (m, 2H).

### Example 54: Preparation of 2-(1-((6-(5-(((4-(3,3-difluorocyclobutyl)pyrimidin-2-yl)oxy)methyl)-1-methyl-1H-1,2,3-triazol-4-yl)-2-methylpyridin-3-yl)ethynyl)cyclopropyl)acetic acid (compound 216)

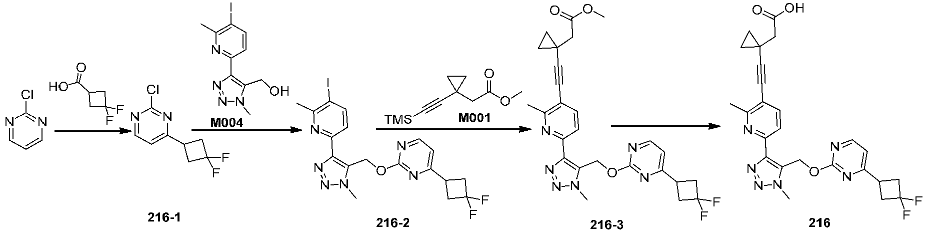

(1) At room temperature, 2-chloropyrimidine (2.0 g, 17.46 mmol) was dissolved in dichloromethane (10 mL) and water (10 mL), followed by the addition of 3,3-difluorocyclobutanecarboxylic acid (2.14 mg, 15.72 mmol), ammonium persulfate (3.98 g, 17.46 mmol), and silver nitrate (593 mg, 3.50 mmol). The reaction mixture was stirred at room temperature for 16 hours. The reaction mixture was quenched with aqueous sodium bicarbonate solution (20 mL), filtered through diatomite, and the filtrate was extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 216-1 (250 mg). LC-MS: [M+H]⁺ = 205.00.
(2) At room temperature, compound 216-1 (290 mg, 1.41 mmol) and compound M004 (466.6 mg, 1.41 mmol) were dissolved in tetrahydrofuran (8 mL). After cooling the reaction mixture to 0°C, potassium *tert*-butoxide (317 mg, 2.83 mmol) was added, and the reaction mixture was stirred at room temperature for 2 hours. The reaction mixture was quenched with aqueous ammonium chloride solution (20 mL) and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 216-2 (260 mg). LC-MS: [M+H]⁺ = 499.9.
(3) At room temperature, compound 216-2 (232.5 mg, 0.465 mmol) and compound M001 (195 mg, 0.93 mmol) were dissolved in 1,4-dioxane (4 mL), followed by the addition of bis(triphenylphosphine)palladium(II) chloride (33 mg, 0.047 mmol), copper(I) iodide (18 mg, 0.93 mmol), cesium fluoride (283 mg, 1.86 mmol), potassium carbonate (129 mg, 0.93 mmol), and triethylamine (142 mg, 1.40 mmol). The reaction mixture was purged with nitrogen, heated to 90°C under a nitrogen atmosphere, and stirred for 3 hours. The reaction mixture was cooled to room temperature, diluted with water (10 mL), and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 216-3 (150 mg). LC-MS: [M+H]⁺ =509.00.
(4) At room temperature, compound 216-3 (150 mg, 0.29 mmol) was dissolved in methanol (3 mL) and water (1 mL), followed by the addition of lithium hydroxide monohydrate (75 mg, 1.47 mmol). The reaction mixture was stirred at room temperature for 2 hours. The reaction mixture was neutralized (pH = 7) with dilute hydrochloric acid (1 M). The mixture was extracted with ethyl acetate (5 mL × 3). The combined organic phases were concentrated under reduced pressure. The residue was purified by preparative high-performance liquid chromatography (0.1% ammonia in water) and lyophilized to obtain compound 216 (60.3 mg).

LC-MS: [M+H]⁺ =495.05.

¹H NMR (400 MHz, DMSO-*d*₆) δ 8.56 (d, *J =* 5.0 Hz, 1H), 7.87 (d, *J =* 8.2 Hz, 1H), 7.73 (d, *J =* 8.2 Hz, 1H), 7.17 (d, *J =* 5.0 Hz, 1H), 6.07 (s, 2H), 4.15 (s, 3H), 3.51 - 3.45 (m, 1H), 2.95 - 2.76 (m, 4H), 2.43 (s, 2H), 2.37 (s, 3H), 1.06 - 1.01 (m, 2H), 0.96 - 0.90 (m, 2H).

### Example 55: Preparation of 2-(1-((2-methyl-6-(1-methyl-5-(((4-propylpyrimidin-2-yl)oxy)methyl)-1H-1,2,3-triazol-4-yl)pyridin-3-yl)ethynyl)cyclopropyl)acetic acid (compound 217)

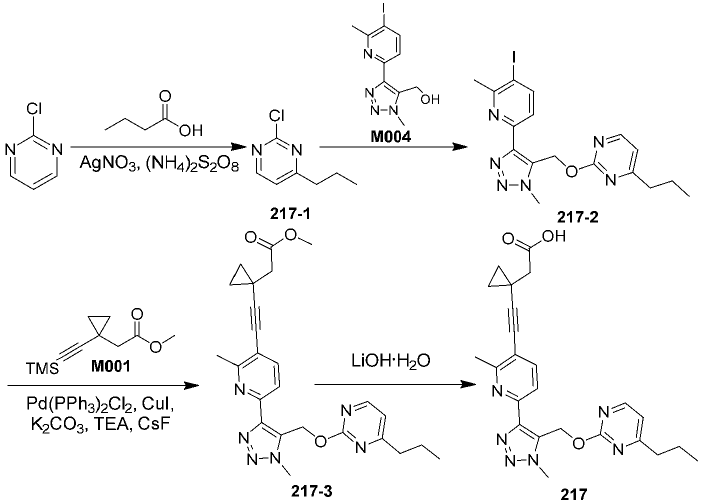

The preparation of compound 217 was referred to the method for preparing compound 216 in Example 54, wherein the starting material was replaced from 3,3-difluorocyclobutanecarboxylic acid to butyric acid, and compound 217 (19.7 mg) was obtained. LC-MS: [M+H]⁺ =447.10.

¹H NMR (400 MHz, DMSO-*d*₆) δ 8.47 (d, *J =* 4.8 Hz, 1H), 7.83 (d, *J =* 8.0 Hz, 1H), 7.71 (d, *J =* 8.4 Hz, 1H), 7.03 (d, *J* = 4.8 Hz, 1H), 6.02 (s, 2H), 4.14 (s, 3H), 2.53 (d, *J* = 7.6 Hz, 2H), 2.38 (s, 3H), 2.35 (s, 2H), 1.63 - 1.52 (m, 2H), 0.97 (t, *J=* 10.8 Hz, 2H), 0.91 (t, *J=* 10.8 Hz, 2H), 0.84 (t, *J =* 14.4 Hz, 3H).

### Example 56: Preparation of 2-(1-((2-cyclopropyl-6-(5-((((4-fluorobutyl)(methyl)carbamoyl)oxy)methyl)-1-methyl-1H-1,2,3-triazol-4-yl)pyridin-3-yl)ethynyl)cyclopropyl)acetic acid (compound 218)

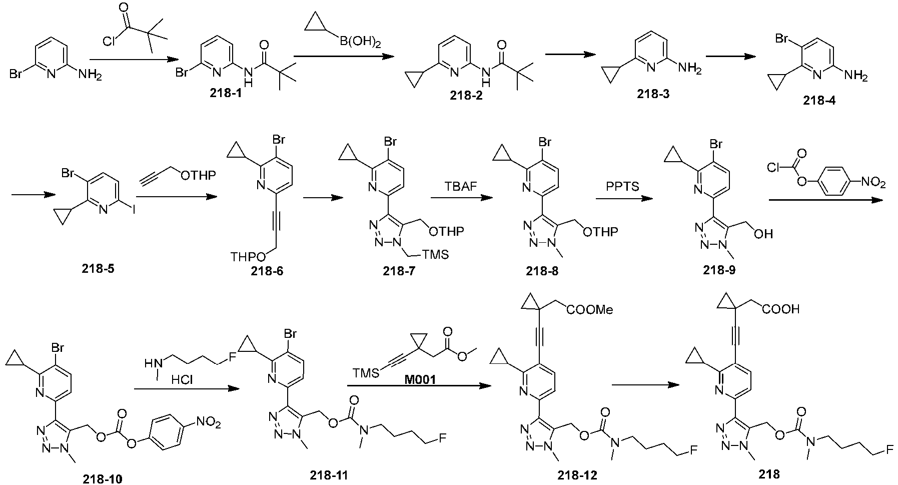

(1) At room temperature, 2-amino-6-bromopyridine (12.2 g, 70.52 mmol) and *N,N-*diisopropylethylamine (19.13 g, 148.1 mmol) were dissolved in dichloromethane (120 mL), and the reaction mixture was cooled to 0°C, followed by the addition of pivaloyl chloride (10.23 g, 84.62 mmol) under stirring. After warming to room temperature, the reaction mixture was stirred for an additional 1 hour. The reaction mixture was quenched with aqueous sodium bicarbonate solution (200 mL) and extracted with dichloromethane (100 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 218-1 (17.2 g). LC-MS: [M+H]⁺= 257.10.
(2) At room temperature, compound 218-1 (23 g, 89.45 mmol), cyclopropylboronic acid (19.21 g, 223.62 mmol), tripotassium phosphate (66.46 g, 313.07 mmol), palladium acetate (2 g, 8.94 mmol), and tricyclohexylphosphine (5.02 g, 17.89 mmol) were dissolved in toluene (120 mL) and water (30 mL). The reaction mixture was purged with nitrogen, heated to 100°C under a nitrogen atmosphere, and stirred for 6 hours. After cooling to room temperature, the reaction mixture was quenched with water (200 mL) and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 218-2 (19 g). LC-MS: [M+H]⁺ = 219.10.
(3) At room temperature, compound 218-2 (19 g, 87.04 mmol) was dissolved in 1,4-dioxane (200 mL), and hydrochloric acid (9 M, 200 mL) was added under stirring. The reaction mixture was heated to 100°C and stirred for 16 hours. The reaction mixture was cooled to room temperature, and sodium hydroxide (3 M) was added dropwise while maintaining the internal temperature below 25°C to adjust the pH of the reaction mixture to 4 to 5. The mixture was extracted with ethyl acetate (300 mL × 3). The combined organic phases were washed with water (500 mL) and saturated brine (500 mL), dried over anhydrous sodium sulfate, filtered, and the filtrate was concentrated under reduced pressure. The residue was purified by silica gel column chromatography (petroleum ether/ethyl acetate = 5/1) to obtain compound 218-3 (16 g, crude). LC-MS: [M+H]⁺= 135.15.
(4) At room temperature, compound 218-3 (11 g, 82.00 mmol) was dissolved in methanol (110 mL), and the reaction mixture was cooled to 0°C. N-Bromosuccinimide (11.67 g, 65.6 mmol) was added in portions under stirring. The reaction mixture was stirred at this temperature for 0.5 hours, then warmed to room temperature, and stirred for an additional 0.5 hours. The reaction mixture was concentrated under reduced pressure. The residue was purified by silica gel column chromatography (petroleum ether/ethyl acetate = 5/1) to obtain compound 218-4 (8.5 g). LC-MS: [M+H]⁺ =212.95.
(5) At room temperature, compound 218-4 (2.0 g, 9.39 mmol) was dissolved in acetonitrile (100 mL). Copper(I) iodide (2.68 g, 14.08 mmol) and diiodomethane (20.11 g, 75.09 mmol) were added, followed by the dropwise addition of *tert*-butyl nitrite (3.87 g, 37.54 mmol). The reaction mixture was heated to 60°C and stirred for 3 hours. The reaction mixture was cooled to room temperature, filtered, and the filtrate was diluted with ethyl acetate (100 mL) and washed with water (100 mL). The organic phase was dried over anhydrous sodium sulfate, filtered, and the filtrate was concentrated under reduced pressure. The residue was purified by silica gel column chromatography (petroleum ether/ethyl acetate = 100/1) to obtain compound 218-5 (1.0 g). LC-MS: [M+H]⁺ =323.80.
(6) At room temperature, compound 218-5 (1.0 g, 3.09 mmol) and pyran (498 mg, 3.55 mmol) were dissolved in acetonitrile (10 mL), followed by the addition of bis(triphenylphosphine)palladium(II) chloride (217 mg, 0.31 mmol), copper(I) iodide (59 mg, 0.31 mmol), and triethylamine (937 mg, 9.26 mmol). Under a nitrogen atmosphere, the reaction mixture was heated to 35°C and stirred for 1 hour. After cooling to room temperature, the reaction mixture was directly concentrated under reduced pressure. The residue was purified by silica gel column chromatography (petroleum ether/ethyl acetate = 10/1) to obtain compound 218-6 (800 mg). LC-MS: [M+H]⁺ =335.95.
(7) At room temperature, compound 218-6 (800 mg, 2.38 mmol) and trimethylsilylmethyl azide (615 mg, 4.76 mmol) were dissolved in anhydrous tetrahydrofuran (10 mL), followed by the addition of pentamethylcyclopentadienylbis(triphenylphosphine)ruthenium(II) chloride (190 mg, 0.24 mmol), tetrabutylammonium iodide (88 mg, 0.24 mmol), and copper(I) iodide (45 mg, 0.24 mmol). Under a nitrogen atmosphere, the reaction was heated to 40°C and stirred for 16 hours. After cooling to room temperature, the reaction mixture was filtered, and the filtrate was diluted with water (20 mL) and extracted twice with ethyl acetate (20 mL × 2). The combined organic phases were washed with saturated brine (20 mL). The organic phase was dried over anhydrous sodium sulfate, filtered, and the filtrate was concentrated under reduced pressure. The residue was purified by silica gel column chromatography (petroleum ether/ethyl acetate = 15/1) to obtain compound 218-7 (1.1 g). LC-MS: [M+H]⁺ =466.96.
(8) At room temperature, compound 218-7 (1.1 g, 2.36 mmol) was dissolved in anhydrous tetrahydrofuran (15 mL), and tetrabutylammonium fluoride (618 mg, 2.36 mmol) was added in portions. The reaction mixture was stirred at room temperature for 0.5 hours. The reaction mixture was diluted with water (30 mL) and extracted with ethyl acetate (20 mL). The organic phase was washed with saturated brine (20 mL). The organic phase was dried over anhydrous sodium sulfate, filtered, and the filtrate was concentrated under reduced pressure. The residue was purified by silica gel column chromatography (petroleum ether/ethyl acetate = 5/1) to obtain compound 218-8 (870 mg). LC-MS: [M+H]⁺ =392.96.
(9) At room temperature, compound 218-8 (870 mg, 2.21 mmol) was dissolved in methanol (10 mL), and pyridiniump-toluenesulfonate (556 mg, 2.21 mmol) was added. The reaction was heated to 60°C and stirred for 1 hour. The reaction mixture was concentrated under reduced pressure to remove methanol, quenched with water (50 mL), and extracted with ethyl acetate (50 mL × 3). The combined organic phases were washed with saturated brine (150 mL), dried over anhydrous sodium sulfate, filtered, and the filtrate was concentrated under reduced pressure to obtain compound 218-9 (650 mg). LC-MS: [M+H]⁺ =308.97.
(10) At room temperature, compound 218-9 (650 mg, 2.10 mmol) was dissolved in dichloromethane (10 mL), followed by the sequential addition of pyridine (333 mg, 4.20 mmol) and 4-nitrophenyl chloroformate (850 mg, 4.20 mmol). The reaction mixture was stirred at room temperature for 1 hour. The reaction mixture was diluted with water (40 mL) and extracted twice with dichloromethane (40 mL × 2). The combined organic phases were washed with saturated brine (40 mL). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 218-10 (800 mg). LC-MS: [M+H]⁺ =475.87.
(11) At room temperature, compound 218-10 (400 mg, 0.84 mmol) was dissolved in dichloromethane (8 mL), followed by the addition of *N,N*-diisopropylethylamine (520 mg, 6.14 mmol) and 4-fluoro-N-methylbutan-1-amine hydrochloride (179 mg, 1.27 mmol). The reaction mixture was stirred at room temperature for 1 hour. The reaction mixture was diluted with water (10 mL) and extracted with dichloromethane (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 218-11 (400 mg). LC-MS: [M+H]⁺ - 441.90.
(12) At room temperature, compound 218-11 (400 mg, 0.91 mmol) and M001 (382 mg, 1.82 mmol) were dissolved in 1,4-dioxane (4 mL), followed by the addition of bis(triphenylphosphine)palladium(II) chloride (64 mg, 0.091 mmol), copper(I) iodide (35 mg, 0.182 mmol), cesium fluoride (552 mg, 3.63 mmol), potassium carbonate (251 mg, 1.82 mmol), and triethylamine (276 mg, 2.73 mmol). The reaction mixture was purged with nitrogen, heated to 90°C under a nitrogen atmosphere, and stirred for 5 hours. The reaction mixture was cooled to room temperature, diluted with water (10 mL), and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 218-12 (200 mg). LC-MS: [M+H]⁺ =498.10.
(13) At room temperature, compound 218-12 (200 mg, 0.40 mmol) was dissolved in methanol (3 mL) and water (1 mL), followed by the addition of lithium hydroxide monohydrate (85 mg, 2.01 mmol). The reaction mixture was stirred at room temperature for 2 hours. The reaction mixture was neutralized (pH = 7) with dilute hydrochloric acid (1 M). The mixture was extracted with ethyl acetate (3 mL × 3). The combined organic phases were dried over anhydrous sodium sulfate, filtered, and the filtrate was concentrated under reduced pressure. The residue was purified by preparative high-performance liquid chromatography (0.1% ammonia in water conditions) and lyophilized to obtain compound 218 (116.7 mg). LC-MS: [M+H]⁺ =484.15.

¹H NMR (400 MHz, DMSO-*d₆*) δ 7.76 (d, *J =* 8.2 Hz, 1H), 7.69 (d, *J =* 8.2 Hz, 1H), 5.55 (s, 2H), 4.33 (dd, *J =* 74.6, 47.6 Hz, 2H), 4.11 (s, 3H), 3.16 (d, *J =* 43.4 Hz, 2H), 2.75 (d, *J =* 12.8 Hz, 3H), 2.67 - 2.59 (m, 1H), 2.42 (s, 2H), 1.64 - 1.48 (m, 2H), 1.45 - 1.30 (m, 2H), 1.08 - 0.97 (m, 5H), 0.96 - 0.89 (m, 2H).

### Example 57: Preparation of trans-2-((6-(5-(((((R)-3,3-difluoropentan-2-yl)oxy)carbonyl)amino)-1-methyl-1H-1,2,3-triazol-4-yl)-2-methylpyridin-3-yl)ethynyl)cyclopentane-1-carboxylic acid (compound 219), compound 219-A, and compound 219-B

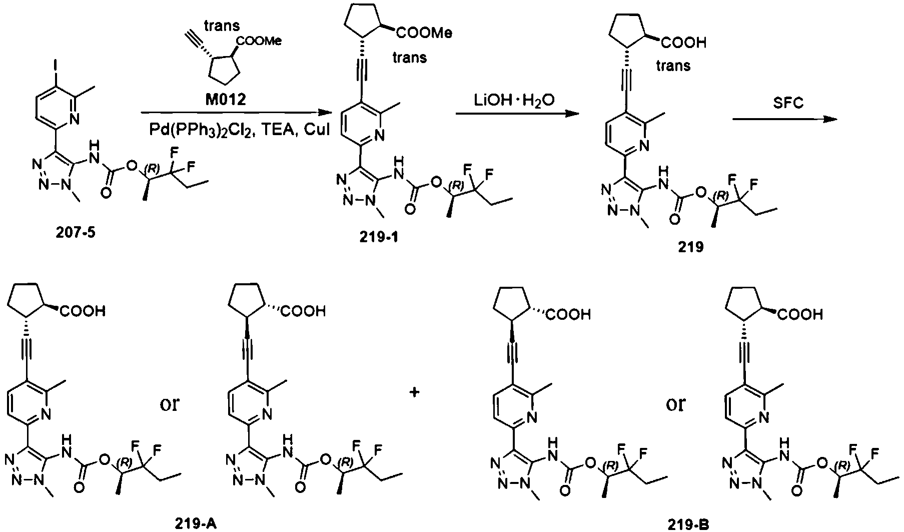

The preparation of compound 219 was referred to the method for preparing compound 212 in Example 50, wherein compound 033-5 was replaced with compound M012, and compound 219 (166 mg, containing a pair of trans configurations) was obtained. LC-MS: [M+H]⁺ =476.10.

Compound 219 (166 mg, 0.35 mmol) was subjected to chiral separation (Daicel Chiralpak IG column, 250 × 30 mm I.D., 5 µm; flow rate: 55 g/min; column temperature: room temperature; mobile phase A: supercritical carbon dioxide; mobile phase B: EtOH and NH₃H₂O (NH₃H₂O accounts for 0.1% of the volume of mobile phase B); volume ratio of mobile phase A to B: 85:15; isocratic elution; injection volume: 3 µL; detection wavelength: 220 nm) to obtain compound 219-A (retention time Rt = 7.974 min, 43.6 mg) and compound 219-B (retention time Rt = 10.093 min, 36.7 mg).

LCMS: [M+H]⁺= 476.10.

Compound 219-A: ¹H NMR (400 MHz, DMSO-*d₆*) δ 9.90 (s, 1H), 7.77 (s, 2H), 4.99 (s, 1H), 3.89 (s, 3H), 3.15 (dd, *J =* 15.6, 7.8 Hz, 1H), 2.78 (dd, *J =* 16.2, 8.2 Hz, 1H), 2.53 (s, 3H), 2.17 - 1.64 (m, 8H), 1.39 - 1.17 (m, 3H), 0.90 (s, 3H).

Compound 219-B: ¹H NMR (400 MHz, DMSO-*d₆*) δ 9.83 (s, 1H), 7.77 (s, 2H), 4.98 (s, 1H), 3.90 (s, 3H), 3.15 (dd, *J =* 15.6, 7.8 Hz, 1H), 2.79 (dd, *J =* 16.2, 8.2 Hz, 1H), 2.53 (s, 3H), 2.17 - 1.61 (m, 8H), 1.44 - 1.11 (m, 3H), 0.95 (s, 3H).

### Example 58: Preparation of (R)-2-(1-((2-methyl-6-(1-methyl-5-(((4-methylpentan-2-yl)oxy)carbonyl)amino)-1H-1,2,3-triazol-4-yl)pyridin-3-yl)ethynyl)cyclopropyl)acetic acid (compound 220)

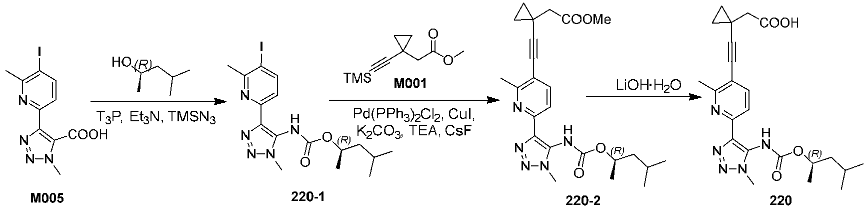

The preparation of compound 220 was referred to the method for preparing compound 010 in Example 10, wherein the starting material 2-pentanol was replaced with (R)-4-methyl-2-pentanol, and compound 220 (78.4 mg) was obtained.

LC-MS: [M+H]⁺= 440.10.

¹H NMR (400 MHz, DMSO-*d*₆) δ 9.41 (s, 1H), 7.74 (d, *J =* 8.2 Hz, 1H), 7.69 (d, *J =* 8.2 Hz, 1H), 4.53 (s, 1H), 3.88 (s, 3H), 2.51 (s, 3H), 2.44 (s, 2H), 1.73 (s, 1H), 1.22 - 0.67 (m, 13H).

### Example 59: Preparation of trans-2-((6-(5-chloro-3-(((((R)-4,4-difluoropentan-2-yl)oxy)carbonyl)amino)thiophen-2-yl)-2-methylpyridin-3-yl)ethynyl)cyclobutane-1-carboxylic acid (compound 221)

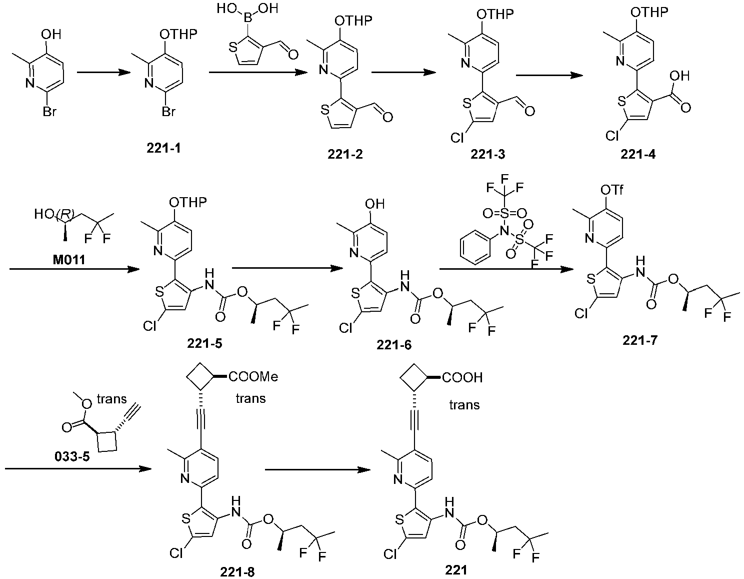

(1) At room temperature, 6-bromo-3-hydroxy-2-methylpyridine (1 g, 5.32 mmol) was dissolved in dichloromethane (5 mL), followed by the addition of pyridine hydrochloride (61 mg, 0.53 mmol) and 3,4-dihydro-2*H*-pyran (671 mg, 7.98 mmol). The reaction mixture was stirred at room temperature for 16 hours. The reaction mixture was poured into water (20 mL) and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 221-1 (1.4 g). LC-MS: [M+H]⁺= 271.95.
(2) At room temperature, compound 221-1 (1.2 g, 4.41 mmol) and 3-formylthiophene-2-boronic acid (0.9 g, 5.73 mmol) were dissolved in *N,N*-dimethylformamide (90 mL), followed by the addition of bis(tri-*tert*-butylphosphine)palladium(0) (230 mg, 0.44 mmol) and potassium carbonate (1.8 g, 13.23 mmol). The reaction mixture was heated to 90°C under a nitrogen atmosphere and stirred for an additional 2 hours. The reaction mixture was cooled to room temperature, then poured into water (30 mL) to quench, and extracted with ethyl acetate (30 mL × 3). The combined organic phases were washed with saturated aqueous ammonium chloride solution (20 mL × 3). The organic phase was dried over anhydrous sodium sulfate, filtered, and the filtrate was concentrated under reduced pressure. The residue was purified by silica gel column chromatography (petroleum ether/ethyl acetate = 5/1) to obtain compound 221-2 (1.0 g). LC-MS: [M+H]⁺= 304.05.
(3) At room temperature, compound 221-2 (1.0 g, 3.30 mmol) was dissolved in *N,N-*dimethylformamide (20 mL), and *N*-chlorosuccinimide (661 mg, 4.95 mmol) was added. The reaction mixture was stirred at room temperature for 16 hours. The reaction mixture was poured into water (30 mL) and extracted with ethyl acetate (30 mL × 3). The combined organic phases were washed with saturated aqueous ammonium chloride solution (20 mL × 3). The organic phase was dried over anhydrous sodium sulfate, filtered, and the filtrate was concentrated under reduced pressure. The residue was purified by silica gel column chromatography (petroleum ether/ethyl acetate = 3/1) to obtain compound 221-3 (700 mg). LCMS: [M+H]⁺ =338.00.
(4) At room temperature, compound 221-3 (500 mg, 1.48 mmol) was dissolved in acetonitrile (5 mL) and water (5 mL), followed by the addition of potassium permanganate (468 mg, 2.96 mmol). The reaction mixture was stirred at room temperature for 16 hours. The reaction mixture was adjusted to pH = 9 to 10 with aqueous sodium hydroxide solution (2 N), filtered under reduced pressure, and the filter cake was washed with water (30 mL). The filtrate was back-extracted with ethyl acetate (20 mL × 3), and the aqueous phase was adjusted to pH = 5 to 6 with dilute hydrochloric acid (1 M), then filtered. The filter cake was directly dried under reduced pressure to obtain compound 221-4 (350 mg). LCMS: [M+H]⁺ =354.00.
(5) At room temperature, compound 221-4 (450 mg, 1.27 mmol) was dissolved in anhydrous tetrahydrofuran (10 mL), followed by the addition of propylphosphonic anhydride (1.2 g, 1.91 mmol, 50% in ethyl acetate), triethylamine (643 mg, 6.35 mmol), and azidotrimethylsilane (293 mg, 2.54 mmol). The reaction mixture was stirred at room temperature for 0.5 hours. Compound M011 (315 mg, 2.54 mmol) was added to the reaction mixture. The reaction mixture was heated to 70°C and stirred for an additional 1 hour. After cooling to room temperature, the reaction mixture was poured into water (20 mL) and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound 221-5 (250 mg). LCMS: [M+H]⁺ =475.10.
(6) At room temperature, compound 221-5 (270 mg, 0.57 mmol) was dissolved in 1,4-dioxane (3 mL), followed by the addition of aqueous hydrochloric acid solution (3 mL, 3 M). The reaction mixture was stirred at room temperature for 2 hours. The reaction mixture was poured into saturated aqueous sodium bicarbonate solution (30 mL) and extracted with dichloromethane (30 mL × 3). The combined organic phases were dried over anhydrous sodium sulfate, filtered, and the filtrate was concentrated under reduced pressure to obtain compound 221-6 (160 mg). LCMS: [M+H]⁺ =391.00.
(7) At room temperature, compound 221-6 (150 mg, 0.38 mmol) and N-phenyl-bis(trifluoromethanesulfonyl)imide (136 mg, 0.38 mmol) were dissolved in dichloromethane (5 mL). After cooling the reaction mixture to 0°C, triethylamine (58 mg, 0.57 mmol) was added. The reaction mixture was slowly warmed to room temperature and stirred for an additional 16 hours. The reaction mixture was poured into water (20 mL) to quench and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 221-7 (200 mg). LCMS: [M+H]⁺ =523.00.
(8)At room temperature, compound 221-7 (180 mg, 0.34 mmol) and compound 033-5 (94 mg, 0.68 mmol) were dissolved in 1,4-dioxane (5 mL), followed by the addition of bis(triphenylphosphine)palladium(II) chloride (48 mg, 0.068 mmol), copper(I) iodide (13 mg, 0.068 mmol), triethylamine (103 mg, 1.02 mmol), and potassium carbonate (94 mg, 0.68 mmol). The reaction mixture was heated to 80°C under a nitrogen atmosphere and stirred for an additional 2 hours. After cooling to room temperature, the reaction mixture was quenched by pouring into water (20 mL) and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 221-8 (80 mg, containing a pair of trans configurations). LCMS: [M+H]⁺ =511.10.
(9)At room temperature, compound 221-8 (110 mg, 0.22 mmol) was dissolved in methanol (2 mL) and water (2 mL), followed by the addition of lithium hydroxide monohydrate (28 mg, 0.66 mmol). The reaction mixture was stirred at room temperature for 1 hour. The reaction mixture was diluted with water (10 mL) and then concentrated under reduced pressure to remove methanol. After washing the residue with dichloromethane (10 mL × 3), the aqueous phase was adjusted to pH = 3 to 4 with dilute hydrochloric acid (1 M) and then extracted with ethyl acetate (10 mL × 3). The combined organic phases were dried over anhydrous sodium sulfate, filtered, and the filtrate was concentrated under reduced pressure. The residue was purified by high-performance liquid chromatography (0.1% ammonia in water system) and lyophilized to obtain compound 221 (30.7 mg, containing a pair of trans configurations). LC-MS: [M+H]⁺ =497.05.

¹H NMR (400 MHz, DMSO-*d₆*) δ 11.23 (s, 1H), 7.73 (d, J = 8.0 Hz, 1H), 7.59 (s, 1H), 7.26 (d, J = 8.4 Hz, 1H), 5.07 - 4.99 (m, 1H), 3.45 - 3.38 (m, 1H), 3.18 - 3.11 (m, 1H), 2.54 (s, 3H), 2.37 - 2.15 (m, 3H), 2.14 - 1.97 (m, 3H), 1.62 (t, J = 19.2 Hz, 3H), 1.29 (d, J = 6.4 Hz, 3H).

### Example 60: Preparation of 2-(1-((6-(5-((6-isopropylpyrazin-2-yl)amino)-1-methyl-1H-pyrazol-4-yl)-2-methylpyridin-3-yl)ethynyl)cyclopropyl)acetic acid (compound 222)

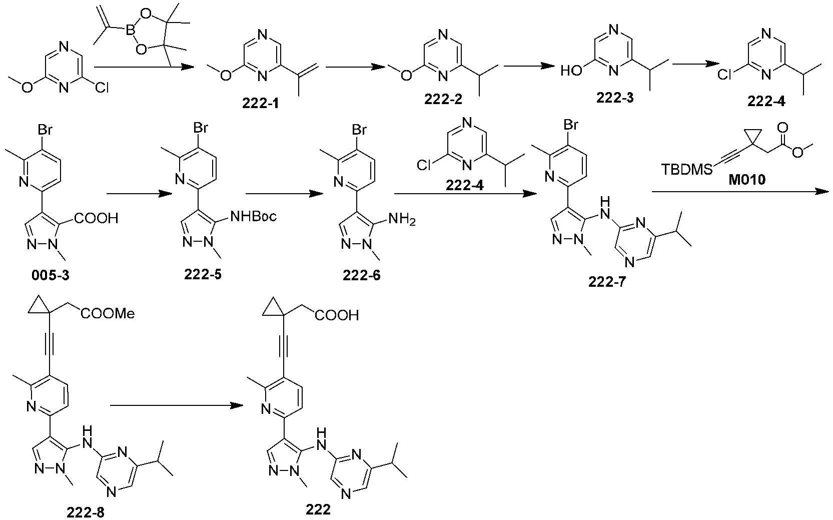

(1) At room temperature, 2-chloro-6-methoxypiperazine (1 g, 6.92 mmol) was dissolved in 1,4-dioxane (10 m) and water (2 mL), followed by the addition of isopropenylboronic acid pinacol ester (1.2 g, 7.27 mmol), [1,1'-bis(diphenylphosphino)ferrocene]dichloropalladium(II) (102 mg, 0.14 mmol), and potassium carbonate (1.9 g, 13.84 mmol). The reaction mixture was heated to 90°C under a nitrogen atmosphere and stirred for 16 hours. After cooling to room temperature, the reaction mixture was quenched by pouring into water (30 mL) and extracted with ethyl acetate (30 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 222-1 (1 g). LC-MS: [M+H]⁺= 151.05.
(2) At room temperature, compound 222-1 (1.0 g, 6.66 mmol) was dissolved in ethyl acetate (10 mL), followed by the addition of wet palladium on carbon (100 mg, purity: 10 %). The reaction mixture was stirred under a hydrogen atmosphere at room temperature for 3 hours. The reaction mixture was filtered under reduced pressure, and the filtrate was concentrated under reduced pressure to obtain compound 222-2 (800 mg). LC-MS: [M+H]⁺= 153.05.
(3) At room temperature, compound 222-2 (600 mg, 3.94 mmol) was dissolved in a solution of hydrogen chloride in 1,4-dioxane (6 mL, 4 M). The reaction mixture was heated to 80°C and stirred for 1 hour. The reaction mixture was cooled to room temperature, poured into water (20 mL) to dilute, and the pH of the solution was adjusted to 9 to 10 with sodium bicarbonate, followed by extraction with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 222-3 (240 mg). LCMS: [M+H]⁺ =139.05.
(4) At room temperature, compound 222-3 (270 mg, 1.95 mmol) and *N,N-*dimethylformamide (143 mg, 1.95 mmol) were dissolved in phosphorus oxychloride (3.9 g, 25.35 mmol). The reaction mixture was heated to 100°C and stirred for 5 hours. The reaction mixture was cooled to room temperature and slowly added dropwise to saturated aqueous sodium bicarbonate solution (50 mL) to quench, followed by extraction with ethyl acetate (30 mL × 3). The combined organic phases were washed with saturated aqueous ammonium chloride solution (20 mL × 3), then dried over anhydrous sodium sulfate, filtered, and the filtrate was concentrated under reduced pressure. The residue was purified by silica gel column chromatography (petroleum ether/ethyl acetate = 10/1) to obtain compound 222-4 (250 mg). LCMS: [M+H]⁺ =157.00.
(5) At room temperature, compound 005-3 (200 mg, 0.68 mmol) was dissolved in tert-butanol (5 mL), followed by the addition of diphenylphosphoryl azide (561 mg, 2.04 mmol) and triethylamine (206 mg, 2.04 mmol). The reaction mixture was heated to 80°C and stirred for 4 hours. After cooling to room temperature, the reaction mixture was quenched by pouring into water (10 mL) and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound 222-5 (200 mg). LCMS: [M+H]⁺ =367.05.
(6) At room temperature, compound 222-5 (200 mg, 0.54 mmol) was dissolved in dichloromethane (2 mL), followed by the addition of a solution of hydrogen chloride in 1,4-dioxane (2 mL, 4 M). The reaction mixture was stirred at room temperature for 16 hours. The reaction mixture was directly concentrated under reduced pressure. The residue was dissolved in water (10 mL), and the solution was adjusted to pH = 8 to 9 with saturated sodium bicarbonate, then extracted with dichloromethane (10 mL × 3). The combined organic phases were dried over anhydrous sodium sulfate, filtered, and the filtrate was concentrated under reduced pressure to obtain compound 222-6 (150 mg, crude). LCMS: [M+H]⁺ =266.95.
(7) At room temperature, compound 222-6 (150 mg, 0.56 mmol) was dissolved in anhydrous tetrahydrofuran (2 mL), and the reaction mixture was cooled to 0°C, followed by the addition of sodium hydride (45 mg, 1.12 mmol, 60% dispersed in mineral oil). The reaction mixture was stirred at 0°C for 0.5 hours. Compound 222-4 (88 mg, 0.56 mmol) was added. The reaction mixture was heated to 70°C and stirred for an additional 2 hours. The reaction mixture was cooled to room temperature, then slowly added to saturated aqueous sodium bicarbonate solution (10 mL) to quench, and extracted with dichloromethane (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 222-7 (80 mg). LCMS: [M+H]⁺ =387.05.
(8) At room temperature, compound 222-7 (80 mg, 0.21 mmol) and compound M010 (106 mg, 0.42 mmol) were dissolved in 1,4-dioxane (2 mL), followed by the addition of bis(triphenylphosphine)palladium(II) chloride (29 mg, 0.04 mmol), copper(I) iodide (8 mg, 0.04 mmol), triethylamine (64 mg, 0.63 mmol), potassium carbonate (58 mg, 0.42 mmol), and a solution of tetrabutylammonium fluoride in tetrahydrofuran (110 mg, 0.42 mmol, 1 M). The reaction mixture was heated to 90°C under a nitrogen atmosphere and stirred for an additional 2 hours. The reaction mixture was cooled to room temperature, then poured into water (10 mL) to dilute, and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 222-8 (100 mg, crude). LCMS: [M+H]⁺ =445.20.
(9) At room temperature, compound 222-8 (100 mg, 0.22 mmol) was dissolved in methanol (3 mL) and water (3 mL), followed by the addition of lithium hydroxide monohydrate (28 mg, 0.66 mmol). The reaction mixture was stirred at room temperature for 1 hour. The reaction mixture was diluted with water (10 mL) and concentrated under reduced pressure to remove methanol. The residue was washed with dichloromethane (10 mL × 3), and the aqueous phase was adjusted to pH = 3 to 4 with dilute hydrochloric acid (1 M), then extracted with ethyl acetate (10 mL × 3). The combined organic phases were dried over anhydrous sodium sulfate, filtered, and the filtrate was concentrated under reduced pressure. The residue was purified by high-performance liquid chromatography (0.1% ammonia in water system) and lyophilized to obtain compound 222 (31.3 mg). LC-MS: [M+H]⁺=431.5.

¹H NMR (400 MHz, DMSO-*d₆*) δ 9.18 (s, 1H), 7.98 (s, 1H), 7.88 (s, 1H), 7.83 (s, 1H), 7.47 (d, J = 8.0 Hz, 1H), 7.30 (d, J = 8.0 Hz, 1H), 3.67 (s, 3H), 2.80 - 2.73 (m, 1H), 2.38 (s, 2H), 2.27 (s, 3H), 1.02 (d, J = 7.2 Hz, 6H), 0.99 - 0.85 (m, 4H).

### Example 61: Preparation of 2-(1-((6-(5-((((2-chloro-5-fluoropyridin-3-yl)methoxy)carbonyl)amino)methyl)-1-methyl-1H-1,2,3-triazol-4-yl)-2-methylpyridin-3-yl)ethynyl)cyclopropyl)acetic acid (compound 117)

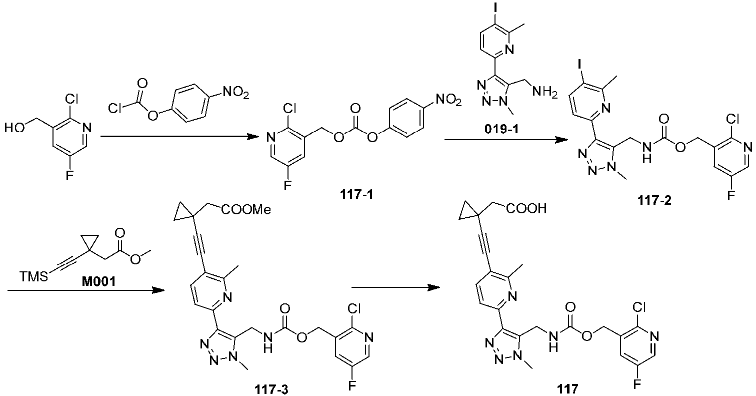

(1) At room temperature, 2-chloro-3-hydroxymethyl-5-fluoropyridine (500 mg, 3.09 mmol) was dissolved in dichloromethane (10 mL). After cooling the reaction mixture to 0°C, p-nitrophenyl chloroformate (1.2 g, 6.18 mmol) and pyridine (488 mg, 6.18 mmol) were added. After warming to room temperature, the reaction mixture was stirred for an additional 1 hour. The reaction mixture was diluted with water (15 mL) and extracted with dichloromethane (20 mL × 3). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 2/1) to obtain compound 117-1 (1.6 g, crude). LC-MS: [M+H]⁺= 326.95.
(2) At room temperature, compound 117-1 (400 mg, 1.23 mmol) and compound 019-1 (488 mg, 1.48 mmol) were dissolved in dichloromethane, followed by the addition of *N,N-*diisopropylethylamine (476 mg, 3.69 mmol). The reaction mixture was stirred at room temperature for 1 hour. The reaction mixture was diluted with water (15 mL) and extracted with dichloromethane (20 mL × 3). The combined organic phases were washed with saturated brine (30 mL), dried over anhydrous sodium sulfate, filtered, and the filtrate was concentrated under reduced pressure. The residue was purified by silica gel column chromatography (petroleum ether/ethyl acetate = 2/1) to obtain compound 117-2 (320 mg). LC-MS: [M+H]⁺ = 517.90.
(3) At room temperature, compound 117-2 (352 mg, 0.68 mmol) and M001 (286 mg, 1.36 mmol) were dissolved in 1,4-dioxane (8 mL), followed by the addition of triethylamine (206 mg, 2.04 mmol), potassium carbonate (188 mg, 1.36 mmol), cesium fluoride (413 mg, 2.72 mmol), bis(triphenylphosphine)palladium(II) chloride (48 mg, 0.068 mmol), and copper(I) iodide (13 mg, 0.068 mmol). Under a nitrogen atmosphere, the reaction mixture was heated to 90°C and stirred for 3 hours. The reaction mixture was cooled to room temperature, diluted with water (10 mL), and extracted with ethyl acetate (20 mL × 3). The combined organic phases were washed with saturated brine (30 mL). The organic phase was dried over anhydrous sodium sulfate, filtered, and the filtrate was concentrated under reduced pressure. The residue was purified by silica gel column chromatography (petroleum ether/ethyl acetate = 1/1) to obtain compound 117-3 (180 mg). LC-MS: [M+H]⁺= 527.00.
(4) At room temperature, compound 117-3 (180 mg, 0.34 mmol) was dissolved in methanol (6 mL) and water (3 mL), followed by the addition of lithium hydroxide monohydrate (43 mg, 1.02 mmol). The reaction mixture was stirred at room temperature for 2 hours. The reaction system was diluted with water (6 mL), and the pH of the solution was adjusted to 5 to 6 with dilute hydrochloric acid (1 M). The mixture was extracted with ethyl acetate (10 mL × 3). The combined organic phases were washed with saturated brine (20 mL). The organic phase was dried over anhydrous sodium sulfate, filtered, and the filtrate was concentrated under reduced pressure. The residue was purified by high-performance liquid chromatography (0.1% ammonia in water conditions) and then lyophilized to obtain compound 117 (74.3 mg).

LC-MS [M+H]⁺= 513.05.

¹H NMR (400 MHz, DMSO-*d₆*) δ 8.43 (d, *J* = 4.0 Hz, 1H), 7.86 - 7.79 (m, 3H), 7.72 (d, *J* = 8.0 Hz, 1H), 5.07 (s, 2H), 4.87 (d, *J =* 4.0 Hz, 2H), 4.07 (s, 3H), 2.55 (s, 3H), 2.44 (s, 2H), 1.06 - 1.03 (m, 2H), 0.96 - 0.93 (m, 2H).

### Example 62: Preparation of 2-(1-((6-(5-((6-isopropoxypyrazin-2-yl)amino)-1-methyl-1/7-1,2,3-triazol-4-yl)-2-methylpyridin-3-yl)ethynyl)cyclopropyl)acetic acid (compound 125)

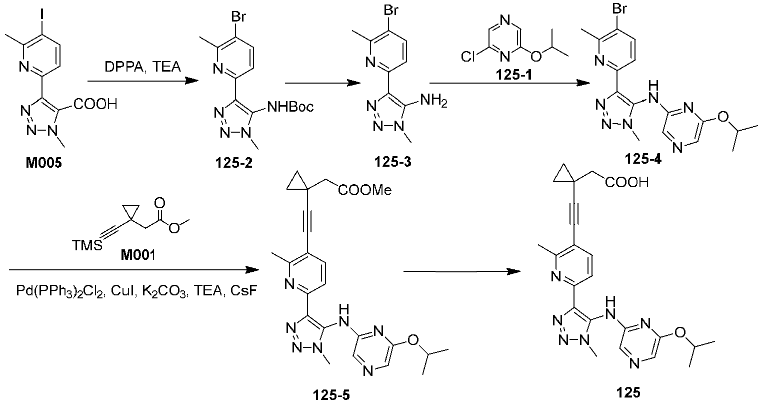

The preparation of compound 125 was referred to the method for preparing compound 222 in Example 60, wherein intermediate 222-4 was replaced with 125-1, intermediate 005-3 was replaced with M005, and intermediate M010 was replaced with M001, and compound 125 (91 mg) was obtained. LC-MS: [M+H]⁺ =448.10.

¹H NMR (400 MHz, DMSO-*d₆*) δ 9.30 (s, 1H), 7.77 - 7.71 (m, 2H), 7.63 (d, *J =* 8.0 Hz, 1H), 7.50 (s, 1H), 4.67-4.61 (m, 1H), 3.94 (s, 3H), 2.40 (s, 2H), 2.19 (s, 3H), 1.00 (d, *J =* 6.0 Hz, 8H), 0.95 - 0.89 (m, 2H).

The preparation method for compound 125-1 is as follows:

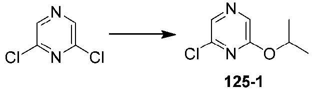

At room temperature, isopropanol (1.0 g, 16.6 mmol) was dissolved in anhydrous tetrahydrofuran (26 mL). After cooling the reaction to 0°C, sodium hydride (1.14 g, 28.5 mmol; 60% dispersed in mineral oil) was added. The reaction mixture was stirred at 0°C for 0.5 hours, then 2,6-dichloropyrazine (2.36 g, 15.8 mmol) was added. After warming to room temperature, the reaction mixture was stirred for an additional 1.5 hours. The reaction mixture was quenched with saturated aqueous ammonium chloride solution (20 mL) and extracted with ethyl acetate (20 mL × 3). The combined organic phases were washed with saturated brine (30 mL). The organic phase was dried over anhydrous sodium sulfate, filtered, and the filtrate was concentrated under reduced pressure. The residue was purified by silica gel column chromatography (petroleum ether/ethyl acetate = 1/0) to obtain compound 125-1 (2.0 g). LC-MS: [M+H]⁺= 173.06.

### Example 63: Preparation of 2-(1-((2-ethyl-6-(1-methyl-5-(((propoxycarbonyl)amino)methyl)-1H-1,2,3-triazol-4-yl)pyridin-3-yl)ethynyl)cyclopropyl)acetic acid (compound 193)

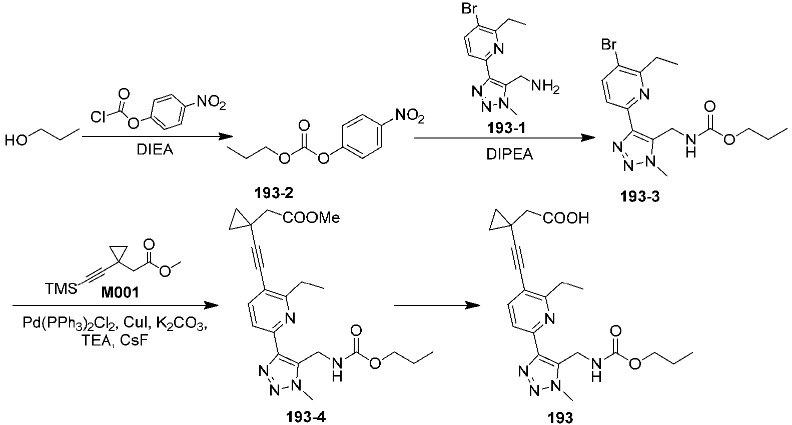

The preparation of compound 193 was referred to the method for preparing compound 117 in Example 61, wherein the starting material 2-chloro-3-hydroxymethyl-5-fluoropyridine was replaced with n-propanol, and compound 019-1 was replaced with compound 193-1, and compound 193 (66.9 mg) was obtained. LC-MS: [M+H]⁺ =426.15.

¹H NMR (400 MHz, DMSO-*d₆*) δ 7.83 (d, *J =* 8.0 Hz, 1H), 7.73 (d, *J =* 8.0 Hz, 1H), 7.47 (s, 1H), 4.83 (d, *J* = 4.8 Hz, 2H), 4.06 (s, 3H), 3.88 (t, *J =* 12.4 Hz, 2H), 2.93 (dd, *J =* 7.2, 7.6 Hz, 2H), 2.46 (s, 2H), 1.58 - 1.41 (m, 2H), 1.24 (t, *J =* 14.8 Hz, 3H), 1.05 (dd, *J =* 4.8,3.6 Hz, 2H), 0.95 (dd, *J =* 4.0, 4.8 Hz, 2H), 0.83 (t, *J* = 14.4 Hz, 3H).

The preparation method of compound 193-1 is as follows:

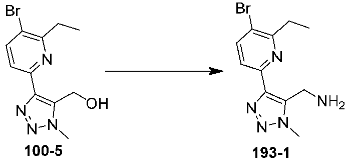

At room temperature, compound 100-5 (0.5 g, 1.7 mmol) was dissolved in anhydrous tetrahydrofuran (20 mL), followed by the addition of 1,8-diazabicyclo[5.4.0]undec-7-ene (512 mg, 3.4 mmol) and diphenylphosphoryl azide (697 mg, 2.6 mmol). The reaction mixture was stirred at room temperature for 18 hours. Then, triphenylphosphine (883 mg, 3.4 mmol) and water (4 mL) were added. The reaction mixture was stirred at room temperature for an additional 3 hours. The reaction mixture was diluted with water (20 mL) and extracted twice with ethyl acetate (30 mL × 2). The combined organic phases were washed with saturated sodium chloride solution (20 mL × 3), then dried over anhydrous sodium sulfate, filtered, and the filtrate was concentrated under reduced pressure. The residue was purified by silica gel column chromatography (dichloromethane/methanol/triethylamine = 10/1/0.05) to obtain compound 193-1 (0.38 g). LC-MS: [M+H]⁺ =298.00.

### Example 64: Preparation of trans-2-((2-methyl-6-(1-methyl-5-(((((R)-pentan-2-yl)oxy)carbonyl)amino)-1H-1,2,3-triazol-4-yl)pyridin-3-yl)ethynyl)cyclopropane-1-carboxylic acid (compound 224), compound 224-A, and compound 224-B

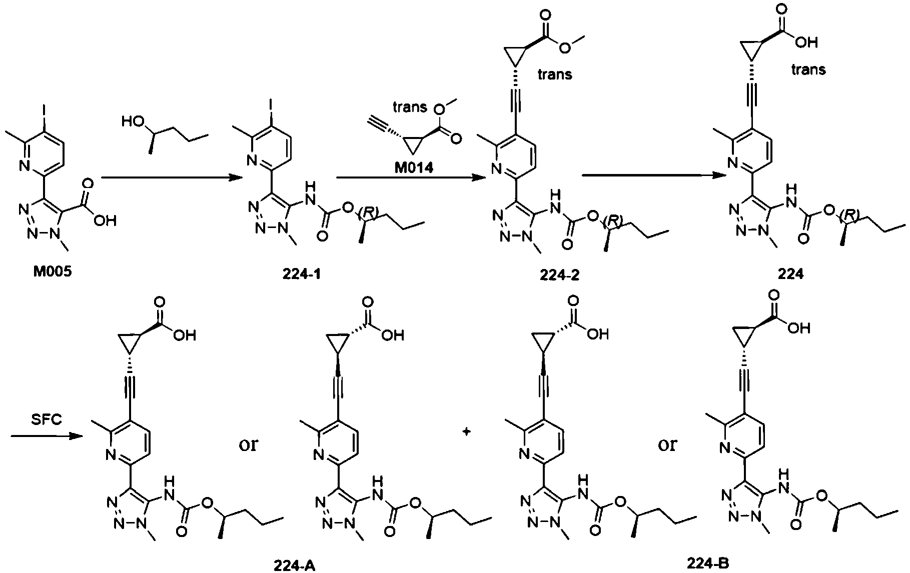

(1) At room temperature, compound M005 (1.3 g, 3.78 mmol) was dissolved in tetrahydrofuran (30 mL), followed by the addition of propylphosphonic anhydride (3.6 g, 5.67 mmol, 50% in ethyl acetate), azidotrimethylsilane (870 mg, 7.56 mmol), and triethylamine (1.9 g, 18.89 mmol). The reaction mixture was stirred at room temperature for 0.5 hours, then (R)-2-pentanol (666 mg, 7.56 mmol) was added. The reaction mixture was heated to 70°C and stirred for 1 hour. After cooling to room temperature, the reaction mixture was poured into water (50 mL) to quench and extracted with ethyl acetate (50 mL × 3). The combined organic phases were dried over anhydrous sodium sulfate, filtered, and the filtrate was concentrated under reduced pressure. The residue was purified by silica gel plate chromatography (petroleum ether/ethyl acetate = 3/1) to obtain compound 224-1 (1.4 g). LC-MS: [M+H]⁺=430.00.
(2) At room temperature, compound 224-1 (400 mg, 0.93 mmol) was dissolved in 1,4-dioxane (15 mL), followed by the addition of compound M014 (231 mg, 1.86 mmol), triethylamine (283 mg, 2.79 mmol), copper(I) iodide (35 mg, 0.19 mmol), bis(triphenylphosphine)palladium(II) chloride (65 mg, 0.09 mmol), and potassium carbonate (258 mg, 1.86 mmol). The reaction mixture was heated to 90°C under a nitrogen atmosphere and stirred for 1 hour. After cooling to room temperature, the reaction mixture was quenched by pouring into water (30 mL) and extracted with ethyl acetate (30 mL × 3). The combined organic phases were dried over anhydrous sodium sulfate, filtered, and the filtrate was concentrated under reduced pressure. The residue was purified by silica gel thin-layer chromatography (petroleum ether/ethyl acetate = 1/1) to obtain compound 224-2 (320 mg, containing a pair of trans isomers). LC-MS: [M+H]⁺=426.15.
(3) At room temperature, compound 224-2 (400 mg, 0.94 mmol) was dissolved in methanol (20 mL) and water (7 mL), followed by the addition of lithium hydroxide monohydrate (118 mg, 2.82 mmol). The reaction mixture was stirred at room temperature for 1 hour. The reaction mixture was diluted with water (20 mL) and concentrated under reduced pressure to remove methanol. The residue was washed with dichloromethane (10 mL × 3). The aqueous phase was adjusted to pH = 3 to 4 with dilute hydrochloric acid (1 M) and extracted with ethyl acetate (20 mL × 3). The combined organic phases were dried over anhydrous sodium sulfate, filtered, and the filtrate was concentrated under reduced pressure. The residue was purified by high-performance liquid chromatography (0.1% ammonia in water system) and lyophilized to obtain compound 224 (260.3 mg, containing a pair of trans isomers). LC-MS: [M+H]⁺=412.15.
   ¹H NMR (600 MHz, DMSO-*d₆*) δ 9.51 (s, 1H), 7.72 (q, J = 8.4 Hz, 2H), 4.68 (s, 1H), 3.86 (s, 3H), 2.49 (s, 3H), 1.70 - 1.67 (m, 1H), 1.61 - 1.59 (m, 1H), 1.43 - 0.98 (m, 7H), 0.95 - 0.66 (m, 5H).
(4) At room temperature, compound 224 (250 mg, 0.61 mmol) was subjected to chiral separation (Daicel Chiralpak IK column, 250 × 25 mm I.D., 10 µm; mobile phase A: supercritical carbon dioxide; mobile phase B: EtOH and NH₃H₂O (NH₃H₂O accounts for 0.1% of the volume of mobile phase B); volume ratio of mobile phase A to B: 65:35; flow rate: 70 g/min; isocratic elution; column temperature: room temperature; injection volume: 3 µL; detection wavelength: 220 nm) to obtain compound 224-A (retention time Rt = 9.498 min, 88.7 mg) and compound 224-B (retention time Rt = 7.200 min, 103.5 mg). LC-MS: [M+H]⁺=412.10.

Compound 224-A: ¹H NMR (400 MHz, DMSO-*d₆*) δ 9.42 (s, 1H), 7.74 (q, J = 8.0 Hz, 2H), 4.68 (s, 1H), 3.86 (s, 3H), 2.50 (s, 3H), 1.85 - 1.80 (m, 2H), 1.60 - 0.96 (m, 9H), 0.81 (s, 3H).

Compound 224-B: ¹H NMR (400 MHz, DMSO-*d₆*) δ 9.47 (s, 1H), 7.72 (q, J = 8.0 Hz, 2H), 4.68 (s, 1H), 3.86 (s, 3H), 2.50 (s, 3H), 1.74 - 1.57 (m, 2H), 1.56 - 0.89 (m, 9H), 0.81 (s, 3H).

### Example 65: Preparation of the trifluoroacetate 2-(1-((6-(5-(((butyl(methyl)carbamoyl)oxy)methyl)-1-methyl-1H-1,2,3-triazol-4-yl)-2-methoxypyridin-3-yl)ethynyl)cyclopropyl)acetic acid (compound 235)

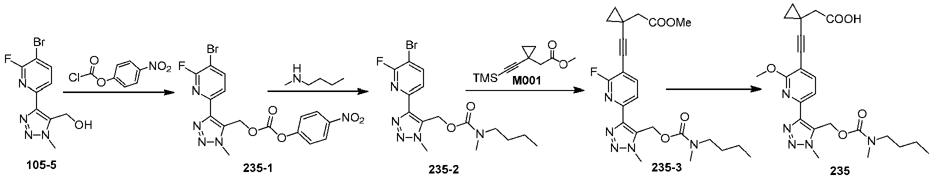

(1) At room temperature, compound 105-5 (1.4 g, 4.89 mmol) was dissolved in dichloromethane (40 mL), followed by the sequential addition of pyridine (0.77 g, 9.74 mmol) and 4-nitrophenyl chloroformate (1.97 g, 9.74 mmol). The reaction mixture was stirred at room temperature for 1 hour. The reaction mixture was diluted with water (40 mL) and extracted with dichloromethane (40 mL × 2). The combined organic phases were washed with saturated brine (40 mL). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 235-1 (2.2 g). LC-MS: [M+H]⁺ =451.90.
(2) At room temperature, compound 235-1 (2.2 g, 4.88 mmol) and *N*-methylbutylamine (1.1 g, 12.2 mmol) were dissolved in dichloromethane (25 mL), followed by the addition of *N,N*-diisopropylethylamine (1.89 g, 14.63 mmol). The reaction mixture was stirred at room temperature for 1 hour. The reaction mixture was diluted with water (20 mL) and extracted with dichloromethane (20 mL × 2). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 3/1) to obtain compound 235-2 (1.8 g). LC-MS: [M+H]⁺ =402.00.
(3) At room temperature, compound 235-2 (200 mg, 0.5 mmol) and compound M001 (158 mg, 0.75 mmol) were dissolved in 1,4-dioxane (4 mL), followed by the addition of triethylamine (152 mg, 1.55 mmol), potassium carbonate (138 mg, 1.0 mmol), cesium fluoride (304 mg, 2.0 mmol), bis(triphenylphosphine)palladium(II) chloride (35.1 mg, 0.05 mmol), and copper(I) iodide (10 mg, 0.05 mmol). Under a nitrogen atmosphere, the reaction mixture was heated to 90°C and stirred for 3 hours. After cooling to room temperature, the reaction mixture was diluted with water (10 mL) and extracted with ethyl acetate (10 mL × 3). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 1/1) to obtain compound 235-3 (180 mg). LC-MS: [M+H]⁺ =458.10.
(4) At room temperature, compound 235-3 (200 mg, 0.44 mmol) was dissolved in tetrahydrofuran (2 mL) and methanol (2 mL), followed by the addition of a solution of lithium hydroxide monohydrate (55 mg, 1.31 mmol) in water (0.5 mL). The reaction mixture was stirred at room temperature for 4 hours. The reaction mixture was adjusted to pH = 7 with dilute hydrochloric acid (1 M), and methanol and tetrahydrofuran were removed by concentration under reduced pressure. The residue was dissolved in ethyl acetate (4 mL). The solution was back-extracted with saturated aqueous sodium bicarbonate solution (3 mL × 3). The aqueous phase was adjusted to pH = 5 with dilute hydrochloric acid (1 M) and then extracted with ethyl acetate (5 mL × 3). The combined organic phases were washed with saturated brine (5 mL), dried over anhydrous sodium sulfate, filtered, and the filtrate was concentrated under reduced pressure. The residue was purified by high-performance liquid chromatography (0.5% trifluoroacetic acid conditions) and lyophilized to obtain the trifluoroacetate salt of compound 235 (23 mg). LC-MS: [M+H]⁺ =456.15.

¹H NMR (400 MHz, DMSO-*d₆*) δ 7.74 (d, *J* = 7.6 Hz, 1H), 7.63 (d, *J* = 7.6 Hz, 1H), 7.24 - 6.90 (m, 1H), 5.64 (d, *J* = 8.0 Hz, 2H), 4.11 (s, 3H), 3.90 (s, 4H), 3.14 (t, *J* = 6.8 Hz, 1H), 3.04 (t, *J =* 6.8 Hz, 1H), 2.73 (d, *J* = 10.0 Hz, 3H), 2.41 (s, 2H).

### Example 66: Preparation of 2-(1-((6-(5-(((1-(bicyclo[1.1.1]pentan-1-yl)ethoxy)carbonyl)amino)-1-methyl-1H-1,2,3-triazol-4-yl)-2-fluoropyridin-3-yl)ethynyl)cyclopropyl)acetic acid (compound 237), compound 237-A, and compound 237-B

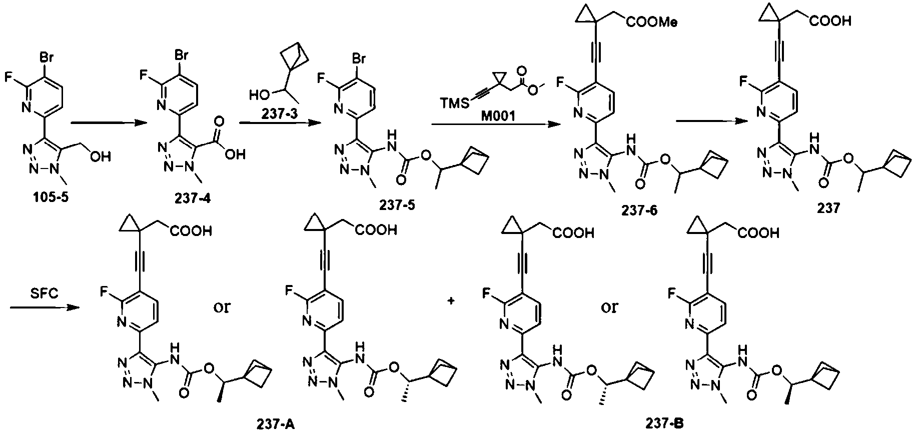

(1) At room temperature, compound 105-5 (1.0 g, 3.48 mmol) was dissolved in acetonitrile (10 mL) and water (10 mL), followed by the addition of potassium permanganate (1.1 g, 6.96 mmol). The reaction mixture was stirred at room temperature for 16 hours. The pH of the reaction mixture was adjusted to 13 with aqueous sodium hydroxide solution (15% in water), and ethyl acetate (15 mL) was added. Under an ice-water bath, saturated aqueous sodium sulfite solution (30 mL) was added dropwise until the purple-red color faded. The resulting mixture was filtered, and the filter cake was washed with water (10 mL × 3). The phases of the filtrate were separated, and the aqueous phase was adjusted to pH = 5 with dilute hydrochloric acid (1 M), then filtered. The filter cake was washed twice with water (10 mL × 2), twice with ethanol (10 mL × 2), and twice with petroleum ether (10 mL × 2). The filter cake was dried under reduced pressure to obtain compound 237-4 (710 mg). LC-MS: [M+H]⁺ =300.90.
(2) At room temperature, compound 237-4 (0.16 g, 0.53 mmol) was dissolved in anhydrous tetrahydrofuran (5 mL), followed by the addition of propylphosphonic anhydride (1 g, 1.6 mmol, 50% in ethyl acetate), azidotrimethylsilane (183 mg, 1.6 mmol), and triethylamine (215 mg, 2.1 mmol). The reaction mixture was stirred at room temperature for 1 hour. Compound 237-3 (119 mg, 1.06 mmol) was added to the reaction mixture, and the reaction mixture was then heated to 90°C and stirred for an additional 1 hour. After cooling to room temperature, the reaction mixture was directly concentrated under reduced pressure. The residue was purified by silica gel column chromatography (ethyl acetate/petroleum ether = 1/2) to obtain compound 237-5 (210 mg). LC-MS: [M+H]⁺ =409.95.
(3) At room temperature, compound 237-5 (200 mg, 0.49 mmol) and compound M001 (206 mg, 0.98 mmol) were dissolved in 1,4-dioxane (5 mL), followed by the addition of bis(triphenylphosphine)palladium(II) chloride (35 mg, 0.049 mmol), copper(I) iodide (9.3 mg, 0.049 mmol), triethylamine (248 mg, 2.5 mmol), potassium carbonate (136 mg, 0.98 mmol), and cesium fluoride (298 mg, 1.96 mmol). Under a nitrogen atmosphere, the reaction mixture was heated to 90°C and stirred for 2 hours. After cooling to room temperature, the reaction mixture was diluted with water (10 mL) and extracted twice with ethyl acetate (20 mL × 2). The combined organic phases were dried over anhydrous sodium sulfate, filtered, and the filtrate was concentrated under reduced pressure. The residue was purified by silica gel column chromatography (ethyl acetate/petroleum ether = 1/3) to obtain compound 237-6 (163 mg). LC-MS: [M+H]⁺ =468.15.
(4) At room temperature, compound 237-6 (163 mg, 0.35 mmol) was dissolved in methanol (3 mL) and water (1 mL), followed by the addition of lithium hydroxide (84 mg, 3.5 mmol). The reaction mixture was stirred at room temperature for 1 hour. After the reaction mixture was diluted with water (20 mL), methanol was removed by concentration under reduced pressure. The residue was extracted with ethyl acetate (20 mL × 3), and the aqueous phase was adjusted to pH = 3 to 4 with dilute hydrochloric acid (1 M), followed by extraction with ethyl acetate (20 mL × 3). The combined organic phases were dried over anhydrous sodium sulfate, filtered, and the filtrate was concentrated under reduced pressure. The residue was purified by high-performance liquid chromatography (0.1% ammonia in water conditions) and then lyophilized to obtain compound 237 (102 mg). LC-MS: [M+H]⁺ =454.20.
   ¹H NMR (400 MHz, DMSO-*d*₆) δ 10.97 - 9.22 (m, 1H), 8.05 - 7.96 (m, 1H), 7.86 (d, *J=* 8.0 Hz, 1H), 4.68 (s, 1H), 3.90 (s, 3H), 2.44 (s, 2H), 1.69 (s, 6H), 1.34 - 1.03 (m, 5H), 1.02 - 0.71 (m, 3H).
(5) At room temperature, compound 237 (95 mg, 210 µmol) was subjected to chiral separation (Daicel Chiralpak IG column, 250 × 30 mm I.D., 5 µm; flow rate: 55 g/min; column temperature: room temperature; mobile phase A: supercritical carbon dioxide; mobile phase B: EtOH and NH₃H₂O (NH₃H₂O accounts for 0.1% of the volume of mobile phase B); volume ratio of mobile phase A to B: 85:15; isocratic elution; injection volume: 4 µL; detection wavelength: 220 nm) to obtain compound 237-A (retention time Rt = 11.459 min, 19.6 mg) and compound 237-B (retention time Rt = 12.875 min, 19.8 mg). LCMS: [M+H]⁺=454.15.

Compound 237-A: ¹H NMR (400 MHz, DMSO-*d*₆) δ 9.64 (s, 1H), 8.06 - 7.96 (m, 1H), 7.87 (d, *J* = 8.0 Hz, 1H), 4.68 (s, 1H), 3.88 (s, 3H), 2.45 (s, 2H), 1.70 (s, 6H), 1.37 - 1.10 (m, 3H), 1.10 - 0.98 (m, 3H), 0.96 (dd, *J* = 4.0*,* 5.2 Hz, 2H).

Compound 237-B: ¹H NMR (400 MHz, DMSO-*d₆*) δ 9.65 (s, 1H), 8.06 - 7.97 (m, 1H), 7.86 (d, *J* = 8.0Hz, 1H), 4.68 (s, 1H), 3.88 (s, 3H), 2.45 (s, 2H), 1.69 (s, 6H), 1.35 - 1.10 (m, 3H), 1.09 - 0.99 (m, 3H), 0.96 (dd, *J* = 4.0, 5.2 Hz, 2H).

### Example 67: Preparation of 2-(1-((6-(5-((((3-fluoropentyl)(methyl)carbamoyl)oxy)methyl)-1-methyl-1H-1,2,3-triazol-4-yl)-2-methylpyridin-3-yl)ethynyl)cyclopropyl)acetic acid (compound 238)

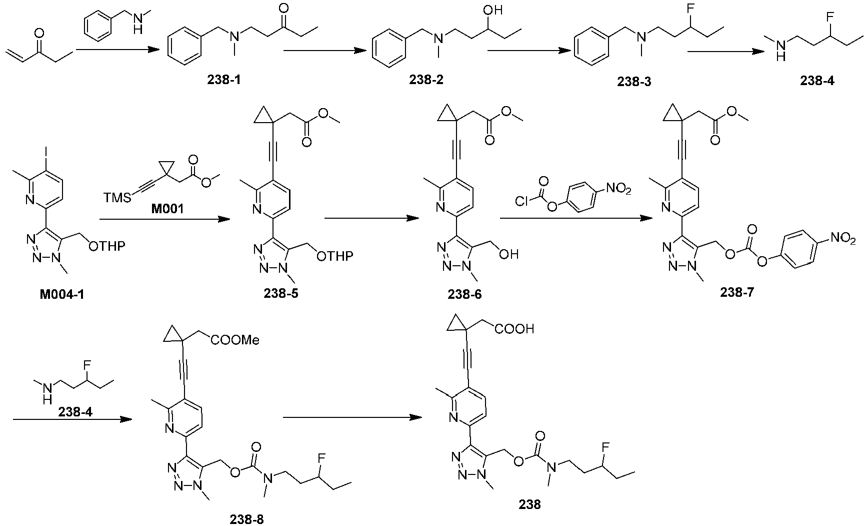

(1) At room temperature, 1-penten-3-one (5.10 g, 60.65 mmol) was dissolved in methanol (50 mL), and the reaction mixture was cooled to 0°C, followed by the dropwise addition of *N*-methyl-1-phenylmethanamine (7.0 g, 57.76 mmol). After warming to room temperature, the reaction mixture was stirred for an additional 2 hours. The reaction mixture was directly concentrated under reduced pressure. The residue was purified by silica gel column chromatography (dichloromethane/methanol = 20/1) to obtain compound 238-1 (10.5 g). LC-MS: [M+H]⁺= 206.10.
(2) At room temperature, compound 238-1 (3.0 g, 14.61 mmol) was dissolved in methanol (30 mL), and the reaction mixture was cooled to 0°C, followed by the portionwise addition of sodium borohydride (553 mg, 14.61 mmol). After the addition was completed, the reaction mixture was warmed to room temperature and stirred for an additional 1 hour. The reaction mixture was quenched with aqueous ammonium chloride solution (30 mL) and extracted with ethyl acetate (30 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 238-2 (2.6 g). LC-MS: [M+H]⁺= 208.15.
(3) At room temperature, compound 238-2 (2.6 g, 12.54 mmol) was dissolved in dichloromethane (30 mL), and the reaction mixture was cooled to -78°C, followed by the dropwise addition of diethylaminosulfur trifluoride (3.03 g, 4.26 mmol). After the dropwise addition was completed, the reaction mixture was slowly warmed to room temperature and stirred for an additional 2 hours. The reaction mixture was quenched with saturated aqueous sodium bicarbonate solution (20 mL) and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 238-3 (700 mg). LC-MS: [M+H]⁺- 210.10.
(4) At room temperature, compound 238-3 (600 mg, 1.06 mmol) was dissolved in methanol (6 mL), followed by the addition of palladium hydroxide on carbon (10%, 150 mg) and palladium on carbon (10%, 150 mg). Under a hydrogen balloon atmosphere, the reaction mixture was heated to 40°C and stirred for an additional 16 hours. After cooling to room temperature, the reaction mixture was directly filtered under reduced pressure. The filter cake was washed with methanol (10 mL). The resulting filtrate was added with a solution of hydrogen chloride in methanol (5 mL, 4 M) and directly concentrated under reduced pressure to obtain the hydrochloride of compound 238-4 (1.0 g, crude). LC-MS: [M+H]⁺= 120.15.
(5) At room temperature, M004-1 (3.0 g, 7.24 mmol) and M001 (3.05 g, 14.48 mmol) were dissolved in 1,4-dioxane (30 mL), followed by the addition of bis(triphenylphosphine)palladium(II) chloride (508 mg, 0.724 mmol), copper(I) iodide (275 mg, 1.45 mmol), cesium fluoride (4.4 g, 28.97 mmol), potassium carbonate (2.0 g, 14.48 mmol), and triethylamine (2.2 g, 21.73 mmol). Under a nitrogen atmosphere, the reaction mixture was heated to 90°C and stirred for an additional 5 hours. The reaction mixture was cooled to room temperature, diluted with water (100 mL), and extracted with ethyl acetate (100 mL × 3). The organic phases were combined, dried over anhydrous sodium sulfate, filtered, and the filtrate was concentrated under reduced pressure. The residue was purified by silica gel column chromatography (petroleum ether/ethyl acetate = 2/1) to obtain compound 238-5 (2.5 g). LC-MS: [M+H]⁺ =425.05.
(6) At room temperature, compound 238-5 (2.5 g, 5.89 mmol) was dissolved in methanol (30 mL), followed by the addition of pyridinium p-toluenesulfonate (1.78 g, 7.07 mmol). The reaction mixture was heated to 60°C and stirred for an additional 2 hours. After cooling to room temperature, the reaction mixture was quenched with water (30 mL) and extracted with ethyl acetate (30 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 238-6 (1.4 g). LC-MS: [M+H]⁺ = 341.00.
(7) At room temperature, compound 238-6 (1.4 g, 4.11 mmol) was dissolved in dichloromethane (30 mL), followed by the addition of pyridine (650 mg, 8.23 mmol) and 4-nitrophenyl chloroformate (1.66 g, 8.23 mmol). The reaction mixture was stirred at room temperature for 1 hour. The reaction mixture was diluted with water (30 mL) and extracted with dichloromethane (30 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 238-7 (1.8 g). LC-MS: [M+H]⁺ = 506.05.
(8) At room temperature, compound 238-7 (200 mg, 0.396 mmol) was dissolved in dichloromethane (8 mL), followed by the addition of *N,N-*diisopropylethylamine (255 mg, 1.98 mmol) and compound 238-4 (200 mg, crude hydrochloride). The reaction mixture was stirred at room temperature for 1 hour. The reaction mixture was diluted with water (10 mL) and extracted with dichloromethane (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 238-8 (120 mg). LC-MS: [M+H]⁺ = 486.10.
(9) At room temperature, compound 238-8 (120 mg, 0.25 mmol) was dissolved in methanol (3 mL) and water (1 mL), followed by the addition of lithium hydroxide monohydrate (31 mg, 0.74 mmol). The reaction mixture was stirred at room temperature for 2 hours. After neutralizing the reaction mixture with dilute hydrochloric acid (1 M) (pH = 7), the mixture was extracted with ethyl acetate (3 mL × 3). The combined organic phases were dried over anhydrous sodium sulfate, filtered, and the filtrate was concentrated under reduced pressure. The residue was purified by preparative high-performance liquid chromatography (0.1% ammonia in water) and lyophilized to obtain compound 238 (38.5 mg). LC-MS: [M+H]⁺ =472.25.

¹H NMR (400 MHz, CD₃OD) δ 7.84 (d, *J* = 8.2 Hz, 1H), 7.72 (d, *J* = 8.2 Hz, 1H), 5.65 (d, *J =* 12.8 Hz, 2H), 4.51 - 4.17 (m, 1H), 4.11 (s, 3H), 3.34 - 3.16 (m, 2H), 2.78 (d, *J* = 20.6 Hz, 3H), 2.54 (s, 3H), 2.45 (s, 2H), 1.82 - 1.67 (m, 1H), 1.63 - 1.48 (m, 2H), 1.43 - 1.21 (m, 1H), 1.08 - 1.02 (m, 2H), 0.98 - 0.92 (m, 2H), 0.91 - 0.70 (m, 3H).

### Example 68: Preparation of 2-(1-((6-(5-((((3-fluoro-3-methylbutyl)(methyl)carbamoyl)oxy)methyl)-1-methyl-1H-1,2,3-triazol-4-yl)-2-methylpyridin-3-yl)ethynyl)cyclopropyl)acetic acid (compound 239)

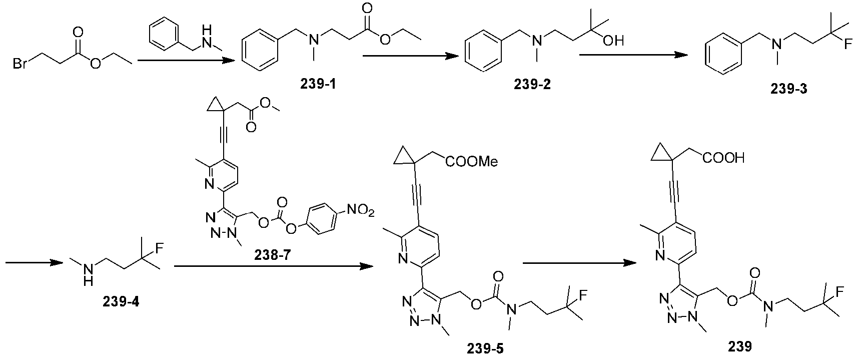

(1) At room temperature, ethyl 3-bromopropionate (10 g, 55.24 mmol) and *N*-methyl-1-phenylmethanamine (7.36 g, 60.76 mmol) were dissolved in acetonitrile (200 mL), followed by the addition of potassium carbonate (11.45 g, 82.86 mmol) and potassium iodide (0.917 g, 5.52 mmol). Under a nitrogen atmosphere, the reaction mixture was heated to 80°C and stirred for an additional 16 hours. The reaction mixture was cooled to room temperature, diluted with water (100 mL), and extracted with ethyl acetate (100 mL × 3). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 2/1) to obtain compound 239-1 (10.5 g). LC-MS: [M+H]⁺ =222.15.
(2) At room temperature, compound 239-1 (2.1 g, 9.5 mmol) was dissolved in anhydrous tetrahydrofuran (30 mL), and the reaction mixture was cooled to -78°C. Methylmagnesium bromide (9.5 mL, 28.5 mmol, 3 M in diethyl ether) was added dropwise. After the dropwise addition was completed, the reaction mixture was slowly warmed to room temperature and stirred for an additional 2 hours. The reaction mixture was cooled to 0°C, and saturated aqueous ammonium chloride solution (20 mL) was added to quench. Then, the mixture was extracted twice with ethyl acetate (20 mL × 2). The organic phases were combined, washed with saturated brine (40 mL), dried over anhydrous sodium sulfate, filtered, and the filtrate was concentrated under reduced pressure. The residue was purified by silica gel column chromatography (petroleum ether/ethyl acetate = 4/1) to obtain compound 239-2 (2.5 g). LC-MS: [M+H]⁺ =208.20.
(3) At room temperature, a solution of compound 239-2 (2.0 g, 9.66 mmol) in dichloromethane (20 mL) was added dropwise to a solution of diethylaminosulfur trifluoride in dichloromethane (20 mL) that had been cooled to 0°C. After the dropwise addition was completed, the reaction mixture was warmed to room temperature and stirred for an additional 1 hour. The reaction mixture was cooled to 0°C, then quenched with saturated aqueous sodium bicarbonate solution (20 mL), diluted with water (20 mL), and extracted twice with dichloromethane (20 mL × 2). The combined organic phases were dried over anhydrous sodium sulfate, filtered, and the filtrate was concentrated under reduced pressure. The residue was purified by column chromatography (petroleum ether/ethyl acetate = 5/1) to obtain compound 239-3 (1.6 g, crude). LC-MS of compound 239-3: [M+H]⁺ =210.15.
(4) At room temperature, compound 239-3 (1.8 g, crude) was dissolved in methanol (20 mL), followed by the addition of palladium on carbon (1.0 g, 10%) and palladium hydroxide on carbon (1.0 g, 5%). Under a hydrogen atmosphere, the reaction mixture was heated to 60°C and stirred for an additional 2 hours. After cooling to room temperature, the reaction mixture was directly filtered, and the filter cake was washed with methanol (10 mL). The resulting filtrate was added with a solution of hydrogen chloride in methanol (10 mL, 4 M), stirred for 10 minutes, and directly concentrated under reduced pressure to obtain the hydrochloride of compound 239-4 (1.8 g, crude). LC-MS: [M+H]⁺ =120.20.
(5) At room temperature, compound 238-7 (140 mg, 0.30 mmol) was dissolved in dichloromethane (3 mL), followed by the addition of *N,N-*diisopropylethylamine (768 mg, 5.95 mmol) and the hydrochloride of compound 239-4 (140 mg, crude). The reaction mixture was stirred at room temperature for 2 hours. The reaction mixture was diluted with water (5 mL) and extracted with dichloromethane (5 mL × 3). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 2/1) to obtain compound 239-5 (150 mg, crude). LC-MS: [M+H]⁺ =486.10.
(6) At room temperature, compound 239-5 (300 mg, crude) was dissolved in anhydrous tetrahydrofuran (3 mL) and methanol (3 mL), followed by the addition of a solution of lithium hydroxide monohydrate (130 mg, 3.09 mmol) in water (1 mL). The reaction mixture was stirred at room temperature for 2 hours. Ethyl acetate (5 mL) and water (5 mL) were added to the reaction mixture, and the organic phase was separated. The aqueous phase was adjusted to pH = 5 with dilute hydrochloric acid (1 M) and then extracted with ethyl acetate (5 mL × 3). The combined organic phases were washed with saturated brine (10 mL), dried over anhydrous sodium sulfate, filtered, and the filtrate was concentrated under reduced pressure. The crude product was purified by high-performance liquid chromatography (0.5% hydrochloric acid conditions) and lyophilized to obtain compound 239 (37.8 mg).

LC-MS: [M+H]⁺= 472.1.

¹H NMR (400 MHz, DMSO-*d₆*) δ 7.84 (d, *J* = 8.0 Hz, 1H), 7.73 (d, *J* = 8.0 Hz, 1H), 5.65 (d, *J* = 10.0 Hz, 2H), 4.11 (s, 3H), 3.17 (d, *J* = 7.2 Hz, 2H), 2.77 (d, *J* = 19.2 Hz, 3H), 2.54 (s, 3H), 2.45 (s, 2H), 1.84 - 1.71 (m, 1H), 1.65 - 1.54 (m, 1H), 1.33 - 1.22 (m, 3H), 1.10 (d, *J* = 22.0 Hz, 3H), 1.07 - 1.04 (m, 2H), 0.95 (m, 2H).

### Example 69: Preparation of 2-(1-((6-(5-((((3,3-difluoropentyl)(methyl)carbamoyl)oxy)methyl)-1-methyl-1H-1,2,3-triazol-4-yl)-2-methylpyridin-3-yl)ethynyl)cyclopropyl)acetic acid (compound 241)

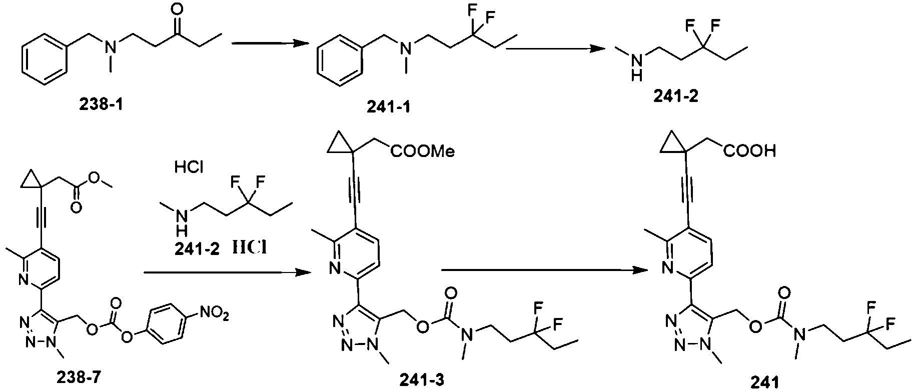

The preparation of compound 241 was referred to the method for preparing compound 238 in Example 67, wherein the hydrochloride of intermediate compound 238-4 was replaced with the hydrochloride of intermediate compound 241-2, and compound 241 (50.8 mg) was obtained. LC-MS: [M+H]⁺ = 490.20.

¹H NMR (400 MHz, DMSO-*d₆*) δ 7.85 (d, *J =* 8.1 Hz, 1H), 7.73 (d, *J =* 8.1 Hz, 1H), 5.66 (d, *J =* 16.0 Hz, 2H), 4.11 (s, 3H), 3.25 (d, *J* = 6.8 Hz, 2H), 2.78 (d, *J* = 19.6 Hz, 3H), 2.53 (s, 3H), 2.46 (s, 2H), 2.15 - 2.00 (m, 1H), 1.96 - 1.79 (m, 2H), 1.64 (m, 1H), 1.06 (m, 2H), 0.96 (m, 2H), 0.93 - 0.69 (m, 3H).

The preparation steps for the hydrochloride of compound 241-2 are as follows:
(1) At room temperature, in a sealed tube, compound 238-1 (1.4 g, 6.8 mmol) was dissolved in dichloromethane (20 mL), followed by the addition of triethylamine (688 mg, 6.8 mmol), triethylamine trihydrofluoride (2.2 g, 13.6 mmol), and (diethylamino)difluoroindole tetrafluoroborate (3.12 g, 13.6 mmol). The reaction mixture was stirred at room temperature for 16 hours. The reaction mixture was added dropwise to saturated aqueous sodium bicarbonate solution (30 mL) that had been cooled to 0°C to quench, followed by extraction with dichloromethane (20 mL × 2). The organic phases were combined, washed with saturated brine (40 mL), dried over anhydrous sodium sulfate, filtered, and the filtrate was concentrated under reduced pressure. The residue was purified by silica gel column chromatography (petroleum ether/ethyl acetate = 4/1) to obtain compound 241-1 (1.2 g). LC-MS: [M+H]+ =228.15.
(2) At room temperature, compound 241-1 (1.2 g, 5.3 mmol) was dissolved in methanol (7 mL), followed by the addition of wet palladium on carbon (0.6 g, 10%), palladium hydroxide on carbon (0.6 g, 10%), and a solution of hydrogen chloride in methanol (7 mL, 4 M). Under a hydrogen balloon atmosphere, the reaction mixture was heated to 45°C and stirred for an additional 2 hours. After cooling to room temperature, the reaction mixture was directly filtered, and the filter cake was washed with methanol (60 mL). The filtrate was directly concentrated under reduced pressure to obtain the hydrochloride of compound 241-2 (crude, 1.2 g). LC-MS: [M+H]+ =138.20.

### Example 70: Preparation of compound 242-A and compound 242-B

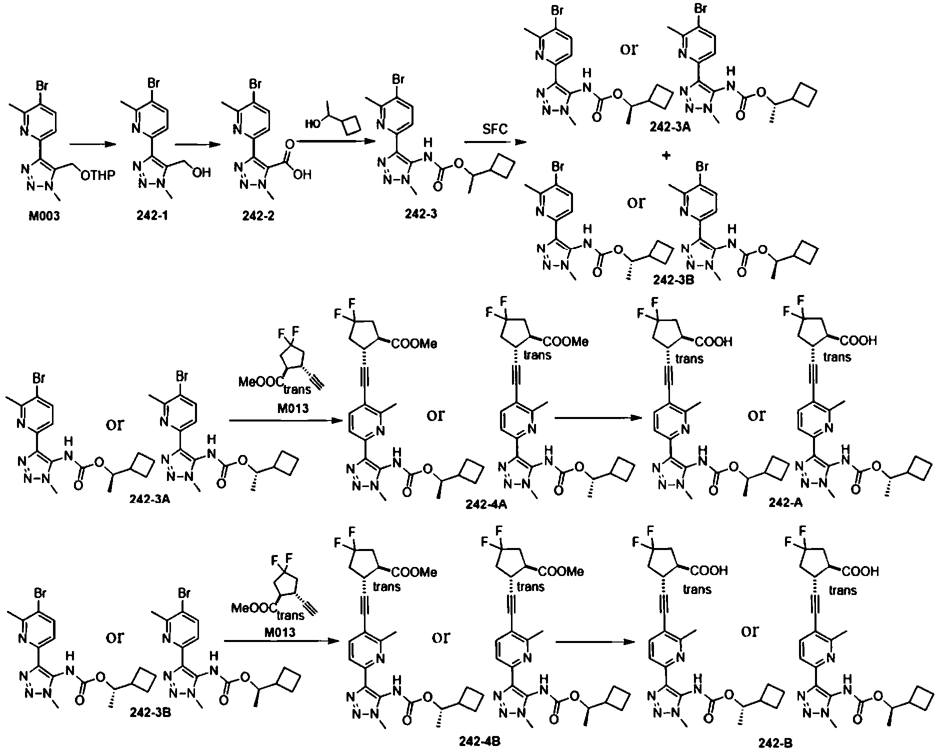

(1) At room temperature, compound M003 (6.0 g, 16.34 mmol) was dissolved in methanol (60 mL), and pyridinium p-toluenesulfonate (4.1 g, 16.34 mmol) was added. The reaction was heated to 60°C and stirred for 1 hour. After cooling to room temperature, the reaction mixture was directly concentrated under reduced pressure. The residue was diluted with water (60 mL) and extracted with ethyl acetate (40 mL × 2). The combined organic phases were washed with saturated aqueous sodium bicarbonate solution (60 mL) and saturated brine (60 mL). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 242-1 (4.5 g). LC-MS: [M+H]⁺ = 282.95.
(2) At room temperature, compound 242-1 (600 mg, 2.1 mmol) was dissolved in acetonitrile (6 mL) and water (4.5 mL), followed by the addition of potassium permanganate (664 mg, 4.2 mmol). The reaction mixture was stirred at room temperature for 22 hours. The reaction mixture was added with aqueous sodium hydroxide solution (10 mL, 2 M) and extracted with ethyl acetate (10 mL × 3). The combined aqueous phases were acidified with dilute hydrochloric acid (1 M) to pH = 2 to 3, filtered, and the filter cake was washed with water (10 mL). The resulting solid was concentrated under reduced pressure to obtain compound 242-2 (476 mg, crude). LC-MS: [M+H]⁺= 298.85.
(3) At room temperature, compound 242-2 (1 g, 3.37 mmol) was dissolved in tetrahydrofuran (10 mL), followed by the addition of propylphosphonic anhydride (6.4 g, 10.1 mmol, 50% in ethyl acetate), azidotrimethylsilane (1.16 g, 10.1 mmol), and triethylamine (1.36 g, 13.5 mmol). The reaction mixture was stirred at room temperature for 1 hour. 1-Cyclobutylethanol (0.51 g, 5.05 mmol) was added, and the reaction mixture was heated to 90°C and stirred for an additional 1 hour. After cooling to room temperature, the reaction mixture was directly concentrated under reduced pressure. The residue was purified by silica gel column chromatography (ethyl acetate/petroleum ether = 1/5) to obtain a crude product, which was further purified by high-performance liquid chromatography (0.1% ammonia in water conditions) to obtain compound 242-3 (740 mg). LC-MS: [M+H]⁺ =396.00.
(4) At room temperature, compound 242-3 (700 mg, 1.78 mmol) was subjected to chiral separation (Daicel Chiralpak AY column, 250 × 25 mm I.D., 10 µm; mobile phase A: supercritical carbon dioxide; mobile phase B: EtOH; volume ratio of mobile phase A to B: 75:25; isocratic elution; flow rate: 60 g/min; column temperature: room temperature; injection volume: 2 µL; detection wavelength: 254 nm) to obtain compound 242-3A (retention time Rt = 9.834 min, 300 mg) and compound 242-3B (retention time Rt = 10.785 min, 295 mg). LC-MS: [M+H]⁺ =394.05.
(5) At room temperature, compound 242-3A (250 mg, 0.63 mmol) and compound M013 (237 mg, 1.26 mmol) were dissolved in 1,4-dioxane (8 mL), followed by the addition of potassium carbonate (174 mg, 1.26 mmol), bis(triphenylphosphine)palladium(II) chloride (44 mg, 63 µmol), copper(I) iodide (12 mg, 63 µmol), and triethylamine (319 mg, 3.15 mmol). Under a nitrogen atmosphere, the reaction mixture was heated to 100°C and stirred for an additional 3 hours. After cooling to room temperature, the reaction mixture was diluted with water (10 mL) and extracted with ethyl acetate (10 mL × 2). The combined organic phases were dried over anhydrous sodium sulfate, filtered, and the filtrate was concentrated under reduced pressure. The residue was purified by silica gel column chromatography (ethyl acetate/petroleum ether = 1/4) to obtain compound 242-4A (350 mg, containing a pair of trans isomers). LC-MS: [M+H]⁺ =502.15.
(6) At room temperature, compound 242-4A (350 mg, 0.7 mmol) was dissolved in methanol (3 mL) and water (1 mL), followed by the addition of lithium hydroxide (168 mg, 7 mmol). The reaction mixture was stirred at room temperature for 1 hour. After diluting with water (10 mL), the reaction mixture was concentrated under reduced pressure. The residue was extracted with ethyl acetate (5 mL × 3), and the combined aqueous phases were adjusted to pH = 3 to 4 with dilute hydrochloric acid (1 M), then extracted again with ethyl acetate (5 mL × 3). The combined organic phases were dried over anhydrous sodium sulfate, filtered, and the filtrate was concentrated under reduced pressure. The residue was purified by high-performance liquid chromatography (0.1% ammonia in water conditions) and lyophilized to obtain compound 242-A (150.3 mg, containing a pair of trans isomers). LC-MS: [M+H]⁺ =488.20.
   ¹H NMR (400 MHz, DMSO-*d₆*) δ 7.80 - 7.73 (m, 2H), 4.67 (s, 1H), 3.88 (s, 3H), 3.46 (dd, *J* = 12.0, 8.0 Hz, 3H), 2.96 (dd, *J* = 8.0, 12.0 Hz, 1H), 2.71 - 2.58 (m, 1H), 2.55 (s, 3H), 2.35 - 2.18 (m, 2H), 2.09 - 1.42 (m, 2H), 1.15 (s, 3H).
(7) At room temperature, compound 242-3B (245 mg, 0.62 mmol) and compound M013 (234 mg, 1.24 mmol) were dissolved in 1,4-dioxane (8 mL), followed by the addition of potassium carbonate (172 mg, 1.24 mmol), bis(triphenylphosphine)palladium(II) chloride (43.5 mg, 62 µmol), copper(I) iodide (12 mg, 62 µmol), and triethylamine (314 mg, 3.1 mmol). Under a nitrogen atmosphere, the reaction mixture was heated to 100°C and stirred for an additional 3 hours. The reaction mixture was cooled to room temperature, diluted with water (10 mL), and extracted twice with ethyl acetate (10 mL × 2). The combined organic phases were dried over anhydrous sodium sulfate, filtered, and the filtrate was concentrated under reduced pressure. The residue was purified by silica gel column chromatography (ethyl acetate/petroleum ether = 1/4) to obtain compound 242-4B (252 mg). LC-MS: [M+H]⁺ =502.20.
(8) At room temperature, compound 242-4B (252 mg, 0.5 mmol) was dissolved in methanol (3 mL) and water (1 mL), followed by the addition of lithium hydroxide (120 mg, 5 mmol). The reaction mixture was stirred at room temperature for 1 hour. The reaction mixture was diluted with water (10 mL), concentrated under reduced pressure, and the residue was extracted with ethyl acetate (5 mL × 3). The combined aqueous phases were adjusted to pH = 3 to 4 with dilute hydrochloric acid (1 M) and then extracted with ethyl acetate (5 mL × 3). The combined organic phases were dried over anhydrous sodium sulfate, filtered, and the filtrate was concentrated under reduced pressure. The residue was purified by high-performance liquid chromatography (0.1% ammonia in water conditions) and lyophilized to obtain compound 242-B (135.7 mg). LC-MS: [M+H]⁺ =488.20.

¹H NMR (400 MHz, DMSO-*d₆*) δ 7.75 (dd, *J* = 8.0,12.0 Hz, 2H), 4.65 (s, 1H), 3.87 (s, 3H), 3.45 (dd, *J* = 8.0, 12.0 Hz, 3H), 2.91 (dd, *J =* 12.0,8.0 Hz, 1H), 2.69 - 2.55 (m, 1H), 2.53 (s, 3H), 2.34 - 2.19 (m, 2H), 2.09- 1.32 (m, 1H), 1.04 (s, 3H).

### Example 71: Preparation of trans-2-((6-(5-(((((R)-4-fluoro-4-methylpentan-2-yl)oxy)carbonyl)amino)-1-methyl-1H-1,2,3-triazol-4-yl)-2-methylpyridin-3-yl)ethynyl)cyclopentane-1-carboxylic acid (compound 244), compound 244-A, and compound 244-B

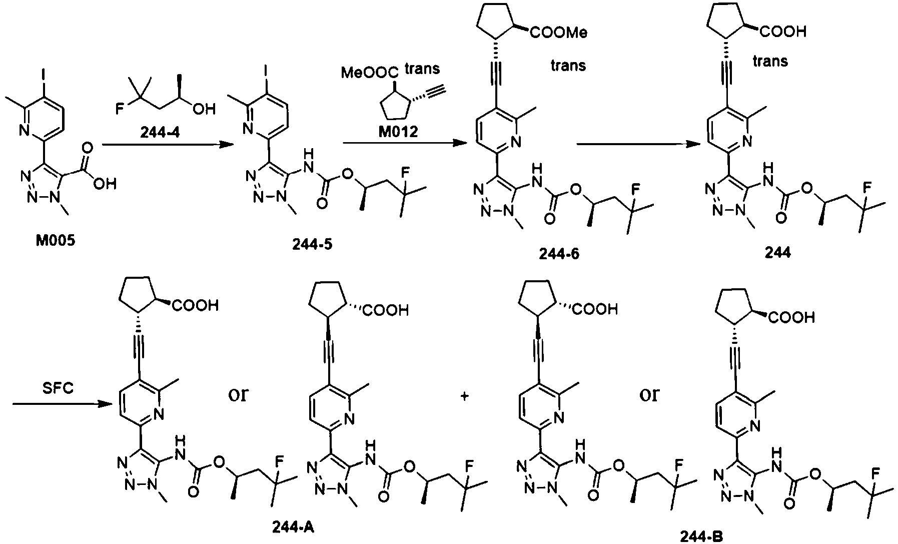

The preparation of compound 244 was referred to the method for preparing compound 212 in Example 50, wherein compound 207-4 was replaced with compound 244-4 and compound 033-5 was replaced with compound M012, and compound 244 (35.3 mg, containing a pair of trans isomers) was obtained. LC-MS: [M+H]⁺ =472.20.

¹H NMR (400 MHz, DMSO-*d₆*) δ 9.46 (s, 1H), 7.77 - 7.71 (m, 2H), 4.99 - 4.83 (s, 1H), 3.85 (s, 3H), 3.18 - 3.09 (m, 1H), 2.75 (dd, *J =* 16.4, 8.0 Hz, 1H), 2.51 (s, 3H), 2.13 - 1.98 (m, 3H), 1.78 - 1.63 (m, 5H), 1.25 (s, 9H).

At room temperature, compound 244 (317.0 mg) was subjected to chiral separation (Daicel Chiral IG column, 250 × 30 mm I.D., 5 µm; mobile phase A: supercritical carbon dioxide; mobile phase B: IPA and NH₃H₂O (NH₃H₂O accounts for 0.1% of the volume of mobile phase B); volume ratio of mobile phase A to B: 80:20; isocratic elution; flow rate: 60 g/min; column temperature: room temperature; injection volume: 3 µL; detection wavelength: 220 nm) to obtain compound 244-A (retention time Rt = 8.840 min, 88.6 mg) and compound 244-B (retention time Rt = 11.336 min, 112.1 mg). LCMS:[M+1]⁺=472.15.

Compound 244-A: ¹H NMR (400 MHz, DMSO-*d₆*) δ 9.46 (s, 1H), 7.97 - 7.58 (m, 2H), 4.93 (s, 1H), 3.87 (s, 3H), 3.18 - 3.12 (m,1H), 2.82 - 2.75 (m, 1H), 2.53 (s, 3H), 2.19 - 1.96 (m, 3H), 1.85 - 1.64 (m, 5H), 1.30 (d, *J* = 16.4 Hz, 9H).

Compound 244-B: ¹H NMR (400 MHz, DMSO-*d₆*) δ 9.46 (s, 1H), 7.97 - 7.55 (m, 2H), 4.93 (s, 1H), 3.87 (s, 3H), 3.18 - 3.12 (m,1H), 2.82 - 2.75 (m, 1H), 2.53 (s, 3H), 2.19 - 1.92 (m, 3H), 1.88 - 1.64 (m, 5H), 1.50 - 1.06 (m, 9H).

The preparation method for (*R*)-4-fluoro-4-methylpentan-2-ol (compound 244-4) is as follows: 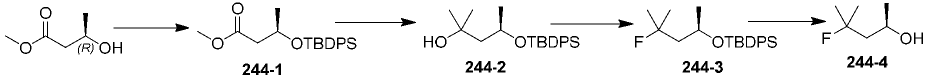
(1) At room temperature, methyl (*R*)-3-hydroxybutyrate (6 g, 50.8 mmol) was dissolved in dichloromethane (90 mL). Under a nitrogen atmosphere, the reaction mixture was cooled to 0°C, followed by the addition of imidazole (6 g, 88.2 mmol) and stirring for 10 minutes. Then, *tert*-butyldiphenylsilyl chloride (15.4 g, 56.0 mmol) was added dropwise. After the dropwise addition was completed, the reaction mixture was warmed to room temperature and stirred for an additional 4 hours. The reaction mixture was quenched with saturated ammonium chloride solution (50 mL), diluted with water (50 mL), and extracted with dichloromethane (50 mL × 3). The combined organic phases were washed with saturated brine (50 mL), dried over anhydrous sodium sulfate, filtered, and the filtrate was concentrated under reduced pressure. The residue was purified by silica gel column chromatography (petroleum ether/ethyl acetate = 20/1) to obtain compound 244-1 (18 g). LC-MS: [M+H]⁺ =357.56.
(2) At room temperature, compound 244-1 (2.5 g, 7.01 mmol) was dissolved in anhydrous tetrahydrofuran (25 mL), and the reaction mixture was cooled to -78°C (dry ice-ethyl acetate bath), followed by the dropwise addition of methylmagnesium bromide (9.35 mL, 28.05 mmol, 3 M in tetrahydrofuran). After the dropwise addition was completed, the reaction mixture was slowly warmed to room temperature and stirred for an additional 1 hour. The reaction mixture was quenched with water (20 mL) and extracted with ethyl acetate (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 20/1) to obtain compound 244-2 (2.3 g). LC-MS: [M+H]⁺ = 357.10.
(3) At room temperature, compound 244-2 (2.0 g, 5.61 mmol) was dissolved in dichloromethane (20 mL), and the reaction mixture was cooled to 0°C. Diethylaminosulfur trifluoride (1.36 g, 8.43 mmol) was added dropwise. After warming to room temperature, the reaction mixture was stirred for an additional 2 hours. The reaction mixture was quenched with aqueous sodium bicarbonate solution (20 mL) and extracted with dichloromethane (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 50/1) to obtain compound 244-3 (2.0 g). LC-MS: [M+H]⁺ = 359.10.
(4) At room temperature, compound 244-3 (2.0 g, 5.58 mmol) was dissolved in anhydrous tetrahydrofuran (20 mL), and tetrabutylammonium fluoride (2.06 g, 6.69 mmol) was added. The reaction mixture was stirred at room temperature for 16 hours. The reaction mixture was quenched with water (30 mL) and extracted with ethyl acetate (30 mL × 3). The organic phases were combined, dried over anhydrous sodium sulfate, filtered, and the filtrate was concentrated under reduced pressure. The residue was purified by silica gel column chromatography (petroleum ether/ethyl acetate = 10/1) to obtain compound 244-4 (400 mg). LC-MS: [M+H]⁺ = 121.17.

### Example 72: Preparation of trans-2-((6-(5-(R)-4,4-difluoropentan-2-yl)oxy)carbonyl)amino)-1-methyl-1H-1,2,3-triazol-4-yl)-2-methylpyridin-3-yl)ethynyl)cyclopentane-1-carboxylic acid (compound 246), compound 246-A, and compound 246-B

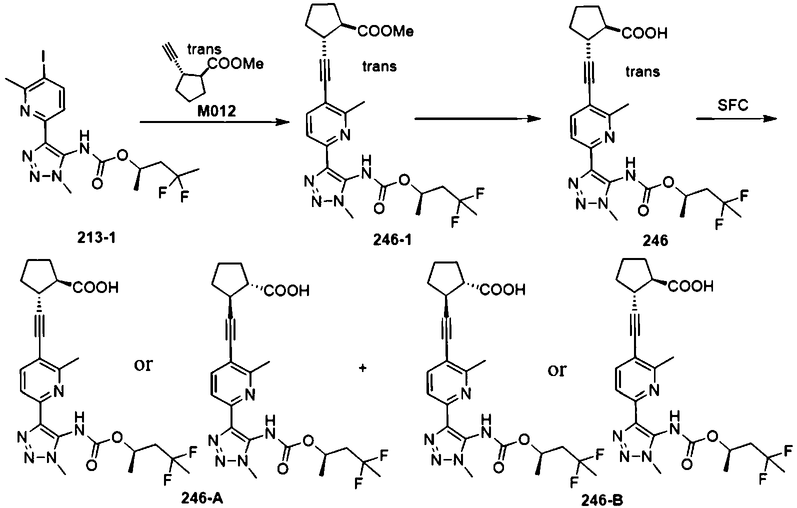

The preparation of compound 246 was referred to the method for preparing compound 212 in Example 50, wherein compound 033-5 was replaced with compound M012 and compound 207-5 was replaced with compound 213-1, and compound 246 (148.9 mg, containing a pair of trans isomers) was obtained. LC-MS: [M+H]⁺=476.15.

¹H NMR (400 MHz, DMSO-*d₆*) δ 7.79 - 7.73 (m, 2H), 4.99 (s, 1H), 3.87 (s, 3H), 3.16 (m, 1H), 2.78 (m, 1H), 2.53 (s, 3H), 2.08 (m, 4H), 1.82 - 1.68 (m, 4H), 1.60 (s, 3H), 1.23 (s, 3H).

Compound 246 (133.5 mg) was subjected to chiral separation (Daicel ChiralpakAD column, 250 × 25 mm I.D., 10 µm; mobile phase A: supercritical carbon dioxide; mobile phase B: EtOH and NH₃H₂O (NH₃H₂O accounts for 0.1 % of the volume of mobile phase B); volume ratio of mobile phase A to B: 80:20; isocratic elution; injection volume: 3 µL; detection wavelength: 220 nm; flow rate: 60 g/min; column temperature: room temperature) to obtain compound 246-A (retention time Rt = 10.408 min, 28.9 mg) and compound 246-B (retention time Rt = 14.464 min, 37.7 mg). LC-MS: [M+H]⁺ =476.15.

Compound 246-A: ¹H NMR (400 MHz, DMSO-*d₆*) δ 9.53 (s, 1H), 7.88 - 7.68 (m, 2H), 4.98 (s, 1H), 3.87 (s, 3H), 3.15 (m, 1H), 2.79 (m, 1H), 2.53 (s, 3H), 2.34 - 1.93 (m, 4H), 1.82 - 1.67 (m, 4H), 1.60 (s, 3H), 1.23 (s, 3H).

Compound 246-B: ¹H NMR (400 MHz, DMSO-*d₆*) δ 9.53 (s, 1H), 7.97 - 7.65 (m, 2H), 4.98 (s, 1H), 3.87 (s, 3H), 3.15 (m, 1H), 2.79 (m, 1H), 2.53 (s, 3H), 2.33 - 1.93 (m, 4H), 1.74 (m, 4H), 1.60 (s, 3H), 1.23 (s, 3H).

### Example 73: Preparation of (R)-2-(1-((6-(5-((((3,3-difluoropentan-2-yl)oxy)carbonyl)amino)-1-methyl-1H-1,2,3-triazol-4-yl)-2-methylpyridin-3-yl)ethynyl)-3-methoxycyclobutylacetic acid (compound 279)

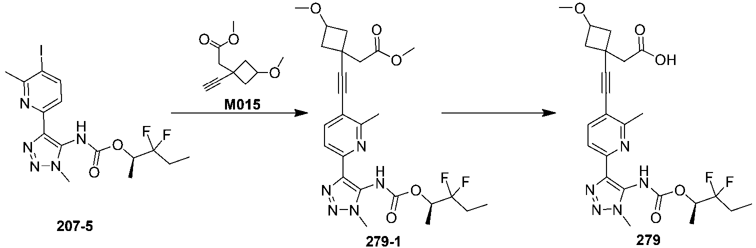

(1) At room temperature, compound 207-5 (150 mg, 0.32 mmol) and compound M015 (88 mg, 0.48 mmol) were dissolved in anhydrous 1,4-dioxane (3 mL), followed by the addition of potassium carbonate (89 mg, 0.64 mmol), bis(triphenylphosphine)palladium(II) chloride (23 mg, 32 µmol), copper(I) iodide (6 mg, 32 µmol), and triethylamine (162 mg, 1.6 mmol). Under a nitrogen atmosphere, the reaction mixture was heated to 80°C and stirred for an additional 1 hour. The reaction mixture was cooled to room temperature, diluted with water (10 mL), and extracted with ethyl acetate (10 mL × 2). The combined organic phases were washed with saturated brine (10 mL), dried over anhydrous sodium sulfate, filtered, and the filtrate was concentrated under reduced pressure. The residue was purified by silica gel column chromatography (ethyl acetate/petroleum ether = 3/1) to obtain compound 279-1 (160 mg, containing cis-trans isomers). LC-MS: [M+H]⁺ =520.20.
(2) At room temperature, compound 279-1 (160 mg, 0.31 mmol) was dissolved in methanol (3 mL) and water (1 mL), followed by the addition of lithium hydroxide (74 mg, 3.1 mmol). The reaction mixture was stirred at room temperature for 4 hours. The reaction mixture was diluted with water (10 mL) and concentrated under reduced pressure to remove methanol. The residue was extracted with ethyl acetate (10 mL × 3). The combined aqueous phases were adjusted to pH = 3 to 4 with dilute hydrochloric acid (1 M) and then extracted with ethyl acetate (10 mL × 3). The combined organic phases were dried over anhydrous sodium sulfate, filtered, and the filtrate was concentrated under reduced pressure. The residue was purified by high-performance liquid chromatography (0.1% hydrochloric acid conditions) and lyophilized to obtain the hydrochloride of compound 279 (45.0 mg, containing cis-trans isomers). LC-MS: [M+H]⁺ =506.20.

¹H NMR (400 MHz, DMSO-*d₆*) δ 9.92 (s, 1H), 7.81 - 7.71 (m, 2H), 4.99 (s, 1H), 4.14 - 4.05 (m, 1H), 3.99 (dt, *J* = 8.0, 16.0 Hz, 1H), 3.90 (s, 3H), 3.15 (d, *J* = 8.0 Hz, 3H), 2.70 (d, *J* = 4.0Hz, 2H), 2.67 - 2.60 (m, 2H), 2.53 (d, *J* = 4.0 Hz, 3H), 2.31 - 2.21 (m, 1H), 2.18 - 2.09 (m, 1H), 1.97 (s, 1H), 1.28 (s, 3H), 0.96 (s, 3H).

### Example 74: Preparation of 2-(1-((6-(5-((((5-fluoropentan-2-yl)oxy(carbonyl)amino)-1-methyl-1H-1,2,3-triazol-4-yl)-2-methylpyridin-3-yl)ethynyl)cyclopropyl)acetic acid (compound 280)

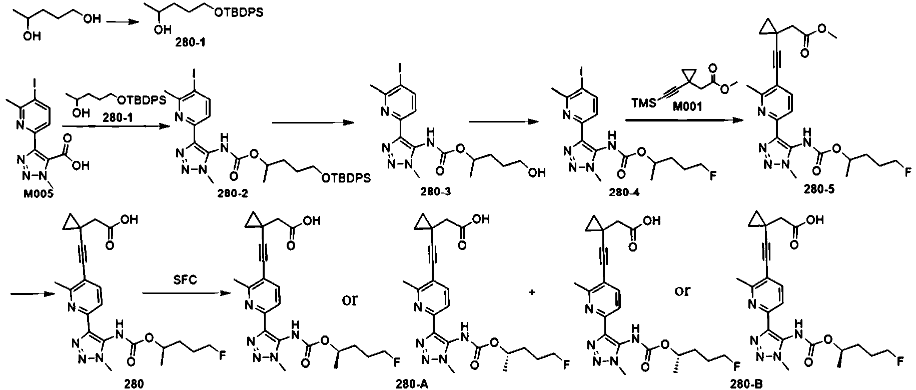

(1) At room temperature, 1,4-pentanediol (2.1 g, 20.2 mmol) was dissolved in *N,N-*dimethylformamide (30 mL), followed by the addition of tert-butyldiphenylsilyl chloride (6.1 g, 22.2 mmol) and imidazole (1.5 g, 22.2 mmol). The reaction mixture was stirred at room temperature for 2 hours. The reaction mixture was diluted with water (100 mL) and extracted with ethyl acetate (50 mL × 3). The combined organic phases were washed with saturated brine (100 mL), dried over anhydrous sodium sulfate, filtered, and the filtrate was concentrated under reduced pressure. The residue was purified by silica gel column chromatography (petroleum ether/ethyl acetate = 5/1) to obtain compound 280-1 (5.9 g). LC-MS: [M+H]⁺ = 343.15.
(2) At room temperature, compound M005 (700 mg, 2.02 mmol) was dissolved in tetrahydrofuran (12 mL), followed by the addition of triethylamine (615 mg, 6.09 mmol), propylphosphonic anhydride (2.6 g, 4.06 mmol, 50% in ethyl acetate), and azidotrimethylsilane (700 mg, 6.09 mmol). Under a nitrogen atmosphere, the reaction mixture was stirred at room temperature for 30 minutes, then heated to 60°C, and stirred for 1 hour. The reaction mixture was cooled to room temperature, and 280-1 (2.1 g, 6.10 mmol) was added. The reaction mixture was heated to 70°C and stirred for an additional 1 hour. The reaction mixture was cooled to room temperature, diluted with water (20 mL), and extracted with ethyl acetate (10 mL × 2). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 5/1) to obtain compound 280-2 (2.0 g, crude). LC-MS: [M+H]⁺= 684.95.
(3) At room temperature, 280-2 (2.12 g, 3.1 mmol) was dissolved in tetrahydrofuran (20 mL), and tetrabutylammonium iodide (2.2 g, 15.7 mmol) was added in portions. The reaction mixture was stirred at room temperature for 1 hour. The reaction mixture was diluted with water (40 mL) and extracted with ethyl acetate (20 mL × 3). The combined organic phases were washed with saturated brine (40 mL), dried over anhydrous sodium sulfate, filtered, and the filtrate was concentrated under reduced pressure. The residue was purified by silica gel column chromatography (petroleum ether/ethyl acetate = 1/1) to obtain compound 280-3 (570 mg). LC-MS: [M+H]⁺ =446.90.
(4) At room temperature, 280-3 (215 mg, 0.48 mmol) was dissolved in dichloromethane (4 mL), and under a nitrogen atmosphere, the reaction mixture was cooled to -70°C, followed by the dropwise addition of diethylaminosulfur trifluoride (92.7 mg, 0.58 mmol). After slowly warming to room temperature, the reaction mixture was stirred for an additional 0.5 hours. The reaction mixture was cooled to 0°C, and saturated aqueous sodium bicarbonate solution (5 mL) was added to quench the reaction. The mixture was extracted with dichloromethane (2 mL × 3). The combined organic phases were washed with saturated brine (5 mL), dried over anhydrous sodium sulfate, filtered, and the filtrate was concentrated under reduced pressure. The residue was purified by silica gel column chromatography (petroleum ether/ethyl acetate = 2/1) to obtain compound 280-4 (110 mg). LC-MS: [M+H]⁺ =446.95.
(5) At room temperature, 280-4 (125 mg, 0.28 mmol) and compound M001 (87 mg, 0.42 mmol) were dissolved in 1,4-dioxane (3 mL), followed by the addition of bis(triphenylphosphine)palladium(II) chloride (20 mg, 0.028 mmol), copper(I) iodide (3 mg, 0.028 mmol), triethylamine (85 mg, 0.84 mmol), potassium carbonate (78 mg, 0.56 mmol), and cesium fluoride (170 mg, 1.12 mmol). Under a nitrogen atmosphere, the reaction mixture was heated to 90°C and stirred for 3 hours. After cooling to room temperature, the reaction mixture was diluted with water (5 mL) and extracted with ethyl acetate (5 mL × 3). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 2/1) to obtain 280-5 (50 mg). LC-MS: [M+H]⁺= 458.12.
(6) At room temperature, 280-5 (100 mg, 0.22 mmol) was dissolved in tetrahydrofuran (1 mL) and methanol (1 mL), followed by the addition of a solution of lithium hydroxide monohydrate (46 mg, 1.09 mmol) in water (0.5 mL). The reaction mixture was stirred at room temperature for 2 hours. The reaction mixture was diluted with water (5 mL), and methanol and tetrahydrofuran were removed by concentration under reduced pressure. The residue was extracted with ethyl acetate (5 mL × 2). The aqueous phase was adjusted to pH = 5 with dilute hydrochloric acid (1 M), then extracted with ethyl acetate (5 mL × 3). The combined organic phases were washed with saturated brine (10 mL), dried over anhydrous sodium sulfate, filtered, and the filtrate was concentrated under reduced pressure. The residue was purified by high-performance liquid chromatography (0.1% ammonia in water conditions) and lyophilized to obtain compound 280 (28.1 mg). LC-MS: [M+H]⁺ = 444.05.
(7) Compound 280 (128 mg) was subjected to chiral separation (Daicel Chiralpak IK column, 250 × 25 mm I.D., 10 µm; mobile phase A: supercritical carbon dioxide; mobile phase B: isopropanol and NH₃H₂O (NH₃H₂O accounts for 0.1% of the volume of mobile phase B); volume ratio of mobile phase A to B: 30:70; isocratic elution; flow rate: 80 g/min, column temperature: room temperature; injection volume: 6 µL; detection wavelength: 220 nm) to obtain compound 280-A (retention time Rt = 3.582 min, 2.6 mg) and compound 280-B (retention time Rt = 3.769 min, 1.0 mg). LC-MS: [M+H]⁺= 444.05.

Compound 280-A: ¹H NMR (400 MHz, DMSO-*d₆*) δ 9.44 (s, 1H), 7.74 (d, *J* = 8.0 Hz, 1H), 7.70 (d, *J* = 8.0 Hz, 1H), 4.74 (s, 1H), 4.40 (d, *J* = 45.2 Hz, 2H), 3.88 (s, 3H), 2.51 (s, 3H), 2.45 (s, 2H), 1.60 (s, 4H), 1.20 (d, *J* = 24.0 Hz, 3H), 1.05 (m, 2H), 0.95 (m, 2H).

Compound 280-B: ¹H NMR (400 MHz, DMSO-*d₆*) δ 9.44 (s, 1H), 7.72 (d, *J* = 8.0 Hz, 1H), 7.67 (d, *J* = 8.0 Hz, 1H), 4.72 (s, 2H), 4.38 (d, *J* = 48.0 Hz, 3H), 3.86 (s, 3H), 2.49 (s, 2H), 2.42 (s, 4H), 1.57 (s, 4H), 1.19 - 1.06 (m, 3H), 1.02 (t, *J* = 5.2 Hz, 2H), 0.93 (m, 2H).

### Example 75: Preparation of 2-(1-((6-(5-((((2-cyclopropylethyl)(methyl)carbamoyl)oxy)methyl)-1-methyl-1H-1,2,3-triazol-4-yl)-2-methylpyridin-3-yl)ethynyl)cyclopropyl)acetic acid (compound 281)

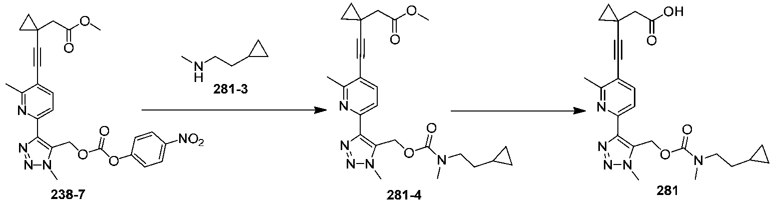

The preparation of compound 281 was referred to the method for preparing compound 238 in Example 67, wherein the hydrochloride of intermediate compound 238-4 was replaced with the hydrochloride of intermediate compound 281-3, and compound 281 (78.4 mg) was obtained. LC-MS [M+H]⁺ =452.05.

¹H NMR (400 MHz, DMSO-*d₆*) δ 7.83 (d, *J =* 8.0 Hz, 1H), 7.72 (d, *J =* 8.0 Hz, 1H), 5.63 (d, *J =* 13.6 Hz, 2H), 4.11 (s, 3H), 3.14 (m, 2H), 2.77 (d, *J =* 19.6 Hz, 3H), 2.53 (s, 3H), 2.44 (s, 2H), 1.34 (d, *J =* 6.4 Hz, 1H), 1.15 (d, *J* = 7.2 Hz, 1H), 1.05 (m, 2H), 0.95 (m, 2H), 0.47 (m, 2H), 0.19 (d, *J =* 7.2 Hz 1H), - 0.01 (s, 1H), -0.24 (s, 1H).

### Example 76: Preparation of trans-2-((6-(5-((((3,3-difluorobutyl)(methyl)carbamoyl)oxy)methyl)-1-methyl-1H-1,2,3-triazol-4-yl)-2-methylpyridin-3-yl)ethynyl)cyclopentane-1-carboxylic acid (compound 282), compound 282-A, and compound 282-B

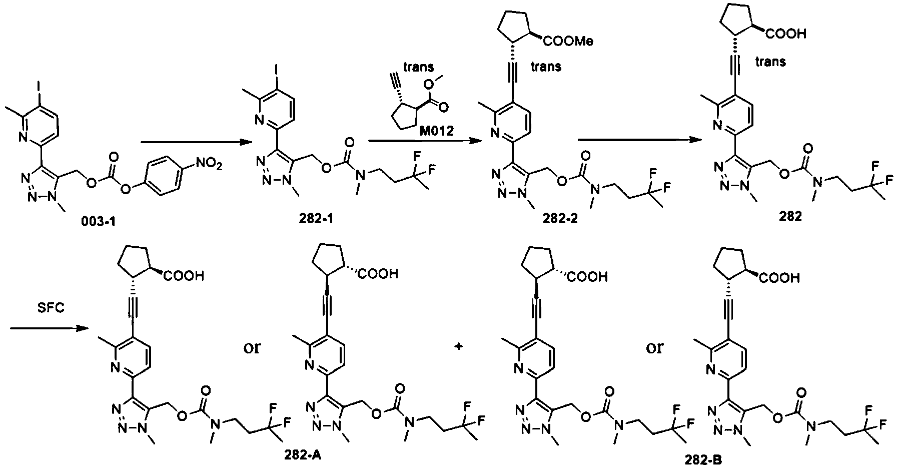

(1) At room temperature, compound 003-1 (400 mg, 0.81 mmol) was dissolved in dichloromethane (10 mL), followed by the addition of a mixture of 037-3 acid salt and 037-4 hydrochloride (199 mg, 1.62 mmol) and *N,N*-diisopropylethylamine (313 mg, 2.42 mmol). The reaction mixture was stirred at room temperature for 1 hour. The reaction mixture was poured into water (20 mL) and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 282-1 (320 mg). LC-MS: [M+H]⁺=480.00.
(2) At room temperature, compound 282-1 (220 mg, 0.46 mmol) and compound M012 (280 mg, 0.92 mmol, containing a pair of trans isomers) were dissolved in 1,4-dioxane (4 mL), followed by the addition of bis(triphenylphosphine)palladium(II) chloride (32 mg, 0.046 mmol), copper(I) iodide (18 mg, 0.092 mmol), and triethylamine (140 mg, 1.38 mmol). Under a nitrogen atmosphere, the reaction mixture was heated to 90°C and stirred for 6 hours. The reaction mixture was cooled to room temperature, diluted with water (10 mL), and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 282-2 (200 mg, containing a pair of trans isomers). LC-MS: [M+H]⁺ =504.25.
(3) At room temperature, 282-2 (200 mg, 0.35 mmol) was dissolved in methanol (3 mL) and water (1 mL), followed by the addition of lithium hydroxide monohydrate (74 mg, 1.75 mmol). The reaction mixture was stirred at room temperature for 2 hours. After neutralizing the reaction mixture with dilute hydrochloric acid (1 M) (pH = 7), the mixture was extracted with ethyl acetate (3 mL × 3). The combined organic phases were dried over anhydrous sodium sulfate, filtered, and the filtrate was concentrated under reduced pressure. The residue was purified by preparative high-performance liquid chromatography (0.1% ammonia in water) and lyophilized to obtain compound 282 (76.3 mg, containing a pair of trans isomers). LC-MS: [M+H]⁺ =490.20.
   ¹H NMR (400 MHz, DMSO-*d₆*) δ 7.86 (d, *J* = 8.0 Hz, 1H), 7.79 (d, *J* = 8.0 Hz, 1H), 5.66 (d, *J* = 13.4 Hz, 2H), 4.11 (s, 3H), 3.41 - 3.21 (m, 2H), 3.16 (dd, *J* = 15.6, 7.8 Hz, 1H), 2.83 - 2.72 (m, 4H), 2.55 (s, 3H), 2.16 - 1.87 (m, 4H), 1.80 - 1.67 (m, 4H), 1.63 - 1.35 (m, 3H).
(4) At room temperature, compound 282 (56.3 mg, 0.12 mmol) was subjected to chiral separation (Daicel Chiralpak IG column, 250 × 30 mm I.D., 5 µm; flow rate: 60 g/min; column temperature: room temperature; mobile phase A: supercritical carbon dioxide; mobile phase B: IPA and NH₃H₂O (NH₃H₂O accounts for 0.1% of the volume of mobile phase B); volume ratio of mobile phase A to B: 80:20; isocratic elution; injection volume: 3 µL; detection wavelength: 220 nm) to obtain compound 282-A (retention time Rt = 16.742 min, 8.2 mg) and compound 282-B (retention time Rt = 23.102 min, 4.8 mg). LCMS: [M+H]⁺ = 490.20.

### Example 77: Preparation of 2-(1-((6-(5-((((5,5-difluoropentan-2-yl)oxy)carbonyl)amino)-1-methyl-1H-1,2,3-triazol-4-yl)-2-methylpyridin-3-yl)ethynyl)cyclopropyl)acetic acid (compound 283)

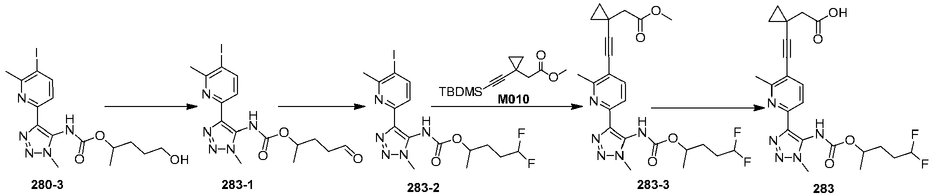

(1) At room temperature, compound 280-3 (518 mg, 1.16 mmol) was dissolved in dichloromethane (8 mL), and sodium bicarbonate (487 g, 5.80 mmol) was added. The reaction mixture was cooled to 0°C, and 1,1,1-triacetoxy-1,1-dihydro-1,2-benziodoxol-3(1*H*)-one (542 g, 1.27 mmol) was added. The reaction mixture was stirred at room temperature for 3 hours. The reaction mixture was diluted with water (20 mL) and washed with dichloromethane (10 mL × 3). The combined organic phases were washed with saturated brine (20 mL), dried over anhydrous sodium sulfate, filtered, and the filtrate was concentrated under reduced pressure. The residue was purified by silica gel column chromatography (petroleum ether/ethyl acetate = 2/1) to obtain compound 283-1 (280 mg). LC-MS: [M+H]⁺ =444.90.
(2) At room temperature, 283-1 (316 mg, 0.71 mmol) was dissolved in dichloromethane. Under a nitrogen atmosphere, the reaction mixture was cooled to -78°C (dry ice-ethyl acetate bath), and diethylaminosulfur trifluoride (229 mg, 1.42 mmol) was slowly added dropwise. The reaction mixture was stirred at this temperature for 0.5 hours. After slowly warming to room temperature, the reaction was stirred for an additional 1 hour. Saturated aqueous sodium carbonate solution (10 mL) was added to the reaction mixture. The mixture was extracted with dichloromethane (5 mL × 3). The organic phase was washed with saturated brine (10 mL), dried over anhydrous sodium sulfate, filtered, and the filtrate was concentrated under reduced pressure. The residue was purified by silica gel column chromatography (petroleum ether/ethyl acetate = 3/1) to obtain compound 283-2 (230 mg). LC-MS: [M+H]⁺ =464.95.
(3) At room temperature, 283-2 (190 mg, 0.408 mmol) and compound M010 (230 mg, 0.611 mmol) were dissolved in 1,4-dioxane (4 mL), followed by the addition of bis(triphenylphosphine)palladium(II) chloride (58 mg, 0.0816 mmol), copper(I) iodide (16 mg, 0.0816 mmol), cesium fluoride (258 mg, 1.632 mmol), potassium carbonate (113 mg, 0.816 mmol), and triethylamine (124 mg, 1.22 mmol). Under a nitrogen atmosphere, the reaction mixture was heated to 90°C and stirred for an additional 2 hours. The reaction mixture was cooled to room temperature, diluted with water (15 mL), and extracted with ethyl acetate (10 mL × 3). The combined organic phases were washed with water (10 mL) and saturated brine (10 mL), dried over anhydrous sodium sulfate, filtered, and the filtrate was concentrated under reduced pressure. The residue was purified by silica gel column chromatography (petroleum ether/ethyl acetate = 2/1) to obtain compound 283-3 (135 mg). LC-MS: [M+H]⁺= 476.05.
(4) At room temperature, 283-3 (135 mg, 0.28 mmol) was dissolved in tetrahydrofuran (1.5 mL) and methanol (1.5 mL), followed by the addition of a solution of lithium hydroxide monohydrate (60 mg, 1.42 mmol) in water (0.5 mL). The reaction mixture was stirred at room temperature for 2 hours. The reaction mixture was diluted with water (5 mL), and methanol and tetrahydrofuran were removed by concentration under reduced pressure. Ethyl acetate (5 mL) and water (5 mL) were added to the residue. The aqueous phase was adjusted to pH = 5 with dilute hydrochloric acid (1 M), then extracted with ethyl acetate (5 mL × 3). The combined organic phases were washed with saturated brine (5 mL), dried over anhydrous sodium sulfate, filtered, and the filtrate was concentrated under reduced pressure. The residue was purified by high-performance liquid chromatography (0.1% hydrochloric acid conditions) and lyophilized to obtain compound 283 (17.8 mg). LC-MS: [M+H]⁺= 462.05.

¹H NMR (400 MHz, DMSO-*d₆*) δ 7.72 (d, *J* = 8.0 Hz, 1H), 7.64 (d, *J* = 8.0 Hz, 1H), 6.05 (t, *J* = 57.2 Hz, 1H), 4.72 (m, 1H), 3.84 (s, 3H), 2.49 (d, *J* = 1.6 Hz, 3H), 2.18 (s, 2H), 1.79 (s, 2H), 1.58 (s, 2H), 1.15 (s, 3H), 0.94 (t, *J* = 5.2 Hz, 2H), 0.90 - 0.85 (m, 2H).

### Example 78: Preparation of 2-(1-((6-(5-((((6-fluorohexan-2-yl)oxy)carbonyl)amino)-1-methyl-1H-1,2,3-triazol-4-yl)-2-methylpyridin-3-yl)ethynyl)cyclopropyl)acetic acid (compound 284)

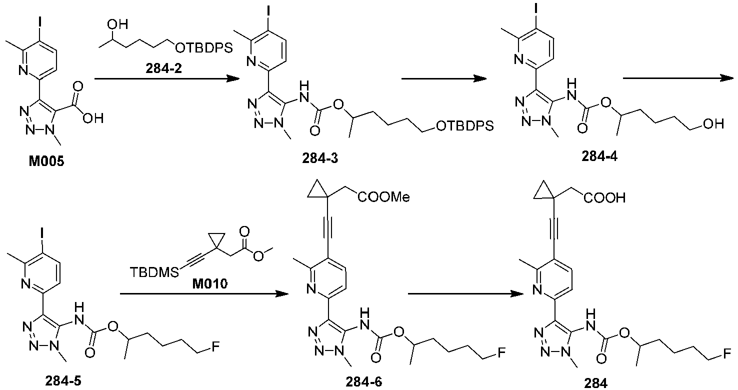

The preparation of compound 284 was referred to the method for preparing compound 280 in Example 74, wherein compound 280-1 was replaced with compound 284-2 and compound M001 was replaced with compound M010, and compound 284 (13.6 mg) was obtained. LC-MS: [M+H]⁺ =458.20.

¹H NMR (400 MHz, DMSO-*d₆*) δ 7.74 - 7.68 (m, 2H), 4.70 (s, 1H), 4.43 - 4.31 (m, 2H), 3.88 (s, 3H), 2.51 (s, 3H), 2.43 (s, 2H), 1.75 - 1.17 (m, 9H), 1.05 - 0.92 (m, 4H).

The preparation method for compound 284-2 is as follows: 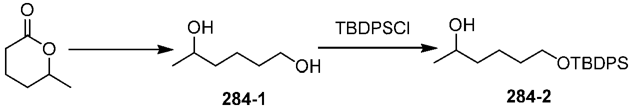
(1) At room temperature, delta-hexalactone (5.0 g, 43.86 mmol) was dissolved in anhydrous tetrahydrofuran (50 mL). After cooling the reaction mixture to 0°C, lithium aluminum hydride (3.3 g, 87.72 mmol) was added in portions. After the addition was completed, the reaction mixture was warmed to room temperature and stirred for an additional 4 hours. The reaction mixture was cooled to 0°C, quenched with ice water (10 mL), followed by the addition of tetrahydrofuran (100 mL) and anhydrous sodium sulfate (10 g). The mixture was stirred at room temperature for 10 minutes, filtered through diatomite, and the filtrate was dried over anhydrous sodium sulfate, filtered, and the filtrate was concentrated under reduced pressure. The residue was purified by silica gel column chromatography (dichloromethane/methanol = 10/1) to obtain compound 284-1 (5.0 g). LC-MS: [M+H]⁺ = 119.17.
(2) At room temperature, compound 284-1 (2.0 g, 16.95 mmol) was dissolved in *N,N-*dimethylformamide (20 mL), followed by the addition of imidazole (1.7 g, 25.42 mmol) and *tert-*butyldiphenylsilyl chloride (4.6 g, 16.95 mmol). The reaction mixture was stirred at room temperature for 3 hours. The reaction mixture was diluted with water (20 mL) and extracted with ethyl acetate (40 mL × 3). The combined organic phases were washed with saturated sodium chloride (50 mL), dried over anhydrous sodium sulfate, filtered, and the filtrate was concentrated under reduced pressure. The residue was purified by silica gel column chromatography (petroleum ether/ethyl acetate = 10/1) to obtain compound 284-2 (4.6 g). LC-MS: [M+H]⁺= 357.20.

### Example 79: Preparation of 2-(1-((6-(5-(((1-(3-fluorocyclobutyl)ethoxy)carbonyl)amino)-1-methyl-1H-1,2,3-triazol-4-yl)-2-methylpyridin-3-yl)ethynyl)cyclopropyl)acetic acid (compound 285), compound 285-A, and compound 285-B

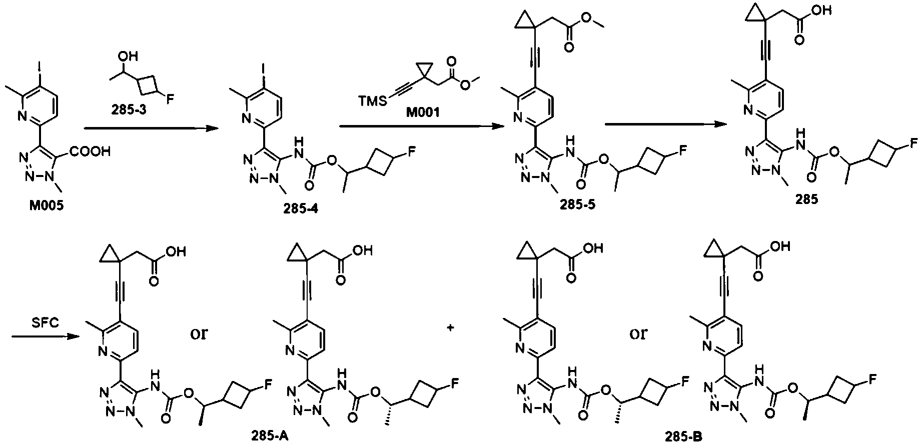

The preparation of compound 285 was referred to the preparation of compound 008 in Example 8, wherein the starting material 1-cyclopropylethanol was replaced with compound 285-3, and compound 285 (47.9 mg) was obtained. LC-MS: [M+H]⁺ =468.20.

¹H NMR (400 MHz, DMSO-*d₆*) δ 7.75 - 7.69 (m, 2H), 5.00 (s, 1H), 3.88 (s, 3H), 2.52 (s, 3H), 2.43 (s, 2H), 2.23 - 1.53 (m, 6H), 1.29 - 0.90 (m, 7H).

Compound 285 (40 mg, 0.085 mmol) was subjected to chiral separation (Daicel Chiralpak IK column, 250 × 25 mm I.D., 10 µm; mobile phase A: supercritical carbon dioxide; mobile phase B: IPA and NH₃H₂O (NH₃H₂O accounts for 0.1% of the volume of mobile phase B); volume ratio of mobile phase A to B: 60:40; isocratic elution; flow rate: 70 g/min; column temperature: room temperature; injection volume: 2 µL; detection wavelength: 220 nm) to obtain compound 285-A (retention time Rt = 9.705 min, 6.1 mg) and compound 285-B (retention time Rt = 11.640 min, 6.3 mg).

LC-MS: [M+H]⁺ =468.20.

Compound 285-A: ¹H NMR (400 MHz, DMSO-*d₆*) δ 7.75 - 7.69 (m, 2H), 5.00 (s, 1H), 3.88 (s, 3H), 2.52 (s, 3H), 2.44 (s, 2H), 2.22 - 1.69 (m, 6H), 1.23 (s, 3H), 1.06 - 1.03 (m, 2H), 0.96 - 0.93 (m, 2H).

Compound 285-B: ¹H NMR (400 MHz, DMSO-*d₆*) δ 7.75 - 7.69 (m, 2H), 5.00 (s, 1H), 3.88 (s, 3H), 2.52 (s, 3H), 2.44 (s, 2H), 1.99 (s, 6H), 2.07 - 1.82 (m, 3H), 1.06 - 1.01 (m, 2H), 0.95 - 0.93 (m, 2H).

### Example 80: Preparation of (R)-2-(1-((6-(5-chloro-3-((((3,3-difluoropentan-2-yl)oxy)carbonyl)amino)thiophen-2-yl)-2-methylpyridin-3-yl)ethynyl)cyclopropyl)acetic acid (compound 286)

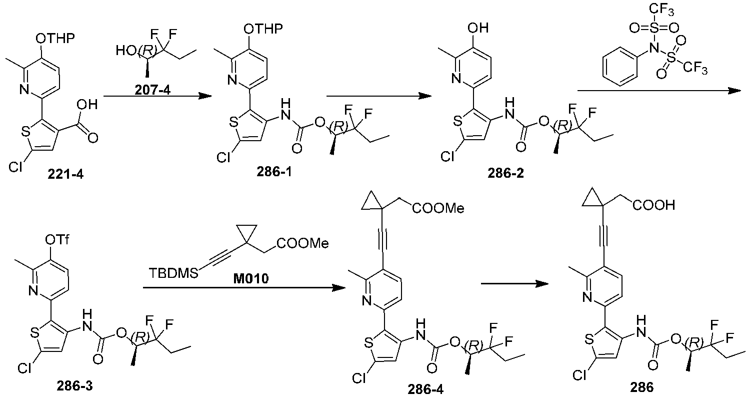

The preparation of compound 286 was referred to the method for preparing compound 221 in Example 59, wherein compound M011 was replaced with compound 207-4 and compound 033-5 was replaced with compound M010, and compound 286 (19.0 mg) was obtained. LC-MS: [M+H]⁺=497.10.

¹H NMR (400 MHz, DMSO-*d₆*) δ 11.36 (s, 1H), 7.66 (d, J = 8.0 Hz, 1H), 7.52 (s, 1H), 7.27 (d, J = 8.4 Hz, 1H), 5.07 - 4.97 m, 1H), 2.50 (s, 3H), 2.42 (s, 2H), 1.99 - 1.87 (m, 2H), 1.29 (d, J = 6.4 Hz, 3H), 1.04 - 1.02 (m, 2H), 0.97 - 0.90 (m, 5H).

### Example 81: Preparation of (R)-2-((6-(5-((((3,3-difluoropentan-2-yl)oxy)carbonyl)amino)-1-methyl-1H-1,2,3-triazol-4-yl)-2-methylpyridin-3-yl)ethynyl)nicotinic acid (compound 472)

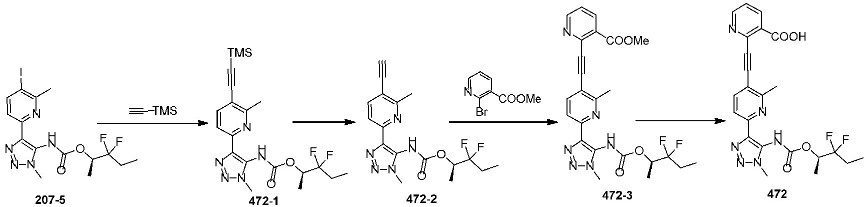

(1) At room temperature, compound 207-5 (400 mg, 0.86 mmol) was dissolved in 1,4-dioxane (8 mL), followed by the addition of trimethylsilylacetylene (169 mg, 1.72 mmol), bis(triphenylphosphine)palladium(II) chloride (121 mg, 0.17 mmol), copper(I) iodide (33 mg, 0.17 mmol), and triethylamine (261 mg, 2.58 mmol). The reaction mixture was heated to 80°C under a nitrogen atmosphere and stirred for 2 hours. The reaction mixture was cooled to room temperature, then poured into water (20 mL) to dilute, and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 472-1 (300 mg). LC-MS: [M+H]⁺ =436.10.
(2) At room temperature, compound 472-1 (320 mg, 0.73 mmol) was dissolved in anhydrous tetrahydrofuran (3 mL), followed by the addition of a solution of tetrabutylammonium fluoride in tetrahydrofuran (0.7 mL, 0.73 mmol, 1 M in tetrahydrofuran). The reaction mixture was stirred at room temperature for 0.5 hours. The reaction mixture was poured into water (20 mL) to dilute and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 472-2 (190 mg). LC-MS: [M+H]⁺= 364.10.
(3) At room temperature, compound 472-2 (140 mg, 0.39 mmol) and methyl 2-bromonicotinate (169 mg, 0.78 mmol) were dissolved in 1,4-dioxane (2 mL), followed by the addition of bis(triphenylphosphine)palladium(II) chloride (55 mg, 0.08 mmol), copper(I) iodide (15 mg, 0.08 mmol), and triethylamine (237 mg, 2.34 mmol). The reaction mixture was heated to 80°C under a nitrogen atmosphere and stirred for 2 hours. After cooling to room temperature, the reaction mixture was poured into water (20 mL) and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 472-3 (122 mg). LCMS: [M+H]⁺ =499.20.
(4) At room temperature, compound 472-3 (122 mg, 0.24 mmol) was dissolved in methanol (2 mL) and water (1 mL), followed by the addition of lithium hydroxide monohydrate (30 mg, 0.72 mmol). The reaction mixture was stirred at room temperature for 1 hour. The reaction mixture was diluted with water (10 mL) and concentrated under reduced pressure. The residue was extracted with dichloromethane (10 mL × 3). The aqueous phase was adjusted to pH = 3 to 4 with dilute hydrochloric acid (1 M) and extracted with ethyl acetate (10 mL × 3). The combined organic phases were dried over anhydrous sodium sulfate, filtered, and the filtrate was concentrated under reduced pressure. The residue was purified by high-performance liquid chromatography (0.1% ammonia in water system) and lyophilized to obtain compound 472 (65.4 mg). LCMS: [M+H]⁺ =485.15.

¹H NMR (400 MHz, DMSO-*d₆*) δ 8.36 (dd, J = 4.8, 2.0 Hz, 1H), 7.82 - 7.80 (m, 2H), 7.66 (d, J = 8.4 Hz, 1H), 7.21 (dd, J = 7.6, 4.8 Hz, 1H), 4.98 - 4.81 (m, 1H), 3.60 (s, 3H), 2.63 (s, 3H), 1.96 - 1.76 (m, 2H), 1.15 (d, J = 6.4 Hz, 3H), 0.90 (t, J = 7.6 Hz, 3H).

### Example 82: Preparation of (R)-3-((6-(5-((((3,3-difluoropentan-2-yl)oxy)carbonyl)amino)-1-methyl-1H-1,2,3-triazol-4-yl)-2-methylpyridin-3-yl)ethynyl)picolinic acid (compound 473)

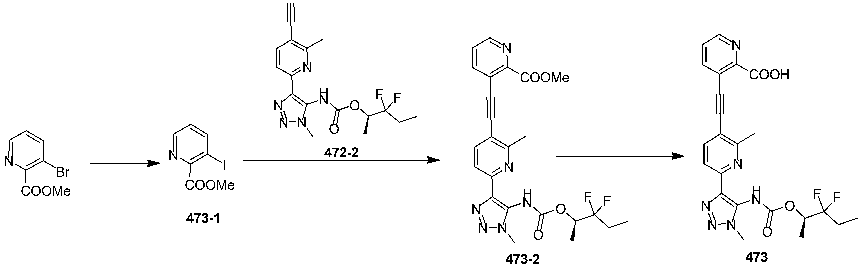

The preparation of compound 473 was referred to the preparation of compound 472 in Example 81, wherein the starting material methyl 2-bromonicotinate was replaced with compound 473-1, and compound 473 (12.5 mg) was obtained. LCMS: [M+H]⁺ =485.20.

¹H NMR (400 MHz, DMSO-*d₆*) δ 9.98 (s, 1H), 8.63 (d, J = 3.6 Hz, 1H), 8.15 (dd, J = 8.0, 1.6 Hz, 1H), 7.95 (d, J = 8.4 Hz, 1H), 7.86 (d, J = 8.0 Hz, 1H), 7.63 (dd, J = 8.0, 4.8 Hz, 1H), 4.99 (s, 1H), 3.90 (s, 3H), 2.66 (s, 3H), 2.18 - 0.55 (m, 8H).

The preparation method for compound 473-1 is as follows:
At room temperature, methyl 3-bromopicolinate (1.0 g, 4.63 mmol) was dissolved in 1,4-dioxane (20 mL), followed by the addition of sodium iodide (3.5 g, 23.15 mmol), *N,N*'-dimethylethylenediamine (240 mg, 2.78 mmol), and copper(I) iodide (440 mg, 2.31 mmol). The reaction mixture was heated to 110°C and stirred for an additional 18 hours. The reaction mixture was cooled to room temperature, poured into water (20 mL) to dilute, and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 473-1 (0.7 g). LC-MS: [M+H]⁺ =263.85.

### Example 83: Preparation of (R)-4-((6-(5-((((3,3-difluoropentan-2-yl)oxy)carbonyl)amino)-1-methyl-1H-1,2,3-triazol-4-yl)-2-methylpyridin-3-yl)ethynyl)nicotinic acid (compound 474)

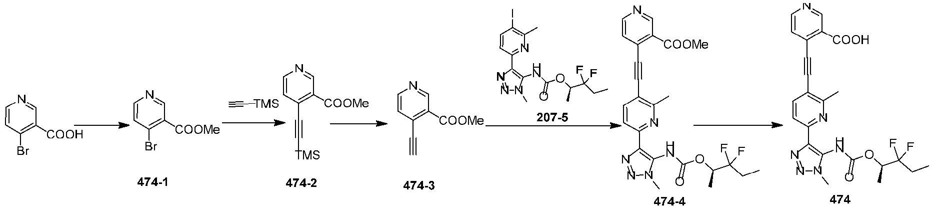

(1) At room temperature, 4-bromonicotinic acid (2.0 g, 9.90 mmol) was dissolved in dichloromethane (20 mL) and methanol (10 mL), and the reaction mixture was cooled to 0°C, followed by the dropwise addition of trimethylsilyldiazomethane (1.8 g, 15.84 mmol, 2 M in n-hexane). The reaction mixture was stirred at 0°C for 1 hour. The reaction mixture was warmed to room temperature and stirred for an additional 0.5 hours. The reaction mixture was poured into water (30 mL) to quench and extracted with dichloromethane (30 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 474-1 (1.0 g). LC-MS: [M+H]⁺=215.90.
(2) At room temperature, compound 474-1 (800 mg, 3.70 mmol) was dissolved in 1,4-dioxane (10 mL), followed by the addition of trimethylsilylacetylene (727 mg, 7.40 mmol), bis(triphenylphosphine)palladium(II) chloride (519 mg, 0.74 mmol), copper(I) iodide (141 mg, 0.74 mmol), and triethylamine (1.1 g, 11.10 mmol). The reaction mixture was heated to 80°C under a nitrogen atmosphere and stirred for 1 hour. After cooling to room temperature, the reaction mixture was poured into water (50 mL) to dilute, extracted with ethyl acetate (20 mL × 3), and the combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 474-2 (800 mg). LC-MS: [M+H]⁺ =234.05.
(3) At room temperature, compound 474-2 (1.0 g, 4.29 mmol) was added to a solution of tetrabutylammonium fluoride in tetrahydrofuran (4.3 mL, 4.29 mmol, 1 M in tetrahydrofuran). The reaction mixture was stirred at room temperature for 0.5 hours. The reaction mixture was poured into water (20 mL) and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 474-3 (400 mg). LCMS: [M+H]⁺ =162.00.
(4) At room temperature, compound 207-5 (150 mg, 0.32 mmol) was dissolved in 1,4-dioxane (3 mL), followed by the addition of compound 474-3 (103 mg, 0.64 mmol), bis(triphenylphosphine)palladium(II) chloride (45 mg, 0.06 mmol), copper(I) iodide (12 mg, 0.06 mmol), and triethylamine (97 mg, 0.96 mmol). The reaction mixture was heated to 50°C under a nitrogen atmosphere and stirred for 1 hour. The reaction mixture was cooled to room temperature, then poured into water (20 mL) to dilute, and extracted with ethyl acetate (20 mL × 3). The combined organic phases were dried over anhydrous sodium sulfate, filtered, and the filtrate was concentrated under reduced pressure. The residue was purified by silica gel column chromatography (petroleum ether/ethyl acetate = 1/1) to obtain compound 474-4 (190 mg). LCMS: [M+H]⁺ =499.20.
(5) At room temperature, compound 474-4 (190 mg, 0.23 mmol) was dissolved in methanol (4 mL) and water (2 mL), followed by the addition of lithium hydroxide monohydrate (29 mg, 0.69 mmol). The reaction mixture was stirred at room temperature for 1 hour. The reaction mixture was diluted with water (10 mL) and concentrated under reduced pressure. The residue was extracted with dichloromethane (10 mL × 3). The aqueous phase was adjusted to pH = 3 to 4 with dilute hydrochloric acid (1 M) and extracted with ethyl acetate (10 mL × 3). The combined organic phases were dried over anhydrous sodium sulfate, filtered, and the filtrate was concentrated under reduced pressure. The residue was purified by high-performance liquid chromatography (0.1% hydrochloric acid system) and lyophilized to obtain compound 474 (63 mg). LCMS: [M+H]⁺ =485.20.

¹H NMR (400 MHz, DMSO-*d₆*) δ 9.99 (s, 1H), 9.13 - 9.07 (m, 1H), 8.82 - 8.78 (m, 1H), 8.03 - 7.99 (m, 1H), 7.89 (d, *J =* 8.0 Hz, 1H), 7.85 - 7.72 (m, 1H), 4.99 (s, 1H), 3.90 (s, 3H), 2.69 (s, 3H), 1.96 (s, 1H), 1.30 - 0.94 (m, 7H).

### Example 84: Preparation of (R)-3-((6-(5-((((3,3-difluoropentan-2-yl)oxy)carbonyl)amino)-1-methyl-1H-1,2,3-triazol-4-yl)-2-methylpyridin-3-yl)ethynyl)isonicotinic acid (compound 475)

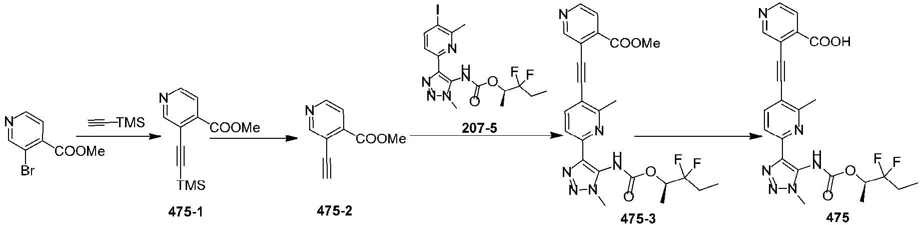

The preparation of compound 475 was referred to the method for preparing compound 474 in Example 83, wherein compound 474-1 was replaced with methyl 3-bromoisonicotinate as the starting material, and compound 475 (85.7 mg) was obtained. LC-MS: [M+H]⁺ =485.15.

¹H NMR (400 MHz, DMSO-*d₆*) δ 9.95 (s, 1H), 8.84 (s, 1H), 8.62 (d, J = 4.8 Hz, 1H), 7.94 (d, J = 8.4 Hz, 1H), 7.85 (d, J = 8.0 Hz, 1H), 7.68 (d, J = 5.2 Hz, 1H), 4.98 (s, 1H), 3.89 (s, 3H), 2.67 (s, 3H), 2.11 - 0.76 (m, 8H).

### Example 85: Preparation of (1-((6-(5-((butyl(methyl)carbamoyl)oxy)methyl)-1-methyl-1H-1,2,3-triazol-4-yl)-2-methylpyridin-3-yl)ethynyl)cyclopropyl)methanesulfonic acid (compound 190)

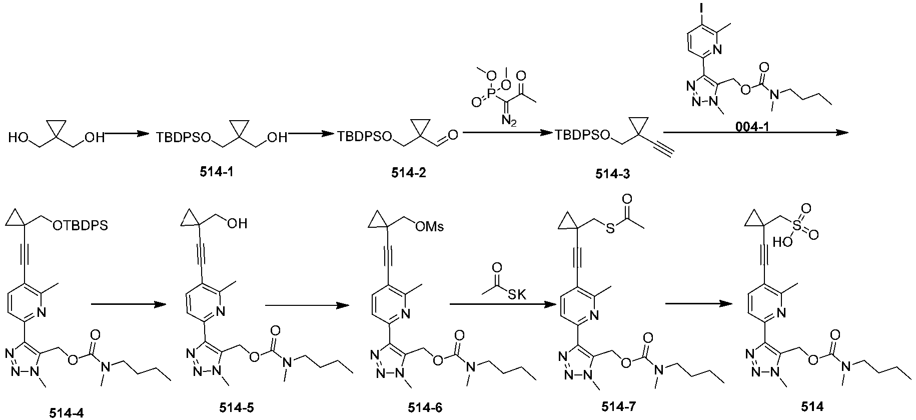

(1) At room temperature, 1,1-bis(hydroxymethyl)cyclopropane (2 g, 19.58 mmol) was dissolved in *N,N*-dimethylformamide (60 mL). Under a nitrogen atmosphere, the reaction mixture was cooled to 0°C, and sodium hydride (783 mg, 19.58 mmol, 60% dispersed in mineral oil) was slowly added. The reaction mixture was stirred at 0°C for 1 hour, followed by the dropwise addition of *tert*-butyldiphenylsilyl chloride (5.4 g, 19.58 mmol). After warming to room temperature, the reaction mixture was stirred for 16 hours. The reaction mixture was slowly added to saturated aqueous ammonium chloride solution (100 mL) and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 514-1 (5.0 g). LCMS: [M+H]⁺ =341.10.
(2) At room temperature, oxalyl chloride (3.7 g, 29.36 mmol) was dissolved in dichloromethane (50 mL). The reaction mixture was cooled to -78°C (dry ice-ethyl acetate), and a solution of dimethyl sulfoxide (4.6 g, 58.72 mmol) in dichloromethane (20 mL) was slowly added dropwise. After the dropwise addition was completed, the reaction mixture was stirred at -78°C for 30 minutes, and then a solution of compound 514-1 (5.0 g, 14.68 mmol) in dichloromethane (30 mL) was added dropwise. The reaction mixture was stirred at -78°C for an additional 30 minutes. Triethylamine (11.9 g, 117.44 mmol) was then added dropwise, while maintaining the internal temperature below -70°C. After the dropwise addition was completed, the reaction mixture was warmed to room temperature and stirred for an additional 30 minutes. The reaction mixture was slowly added to water (100 mL) and extracted with dichloromethane (100 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 514-2 (4.5 g). LCMS: [M+H]⁺ =338.52.
(3) At room temperature, dimethyl (1-diazo-2-oxopropyl)phosphonate (3.8 g, 19.93 mmol) was dissolved in methanol (120 mL). The reaction mixture was cooled to 0°C, and potassium carbonate (3.7 g, 26.58 mmol) was added. After warming to room temperature, the reaction mixture was stirred for 30 minutes. Then, compound 514-2 (4.5 g, 13.29 mmol) was added. The reaction mixture was stirred at room temperature for 2 hours. The reaction mixture was slowly added to water (100 mL) and extracted with dichloromethane (100 mL × 3). The combined organic phases were dried over anhydrous sodium sulfate, filtered, and the filtrate was concentrated under reduced pressure. The residue was purified by silica gel column chromatography (petroleum ether/ethyl acetate = 20/1) to obtain compound 514-3 (4.0 g). LCMS: [M+H]⁺ =334.53.
(4) At room temperature, compound 004-1 (500 mg, 1.13 mmol) was dissolved in 1,4-dioxane (10 mL), followed by the addition of compound 514-3 (756 mg, 2.26 mmol), bis(triphenylphosphine)palladium(II) chloride (79 mg, 0.11 mmol), copper(I) iodide (43 mg, 0.23 mmol), potassium carbonate (312 mg, 2.26 mmol), and triethylamine (686 mg, 6.78 mmol). Under a nitrogen atmosphere, the reaction mixture was stirred at 80°C for 2 hours. The reaction mixture was poured into water (30 mL) and extracted with ethyl acetate (30 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound 514-4 (700 mg). LCMS: [M+H]⁺ =650.20.
(5) At room temperature, tetrabutylammonium fluoride (0.6 g, 2.31 mmol, 1 M in tetrahydrofuran) was added to a solution of compound 514-4 (1.0 g, 1.54 mmol) in tetrahydrofuran (2 mL). The reaction mixture was stirred at room temperature for 3 hours. The reaction mixture was diluted with ethyl acetate (30 mL) and washed with saturated aqueous ammonium chloride solution (20 mL × 3). The organic phase was dried over anhydrous sodium sulfate, filtered, and the filtrate was concentrated under reduced pressure. The residue was purified by silica gel column chromatography (petroleum ether/ethyl acetate = 2/1) to obtain compound 514-5 (520 mg). LCMS: [M+H]⁺ =412.10.
(6) At room temperature, compound 514-5 (420 mg, 1.02 mmol) was dissolved in dichloromethane (20 mL), and the reaction mixture was cooled to 0°C, followed by the addition of triethylamine (310 mg, 3.06 mmol) and methanesulfonyl chloride (175 mg, 1.53 mmol). The reaction mixture was warmed to room temperature and stirred for 1 hour. The reaction mixture was poured into water (20 mL) and extracted with dichloromethane (20 mL × 3). The combined organic phases were dried over anhydrous sodium sulfate, filtered, and the filtrate was concentrated under reduced pressure to obtain compound 514-6 (560 mg, crude). LCMS: [M+H]⁺ =490.15.
(7) At room temperature, compound 514-6 (560 mg, 1.14 mmol) was dissolved in ethanol (10 mL), and potassium thioacetate (156 mg, 1.37 mmol) was added. The reaction mixture was heated to 45°C and stirred for 16 hours. The reaction mixture was poured into water (20 mL) and extracted with dichloromethane (30 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound 514-7 (400 mg). LCMS: [M+H]⁺ =470.10.
(8) Formic acid (967 mg, 21.00 mmol) and hydrogen peroxide (476 mg, 4.20 mmol, purity: 30%) were cooled to 0°C, stirred for 0.5 hours, and a solution of compound 514-7 (100 mg, 0.21 mmol) in tetrahydrofuran (1 mL) was added. The reaction mixture was stirred at room temperature for 3 hours. The reaction mixture was diluted with water (10 mL) and extracted with dichloromethane (10 mL × 3). The combined organic phases were dried over anhydrous sodium sulfate, filtered, and the filtrate was concentrated under reduced pressure. The residue was purified by high-performance liquid chromatography (0.1% trifluoroacetic acid conditions) and lyophilized to obtain compound 514 (45.7 mg). LCMS: [M+H]⁺ =476.10.

¹H NMR (400 MHz, DMSO-*d₆*) δ 7.80 (d, J = 8.4 Hz, 1H), 7.68 (d, J = 8.0 Hz, 1H), 5.60 (s, 2H), 4.08 (s, 3H), 3.42 - 3.38 (m, 3H), 3.15 - 3.03 (m, 2H), 2.76 - 2.63 (m, 3H), 2.53 (s, 2H), 1.52 - 0.42 (m, 11H).

### Example 86: Preparation of 2-(1-((6-(5-((((5-fluoropentan-2-yl)(methyl)carbamoyl)oxy)methyl)-1-methyl-1H-1,2,3-triazol-4-yl)-2-methylpyridin-3-yl)ethynyl)cyclopropyl)acetic acid (compound 390)

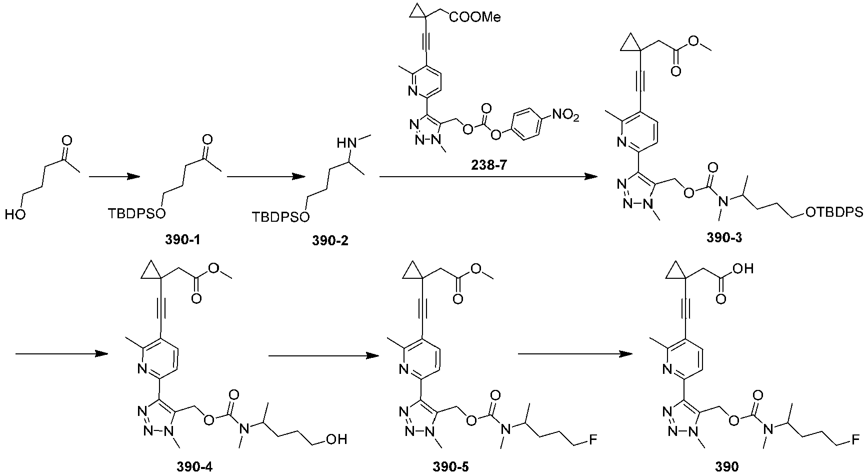

(1) At room temperature, 3-acetyl-1-propanol (5 g, 48.96 mmol) and imidazole (5.0 g, 73.44 mmol) were dissolved in dichloromethane (100 mL), and tert-butyldiphenylsilyl chloride (16.15 g, 58.75 mmol) was added to the reaction mixture under stirring. The reaction was stirred at room temperature for 1 hour. The reaction mixture was poured into saturated sodium bicarbonate solution (100 mL) to quench, followed by extraction with dichloromethane (100 mL × 3). The combined organic phases were sequentially washed with water (100 mL) and saturated brine (100 mL), dried over anhydrous sodium sulfate, filtered, and the filtrate was concentrated to obtain compound 390-1 (18 g). LC-MS: [M+H]⁺ =341.10.
(2) At room temperature, compound 390-1 (2 g, 5.87 mmol) and a solution of methylamine in methanol (1.35 g, 11.74 mmol, 27wt% in methanol) were dissolved in methanol (20 mL), followed by the addition of glacial acetic acid (0.7 g, 11.74 mmol). The reaction mixture was stirred at room temperature for 30 minutes, then sodium cyanoborohydride (0.74 g, 11.74 mmol) was added, and the reaction mixture was heated to 45°C and stirred for an additional 2 hours. The reaction mixture was quenched with saturated aqueous sodium bicarbonate solution (50 mL) and extracted with dichloromethane (20 mL × 3). The combined organic phases were sequentially washed with water (30 mL) and saturated brine (30 mL), dried over anhydrous sodium sulfate, filtered, and the filtrate was concentrated under reduced pressure. The residue was purified by silica gel column chromatography (dichloromethane/methanol = 15/1) to obtain compound 390-2 (450 mg). LC-MS: [M+H]⁺ =356.05.
(3) At room temperature, compound 238-7 (170 mg, 0.34 mmol) was dissolved in dichloromethane (2 mL), and the reaction mixture was cooled to 0°C, followed by the addition of *N,N*-diisopropylethylamine (93 mg, 0.72 mmol) and compound 390-2 (180 mg, 0.51 mmol). The reaction mixture was stirred at room temperature for 1 hour. The reaction mixture was diluted with water (10 mL) and extracted with dichloromethane (10 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 390-3 (300 mg). LC-MS: [M+H]⁺ = 722.25.
(4) At room temperature, compound 390-3 (270 mg, 0.37 mmol) was dissolved in tetrahydrofuran (5 mL), and tetrabutylammonium fluoride (166 mg, 0.44 mmol, 70%) was added dropwise under stirring. The reaction mixture was stirred at room temperature for 1 hour. The reaction mixture was diluted with water (20 mL) and extracted with ethyl acetate (20 mL × 3). The combined organic phases were sequentially washed with water (30 mL) and saturated brine (30 mL), dried over anhydrous sodium sulfate, filtered, and the filtrate was concentrated under reduced pressure. The residue was purified by silica gel column chromatography (petroleum ether/ethyl acetate = 5/1) to obtain compound 390-4 (160 mg). LC-MS: [M+H]⁺=484.15.
(5) At room temperature, compound 390-4 (130 mg, 0.27 mmol) was dissolved in dichloromethane (2 mL), and the reaction mixture was cooled to 0°C, followed by the dropwise addition of diethylaminosulfur trifluoride (65 mg, 0.41 mmol). After the dropwise addition was completed, the reaction mixture was stirred at room temperature for 1 hour. The reaction mixture was quenched with saturated aqueous sodium bicarbonate solution (20 mL) and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 2/1) to obtain compound 390-5 (150 mg, crude). LC-MS: [M+H]⁺= 486.15.
(6) At room temperature, compound 390-5 (150 mg, crude) was dissolved in methanol (3 mL) and water (1 mL), followed by the addition of lithium hydroxide monohydrate (65 mg, 1.55 mmol). The reaction mixture was stirred at room temperature for 2 hours. The reaction mixture was adjusted to pH = 7 with dilute hydrochloric acid (1 M) and extracted with ethyl acetate (3 mL × 3). The combined organic phases were dried over anhydrous sodium sulfate, filtered, and the filtrate was concentrated under reduced pressure. The residue was purified by preparative high-performance liquid chromatography (0.1% ammonia in water) and lyophilized to obtain compound 390 (12.3 mg). LC-MS: [M+H]⁺ =472.10.

¹H NMR (400 MHz, DMSO-*d₆*) δ 7.82 (d, *J* = 8.0 Hz, 1H), 7.71 (d, *J* = 8.0 Hz, 1H), 5.76 - 5.47 (m, 2H), 4.47 - 4.20 (m, 2H), 4.13 - 4.10 (m, 1H), 4.08 (s, 3H), 2.57 (d, *J* = 28.6 Hz, 3H), 2.51 (s, 3H), 2.43 (s, 2H), 1.53 - 1.19 (m, 4H), 1.08 - 0.88 (m, 7H).

### Example 87: Preparation of the hydrochloride of 2-(1-((6-(5-(3-(butyl(methyl)amino)-2,2-difluoro-3-oxopropyl)-1-methyl-1H-1,2,3-triazol-4-yl)-2-methylpyridin-3-yl)ethynyl)cyclopropyl)acetic acid (compound 403)

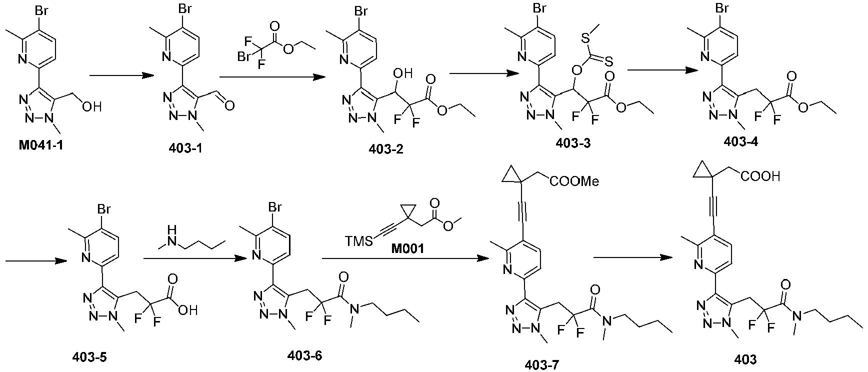

(1) At room temperature, compound M041-1 (730 mg, 2.58 mmol) was dissolved in dichloromethane (10 mL), and sodium bicarbonate (1.08 g, 12.9 mmol) and Dess-Martin periodinane (1.31 g, 3.09 mmol) were added. The reaction mixture was stirred at room temperature for 2 hours. The reaction mixture was filtered, and saturated aqueous sodium bicarbonate solution (15 mL) was added to the filtrate, followed by extraction with dichloromethane (10 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1 to 1/1) to obtain compound 403-1 (650 mg). LC-MS: [M+H]⁺= 281.0.
(2) At room temperature, zinc powder (1.39 g, 21.35 mmol) was dispersed in anhydrous tetrahydrofuran (20 mL). Under a nitrogen atmosphere, trimethylsilyl chloride (230 mg, 2.13 mmol) was added. The reaction mixture was stirred at room temperature for 5 minutes, then heated to 45°C, and ethyl bromodifluoroacetate (2.60 g, 12.81 mmol) was slowly added dropwise. After the dropwise addition was completed, the reaction mixture was stirred at 45°C for an additional 0.5 hours, then cooled to room temperature and allowed to stand. The supernatant was taken and slowly added dropwise to a solution of compound 403-1 (1.2 g, 4.27 mmol) in tetrahydrofuran (20 mL) at room temperature. After the dropwise addition was completed, the reaction mixture was heated to 75°C and stirred for 0.5 hours. The reaction mixture was cooled to 0°C, diluted with ice water (40 mL), and then adjusted to pH = 3 to 4 with dilute hydrochloric acid (1 M). The aqueous phase was extracted with ethyl acetate (40 mL × 3). The combined organic phases were washed with saturated aqueous sodium chloride solution (50 mL). The organic phase was dried over anhydrous sodium sulfate, filtered, and the filtrate was concentrated under reduced pressure. The residue was purified by silica gel column chromatography (petroleum ether/ethyl acetate = 3/1) to obtain compound 403-2 (1.0 g). LC-MS: [M +H]⁺ = 405.00.
(3) At room temperature, compound 403-2 (1.00 g, 2.47 mmol) was dissolved in *N,N-*dimethylformamide (15 mL), followed by the sequential addition of 1,8-diazabicyclo[5.4.0]undec-7-ene (1.50 g, 9.90 mmol) and carbon disulfide (1.88 g, 24.75 mmol). Under a nitrogen atmosphere, the reaction mixture was stirred at room temperature for 1 hour, and then iodomethane (3.50 g, 24.75 mmol) was added. The reaction mixture was stirred at room temperature for an additional 1 hour. The reaction mixture was diluted with water (30 mL) and extracted with ethyl acetate (30 mL × 3). The combined organic phases were washed with saturated aqueous sodium bicarbonate solution (30 mL) and saturated aqueous sodium chloride solution (30 mL). The organic phase was dried over anhydrous sodium sulfate, filtered, and the filtrate was concentrated under reduced pressure to obtain compound 403-3 (1.2 g, crude). LC-MS [M+H]⁺=496.90.
(4) At room temperature, compound 403-3 (1.2 g, crude) was dissolved in toluene (5 mL), followed by the addition of tributyltin hydride (1.40 g, 4.84 mmol) and azobisisobutyronitrile (79 mg, 0.48 mmol). Under a nitrogen atmosphere, the reaction mixture was heated to 90°C and stirred for 1.5 hours. After cooling to room temperature, the reaction mixture was concentrated under reduced pressure to obtain compound 403-4 (2.0 g, crude). LC-MS:[M +H]⁺ = 389.00.
(5) At room temperature, compound 403-4 (2.0 g, crude) was dissolved in methanol (10 mL) and water (3 mL), followed by the addition of lithium hydroxide monohydrate (509 mg, 12.12 mmol). The reaction mixture was stirred at room temperature for 1 hour. The reaction mixture was concentrated under reduced pressure. The residue was diluted with water (10 mL) and extracted with ethyl acetate (20 mL × 2). The combined aqueous phases were adjusted to pH = 3 to 4 with dilute hydrochloric acid (1 M) and then extracted with ethyl acetate (20 mL × 3). The combined organic phases were washed with saturated aqueous sodium chloride solution (30 mL). The organic phase was dried over anhydrous sodium sulfate, filtered, and the filtrate was concentrated under reduced pressure to obtain compound 403-5 (700 mg, crude). LC-MS [M+H]⁺=360.95.
(6) At room temperature, compound 403-5 (240 mg, 0.660 mmol) was dissolved in *N,N-*dimethylformamide (3 mL), followed by the addition of (7-azabenzotriazol-1-yl)-*N,N,N',N'-*tetramethyluronium hexafluorophosphate (301 mg, 0.790 mmol), *N,N*-diisopropylethylamine (256 mg, 1.98 mmol), and N-methyl-1-butylamine (69.0 mg, 0.790 mmol). The reaction mixture was stirred at room temperature for 1 hour. The reaction mixture was diluted with water (10 mL) and extracted with ethyl acetate (5 mL × 3). The combined organic phases were washed with saturated brine (10 mL × 2), dried over anhydrous sodium sulfate, filtered, and the filtrate was concentrated under reduced pressure. The residue was purified by silica gel column chromatography (petroleum ether/ethyl acetate = 3/1) to obtain compound 403-6 (150 mg). LC-MS: [M+H]⁺=432.00.
(7) At room temperature, compound M001 (184 mg, 0.880 mmol) was added to a solution of tetrabutylammonium fluoride in tetrahydrofuran (1 M, 1 mL), and the reaction mixture was stirred at room temperature for 30 minutes. 2-Methyltetrahydrofuran (1 mL) and water (1 mL) were added to the reaction mixture, and the organic phase was sequentially washed with water (1 mL) and saturated brine (0.5 mL). The organic phase was dried over anhydrous sodium sulfate and filtered. The filtrate was added to a solution containing compound 403-6 (150 mg, 0.350 mmol), bis(triphenylphosphine)palladium(II) chloride (24.6 mg, 0.0350 mmol), copper(I) iodide (6.67 mg, 0.0350 mmol), triethylamine (106 mg, 1.05 mmol), and potassium carbonate (96.8 mg, 0.700 mmol) in tetrahydrofuran (1 mL). Under a nitrogen atmosphere, the reaction mixture was heated to 80°C and reacted for 2 hours. The reaction mixture was cooled to room temperature, diluted with water (3 mL), and extracted with ethyl acetate (2 mL × 2). The combined organic phases were washed with saturated brine (3 mL), dried over anhydrous sodium sulfate, filtered, and the filtrate was concentrated under reduced pressure. The residue was purified by silica gel column chromatography (petroleum ether/ethyl acetate = 1/1) to obtain compound 403-7 (130 mg). LC-MS: [M+H]⁺=488.10.
(8) At room temperature, compound 403-7 (110 mg, 0.230 mmol) was dissolved in 1,4-dioxane (6 mL), and concentrated hydrochloric acid (252 mg, 6.90 mmol) was added. The reaction mixture was stirred at room temperature for 16 hours. The reaction mixture was added with saturated brine (5 mL) and extracted with ethyl acetate (3 mL × 3). The combined organic phases were washed with saturated brine (5 mL). After drying over anhydrous sodium sulfate, the mixture was filtered, and the filtrate was concentrated under reduced pressure. The residue was purified by preparative high-performance liquid chromatography (0.1% hydrochloric acid conditions), concentrated under reduced pressure, and lyophilized to obtain the hydrochloride of compound 403 (36.3 mg). LC-MS: [M+H]⁺=474.15.

¹H NMR (400 MHz, DMSO-*d₆*) δ 7.84 (t, *J =* 7.2 Hz, 1H), 7.73 (d, *J* = 8.2 Hz, 1H), 4.41 (m, 2H), 4.06 (s, 3H), 3.30 - 3.21 (m, 2H), 3.00 (s, 2H), 2.81 (s, 1H), 2.50 - 2.48 (m, 3H), 2.45 (s, 2H), 1.50 - 1.30 (m, 2H), 1.17 (m, 2H), 1.06 (d, *J* = 6.8 Hz, 2H), 0.98 - 0.90 (m, 2H), 0.82 (m, 3H).

### Example 88: Preparation of the hydrochloride of 2-(1-((6-(5-(2,2-difluoro-3-((4-fluorobutyl)(methyl)amino)-3-oxopropyl)-1-methyl-1H-1,2,3-triazol-4-yl)-2-methylpyridin-3-yl)ethynyl)cyclopropyl)acetic acid (compound 404)

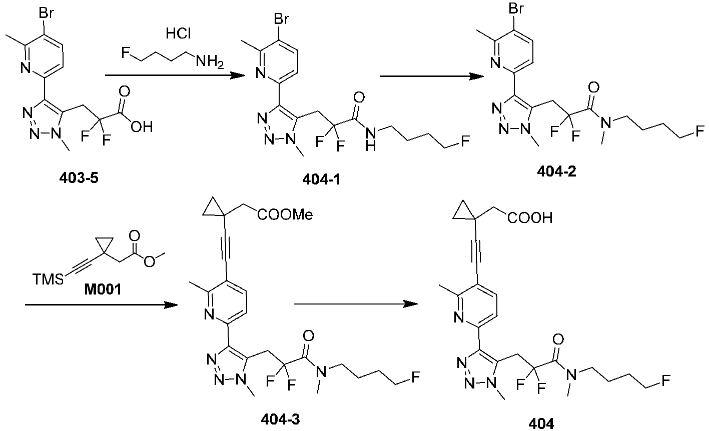

The preparation of compound 404 was referred to the method for preparing compound 403 in Example 87, wherein intermediate compound 403-6 was replaced with intermediate compound 404-2, and the hydrochloride of compound 404 (10.1 mg) was obtained. LC-MS: [M+H]⁺=492.15.

¹H NMR (400 MHz, DMSO-*d₆*) δ 7.84 (t, J = 8.0 Hz, 1H), 7.73 (d, J = 8.2 Hz, 1H), 4.40 (m, 2H), 4.06 (s, 3H), 3.34 (m, 2H), 2.89 (d, J = 85.2 Hz, 3H), 2.52 (d, J = 10.4 Hz, 4H), 2.45 (s, 2H), 1.99 - 1.55 (m, 6H), 1.05 (t, J = 5.6 Hz, 2H), 0.95 (t, J = 5.6 Hz, 2H).

The preparation method for intermediate compound 404-2 is as follows:
(1) At room temperature, compound 403-5 (200 mg, 0.550 mmol) was dissolved in dichloromethane (4 mL), followed by the addition of propylphosphonic anhydride (525 mg, 0.830 mmol) and *N,N-*diisopropylethylamine (213 mg, 1.65 mmol). The reaction mixture was stirred at room temperature for 0.5 hours, then 4-fluorobutan-1-amine hydrochloride (65.0 mg, 0.720 mmol) was added. The reaction mixture was stirred at room temperature for 1 hour. The reaction mixture was diluted with water (5 mL) and extracted with dichloromethane (3 mL × 3). The combined organic phases were washed with saturated brine (5 mL), dried over anhydrous sodium sulfate, filtered, and the filtrate was concentrated under reduced pressure. The residue was purified by silica gel column chromatography (petroleum ether/ethyl acetate = 3/1) to obtain compound 404-1 (190 mg). LC-MS: [M+H]⁺=435.90.
(2) At room temperature, compound 404-1 (190 mg, 0.440 mmol) was dissolved in *N,N-*dimethylformamide (5 mL), followed by the addition of cesium carbonate (1.43 g, 4.40 mmol) and iodomethane (312 mg, 2.2 mmol). The reaction mixture was stirred at room temperature for an additional 16 hours. The reaction mixture was diluted with water (10 mL) and extracted with ethyl acetate (5 mL × 3). The combined organic phases were washed with saturated brine (10 mL × 2), dried over anhydrous sodium sulfate, filtered, and the filtrate was concentrated under reduced pressure. The residue was purified by silica gel column chromatography (petroleum ether/ethyl acetate = 1/1) to obtain compound 404-2 (110 mg). LC-MS: [M+H]⁺=447.95.

### Example 89: Preparation of the hydrochloride of 2-(1-((6-(5-(3-((cyclobutylmethyl)((methyl)amino)-2,2-difluoro-3-oxopropyl)-1-methyl-1H-1,2,3-triazol-4-yl)-2-methylpyridin-3-yl)ethynyl)cyclopropyl)acetic acid (compound 405)

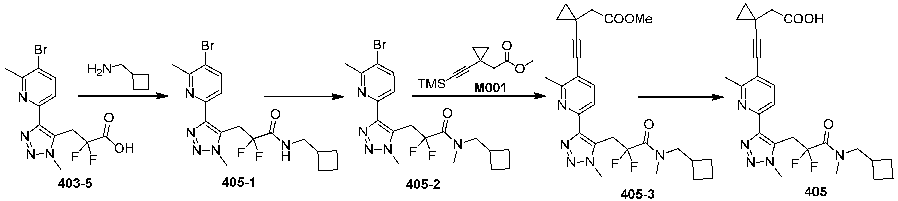

The preparation of compound 405 was referred to the method for preparing compound 404 in Example 88, wherein 4-fluorobutan-1-amine hydrochloride was replaced with cyclobutylmethylamine, and the hydrochloride of compound 405 (52.6 mg) was obtained. LC-MS: [M+H]⁺=486.15.

¹H NMR (400 MHz, DMSO-*d₆*) δ 7.84 (t, J = 8.0 Hz, 1H), 7.73 (d, J = 8.2 Hz, 1H), 4.40 (m, 2H), 4.06 (s, 3H), 3.34 (m, 2H), 2.89 (d, J = 85.2 Hz, 3H), 2.52 (d, J = 10.4 Hz, 4H), 2.45 (s, 2H), 1.99 - 1.55 (m, 6H), 1.05 (t, J = 5.6 Hz, 2H), 0.95 (t, J = 5.6 Hz, 2H).

### Example 90: Preparation of (R)-2-(3-((6-(5-((((4,4-difluoropentan-2-yl)oxy)carbonyl)amino)-1-methyl-1H-1,2,3-triazol-4-yl)-2-methylpyridin-3-yl)ethynyl)-1-methylazetidin-3-yl)acetic acid (compound 480)

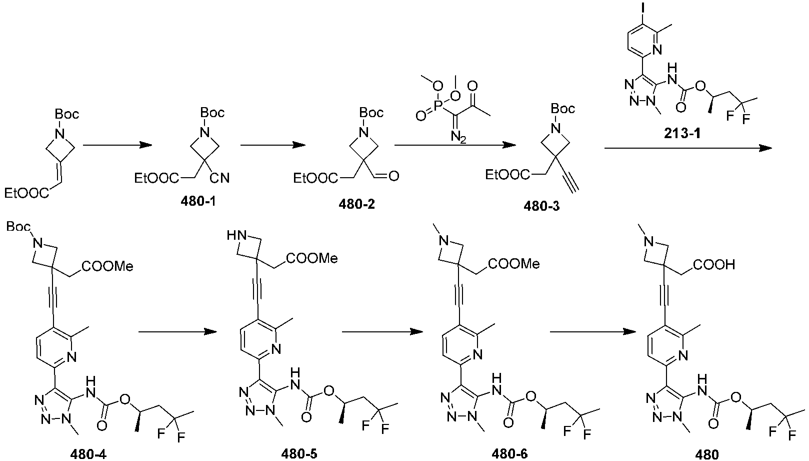

(1) At room temperature, trimethylsilyl cyanide (1.6 g, 16.58 mmol) was added to tetrabutylammonium fluoride (4.3 g, 16.58 mmol). After stirring the reaction mixture at room temperature for 0.5 hours, a solution of *tert*-butyl 3-(2-ethoxy-2-oxoethylidene)azetidine-1-carboxylate (2.0 g, 8.29 mmol) in tetrahydrofuran (10 mL) was added. The reaction mixture was heated to 50°C and stirred for an additional 3 hours. After cooling to room temperature, the reaction mixture was poured into water (50 mL) to dilute and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1) to obtain compound 480-1 (2.2 g). LC-MS [M+H]⁺ = 269.14.
(2) At room temperature, compound 480-1 (1.3 g, 4.85 mmol) was dissolved in pyridine (20 mL), glacial acetic acid (10 mL), and water (10 mL), followed by the addition of Raney nickel (285 mg, 4.85 mmol). The reaction mixture was heated to 60°C and stirred for 4 hours. After cooling to room temperature, the reaction mixture was diluted with ethyl acetate (50 mL) and sequentially washed with dilute hydrochloric acid (30 mL × 3) and saturated aqueous sodium bicarbonate solution (30 mL × 3). The organic phase was dried over anhydrous sodium sulfate, filtered, and the filtrate was concentrated under reduced pressure to obtain compound 480-2 (640 mg). LC-MS [M+H]⁺=271.14.
(3) At room temperature, dimethyl (1-diazo-2-oxopropyl)phosphonate (680 mg, 3.54 mmol) was dissolved in methanol (10 mL), and the reaction mixture was cooled to 0°C, followed by the addition of potassium carbonate (815 mg, 5.90 mmol). The reaction mixture was warmed to room temperature and stirred for 0.5 hours. Then, compound 480-2 (640 mg, 2.36 mmol) was added. The reaction mixture was stirred at room temperature for an additional 2 hours. The reaction mixture was poured into water (50 mL), extracted with dichloromethane (50 mL × 3), and the combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 480-3 (320 mg). LC-MS [M+H]⁺=268.15.
(4) At room temperature, compound 213-1 (220 mg, 0.47 mmol) was dissolved in 1,4-dioxane (5 mL), followed by the addition of compound 480-3 (238 mg, 0.94 mmol), bis(triphenylphosphine)palladium(II) chloride (66 mg, 0.09 mmol), copper(I) iodide (18 mg, 0.09 mmol), potassium carbonate (130 mg, 0.94 mmol), and triethylamine (285 mg, 2.82 mmol). Under a nitrogen atmosphere, the reaction mixture was heated to 50°C and stirred for 2 hours. After cooling to room temperature, the reaction mixture was poured into water (30 mL) to dilute and extracted with ethyl acetate (30 mL × 3). The combined organic phases were dried over anhydrous sodium sulfate, filtered, and the filtrate was concentrated under reduced pressure. The residue was purified by silica gel column chromatography (dichloromethane/methanol = 10/1) to obtain compound 480-4 (260 mg). LCMS: [M+H]⁺ =591.25.
(5) At room temperature, compound 480-4 (250 mg, 0.42 mmol) was dissolved in dichloromethane (5 mL), and trifluoroacetic acid (5 mL) was slowly added. The reaction mixture was stirred at room temperature for 1 hour. The reaction mixture was concentrated under reduced pressure to remove trifluoroacetic acid. The residue was dissolved in water (10 mL), and the solution was adjusted to pH = 8 to 9 with saturated sodium bicarbonate solution. The mixture was then extracted with dichloromethane (20 mL × 3). The combined organic phases were dried over anhydrous sodium sulfate, filtered, and the filtrate was concentrated under reduced pressure to obtain compound 480-5 (180 mg). LCMS: [M+H]⁺ =491.20.
(6) At room temperature, compound 480-5 (150 mg, 0.31 mmol) was dissolved in dichloromethane (2 mL), followed by the addition of formaldehyde (74 mg, 0.91 mmol, 37% in water) and sodium triacetoxyborohydride (394 mg, 1.86 mmol). The reaction mixture was stirred at room temperature for 0.5 hours. The reaction mixture was poured into saturated aqueous ammonium chloride solution (20 mL) and extracted with dichloromethane (20 mL × 3). The combined organic phases were dried over anhydrous sodium sulfate, filtered, and the filtrate was concentrated under reduced pressure. The residue was purified by silica gel column chromatography (dichloromethane/methanol = 10/1) to obtain compound 480-6 (150 mg). LCMS: [M+H]⁺ =505.20.
(7) At room temperature, compound 480-6 (180 mg, 0.36 mmol) was dissolved in tetrahydrofuran (3 mL) and water (3 mL), followed by the addition of lithium hydroxide monohydrate (45 mg, 1.08 mmol). The reaction mixture was stirred at room temperature for 1 hour. The reaction mixture was diluted with water (10 mL) and concentrated under reduced pressure to remove methanol. The residue was extracted with dichloromethane (10 mL × 3). The combined aqueous phases were adjusted to pH = 3 to 4 with dilute hydrochloric acid (1 M) and then extracted with ethyl acetate (10 mL × 3). The combined organic phases were dried over anhydrous sodium sulfate, filtered, and the filtrate was concentrated under reduced pressure. The residue was purified by high-performance liquid chromatography (0.1% ammonia in water system) and lyophilized to obtain compound 480 (75.7 mg). LCMS: [M+H]⁺ =491.20.

¹H NMR (400 MHz, DMSO-*d₆*) δ 9.55 (s, 1H), 7.70 (q, J = 8.0 Hz, 2H), 4.96 (s, 1H), 3.84 (s, 3H), 3.26 - 3.25 (m, 4H), 2.53 - 2.51 (m, 5H), 2.19 - 2.04 (m, 5H), 1.57 (t, J = 18.0 Hz, 3H), 1.19 (s, 3H).

### Example 91: Preparation of (R)-6-chloro-3-((6-(5-((((3,3-difluoropentan-2-yl)oxy)carbonyl)amino)-1-methyl-1H-1,2,3-triazol-4-yl)-2-methylpyridin-3-yl)ethynyl)picolinic acid (compound 483)

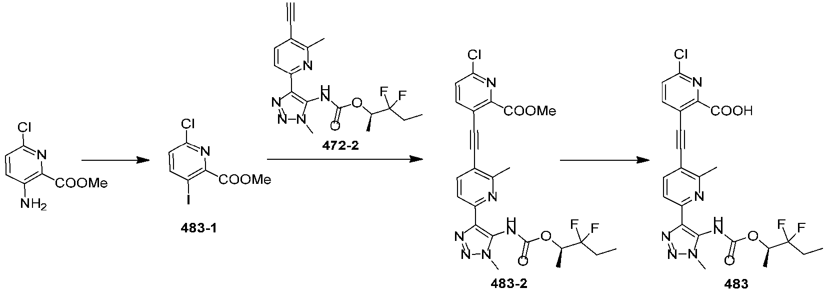

The preparation of compound 483 was referred to the method for preparing compound 472 in Example 81, wherein the starting material methyl 2-bromonicotinate was replaced with compound 483-1 (167 mg, 0.56 mmol), and compound 483 (31.5 mg) was obtained. LCMS: [M+H]⁺ =519.10.

¹H NMR (400 MHz, DMSO-*d₆*) δ 9.94 (s, 1H), 8.20 (d, J = 8.4 Hz, 1H), 7.95 (d, J = 8.0 Hz, 1H), 7.87 (d, J = 8.0 Hz, 1H), 7.77 (d, J = 8.4 Hz, 1H), 4.98 (s, 1H), 3.89 (s, 3H), 2.64 (s, 3H), 1.96 (s, 1H), 1.52 - 1.08 (m, 4H), 0.94 (s, 3H).

The preparation method for compound 483-1 is as follows:
At room temperature, methyl 3-amino-6-chloropicolinate (200 mg, 1.07 mmol) was dissolved in hydrochloric acid (2 mL, 6 M), and the reaction mixture was cooled to 0°C, followed by the addition of sodium nitrite (96 mg, 1.39 mmol). The reaction mixture was warmed to room temperature and stirred for 2 hours. The reaction mixture was then added dropwise to a solution of potassium iodide (213 mg, 1.28 mmol) in water (6 mL) at 60°C. The reaction mixture was stirred at 60°C for 0.5 hours. The reaction mixture was cooled to room temperature, poured into water (10 mL) to dilute, and extracted with dichloromethane (20 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1) to obtain compound 483-1 (240 mg). LC-MS: [M+H]⁺ =297.85.

### Example 92: Preparation of (R)-(5-((2-methyl-6-(1-methyl-5-(((pentan-2-yloxy)carbonyl)amino)-1H-1,2,3-triazol-4-yl)pyridin-3-yl)ethynyl)spiro[2.3]hexan-5-yl)methanesulfonic acid (compound 508)

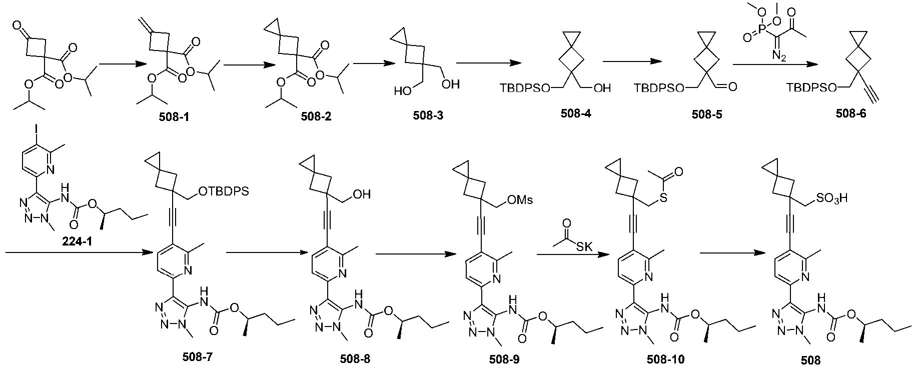

The preparation of compound 508 was referred to the method for preparing compound 190 in Example 85, wherein the starting material 1,1-bis(hydroxymethyl)cyclopropane was replaced with compound 508-3, and compound 508 (56.8 mg) was obtained. LCMS: [M+H]⁺ =502.05.

¹H NMR (400 MHz, DMSO-*d₆*) δ 9.57 (s, 1H), 7.86 - 7.68 (m, 2H), 4.70 (s, 1H), 3.87 (s, 3H), 3.00 - 2.96 (m, 2H), 2.66 - 2.58 (m, 5H), 2.36 - 2.34 (m, 2H), 1.51 - 0.82 (m, 10H), 0.47 - 0.38 (m, 4H).

The preparation method for compound 508-3 is as follows:
(1) At room temperature, methyltriphenylphosphonium bromide (23 g, 64.4 mmol) was dissolved in tetrahydrofuran (140 mL). Under a nitrogen atmosphere, the reaction mixture was cooled to 0°C, and a solution of potassium tert-butoxide in tetrahydrofuran (1.0 M, 64.4 mL, 64.4 mmol) was slowly added dropwise. The reaction mixture was stirred at room temperature for 1 hour, and then a solution of diisopropyl 3-oxocyclobutane-1,1-dicarboxylate (12 g, 49.5 mmol) in tetrahydrofuran (60 mL) was added dropwise. After warming to room temperature, the reaction mixture was stirred for 1 hour. The reaction mixture was slowly added to saturated aqueous ammonium chloride solution (30 mL) to quench and extracted with ethyl acetate (30 mL × 2). The combined organic phases were dried over anhydrous sodium sulfate, filtered, and the filtrate was concentrated under reduced pressure. The residue was purified by silica gel column chromatography (petroleum ether/ethyl acetate = 10/1) to obtain compound 508-1 (9.6 g). LCMS: [M+H]⁺ =241.00.
(2) At room temperature, diethylzinc (13.7 g, 111.1 mmol, 1.0 M in tetrahydrofuran) was dissolved in dichloromethane (100 mL). Under a nitrogen atmosphere, the reaction mixture was cooled to 0°C, and a solution of trifluoroacetic acid (12.7 g, 111.1 mmol) in dichloromethane (20 mL) was slowly added dropwise. After the dropwise addition was completed, the reaction mixture was stirred at 0°C for 30 minutes, and then a solution of diiodomethane (11.9 g, 117.44 mmol) in dichloromethane (20 mL) was slowly added dropwise. The reaction mixture was stirred at 0°C for an additional 1 hour, and then a solution of compound 508-1 (8.9 g, 37.1 mmol) in dichloromethane (20 mL) was added. After the dropwise addition was completed, the reaction mixture was warmed to room temperature and stirred for 16 hours. The reaction mixture was slowly added to saturated aqueous ammonium chloride solution (100 mL) and extracted with dichloromethane (100 mL). The organic phase was dried over anhydrous sodium sulfate, filtered, and the filtrate was concentrated under reduced pressure. The residue was purified by silica gel column chromatography (petroleum ether/ethyl acetate = 10/1) to obtain compound 508-2 (14.1 g, crude). LCMS: [M+H]⁺ =255.05.
(3) At room temperature, lithium aluminum hydride (2.9 g, 76.7 mmol) was dissolved in tetrahydrofuran (130 mL). Under a nitrogen atmosphere, the reaction mixture was cooled to 0°C, and a solution of compound 508-2 (13 g, 51.1 mmol) in tetrahydrofuran (50 mL) was added. After the dropwise addition was completed, the reaction mixture was warmed to room temperature and stirred for 16 hours. Water (5 mL), 50% aqueous sodium hydroxide solution (2 mL), and water (10 mL) were sequentially added slowly to the reaction mixture. The mixture was filtered through diatomite, and the filter cake was washed with ethyl acetate (30 mL). The filtrate was diluted with water (50 mL) and extracted with ethyl acetate (100 mL). The organic phase was dried over anhydrous sodium sulfate, filtered, and the filtrate was concentrated under reduced pressure. The residue was purified by silica gel column chromatography (petroleum ether/ethyl acetate = 0/1) to obtain compound 508-3 (3 g). LCMS: [M+H]⁺ =143.20.

The compounds in the following table were prepared by referring to the preparation methods of the above Example 8, Example 10, Example 13, Example 23, Example 27, Example 33, Example 43, Example 45, Example 50, Example 64, Example 73, *etc.* Taking Example 8 as an example, 1-cyclopropylethanol in Example 8 was replaced with Reactant 1 corresponding to the following table, and compound M001 was replaced with Reactant 2 corresponding to the following table:

| No. | Compound structure | Reactant 1 | Reactant 2 | Characterization |
|---|---|---|---|---|
| 010-A | 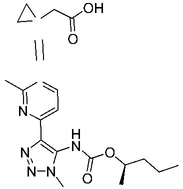 | 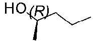 | 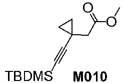 | LC-MS: [M+H]⁺ =426.15. |
| | | | | ¹H NMR (400 MHz, DMSO-*d₆*) δ 12.23 (s, 1H), 9.39 (s, 1H), 7.75-7.69 (m, 2H), 4.70 (s, 1H), 3.88 (s, 3H), 3.32 (s, 2H), 2.51 (s, 3H), 2.45 (s, 2H), 1.30-1.23 (m, 5H), 1.07-1.04 (m, 2H), 0.96-0.94 (m, 2H), 0.84 (s, 3H). |
| 053 | 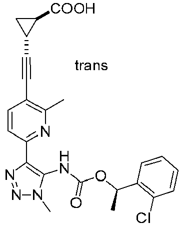 | 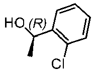 | 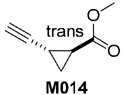 | LC-MS: [M+H]⁺ =480.15. |
| | | | | The compound contains a pair of trans isomers. |
| | | | | ¹H NMR (400 MHz, DMSO-*d₆*) δ9.76 (s, 1H), 7.71 (s, 2H), 7.37 (d, *J* = 44.0 Hz, 4H), 5.98 (s, 1H), 3.88 (s, 3H), 2.44 (s, 3H), 1.78 - 1.65 (m, 2H), 1.65 - 1.20 (m, 3H), 1.19 - 1.13 (m, 1H), 1.02 - 0.94 (m, 1H). |
| 081 | 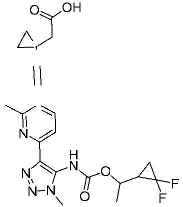 | 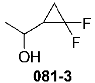 | 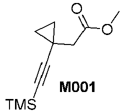 | LC-MS: [M+H]⁺ =460.15. |
| | | | | ¹H NMR (400 MHz, DMSO-*d₆*) δ 7.73 (d, *J* = 8.0 Hz, 1H), 7.61 (d, *J* = 8.0 Hz, 1H), 4.59 - 4.46 (m, 1H), 3.80 (s, 3H), 2.51 (s, 3H), 2.19 (d, *J* = 4.0 Hz, 2H), 2.02 - 1.90 (m, 1H), 1.64 - 1.54 (m, 1H), 1.47 - 1.35 (m, 1H), 1.29 - 1.23 (m, 3H), 0.95 - 0.88 (m, 4H). |
| 088 | 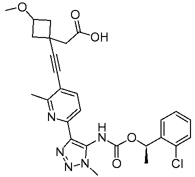 | 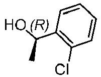 | 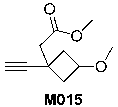 | LC-MS: [M+H]⁺ =538.18. |
| | | | | The compound contains cis-trans isomers. |
| 123 | 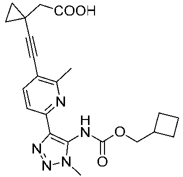 | 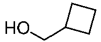 | 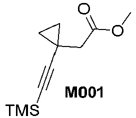 | LC-MS: [M+H]⁺= 424.15. |
| | | | | ¹H NMR (400 MHz, DMSO-*d₆*) δ 12.22 (s, 1H), 9.47 (s, 1H), 7.75 (d, *J* = 8.0 Hz, 1H), 7.69 (d, *J* = 8.0 Hz, 1H), 4.00 (s, 2H), 3.88 (s, 4H), 2.52 (s, 5H), 2.46 (s, 3H), 1.83 (m, 8H), 1.06 (m, 3H), 0.95 (m, 3H). |
| 207' | 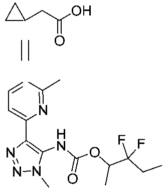 | 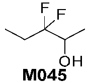 | 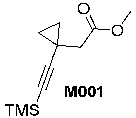 | LC-MS: [M+H]⁺ =462.05. |
| | | | | ¹H NMR (400 MHz, DMSO-*d₆*)δ 7.74 (d, J = 8.2 Hz, 1H), 7.65 (d, J = 8.2 Hz, 1H), 5.04 - 4.91 (m, 1H), 3.85 (s, 3H), 2.51 (s, 2H), 2.26 (s, 2H), 2.05 - 1.78 (m, 3H), 1.23 (s, 3H), 0.96 - 0.88 (m, 7H). |
| 223 | 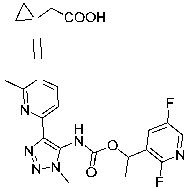 | 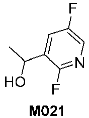 | 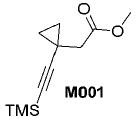 | LC-MS [M+H]⁺= 497.05. |
| | | | | ¹H NMR (400 MHz, DMSO-*d₆*) δ 8.16 (s, 1H), 7.72 - 7.61 (m, 3H), 5.77 (s, 1H), 3.86 (s, 3H), 2.39 - 2.38 (m, 5H), 1.43 (s, 3H), 1.01 (s, 2H), 0.93 (s, 2H). |
| 225 | 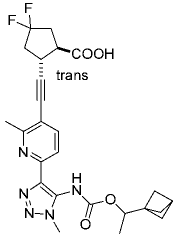 | 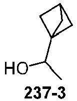 | 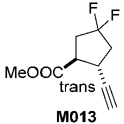 | LC-MS [M+H]⁺= 500.20. |
| 225-B | 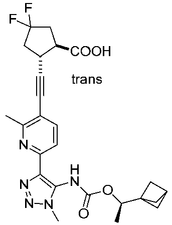 | 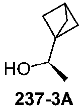 | 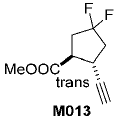 | LC-MS: [M+H]⁺ =500.15. |
| | | | | The compound contains a pair of trans isomers. |
| | | | | ¹H NMR (400 MHz, DMSO-*d₆*) δ9.47 (s, 1H), 7.83 - 7.74 (m, 2H), 4.66 (s, 1H), 3.88 (s, 3H), 3.51 - 3.42 (m, 2H), 3.11 (d, *J* = 8.0 Hz, 1H), 2.76 - 2.58 (m, 2H), 2.56 (s, 3H), 2.41 - 2.23 (m, 2H), 1.90 - 1.35(m, 6H), 1.33 - 0.95 (m, 3H). |
| 226 | 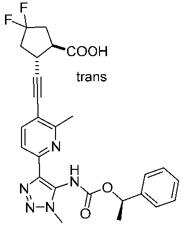 | 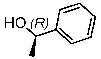 | 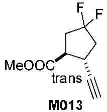 | LC-MS: [M+H]⁺ =510.20. |
| | | | | The compound contains a pair of trans isomers. |
| | | | | ¹H NMR (400 MHz, DMSO-*d₆*) δ 7.76 (s, 2H), 7.29 (s, 5H), 5.73 (s, 1H), 3.88 (s, 3H), 3.50 - 3.43 (m, 1H), 3.15 - 3.08 (m, 1H), 2.72 - 2.55 (m, 2H), 2.47 (s, 3H), 2.38 - 2.24 (m, 2H), 1.50 (s, 1H). |
| 242 | 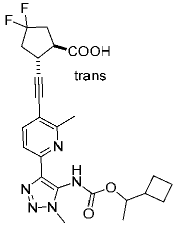 | 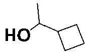 | 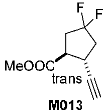 | LC-MS: [M+H]⁺ =488.20 |
| 243 | 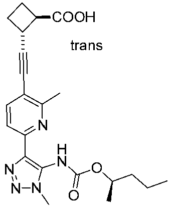 | 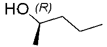 | 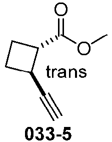 | LC-MS: [M+H]⁺-412.15 |
| | | | | The compound contains a pair of trans isomers. |
| | | | | ¹H NMR (600 MHz, DMSO-*d₆*) δ 9.51 (s, 1H), 7.72 (q, J = 8.4 Hz, 2H), 4.68 (s, 1H), 3.86 (s, 3H), 2.49 (s, 3H), 1.70 - 1.67 (m, 1H), 1.61 - 1.59 (m, 1H), 1.43 - 0.98 (m, 7H), 0.95 - 0.66 (m, 5H). |
| 245 | 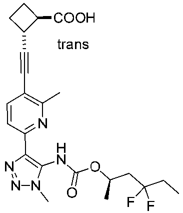 | 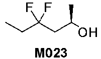 | 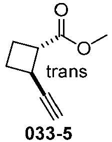 | LC-MS: [M+H]⁺ =476.15. |
| | | | | The compound contains a pair of trans isomers. |
| | | | | ¹H NMR (400 MHz, DMSO-*d₆*) δ9.65 (s, 1H), 7.83 - 7.67 (m, 2H), 4.98 (s, 1H), 3.88 (s, 3H), 3.44 (dd, *J =* 8.0,8.0 Hz, 1H), 3.19 - 3.13 (m, 1H), 2.55 (s, 3H), 2.35 - 2.14 (m, 2H), 2.14 - 1.96 (m, 4H), 1.83 (s, 2H), 1.23 (s, 3H), 0.88 (s, 3H). |
| 247 | 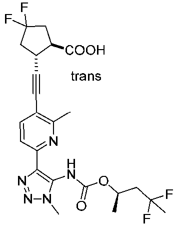 | 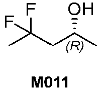 | 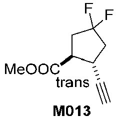 | LC-MS: [M+H]⁺=512.20. |
| | | | | The compound contains a pair of trans isomers. |
| | | | | ¹H NMR (400 MHz, DMSO-*d₆*) δ 7.79 (d, J = 8.2 Hz, 1H), 7.76 (d, J = 8.2 Hz, 1H), 4.99 (s, 1H), 3.88 (s, 3H), 3.50 - 3.43 (m, 2H), 3.01 (m, 1H), 2.72 - 2.57 (m, 1H), 2.54 (s, 3H), 2.46 - 2.13 (m, 4H), 1.60 (s, 3H), 1.23 (s, 3H). |
| 248 | 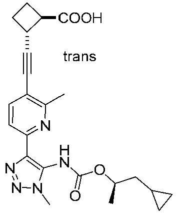 | 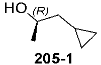 | 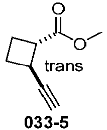 | LC-MS: [M+H]⁺ =438.15. |
| | | | | The compound contains a pair of trans isomers. |
| | | | | ¹H NMR (400 MHz, DMSO-*d₆*) δ 9.42 (s, 1H), 7.80 - 7.74 (m, 2H), 4.76 (s, 1H), 3.88 (s, 3H), 3.48 - 3.42 (m, 1H), 3.23 - 3.18 (m, 1H), 2.54 (s, 3H), 2.26 - 2.06 (m, 4H), 1.40 - -0.13 (m, 10H). |
| 260 | 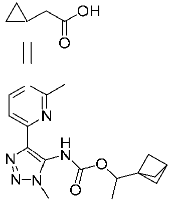 | 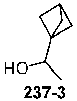 | 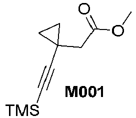 | LC-MS: [M+H]⁺ =450.05. |
| | | | | ¹H NMR (400 MHz, DMSO-*d₆*) δ 7.73 - 7.63 (m, 2H), 4.73 - 4.59 (m, 1H), 3.85 (s, 3H), 2.60 - 2.51 (m, 3H), 2.32 - 2.18 (m, 2H), 1.75 - 1.59 (m, 6H), 1.32 - 0.76 (m, 8H). |
| 260-B | 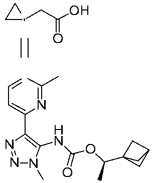 | 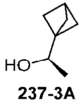 | 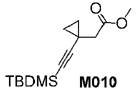 | LC-MS: [M+H]⁺ =450.15. |
| | | | | ¹H NMR (400 MHz, DMSO-*d₆*) δ 12.23 (s, 1H), 9.45 (s, 1H), 7.75 - 7.68 (m, 2H), 4.65 (s, 1H), 3.88 (s, 3H), 2.52 (s, 3H), 2.46 (s, 2H), 1.65 (s, 6H), 1.23 - 0.80 (m, 8H). |
| 263 | 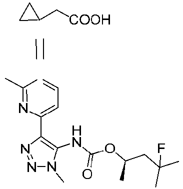 | 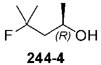 | 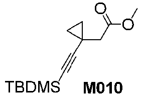 | LC-MS: [M+H]⁺ =458.2. |
| | | | | ¹H NMR (400 MHz, DMSO-*d₆*) δ 9.44 (s, 1H), 7.69 (q, *J* = 8.2 Hz, 2H), 4.91 (s, 1H), 3.85 (s, 3H), 2.49 (s, 3H), 2.42 (s, 2H), 2.13 - 1.53 (m, 2H), 1.48 - 1.06 (m, 9H), 1.02 (dd, *J =* 6.6, 4.4 Hz, 2H), 0.92 (dd, *J* = 6.6, 4.4 Hz, 2H). |
| 264 | 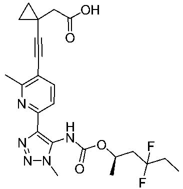 | 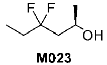 | 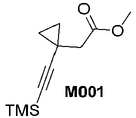 | LC-MS: [M+H]⁺ =476.15. |
| | | | | ¹H NMR (400 MHz, DMSO-*d₆*) δ 7.74 - 7.68(m, 2H), 4.98 (s, 1H), 3.87 (s, 3H), 2.51 (s, 3H), 2.39 (s, 3H), 2.09 (s, 2H), 1.84 (s, 2H), 1.23 (s, 2H), 1.02 - 0.99 (m, 2H), 0.98 - 0.91 (m, 2H), 0.87 (s, 3H). |
| 265 | 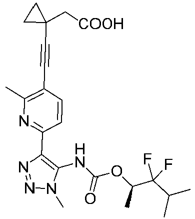 | 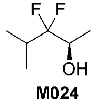 | 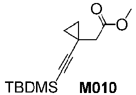 | LC-MS: [M+H]⁺ =476.15. |
| | | | | ¹H NMR (400 MHz, DMSO-*d₆*) δ 12.20 (s, 1H), 9.86 (s, 1H), 7.75 (d, J = 8.2 Hz, 1H), 7.70 (d, J = 8.2 Hz, 1H), 5.07 (s, 1H), 3.89 (s, 3H), 2.51 (s, 3H), 2.45 (s, 2H), 1.49 - 1.14 (m, 4H), 1.06 (m, 3H), 1.01 - 0.81 (m, 7H). |
| 273 | 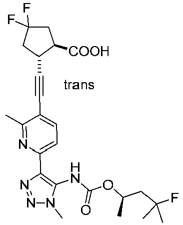 | 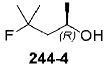 | 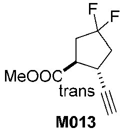 | LC-MS: [M+H]⁺ =508.20. |
| | | | | The compound contains a pair of trans isomers. |
| | | | | ¹H NMR (400 MHz, DMSO-*d₆*) δ 7.86 - 7.68 (m, 2H), 4.94 (s, 1H), 3.87 (s, 3H), 3.46 (d, *J* = 8.8 Hz, 1H), 3.04 (d, *J* = 8.8 Hz, 1H), 2.72 - 2.56(m, 2H), 2.54 (s, 3H), 2.39 - 2.25(m, 2H), 1.82 (s, 2H), 1.27 (s, 9H). |
| 274 | 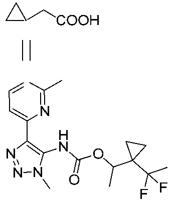 | 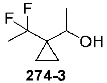 | 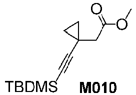 | LC-MS: [M+H]⁺ =488.20. |
| | | | | ¹H NMR (400 MHz, DMSO-*d₆*)δ 7.70 (dd, *J* = 17.5, 8.0 Hz, 2H), 4.75 (s, 1H), 3.86 (s, 3H), 2.50 (s, 3H), 2.41 (s, 2H), 1.88 - 1.37 (m, 3H), 1.31 - 1.06 (m, 3H), 1.01 (s, 2H), 0.92 (s, 2H), 0.85 - 0.38 (m, 4H). |
| 275 | 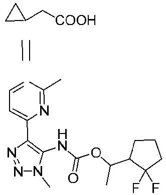 | 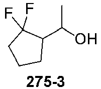 | 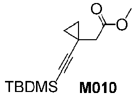 | LC-MS: [M+H]⁺=488.20. |
| | | | | ¹H NMR (400 MHz, DMSO-*d₆*) δ 9.41 (s, 1H), 7.77 - 7.67 (m, 2H), 4.85 (s, 1H), 3.87 (d, *J* = 4.8 Hz, 3H), 2.45 (s, 2H), 2.04 - 1.95 (m, 2H), 1.66 (s, 2H), 1.46 (d, *J* = 7.2 Hz, 1H), 1.33 - 1.12 (m, 8H), 1.06 (m, 2H), 0.95 (m, 2H). |
| 278 | 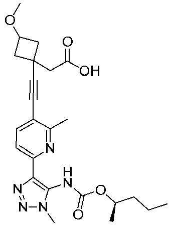 | 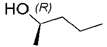 | 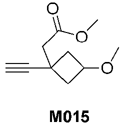 | LC-MS: [M+H]⁺ =470.25. |
| | | | | The compound contains cis-trans isomers. |
| | | | | ¹H NMR (400 MHz, DMSO-*d₆*) δ9.88 (s, 1H), 7.81 - 7.66 (m, 2H), 4.70 (s, 1H), 4.14 - 3.93 (m, 1H), 3.88 (s, 3H), 3.15 (d, *J* = 8.0 Hz, 3H), 2.72 - 2.59 (m, 4H), 2.53 (d, *J* = 8.0 Hz, 3H), 2.30 - 2.06 (m, 2H), 1.66 - 1.00 (m, 7H), 0.84 (s, 3H). |
| 287 | 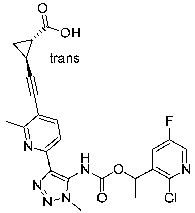 | 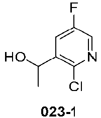 | 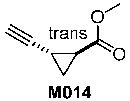 | LC-MS: [M+H]⁺ =499.00. |
| 288 | 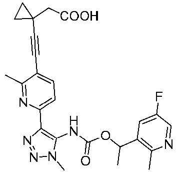 | 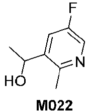 | 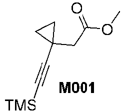 | LC-MS [M+H]⁺ =493.05. |
| | | | | ¹H NMR (400 MHz, DMSO-*d₆*) δ 8.35 (s, 1H), 7.74-7.64 (m, 3H), 5.83 (s, 1H), 3.88 (s, 3H), 2.46 (s, 3H), 2.40 (s, 5H), 1.45 (s, 3H), 1.01 (s, 2H), 0.95 (s, 2H). |
| 289 | 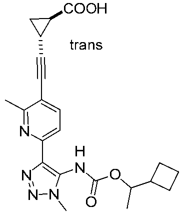 | 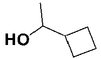 | 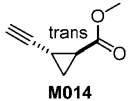 | LC-MS: [M+H]⁺=424.10. |
| | | | | ¹H NMR (400 MHz, DMSO-*d₆*) δ 9.25 (s, 1H), 7.77 (dd, J = 18.0, 8.0 Hz, 2H), 4.66 (s, 1H), 3.88 (s, 3H), 2.53 (s, 3H), 2.37 - 2.33 (m, 2H), 2.03 - 1.67 (m, 7H), 1.36 - 1.25 (m, 2H), 1.05 (s, 3H). |
| 290 | 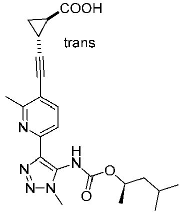 | 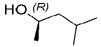 | 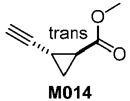 | LC-MS: [M+H]⁺=426.10. |
| | | | | The compound contains a pair of trans isomers. |
| | | | | ¹H NMR (400 MHz, DMSO-*d₆*) δ 9.44 (s, 1H), 7.70 (q, J = 8.0 Hz, 2H), 4.76 - 4.71 (m, 1H), 3.81 (s, 3H), 2.49 (s, 3H), 1.67 - 1.62 (m, 1H), 1.59 - 1.46 (m, 2H), 1.36 (s, 1H), 1.24 - 1.04 (m, 5H), 0.90 - 0.73 (m, 7H). |
| 291 | 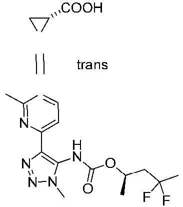 | 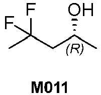 | 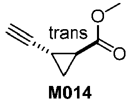 | LC-MS: [M+H]⁺= 448.15. |
| | | | | The compound contains a pair of trans isomers. |
| | | | | ¹H NMR (400 MHz, DMSO-*d₆*) δ 9.51 (s, 1H), 7.80 (d, *J* = 8.2 Hz, 1H), 7.75 (d, *J* = 8.2 Hz, 1H), 4.98 (s, 1H), 3.87 (s, 3H), 2.53 (s, 3H), 2.09 - 2.03 (m, 1H), 2.01 - 1.95 (m, 1H), 1.60 (s, 4H), 1.41 - 1.29 (m, 3H), 1.23 (s, 1H), 1.16 (s, 2H). |
| 292 | 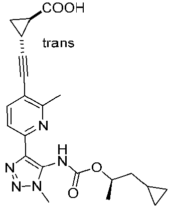 | 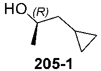 | 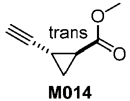 | LC-MS: [M+H]⁺ =424.15. |
| | | | | The compound contains a pair of trans isomers. |
| | | | | ¹H NMR (400 MHz, DMSO-*d₆*) δ 9.37 (s, 1H), 7.80 - 7.73 (m, 2H), 4.75 (s, 1H), 3.88 (s, 3H), 2.52 (s, 3H), 2.06 - 1.93 (m, 2H), 1.46 - 1.03 (m, 7H), 0.63 (s, 1H), 0.35 (s, 2H), 0.00 (s, 2H). |
| 293 | 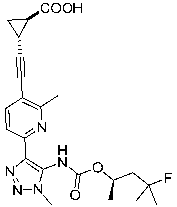 | 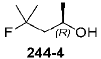 | 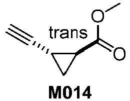 | LC-MS: [M+H]⁺ =444.15. |
| | | | | The compound contains a pair of trans isomers. |
| | | | | ¹H NMR (400 MHz, DMSO-*d₆*) δ 9.48 (s, 1H), 7.75 - 7.68 (m, 2H), 4.92 (s, 1H), 3.84 (s, 3H), 2.49 (s, 3H), 1.94 - 1.70 (m, 2H), 1.68 - 1.50 (m, 2H), 1.37 - 1.15 (m, 8H), 1.10 - 0.81 (m, 3H). |
| 294 | 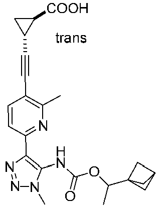 | 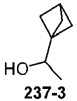 | 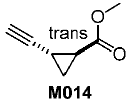 | LC-MS: [M+H]⁺ =436.10. |
| 295 | 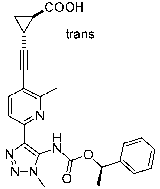 | 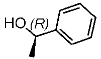 | 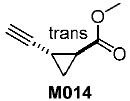 | LC-MS: [M+H]⁺ =446.20 |
| | | | | The compound contains a pair of trans isomers. |
| | | | | ¹H NMR (400 MHz, DMSO-*d₆*) δ 7.77 - 7.72 (m, 2H), 7.29 (s, 5H), 5.72 (s, 1H), 3.87 (s, 3H), 2.45 (s,3H), 1.98 - 1.90 (m, 2H), 1.47 (s, 2H), 1.33 - 1.19 (m, 3H). |
| 296 | 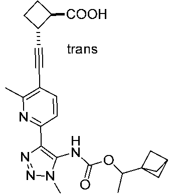 | 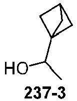 | 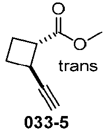 | LC-MS:[M+H]⁺=450.20. |
| 297 | 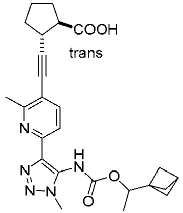 | 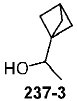 | 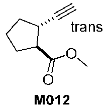 | LC-MS: [M+H]⁺ =464.15. |
| 298 | 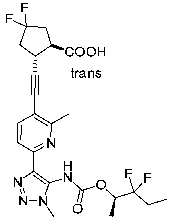 | 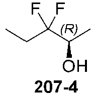 | 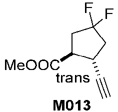 | LC-MS: [M+H]⁺ =512.15. |
| | | | | The compound contains a pair of trans isomers. |
| | | | | ¹H NMR (400 MHz, DMSO-*d₆*)δ 12.86 (s, 1H), 9.92 (s, 1H), 7.80 (d, *J* = 12.0 Hz, 2H), 4.99 (s, 1H), 3.90 (s, 3H), 3.46 (dd, *J =* 12.0, 8.0 Hz, 2H), 3.14 (dd, *J* = 12.0,8.0 Hz, 1H), 2.77 - 2.58 (m, 2H), 2.54 (s, 3H), 2.41 - 2.23 (m, 2H), 1.99 (s, 1H), 1.50 - 1.15 (m, 3H), 1.10 - 0.78 (m, 3H). |
| 299 | 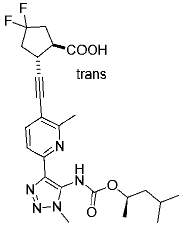 | 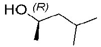 | 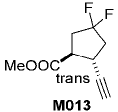 | LC-MS: [M+H]⁺ =490.20. |
| | | | | The compound contains a pair of trans isomers. |
| | | | | ¹H NMR (400 MHz, DMSO-*d₆*) δ 9.43 (s, 1H), 7.78 (dd, *J* = 8.0,8.0 Hz, 2H), 4.77 (s, 1H), 3.88 (s, 3H), 3.46 (dd, *J* = 12.0, 8.0 Hz, 1H), 3.10 (dd, *J* =8.0,8.0 Hz, 1H), 2.75 - 2.59 (m, 1H), 2.54 (s, 3H), 2.38 - 2.24 (m, 2H), 1.34 (s, 2H), 1.18 (s, 4H), 0.83 (s, 7H). |
| 300 | 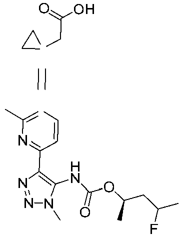 | 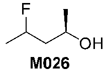 | 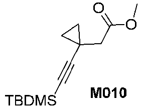 | LC-MS: [M+H]⁺ =444.05. |
| | | | | ¹H NMR (400 MHz, DMSO-*d₆*) δ7.73 (d, *J* = 8.0 Hz, 1H), 7.63 (d, *J* = 8.0 Hz, 1H), 4.81 (dd, *J =* 6.0, 5.6 Hz, 1H), 3.82 (s, 3H), 2.18 (s, 2H), 1.77 - 1.68 (m, 1H), 1.60 (s, 3H), 1.31 - 1.07 (m, 6H), 0.97 - 0.75 (m, 6H). |
| 301 | 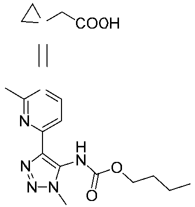 | 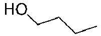 | 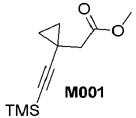 | LC-MS: [M+H]⁺ = 412.10. |
| | | | | ¹H NMR (400 MHz, DMSO-*d₆*) δ 9.43 (s, 1H), 7.75 (d, *J* = 8.0 Hz, 2H), 7.70 (d, *J* = 8.0 Hz, 2H), 4.01 (s, 4H), 3.88 (s, 6H), 2.51 (s, 9H), 2.45 (s, 4H), 1.47 (s, 3H), 1.23 (s, 3H), 1.05 (m, 4H), 0.95 (m, 4H), 0.83 (s, 5H). |
| 302 | 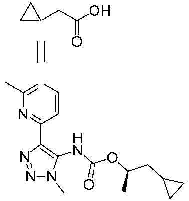 | 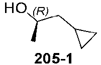 | 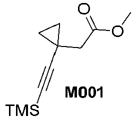 | LC-MS [M+H]⁺= 438.10. |
| | | | | ¹H NMR (400 MHz, DMSO-*d₆*) δ 7.73 - 7.67 (m, 2H), 4.75 (s, 1H), 3.87 (s, 3H), 2.50 (s, 3H), 2.42 (s, 2H), 1.51 - 1.18 (m, 5H), 1.03 - 0.97 (m, 2H), 0.94 - 0.91 (m, 2H), 0.62 (s, 1H), 0.34 (s, 2H), -0.01 (s, 2H). |
| 303 | 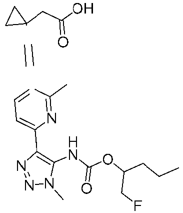 | 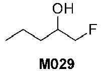 | 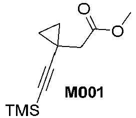 | LC-MS: [M+H]⁺ =444.00. |
| | | | | ¹H NMR (400 MHz, DMSO-*d₆*) δ 9.68 (s, 1H), 7.76 (d, *J* = 8.0 Hz, 1H), 7.70 (d, *J* = 8.0 Hz, 1H), 4.85 - 4.44 (m, 1H), 4.34 - 4.03 (m, 2H), 3.90 (s, 3H), 2.51 (s, 3H), 2.46 (s, 2H), 1.63 - 1.20 (m,4 H), 1.10 - 1.02 (m, 2H), 0.98 - 0.93 (m, 2H), 0.86 (d, *J* = 4.3 Hz, 3H). |
| 304 | 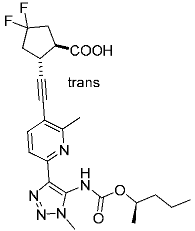 | 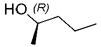 | 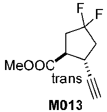 | LC-MS: [M+H]⁺ =476.15. |
| | | | | The compound contains a pair of trans isomers. |
| | | | | ¹H NMR (400 MHz, DMSO-*d₆*) δ7.77 (dd, *J* = 4.0, 8.0 Hz, 2H), 4.71 (s, 1H), 3.88 (s, 3H), 3.46 (dd, *J* = 4.0, 4.0 Hz, 2H), 3.00 (dd, *J* = 8.0,8.0 Hz, 1H), 2.69 - 2.60 (m, 1H), 2.54 (s, 3H), 2.37 - 2.22 (m, 2H), 1.37 (s, 2H), 1.23 (s, 4H), 0.85 (s, 4H). |
| 305 | 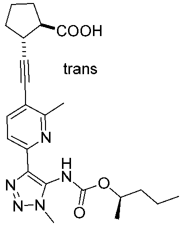 | 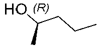 | 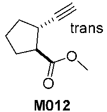 | LC-MS: [M+H]⁺ =440.15. |
| | | | | The compound contains a pair of trans isomers. |
| | | | | ¹H NMR (400 MHz, DMSO-*d₆*) δ 9.37 (s, 1H), 7.82 - 7.65 (m, 2H), 4.69 (s, 1H), 3.86 (s, 3H), 3.14 (dd, *J* = 15.6, 7.8 Hz, 1H), 2.75 (dd, *J* = 16.2, 8.2 Hz, 1H), 2.51 (s, 3H), 2.12 - 1.96 (m, 2H), 1.77 - 1.62 (m, 4H), 1.53 - 0.95 (m, 7H), 0.81 (s, 3H). |
| 306 | 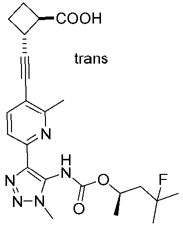 | 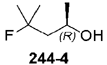 | 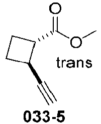 | LC-MS: [M+H]⁺ =458.15. |
| | | | | The compound contains a pair of trans isomers. |
| | | | | ¹H NMR (400 MHz, DMSO-*d₆*) δ 9.48 (s, 1H), 7.77 (q, *J* = 8.0 Hz, 2H), 4.93 (s, 1H), 3.87 (s, 3H), 3.49 - 3.40 (m, 1H), 3.22 - 3.10 (m, 1H), 2.55 (s, 3H), 2.26 - 2.02 (m, 4H), 1.95 - 1.60 (m, 2H), 1.39 - 1.08 (m, 9H). |
| 307 | 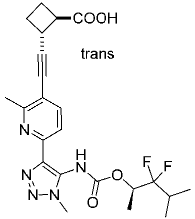 | 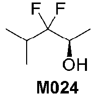 | 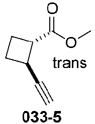 | LC-MS: [M+H]⁺= 476.20. |
| | | | | The compound contains a pair of trans isomers. |
| | | | | ¹H NMR (400 MHz, DMSO-*d₆*) δ 7.78 (d, *J* = 8.2 Hz, 1H), 7.53 (d, *J* = 8.2 Hz, 1H), 5.10 - 4.96 (m, 1H), 3.62 (s, 3H), 2.83 - 2.74 (m, 1H), 2.52 (s, 3H), 2.30 (d, *J =* 19.2 Hz, 1H), 2.14 - 1.89 (m, 4H), 1.59 (s, 1H), 1.18 (d, *J* = 6.2 Hz, 3H), 0.97 (t, *J* = 6.4 Hz, 6H). |
| 308 | 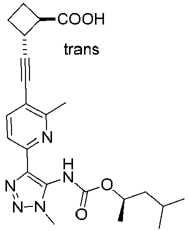 | 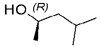 | 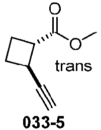 | LC-MS: [M+H]⁺ =440.20. |
| | | | | The compound contains a pair of trans isomers. |
| | | | | ¹H NMR (400 MHz, DMSO-*d₆*) δ9.41 (s, 1H), 7.77 (dd, *J* = 8.0,8.0 Hz, 2H), 4.77 (s, 1H), 3.88 (s, 3H), 3.43 - 3.41 (m, 1H),3.15 (d, *J* = 8.0 Hz, 1H), 2.55 (s, 3H), 2.27 - 2.17(m, 1H), 2.15 - 1.98 (m, 3H), 1.75 - 0.96 (m, 6H), 0.89 (s, 6H). |
| 309 | 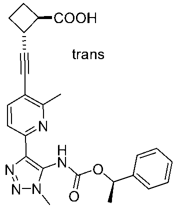 | 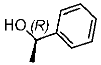 | 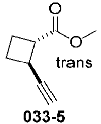 | LC-MS: [M+H]⁺ =460.15. |
| | | | | ¹H NMR (400 MHz, DMSO-*d₆*) δ 7.77 - 7.73 (m, 2H),7.29 (s, 5H), 5.73 (s, 1H), 3.87 (s, 3H), 3.44 (d, *J* = 8.8 Hz, 1H), 3.17 (d, *J* = 8.8 Hz, 1H),2.47(s,3H),2.23 - 2.10 (m, 2H), 2.10 - 2.06 (m, 3H), 1.48 (s, 3H). |
| 310 | 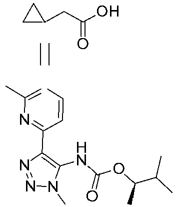 | 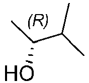 | 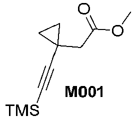 | LC-MS: [M+H]⁺= 426.10. |
| | | | | ¹H NMR (400 MHz, DMSO-*d₆*) δ 9.41 (s, 1H), 7.74 (d, *J* = 8.2 Hz, 1H), 7.69 (d, *J* = 8.2 Hz, 1H), 4.53 (s, 1H), 3.88 (s, 3H), 2.51 (s, 3H), 2.44 (s, 2H), 1.73 (s, 1H), 1.22 - 0.67 (m, 13H). |
| 311 | 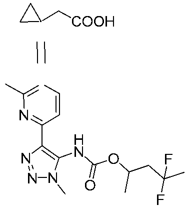 | 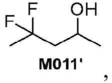 its preparation refers to the method of Preparation Example 9 | 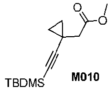 | LC-MS: [M+H]⁺= 462.05. |
| | | | | ¹H NMR (400 MHz, DMSO-*d₆*) δ 7.71 (s, 2H), 5.16 (d, *J* = 4.4 Hz, 1H), 3.99 (s, 3H), 2.61 (s, 3H), 2.47 (s, 2H), 2.35 - 2.02 (m, 2H), 1.61 (t, *J* = 18.4 Hz, 3H), 1.32 (s, 3H), 1.13 - 1.08 (m, 2H), 1.00 - 0.95 (m, 2H). |
| 312 | 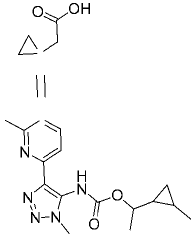 | 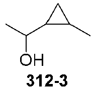 | 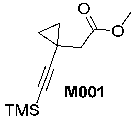 | LC-MS: [M+H]⁺ =474.10. |
| | | | | ¹H NMR (400 MHz, DMSO-*d₆*) δ 9.39 (s, 1H), 7.76 - 7.52 (m, 2H), 4.34 - 4.17 (m, 1H), 3.85 (s, 3H), 2.53 - 2.43 (m, 3H), 2.43 (s, 2H), 1.47 - 0.78 (m, 11H), 0.67 - 0.01 m, 3H). |
| 313 | 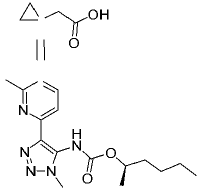 | 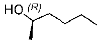 | 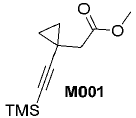 | LC-MS: [M+H]⁺ =440.10. |
| | | | | ¹H NMR (400 MHz, DMSO-d6)¹H NMR (400 MHz, dmso) δ 9.39 (s, 1H), 7.71 (q, *J* = 8.2 Hz, 2H), 4.68 (s, 1H), 3.88 (s, 3H), 2.51 (s, 3H), 2.42 (s, 2H), 1.70 - 1.33 (m, 2H), 1.32 - 1.07 (m, 7H), 1.05 - 0.99 (m, 2H), 0.96 - 0.91 (m, 2H), 0.81 (s, 3H). |
| 314 | 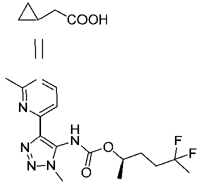 | 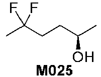 | 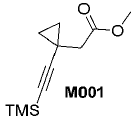 | LC-MS: [M+H]⁺= 476.25. |
| | | | | ¹H NMR (400 MHz, DMSO-*d₆*) δ 7.73 (d, *J* = 8.0 Hz, 1H), 7.68 (d, *J* = 8.0 Hz, 1H), 4.71 (s, 1H), 3.87 (s, 3H), 2.50 - 2.47 (m, 3H), 2.42 (s, 2H), 2.09 - 1.34 (m, 7H), 1.30 - 1.06 (m, 3H), 1.03 (m, 2H), 0.93 (m, 2H). |
| 315 | 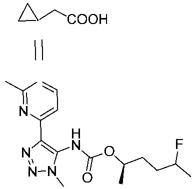 | 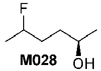 | 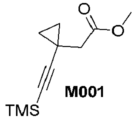 | LC-MS: [M+H]⁺=458.20. |
| | | | | ¹H NMR (400 MHz, DMSO-*d₆*) δ 7.73 (d, *J* = 8.2 Hz, 1H), 7.63 (d, *J* = 8.2 Hz, 1H), 4.76 - 4.49 (m, 2H), 3.83 (s, 3H), 2.51 (s, 3H), 2.18 (s, 2H), 1.57 (d, *J* = 25.6 Hz, 4H), 1.24 (d, *J* = 5.0 Hz, 2H), 1.21 - 1.10 (m, 4H), 0.97 - 0.91 (m, 2H), 0.91 - 0.85 (m, 2H). |
| 316 | 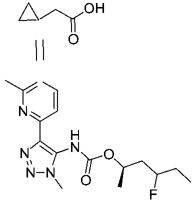 | 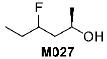 | 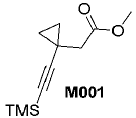 | LC-MS: [M+H]⁺ =458.20. |
| | | | | ¹H NMR (400 MHz, DMSO-*d₆*) δ 7.75 - 7.68 (m, 2H), 4.84 (s, 1H), 3.88 (s, 1H), 3.01 (s, 1H), 2.51 (s, 3H), 2.44 (s, 2H), 1.75 - 1.53 (m, 2H), 1.21 (s, 3H), 1.05 - 1.03 (m, 2H), 0.96 - 0.93 (m, 2H), 0.85 (s, 3H). |
| 317 | 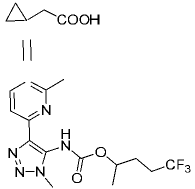 | 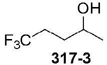 | 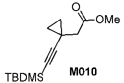 | LC-MS: [M+H]⁺=480.20. |
| | | | | ¹H NMR (400 MHz, DMSO-*d₆*) δ 9.53 (s, 1H), 7.73 (d, *J* = 8.2 Hz, 1H), 7.63 (d, *J* = 8.2 Hz, 1H), 4.73 (m, 1H), 3.83 (d, *J* = 6.0 Hz, 3H), 2.36 - 1.94 (m, 5H), 1.77 - 1.58 (m, 2H), 1.23 (s, 2H), 1.17 (s, 3H), 0.94 (p, *J* = 5.2 Hz, 2H), 0.89 - 0.85 (m, 2H). |
| 318 | 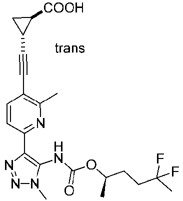 | 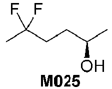 | 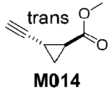 | LC-MS: [M+H]⁺ =462.20. |
| | | | | The compound contains a pair of trans isomers. |
| | | | | ¹H NMR (400 MHz, DMSO-*d₆*) ¹H NMR (400 MHz, DMSO-*d₆*) δ 9.46 (s, 1H), 7.81 - 7.75 (m, 2H), 4.71 (s, 1H), 3.89 (s, 3H), 2.53 (s, 3H), 2.09 - 2.03 (m, 1H), 1.99 - 1.95 (m, 1H), 1.55 - 1.23 (m, 12H). |
| 319 | 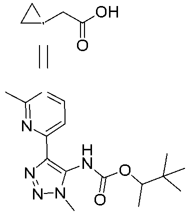 | 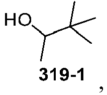 its preparation refers to the preparation method of compound 023-1 in Example 23 | 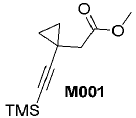 | LC-MS: [M+H]⁺ =440.15. |
| | | | | ¹H NMR (400 MHz, DMSO-*d₆*) δ9.40 (s, 1H), 7.72 (dd, *J =* 8.0,8.0 Hz, 2H), 4.47 (s, 1H), 3.88 (s, 3H), 2.51 (s, 3H), 2.44 (s, 2H), 1.39-1.10 (m, 2H), 1.05 (dd, *J* = 4.0, 4.0 Hz, 3H), 1.03 - 0.95 (m, 2H), 0.92 (dd, *J =* 4.0, 4.0 Hz, 9H). |
| 320 | 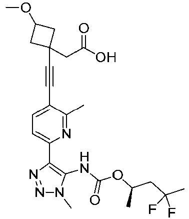 | 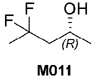 | 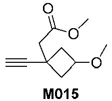 | LC-MS: [M+H]⁺ =506.20. |
| | | | | The compound contains cis-trans isomers. |
| | | | | ¹H NMR (400 MHz, DMSO-*d₆*) δ7.78 -7.71 (m, 2H), 4.99 (d, *J =* 4.0 Hz, 1H), 4.12 - 3.95 (m, 1H), 3.87 (s, 3H), 3.14 (d, *J =* 8.0 Hz, 3H), 2.60 (d, *J =* 8.0 Hz, 3H), 2.53 (d, *J* = 8.0 Hz, 2H), 2.27 - 2.19 (m, 1H), 2.18 - 2.10 (m, 2H), 1.88 (s, 2H), 1.75 (s, 1H), 1.59 (s, 3H), 1.24 (s, 3H). |
| 321 | 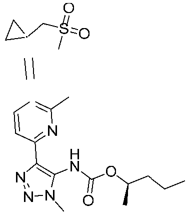 | 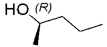 | 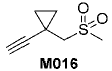 | LC-MS: [M+H]⁺ =460.20. |
| | | | | ¹H NMR (400 MHz, DMSO-*d₆*) δ 9.39 (s, 1H), 7.76 (s, 2H), 4.70 (s, 1H), 3.88 (s, 3H), 3.41 (s, 2H), 3.09 (s, 3H), 2.54 (s, 3H), 1.45 - 0.83 (m, 14H). |
| 322 | 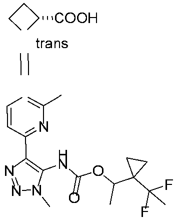 | 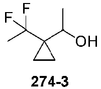 | 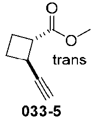 | LC-MS: [M+H]⁺ =488.15. |
| | | | | ¹H NMR (400 MHz, DMSO-*d₆*)δ 7.79 (q, *J* = 8.0 Hz, 2H), 4.79 (s, 1H), 3.89 (s, 3H), 3.45 (dd, *J* = 16.8, 8.4 Hz, 1H), 3.19 (dd, *J* = 17.6, 9.0 Hz, 1H), 2.56 (s, 3H), 2.27 - 2.19 (m, 1H), 2.13 - 2.01 (m, 3H), 1.86 - 1.47 (m, 2H), 1.36 - 0.94 (m, 4H), 0.89 - 0.45 (m, 4H). |
| 323 | 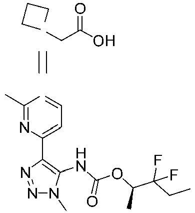 | 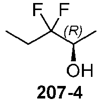 | 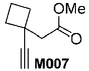 | LC-MS: [M+H]⁺=476.15 |
| | | | | ¹H NMR (400 MHz, DMSO-*d₆*) δ 9.88 (s, 1H), 7.76 - 7.71 (m, 2H), 4.96 (s, 1H), 3.86 (s, 3H), 2.68 (s, 2H), 2.50 (s, 3H), 2.36 - 2.29 (m, 2H), 2.26 - 2.19 (m, 2H), 2.10 - 1.79 (m, 4H), 1.28 (s, 3H), 0.93 (s, 3H). |
| 324 | 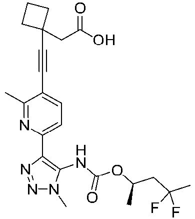 | 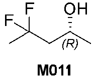 | 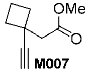 | LC-MS: [M+H]⁺=476.20 |
| | | | | ¹H NMR (400 MHz, DMSO-*d₆*) δ 9.53 (s, 1H), 7.73 (s, 2H), 7.33 - 6.96 (m, 1H), 4.95 (s, 1H), 3.85 (s, 3H), 2.68 (s, 2H), 2.51 (s, 3H), 2.36 - 2.17 (m, 5H), 2.11 - 1.88 (m, 3H), 1.57 (s, 3H), 1.20 (s, 3H). |
| 325 | 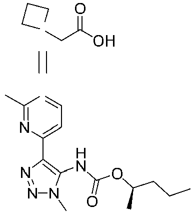 | 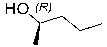 | 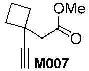 | LC-MS: [M+H]⁺=440.15. |
| | | | | ¹H NMR (400 MHz, DMSO-*d₆*) δ 12.15 (s, 1H), 9.37 (s, 1H), 7.75 - 7.68 (m, 2H), 4.68 (s, 1H), 3.85 (s, 3H), 2.67 (s, 2H), 2.50 (s, 3H), 2.36 - 2.18 (m, 4H), 2.08 - 1.89 (m, 2H), 1.46 - 1.13 (m, 7H), 0.80 (s, 3H). |
| 399 | 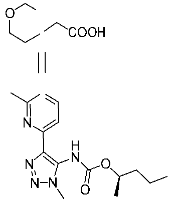 | 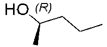 | 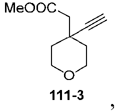 its preparation refers to the preparation method of compound M035 in Preparation Example 37 | LCMS: [M+H]⁺ =470.20. |
| | | | | ¹H NMR (400 MHz, DMSO-*d₆*) δ 7.80 - 7.72 (m, 2H), 4.67 (s, 1H), 3.85 (s, 3H), 3.78 (d, J = 10.0 Hz, 2H), 3.66 (t, J = 11.6 Hz, 2H), 2.54 (s, 3H), 2.51 (s, 2H), 1.84 (d, J = 12.8 Hz, 2H), 1.75 - 1.62 (m, 2H), 1.46 - 1.13 (m, 7H), 0.81 (s, 3H). |
| 402 | 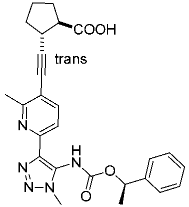 | 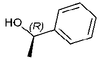 | 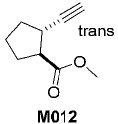 | LC-MS: [M+H]⁺ =474.10. |
| | | | | The compound contains a pair of trans isomers. |
| | | | | ¹H NMR (400 MHz, DMSO-*d₆*) δ9.63 (s, 1H), 7.74 (s, 2H), 7.29 (s, 5H), 5.73 (s, 1H), 3.87 (s, 3H), 3.19 - 3.14 (m, 1H), 2.78 (dd, *J =* 8.0, 8.0 Hz, 1H), 2.45 (s, 3H), 2.18 - 1.95 (m, 2H), 1.74 (d, *J =* 8.0 Hz, 4H), 1.36 (s, 3H). |
| 414 | 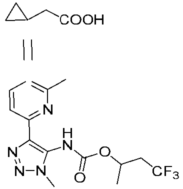 | 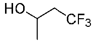 | 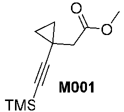 | LCMS:[M+1]⁺=466.00. |
| | | | | The compound contains a pair of enantiomers. |
| | | | | ¹H NMR (400 MHz, DMSO-*d₆*) δ 7.72 (dd, *J =* 18.8, 8.0 Hz, 2H), 5.02 (s, 1H), 3.87 (s, 3H), 2.64 (d*, J* = 18.0 Hz, 3H), 2.43 (s, 2H), 2.06 - 1.99 (m, 2H), 1.23 (s, 3H), 1.04 (s, 2H), 0.95 (s, 2H). |
| 427 | 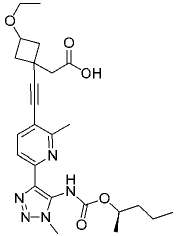 | 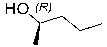 | 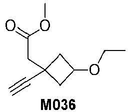 | LC-MS: [M+H]⁺ =484.20. |
| | | | | The compound contains cis-trans isomers. |
| | | | | ¹H NMR (400 MHz, DMSO-*d₆*) δ 9.42 (s, 1H), 7.77 - 7.73 (m, 2H), 4.70 (s, 1H), 4.20 -4.05 (m, 1H), 3.88 (s, 3H), 3.39 - 3.33 (m, 3H), 2.70 (s, 2H), 2.66 - 2.60 (m, 2H), 2.53 (s, 3H), 2.17 - 2.10 (m, 2H), 1.27 - 1.05 (m, 9H), 0.85 - 0.83 (m, 3H). |
| 428 | 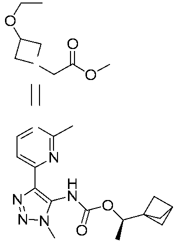 | 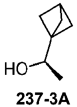 | 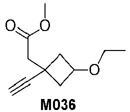 | LC-MS: [M+H]⁺ =508.20. |
| | | | | The compound contains cis-trans isomers. |
| | | | | ¹H NMR (400 MHz, DMSO-*d₆*) δ 9.47 (s, 1H), 7.81 - 7.71 (m, 2H), 4.66 (s, 1H), 4.19 - 4.04 (m, 1H), 3.88 (s, 3H), 3.40 - 3.31 (m, 2H), 2.73 -2.60 (m, 4H), 2.54 (d*, J =* 4.0 Hz, 4H), 2.30 - 2.10 (m, 2H), 1.66 (s, 6H), 1.26 - 0.96 (m, 6H). |
| 429 | 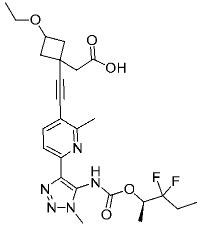 | 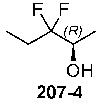 | 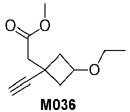 | LC-MS: [M+H]⁺=520.20. |
| | | | | The compound contains cis-trans isomers. |
| | | | | ¹H NMR (400 MHz, DMSO-*d₆*) δ 9.98 (s, 1H), 7.79 (s, 2H), 4.99 (s, 1H), 4.20 - 4.15 (m, 1H), 4.10 - 4.04 (m, 1H), 3.90 (s, 3H), 3.39 - 3.30 (m, 2H), 2.76 - 2.59 (m, 4H), 2.54 (d, *J=* 4.0 Hz, 3H), 2.32 - 2.20 (m, 1H), 2.16 - 2.10 (m, 1H), 2.01 - 1.97 (m, 1H), 1.51 - 1.16 (m, 3H), 1.11 - 1.08 (m, 3H), 0.96 (s, 3H). |
| 430 | 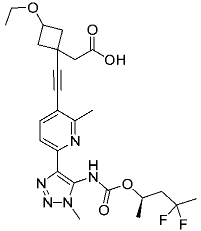 | 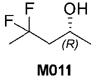 | 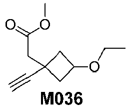 | LC-MS: [M+H]⁺=520.20. |
| | | | | The compound contains cis-trans isomers. |
| | | | | ¹H NMR (400 MHz, DMSO-*d₆*) δ 9.61 (s, 1H), 7.77 (s, 2H), 4.98 (s, 1H), 4.17 - 4.15 (m, 1H), 4.07 - 4.05 (m, 1H), 3.88 (s, 3H), 3.42 - 3.26 (m, 2H), 2.81 - 2.52 (m, 6H), 2.25 - 2.01 (m, 4H), 1.60 (s, 3H), 1.23 (s, 3H), 1.11 - 1.08 (m, 3H). |
| 431 | 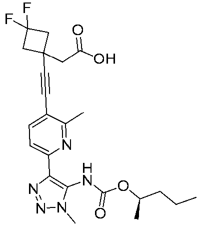 | 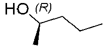 | 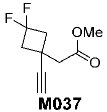 | LC-MS: [M+H]⁺=476.15. |
| | | | | ¹H NMR (400 MHz, DMSO-*d₆*) δ9.40 (s, 1H), 7.82 - 7.70 (m, 2H), 4.70 (s, 1H), 3.88 (s, 3H), 2.98 (dd, *J* = 4.0, 12.0 Hz, 4H), 2.83 (s, 2H), 2.53 (s, 3H), 1.71 - 0.96 (m, 2H), 0.83 (s, 3H). |
| 432 | 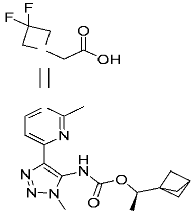 | 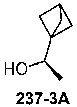 | 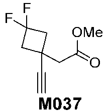 | LC-MS: [M+H]⁺ =500.15. |
| | | | | ¹H NMR (400 MHz, DMSO-*d₆*) δ 9.46 (s, 1H), 7.84 - 7.71 (m, 2H), 4.65 (s, 1H), 3.88 (s, 3H), 3.03 - 2.94 (m, 4H), 2.83 (s, 2H), 2.54 (s, 3H), 2.46 (s, 1H), 1.65 (s, 6H), 1.23 - 0.84 (m, 3H). |
| 447 | 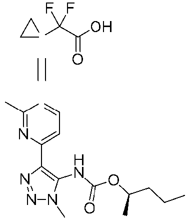 | 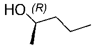 | 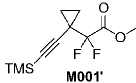 | LC-MS: [M+H]⁺ =462.10. |
| | | | | ¹H NMR (400 MHz, DMSO-*d₆*) δ8.69 (s, 1H), 7.78 - 7.64 (m, 2H), 4.70 (s, 1H), 3.88 (s, 3H), 2.53 (s, 3H), 1.75 (s, 2H), 1.41 - 1.31 (m, 3H), 1.29- 1.06 (m, 4H), 1.00 (s, 2H), 0.84 (s, 3H). |
| 448 | 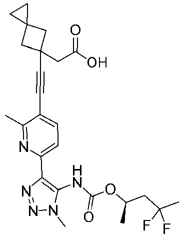 | 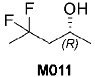 | 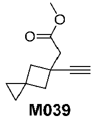 | LC-MS: [M+H]⁺ =502.15. |
| | | | | ¹H NMR (400 MHz, DMSO-*d₆*) δ 9.63 (s, 1H), 7.81 - 7.75 (m, 2H), 4.99 (s, 1H), 3.88 (s, 3H), 2.82 (s, 2H), 2.56 (s, 3H), 2.53 (d, *J* = 11.8 Hz, 2H), 2.36 (d, *J* = 11.8 Hz, 2H), 2.31 - 1.95 (m, 2H), 1.62 (s, 3H), 1.38 - 1.17 (m, 3H), 0.49 (s, 4H). |
| 449 | 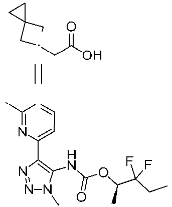 | 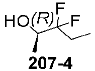 | 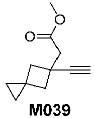 | LC-MS: [M+H]⁺=502.20. |
| | | | | ¹H NMR (400 MHz, DMSO-*d₆*) δ 9.94 (s, 1H), 7.81 - 7.75 (m, 2H), 5.00 (s, 1H), 3.90 (s, 3H), 2.82 (s, 2H), 2.55 - 2.52 (m, 5H), 2.36 (d, *J* = 8.0 Hz, 2H), 2.16 - 1.82 (m, 2H), 1.33 - 1.23 (m, 3H), 0.97 (s, 3H), 0.49 (s, 4H). |
| 464 | 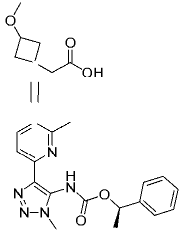 | 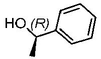 | 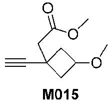 | LC-MS: [M+H]⁺ =504.20. |
| | | | | The compound contains cis-trans isomers. |
| | | | | ¹H NMR (400 MHz, DMSO-*d₆*) δ7.72 (dd, *J* = 8.0,8.0 Hz, 2H), 7.30 (s, 5H), 5.73 (s, 1H), 4.01 - 3.92 (m, 1H), 3.87 (s, 3H), 3.14 (s, 3H), 2.70 - 2.63 (m, 2H), 2.60 (s, 2H), 2.46 (s, 3H), 2.32 - 2.17 (m, 2H), 1.58 - 1.19(m, 3H). |
| 465 | 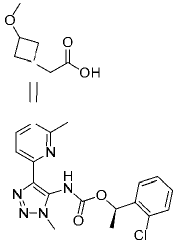 | 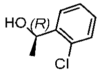 | 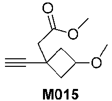 | LC-MS: [M+H]⁺ =538.25. |
| | | | | The compound contains cis-trans isomers. |
| | | | | ¹H NMR (400 MHz, DMSO-*d₆*) δ9.80 (s, 1H), 7.79 - 7.24 (m, 6H), 5.99 (s, 1H), 4.09 (dd, *J* = 8.0, 8.0 Hz, 1H), 3.89 (s, 3H), 3.16 (d, *J* = 4.0 Hz, 3H), 2.70 (d, *J* = 8.0 Hz, 2H), 2.67 - 2.61 (m, 2H), 2.46 (s, 2H), 2.29 - 2.10 (m, 2H), 1.51 (s, 2H), 1.23 (s, 2H). |
| 466 | 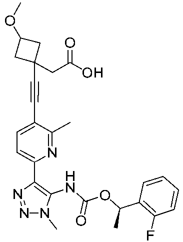 | 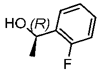 | 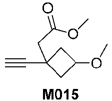 | LC-MS: [M+H]⁺ =522.25. |
| | | | | The compound contains cis-trans isomers. |
| | | | | ¹H NMR (400 MHz, DMSO-*d₆*) δ7.85 - 7.60 (m, 2H), 7.59 - 6.55 (m, 4H), 5.93 (s, 1H), 4.08 (dd, *J=* 8.0, 8.0 Hz, 1H), 3.88 (s, 3H), 3.15 (d, *J* = 8.0 Hz, 3H), 2.70 - 2.56 (m, 4H), 2.44 (d, *J= 4.0* Hz, 3H), 2.26 - 2.09 (m, 2H) , 1.72- 1.15 (m, 3H). |
| 478 | 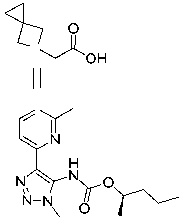 | 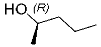 | 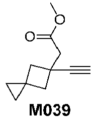 | LCMS:[M+1]⁺=466.15. |
| | | | | ¹H NMR (600 MHz, DMSO-*d₆*) δ 9.45 (s, 1H), 7.77 (s, 2H), 4.71 (s, 1H), 3.89 (s, 3H), 2.81 (s, 2H), 2.55 (s, 3H), 2.52 (d, *J =* 12.0 Hz, 2H), 2.36 (d, *J =* 12.0 Hz, 2H), 1.23 (s, 7H), 0.85 (s, 3H), 0.49 (s, 4H). |
| 479 | 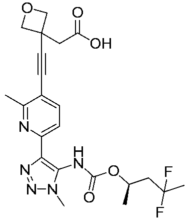 | 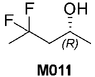 | 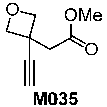 | LCMS: [M+H]⁺ =478.15. |
| | | | | ¹H NMR (400 MHz, DMSO-*d₆*) δ 9.53 (s, 1H), 7.74 (s, 2H), 4.96 (s, 1H), 4.64 (dd, J = 14.4, 5.6 Hz, 4H), 3.84 (s, 3H), 2.61 (s, 2H), 2.52 (s, 3H), 2.22 - 1.99 (m, 2H), 1.63 - 1.52 m, 3H), 1.28 - 1.14 (m, 3H). |
| 481 | 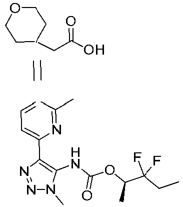 | 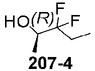 | 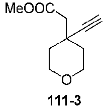 | LCMS: [M+H]⁺ =506.15. |
| | | | | ¹H NMR (400 MHz, DMSO-*d₆*) δ 9.91 (s, 1H), 7.77 (s, 2H), 4.97 (s, 1H), 3.88 (s, 3H), 3.81 - 3.78 (m, 2H), 3.69 - 3.64 (m, 2H), 2.54 - 2.52 (m, 5H), 1.99 - 1.83 (m, 4H), 1.68 - 1.64 (m, 2H), 1.29 - 1.21 (m, 3H), 0.94 (s, 3H). |
| 509 | 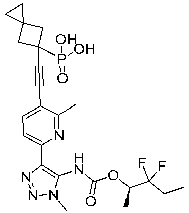 | 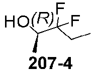 | 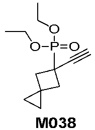 | LCMS: [M+H]⁺ =524.20. |
| | | | | ¹H NMR (600 MHz, DMSO-*d₆*) δ 9.90 (s, 1H), 7.78 - 7.76 m, 2H), 4.97 (s, 1H), 3.88 (s, 3H), 2.78 (dd, J = 16.8, 12.0 Hz, 2H), 2.62 - 2.52 (m, 5H), 2.36 (t, J = 11.4 Hz, 2H), 2.05 - 1.96 (m, 2H), 1.31 - 1.21 (m, 3H), 0.99 - 0.96 (m, 3H), 0.56 - 0.49 (m, 2H), 0.46 - 0.39 (m, 2H). |
| 510 | 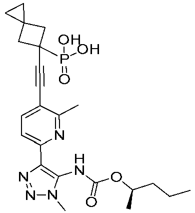 | 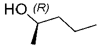 | 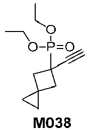 | LCMS: [M+H]⁺ =488.20. |
| | | | | ¹H NMR (400 MHz, DMSO-*d₆*) δ 9.39 (s, 1H), 7.79 - 7.70 (m, 2H), 5.80 (s, 1H), 4.68 (s, 1H), 3.85 (s, 3H), 2.94 (dd, J = 12.0, 6.8 Hz, 1H), 2.78 (dd, J = 16.8, 12.0 Hz, 1H), 2.66 - 2.48 (m, 4H), 2.38 - 2.29 (m, 1H), 2.09 - 1.99 (m, 1H), 1.42 - 1.13 (m, 6H), 0.97 (t, J = 7.6 Hz, 2H), 0.81 (s, 3H), 0.57 - 0.48 (m, 1H), 0.46 - 0.36 (m, 1H). |

The compounds in the following table were prepared by referring to the preparation methods of Example 5, Example 6, Example 8, Example 10, Example 13, Example 23, Example 44, Example 45, Example 48, Example 66, *etc.* Taking Example 5 as an example, compound 005-3 was replaced with the corresponding Reactant 1 in the following table, and the starting material (R)-1-(2-chlorophenyl)ethanol was replaced with the corresponding Reactant 2 in the following table:

| No. | Compound structure | Reactant 1 | Reactant 2 | Characterization |
|---|---|---|---|---|
| 229 | 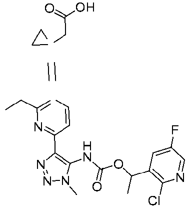 | 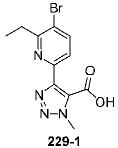 | 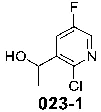 | LC-MS: [M+H]⁺ =527.00. |
| 232 | 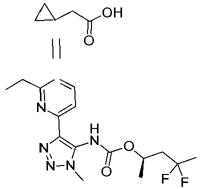 | 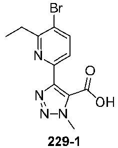 | 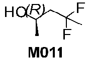 | LC-MS: [M+H]⁺=476.20. |
| | | | | ¹H NMR (400 MHz, DMSO-*d₆)* δ9.50 (s, 1H), 7.72 (dd, *J* = 8.0, 8.0 Hz, 2H), 4.99 (s, 1H), 3.88 (s, 3H), 2.87 (dd, *J =* 8.0, 4.0 Hz, 2H), 2.37 (s, 2H), 1.60 (s, 3H), 1.23 (s, 8H), 0.97 (d, *J* = 16.0 Hz, 4H). |
| 233 | 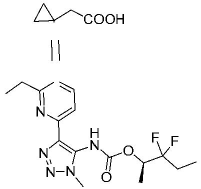 | 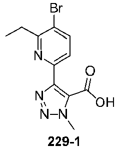 | 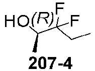 | LC-MS: [M+H]⁺ =476.15. |
| | | | | ¹H NMR (400 MHz, DMSO-*d₆*) δ9.87 (s, 1H), 7.73 (dd, *J =* 8.4, 8.0 Hz, 2H), 5.00 (s, 1H), 3.90 (s, 3H), 2.87 (dd, *J =* 7.6, 7.2 Hz, 2H), 2.45 (s, 2H), 2.18 - 1.63 (m, 2H), 1.50 - 1.15 (m, 6H), 1.16 - 1.00 (m, 3H), 0.95 (dd, *J =* 4.0, 5.2 Hz, 4H). |
| 258 | 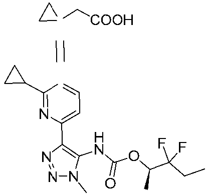 | 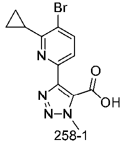 | 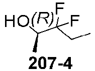 | LC-MS: [M+H]⁺ =488.15. |
| | | | | ¹H NMR (400 MHz, DMSO-*d*₆) δ 12.19 (s, 1H), 9.78 (s, 1H), 7.67 (s, 2H), 5.04 (s, 1H), 3.87 (s, 3H), 2.62 - 2.54 (m, 1H), 2.45 (s, 2H), 2.10 - 1.89 (m, 2H), 1.46 - 1.21 (m, 3H), 1.13 - 0.88 (m, 11H). |
| 259 | 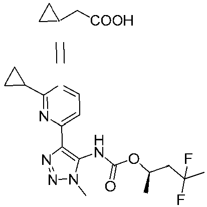 | 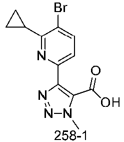 | 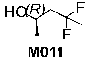 | LC-MS: [M+H]⁺ =488.15. |
| | | | | ¹H NMR (400 MHz, DMSO-*d*₆) δ 12.25 (s, 1H), 9.40 (s, 1H), 7.87 - 7.58 (m, 2H), 5.05 (s, 1H), 3.86 (s, 3H), 2.62 - 2.54 (m, 1H), 2.45 (s, 2H), 2.38 -2.10 (m, 2H), 1.83 - 1.47 (m, 3H), 1.45 - 1.19 (m, 3H), 1.13 - 0.99 (m, 4H), 0.98 - 0.92 (m, 4H). |
| 267 | 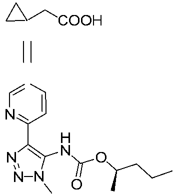 | 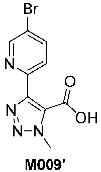 | 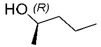 | LC-MS [M+H]⁺ = 412.10. |
| | | | | ¹H NMR (400 MHz, DMSO-*d*₆) δ 9.44 (s, 1H), 8.47 (s, 1H), 7.86 (dd, *J* = 44.4, 8.0 Hz, 2H), 4.70 (s, 1H), 3.88 (s, 3H), 2.44 (s, 2H), 1.43 - 1.16 (m, 6H), 1.09 - 0.67 (m, 8H). |
| 276 | 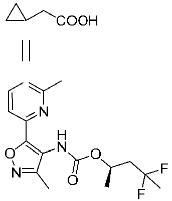 | 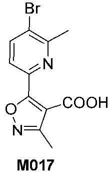 | 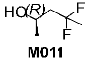 | LC-MS: [M+H]⁺=462.15. |
| | | | | ¹H NMR (400 MHz, DMSO-*d*₆) δ 12.25 (s, 1H), 9.02 (s, 1H), 7.79 (d, *J* = 8.0 Hz, 1H), 7.59 (d, *J* = 8.0 Hz, 1H), 5.00 (s, 1H), 2.56 (s, 3H), 2.47 (s, 2H), 2.19 (s, 3H), 1.79 - 0.92 (m, 12H). |
| 277 | 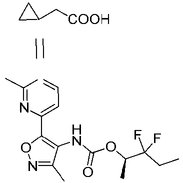 | 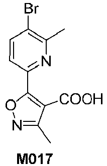 | 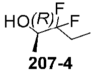 | LC-MS: [M+H]⁺ =462.15. |
| | | | | ¹H NMR (400 MHz, DMSO-*d*₆) δ 12.25 (s, 1H), 9.34 (s, 1H), 7.80 (d, *J* = 8.0 Hz, 1H), 7.60 (d, *J* = 8.0 Hz, 1H), 4.98 (s, 1H), 2.55 (s, 3H), 2.47 (s, 2H), 2.20 (s, 3H), 2.02 - 1.97 (m, 2H), 1.30 - 0.95 (m, 10H). |
| 326 | 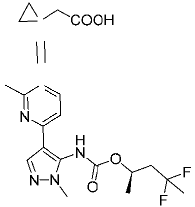 | 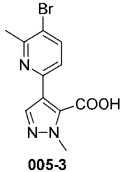 | 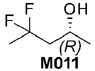 | LC-MS: [M+H]⁺ = 461.15. |
| | | | | ¹H NMR (400 MHz, DMSO-*d₆*) δ 9.42 (s, 1H), 7.89 (s, 1H), 7.54 (d, J = 8.0 Hz, 1H), 7.29 (d, J = 8.4 Hz, 1H), 4.98 (s, 1H), 3.61 (s, 3H), 2.46 (s, 3H), 2.39 (s, 2H), 2.28 - 2.01 (m, 2H), 1.67 - 1.45 (m, 3H), 1.22 (s, 3H), 1.00 - 0.97 (m, 2H), 0.90 - 0.88 (m, 2H). |
| 327 | 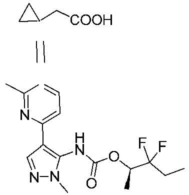 | 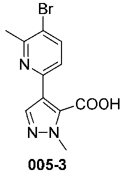 | 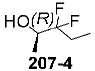 | LC-MS: [M+H]⁺= 461.15. |
| | | | | ¹H NMR (400 MHz, DMSO-*d₆*) δ 9.71 (s, 1H), 7.90 (s, 1H), 7.55 (d, J = 8.4 Hz, 1H), 7.29 (d, J = 8.4 Hz, 1H), 4.95 (s, 1H), 3.62 (s, 3H), 2.45 (s, 3H), 2.39 (s, 2H), 2.02 - 1.79 (m, 2H), 1.36 - 1.15 (m, 3H), 1.02 - 0.78 (m, 7H). |
| 328 | 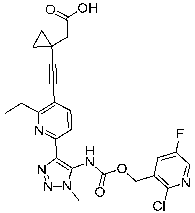 | 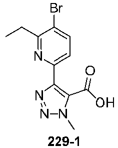 | 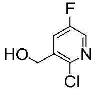 | LC-MS: [M+H]⁺ =513.05. |
| | | | | ¹H NMR (400 MHz, DMSO-*d*₆) δ9.84 (s, 1H), 8.46 (s, 1H), 7.81 (s, 1H), 7.76 (d, *J* = 8.0 Hz, 1H), 7.68 (*d*, *J* = 8.0 Hz, 1H), 5.17 (s, 2H), 3.92 (s, 3H), 2.79 (dd, *J* = 8.0, 8.0 Hz, 2H), 2.44 (s, 2H), 1.13 (dt, *J =* 16.0 Hz, 3H), 1.04 (dd, *J =* 4.0, 4.0 Hz, 2H), 0.96 (d, *J* = 8.0 Hz, 2H). |
| 329 | 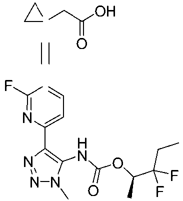 | 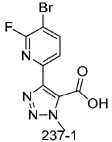 | 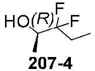 | LC-MS: [M+H]⁺ =466.15. |
| | | | | ¹H NMR (400 MHz, DMSO-*d*₆) δ12.24 (s, 1H), 10.07 (s, 1H), 8.08 - 7.95 (m, 1H), 7.88 (dd, *J* = 4.0,4.0 Hz, 1H), 5.00 (s, 1H), 3.91 (s, 3H), 2.46 (s, 2H), 1.99 (s, 2H), 1.44 - 1.18 (m, 3H), 1.07 (dd, *J =* 4.0, 4.0 Hz, 2H), 1.04 - 0.77 (m, 5H). |
| 330 | 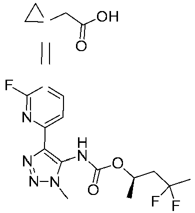 | 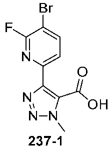 | 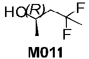 | LC-MS: [M+H]⁺ =466.20. |
| | | | | ¹H NMR (400 MHz, DMSO-*d₆*) δ 8.00 (t, *J* = 8.8 Hz, 1H), 7.87 (d, *J* = 7.6 Hz, 1H), 5.00 (s, 1H), 3.88 (s, 3H), 2.45 (s, 2H), 2.21 (s, 2H), 1.64 (s, 3H), 1.23 (s, 3H), 1.06 (s, 2H), 0.96 (s, 2H). |
| 331 | 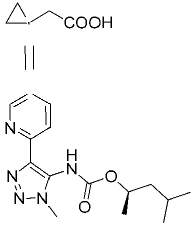 | 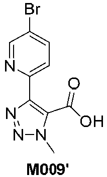 | 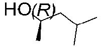 | LC-MS [M+H]⁺= 426.10. |
| | | | | ¹H NMR (400 MHz, DMSO-*d*₆) δ 8.44 (s, 1H), 7.89 (d*, J* = 8.0 Hz, 1H), 7.76 - 7.73 (m, 1H), 4.78 - 4.74 (m, 1H), 3.84 (s, 3H), 2.17 (s, 2H), 1.59 (s, 1H), 1.43 (s, 1H), 1.26 - 1.21 (m, 1H), 1.14 (d, *J* = 4.0 Hz, 3H), 0.94 - 0.82 (m, 10H). |
| 332 | 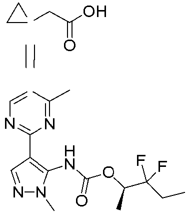 | 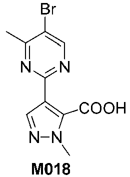 | 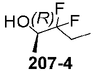 | LCMS: [M+H]⁺ =462.20. |
| | | | | ¹H NMR (400 MHz, DMSO-*d₆*) δ 9.62 (s, 1H), 8.42 (s, 1H), 7.93 (s, 1H), 4.91 (s, 1H), 3.62 (s, 3H), 2.42 (s, 3H), 2.26 (s, 2H), 2.03 - 1.76 (m, 2H), 1.32 - 1.60 (m, 3H), 0.92 - 0.78 (m, 7H). |
| 333 | 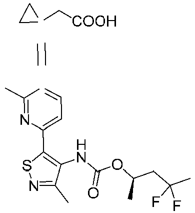 | 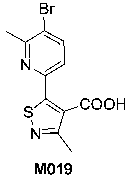 | 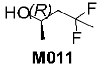 | LC-MS: [M+H]⁺ =478.20. |
| | | | | ¹H NMR (400 MHz, DMSO-*d*₆)δ 9.33 (s, 1H), 7.77 (d*, J* = 8.0 Hz, 1H), 7.54 (d*, J* = 8.0 Hz, 1H), 5.04 (s, 1H), 2.54 (s, 3H), 2.45 (s, 2H), 2.39 - 2.07 (m, 5H), 1.67 (t, *J* = 18.6 Hz, 2H), 1.41 - 1.21 (m, 3H), 1.06 (dd,*J* = 6.4, 4.2 Hz, 2H), 0.96 (dd, *J =* 6.4, 4.2 Hz, 2H). |
| 334 | 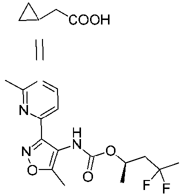 | 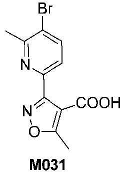 | 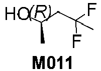 | LC-MS: [M+H]⁺ =462.15. |
| | | | | ¹H NMR (400 MHz, DMSO-*d*₆) δ 8.82 (s, 1H), 7.75 (d*, J* = 8.0 Hz, 1H), 7.61 (d, J = 8.0 Hz, 1H), 4.91 (s, 1H), 2.54 (s, 3H), 2.35 (d, *J* = 1.6 Hz, 5H), 2.19 (s, 2H), 1.62 (s, 3H), 1.24 (s, 3H), 1.00 (t, *J* = 5.2 Hz, 2H), 0.95 (t, *J* = 5.2 Hz, 2H). |
| 335 | 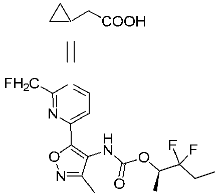 | 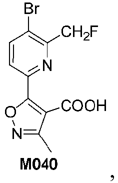 its preparation refers to the method in Preparation Example 15 or the method in Preparation Example 42 | 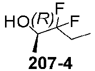 | LC-MS: [M+H]⁺=480.15. |
| | | | | ¹H NMR (400 MHz, DMSO-*d₆*) δ 9.42 (s, 1H), 7.92 (d, J *=* 8.0 Hz, 1H), 7.76 (d, J = 8.0 Hz, 1H), 5.50 (d, J = 51.2 Hz, 2H), 4.94 (s, 1H), 2.43 (s, 2H), 2.18 (s, 3H), 2.02 - 1.82 (m, 2H), 1.32 - 1.20 (m, 3H), 1.07 - 1.05 (m, 2H), 1.00 - 0.83 (m, 5H). |
| 383 | 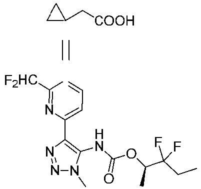 | 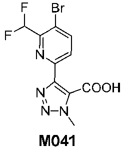 | 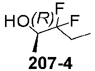 | LC-MS: [M+H]⁺ =498.20. |
| | | | | ¹H NMR (400 MHz, DMSO-*d₆*) δ 12.29 (s, 1H), 9.99 (s, 1H), 8.06 (d, *J* = 8.2 Hz, 1H), 7.95 (d, *J* = 8.2 Hz, 1H), 7.03 (t, *J* = 53.8 Hz, 1H), 4.95 (s, 1H), 3.91 (s, 3H), 2.48 (s, 2H), 1.99 (s, 2H), 1.43 - 1.20 (m, 3H), 1.13 - 1.07 (m, 2H), 1.02 - 0.80 (m, 5H). |
| 384 | 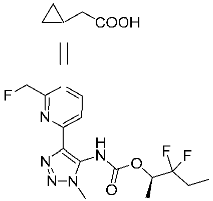 | 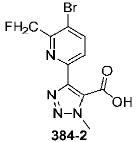 , its preparation refers to the preparation method of compound M040 in Preparation Example 42 | 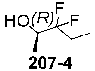 | LC-MS: [M+H]⁺ =480.15. |
| | | | | ¹H NMR (400 MHz, DMSO-*d₆*) δ 12.29 (s, 1H), 9.97 (s, 1H), 7.95 (dd, *J* = 8.2, 1.3 Hz, 1H), 7.86 (d, *J* = 8.2 Hz, 1H), 5.53 (s, 1H), 5.41 (s, 1H), 4.97 (s, 1H), 3.91 (s, 3H), 2.47 (s, 2H), 2.15 - 1.74 (m, 2H), 1.43 - 1.15 (m, 3H), 1.11 - 1.06 (m, 2H), 1.04 - 0.82 (m, 5H). |
| 443 | 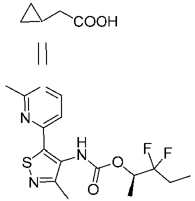 | 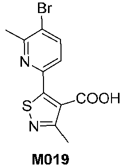 | 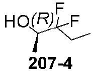 | LC-MS: [M+H]⁺ =478.20. |
| | | | | ¹H NMR (400 MHz, DMSO-*d*₆)δ 12.26 (s, 1H), 9.63 (s, 1H), 7.80 (d, *J* = 8.2 Hz, 1H), 7.52 (d, *J* = 8.2 Hz, 1H), 5.02 (s, 1H), 2.54 (s, 3H), 2.47 (s, 2H), 2.28 (s, 3H), 2.08 - 1.87 (m, 2H), 1.32 (d, *J =* 5.6 Hz, 3H), 1.07 (t, *J =* 5.4 Hz, 2H), 1.02 - 0.92 (m, 5H). |
| 444 | 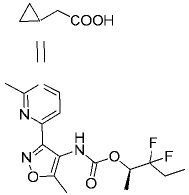 | 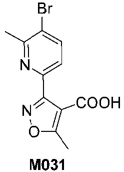 | 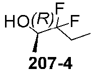 | LC-MS: [M+H]⁺ =462.15. |
| | | | | ¹H NMR (400 MHz, DMSO-*d₆*) δ 12.24 (s, 1H), 9.10 (s, 1H), 7.76 (d, *J* = 8.0 Hz, 1H), 7.62 (d, *J* = 8.0 Hz, 1H), 4.92 (s, 1H), 2.54 (s, 3H), 2.47 (s, 2H), 2.37 (s, 3H), 2.06 - 1.87 (m, 2H), 1.23 (s, 5H), 1.08 (d, *J* = 6.8 Hz, 2H), 0.96 (d, *J=* 6.8 Hz, 3H). |
| 445 | 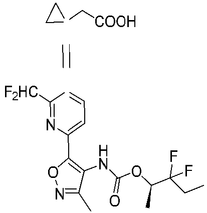 | 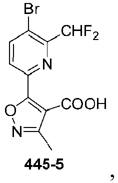 its preparation refers to the preparation method of compound M041 in Preparation Example 43 | 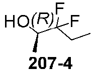 | LC-MS: [M+H]⁺=498.15. |
| | | | | ¹H NMR (400 MHz, DMSO-*d₆*) δ 12.28 (s, 1H), 9.41 (s, 1H), 8.01 (d, J = 8.4 Hz, 1H), 7.87 (d, J = 8.4 Hz, 1H), 7.07 (t, J = 53.6 Hz, 1H), 4.92 (s, 1H), 2.45 (s, 2H), 2.19 (s, 3H), 1.91 (s, 2H), 1.25 (s, 3H), 1.10 - 1.07 (m, 2H), 0.97 - 0.88 (m, 5H). |
| 450 | 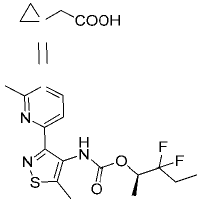 | 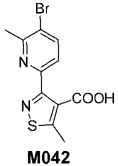 | 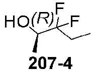 | LC-MS: [M+H]⁺=478.15. |
| | | | | ¹H NMR (400 MHz, DMSO-*d₆*) δ 9.55 (s, 1H), 7.81 - 7.71 (m, 2H), 4.92 (s, 1H), 2.55 (s, 3H), 2.47 (s, 2H), 2.42 (s, 3H), 1.90 (s, 2H), 1.23 (s, 3H), 1.07 (m, 2H), 0.94 (m, 5H). |
| 451 | 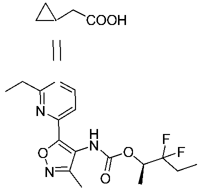 | 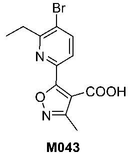 , its preparation refers to the method in Preparation Example 15 (see Preparation Example 45 for details) | 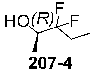 | LC-MS: [M+H]⁺=476.25. |
| | | | | ¹H NMR (400 MHz, DMSO-*d₆*) δ 12.26 (s, 1H), 9.31 (s, 1H), 7.80 (d, *J* = 8.1 Hz, 1H), 7.61 (d, *J* = 8.1 Hz, 1H), 4.99 (s, 1H), 2.91 (q, *J* = 7.5 Hz, 2H), 2.47 (s, 2H), 2.21 (s, 3H), 2.11 - 1.74 (m, 2H), 1.26 (m, 6H), 1.08 (m, 2H), 0.97 (m, 5H). |
| 452 | 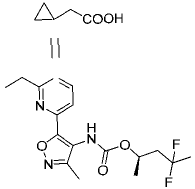 | 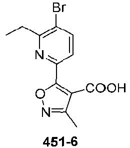 | 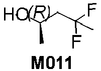 | LC-MS: [M+H]⁺=476.25. |
| | | | | ¹H NMR (400 MHz, DMSO-*d₆*)δ 12.26 (s, 1H), 9.00 (s, 1H), 7.80 (d, J = 8.0 Hz, 1H), 7.60 (d, J = 8.0 Hz, 1H), 5.00 (s, 1H), 2.91 (q, J = 7.6 Hz, 2H), 2.47 (s, 2H), 2.22 (d, J = 10.8 Hz, 5H), 1.62 (s, 3H), 1.24 (t, J = 7.6 Hz, 6H), 1.07 (m, 2H), 0.97 (m, 2H). |
| 453 | 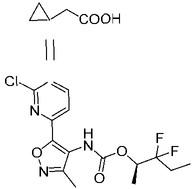 | 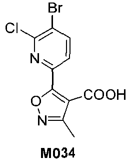 | 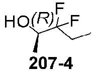 | LC-MS: [M+H]⁺ =482.10. |
| | | | | ¹H NMR (400 MHz, DMSO-*d₆*) δ 12.25 (s, 1H), 9.46 (s, 1H), 8.00 (d, *J* = 8.0 Hz, 1H), 7.75 (d, *J* = 8.0 Hz, 1H), 4.96 (s, 1H), 2.46 (s, 2H), 2.19 (s, 3H), 2.10 - 1.76 (m, 2H), 1.38 - 1.09 (m, 3H), 1.09 - 1.02 (m, 2H), 1.00 - 0.81 (m, 5H). |
| 454 | 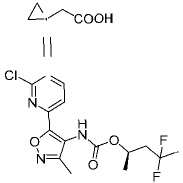 | 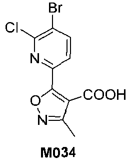 | 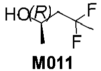 | LC-MS: [M+H]⁺ =482.10. |
| | | | | ¹H NMR (400 MHz, DMSO-*d₆*) δ 12.23 (s, 1H), 9.14 (s, 1H), 7.99 (d, *J* = 8.0 Hz, 1H), 7.73 (d, *J* = 8.0 Hz, 1H), 4.98 (s, 1H), 2.46 (s, 2H), 2.17 (s, 5H), 1.62 (t, *J* = 18.0 Hz, 3H), 1.37 - 1.15 (m, 3H), 1.09 - 1.02 (m, 2H), 1.00 - 0.81 (m, 5H). |
| 455 | 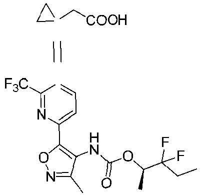 | 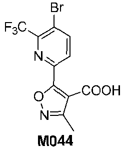 | 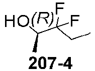 | LC-MS: [M+H]⁺=516.10. |
| | | | | ¹H NMR (400 MHz, DMSO-*d₆*) δ 12.26 (s, 1H), 9.48 (s, 1H), 8.16 (d, *J* = 8.4 Hz, 1H), 8.02 (d, *J =* 8.4 Hz, 1H), 4.96 (s, 1H), 2.48 (s, 2H), 2.23 (s, 3H), 2.00 (m, 2H), 1.28 (d*, J =* 5.2 Hz, 3H), 1.13 - 1.06 (m, 2H), 1.05 - 0.91 (m, 5H). |
| 456 | 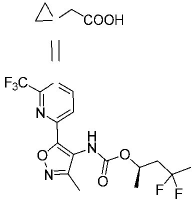 | 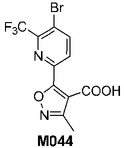 | 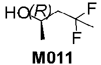 | LC-MS: [M+H]⁺=516.20. |
| | | | | ¹H NMR (400 MHz, DMSO-*d₆*) δ 9.16 (s, 1H), 8.15 (d*, J* = 8.2 Hz, 1H), 8.01 (d, *J* = 8. Hz, 1H), 4.97 (s, 1H), 2.47 (s, 2H), 2.32 - 2.06 (m, 5H), 1.77 - 1.45 (m, 3H), 1.37 - 1.16 (m, 3H), 1.10 - 1.05 (m, 2H), 1.04 - 0.99 (m, 2H). |
| 469 | 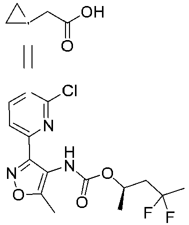 | 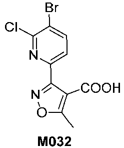 | 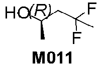 | LCMS:[M+1]+=482.10 |
| | | | | ¹H NMR (400 MHz, DMSO-*d₆*) δ 8.87 (s, 1H), 7.99 (d*, J =* 8.0 Hz, 1H), 7.78 (d*, J =* 8.0 Hz, 1H), 4.91 (s, 1H), 2.46 (s, 2H), 2.36 (s, 3H), 2.23 - 2.10 (m, 2H), 1.63 (t, *J* = 19.2 Hz, 3H), 1.41 - 1.14 (m, 3H), 1.10 - 1.07 (m, 2H), 1.00 - 0.98 (m, 2H). |
| 477 | 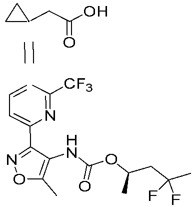 | 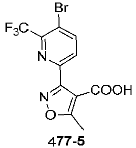 | 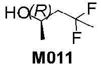 | LCMS:[M+1]⁺=516.10 |
| | | | | ¹H NMR (600 MHz, DMSO-*d₆*) δ 8.87 (s, 1H), 8.13 - 8.04 (m, 2H), 4.88 (s, 1H), 2.47 (s, 2H), 2.37 (s, 3H), 2.30 - 2.05 (m, 2H), 1.61 (t, *J* = 24.0 Hz, 3H), 1.26 - 1.20 (m, 3H), 1.11 - 1.07 (m, 2H), 1.02 - 1.00 (m, 2H). |
| 494 | 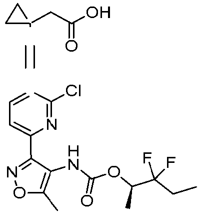 | 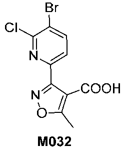 | 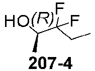 | LCMS:[M+1]⁺=482.10 |
| | | | | ¹H NMR (400 MHz, DMSO-*d₆*) δ 9.20 (s, 1H), 7.99 (d*, J* = 8.0 Hz, 1H), 7.79 (d, *J=* 8.0 Hz, 1H), 4.93 - 4.87 (m, 1H), 2.46 (s, 2H), 2.37 (s, 3H), 2.15 - 1.73 (m, 2H), 1.28 (d, *J=* 6.4 Hz, 3H), 1.09 - 1.07 (m, 2H), 1.04 - 0.85 (m, 5H). |
| 495 | 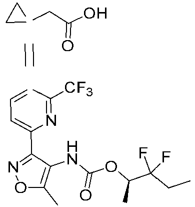 | 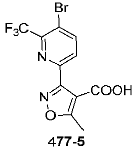 | 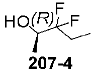 | LC-MS: [M+H]⁺=516.05. |
| | | | | ¹H NMR (400 MHz, DMSO-*d₆*) δ9.19 (s, 1H), 8.09 (dd, *J* = 8.0, 8.0 Hz, 2H), 4.87 (d, *J* = 4.0 Hz, 1H), 2.45 (s, 2H), 2.39 (s, 3H), 2.06 - 1.73 (m, 2H), 1.23 (d, *J* = 8.0 Hz, 3H), 1.07 (d, *J* = 4.0 Hz, 2H), 1.00 (d, *J* = 4.0 Hz, 2H), 0.97 - 0.81 (m, 3H). |
| 496 | 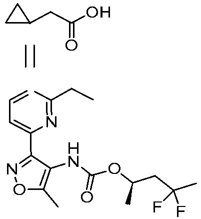 | 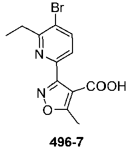 | 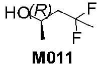 | LC-MS: [M+H]⁺ =476.20. |
| | | | | ¹H NMR (400 MHz, DMSO-*d₆*) δ 12.24 (s, 1H), 8.88 - 8.40 (m, 1H), 7.75 (d, *J* = 8.0 Hz, 1H), 7.62 (d, *J* = 8.0 Hz, 1H), 5.02 - 4.68 (m, 1H), 2.92 - 2.81 (m, 2H), 2.44 (s, 2H), 2.35 (s, 3H), 2.26 - 1.71 (m, 2H), 1.70 - 1.34 (m, 3H), 1.33 - 1.09 (m, 6H), 1.07 - 1.01 (m, 2H), 0.96 - 0.92 (m, 2H). |
| 497 | 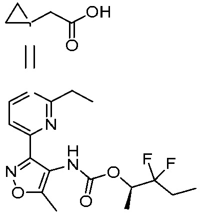 | 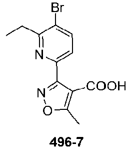 | 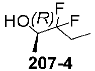 | LC-MS: [M+H]⁺ =476.20. |
| | | | | ¹H NMR (400 MHz, DMSO-*d₆*) δ 12.24 (s, 1H), 9.11 (s, 1H), 7.77 (d,*J* = 8.0 Hz, 1H), 7.64 (d, *J =* 8.0 Hz, 1H), 5.09 - 4.65 (m, 1H), 2.95 - 2.83 (m, 2H), 2.47 (s, 2H), 2.39 (s, 3H), 2.04 - 1.47 (m, 2H), 1.44 - 1.09 (m, 6H), 1.09 - 1.03 (m, 2H), 0.98 - 0.95 (m, 3H), 0.94 - 0.73 (m, 2H). |
| 515 | 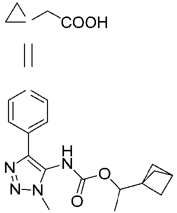 | 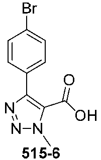 | 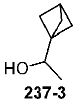 | LC-MS: [M+H]⁺=435.15. |
| | | | | The compound contains a pair of enantiomers. |
| | | | | ¹H NMR (400 MHz, DMSO-*d₆*) δ 7.73 (d, *J* = 8.4 Hz, 2H), 7.40 (d, *J* = 8.4 Hz, 2H), 4.74 (s, 1H), 3.86 (s, 3H), 2.42 (s, 2H), 1.57 (t, *J* = 103.6 Hz, 7H), 1.20 (d, *J* = 28.2 Hz, 3H), 1.01 (t, *J* = 2.8 Hz, 2H), 0.91 (m, 2H). |
| 516 | 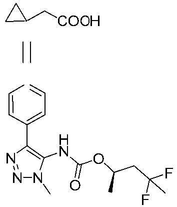 | 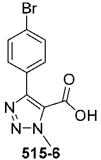 | 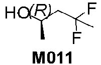 | LC-MS: [M+H]⁺=447.10. |
| | | | | ¹H NMR (400 MHz, DMSO-*d₆*) δ 7.71 (d, *J* = 8.4 Hz, 2H), 7.38 (d, *J* = 8.4 Hz, 2H), 5.07 (s, 1H), 3.85 (s, 3H), 2.41 (s, 2H), 2.29 (d, *J* = 32.4 Hz, 2H), 1.70 (d, *J* = 40.0 Hz, 3H), 1.26 (d, *J* = 24.3 Hz, 3H), 1.01 (m, 2H), 0.91 (m, 2H). |

The compounds in the following table were prepared by referring to the preparation method of compound 212 in Example 50, wherein compound M005 was replaced with the corresponding Reactant 1 in the following table, and compound 207-4 was replaced with the corresponding Reactant 2 in the following table:

| No. | Compound structure | Reactant 1 | Reactant 2 | Characterization |
|---|---|---|---|---|
| 336 | 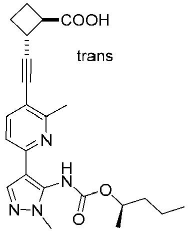 | 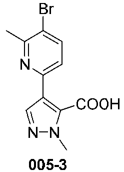 | 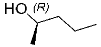 | LC-MS: [M+H]⁺= 425.20. |
| | | | | The compound contains a pair of trans isomers. |
| | | | | ¹H NMR (400 MHz, DMSO-d6) δ 9.33 (s, 1H), 7.94 (s, 1H), 7.69 (d, J *=* 8.4 Hz, 1H), 7.37 (d, J = 8.0 Hz, 1H), 4.73 (s, 1H), 3.66 (s, 3H), 3.47 - 3.40 (m, 1H), 3.20 - 3.14 (m, 1H), 2.54 (s, 3H), 2.25 - 2.03 (m, 4H), 1.55 - 0.66 (m, 10H). |
| 337 | 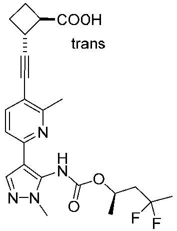 | 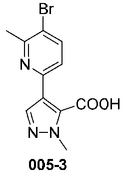 | 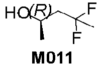 | LC-MS: [M+H]⁺ = 461.15. |
| | | | | The compound contains a pair of trans isomers. |
| | | | | ¹H NMR (400 MHz, DMSO-d6) δ 7.90 (s, 1H), 7.63 (d, J = 8.4 Hz, 1H), 7.31 (d, J = 8.4 Hz, 1H), 4.98 (s, 1H), 3.61 (s, 3H), 3.41 - 3.34 (m, 1H), 3.13 - 3.06 (m, 1H), 2.49 (s, 3H), 2.18 - 2.14 (m, 1H), 2.06 - 1.98 (m, 3H), 1.57 (s, 2H), 1.21 (s, 3H), 0.83 (s, 3H). |
| 338 | 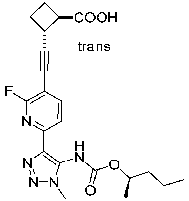 | 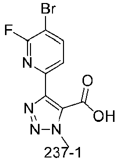 | 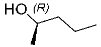 | LC-MS: [M+H]⁺=430.10. |
| | | | | The compound contains a pair of trans isomers. |
| | | | | ¹H NMR (400 MHz, DMSO-*d₆*) δ 8.14 - 8.04 (m, 1H), 7.87 (d, *J* = 8.0 Hz, 1H), 4.72 (s, 1H), 3.89 (s, 3H), 3.44 (d, *J* = 8.4 Hz, 1H), 3.13 (d, *J* = 8.8 Hz, 1H), 2.22 - 2.18 (m, 1H), 2.12 - 2.00 (m, 3H), 1.19 (s, 7H), 0.86 (s, 3H) |
| 339 | 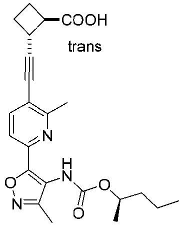 | 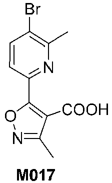 | 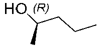 | LC-MS: [M+H]⁺ =426.15. |
| | | | | The compound contains a pair of trans isomers. |
| | | | | ¹H NMR (400 MHz, DMSO-*d₆*) δ 8.93 (s, 1H), 7.89 (d, *J* = 8.0 Hz, 1H), 7.61 (d, *J* = 8.0 Hz, 1H), 4.71 (s, 1H), 3.48 - 3.41 (m, 1H), 3.18 - 3.11 (m, 1H), 2.59 (s, 3H), 2.24 (s, 1H), 2.20 (s, 3H), 2.13 - 1.98 (m, 3H), 1.64 - 1.05 (m, 7H), 0.85 (s, 3H). |
| 340 | 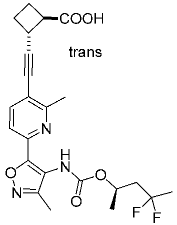 | 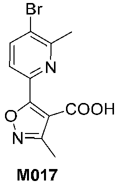 | 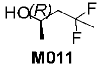 | LC-MS: [M+H]⁺ =462.15. |
| | | | | The compound contains a pair of trans isomers. |
| | | | | ¹H NMR (400 MHz, DMSO-*d₆*) δ 9.05 (s, 1H), 7.89 (d, *J* = 8.0 Hz, 1H), 7.61 (d, *J =* 8.10Hz, 1H), 4.99 (s, 1H), 3.48 - 3.42 (m, 1H), 3.19 - 3.12 (m, 1H), 2.59 (s, 3H), 2.28 - 2.17 (m, 5H), 2.11 - 2.05 (m, 4H), 1.62 (s, 3H), 1.25 (s, 3H). |
| 486 | 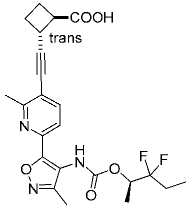 | 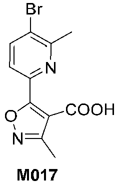 | 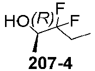 | LC-MS: [M+H]⁺ =462.20. |
| | | | | The compound contains a pair of trans isomers. |
| | | | | ¹H NMR (400 MHz, DMSO-*d*₆) δ 12.43 (s, 1H), 9.37 (s, 1H), 7.90 (d, *J* = 8.0 Hz, 1H), 7.63 (d, *J* = 8.0 Hz, 1H), 4.99 (s, 1H), 3.54 - 3.44 (m, 1H), 3.28 - 3.19 (m, 1H), 2.59 (s, 3H), 2.40 - 1.84 (m, 9H), 1.29 - 1.23 (m, 3H), 0.97 (s, 3H). |

The compounds in the following table were prepared by referring to the preparation method of compound 209 in Example 47 or compound 210 in Example 48, wherein compound M010 was replaced with the corresponding Reactant 1 in the following table, and compound 209-6 was replaced with the corresponding Reactant 2 in the following table:

| No. | Compound structure | Reactant 1 | Reactant 2 | Characterization |
|---|---|---|---|---|
| 461 | 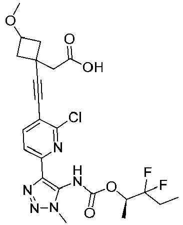 | 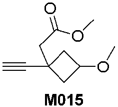 | 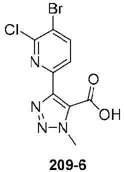 | LC-MS: [M+H]⁺ =526.25. |
| | | | | The compound contains cis-trans isomers. |
| | | | | ¹H NMR (400 MHz, DMSO-*d₆*) δ 10.10 (s, 1H), 7.96 - 7.94 (m, 2H), 5.00 (s, 1H), 4.12 - 3.98 (m, 1H), 3.91 (s, 3H), 3.15 (d, *J* = 7.2 Hz, 3H), 2.71 (d, *J* = 6.8 Hz, 2H), 2.68 - 2.62 (m, 2H), 2.31 - 2.23 (m, 1H), 2.18 - 2.12 (m, 1H), 2.00 (d*, J* = 7.6 Hz, 2H), 1.36 (s, 3H), 0.99 (s, 3H). |
| 462 | 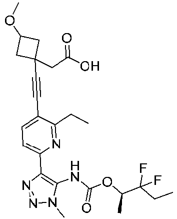 | 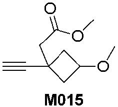 | 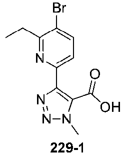 | LC-MS: [M+H]⁺=520.20. |
| | | | | The compound contains cis-trans isomers. |
| | | | | ¹H NMR (400 MHz, DMSO-*d₆*) δ 12.26 (s, 1H), 9.87 (s, 1H), 7.81 - 7.72 (m, 2H), 5.00 (s, 1H), 4.04 (m, 1H), 3.90 (s, 3H), 3.15 (d, *J* = 6.4 Hz, 3H), 2.93 - 2.84 (m, 2H), 2.72 - 2.60 (m, 4H), 2.29 - 2.20 (m, 1H), 2.17 - 2.09 (m, 1H), 2.00 (d*, J* = 8.2 Hz, 2H), 1.37 - 1.20 (m, 6H), 0.92 (d, *J* = 39.2 Hz, 3H). |
| 463 | 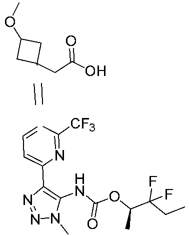 | 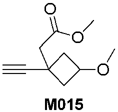 | 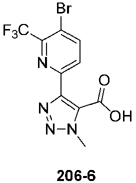 | LC-MS: [M+H]⁺=560.15. |
| | | | | The compound contains cis-trans isomers. |
| | | | | ¹H NMR (400 MHz, DMSO-*d₆*) δ 12.26 (s, 1H), 10.10 (s, 1H), 8.20 (m, 1H), 8.09 (t, *J* = 8.0 Hz, 1H), 4.96 (s, 1H), 4.04 (m, 1H), 3.94 (d, *J* = 14.4 Hz, 3H), 3.15 (d, *J* = 6.4 Hz, 3H), 2.72 - 2.60 (m, 4H), 2.28 - 2.20 (m, 1H), 2.18 - 2.11 (m, 1H), 2.00 (d, *J* = 8.0 Hz, 2H), 1.27 (d, *J* = 28.4 Hz, 3H), 0.98 (s, 3H). |
| 467 | 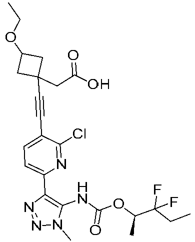 | 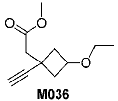 | 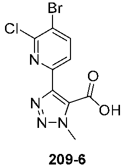 | LCMS:[M+1]⁺=540.15. |
| | | | | The compound contains cis-trans isomers. |
| | | | | ¹H NMR (400 MHz, DMSO-*d₆*) δ 12.25 (s, 1H), 10.10 (s, 1H), 8.02 - 7.90 (m, 2H), 5.00 (s, 1H), 4.19 - 4.10 (m, 1H), 3.91 (s, 3H), 3.40 - 3.34 (m, 2H), 2.70 (d*, J* = 7.2 Hz, 2H), 2.68 - 2.62 (m, 2H), 2.29 - 2.12 (m, 2H), 2.01 (s, 2H), 1.36 (s, 3H), 1.10 (t, *J* = 7.2 Hz, 3H), 0.99 (s, 3H). |
| 468 | 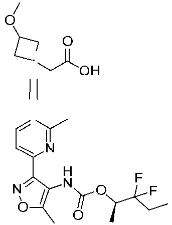 | 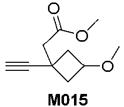 | 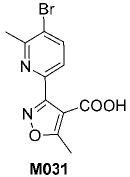 | LC-MS:[M+1]⁺=506.15 |
| | | | | The compound contains cis-trans isomers. |
| | | | | ¹H NMR (400 MHz, DMSO-*d₆*) δ 9.11 (s, 1H), 7.79 (s, 1H), 7.63 (d*, J* = 4.4 Hz, 1H), 4.92 (s, 1H), 4.01 (d, *J* = 47.2 Hz, 1H), 3.13 (d*, J* = 7.2 Hz, 3H), 2.67 - 2.54 (m, 7H), 2.37 (s, 3H), 2.22 - 2.15 (m, 2H), 1.92 (s, 2H), 1.25 (d, *J* = 10.0 Hz, 3H), 0.94 (d, *J* = 6.4 Hz, 3H). |
| 470 | 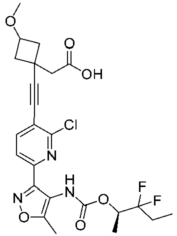 | 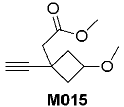 | 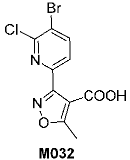 | LCMS:[M+1]⁺=526.15 |
| | | | | Note: The compound contains cis-trans isomers. |
| | | | | ¹H NMR (400 MHz, DMSO-*d₆*) δ 9.21 (s, 1H), 8.02 (t, *J* = 7.6 Hz, 1H), 7.87 - 7.73 (m, 1H), 4.94 - 4.88 (m,1H), 4.12 - 3.95 (m, 1H), 3.14 (d, *J* = 7.2 Hz, 3H), 2.67 - 2.61 (m, 4H), 2.38 (s, 3H), 2.28 - 2.13 (m, 2H), 2.06 - 1.81 (m, 2H), 1.28 (d, *J =* 6.4 Hz, 3H), 0.95 (t, *J =* 7.2 Hz, 3H). |
| 471 | 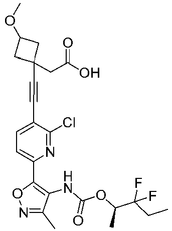 | 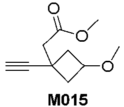 | 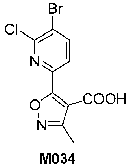 | LC-MS: [M+H]⁺ =526.20. |
| | | | | The compound contains cis-trans isomers. |
| | | | | ¹H NMR (400 MHz, DMSO-*d₆*) δ 12.27 (s, 1H), 9.48 (s, 1H), 8.06 - 8.00 (m, 1H), 7.77 (dd, *J* = 8.4, 2.4 Hz, 1H), 4.97 (s, 1H), 4.10 - 3.89 (m, 1H), 3.12 (d, *J* = 6.8 Hz, 3H), 2.69 (d, *J* = 6.4 Hz, 2H), 2.66 - 2.61 (m, 2H), 2.27 -2.22 (m, 1H), 2.19 (s, 3H), 2.15 - 2.10 (m, 1H), 2.07 - 1.80 (m, 2H), 1.34 - 1.18 (m, 3H), 1.01 - 0.86 (m, 3H). |
| 476 | 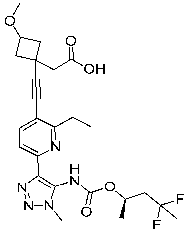 | 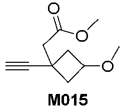 | 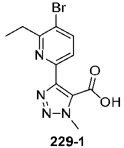 | LC-MS: [M+H]⁺=520.20. |
| | | | | The compound contains cis-trans isomers. |
| | | | | ¹H NMR (400 MHz, DMSO-*d₆*)δ 7.76 (t, *J* = 7.6 Hz, 2H), 5.00 (s, 1H), 4.11 - 3.96 (m, 1H), 3.88 (s, 3H), 3.14 (d, *J* = 6.6 Hz, 3H), 2.94 - 2.84 (m, 2H), 2.71 - 2.56 (m, 4H), 2.31 - 1.98 (m, 4H), 1.59 (s, 3H), 1.34 - 1.04 (m, 6H). |
| 487 | 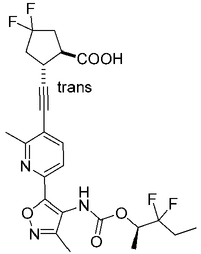 | 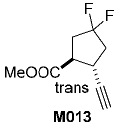 | 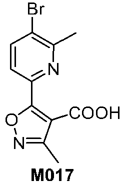 | LC-MS: [M+H]⁺ =512.15. |
| | | | | The compound contains a pair of trans isomers. |
| | | | | ¹H NMR (400 MHz, DMSO-*d*₆) δ 9.37 (s, 1H), 7.90 (d, *J* = 8.0 Hz, 1H), 7.64 (d, *J* = 8.0 Hz, 1H), 4.98 (s, 1H), 3.19 - 3.12 (m, 1H), 2.81 - 2.51 (m, 6H), 2.43 - 2.26 (m, 2H), 2.21 (s, 3H), 2.00 - 1.97 (m, 2H), 1.29 - 1.23 (m, 3H), 0.96 (s, 3H). |
| 488 | 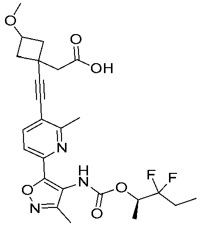 | 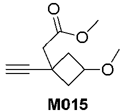 | 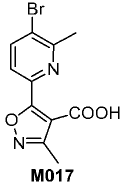 | LC-MS: [M+H]⁺=506.25. |
| | | | | The compound contains cis-trans isomers. |
| | | | | ¹H NMR (400 MHz, DMSO-*d*₆) δ 9.36 (s, 1H), 7.85 (dd, *J* = 8.0, 4.0 Hz, 1H), 7.63 (dd, *J* = 8.0, 4.0 Hz, 1H), 4.99 (s, 1H), 4.15 - 3.95 (m, 1H), 3.15 (d, *J* = 8.0 Hz, 3H), 2.73 - 2.62 (m, 4H), 2.57 (d, *J* = 8.0 Hz, 3H), 2.30 - 2.12 (m, 5H), 2.20 - 1.97 (m, 2H), 1.29 - 1.23 (m, 3H), 1.04 - 0.85 (m, 3H). |
| 489 | 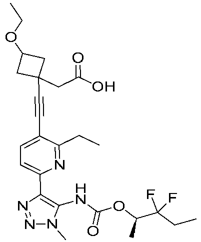 | 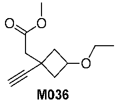 | 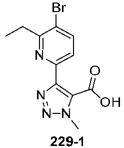 | LC-MS: [M+H]⁺ =534.25. |
| | | | | The compound contains cis-trans isomers. |
| | | | | ¹H NMR (400 MHz, DMSO-*d₆*) δ9.88 (s, 1H), 7.80 - 7.71 (m, 2H), 4.99 (s, 1H), 4.23 - 4.03 (m, 1H), 3.90 (s, 3H), 3.36 (s, 2H), 2.92 - 2.83 (m, 2H), 2.69 (d, *J* = 8.0 Hz, 2H),2.67 - 2.60 (m, 2H), 2.28 - 2.09 (m, 2H), 2.08 - 1.65 (m, 2H), 1.31 (s, 2H), 1.27- 1.18 (m, 4H), 1.10 (t, *J* =16.0 Hz, 3H), 1.06- 0.81 (m, 3H). |
| 490 | 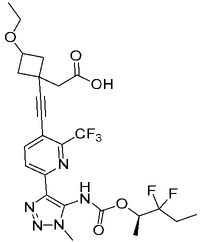 | 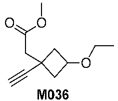 | 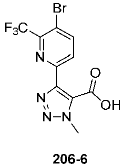 | LC-MS: [M+H]⁺=574.20. |
| | | | | The compound contains cis-trans isomers. |
| | | | | ¹H NMR (400 MHz, DMSO-*d*₆) δ 12.24 (s, 1H), 10.10 (s, 1H), 8.20 - 8.18 (m, 1H), 8.10 - 8.07 (m, 1H), 4.96 (s, 1H), 4.20 - 4.04 (m, 1H), 3.92 (s, 3H), 3.38 - 3.34 (m, 2H), 2.75 - 2.58 (m, 4H), 2.33 - 1.77 (m, 4H), 1.40 - 1.20 (m, 3H), 1.11 - 1.08 (m, 3H), 0.98 (s, 3H). |
| 491 | 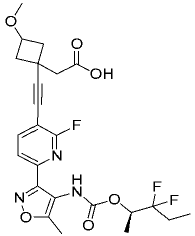 | 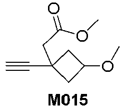 | 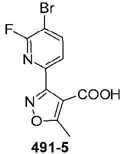 | LC-MS: [M+H]⁺=510.20. |
| | | | | The compound contains cis-trans isomers. |
| | | | | ¹H NMR (400 MHz, DMSO-*d*₆) δ 12.30 (s, 1H), 9.20 (s, 1H), 8.12 - 8.07 (m, 1H), 7.78 (d*, J* = 8.0 Hz, 1H), 4.98 - 4.88 (m, 1H), 4.09 - 3.96 (m, 1H), 3.14 (d, *J* = 8.0 Hz, 3H), 2.71 (d, *J* = 8.0 Hz, 2H), 2.65 - 2.62 (m, 2H), 2.37 (s, 3H), 2.29 - 2.22 (m, 1H), 2.19 - 2.10 (m, 1H), 2.01 - 1.88 (m, 2H), 1.27 - 1.23 (m, 3H), 0.95 (t, *J* = 8.0 Hz, 3H). |
| 492 | 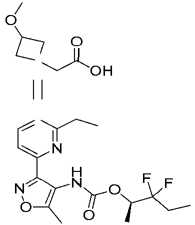 | 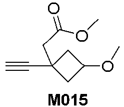 | 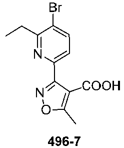 | LC-MS: [M+H]⁺=520.25. |
| | | | | The compound contains cis-trans isomers. |
| | | | | ¹H NMR (400 MHz, DMSO-*d₆*) δ 12.17 (s, 1H), 9.16 - 8.62 (m, 1H), 7.87 - 7.74 (m, 1H), 7.71 - 7.57 (m, 1H), 4.98 - 4.70 (m, 1H), 4.08 - 3.96 (m, 1H), 3.16 - 3.09 (m, 3H), 2.93 - 2.84 (m, 2H), 2.68 (d, *J* = 10.8 Hz, 2H), 2.65 - 2.59 (m, 2H), 2.37 (s, 3H), 2.26 - 2.20 (m, 1H), 2.14 - 2.09 (m, 1H), 2.00 - 1.46 (m, 2H), 1.29 - 1.15 (m, 6H), 0.95 - 0.74 (m, 3H). |
| 493 | 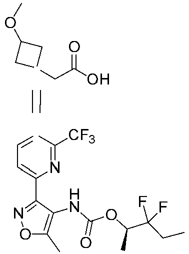 | 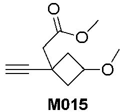 | 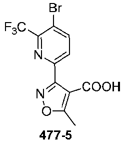 | LC-MS: [M+H]⁺=560.15. |
| | | | | The compound contains cis-trans isomers. |
| | | | | ¹H NMR (400 MHz, DMSO-*d₆*) δ 9.18 (s, 1H), 8.21 - 8.02 (m, 2H), 4.88 (s, 1H), 4.09 - 3.97 (m, 1H), 3.15 (d, *J* = 4.0 Hz, 3H), 2.75 - 2.58 (m, 4H), 2.39 (s, 3H), 2.28 - 2.20 (m, 1H), 2.19 - 2.11 (m, 1H), 2.01 - 1.81 (m, 2H), 1.23 (d, *J* = 4.0 Hz, 3H), 0.95 (t, *J* = 12.0 Hz, 2H), 0.90 - 0.68 (m, 1H). |
| 498 | 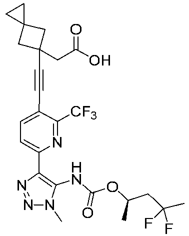 | 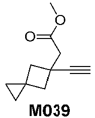 | 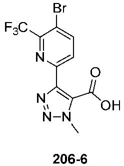 | LC-MS: [M+H]⁺=556.20. |
| | | | | ¹H NMR (400 MHz, DMSO-*d₆*) δ 12.25 (s, 1H), 9.74 (s, 1H), 8.19 (d, *J* = 8.3 Hz, 1H), 8.09 (d, *J* = 8.3 Hz, 1H), 4.97 (s, 1H), 3.90 (s, 3H), 2.81 (s, 2H), 2.50 (d, *J* = 1.6 Hz, 2H), 2.32 m, 4H), 1.46 (d, *J* = 137.7 Hz, 6H), 0.48 (s, 4H). |
| 499 | 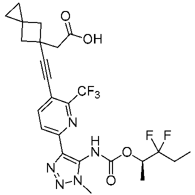 | 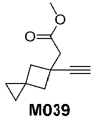 | 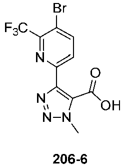 | LC-MS: [M+H]⁺=556.20. |
| | | | | ¹H NMR (400 MHz, DMSO-*d₆*) δ 12.26 (s, 1H), 10.10 (s, 1H), 8.20 (d, *J* = 8.4 Hz, 1H), 8.09 (d, *J =* 8.4 Hz, 1H), 4.96 (s, 1H), 3.92 (s, 3H), 2.81 (s, 2H), 2.51 (s, 2H), 2.37 (d, *J* = 12.0 Hz, 2H), 2.00 (d, *J* = 8.0 Hz, 2H), 1.27 (d, *J* = 27.6 Hz, 3H), 0.98 (s, 3H), 0.48 (s, 4H). |
| 500 | 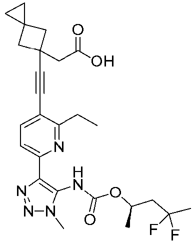 | 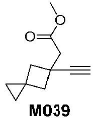 | 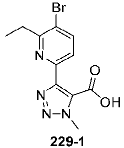 | LC-MS: [M+H]⁺=516.25. |
| | | | | ¹H NMR (400 MHz, DMSO-*d*₆) δ 9.52 (s, 1H), 7.77 (s, 2H), 5.00 (s, 1H), 3.88 (s, 3H), 2.94 - 2.88 (m, 2H), 2.81 (s, 2H), 2.53 (s, 2H), 2.38 - 2.17 (m, 4H), 1.61 (s, 3H), 1.25 (t, *J* = 8.0 Hz, 6H), 0.49 (s, 4H). |
| 501 | 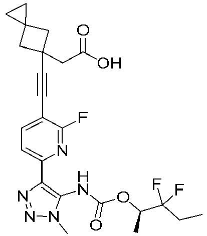 | 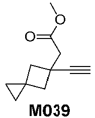 | 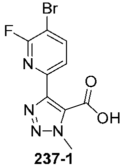 | LC-MS: [M+H]⁺=506.15. |
| | | | | ¹H NMR (600 MHz, DMSO-*d₆*)δ 12.27 (s, 1H), 10.09 (m, 1H), 8.06 - 8.02 (m, 1H), 7.91 (d*, J* = 7.8 Hz, 1H), 5.02 (s, 1H), 3.91 (s, 3H), 2.82 (s, 2H), 2.54 (d, *J* = 12.2 Hz, 2H), 2.34 (d, *J* = 12.2 Hz, 2H), 2.00 (dd, *J* = 15.0, 7.2 Hz, 2H), 1.45 - 1.21 (m, 3H), 0.99 (s, 3H), 0.57 - 0.43 (m, 4H). |
| 502 | 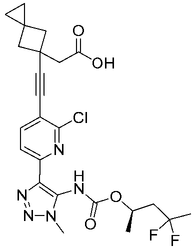 | 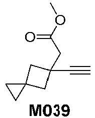 | 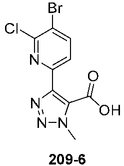 | LCMS:[M+1]⁺=522.15 |
| | | | | ¹H NMR (400 MHz, DMSO-*d₆*) δ 12.23 (s, 1H), 9.74 (s, 1H), 7.94 (q, *J =* 8.0Hz, 2H), 5.00 (s, 1H), 3.89 (s, 3H), 2.82 (s, 2H), 2.54 (d, *J =* 11.6 Hz, 2H), 2.36 (d*, J* = 11.6 Hz, 4H), 1.66 (s, 3H), 1.34 (s, 3H), 0.49 (s, 4H). |
| 503 | 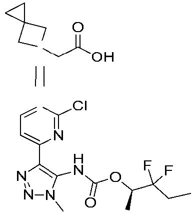 | 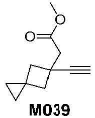 | 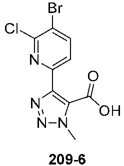 | LCMS:[M+1]⁺=522.15 |
| | | | | ¹H NMR (400 MHz, DMSO-*d₆*) δ 12.25 (s, 1H), 10.10 (s, 1H), 8.13 - 7.76 (m, 2H), 5.01 (s, 1H), 3.91 (s, 3H), 2.83 (s, 2H), 2.55 (d*, J* = 12.0 Hz, 2H), 2.37 (d, *J* = 12.0 Hz, 2H), 2.00 (d, *J* = 7.8 Hz, 2H), 1.36 (s, 3H), 0.99 (s, 3H), 0.49 (s, 4H). |
| 504 | 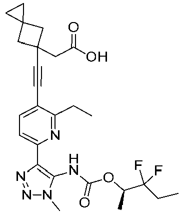 | 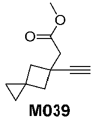 | 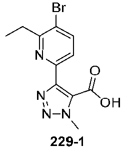 | LC-MS: [M+H]⁺=516.25. |
| | | | | ¹H NMR (400 MHz, DMSO-*d*₆) δ 9.88 (s, 1H), 7.83 - 7.71 (m, 2H), 5.00 (s, 1H), 3.90 (s, 3H), 2.93 - 2.88 (m, 2H), 2.81 (s, 2H), 2.53 (s, 2H), 2.37 (d, *J* = 12 Hz, 2H), 1.97 (s, 2H), 1.43 - 1.18 (m, 6H), 0.96 (s, 3H), 0.49 (s, 4H). |
| 505 | 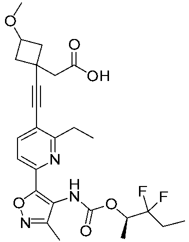 | 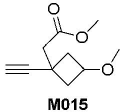 | 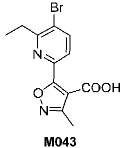 | LC-MS: [M+H]⁺=520.20. |
| | | | | The compound contains cis-trans isomers. |
| | | | | ¹H NMR (400 MHz, DMSO-*d₆*) δ 12.28 (s, 1H), 9.33 (s, 1H), 7.85 (m, 1H), 7.63 (m, 1H), 4.99 (s, 1H), 4.13 - 3.94 (m, 1H), 3.15 (d, *J* = 6.4 Hz, 3H), 2.98 - 2.88 (m, 2H), 2.71 (d, *J* = 7.0 Hz, 2H), 2.64 (m, 2H), 2.22 (s, 3H), 2.00 (m, 3H), 1.23 (t, *J* = 6.0 Hz, 7H), 0.97 (s, 3H). |
| 506 | 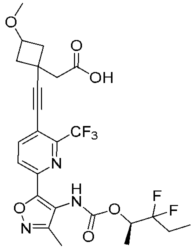 | 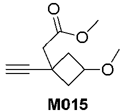 | 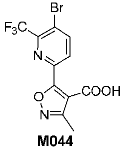 | LC-MS: [M+H]⁺=560.15. |
| | | | | The compound contains cis-trans isomers. |
| | | | | ¹H NMR (400 MHz, DMSO-*d₆*) δ 12.28 (s, 1H), 9.49 (s, 1H), 8.20 (t, *J* = 8.0 Hz, 1H), 8.09 - 8.02 (m, 1H), 4.96 (s, 1H), 4.11 - 3.96 (m, 1H), 3.15 (d, *J* = 6.8 Hz, 3H), 2.71 (d, *J* = 6.4 Hz, 2H), 2.68 - 2.60 (m, 2H), 2.24 (s, 3H), 2.19 - 2.10 (m, 1H), 2.06 - 1.83 (m, 2H), 1.35 - 1.19 (m, 4H), 0.97 (s, 3H). |
| 507 | 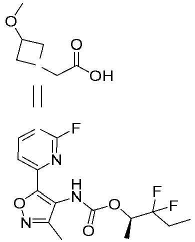 | 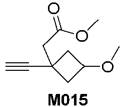 | 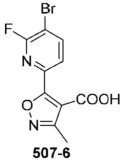 , its preparation refers to the preparation method of compound M044 in Preparation Example 46 | LC-MS: [M+H]⁺=524.05. |
| | | | | The compound contains cis-trans isomers. |
| | | | | ¹H NMR (400 MHz, CDCl₃) δ 7.88 (m, 2H), 7.65 (d*, J* = 7.6 Hz, 1H), 5.15 - 5.02 (m, 1H), 3.95 (p, *J =* 6.8 Hz, 1H), 3.73 (d, *J* = 5.6 Hz, 3H), 3.26 (s, 3H), 2.74 - 2.66 (m, 4H), 2.52 - 2.41 (m, 5H), 2.04 - 1.86 (m, 2H), 1.40 (d, *J* = 6.4 Hz, 3H), 1.06 (t, *J* = 7.6 Hz, 3H). |

The compounds in the following table were prepared by referring to the preparation method of compound 209 in Example 47 or compound 210 in Example 48, wherein compound M010 was replaced with compound M012, and compound 209-6 was replaced with the corresponding reactant in the following table:

| No. | Compound structure | Reactant | Characterization |
|---|---|---|---|
| 341 | 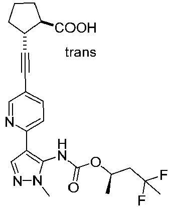 | 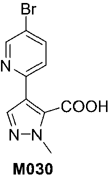 | LC-MS: [M+H]⁺= 461.20. |
| | | | The compound contains a pair of trans isomers. |
| | | | ¹H NMR (400 MHz, DMSO-*d₆*) δ 9.45 (s, 1H), 8.43 (s, 1H), 7.91 (s, 1H), 7.69 (d, J = 7.2 Hz, 1H), 7.46 (d, J = 8.4 Hz, 1H), 4.98 (s, 1H), 3.61 (s, 3H), 3.08 (dd, J = 16.0, 8.0 Hz, 1H), 2.70 (dd, J = 16.0, 8.0 Hz, 1H), 2.28 - 1.91 (m, 4H), 1.76 - 1.49 (m, 7H), 1.22 (s, 3H). |
| 342 | 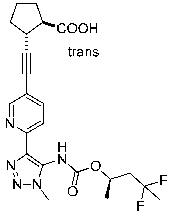 | 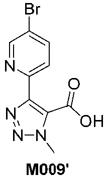 | LC-MS: [M+H]⁺=462.10. |
| | | | The compound contains a pair of trans isomers. |
| | | | ¹H NMR (400 MHz, DMSO-*d*₆) δ 8.51 (s, 1H), 7.93 (d, *J* = 8.0 Hz, 1H), 7.86 - 7.84 (m, 1H), 4.98 (s, 1H), 3.88 (s, 3H), 3.18 - 3.12 (m, 1H), 2.78 - 2.72 (m, 1H), 2.16 - 1.93 (m, 3H), 1.75 - 1.64 (m, 8H), 1.24 (s, 3H). |
| 343 | 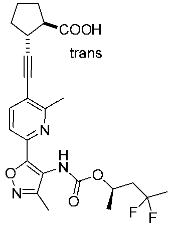 | 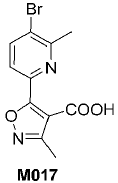 | LC-MS: [M+H]⁺=476.20. |
| | | | The compound contains a pair of trans isomers. |
| | | | ¹H NMR (400 MHz, DMSO-*d*₆) δ 9.04 (s, 1H), 7.86 (d, *J* = 8.0 Hz, 1H), 7.61 (d, *J =* 8.0 Hz, 1H), 5.00 (s, 1H), 3.21 - 3.15 (m, 1H), 2.80 - 2.74 (m, 1H), 2.57 (s, 3H), 2.35 - 1.99 (m, 7H), 1.82 - 1.53 (m, 7H), 1.25 (s, 3H). |
| 344 | 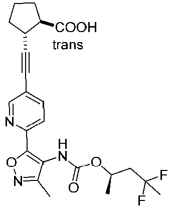 | 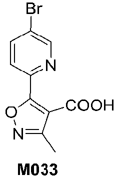 | LC-MS: [M+H]⁺=462.20. |
| | | | The compound contains a pair of trans isomers. |
| | | | ¹H NMR (400 MHz, DMSO-*d₆*) δ 9.08 (s, 1H), 8.65 (s, 1H), 7.96 (m, 1H), 7.78 (d, *J* = 8.2 Hz, 1H), 5.00 (d, *J* = 5.0 Hz, 1H), 3.18 - 3.13 (m, 1H), 2.80 (m, 1H), 2.30 - 1.99 (m, 7H), 1.81 - 1.53 (m, 7H), 1.25 (d, *J* = 9.2 Hz, 3H). |
| 485 | 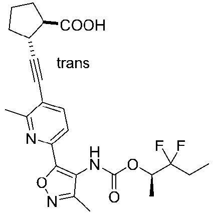 | 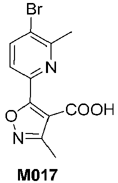 | LC-MS: [M+H]⁺=476.15. |
| | | | The compound contains a pair of trans isomers. |
| | | | ¹H NMR (400 MHz, DMSO-*d*₆) δ 12.40 (s, 1H), 9.36 (s, 1H), 7.88 (d, *J* = 8.0 Hz, 1H), 7.62 (d, *J* = 8.0 Hz, 1H), 4.98 (s, 1H), 3.20 - 3.14 (m, 1H), 2.84 - 2.78 (m, 1H), 2.57 (s, 3H), 2.20 (s, 3H), 2.17 - 1.98 (m, 3H), 1.90 - 1.64 (m, 5H), 1.29 - 1.23 (m, 3H), 0.96 (s, 3H). |

The compounds in the following table were prepared by referring to the preparation method of compound 117 in Example 61, wherein the starting material 2-chloro-3-hydroxymethyl-5-fluoropyridine was replaced with the corresponding starting materials in the following table:

| No. | Compound structure | Starting material | Characterization |
|---|---|---|---|
| 116' | 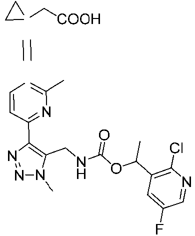 | 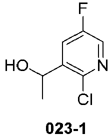 | LC-MS: [M+H]⁺=527.15. |
| | | | ¹H NMR (400 MHz, CDCl₃) δ 8.40 (d, *J* = 2.4 Hz, 1H), 7.83 -7.76 (m, 2H), 7.75 -7.66 (m, 2H), 5.84 - 5.65 (m, 1H), 5.02 - 4.73 (m, 2H), 4.01 (s, 3H), 2.48 (s, 3H), 2.42 (s, 2H), 1.42 (d, *J* = 6.4 Hz, 3H), 1.05 - 0.99 (m, 2H), 0.95 - 0.91 (m, 2H). |
| 116 | 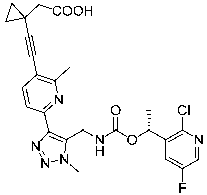 | 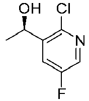 | LC-MS [M+H]⁺ =527.15. |
| 119 | 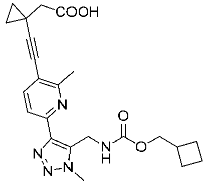 | 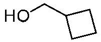 | LC-MS [M+H]⁺ = 438.15. |
| | | | ¹H NMR (400 MHz, DMSO-*d*₆) δ 7.82 *(d, J=* 8.0 Hz, 1H), 7.72 (d, *J* = 8.0 Hz, 1H), 7.55 (s, 1H), 4.79 (d, *J* = 4.0 Hz, 2H), 4.06 (s, 3H), 3.92 - 3.88 (m 2H), 2.57 (s, 3H), 2.52 - 2.51 (m, 1H), 2.45 (s, 2H), 2.01 - 1.90 (m, 2H), 1.85 -1.73 (m, 2H), 1.72 - 1.56 (m, 2H), 1.07 - 1.04 (m, 2H), 0.96 - 0.94 (m, 2H). |
| 120 | 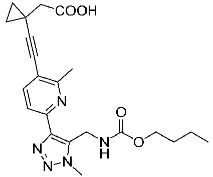 | 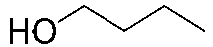 | LC-MS [M+H]⁺= 426.10. |
| | | | ¹H NMR (400 MHz, DMSO-*d*₆) δ 7.82 (d, *J* = 8.0 Hz, 1H), 7.72 (d, *J =* 8.0 Hz, 1H), 7.50 (s, 1H), 4.79 (d, *J=* 4.0 Hz, 2H), 4.06 (s, 3H), 3.91 - 3.94 (m, 2H), 2.57 (s, 3H), 2.45 (s, 2H), 1.53 - 1.43 (m, 2H), 1.29 - 1.24 (m, 2H), 1.06 - 1.04 (m, 2H), 0.96 - 0.94 (m, 2H), 0.86 - 0.82 (m, 3H). |
| 121 | 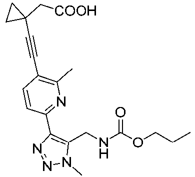 | 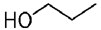 | LC-MS [M+H]⁺ = 412.19 |
| 253 | 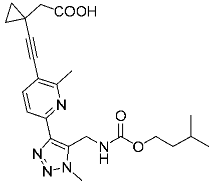 | 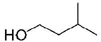 | LC-MS [M+H]⁺= 440.10. |
| | | | ¹H NMR (400 MHz, DMSO-*d*₆) δ 7.82 (d, *J* = 8.0 Hz, 1H), 7.72 (d, *J =* 8.0 Hz, 1H), 7.49 (s, 1H), 4.79 (s, 2H), 4.06 (s, 3H), 3.96 (s, 2H), 2.57 (s, 3H), 2.45 (s, 2H), 1.64 - 1.51 (m, 1H), 1.46 - 1.31 (m, 2H), 1.07 - 0.94 (m, 4H), 0.83 (s, 6H). |
| 345 | 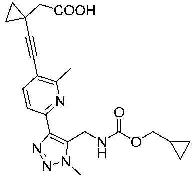 | 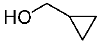 | LC-MS [M+H]⁺= 424.10. |
| | | | ¹H NMR (400 MHz, DMSO-*d*₆) δ 7.82 (d, *J* = 8.0 Hz, 1H), 7.72 (d, *J =* 8.0 Hz, 1H), 7.55 (s, 1H), 4.80 (d, *J* = 4.0 Hz, 2H), 4.07 (s, 3H), 3.77 (d, *J* = 8.0 Hz, 2H), 2.57 (s, 3H), 2.44 (s, 2H), 1.09 - 0.91 (m, 5H), 0.46 - 0.44 (m, 2H), 0.20 - 0.19 (m, 2H). |
| 346 | 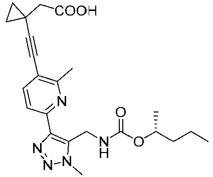 | 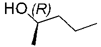 | LC-MS [M+H]⁺ = 440.10. |
| | | | ¹H NMR (400 MHz, DMSO-*d*₆) δ 7.82 (d, *J* = 8.0 Hz, 1H), 7.72 (d, *J =* 8.0 Hz, 1H), 7.44 (s, 1H), 4.79 (d, *J =* 4.0 Hz, 2H), 4.66 - 4.63 (m, 1H), 4.05 (s, 3H), 2.57 (s, 3H), 2.46 (s, 2H), 1.54 - 1.30 (m, 2H), 1.30 -1.17 (m, 2H), 1.10 1.04 (m, 5H), 0.96 - 0.93 (m, 2H), 0.84 - 0.80 (t, *J =* 6.9 Hz, 3H). |
| 347 | 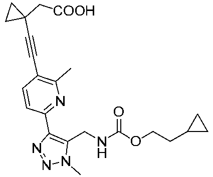 | 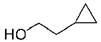 | LC-MS: [M+H]⁺ =438.05. |
| | | | ¹H NMR (400 MHz, DMSO-*d*₆) δ7.81 (d, *J =* 8.0 Hz, 1H), 7.71 (d, *J =* 8.0 Hz, 1H), 7.50 (s, 1H), 4.79 (d, *J =* 4.8 Hz, 2H), 4.06 (s, 3H), 3.97 (t, *J* = 12.0 Hz, 2H), 2.57 (s, 3H), 2.41 (s, 2H), 1.40 (d, *J* = 6.4 Hz, 2H), 1.07 - 0.89 (m, 4H), 0.64 (s, 1H), 0.34 (d*, J =* 7.2 Hz, 2H), 0.01 (d, *J* = 4.0 Hz, 1H). |
| 348 | 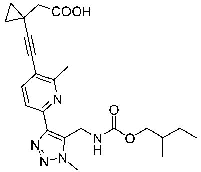 | 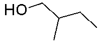 | LC-MS [M+H]⁺ =440.10. |
| | | | ¹H NMR (400 MHz, DMSO-*d₆*) δ 7.82 (d, *J* = 8.0 Hz, 1H), 7.72 (d, *J =* 8.0 Hz, 1H), 7.53 (s, 1H), 4.79 (d, *J* = 4.4 Hz, 2H), 4.06 (s, 3H), 3.78 (m, 2H), 2.57 (s, 3H), 2.44 (s, 2H), 1.58 (d, *J* = 6.4 Hz, 1H), 1.32 (d, *J* = 6.8 Hz, 1H), 1.10 (s, 1H), 1.04 (m, 2H), 0.95 (m, 2H), 0.81 (d, *J* = 6.0 Hz, 6H). |

The compounds in the following table were prepared by referring to the preparation methods of the above Example 2, Example 3, Example 4, Example 9, Example 16, Example 17, Example 56, Example 65, *etc.* Taking Example 3 or Example 9 as an example, compound M004 in Example 3 or Example 9 was replaced with the corresponding Reactant 1 in the following table, and the starting material N-methylisobutylamine or (1-fluorocyclobutyl)methylamine hydrochloride was replaced with the corresponding Reactant 2 in the following table:

| No. | Compound structure | Reactant 1 | Reactant 2 | Characterization |
|---|---|---|---|---|
| 068 | 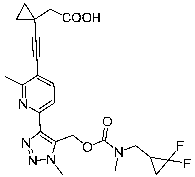 | 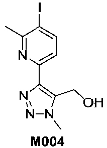 | 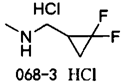 | LC-MS: [M+H]⁺ =474.10. |
| | | | | ¹H NMR (400 MHz, CD₃OD) δ 7.84 (d, *J* = 8.2 Hz, 1H), 7.72 (d, *J =* 8.2 Hz, 1H), 5.66 (d, *J* = 20.0 Hz, 2H), 4.11 (s, 3H), 3.58-3.01 (m, 2H), 2.81 (d, *J* = 19.6 Hz, 3H), 2.53 (s, 3H), 2.43 (s, 2H), 2.00 - 1.18 (m, 3H), 1.08 - 1.00 (m, 2H), 0.98 - 0.87 (m, 2H). |
| 073 | 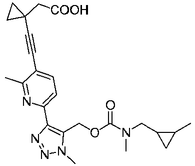 | 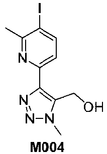 | 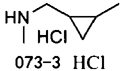 | LC-MS: [M+H]⁺ =452.10. |
| | | | | ¹H NMR (400 MHz, DMSO-*d*₆) δ7.84 (d, *J =* 8.0 Hz, 1H), 7.72 (d, *J* = 8.0 Hz, 1H), 5.65 (d, *J* = 12.0 Hz, 2H), 4.11 (s, 3H), 3.07 (d, *J* = 12.0 Hz, 2H), 2.81 (d, *J* = 16.0 Hz, 3H), 2.54 (s, 3H), 2.43 (s, 2H), 1.04 (dt, *J* = 12.0 Hz, 2H), 0.99 - 0.91 (m, 3H), 0.82 (s, 2H), 0.66 - 0.37 (m, 2H), 0.36 - 0.37 (m, 2H). |
| 082 | 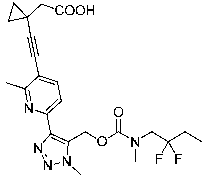 | 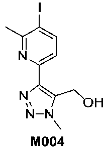 | 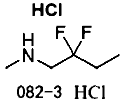 | LC-MS: [M+H]⁺ =476.10. |
| | | | | ¹H NMR (400 MHz, DMSO-*d*₆) δ7.85 (d, *J =* 8.0 Hz, 1H), 7.73 (d, *J* = 8.0 Hz, 1H), 5.69 (d, *J* = 16.0 Hz, 2H), 4.11 (d*, J* = 4.0 Hz, 3H), 3.72 (s, 1H), 2.88 - 2.76 (m, 3H), 2.53 (s, 3H), 2.43 (s, 2H), 2.07 - 2.01 (m, 1H), 1.86 - 1.78 (m, 1H), 1.69 - 1.62(m, 1H), 1.05 - 1.03 (m, 2H), 0.98 - 0.88 (m, 1H), 0.79 - 0.66 (m, 2H). |
| 089 | 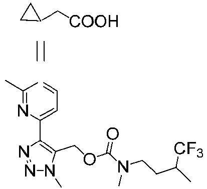 | 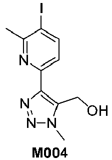 | 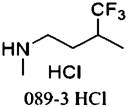 | LC-MS: [M+H]⁺= 508.15. |
| | | | | ¹H NMR (400 MHz, DMSO-*d*₆) δ 7.84 (d, *J* = 8.0 Hz, 1H), 7.72 (d, *J* = 8.0 Hz, 1H), 5.63 (q, *J* = 12.0 Hz, 2H), 4.11 (s, 3H), 3.24 (t, *J* = 48.4 Hz, 3H), 2.78 (d, *J =* 28.4 Hz, 3H), 2.52 (d, *J* = 1.6 Hz, 3H), 2.43 (s, 2H), 2.37 - 2.03 (m, 1H), 1.87 - 1.63 (m, 1H), 1.33 (d*, J =* 79.6 Hz, 1H), 1.07 (d,*J* = 6.0 Hz, 1H), 1.03 (t, *J =* 5.6 Hz, 2H), 0.95 (m, 2H), 0.74 (d, *J* = 6.4 Hz, 1H). |
| 094 | 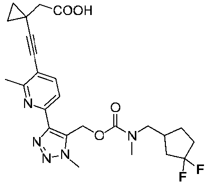 | 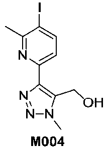 | 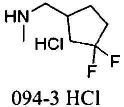 | LC-MS: [M+H]⁺ =502.15. |
| | | | | ¹H NMR (400 MHz, DMSO-*d*₆) δ7.82 (d*, J =* 8.0 Hz, 1H), 7.70 (d*, J =* 8.0 Hz, 1H), 5.63 (d, *J* = 20.0 Hz, 2H), 4.09 (s, 3H), 3.20 (s, 2H), 3.08 (s, 1H), 2.76 (d, *J* = 20.0 Hz, 3H), 2.51 (s, 3H), 2.42 (s, 2H), 2.25 - 2.00 (m, 2H), 1.94 - 1.73 (m, 2H), 1.59 (s, 1H), 1.45 - 1.15 (m, 1H), 1.03 (dd, *J* = 4.0, 4.0 Hz, 2H), 0.93 (dd, *J =* 4.0, 4.0 Hz, 2H). |
| 095 | 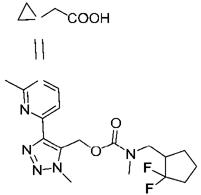 | 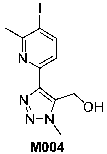 | 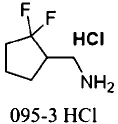 , its preparation refers to the preparation method of the hydrochlorid e of compound 026-3 in Example 26 | LC-MS: [M+H]⁺=502.10. |
| | | | | ¹H NMR (400 MHz, CD₃OD)δ 7.82 (d, *J* = 8.2 Hz, 1H), 7.71 (d, *J* = 8.2 Hz, 1H), 5.70 - 5.55 (m, 2H), 4.09 (s, 3H), 3.20 - 3.15 (m, 1H), 2.76 (d, *J* = 15.4 Hz, 3H), 2.52 (s, 3H), 2.43 (s, 2H), 2.23 - 1.16 (m, 8H), 1.06 - 1.01 (m, 2H), 0.96 - 0.91 (m, 2H). |
| 098 | 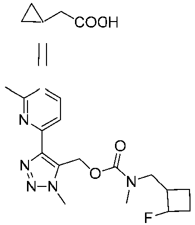 | 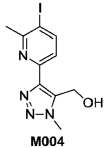 | 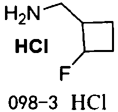 | LC-MS: [M+H]⁺ =470.15. |
| | | | | ¹H NMR (400 MHz, CD₃OD)δ 7.82 (d, *J* = 8.2 Hz, 1H), 7.71 (d, *J =* 8.2 Hz, 1H), 5.73 - 5.50 (m, 2H), 4.09 (s, 3H), 4.00 - 3.69 (m, 1H), 3.58 - 3.47 (m, 1H), 3.43 - 3.33 (m, 2H), 2.82 (d, *J* = 24.0 Hz, 3H), 2.51 (s, 3H), 2.42 (s, 2H), 1.07 - 1.00 (m, 2H), 0.96 - 0.90 (m, 2H), 0.85 - 0.23 (m, 4H). |
| 099 | 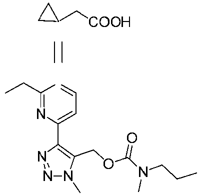 | 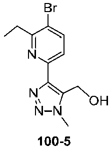 | 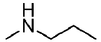 | LC-MS: [M+H]⁺ =440.10. |
| | | | | ¹H NMR (400 MHz, DMSO-*d₆*)δ 7.84 (d, *J* = 8.0 Hz, 1H), 7.72 (d, *J=* 8.0 Hz, 1H), 5.67 (d*, J =* 4.0 Hz, 2H), 4.12 (s, 3H), 3.13 - 3.12 (m, 1H), 3.03 - 2.90 (m, 1H), 2.90 - 2.88 (m, 2H), 2.78 - 2.73 (m, 3H), 2.43 (s, 2H), 1.45 (d, *J =* 4.0 Hz, 1H), 1.35-1.27 (m, 1H), 1.26 - 1.20 (m, 3H), 1.08 - 0.98 (m, 2H), 0.94 (dd, *J =* 4.0, 4.0 Hz, 2H), 0.81 - 0.60 (m, 3H). |
| 100 | 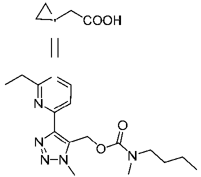 | 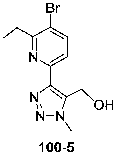 | 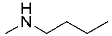 | LC-MS: [M+H]⁺ =454.20. |
| | | | | ¹H NMR (400 MHz, DMSO-*d₆*)δ 7.83 (d, *J* = 8.0 Hz, 1H), 7.72 (d, *J* = 8.0 Hz, 1H), 5.65 (s, 2H), 4.11 (s, 3H), 3.16 (s, 1H), 3.04 (s, 1H), 2.90 (dd, *J* = 8.0 Hz, 8.0 Hz ,2H), 2.73 (d, *J* = 16.0 Hz, 3H), 2.37 (s, 2H), 1.40 (s, 1H), 1.26 (s, 1H), 1.23 (d, *J* = 8.0 Hz, 4H), 0.99 (t, *J* =8.0 Hz, 3H), 0.94 (t, *J* = 12.0 Hz, 2H), 0.87 (t, *J =* 12.0 Hz, 1H), 0.64 (t, *J =* 12.0 Hz, 2H). |
| 101 | 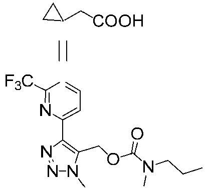 | 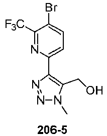 | 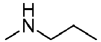 | LC-MS [M+H]⁺ = 480.10. |
| | | | | ¹H NMR (400 MHz, DMSO-*d*₆) δ 8.26 (d, *J* = 8.0 Hz, 1H), 8.08 (d, *J* = 8.0 Hz, 1H), 5.56 (d*, J* = 8.0 Hz, 2H), 4.14 (s, 3H), 3.11 - 3.01 (m, 2H), 2.73 - 2.67 (m, 3H), 2.44 (s, 2H), 1.46 - 1.41 (m, 1H), 1.33 - 1.18 (m, 2H), 1.07 - 0.97 (m, 4H), 0.79 - 0.76 (m, 1H), 0.61 - 0.57 (m, 1H). |
| 102 | 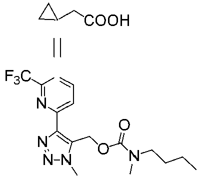 | 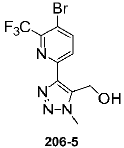 | 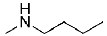 | LC-MS [M+H]⁺ = 494.10. |
| | | | | ¹H NMR (400 MHz, DMSO-*d*₆) δ 8.25 (d, *J* = 8.0 Hz, 1H), 8.07 (d, *J =* 8.0 Hz, 1H), 5.56 (d*, J =* 8.0 Hz, 2H), 4.14 (s, 3H), 3.14 - 3.03 (m, 2H), 2.72 (d, *J* = 16.0 Hz, 3H), 2.45 (s, 2H), 1.39 (s, 1H), 1.20 (s, 2H), 1.04 - 0.99 (m, 5H), 0.84 (s, 1H), 0.64 - 0.61 (m, 2H). |
| 105 | 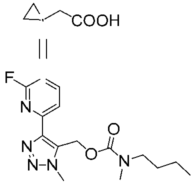 | 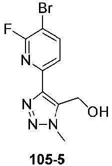 | 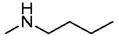 | LC-MS: [M+H]⁺ =444.10. |
| | | | | ¹H NMR (400 MHz, DMSO-*d*₆) δ 8.03 (m, 1H), 7.95 (m, 1H), 5.54 (d, *J* = 16.0 Hz, 2H), 4.12 (s, 3H), 3.16 (s, 2H), 3.08 (d, *J* = 6.4 Hz, 2H), 2.75 (d, *J=* 6.4 Hz, 3H), 2.43 (s, 2H), 1.40 (d, *J= 6.8* Hz, 1H), 1.29 - 1.16 (m, 2H), 1.07 - 0.99 (m, 3H), 0.96 (m, 2H), 0.77 (m, 3H). |
| 106 | 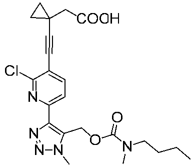 | 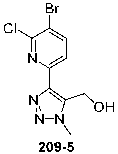 | 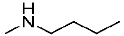 | LC-MS: [M+H]⁺ =460.17 |
| 230 | 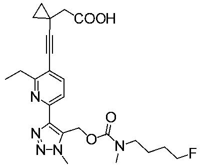 | 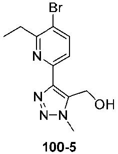 | 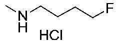 | LC-MS: [M+H]⁺ =472.10. |
| | | | | ¹H NMR (400 MHz, DMSO-*d*₆)δ7.84 (d, *J* = 8.0 Hz, 1H), 7.73 (d, *J* = 12.0 Hz, 1H), 5.67 (s, 2H), 4.52 - 4.26 (m, 2H), 4.12 (s, 3H), 3.21 (s, 1H), 3.11 (s, 1H), 2.90 (dd, *J* = 8.0,8.0 Hz, 2H), 2.75 (d, *J* = 20.0 Hz, 3H), 2.42 (s, 2H), 1.66-1.48 (m, 2H), 1.36 (s, 2H), 1.24 (t, *J* = 16.0 Hz, 3H), 1.07 - 0.99 (m, 2H), 0.98 - 0.91 (m, 2H). |
| 231 | 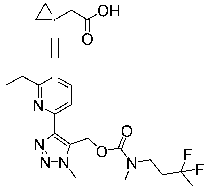 | 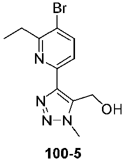 | Mixture of 037-3 hydrochlorid e and 037-4 hydrochlorid e | LC-MS: [M+H]⁺ =490.20. |
| | | | | ¹H NMR (400 MHz, DMSO-*d*₆)δ7.83 (d, *J =* 8.4 Hz, 1H), 7.71 (d, *J =* 8.0 Hz, 1H), 5.70 (s, 2H), 4.11 (s, 3H), 3.33 - 3.20 (m, 2H), 2.91 (q, *J =* 22.4 Hz, 2H), 2.77 (d, *J* = 16.4 Hz, 3H), 2.19 (s, 2H), 2.15 - 1.87 (m, 2H), 1.51 (dt, *J =* 38.0, 38.0 Hz, 3H), 1.24 (t, *J =* 14.8 Hz, 3H), 1.00 - 0.93 (m, 2H), 0.92 - 0.86 (m, 2H). |
| 349 | 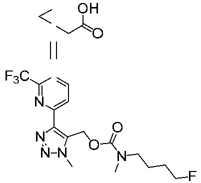 | 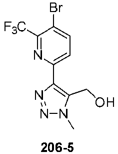 | 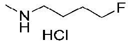 | LC-MS: [M+H]⁺ =512.47 |
| 350 | 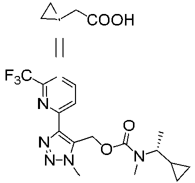 | 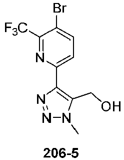 | 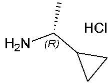 | LC-MS: [M+H]⁺= 506.49 |
| 351 | 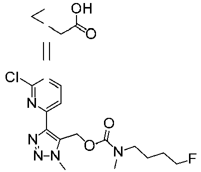 | 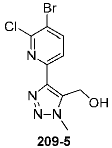 | 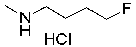 | LC-MS: [M+H]⁺ = 478.92 |
| 352 | 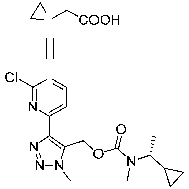 | 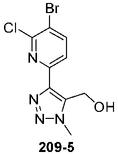 | 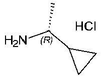 | LC-MS: [M+H]⁺ = 472.94 |
| 353 | 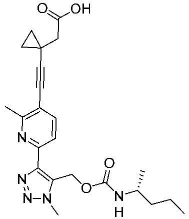 | 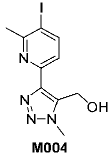 | 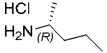 | LC-MS [M+H]⁺ = 440.10. |
| | | | | ¹H NMR (400 MHz, DMSO-*d*₆) δ 7.83 (d, *J* = 8.0 Hz, 1H), 7.72 (d, *J* = 8.0 Hz, 1H), 7.12 (d*, J =* 8.0 Hz, 1H), 5.69 - 5.60 (m, 2H), 4.09 (s, 3H), 3.50 - 3.47 (m, 1H), 2.54 (s, 3H), 2.39 (s, 2H), 1.38 - 1.20 (m, 4H), 1.02 - 0.99 (m, 5H), 0.96 - 0.92 (m, 2H), 0.84 - 0.81 (m, 3H). |
| 354 | 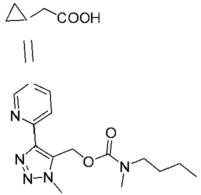 | 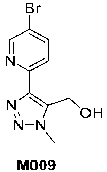 | 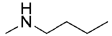 | LC-MS: [M+H]⁺ =426.15. |
| | | | | ¹H NMR (400 MHz, CD₃OD) δ 8.54 (s, 1H), 8.02 (d, *J* = 8.2 Hz, 1H), 7.83 (d, *J=* 8.2 Hz, 1H), 5.62 (d, *J* = 12.2 Hz, 2H), 4.11 (s, 3H), 3.11 (d, *J* = 39.8 Hz, 2H), 2.74 (d, *J* = 11.4 Hz, 3H), 2.44 (s, 2H), 1.41 (s, 1H), 1.29 - 1.14 (m, 2H), 1.09 - 0.98 (m, 3H), 0.97 - 0.91 (m, 2H), 0.89 - 0.64 (m, 3H). |
| 388 | 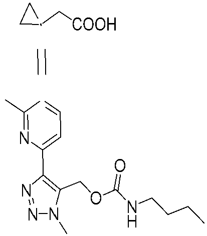 | 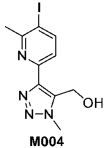 | 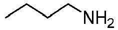 | LC-MS: [M+H]⁺ =426.10. |
| | | | | ¹H NMR (400 MHz, DMSO-*d*₆) δ 7.84 (d, *J* = 8.0 Hz, 1H), 7.73 (d*, J =* 8.0 Hz, 1H), 7.25 (t, *J =* 5.4 Hz, 1H), 5.65 (s, 2H), 4.09 (s, 3H), 2.97 (dd, *J* = 12.8, 6.4 Hz, 2H), 2.54 (s, 3H), 2.45 (s, 2H), 1.41 - 1.18 (m, 4H), 1.06 (s, 2H), 0.95 (s, 2H), 0.84 (t, *J =* 7.2 Hz, 3H). |
| 389 | 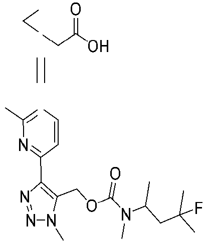 | 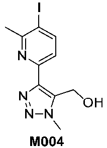 | 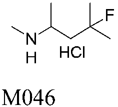 hydrochlorid e | LC-MS: [M+H]⁺ =486.10. |
| | | | | The compound contains a pair of enantiomers. |
| | | | | ¹H NMR (400 MHz, DMSO-*d*₆) δ 7.84 (d, *J* = 8.0 Hz, 1H), 7.73 (d*, J* = 8.0 Hz, 1H), 5.68 (s, 2H), 4.47 - 4.33 (m, 1H), 4.10 (d, *J* = 10.5 Hz, 3H), 2.61 (d, *J* = 30.2 Hz, 3H), 2.54 (s, 3H), 2.45 (s, 2H), 1.96 - 1.43 (m, 2H), 1.26 (dd,*J* = 21.6, 4.8 Hz, 3H), 1.12 - 0.91 (m, 9H). |
| 391 | 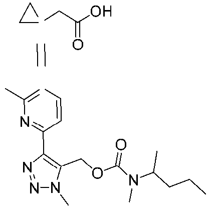 | 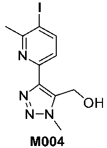 | 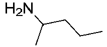 | LC-MS: [M+H]⁺ =454.15. |
| | | | | The compound contains a pair of enantiomers. |
| | | | | ¹H NMR (400 MHz, DMSO-*d*₆) δ 7.84 (d, *J* = 8.0 Hz, 1H), 7.73 (d*, J* = 8.0 Hz, 1H), 5.70 - 5.55 (m, 2H), 4.19 - 3.79 (m, 4H), 2.62-2.56 (m, 3H), 2.54 (s, 3H), 2.46 (s, 2H), 1.52 - 1.10 (m, 4H), 1.06 (dd, *J* = 6.4, 4.4 Hz, 2H), 1.03 - 0.89 (m, 5H), 0.74 (dt, *J* = 78.8, 6.8 Hz, 3H). |

The compounds in the following table were prepared by referring to the preparation method of compound 282 in Example 76, wherein compound M012 in Example 76 was replaced with the corresponding Reactant 1 in the following table, and the mixture of 037-3 hydrochloride and 037-4 hydrochloride was replaced with the corresponding Reactant 2 in the following table:

| No. | Compound structure | Reactant 1 | Reactant 2 | Characterization |
|---|---|---|---|---|
| 109 | 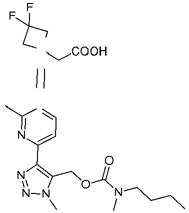 | 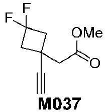 | 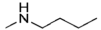 | LC-MS: [M+H]⁺ =490.15. |
| | | | | ¹H NMR (400 MHz, DMSO-*d*₆) δ 7.88 (d, *J =* 8.0 Hz, 1H), 7.78 (d, *J =* 8.0 Hz, 1H), 5.64 (d, *J* = 12.0 Hz, 2H), 4.11 (s, 3H), 3.08-2.93 (m, 6H), 2.83 (s, 2H), 2.75 (d, *J* = 20.0 Hz, 3H), 2.56 (s, 3H), 1.41 (s, 1H), 1.22 (s, 2H), 1.02 (d, *J* =8.0 Hz, 1H), 0.92-0.58 (m, 3H). |
| 111 | 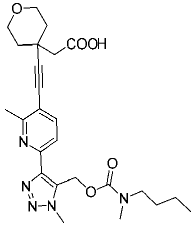 | 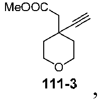 its preparation refers to the preparation method of compound M035 in Preparation Example 37 | 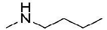 | LCMS: [M+H]⁺ =484.15. |
| | | | | ¹H NMR (400 MHz, DMSO-d6) δ 7.88 (d, J = 8.4 Hz, 1H), 7.81 (d, J = 8.4 Hz, 1H), 5.65 (d, J = 15.6 Hz, 2H), 4.12 (s, 3H), 3.81 (d, J = 10.0 Hz, 2H), 3.69 (t, J = 11.6 Hz, 2H), 3.22 - 3.01 (m, 2H), 2.75 (d, J = 15.2 Hz, 3H), 2.59 (s, 3H), 2.53 (s, 2H), 1.87 (d, J = 13.2 Hz, 2H), 1.75 - 1.68 (m, 2H), 1.42 (s, 1H), 1.22 - 1.21 (m, 2H), 1.03 - 0.97 (mz, 1H), 0.91 - 0.63 (m, 3H). |
| 112 | 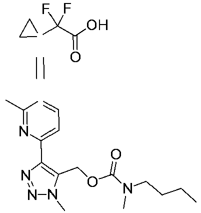 | 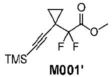 | 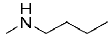 | LC-MS: [M+H]⁺ =476.15. |
| | | | | ¹H NMR (400 MHz, DMSO-*d*₆) δ7.86 (d, *J* = 8.0 Hz, 1H), 7.77 (d, *J* = 8.0 Hz, 1H), 5.64 (d, *J =* 12.0Hz, 2H), 4.11 (s, 3H), 3.18 (s, 1H), 3.06 (s, 1H), 2.75 (d, *J =* 16.0 Hz, 3H), 2.55 (s, 3H), 1.47 - 1.30 (m, 3H), 1.23 (s, 2H), 1.13 (s, 2H), 1.02 (d, *J* = 4.0 Hz, 1H), 0.92 - 0.60 (m, 3H). |
| 113 | 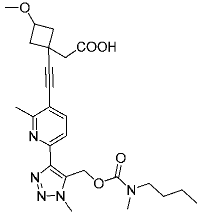 | 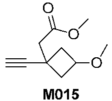 | 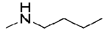 | LC-MS: [M+H]⁺ = 484.15. |
| | | | | The compound contains cis-trans isomers. |
| | | | | ¹H NMR (400 MHz, DMSO-*d*₆) δ 7.94 - 7.70 (m, 2H), 5.76 - 5.53 (m, 2H), 4.15 - 3.93 (m, 4H), 3.17 - 3.13 (m, 4H), 3.07 - 3.04 (m, 1H), 2.78 - 2.71 (m, 3H), 2.69 - 2.60 (m, 4H), 2.55 (d, *J* = 6.0 Hz, 3H), 2.27 - 2.11 (m, 2H), 1.48 - 1.35 (m, 1H), 1.28 - 1.15 (m, 2H), 1.08 - 0.96 (m, 1H), 0.89 - 0.63 (m, 3H). |
| 114 | 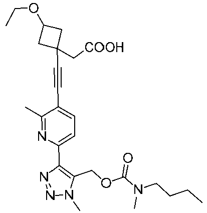 | 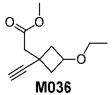 | 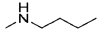 | LC-MS: [M+H]⁺ =498.25. |
| | | | | The compound contains cis-trans isomers. |
| | | | | ¹H NMR (400 MHz, DMSO-*d*₆) δ 7.87 (d, *J* = 8.0 Hz, 1H), 7.77 (d, *J =* 8.0 Hz, 1H), 5.64 (d, *J* = 16.0 Hz, 2H), 4.20 - 4.16 (m, 1H), 4.12 (s, 3H), 3.40 - 3.31 (m, 2H), 3.18 (s, 1H), 3.06 (s, 1H), 2.79 - 2.62 (m, 7H), 2.55 (d, *J* = 8.0 Hz, 3H), 2.32 - 2.21 (m, 1H), 2.17 - 2.11 (m, 1H), 1.43 - 1.40 (m, 1H), 1.27 - 1.17 (m, 2H), 1.12 - 1.01 (m, 4H), 0.88 - 0.85 (m, 1H), 0.68 - 0.65 (m, 2H). |
| 115 | 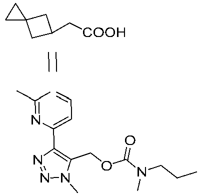 | 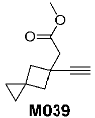 | 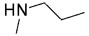 | LC-MS: [M+H]⁺ =466.15. |
| | | | | ¹H NMR (400 MHz, DMSO-*d*₆) δ 7.87 (d, *J* = 8.0 Hz, 1H), 7.78 (d, *J* = 8.0 Hz, 1H), 5.65 (d, *J* = 8.0 Hz, 2H), 4.12 (s, 3H), 3.14 - 3.04 (m, 2H), 2.85 - 2.65 (m, 5H), 2.62 - 2.51 (m, 5H), 2.37 (d, *J* = 12.0 Hz, 2H), 1.51 - 1.25 (m, 2H), 0.89 - 0.64 (m, 3H), 0.49 (s, 4H). |
| 249 | 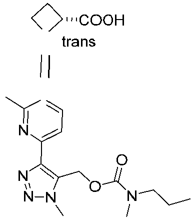 | 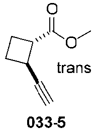 | 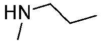 | LC-MS [M+H]⁺ = 426.48 |
| | | | | The compound contains a pair of trans isomers. |
| | | | | ¹H NMR (400 MHz, DMSO-*d*₆) δ7.83 (dd, *J =* 8.0,8.0 Hz, 2H), 5.64 (d, *J* = 12.0 Hz, 2H), 4.11 (s, 3H), 3.44 (d, *J* = 8.0 Hz, 1H), 3.20 - 3.15(m, 2H) ,3.16 (s, 1H), 2.75 (d, *J* = 20.0 Hz, 3H), 2.57 (s, 3H),2.46-2.01 (m, 4H),1.31-1.27 (m, 2H), 0.71 - 0.633 (m, 3H). |
| 250 | 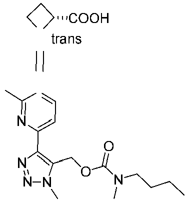 | 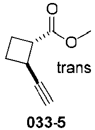 | 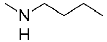 | LC-MS [M+H]⁺ = 440.51. |
| | | | | The compound contains a pair of trans isomers. |
| | | | | ¹H NMR (400 MHz, DMSO-*d*₆) δ 7.85 (dd, *J =* 19.2, 8.0 Hz, 2H), 5.64 (d, *J* = 12.4 Hz, 2H), 4.12 (s, 3H), 3.30 - 3.14 (m, 3H), 3.06 (s, 1H), 2.75 (d*, J =* 19.2 Hz, 3H), 2.57 (s, 3H), 2.24 (t, *J =* 8.8 Hz, 1H), 2.18 - 2.02 (m, 3H), 1.42 (s, 1H), 1.23 (s, 2H), 1.02 (d, *J* = 6.8 Hz, 1H), 0.86 - 0.67 (m, 3H). |
| 251 | 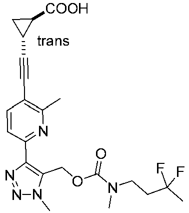 | 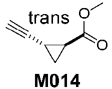 | Mixture of 037-3 hydrochloride and 037-4 hydrochloride | LC-MS [M+H]⁺ = 462.46 |
| | | | | The compound contains a pair of trans isomers. |
| 252 | 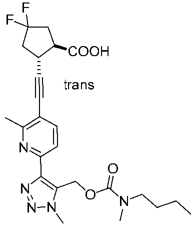 | 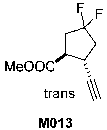 | 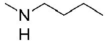 | LC-MS [M+H]⁺ = 490.20. |
| | | | | The compound contains a pair of trans isomers. |
| | | | | ¹H NMR (400 MHz, DMSO-*d*₆) δ 7.85 (d, J = 8.0 Hz, 1H), 7.77 (d, J = 8.0Hz, 1H), 5.64 (d, J = 12.0Hz, 2H), 4.11 (s, 3H), 3.54-3.48(m, 1H), 3.34 (s, 3H), 3.18 (s, 1H), 3.06 (s, 1H), 2.81 - 2.62 (m, 4H), 2.56 (s, 3H), 2.40 - 2.12 (m, 3H), 1.41 - 1.01 (m, 3H), 0.87 (s, 1H), 0.67 - 0.64 (m, 1H). |
| 355 | 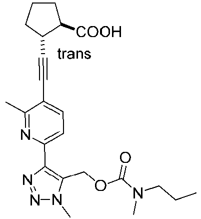 | 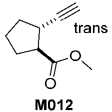 | 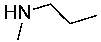 | LC-MS: [M+H]⁺ = 440.15. |
| | | | | The compound contains a pair of trans isomers. |
| | | | | ¹H NMR (400 MHz, DMSO-*d*₆) δ 7.83 (d, J = 8.0 Hz, 1H), 7.76 (d, J = 8.0 Hz, 1H), 5.64 (d, J = 11.2 Hz, 2H), 4.11 (s, 3H), 3.25 - 3.19 (m, 1H), 3.19 - 3.10 (m, 1H), 3.07 - 2.98 (m, 1H), 2.75 (d, J = 19.6 Hz, 3H), 2.69 - 2.60 (m, 1H), 2.55 (s, 3H), 2.10 - 2.00 (m, 1H), 2.00 - 1.90 (m, 1H), 1.80 - 1.59 (m, 4H), 1.45 (d, J = 7.2 Hz, 1H), 1.30 (d, J = 6.4 Hz, 1H), 0.71 (m, 3H). |
| 356 | 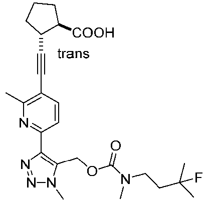 | 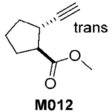 | 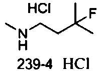 | LC-MS: [M+H]⁺ =486.20. |
| | | | | The compound contains a pair of trans isomers. |
| | | | | ¹H NMR (400 MHz, DMSO-*d*₆) δ 7.86 (d, J = 8.2 Hz, 1H), 7.79 (d, J = 8.2 Hz, 1H), 5.65 (d, J = 12.8 Hz, 2H), 4.12 (s, 3H), 3.34 - 3.26 (m, 1H), 3.16 (dd, J = 15.4, 7.6 Hz, 2H), 2.82 - 2.72 (m, 4H), 2.55 (s, 3H), 2.16 - 1.99 (m, 2H), 1.82 - 1.54 (m, 6H), 1.30 (d, J = 21.6 Hz, 3H), 1.10 (d, J = 21.6 Hz, 3H) |
| 357 | 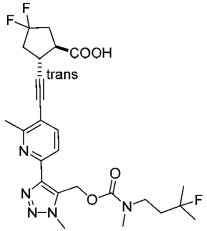 | 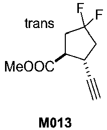 | 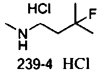 | LC-MS: [M+H]⁺ =522.20. |
| | | | | The compound contains a pair of trans isomers. |
| | | | | ¹H NMR (400 MHz, DMSO-*d*₆)δ7.88 (d, J = 4.0 Hz, 1H), 7.82 (d, J = 4.0 Hz, 1H), 5.66 (d, J = 12.0 Hz, 2H), 4.12 (d, J = 4.0 Hz, 3H), 3.47 (dd, J = 8.0, 8.0 Hz, 2H), 3.19 - 3.07 (m, 2H), 2.77 (d, J = 20.0 Hz, 3H), 2.72 - 2.63 (m, 2H), 2.62 - 2.53 (m, 4H), 2.39 - 2.25 (m, 2H), 1.83 - 1.72 (m, 1H), 1.64 - 1.54 (m, 1H), 1.31 (d, J = 12.0 Hz, 3H), 1.10 (d, J = 12.0 Hz, 3H) |
| 358 | 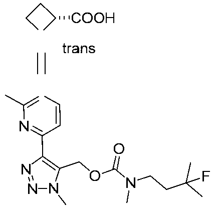 | 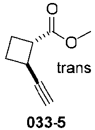 | 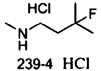 | LC-MS: [M+H]⁺=472.20 |
| | | | | The compound contains a pair of trans isomers. |
| | | | | ¹H NMR (400 MHz, DMSO-*d*₆) δ 7.84 (dd, J = 20.0, 8.0 Hz, 2H), 5.66 (d, J = 10.4 Hz, 2H), 4.12 (s, 3H), 3.49 - 3.42 (m, 1H), 3.32 - 3.28 (m, 1H), 3.21 - 3.14 (m, 2H), 2.77 (d, J = 20.0 Hz, 3H), 2.57 (s, 3H), 2.26 - 2.21 (m, 1H), 2.13 - 2.03 (m, 3H), 1.84 - 1.71 (m, 1H), 1.66 - 1.52 (m, 1H), 1.31 (d, J = 21.6 Hz, 3H), 1.10 (d, J = 21.6 Hz, 3H). |
| 359 | 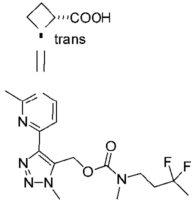 | 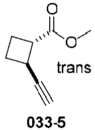 | Mixture of 037-3 hydrochloride and 037-4 hydrochloride | LC-MS: [M+H]⁺=476.20 |
| | | | | The compound contains a pair of trans isomers. |
| | | | | ¹H NMR (400 MHz, DMSO-*d*₆) δ 7.83 (q, J = 8.0 Hz, 2H), 5.65 (d, J = 13.2 Hz, 2H), 4.10 (s, 3H), 3.47 - 3.40 (m, 2H), 3.22 - 3.15 (m, 2H), 2.76 (d, J = 18.0 Hz, 3H), 2.55 (s, 3H), 2.23 - 2.19 m, 1H), 2.13 - 2.01 (m, 4H), 1.98 - 1.86 (m, 1H), 1.64 - 1.34 (m, 3H). |
| 360 | 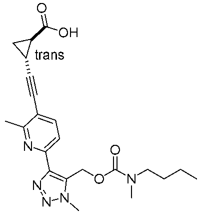 | 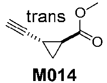 | 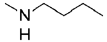 | LC-MS: [M+H]⁺ =426.10. |
| | | | | The compound contains a pair of trans isomers. |
| 361 | 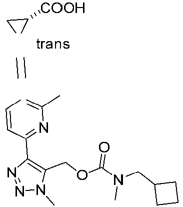 | 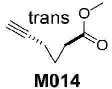 | 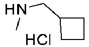 | LC-MS [M+H]⁺ =438.15. |
| | | | | The compound contains a pair of trans isomers. |
| | | | | ¹H NMR (400 MHz, DMSO-*d*₆) δ 7.83 (m, 2H), 5.63 (d, J = 13. Hz, 1H), 4.12 (s, 2H), 3.23 (d*,* J = 6.8 Hz, 1H), 3.10 (d, J = 6.8 Hz, 1H), 2.73 (d, J = 19.6 Hz, 3H), 2.56 (s, 3H), 2.32 - 2.30 (m, 1H), 2.05 - 1.91 (m, 1H), 1.84 - 1.55 (m, 2H), 1.46 (m, 1H), 1.33 (m, 1H), 1.29 - 1.22 (m, 1H). |
| 362 | 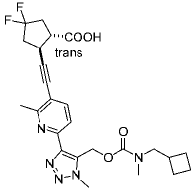 | 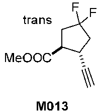 | 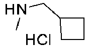 | LC-MS [M+H]⁺ = 502.20. |
| | | | | The compound contains a pair of trans isomers. |
| | | | | ¹H NMR (400 MHz, DMSO-*d*₆) δ 7.87 (d, J = 8.0 Hz, 1H), 7.82 (d, J = 8.0 Hz, 1H), 5.64 (d, J = 12.0 Hz, 2H), 4.12 (s, 3H), 3.50 - 3.43 (m, 2H), 3.24 - 3.22 (m, 1H), 3.15 - 3.08 (m, 2H), 2.79 - 2.66 (m, 4H), 2.58 (s, 3H), 2.43 - 2.21 (m, 3H), 1.97 - 1.43 (m, 6H) |

The compounds in the following table were prepared by referring to the preparation method of compound 019 in Example 19, wherein compound M006 was replaced with the corresponding Reactant 1 in the following table, and the starting material 2-chloro-4-isopropylpyrimidine was replaced with the corresponding Reactant 2 in the following table:

| No. | Compound structure | Reactant 1 | Reactant 2 | Characterization |
|---|---|---|---|---|
| 118 | 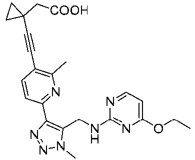 | 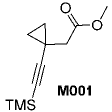 | 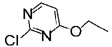 | LC-MS [M+H]⁺ =448.20. |
| | | | | ¹H NMR (400 MHz, DMSO-*d*₆) δ 8.00 (d*, J* = 5.2 Hz, 1H), 7.85 (d*, J* = 8.0 Hz, 1H), 7.73 (d*, J* = 8.0 Hz, 1H), 7.52 (s, 1H), 6.01 (d*, J* = 5.6 Hz, 1H), 5.03 (s, 2H), 4.11 (s, 5H), 2.56 (s, 3H), 2.40 (s, 2H), 1.14 (s, 3H), 1.02 (s, 2H), 0.96 (s, 2H). |
| 254 | 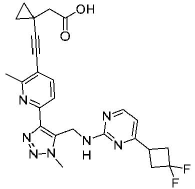 | 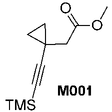 | 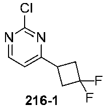 | LC-MS [M+H]⁺= 494.10. |
| | | | | ¹H NMR (400 MHz, DMSO-*d*₆) δ 8.21 (d, *J* = 4.0 Hz, 1H), 7.85 (d, *J* = 8.0 Hz, 1H), 7.73 (d, *J* = 8.0 Hz, 1H), 7.58 - 7.55 (m, 1H), 6.61 (d, *J* = 4.0 Hz, 1H), 5.03 (d*, J* = 4.0 Hz, 2H), 4.12 (s, 3H), 2.80 - 2.67 (m, 5H), 2.55 (s, 3H), 2.44 (s, 2H), 1.06-1.03 (m, 2H), 0.97-0.93 (m, 2H). |
| 255 | 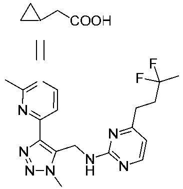 | 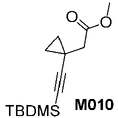 | 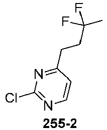 | LC-MS: [M+H]⁺ =495.05. |
| | | | | ¹H NMR (400 MHz, DMSO-*d₆*)δ 8.17 (d, *J* = 5.0 Hz, 1H), 7.84 (d, *J* = 8.2 Hz, 1H), 7.73 (d, *J* = 8.2 Hz, 1H), 7.42 (t, *J* = 5.8 Hz, 1H), 6.58 (d*, J* = 5.0 Hz, 1H), 4.99 (d, *J* = 5.8 Hz, 2H), 4.14 (s, 3H), 2.64 - 2.57 (m, 2H), 2.57 (s, 3H), 2.44 (s, 2H), 2.23 - 2.06 (m, 2H), 1.59 (t, *J =* 19.0 Hz, 3H), 1.04 (dd, *J* = 6.6, 4.4 Hz, 2H), 0.95 (dd, *J* = 6.6, 4.4 Hz, 2H). |
| 256 | 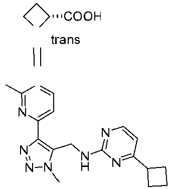 | 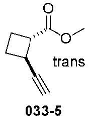 | 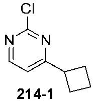 | LC-MS: [M+H]⁺=458.20. |
| | | | | ¹H NMR (400 MHz, DMSO-*d*₆) δ 8.16 (d, *J* = 4.8 Hz, 1H), 7.88 (d, *J* = 8.2 Hz, 1H), 7.82 (d, *J* = 8.2 Hz, 1H), 7.43 (t, *J =* 5.6 Hz, 1H), 6.49 (d*, J =* 5.0 Hz, 1H), 5.03 (d, *J* = 5.4 Hz, 2H), 4.15 (s, 3H), 3.46 (m, 1H), 3.22 - 3.15 (m, 1H), 2.61 (s, 3H), 2.24 (t, *J* = 8.5 Hz, 1H), 2.10 (m, 7H), 1.98 - 1.85 (m, 1H), 1.68 (s, 1H). |
| 363 | 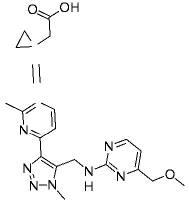 | 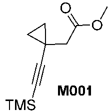 | 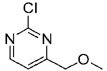 | LC-MS [M+H]⁺= 448.10. |
| | | | | ¹H NMR (400 MHz, DMSO-*d*₆) δ 8.27 (d, *J =* 4.0 Hz, 1H), 7.84 (d, *J =* 8.0 Hz, 1H), 7.73 (d, *J* = 8.0 Hz, 1H), 7.46 - 7.43 (m, 1H), 6.63 (d, *J =* 4.0 Hz, 1H), 4.96 (d, *J= 8.0* Hz, 2H), 4.18 (s, 2H), 4.16 (s, 3H), 3.32 (s, 3H), 2.57 (s, 3H), 2.46 (s, 2H), 1.07 - 1.04 (m, 2H), 0.97 - 0.94 (m, 2H). |
| 364 | 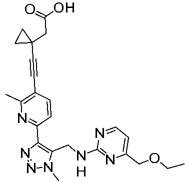 | 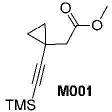 | 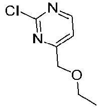 | LC-MS [M+H]⁺ =462.52. |
| | | | | ¹H NMR (400 MHz, DMSO-*d*₆) δ 8.35 (d, *J* = 5.2 Hz, 1H), 7.86 (d, *J* = 8.0 Hz, 1H), 7.77 (d, *J =* 8.0 Hz, 1H), 6.77 (d, *J* = 5.2 Hz, 1H), 5.01 (s, 2H), 4.28 (s, 2H), 4.17 (s, 3H), 3.54 - 3.46 (m, 2H), 2.59 (s, 3H), 2.46 (s, 2H), 1.23 (s, 1H), 1.16 (t, *J =* 7.2 Hz, 3H), 1.06 (s, 2H), 0.97 (s, 2H). |
| 365 | 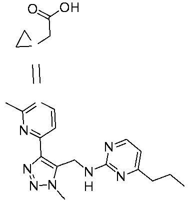 | 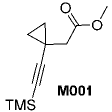 | 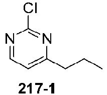 | LC-MS [M+H]⁺= 446.10. |
| | | | | ¹H NMR (400 MHz, DMSO-*d*₆) δ 8.14 (d, *J* = 4.8 Hz, 1H), 7.83 (d, *J* = 8.0 Hz, 1H), 7.73 (d, *J* = 8.0 Hz, 1H), 7.36 (m, 1H), 6.49 (d, *J* = 5.2 Hz, 1H), 4.97 (d, *J* = 6.0 Hz, 2H), 4.15 (s, 3H), 2.57 (s, 3H), 2.43 (s, 2H), 2.36 (m,2H), 1.51 (m, 2H), 1.04 (m, 2H), 0.95 (m, 2H), 0.83 (t, *J* = 7.2 Hz, 3H). |
| 366 | 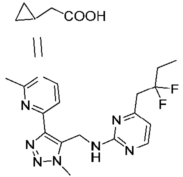 | 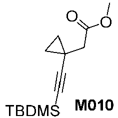 | 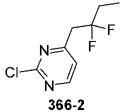 , its preparation refers to the method of Preparation Example 32 | LC-MS [M+H]⁺ = 496.52 |
| 367 | 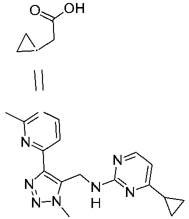 | 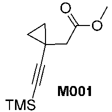 | 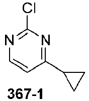 , its preparation refers to the preparation method of compound 214-1 in Example 52 | LC-MS: [M+H]⁺ =444.10. |
| | | | | ¹H NMR (400 MHz, DMSO-*d*₆) δ8.05 (d, *J* = 4.8 Hz, 1H), 7.85 (d, *J* = 4.4 Hz, 1H), 7.73 (d, *J* = 8.0 Hz, 1H), 7.42-7.31(m, 1H), 6.53 (d, *J* = 5.2 Hz, 1H), 4.97 (d, *J* = 6.0 Hz, 2H), 4.10 (s, 3H), 2.56 (s, 3H), 2.44 (s, 2H), 1.86 - 1.73 (m, 1H), 1.05 (dd, *J=* 5.2, 3.6 Hz, 2H), 0.95 (dd, *J* =4.0*,* 4.8 Hz, 2H), 0.88- 0.68 (m, 4H). |
| 368 | 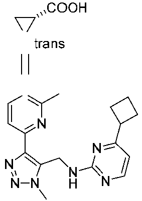 | 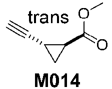 | 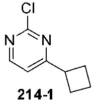 | LC-MS: [M+H]⁺= 444.10. |
| | | | | The compound contains a pair of trans isomers. |
| | | | | ¹H NMR (400 MHz, DMSO-*d*₆) δ 8.15 (d, *J* = 4.8 Hz, 1H), 7.86 (d, *J* = 8.0 Hz, 1H), 7.81 (d, *J =* 8.0 Hz, 1H), 7.41 (t, *J =* 5.6 Hz, 1H), 6.49 (d*, J =* 5.2 Hz, 1H), 5.01 (d, *J* = 5.6 Hz, 2H), 4.15 (s, 3H), 2.58 (s, 3H), 2.47 (s, 2H), 2.08 (d*, J* = 8.1 Hz, 4H), 1.92 (d, *J* = 7.6 Hz, 1H), 1.86 (m, 1H), 1.67 (s, 1H), 1.31 - 1.24 (m, 1H), 1.17 (t, *J* = 8.8 Hz, 1H). |

The compounds in the following table were prepared by referring to the preparation method of compound 216 in Example 54, wherein the starting material 3,3-difluorocyclobutanecarboxylic acid was replaced with the corresponding Raw material 1 in the following table, or intermediate 216-1 was replaced with the corresponding Raw material 2 in the following table:

| No. | Compound structure | Raw material 1 | Raw material 2 | Characterization |
|---|---|---|---|---|
| 194 | 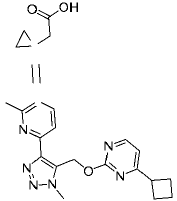 | 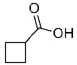 | / | LC-MS: [M+H]⁺ = 459.05. |
| | | | | ¹H NMR (400 MHz, DMSO-*d*₆) δ 8.49 (d, *J* = 5.2 Hz, 1H), 7.87 (d, *J* = 8.0 Hz, 1H), 7.73 (d*, J* = 8.0 Hz, 1H), 7.03 (d*, J* = 5.2 Hz, 1H), 6.08 (s, 2H), 4.15 (s, 3H), 3.51 (m, 2H), 2.42 (s, 2H), 2.39 (s, 2H), 2.20 - 2.12 (m, 4H), 2.03 - 1.88 (m, 1H), 1.78 - 1.67 (m, 1H), 1.03 (m, 2H), 0.93 (m, 2H). |
| 195 | 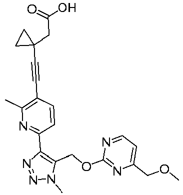 | / | 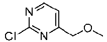 | LC-MS: [M+H]⁺ =449.10. |
| | | | | ¹H NMR (400 MHz, DMSO-*d*₆) δ 8.61 (d, *J* = 5.0 Hz, 1H), 7.85 (d, *J* = 8.2 Hz, 1H), 7.73 (d, *J* = 8.2 Hz, 1H), 7.16 (d, *J* = 5.0 Hz, 1H), 6.02 (s, 2H), 4.34 (s, 2H), 4.15 (s, 3H), 3.37 (s, 3H), 2.43 (s, 2H), 2.38 (s, 3H), 1.05 - 1.01 (m, 2H), 0.96 - 0.91 (m, 2H). |
| 196 | 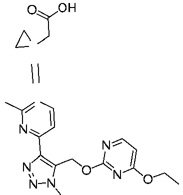 | / | 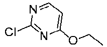 | LC-MS: [M+H]⁺ =449.19. |
| | | | | ¹H NMR (400 MHz, DMSO-*d*₆) δ 8.31 (d, *J* = 5.6 Hz, 1H), 7.87 (d, *J* = 8.0 Hz, 1H), 7.73 (d*, J* = 8.0 Hz, 1H), 6.57 (d, *J* = 5.6 Hz, 1H), 6.03 (s, 2H), 4.23 - 4.16 (m, 2H), 4.14 (s, 3H), 2.43 (s, 2H), 2.41 (s, 3H), 1.23 (t, *J* = 7.2 Hz, 3H), 1.03 (s, 2H), 0.94 (s, 2H). |
| 257 | 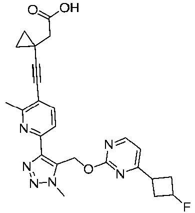 | 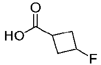 | / | LC-MS [M+H]⁺ =477.05. |
| | | | | ¹H NMR (400 MHz, DMSO-*d*₆) δ 8.52 (d, *J = 5.2* Hz, 1H), 7.87 (d, *J =* 8.0 Hz, 1H), 7.73 (d, *J =* 8.0 Hz, 1H), 7.17 - 7.06 (m, 1H), 6.07 (s, 2H), 5.45 - 4.89 (m, 1H), 4.16 (s, 3H), 3.10 - 2.95 (m, 1H), 2.54 - 2.47 (m, 3H), 2.44 - 2.34 (m, 6H), 1.02 (s, 2H), 0.94 (s, 2H). |
| 369 | 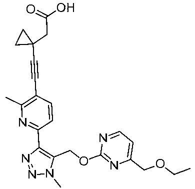 | 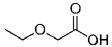 | / | LC-MS: [M+H]⁺ =463.05. |
| | | | | ¹H NMR (400 MHz, DMSO-*d*₆) δ8.61 (d, *J* = 4.8 Hz, 1H), 7.85 (d, *J* = 8.0 Hz, 1H), 7.73 (d, *J* = 8.0 Hz, 1H), 7.19 - 7.15 (m, 1H), 6.02 (s, 2H), 4.36 (s, 2H), 4.15 (s, 3H), 3.57 - 3.52 (m, 2H), 2.42 (s, 2H), 2.38 (s, 3H), 1.18 (t, *J =* 13.6 Hz, 3H), 1.02 (dd, *J* = 5.2, 3.6 Hz, 2H), 0.94 (dd, *J* = 3.6, 5.2 Hz, 2H). |
| 370 | 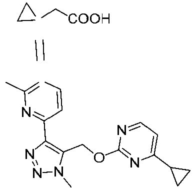 | 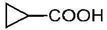 | / | LC-MS: [M+H]⁺ =455.05. |
| | | | | ¹H NMR (400 MHz, DMSO-*d*₆) δ8.37 (d, *J* = 4.8 Hz, 1H), 7.86 (d, *J* = 8.4 Hz, 1H), 7.72 (d, *J* = 8.0 Hz, 1H), 7.10 (d, *J* = 4.8 Hz, 1H), 5.99 (s, 2H), 4.12 (s, 3H), 2.40 (s, 5H), 2.06 - 1.98 (m, 1H), 1.04 - 0.95 (m, 4H), 0.95 - 0.86 (m, 4H). |

### Example 93: Preparation of compound 225-C and compound 225-D

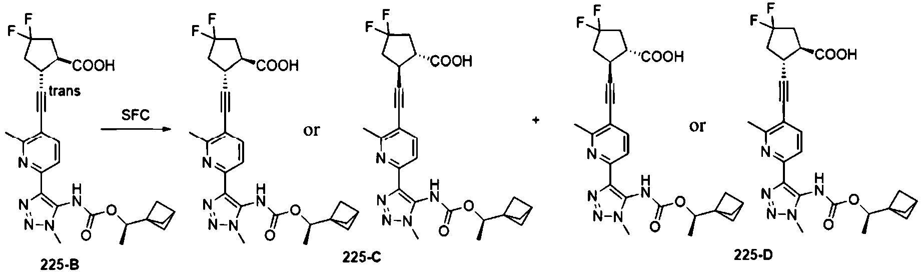

Compound 225-B (38 mg, 79 µmol) was subjected to chiral separation (Daicel Chiralpak IK column, 250 × 25 mm I.D, 10 µm; mobile phase A: supercritical carbon dioxide; mobile phase B: IPA and NH₃H₂O (NH₃H₂O accounts for 0.1 % of the volume of mobile phase B); volume ratio of mobile phase A to B: 75:25; isocratic elution; flow rate: 60 g/min; column temperature: room temperature; injection volume: 0.4 mL; detection wavelength: 220 nm) to obtain compound 225-C (retention time Rt = 7.630 min, 7.7 mg) and compound 225-D (retention time Rt = 10.274 min, 8.4 mg). LC-MS: [M+H]⁺ =500.15.

Compound 225-C: ¹H NMR (400 MHz, DMSO-*d*₆) δ 12.86 (s, 1H), 9.46 (s, 1H), 7.85 - 7.65 (m, 2H), 4.64 (s, 1H), 3.87 (s, 3H), 3.44 (dd, *J* = 8.0, 8.0 Hz, 1H), 3.10 (dd, *J* = 12.0,8.0 Hz, 1H), 2.75 - 2.55 (m, 2H), 2.54 (s, 3H), 2.38 - 2.23 (m, 2H), 1.64 (s, 6H), 1.22 (s, 1H), 1.09 (s, 3H).

Compound 225-D: ¹H NMR (400 MHz, DMSO-*d*₆) δ 12.88 (s, 1H), 9.47 (s, 1H), 7.86 - 7.74 (m, 2H), 4.75 - 4.55 (m, 1H), 3.88 (s, 3H), 3.46 (dd, *J=* 12.0, 8.0 Hz, 1H), 3.13 (dd, *J* =8.0,12.0 Hz, 1H), 2.76 - 2.57 (m, 2H), 2.56 (s, 3H), 2.38 - 2.24 (m, 2H), 1.64 (s, 6H), 1.23 (s, 1H), 1.10 (s, 3H).

### Example 94: Preparation of compound 229-A and compound 229-B

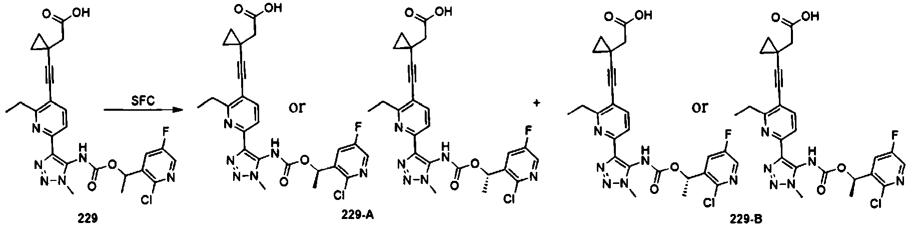

Compound 229 (42 mg) was subjected to chiral separation (Daicel Chiralpak IK column, 250 × 25 mm I.D., 10 µm; mobile phase A: supercritical carbon dioxide; mobile phase B: isopropanol and NH₃H₂O (NH₃H₂O accounts for 0.1% of the volume of mobile phase B); volume ratio of mobile phase A to B: 35:65; isocratic elution; flow rate: 80 g/min; column temperature: room temperature; injection volume: 3 µL; detection wavelength: 254 nm) to obtain compound 229-A (retention time Rt = 1.795 min, 5.2 mg) and compound 229-B (retention time Rt = 1.897 min, 3.1 mg). LC-MS: [M+H]⁺ =527.00.

Compound 229-A: ¹H NMR (400 MHz, DMSO-*d*₆) δ 8.37 (dd, *J* = 4.0, 4.0 Hz, 1H), 7.68 (d, *J* = 12.0 Hz, 1H), 7.64-7.55 (m, 1H), 7.40 (d, *J =* 20.0 Hz, 1H), 5.81 - 5.72 (m, 1H), 3.62 (d, *J=* 32.0 Hz, 3H), 2.80 (dd, *J=* 4.0,8.0 Hz, 2H), 2.18 (s, 2H), 1.37 (d, *J=* 8.0 Hz, 3H), 1.19-1.11 (m, 3H), 0.90 (s, 4H).

Compound 229-B: ¹H NMR (400 MHz, DMSO-*d*₆) δ 8.42-8.34 (m, 1H), 7.69 (d, *J* = 16.0 Hz, 1H), 7.62 (d, *J =* 8.0 Hz, 1H), 7.44 (d, *J=* 8.0 Hz, 1H), 5.79 (t, *J* = 12.0 Hz, 1H), 3.71-3.56 (m, 1H), 2.80 (dd, *J* = 8.0,8.0 Hz, 2H), 2.18 (s, 2H), 1.37 (t, *J* = 16.0 Hz, 3H), 1.19-1.12 (m, 3H), 0.90 (s, 4H).

### Example 95: Preparation of compound 243-A and compound 243-B

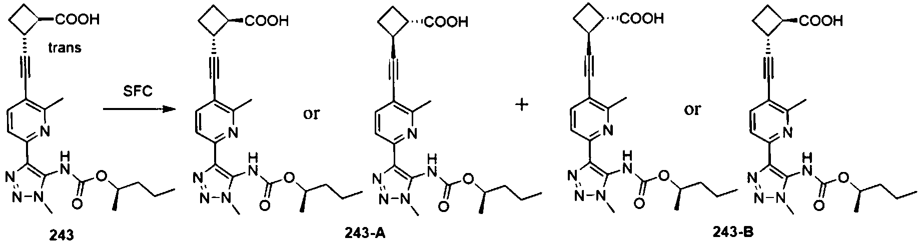

At room temperature, compound 243 (300 mg) was subjected to chiral separation (Daicel Chiralpak IG column, 250 × 25 mm I.D., 5 µm; flow rate: 55 g/min; column temperature: room temperature; mobile phase A: supercritical carbon dioxide; mobile phase B: EtOH and NH₃H₂O (NH₃H₂O accounts for 0.1% of the volume of mobile phase B); volume ratio of mobile phase A to B: 85:15; isocratic elution; injection volume: 2 µL; detection wavelength: 220 nm) to obtain compound 243-A (retention time Rt = 11.647 min, 102.0 mg) and compound 243-B (retention time Rt = 9.181 min, 98.9 mg). LCMS: [M+H]⁺= 426.15.

Compound 243-A: ¹H NMR (400 MHz, DMSO-*d*₆) δ 9.41 (s, 1H), 7.80 (d, *J* = 8.2 Hz, 1H), 7.76 (d, *J* = 8.2 Hz, 1H), 4.71 (s, 1H), 3.88 (s, 3H), 3.43 (m, 1H), 3.23 - 3.14 (m, 1H), 2.55 (s, 3H), 2.22 (t, *J*= 8.8 Hz, 1H), 2.14 - 1.99 (m, 3H), 1.19 (d, *J* = 30.4 Hz, 7H), 0.83 (s, 3H).

Compound 243-B: ¹H NMR (400 MHz, DMSO-*d*₆) δ 12.39 (s, 1H), 9.41 (s, 1H), 7.80 (d, *J=* 8.2 Hz, 1H), 7.76 (d, *J=* 8.2 Hz, 1H), 4.71 (s, 1H), 3.88 (s, 3H), 3.50 - 3.43 (m, 1H), 3.21 (m, 1H), 2.55 (s, 2H), 2.23 (t, *J=* 9.2 Hz, 1H), 2.15 - 2.04 (m, 3H), 1.20 (d, *J=* 24.8 Hz, 7H), 0.83 (s, 3H).

### Example 96: Preparation of compound 245-A and compound 245-B

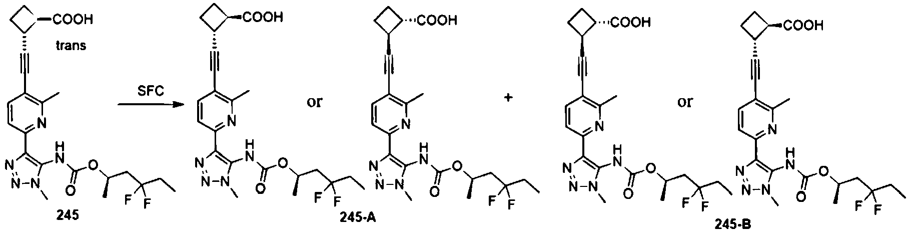

At room temperature, compound 245 (110 mg, 230 µmol) was subjected to chiral separation (Daicel Chiralpak IG column, 250 × 30 mm I.D., 5 µm; flow rate: 55 g/min; column temperature: room temperature; mobile phase A: supercritical carbon dioxide; mobile phase B: EtOH and NH₃H₂O (NH₃H₂O accounts for 0.1% of the volume of mobile phase B); volume ratio of mobile phase A to B: 85:15; isocratic elution; injection volume: 3 µL; detection wavelength: 220 nm) to obtain compound 245-A (retention time Rt = 10.806 min, 14.7 mg) and compound 245-B (retention time Rt = 12.817 min, 20.3 mg). LCMS: [M+H]⁺= 476.15.

Compound 245-A: ¹H NMR (400 MHz, DMSO-*d*₆) δ 9.50 (s, 1H), 7.78 (dd, *J* = 8.0,8.0 Hz, 2H), 4.98 (s, 1H), 3.88 (s, 3H), 3.45 (dd, *J=* 8.0, 8.0 Hz, 1H), 3.18 (dd, *J=* 8.0,8.0 Hz, 1H), 2.55 (s, 3H), 2.34 - 2.15 (m, 2H), 2.15 - 1.99 (m, 4H), 1.84 (s, 2H), 1.23 (s, 3H), 1.00 - 0.76 (m, 3H).

Compound 245-B: ¹H NMR (400 MHz, DMSO-*d*₆) δ 9.50 (s, 1H), 7.78 (dd, *J=* 8.0,8.0 Hz, 2H), 4.98 (s, 1H), 3.88 (s, 3H), 3.45 (dd, *J=* 8.0, 8.0 Hz, 1H), 3.19 dd, *J* = 8.0, 8.0 Hz, 1H), 2.55 (s, 3H), 2.35 - 2.15 (m, 2H), 2.15 - 1.98 (m, 4H), 1.84 (s, 2H), 1.23 (s, 3H), 0.97 - 0.74 (m, 3H).

### Example 97: Preparation of compound 247-A and compound 247-B

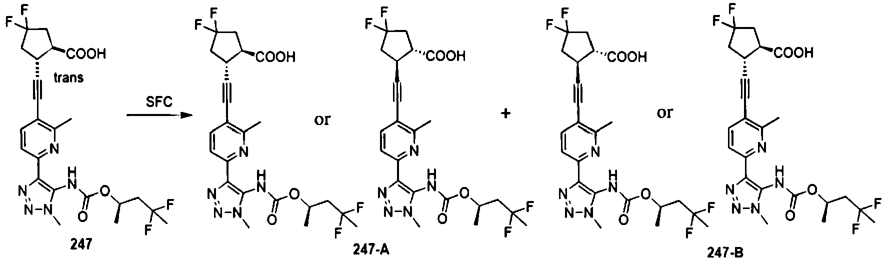

At room temperature, compound 247 (131.5 mg) was subjected to chiral separation (Daicel Chiralpak IK column, 250 × 25 mm I.D., 10 µm; flow rate: 55 g/min; column temperature: room temperature; mobile phase A: supercritical carbon dioxide; mobile phase B: IPA and NH₃H₂O (NH₃H₂O accounts for 0.1% of the volume of mobile phase B); volume ratio of mobile phase A to B: 85:15; isocratic elution; injection volume: 4 µL; detection wavelength: 220 nm) to obtain compound 247-A (retention time Rt = 9.416 min, 34.0 mg) and compound 247-B (retention time Rt = 14.040 min, 32.4 mg). LCMS: [M+H]⁺= 512.15.

Compound 247-A: ¹H NMR (400 MHz, DMSO-*d*₆) δ 13.00 (s, 1H), 9.49 (s, 1H), 7.80 - 7.67 (m, 2H), 4.94 (s, 1H), 3.83 (s, 3H), 3.42 (m, 1H), 3.07 (m, 1H), 2.73 - 2.52 (m, 2H), 2.50 (s, 3H), 2.38 - 1.97 (m, 4H), 1.56 (s, 3H), 1.19 (s, 3H).

Compound 247-B: ¹H NMR (400 MHz, DMSO-*d*₆) δ 12.87 (s, 1H), 9.53 (s, 1H), 7.91 - 7.65 (m, 2H), 4.99 (s, 1H), 3.88 (s, 3H), 3.46 (m, 1H), 3.12 (q, *J=* 9.2 Hz, 1H), 2.75 - 2.56 (m, 2H), 2.54 (s, 3H), 2.42 - 1.98 (m, 4H), 1.60 (s, 3H), 1.23 (s, 3H).

### Example 98: Preparation of compound 248-A and compound 248-B

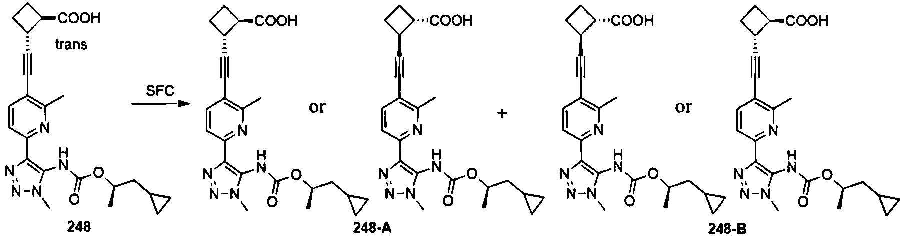

At room temperature, compound 248 (80 mg, 0.18 mmol) was subjected to chiral separation (YMC CHIRALART Cellulose-SC, IC column, 30 × 250 mm; flow rate: 38 mL/min; column temperature: room temperature; mobile phase A: n-hexane; mobile phase B: isopropanol; volume ratio of mobile phase A to B: 33:67; isocratic elution; injection volume: 1.2 mL; detection wavelength: 220 nm) to obtain compound 248-A (retention time Rt = 13.500 min, 19.4 mg) and compound 248-B (retention time Rt = 10.566 min, 19.8 mg). LC-MS: [M+H]+ =438.15.

Compound 248-A: ¹H NMR (400 MHz, DMSO-*d*₆) δ 9.42 (s, 1H), 7.80 - 7.74 (m, 2H), 4.76 (s, 1H), 3.89 (s, 3H), 3.48 - 3.42 (m, 1H), 3.21 - 3.16 (m, 1H), 2.55 (s, 3H), 2.26 - 2.04 (m, 4H), 1.69 - 1.03 (m, 6H), 0.36 (s, 2H), 0.01 (s, 2H).

Compound 248-B: ¹H NMR (400 MHz, DMSO-*d*₆) δ 9.41 (s, 1H), 7.80 - 7.74 (m, 2H), 4.76 (s, 1H), 3.89 (s, 3H), 3.48 - 3.42 (m, 1H), 3.23 - 3.18 (m, 1H), 2.55 (s, 3H), 2.28 - 2.04 (m, 4H), 1.35 - 1.23 (m, 6H), 0.36 (s, 2H), 0.01 (s, 2H).

### Example 99: Preparation of compound 278-A and compound 278-B

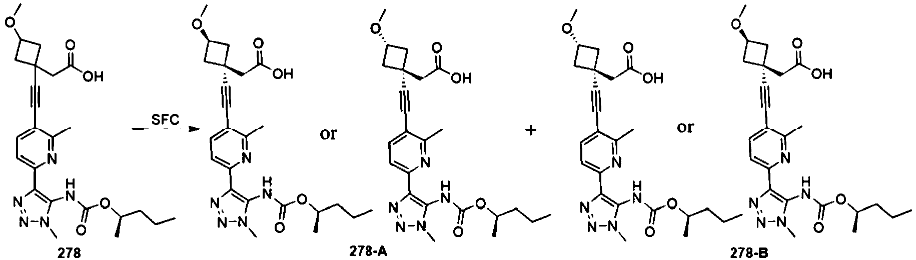

At room temperature, compound 278 (28 mg, 0.06 mmol) was subjected to chiral separation (Daicel Chiralpak IG column, 250 × 30 mm I.D., 5 µm, flow rate: 58 g/min; column temperature: room temperature; mobile phase A: supercritical carbon dioxide; mobile phase B: IPA and NH₃H₂O (NH₃H₂O accounts for 0.1% of the volume of mobile phase B); isocratic elution; volume ratio of mobile phase A to B: 83:17; injection volume: 1 µL, detection wavelength: 220 nm) to obtain compound 278-A (retention time Rt = 7.463 min, 6.5 mg) and compound 278-B (retention time Rt = 8.014 min, 7.9 mg). LCMS: [M+H]⁺=470.25.

Compound 278-B: ¹H NMR (400 MHz, DMSO-*d*₆) δ 9.40 (s, 1H), 7.80 - 7.68 (m, 2H), 4.70 (s, 1H), 4.12 - 4.03 (m, 2H), 3.94 - 3.83 (m, 3H), 3.15 (s, 3H), 2.66 - 2.56 (m, 4H), 2.54 (s, 3H), 2.20 - 2.09 (m, 2H), 1.37 - 1.13 (m, 7H), 0.90 - 0.80 (m, 2H).

### Example 100: Preparation of compound 288-A and compound 288-B

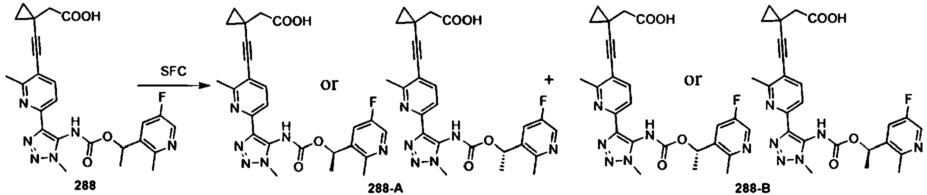

Compound 288 (130 mg, 264 µmol) was subjected to chiral separation (Daicel Chiralpak IG column, 250 × 30 mm I.D., 5 µm; mobile phase A: supercritical carbon dioxide; mobile phase B: EtOH and NH₃H₂O (NH₃H₂O accounts for 0.1% of the volume of mobile phase B); volume ratio of mobile phase A to B: 75:25; isocratic elution; flow rate: 60 g/min; column temperature: room temperature; injection volume: 4 µL; detection wavelength: 220 nm) to obtain compound 288-A (retention time Rt = 9.487 min, 34.4 mg) and compound 288-B (retention time Rt = 12.680 min, 43.2 mg). LC-MS: [M+H]⁺ =493.05.

Compound 288-A: ¹H NMR (400 MHz, DMSO-*d*₆) δ 8.35 (s, 1H), 7.73-7.63 (m, 3H), 5.83 (s, 1H), 3.88 (s, 3H), 2.46 (s, 3H), 2.40 (s, 5H), 1.45 (s, 3H), 1.01 (s, 2H), 0.95 (s, 2H).

Compound 288-B: ¹H NMR (400 MHz, DMSO-*d*₆) δ 8.35 (s, 1H), 7.73-7.63 (m, 3H), 5.83 (s, 1H), 3.88 (s, 3H), 2.46 (s, 3H), 2.42 (s, 5H), 1.47 (s, 2H), 1.02 (s, 2H), 0.96 (s, 2H).

### Example 101: Preparation of compound 292-A and compound 292-B

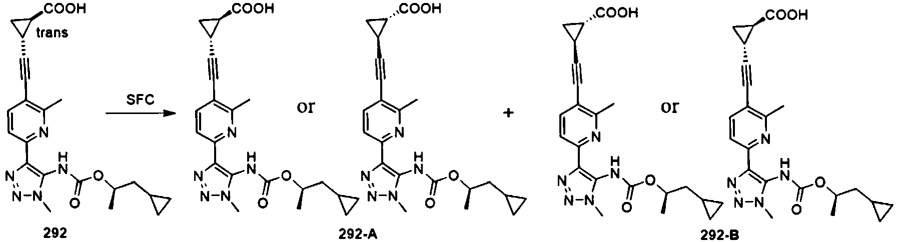

Compound 292 (70 mg, 0.15 mmol) was subjected to chiral separation (Daicel Chiralpak IK column, 250 × 25 mm I.D., 10 µm; mobile phase A: supercritical carbon dioxide; mobile phase B: IPA and NH₃H₂O (NH₃H₂O accounts for 0.1% of the volume of mobile phase B); volume ratio of mobile phase A to B: 30:70; isocratic elution; flow rate: 80 g/min; column temperature: room temperature; injection volume: 3 µL; detection wavelength: 220 nm) to obtain compound 292-A (retention time Rt = 1.560 min, 16.2 mg) and compound 292-B (retention time Rt = 1.830 min, 18.2 mg). LC-MS: [M+H]⁺=424.10.

Compound 292-A: ¹H NMR (400 MHz, DMSO-*d*₆) δ 9.42 (s, 1H), 7.80 - 7.74 (m, 2H), 4.75 (s, 1H), 3.88 (s, 3H), 2.52 (s, 3H), 2.08 - 1.95 (m, 2H), 1.58 - 0.92 (m, 9H), 0.35 - 0.01 (m, 4H).

Compound 292-B: ¹H NMR (400 MHz, DMSO-*d*₆) δ 9.43 (s, 1H), 7.80 - 7.73 (m, 2H), 4.76 (s, 1H), 3.88 (s, 3H), 2.52 (s, 3H), 2.06 - 1.93 (m, 2H), 1.58 - 1.08 (m, 9H), 0.36 (s, 2H), 0.00 (s, 2H).

### Example 102: Preparation of compound 294-A and compound 294-B

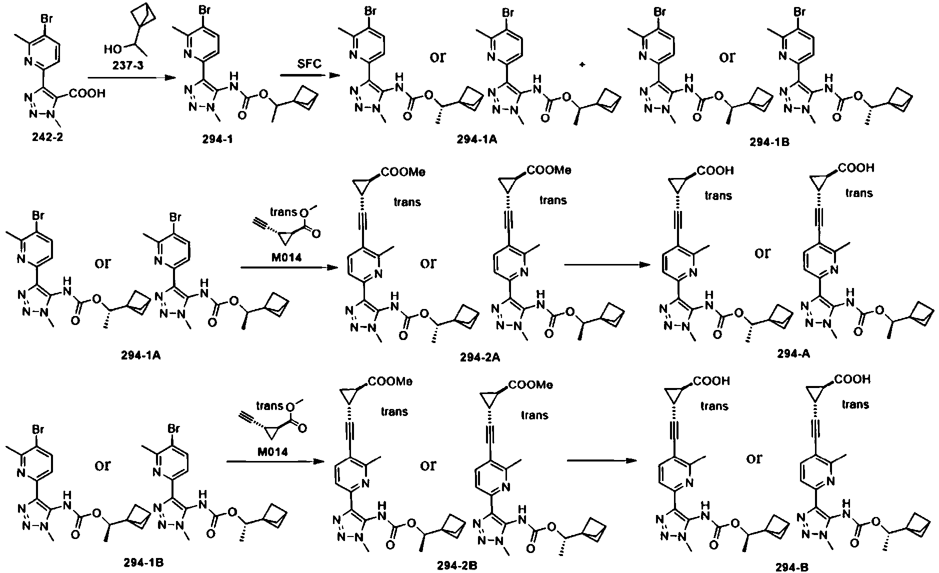

(1) At room temperature, compound 242-2 (330 mg, 1.11 mmol) was dissolved in anhydrous tetrahydrofuran (6 mL), followed by the addition of triethylamine (339 mg, 3.33 mmol), azidotrimethylsilane (383 mg, 3.33 mmol), and propylphosphonic anhydride (1.4 g, 2.23 mmol, 50% in ethyl acetate). The reaction mixture was stirred at room temperature for 0.5 hours. Then, compound 237-3 (250 mg, 2.23 mmol) was added. The reaction mixture was heated to 70°C and stirred for an additional 1 hour. The reaction mixture was cooled to room temperature, diluted with water (10 mL), and extracted with ethyl acetate (20 mL × 3). The organic phases were combined, dried over anhydrous sodium sulfate, filtered, and the filtrate was concentrated under reduced pressure. The residue was purified by silica gel column chromatography (petroleum ether/ethyl acetate = 5/1) to obtain compound 294-1 (420 mg, containing a pair of enantiomers). LC-MS: [M+H]⁺= 407.95.
(2) At room temperature, compound 294-1 (3 g, 7.37 mmol) was subjected to chiral separation (YMC CHIRALART cellulose-SC, 30 × 250 mm; mobile phase A: n-hexane, mobile phase B: isopropanol; volume ratio of mobile phase A to B: 25:75; flow rate: 30 mL/min; column temperature: room temperature; isocratic elution; injection volume: 2 µL; detection wavelength: 220 nm) to obtain compound 294-1A (retention time Rt = 21.5 min, 1.1 g) and compound 294-1B (retention time Rt = 27.1 min, 1.1 g). LC-MS: [M+H]+ =408.00.
(3) At room temperature, compound 294-1A (200 mg, 0.49 mmol) and compound M014 (120 mg, 0.98 mmol) were dissolved in 1,4-dioxane (5 mL), followed by the addition of bis(triphenylphosphine)palladium(II) chloride (34 mg, 0.049 mmol), copper(I) iodide (9 mg, 0.049 mmol), potassium carbonate (135 mg, 0.98 mmol), and triethylamine (149 mg, 1.47 mmol). Under a nitrogen atmosphere, the reaction mixture was heated to 90°C and stirred for 3 hours. The reaction mixture was cooled to room temperature, diluted with water (5 mL), and extracted twice with ethyl acetate (10 mL × 2). The organic phases were combined, dried over anhydrous sodium sulfate, filtered, and the filtrate was concentrated under reduced pressure. The residue was purified by silica gel column chromatography (petroleum ether/ethyl acetate = 2/1) to obtain compound 294-2A (180 mg, containing a pair of trans isomers). LC-MS: [M+H]⁺= 450.15.
(4) At room temperature, compound 294-2A (180 mg, 0.40 mmol) was dissolved in methanol (4 mL) and water (2 mL), followed by the addition of lithium hydroxide monohydrate (50 mg, 1.20 mmol). The reaction mixture was stirred at room temperature for 2 hours. A small amount of water (4 mL) was added to the reaction mixture, and the pH was adjusted to 5 to 6 with dilute hydrochloric acid (1 M). Then, the mixture was extracted with ethyl acetate (10 mL × 3). The organic phases were combined, dried over anhydrous sodium sulfate, filtered, and the filtrate was concentrated under reduced pressure. The residue was purified by preparative high-performance liquid chromatography (0.1% ammonia in water conditions) and lyophilized to obtain compound 294-A (77.2 mg, containing a pair of trans isomers). LC-MS: [M+H]⁺ =436.10.
   ¹H NMR (400 MHz, DMSO-*d*₆) δ 7.80 - 7.74 (m, 2H), 4.65 (s, 1H), 3.88 (s, 3H), 2.54 (s, 3H), 2.12 - 0.83 (m, 14H).
(5) At room temperature, compound 294-1B (200 mg, 0.49 mmol) and compound M014 (120 mg, 0.98 mmol) were dissolved in 1,4-dioxane (5 mL), followed by the addition of bis(triphenylphosphine)palladium(II) chloride (34 mg, 0.049 mmol), copper(I) iodide (9 mg, 0.049 mmol), potassium carbonate (135 mg, 0.98 mmol), and triethylamine (149 mg, 1.47 mmol). Under a nitrogen atmosphere, the reaction mixture was heated to 90°C and stirred for 3 hours. The reaction mixture was cooled to room temperature, diluted with water (5 mL), and extracted twice with ethyl acetate (10 mL × 2). The organic phases were combined, dried over anhydrous sodium sulfate, filtered, and the filtrate was concentrated under reduced pressure. The residue was purified by silica gel column chromatography (petroleum ether/ethyl acetate = 2/1) to obtain compound 294-2B (200 mg). LC-MS: [M+H]⁺ = 450.15.
(6) At room temperature, compound 294-2B (200 mg, 0.44 mmol) was dissolved in methanol (4 mL) and water (2 mL), followed by the addition of lithium hydroxide monohydrate (55 mg, 1.32 mmol). The reaction mixture was stirred at room temperature for 2 hours. A small amount of water (4 mL) was added to the reaction mixture, and the pH was adjusted to 5 to 6 with dilute hydrochloric acid (1 M). Then, the mixture was extracted with ethyl acetate (10 mL × 3). The organic phases were combined, dried over anhydrous sodium sulfate, filtered, and the filtrate was concentrated under reduced pressure. The residue was purified by preparative high-performance liquid chromatography (0.1% ammonia in water conditions) and lyophilized to obtain compound 294-B (77.7 mg, containing a pair of trans isomers). LC-MS: [M+H]⁺ =436.10.

¹H NMR (400 MHz, DMSO-*d*₆) δ 7.80 - 7.74 (m, 2H), 4.65 (s, 1H), 3.88 (s, 3H), 2.54 (s, 3H), 2.09 - 0.85 (m, 14H).

### Example 103: Preparation of compound 295-A and compound 295-B

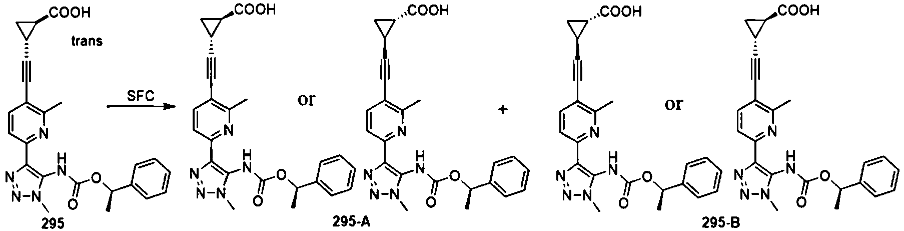

At room temperature, product 295 (124.6 mg, 280 µmol) was subjected to chiral separation (Daicel Chiral IG column, 250 × 30 mm I.D., 5 µm; mobile phase A: supercritical carbon dioxide; mobile phase B: IPA and NH₃H₂O (NH₃H₂O accounts for 0.1% of the volume of mobile phase B); volume ratio of mobile phase A to B: 80:20; isocratic elution; flow rate: 60 g/min; column temperature: room temperature; injection volume: 2 µL; detection wavelength: 220 nm) to obtain compound 295-A (retention time Rt = 12.971 min, 28.4 mg) and compound 295-B (retention time Rt = 10.044 min, 33.9 mg). LC-MS: [M+H]⁺ =446.20.

Compound 295-A: ¹H NMR (400 MHz, DMSO-*d*₆) δ 9.63 (s, 1H), 7.76 - 7.71 (m, 2H), 7.29 (s, 5H), 5.73 (s, 1H), 3.87 (s, 3H), 2.45 (s, 3H), 1.95 - 1.86 (m, 2H), 1.49 (s, 2H), 1.29 - 1.17 (m, 3H).

Compound 295-B: ¹H NMR (400 MHz, DMSO-*d*₆) δ 9.67 (s, 1H), 7.76 - 7.71 (m, 2H), 7.30 - 7.72 (m, 5H), 5.73 (s, 1H), 3.87 (s, 3H), 2.45 (s, 3H), 1.93 - 1.85 (m, 2H), 1.49 (s, 2H), 1.32 - 1.18 (m, 3H).

### Example 104: Preparation of compound 296-A and compound 296-B

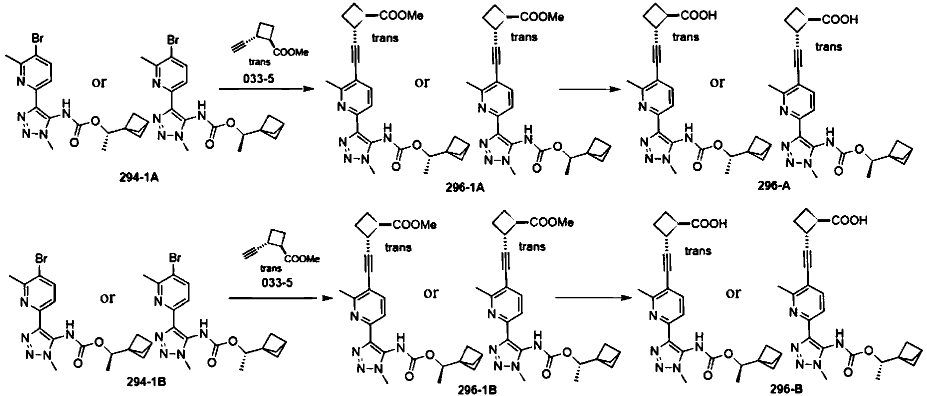

The preparation of compound 296-A and compound 296-B was referred to the preparation of compound 294-A and compound 294-B in Example 102, wherein compound M014 was replaced with compound 033-5, and compound 296-A (19.5 mg, containing a pair of trans isomers) and compound 296-B (50.3 mg, containing a pair of trans isomers) were obtained.

Compound 296-A: LC-MS:[M+H]⁺=450.20;

¹H NMR (400 MHz, DMSO-*d*₆) δ 9.44 (s, 1H), 7.80 (d, *J=* 8.2 Hz, 1H), 7.76 (d, *J=* 8.2 Hz, 1H), 4.66 (s, 1H), 3.88 (s, 3H), 3.45 (m, 2H), 3.19 (m, 2H), 2.56 (s, 3H), 2.22 (t, *J=* 8.4 Hz, 1H), 2.15 - 2.01 (m, 3H), 1.71 (d, *J* = 32.0 Hz, 6H), 1.12 (s, 2H).

Compound 296-B: LC-MS:[M+H]⁺=450.20;

¹H NMR (400 MHz, DMSO-*d*₆) δ 9.49 (s, 1H), 7.80 (d, *J=* 8.2 Hz, 1H), 7.76 (d, *J=* 8.2 Hz, 1H), 4.65 (s, 1H), 3.88 (s, 3H), 3.48 (s, 2H), 3.19 (m, 2H), 2.56 (s, 3H), 2.22 (t, *J* = 8.4 Hz, 1H), 2.13 - 2.01 (m, 3H), 1.58 (m, 6H), 1.11 (d, *J* = 9.6 Hz, 2H).

### Example 105: Preparation of compound 297-A and compound 297-B

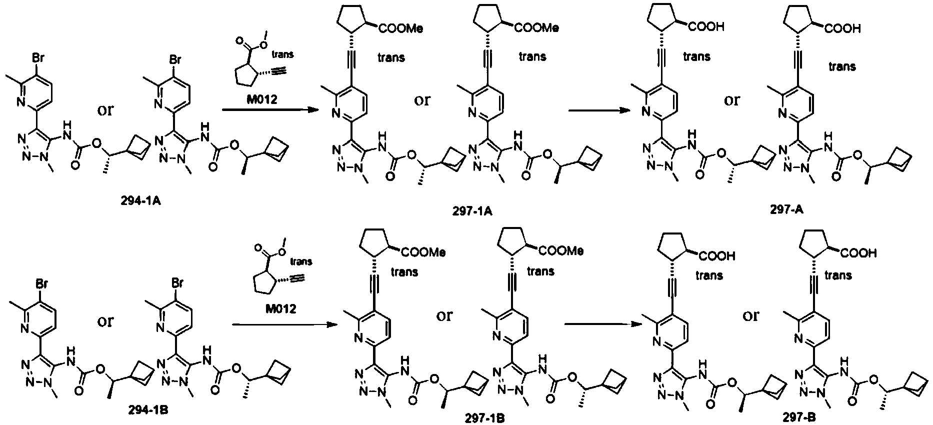

The preparation of compound 297-A and compound 297-B was referred to the preparation of compound 294-A and compound 294-B in Example 102, wherein compound M014 was replaced with compound M012, and compound 297-A (38.9 mg, containing a pair of trans isomers) and compound 297-B (50.3 mg, containing a pair of trans isomers) were obtained.

Compound 297-A: LC-MS:[M+H]⁺= 464.15;

¹H NMR (400 MHz, DMSO-*d*₆) δ 9.48 (s, 1H), 7.74 (s, 2H), 4.65 (s, 1H), 3.88 (s, 3H), 3.22 (dd, *J* = 14.6, 7.4 Hz, 1H), 2.63 (dd, *J=* 15.6, 7.4 Hz, 1H), 2.54 (s, 3H), 2.45 (s, 1H), 2.10 - 1.88 (m, 2H), 1.85 - 1.43 (m, 10H), 1.09 (s, 1H).

Compound 297-B: LC-MS:[M+H]⁺= 464.15;

¹H NMR (400 MHz, DMSO-*d*₆) δ 9.45 (s, 1H), 7.74 (s, 2H), 4.64 (s, 1H), 3.86 (s, 3H), 3.14 (dd, *J* = 15.6, 7.6 Hz, 1H), 2.76 (dd, *J* = 16.2, 8.0 Hz, 1H), 2.53 (s, 3H), 2.44 (s, 1H), 2.13 - 1.95 (m, 2H), 1.78 - 1.51 (m, 10H), 1.08 (s, 3H).

### Example 106: Preparation of compound 304-A and compound 304-B

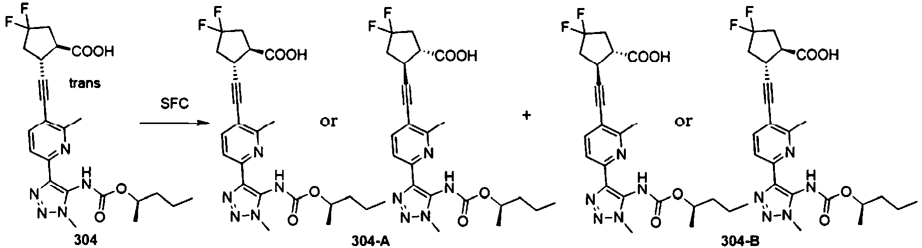

Compound 304 (78 mg, 164.2 µmol) was subjected to chiral separation (Daicel Chiralpak IK column, 250 × 25 mm I.D., 10 µm; mobile phase A: supercritical carbon dioxide; mobile phase B: IPA and NH₃H₂O (NH₃H₂O accounts for 0.1% of the volume of mobile phase B); volume ratio of mobile phase A to B: 72:28; isocratic elution; flow rate: 65 g/min; column temperature: room temperature; injection volume: 2 µL; detection wavelength: 220 nm) to obtain compound 304-A (retention time Rt = 8.732 min, 11.0 mg) and compound 304-B (retention time Rt = 6.579 min, 24.2 mg). LC-MS: [M+H]⁺ =476.15.

Compound 304-A: ¹H NMR (400 MHz, DMSO-*d*₆) δ 9.47 (s, 1H), 7.75 (s, 2H), 5.31 (s, 1H), 4.70 (s, 1H), 3.88 (s, 3H), 3.48 (dd, *J* = 8.0,8.0 Hz, 1H), 2.66 (dd, *J* = 8.4,8.4 Hz, 1H), 2.53 (s, 3H), 2.40 - 2.25 (m, 2H), 2.21 - 2.09 (m, 1H), 1.23 (t, *J* = 15.2 Hz, 6H), 0.95 - 0.70(m, 4H).

Compound 304-B: ¹H NMR (400 MHz, DMSO-*d*₆) δ 9.47 (s, 1H), 7.75 (s, 2H), 5.31 (s, 1H), 4.70 (s, 1H), 3.88 (s, 3H), 3.48 (dd, *J=* 8.4,8.4 Hz, 1H), 2.66 (dd, *J=* 8.4,8.4 Hz, 1H), 2.53 (s, 3H), 2.40 - 2.25 (m, 2H), 2.21 - 2.09 (m, 1H), 1.23 (t, *J* = 14.4 Hz, 6H), 0.97 -0.68(m, 4H).

### Example 107: Preparation of compound 305-A and compound 305-B

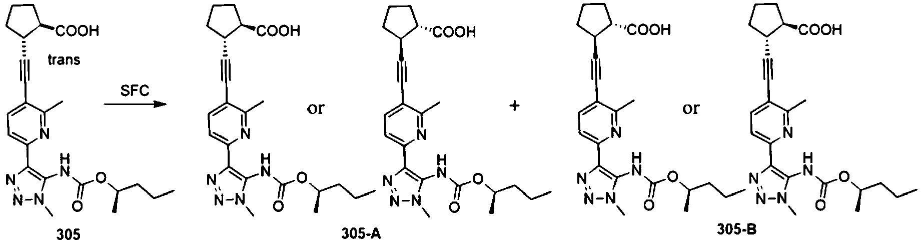

At room temperature, compound 305 (85.6 mg) was subjected to chiral separation (Daicel Chiralpak IG column, 250 × 30 mm I.D., 5 µm; flow rate: 60 g/min; column temperature: room temperature; mobile phase A: supercritical carbon dioxide; mobile phase B: EtOH and NH₃H₂O (NH₃H₂O accounts for 0.1% of the volume of mobile phase B); volume ratio of mobile phase A to B: 80:20; isocratic elution; injection volume: 3 µL; detection wavelength: 220 nm) to obtain compound 305-A (retention time Rt = 8.106 min, 26.1 mg) and compound 305-B (retention time Rt = 10.648 min, 22.7 mg). LCMS: [M+H]⁺= 440.15.

Compound 305-A: ¹H NMR (400 MHz, DMSO-*d*₆) δ 7.91 - 7.67 (m, 2H), 4.70 (s, 1H), 3.88 (s, 3H), 3.15 (dd, *J* = 15.6, 7.8 Hz, 1H), 2.78 (dd, *J=* 16.2, 8.2 Hz, 1H), 2.53 (s, 3H), 2.17 - 1.98 (m, 2H), 1.81 - 1.65 (m, 4H), 1.52 - 0.99 (m, 7H), 0.92 - 0.73 (m, 3H).

Compound 305-B: ¹H NMR (400 MHz, cdcl₃) δ 7.79 - 7.73 (m, 2H), 4.70 (s, 1H), 3.88 (s, 3H), 3.15 (dd, *J* = 15.8, 7.8 Hz, 1H), 2.78 (dd, *J* = 16.2, 8.2 Hz, 1H), 2.53 (s, 3H), 2.18 - 1.98 (m, 2H), 1.81 - 1.65 (m, 4H), 1.52 - 1.05 (m, 7H), 0.91 - 0.75 (m, 3H).

### Example 108: Preparation of compound 306-A and compound 306-B

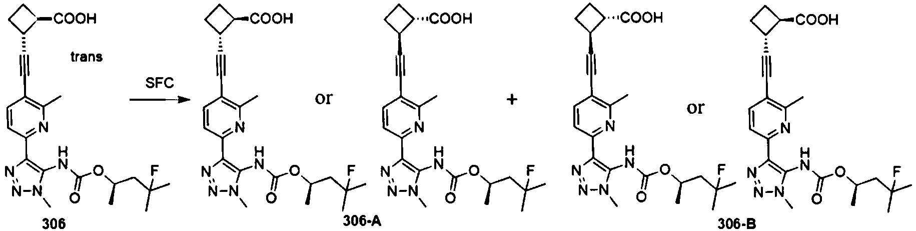

At room temperature, compound 306 (260.1 mg) was subjected to SFC chiral separation (Daicel Chiral IG column, 250 × 30 mm I.D., 5 µm; mobile phase A: supercritical carbon dioxide; mobile phase B: IPA and NH₃H₂O (NH₃H₂O accounts for 0.1% of the volume of mobile phase B); volume ratio of mobile phase A to B: 80:20; isocratic elution; flow rate: 60 g/min; column temperature: room temperature; injection volume: 5 µL; detection wavelength: 220 nm) to obtain compound 306-A (retention time Rt = 10.152 min, 90.9 mg) and compound 306-B (retention time Rt = 12.037 min, 88.5 mg). LCMS: [M+H]⁺= 458.15.

Compound 306-A: ¹H NMR (400 MHz, DMSO-*d₆*) δ 9.47 (s, 1H), 7.81 - 7.75 (m, 2H), 4.93 (s, 1H), 3.87 (s, 3H), 3.45 (d, *J=* 8.8 Hz, 1H), 3.20 (d, *J=* 8.8 Hz, 1H), 2.55 (s, 3H), 2.23 (t, *J=* 8.8 Hz, 1H), 2.14 - 2.03 (m, 3H), 1.82 (s, 2H), 1.42 - 1.09 (m, 9H).

Compound 306-B: ¹H NMR (400 MHz, DMSO-*d₆*) δ 9.47 (s, 1H), 7.81 - 7.75(m,2H), 4.94 (s, 1H), 3.87 (s, 3H), 3.45 (d, *J* = 8.8 Hz, 1H), 3.20 (d, *J=* 8.8 Hz, 1H), 2.55 (s, 3H), 2.24 (d, J = 8.8 Hz, 1H), 2.16 - 2.02 (m, 3H), 1.82 (s, 1H), 1.42 - 1.09 (m, 9H).

### Example 109: Preparation of compound 309-A and compound 309-B

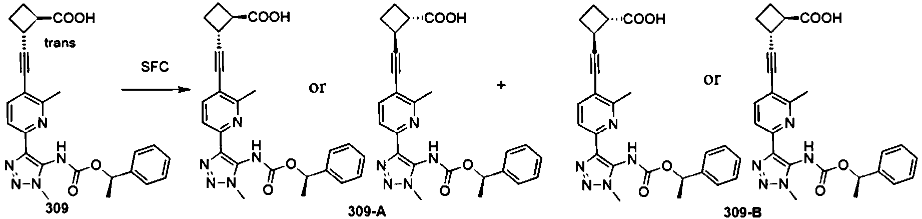

Compound 309 (214 mg, 0.47 mmol) was subjected to chiral separation (YMC CHIRALART cellulose-SC, 30 × 250 mm; flow rate: 30 mL/min; column temperature: room temperature; mobile phase A: n-hexane, mobile phase B: isopropanol; volume ratio of mobile phase A to B: 33:67; isocratic elution; injection volume: 3 µL; detection wavelength: 220 nm) to obtain compound 309-A (retention time Rt = 13.546 min, 47.2 mg) and compound 309-B (retention time Rt = 15.910 min, 58.3 mg). LCMS: [M+H]⁺=460.15.

Compound 309-A: ¹H NMR (400 MHz, DMSO-*d₆*)δ 12.46 (s,1H),9.64 (s,1H), 7.78 - 7.73 (m, 2H), 7.31 (d, *J=* 12.0 Hz, 5H), 5.73 (s, 1H), 3.88 (s, 3H), 3.47 - 3.42 (m, 1H), 3.24 - 3.19 (m, 1H), 2.47 (s, 3H), 2.25 (d, J=8.0 Hz, 2H), 2.14 - 2.07 (m, 3H), 1.47 (s, 3H).

Compound 309-B: ¹H NMR (400 MHz, DMSO-*d₆*) δ 12.41 (s,1H),9.65 (s,1H), 7.78 - 7.73 (m, 2H), 7.31 (d, *J=* 12.0 Hz, 5H), 5.73 (s, 1H), 3.88 (s, 3H), 3.47 - 3.43 (m, 1H), 3.24 - 3.19 (m, 1H), 2.50 (s, 3H), 2.25 (d, J=8.0 Hz, 2H), 2.14 - 2.08 (m, 3H), 1.48 (s, 3H).

### Example 110: Preparation of compound 338-A and compound 338-B

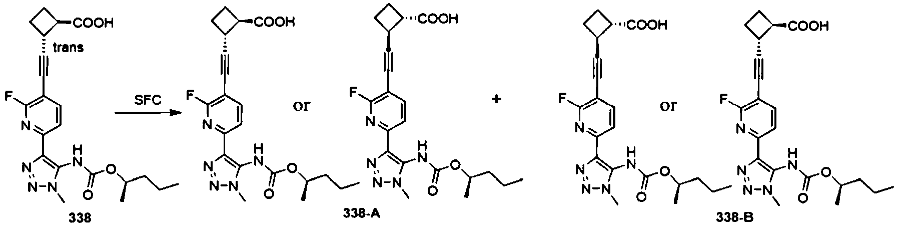

At room temperature, compound 338 (219 mg, 0.51 mmol) was subjected to chiral separation (Daicel Chiral IG column, 250 × 30 mm I.D., 5 µm; mobile phase A: supercritical carbon dioxide; mobile phase B: IPA and NH₃H₂O (NH₃H₂O accounts for 0.1% of the volume of mobile phase B); volume ratio of mobile phase A to B: 60:40; isocratic elution; flow rate: 70 g/min; column temperature: room temperature; injection volume: 3 µL; detection wavelength: 220 nm) to obtain compound 338-A (retention time Rt = 12.749 min, 86.8 mg) and compound 338-B (retention time Rt = 14.022 min, 90.3 mg). LCMS: [M+H]⁺=430.15.

Compound 338-A: ¹H NMR (400 MHz, DMSO-*d*₆) δ 12.60 (s, 1H), 9.62 (s, 1H), 8.28 - 8.00 (m, 1H), 7.88 (d, *J* = 7.6 Hz, 1H), 4.74 (s, 1H), 3.89 (s, 3H), 3.46 (d, *J=* 8.4 Hz, 1H), 3.19 (d, *J=* 8.8 Hz, 1H), 2.23 (t, *J=* 8.4 Hz, 1H), 2.17 - 2.02 (m, 3H), 1.23 (s, 7H), 0.87 (s, 3H).

Compound 338-B: ¹H NMR (400 MHz, DMSO-*d₆*) δ 12.51 (s, 1H), 9.61 (s, 1H), 8.21 - 8.00 (m, 1H), 7.88 (d, *J=* 7.6 Hz, 1H), 4.73 (s, 1H), 3.89 (s, 3H), 3.47 (d, *J=* 8.4 Hz, 1H), 3.20 (d, J = 8.8 Hz, 1H), 2.23 (t, *J =* 8.4 Hz, 1H), 2.16 - 2.02 (m, 3H), 1.23 (s, 7H), 0.86 (s, 3H).

### Example 111: Preparation of compound 340-A and compound 340-B

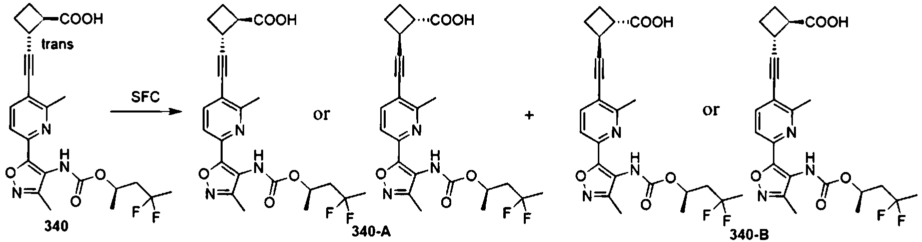

At room temperature, compound 340 (80 mg, 0.17 mmol) was subjected to chiral separation (Daicel Chiralpak IK column, 250 × 25 mm I.D., 10 µm; mobile phase A: supercritical carbon dioxide; mobile phase B: IPA and NH₃H₂O (NH₃H₂O accounts for 0.1% of the volume of mobile phase B); volume ratio of mobile phase A to B: 82:18; isocratic elution; flow rate: 57 g/min; column temperature: room temperature; injection volume: 2 µL; detection wavelength: 220 nm) to obtain compound 340-A (retention time Rt = 15.825 min, 24.9 mg) and compound 340-B (retention time Rt = 16.274 min, 29.8 mg). LC-MS: [M+H]⁺=462.15.

Compound 340-A: ¹H NMR (400 MHz, DMSO-*d*₆) δ 9.04 (s, 1H), 7.89 (d, *J* = 8.0 Hz, 1H), 7.62 (d, *J =* 8.0 Hz, 1H), 4.99 (d, *J* = 4.0 Hz, 1H), 3.49 - 3.43 (m, 1H), 3.22 - 3.17 (m, 1H), 2.59 (s, 3H), 2.28 - 2.17 (m, 5H), 2.16 - 1.95 (m, 4H), 1.62 (s, 3H), 1.33 - 1.32 (m, 3H).

Compound 340-B: ¹H NMR (400 MHz, DMSO-*d*₆) δ 9.04 (s, 1H), 7.89 (d, *J* = 8.0 Hz, 1H), 7.62 (d, *J =* 8.0 Hz, 1H), 5.00 (s, 1H), 3.50 - 3.43 (m, 1H), 3.23 - 3.16 (m, 1H), 2.59 (s, 3H), 2.29 - 2.18 (m, 5H), 2.16 - 2.00 (m, 4H), 1.68 - 1.53 (m, 3H), 1.32 - 1.20 (m, 3H).

### Example 112: Preparation of compound 343-A and compound 343-B

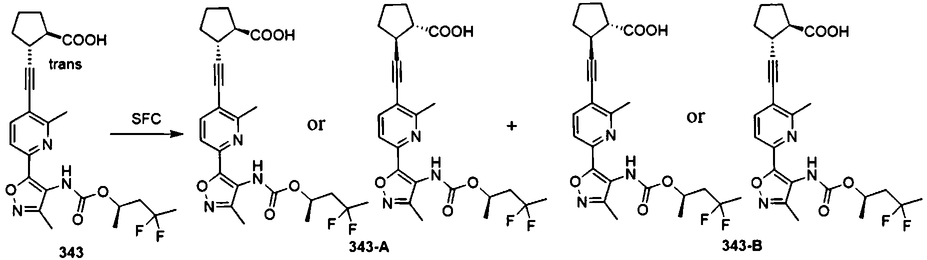

At room temperature, compound 343 (80 mg, 0.16 mmol) was subjected to chiral separation (Daicel Chiralcel OJ column, 250 × 30 mm I.D., 5 µm; mobile phase A: supercritical carbon dioxide; mobile phase B: EtOH and NH₃H₂O (NH₃H₂O accounts for 0.1% of the volume of mobile phase B); volume ratio of mobile phase A to B: 88:12; isocratic elution; flow rate: 53 g/min; column temperature: room temperature; injection volume: 2 µL; detection wavelength: 220 nm) to obtain compound 343-A (retention time Rt = 7.679 min, 19.7 mg) and compound 343-B (retention time Rt = 8.289 min, 17.6 mg). LC-MS: [M+H]⁺=476.15.

Compound 343-A: ¹H NMR (400 MHz, DMSO-*d*₆) δ 9.03 (s, 1H), 7.86 (d, *J* = 8.0 Hz, 1H), 7.61 (d, *J* = 8.0 Hz, 1H), 5.00 (s, 1H), 3.20 - 3.14 (m, 1H), 2.82 - 2.76 (m, 1H), 2.57 (s, 3H), 2.34 - 1.97 (m, 7H), 1.82 - 1.47 (m, 7H), 1.23 (s, 3H).

Compound 343-B: ¹H NMR (400 MHz, DMSO-*d*₆) δ 9.03 (s, 1H), 7.86 (d, *J* = 8.0 Hz, 1H), 7.60 (d, *J =* 8.0 Hz, 1H), 4.98 (s, 1H), 3.21 - 3.17 (m, 1H), 2.79 - 2.73 (m, 1H), 2.57 (s, 3H), 2.23 - 1.98 (m, 7H), 1.81 - 1.56 (m, 7H), 1.23 (s, 3H).

### Example 113: Preparation of compound 357-A and compound 357-B

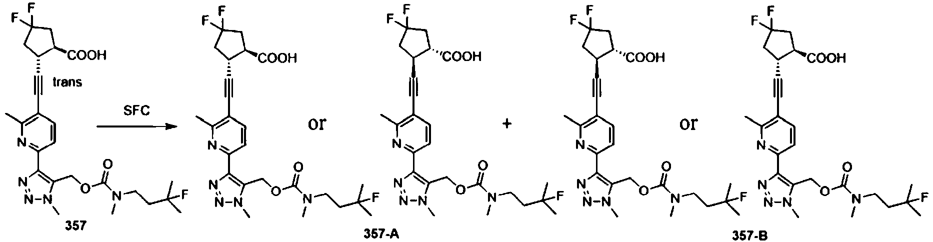

Compound 357 (20.3 mg, 38.9 µmol) was subjected to chiral separation (Daicel Chiralpak AD column, 250 × 50 mm I.D., 10 µm; flow rate: 55 g/min; column temperature: room temperature; mobile phase A: supercritical carbon dioxide; mobile phase B: EtOH and NH₃H₂O (NH₃H₂O accounts for 0.1% of the volume of mobile phase B); volume ratio of mobile phase A to B: 85:15; isocratic elution; injection volume: 3 µL; detection wavelength: 220 nm) to obtain compound 357-A (retention time Rt = 13.506 min, 1.0 mg) and compound 357-B (retention time Rt = 18.936 min, 2.1 mg). LCMS: [M+H]⁺ = 522.20.

### Example 114: Preparation of compound 358-A and compound 358-B

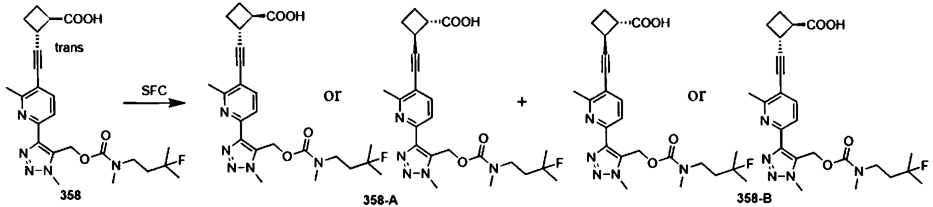

At room temperature, compound 358 (56 mg, 0.12 mmol) was subjected to chiral separation (Daicel Chiralpak OX column, 250 × 25 mm I.D., 10 µm; mobile phase A: supercritical carbon dioxide; mobile phase B: EtOH and NH₃H₂O (NH₃H₂O accounts for 0.1% of the volume of mobile phase B); volume ratio of mobile phase A to B: 80:20; isocratic elution; flow rate: 55 g/min; injection volume: 3 µL; detection wavelength: 220 nm) to obtain compound 358-A (retention time Rt = 18.880 min, 6.1 mg) and compound 358-B (retention time Rt = 16.445 min, 6.9 mg). LC-MS: [M+H]⁺= 472.20.

Compound 358-A: ¹H NMR (400 MHz, DMSO-*d*₆) δ 7.83 (d, J = 8.0 Hz, 1H), 7.76 (d, J = 8.0 Hz, 1H), 5.64 (d, J = 9.2 Hz, 2H), 4.10 (s, 3H), 3.39 - 3.37 (m, 1H), 3.29 - 3.24 (m, 2H), 3.17 - 3.13 (m, 1H), 2.77 - 2.72 (m, 3H), 2.55 (s, 3H), 2.16 - 2.01 (m, 1H), 1.98 - 1.88 (m, 3H), 1.79 - 1.74 (m, 1H), 1.60 - 1.54 (m, 1H), 1.32 - 1.26 (m, 3H), 1.11 - 1.06 (m, 3H).

Compound 358-B: ¹H NMR (400 MHz, DMSO-*d*₆) δ 7.83 (d, J = 8.0 Hz, 1H), 7.76 (d, J = 8.0 Hz, 1H), 5.64 (d, J = 9.2 Hz, 2H), 4.10 (s, 3H), 3.39 - 3.36 (m, 1H), 3.29 - 3.22 (m, 2H), 3.17 -3.12(m, 1H), 2.78 - 2.73 (m, 3H), 2.55 (s, 3H), 2.13 - 2.11 (m, 1H), 2.02 - 1.86 (m, 3H), 1.81 - 1.69 (m, 1H), 1.56 (s, 1H), 1.35 - 1.24 (m, 3H), 1.11 - 1.06 (m, 3H).

### Example 115: Preparation of compound 360-A and compound 360-B

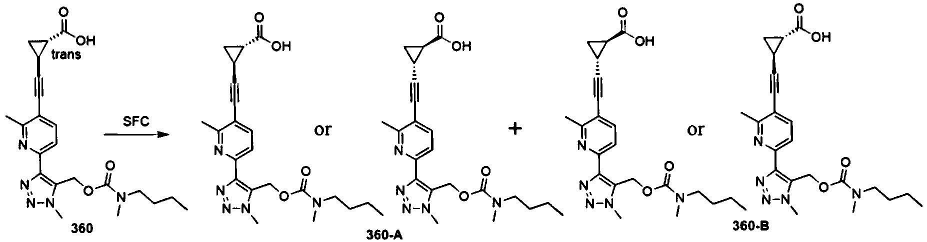

Compound 360 (100 mg) was subjected to chiral separation (CHIRAL ART Amylose-C NEO, AD-H column, 30 × 250 mm; mobile phase A: n-hexane, mobile phase B: ethanol; volume ratio of mobile phase A to B: 83:17; isocratic elution; flow rate: 30 mL/min; column temperature: room temperature; injection volume: 0.4 mL; detection wavelength: 254 nm) to obtain compound 360-A (retention time Rt = 10.346 min, 15.4 mg) and compound 360-B (retention time Rt = 14.749 min, 26.4 mg). LC-MS: [M+H]⁺=426.10.

Compound 360-A: ¹H NMR (400 MHz, DMSO-*d*₆) δ 7.83 (d, *J=* 8.2 Hz, 1H), 7.77 (d, *J* = 8.2 Hz, 1H), 5.64 (d,J= 13.8 Hz, 2H), 4.11 (s, 3H), 3.12 (d, *J* = 51.8 Hz, 2H), 2.75 (d, *J* = 20.8 Hz, 3H), 2.53 (s, 3H), 1.72 - 1.63 (m, 1H), 1.61 - 1.52 (m, 1H), 1.42 (s, 1H), 1.23 (s, 2H), 1.13 - 1.07 (m, 1H), 1.06 - 0.96 (m, 1H), 0.89 (s, 1H), 0.86 - 0.54 (m, 3H).

Compound 360-B: ¹H NMR (400 MHz, DMSO-*d*₆) δ 7.83 (d, *J =* 8.2 Hz, 1H), 7.78 (d, *J* = 8.2 Hz, 1H), 5.64 (d,*J* = 13.8 Hz, 2H), 4.11 (s, 3H), 3.12 (d, *J* = 51.6 Hz, 2H), 2.75 (d, *J* = 20.8 Hz, 3H), 2.54 (s, 3H), 1.71 - 1.64 (m, 1H), 1.60 - 1.53 (m, 1H), 1.42 (s, 1H), 1.27 - 1.18 (m, 2H), 1.13 - 1.07 (m, 1H), 1.05 - 0.98 (m, 1H), 0.94 - 0.90 (m, 1H), 0.88 - 0.61 (m, 3H).

### Example 116: Preparation of compound 462-A and compound 462-B

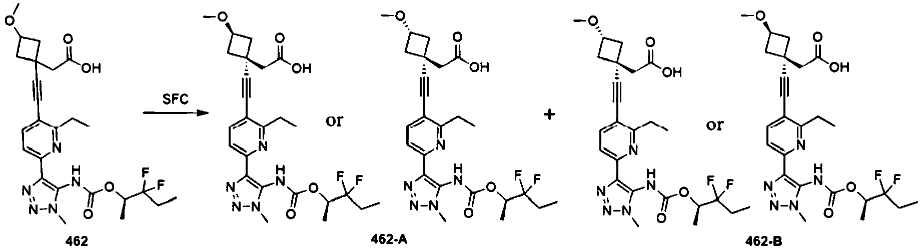

At room temperature, compound 462 (42.8 mg, 0.0820 mmol) was subjected to SFC chiral separation (Daicel Chiralpak IG column, 250 × 30 mm I.D., 5 µm; mobile phase A: supercritical carbon dioxide, mobile phase B: IPA and NH₃H₂O (NH₃H₂O accounts for 0.1% of the volume of mobile phase B); volume ratio of mobile phase A to B: 88:12; isocratic elution; flow rate: 55 g/min; column temperature: room temperature; injection volume: 2µL; detection wavelength: 220 nm) to obtain compound 462-A (retention time Rt = 13.164 min on SFC chromatogram, 6.8 mg) and compound 462-B (retention time Rt = 13.847 min on SFC chromatogram, 8.9 mg). LC-MS: [M+H]⁺=520.20.

### Example 117: Preparation of compound 463-A and compound 463-B

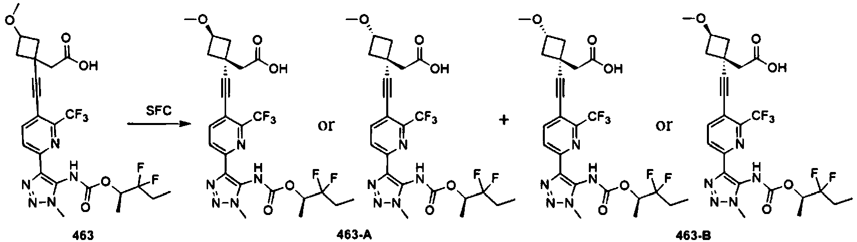

At room temperature, compound 463 (24.0 mg, 0.0430 mmol) was subjected to SFC chiral separation (Daicel Chiralpak OD column, 250 × 30 mm I.D., 5 µm; mobile phase A: supercritical carbon dioxide, mobile phase B: EtOH and NH₃H₂O (NH₃H₂O accounts for 0.1% of the volume of mobile phase B), volume ratio of mobile phase A to B: 88:12; isocratic elution; flow rate: 53 g/min, column temperature: room temperature, injection volume: 3 µL, detection wavelength: 220 nm) to obtain compound 463-A (retention time Rt = 9.828 min on the SFC chromatogram, 2.4 mg) and compound 463-B (retention time Rt = 10.576 min on the SFC chromatogram, 2.9 mg). LC-MS: [M+H]⁺=560.20.

The following compounds were synthesized by referring to the corresponding synthetic methods of Examples 1-107 above:

| Compound No. | LC-MS: [M+H]⁺ | Structural formula | Compound No. | LC-MS: [M+H]⁺ | Structural formula |
|---|---|---|---|---|---|
| 010-B | 426.10 | 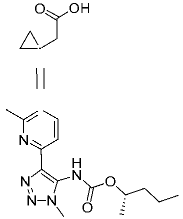 | 175 | 511.18 | 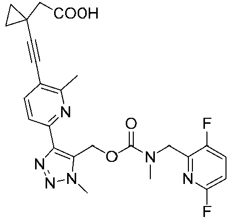 |
| 041 | 534.18 | 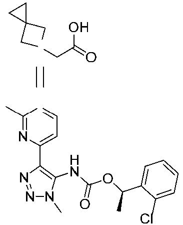 | 176 | 543.12 | 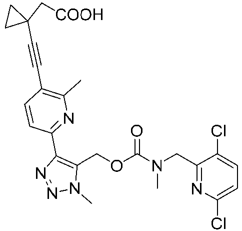 |
| 042 | 510.94 | 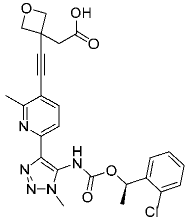 | 177 | 507.21 | 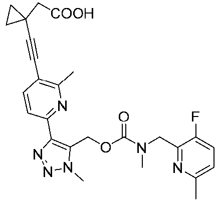 |
| 043 | 458.21 | 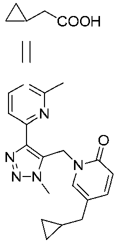 | 178 | 523.18 | 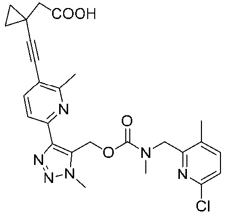 |
| 044 | 458.21 | 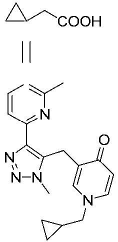 | 179 | 571.10 | 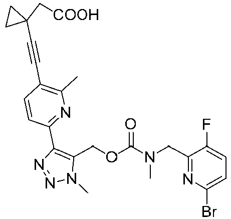 |
| 045 | 448.23 | 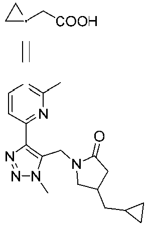 | 180 | 523.18 | 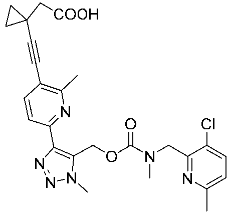 |
| 046 | 448.23 | 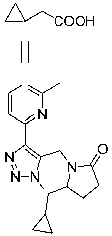 | 181 | 527.15 | 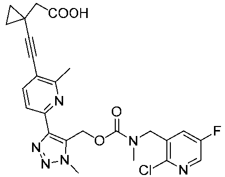 |
| 047 | 434.21 | 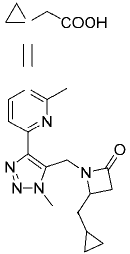 | 182 | 543.12 | 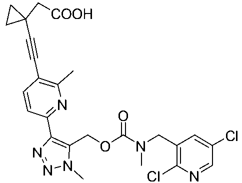 |
| 048 | 546.14 | 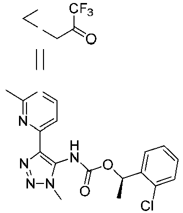 | 183 | 523.18 | 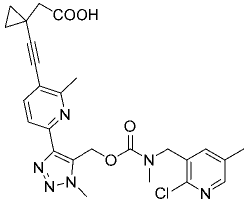 |
| 049 | 532.89 | 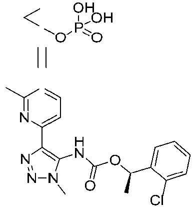 | 184 | 507.21 | 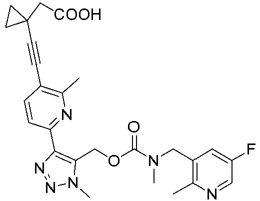 |
| 050 | 510.94 | 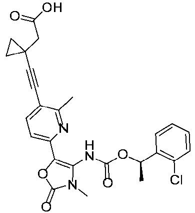 | 185 | 476.19 | 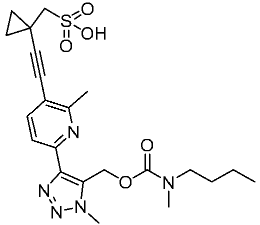 |
| 051 | 532.23 | 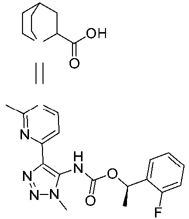 | 186 | 476.20 | 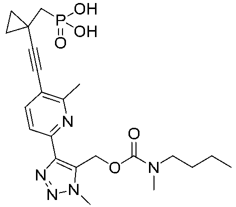 |
| 052 | 506.15 | 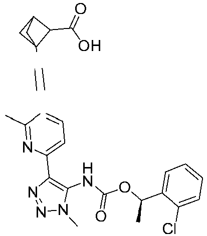 | 187 | 464.24 | 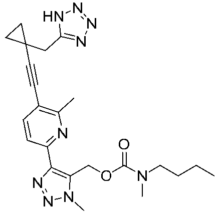 |
| 054 | 548.16 | 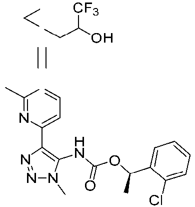 | 188 | 531.23 | 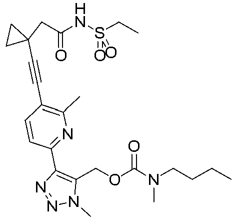 |
| 055 | 494.05 | 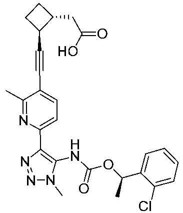 | 189 | 495.26 | 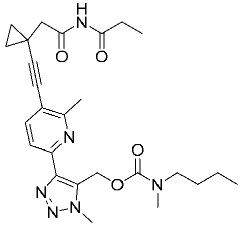 |
| 056 | 500.22 | 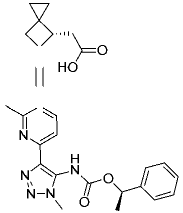 | 190 | 462.17 | 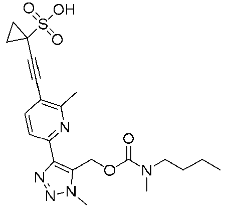 |
| 057 | 500.22 | 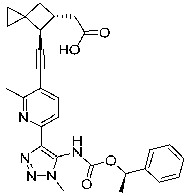 | 191 | 462.18 | 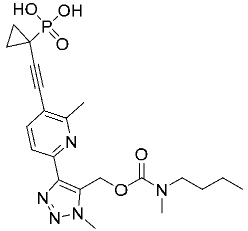 |
| 058 | 500.22 | 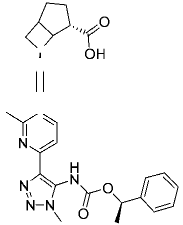 | 192 | 450.23 | 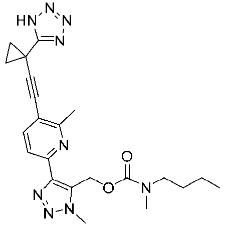 |
| 059 | 506.23 | 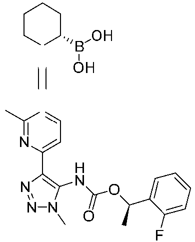 | 197 | 425.21 | 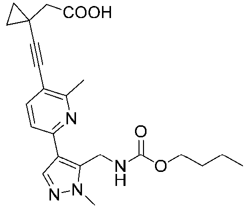 |
| 060 | 504.20 | 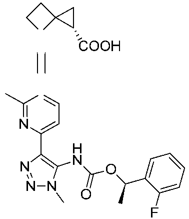 | 198 | 426.20 | 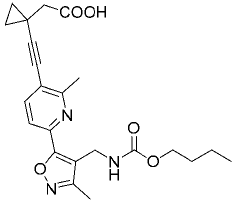 |
| 061 | 520.17 | 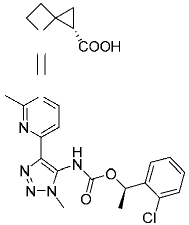 | 199 | 442.17 | 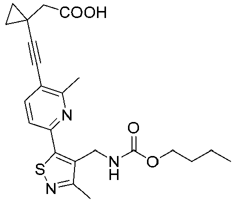 |
| 062 | 488.20 | 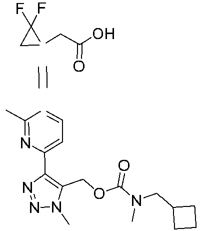 | 200 | 442.17 | 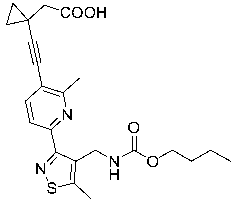 |
| 063 | 524.16 | 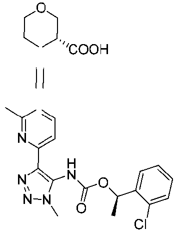 | 201 | 426.20 | 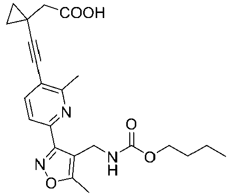 |
| 064 | 524.16 | 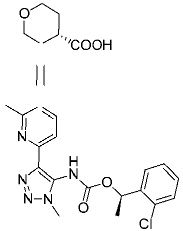 | 202 | 440.22 | 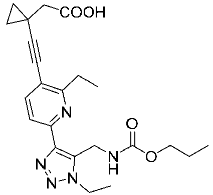 |
| 065 | 508.17 | 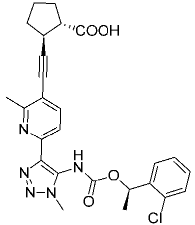 | 203 | 461.12 | 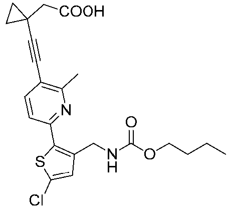 |
| 066 | 524.20 | 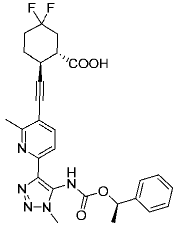 | 225-A | 500.20 | 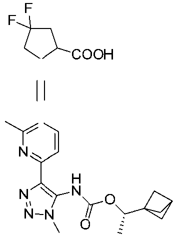 |
| 067 | 524.20 | 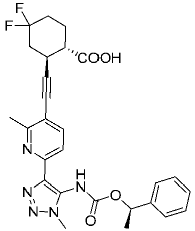 | 234 | 494.48 | 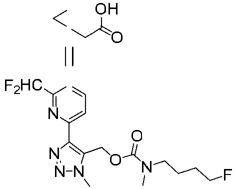 |
| 069 | 464.22 | 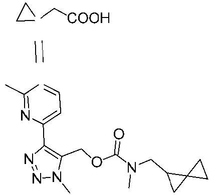 | 236 | 476.49 | 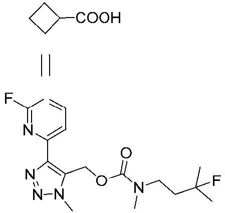 |
| 070 | 490.22 | 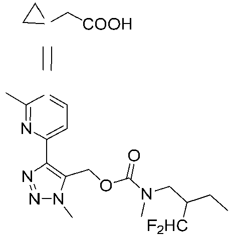 | 240 | 454.05 | 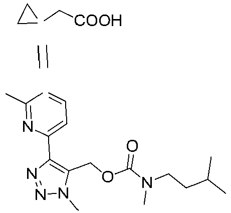 |
| 0071 | 508.21 | 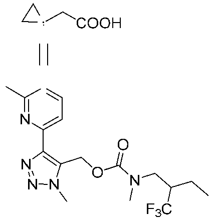 | 260-A | 450.15 | 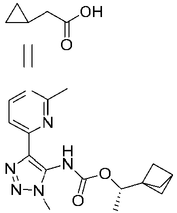 |
| 072 | 464.22 | 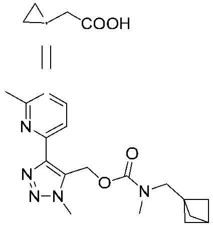 | 261 | 464.53 | 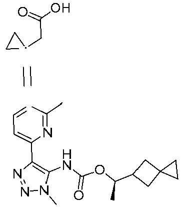 |
| 074 | 510.15 | 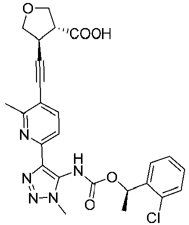 | 262 | 464.53 | 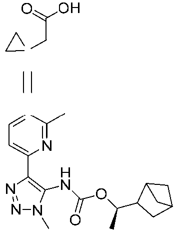 |
| 075 | 544.15 | 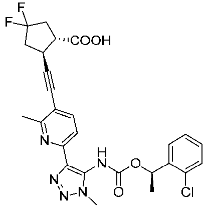 | 266 | 474.47 | 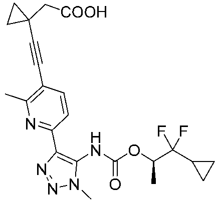 |
| 076 | 522.18 | 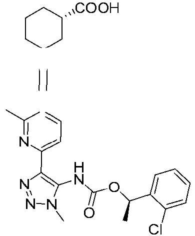 | 268 | 500.53 | 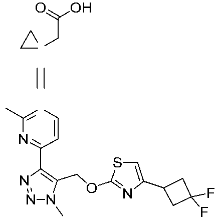 |
| 077 | 456.20 | 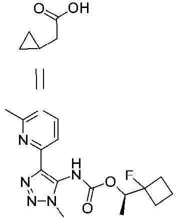 | 269 | 500.53 | 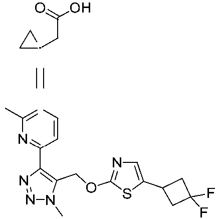 |
| 078 | 474.19 | 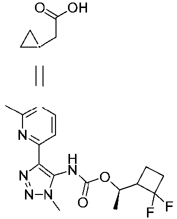 | 270 | 484.47 | 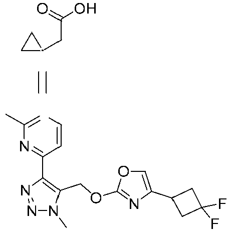 |
| 079 | 456.20 | 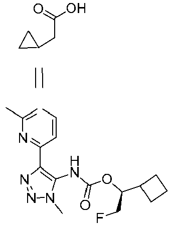 | 271 | 499.55 | 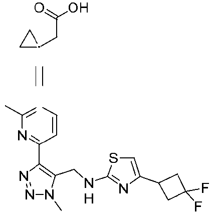 |
| 080 | 474.19 | 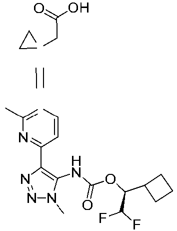 | 272 | 481.47 | 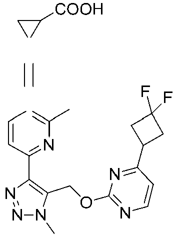 |
| 083 | 508.17 | 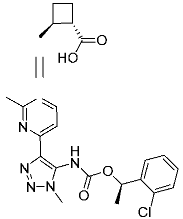 | 287-A | 499.12 | 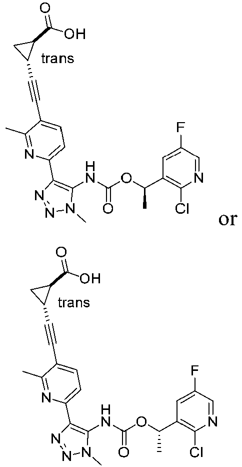 |
| 084 | 494.15 | 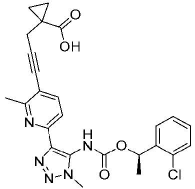 | 287-B | 499.12 | 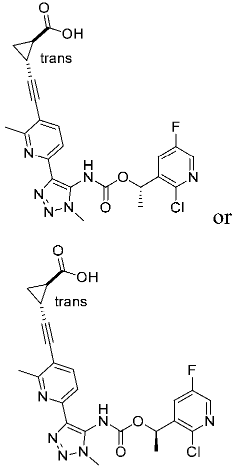 |
| 085 | 520.17 | 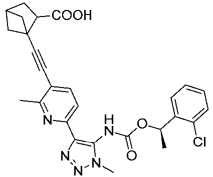 | 371 | 441.21 | 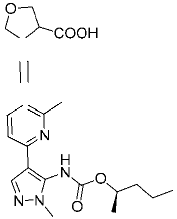 |
| 086 | 509.16 | 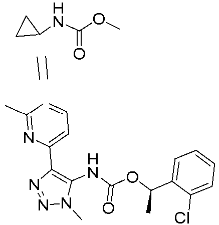 | 372 | 541.17 | 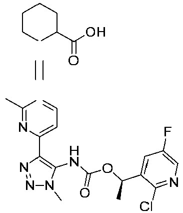 |
| 087 | 458.22 | 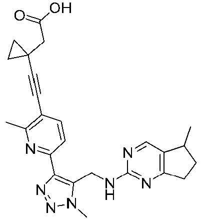 | 373 | 527.15 | 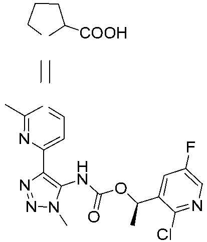 |
| 090 | 490.22 | 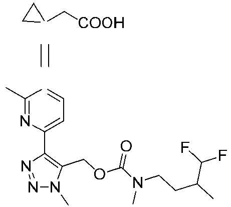 | 374 | 448.17 | 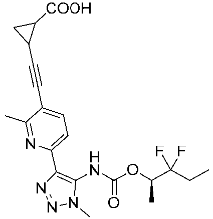 |
| 091 | 458.21 | 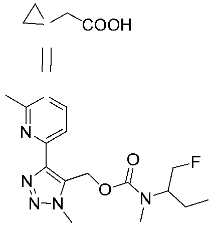 | 375 | 430.18 | 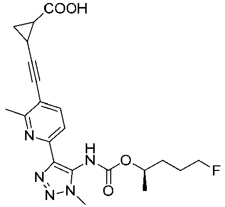 |
| 092 | 452.15 | 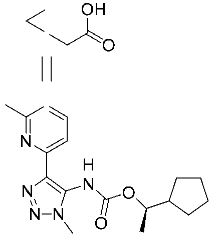 | 376 | 470.15 | 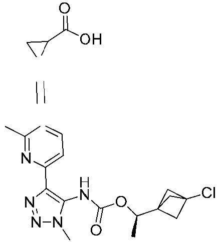 |
| 096 | 458.21 | 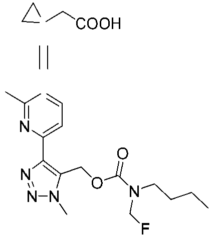 | 377 | 454.18 | 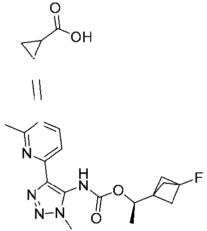 |
| 097 | 476.20 | 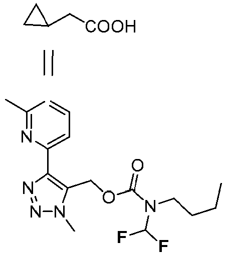 | 378 | 444.20 | 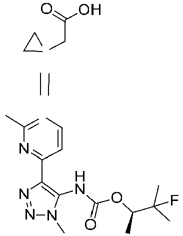 |
| 103 | 462.19 | 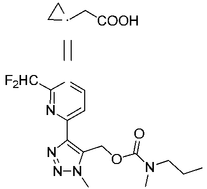 | 379 | 462.19 | 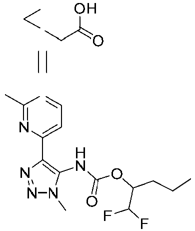 |
| 104 | 476.20 | 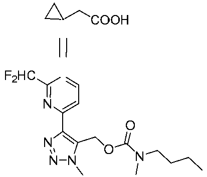 | 380 | 456.20 | 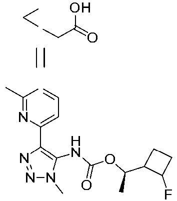 |
| 107 | 454.24 | 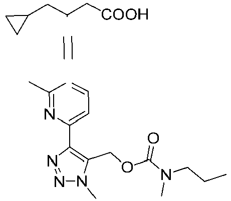 | 381 | 456.20 | 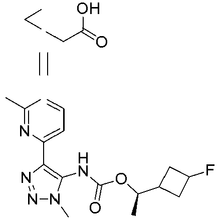 |
| 108 | 440.22 | 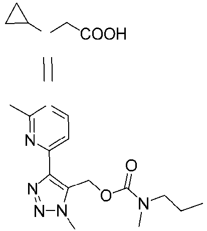 | 382 | 456.22 | 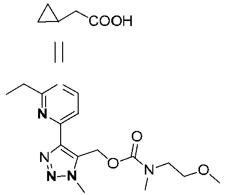 |
| 110 | 470.23 | 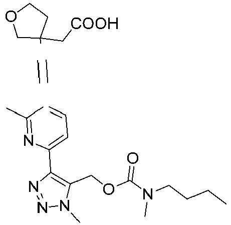 | 385 | 490.22 | 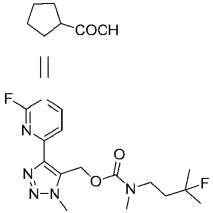 |
| 122 | 497.17 | 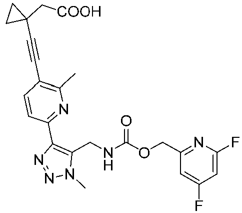 | 386 | 470.15 | 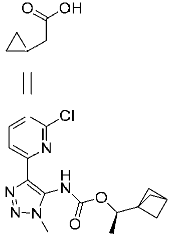 |
| 124 | 432.21 | 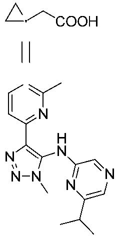 | 387 | 472.23 | 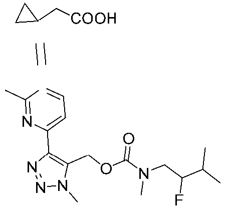 |
| 126 | 531.13 | 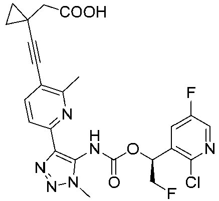 | 392 | 506.19 | 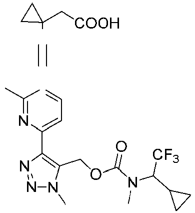 |
| 127 | 549.12 | 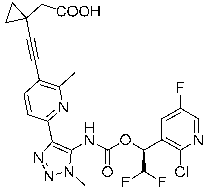 | 393 | 488.20 | 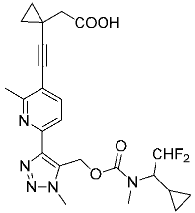 |
| 128 | 499.12 | 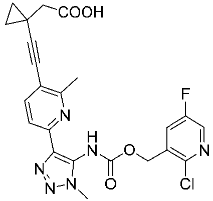 | 394 | 470.21 | 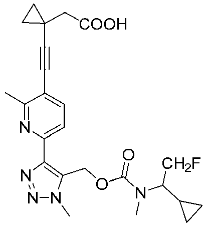 |
| 129 | 539.15 | 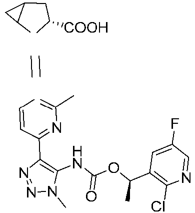 | 395 | 484.23 | 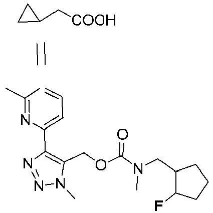 |
| 130 | 539.15 | 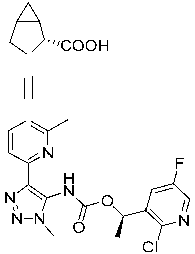 | 396 | 484.23 | 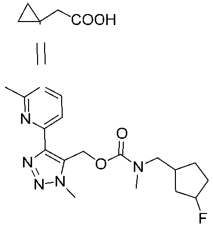 |
| 131 | 539.15 | 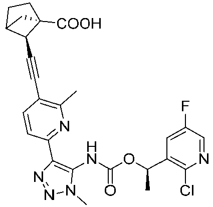 | 398 | 456.22 | 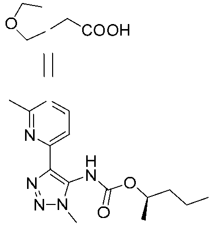 |
| 132 | 539.15 | 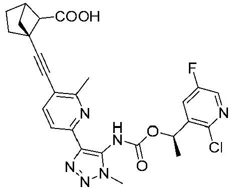 | 400 | 490.22 | 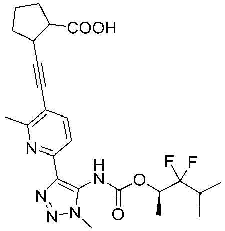 |
| 133 | 539.15 | 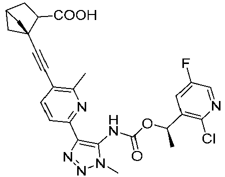 | 401 | 526.20 | 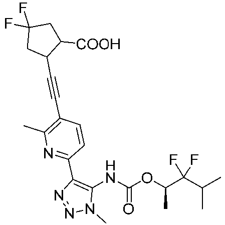 |
| 134 | 553.17 | 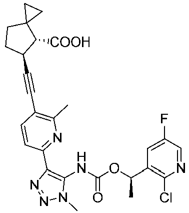 | 406 | 504.21 | 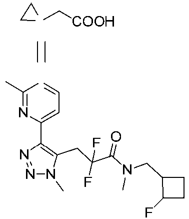 |
| 135 | 553.17 | 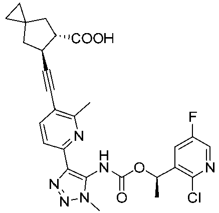 | 407 | 522.21 | 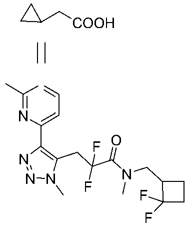 |
| 136 | 553.17 | 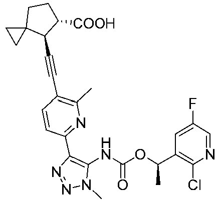 | 408 | 444.20 | 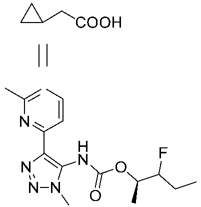 |
| 137 | 563.13 | 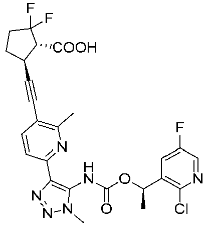 | 409 | 512.22 | 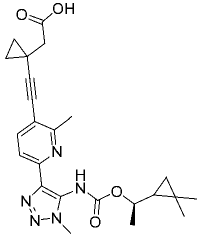 |
| 138 | 563.13 | 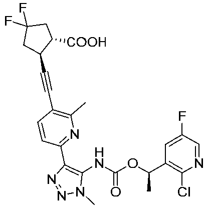 | 410 | 464.22 | 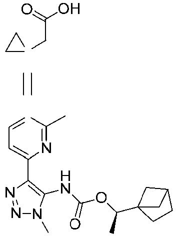 |
| 139 | 563.13 | 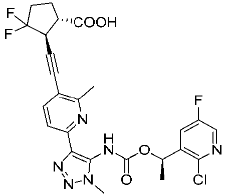 | 411 | 476.20 | 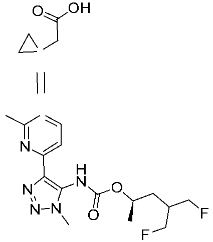 |
| 140 | 563.13 | 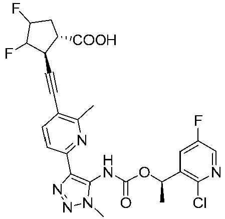 | 412 | 512.18 | 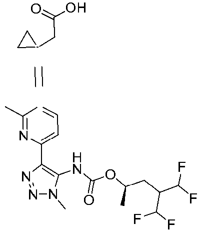 |
| 141 | 563.13 | 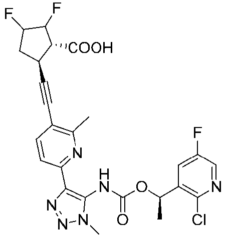 | 413 | 470.21 | 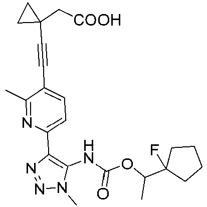 |
| 142 | 563.13 | 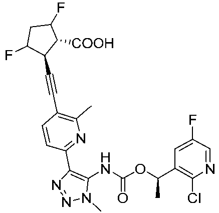 | 415 | 482.21 | 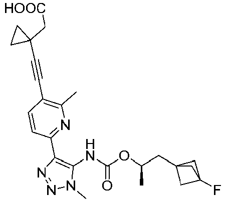 |
| 143 | 595.14 | 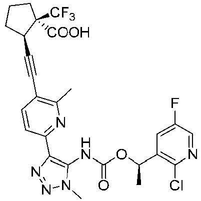 | 416 | 488.20 | 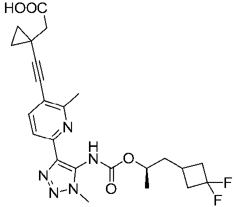 |
| 144 | 577.15 | 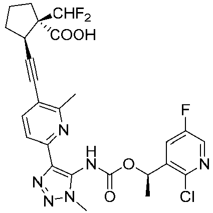 | 417 | 484.17 | 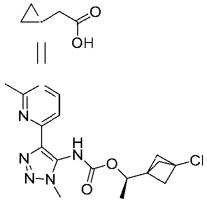 |
| 145 | 541.17 | 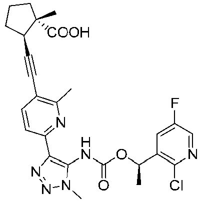 | 418 | 450.21 | 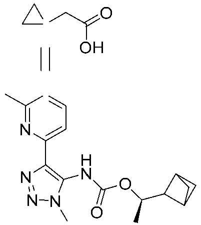 |
| 146 | 545.14 | 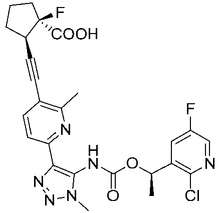 | 419 | 464.22 | 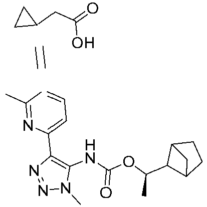 |
| 147 | 552.15 | 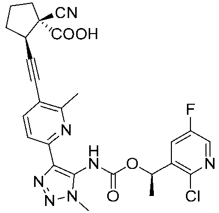 | 420 | 440.22 | 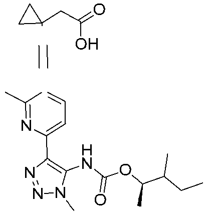 |
| 148 | 543.15 | 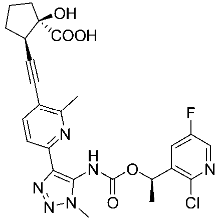 | 421 | 442.20 | 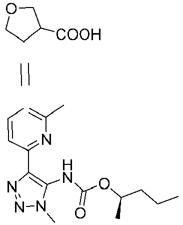 |
| 149 | 557.16 | 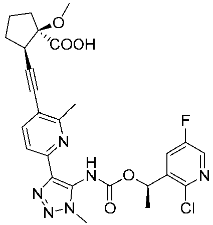 | 422 | 466.20 | 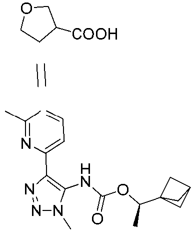 |
| 150 | 545.14 | 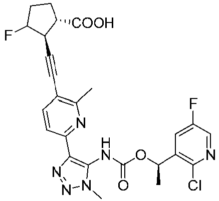 | 423 | 442.19 | 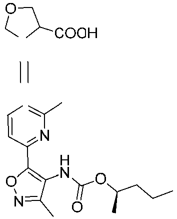 |
| 151 | 561.11 | 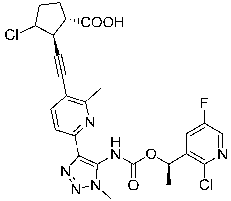 | 424 | 511.12 | 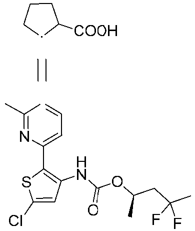 |
| 152 | 552.15 | 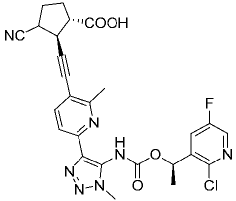 | 425 | 450.21 | 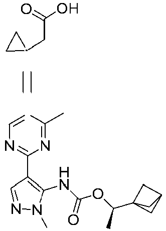 |
| 153 | 541.17 | 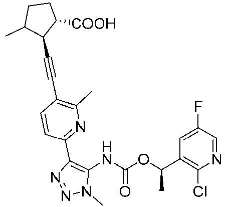 | 426 | 494.23 | 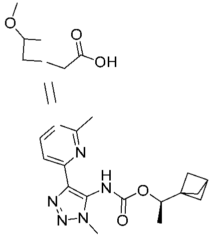 |
| 154 | 595.14 | 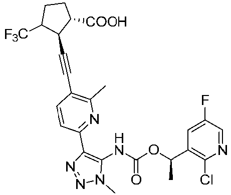 | 433 | 447.21 | 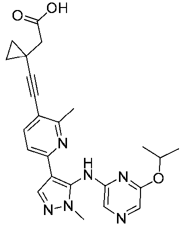 |
| 155 | 577.15 | 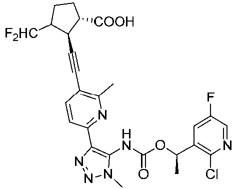 | 434 | 443.21 | 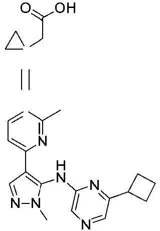 |
| 156 | 557.16 | 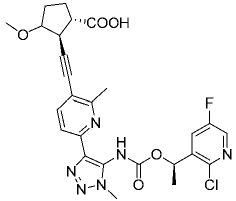 | 435 | 455.21 | 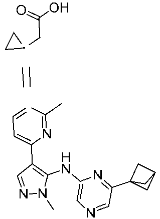 |
| 157 | 545.14 | 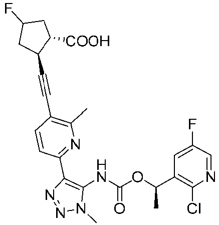 | 436 | 456.21 | 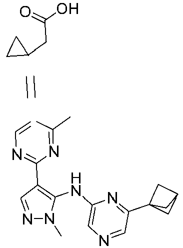 |
| 158 | 545.14 | 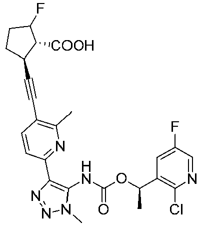 | 437 | 432.21 | 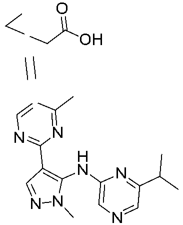 |
| 159 | 595.14 | 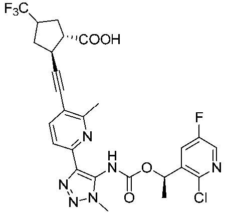 | 438 | 474.19 | 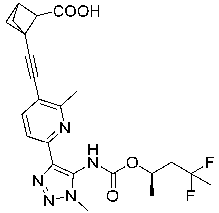 |
| 160 | 595.14 | 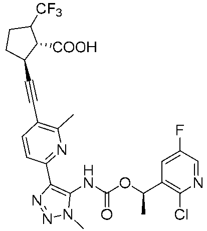 | 439 | 474.19 | 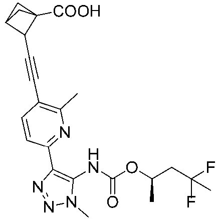 |
| 161 | 549.12 | 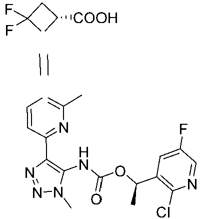 | 440 | 488.20 | 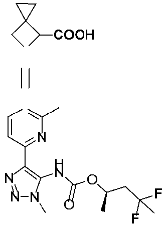 |
| 162 | 549.12 | 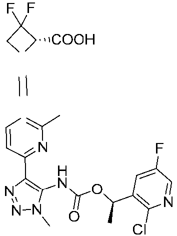 | 441 | 488.20 | 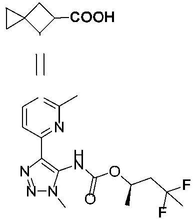 |
| 163 | 549.12 | 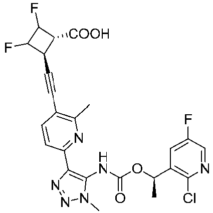 | 442 | 500.20 | 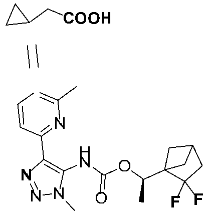 |
| 164 | 460.19 | 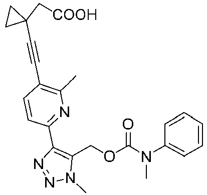 | 446 | 498.16 | 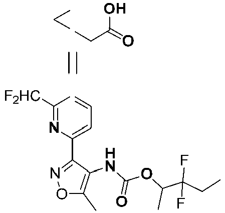 |
| 165 | 461.19 | 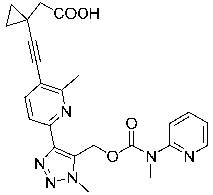 | 457 | 412.19 | 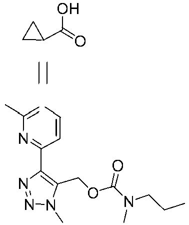 |
| 166 | 461.19 | 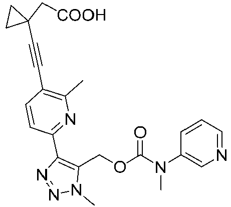 | 458 | 535.07 | 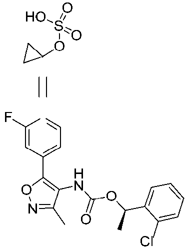 |
| 167 | 467.14 | 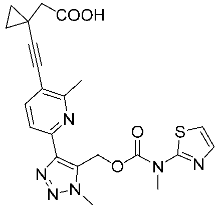 | 459 | 548.10 | 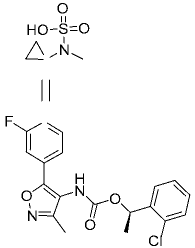 |
| 168 | 474.21 | 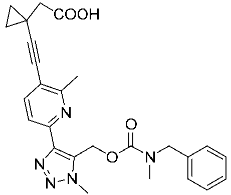 | 460 | 548.10 | 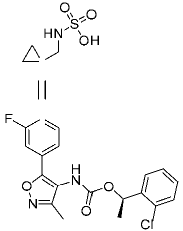 |
| 169 | 475.20 | 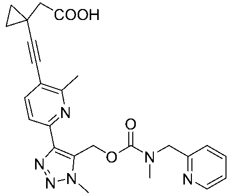 | 482 | 503.10 | 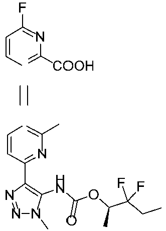 |
| 170 | 475.20 | 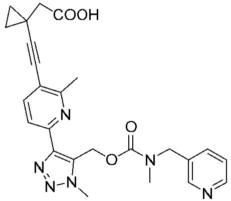 | 484 | 553.10 | 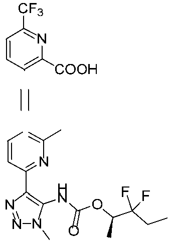 |
| 171 | 475.20 | 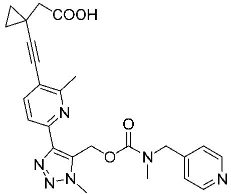 | 511 | 553.10 | 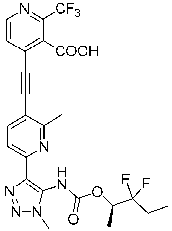 |
| 172 | 481.16 | 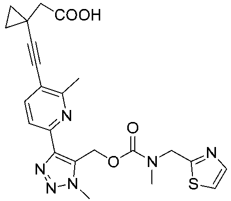 | 512 | 519.10 | 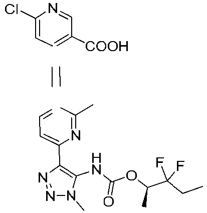 |
| 173 | 465.18 | 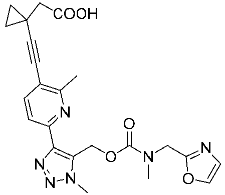 | 513 | 553.10 | 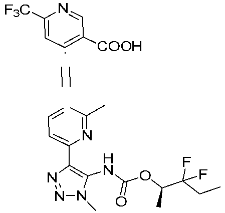 |
| 174 | 527.15 | 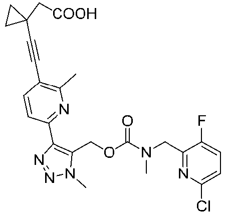 | | | |

### Comparative Example: Control compound 1 and its preparation

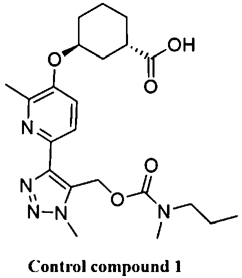

The preparation of control compound 1 was referred to the preparation method described in patent application WO2017223016A1.

### <BiologicalActivity Assay>

### Test Example 1: LPAR1 in vitro biological activity evaluation 1

The antagonist properties of the compounds in the present disclosure were determined using the FLIPR (Fluorometric Imaging Plate Reader) method in CHO-K1 cells (Chinese Hamster Ovary cells K1, ATCC) overexpressing hLPAR1 (human lysophosphatidic acid receptor 1, Accession No.: NM_001401.4).

CHO-K1 cells stably expressing hLPAR1 were placed in a cell culture incubator at 37°C with 5% CO₂ and cultured in F-12 medium containing 10% FBS (fetal bovine serum, Gibco, 10099-141), 1% penicillin-streptomycin (Gibco, 15140-122), and 0.4 mg/mL Hygromycin B (Gibco, 10687010). 18-24 hours before the FLIPR experiment, the cells were seeded into a 96-well plate at a density of 250,000 cells/mL (25,000 cells/well) and incubated overnight in the cell culture incubator. On the day of the experiment, the medium was removed, and the cells were washed with FLIPR buffer (2.5 mM probenecid (Thermo, P36400), 1.3 mM CaCl₂, 3.3 mM Na₂CO₃, 1 mM MgSO₄, 20 mM HEPES (Invitrogen, 15630080), 1× HBSS (Invitrogen, 14065056), 0.1% BSA (BioSharp, 70080030)). 75 µL of 1 mM Fluo-4 AM fluorescent dye (Thermo, F14202) was added to each well and incubated for 1.0 hour at 37°C for dye loading. The 96-well plate was then washed once with buffer, and 50 µL of buffer containing the test compound or vehicle was added to each well, followed by incubation at room temperature for 30 min. The cell plate was then placed into the FLIPR for baseline fluorescence measurement (excitation wavelength at 485 nm, emission wavelength at 525 to 535 nm). Subsequently, 50 µL/well of agonist (final concentration 1 µM oleoyl-L-α-lysophosphatidic acid sodium salt (Sigma, L7260)) or vehicle (ultrapure water) was added, and fluorescence values were measured at 1-second intervals for 2 min. Finally, the output fluorescence counts were analyzed using GraphPad Prism 9.0 software log(inhibitor) vs. response--Variable slope (four parameters) equation to calculate IC₅₀ values.

The IC₅₀ values obtained using the above method are shown in Table 1.

**Table 1: Inhibitory activity data of compounds against LPAR1**

| Compound No. | LPAR1 inhibitory activity IC₅₀ (nM) | Compound No. | LPAR1 inhibitory activity IC₅₀ (nM) |
|---|---|---|---|
| 002 | 83.4 | 217 | 26.6 |
| 004 | 66.3 | 218 | 90.2 |
| 005 | 22.6 | 219-A | 3.3 |
| 006 | 42.0 | 219-B | 11.4 |
| 010 | 56.2 | 220 | 15.5 |
| 010-A | 45.9 | 224-B | 70 |
| 013 | 2.6 | 225-C | 8.2 |
| 014 | 7.7 | 225-D | 14.7 |
| 017 | 42.8 | 226 | 0.4 |
| 018 | 55.2 | 230 | 59.9 |
| 022 | 9.5 | 237-A | 34.4 |
| 023 | 45.2 | 242-A | 7.0 |
| 023-A | 37.2 | 243-A | 34.5 |
| 026 | 52.7 | 243-B | 27.7 |
| 027-A | 2.6 | 244-A | 19.8 |
| 027-B | 49.2 | 244-B | 19.2 |
| 028-A | 17.6 | 245-A | 39.0 |
| 028-B | 60.4 | 245-B | 34.6 |
| 029 | 218.7 | 246-A | 18.3 |
| 030 | 33.6 | 246-B | 25.9 |
| 031-B | 0.2 | 247 | 10.2 |
| 031-A | 0.6 | 248-A | 18.1 |
| 032-A | 0.6 | 248-B | 19.1 |
| 033 | 3.3 | 252 | 30.2 |
| 034 | 52.7 | 253 | 72.3 |
| 036 | 102.9 | 254 | 63.9 |
| 037 | 104.9 | 255 | 71.3 |
| 038 | 51.1 | 260-B | 17.4 |
| 039 | 4.2 | 263 | 52.8 |
| 068 | 129.2 | 264 | 47.3 |
| 072 | 77.4 | 265 | 21.5 |
| 073 | 79.1 | 273 | 14.9 |
| 081 | 173.6 | 278 | 38.1 |
| 082 | 157.3 | 280 | 75.7 |
| 092' | 18.7 | 302 | 24.7 |
| 094 | 30.1 | 306-A | 50.6 |
| 095 | 39.9 | 306-B | 53.6 |
| 100 | 44.3 | 307 | 150.5 |
| 102 | 34.5 | 308 | 10.9 |
| 105 | 56.21 | 310 | 113.8 |
| 117 | 79.4 | 313 | 16.1 |
| 125 | 43.6 | 319 | 123.9 |
| 194 | 42.5 | 324 | 50.3 |
| 205 | 3.1 | 325 | 40.3 |
| 207 | 20.2 | 338-A | 37.5 |
| 209 | 12.7 | 338-B | 21 |
| 210 | 24.9 | 339 | 2.6 |
| 211 | 13.8 | 343 | 5.7 |
| 212-A | 42.1 | 344 | 104.1 |
| 212-B | 25.9 | 360-A | 96.3 |
| 213-A | 48.2 | 362 | 20.4 |
| 213-B | 36.8 | 364 | 203.2 |
| 214 | 9.4 | 458 | 0.1 |
| 215 | 43.0 | 459 | 13.6 |
| 216 | 23.3 | 460 | 11.8 |

Experimental conclusion: The compounds of the present disclosure have good inhibitory effects against LPAR1.

### Test Example 2: LPAR1 in vitro biological activity evaluation 2

The inhibitory effects of the compounds of the present disclosure on the LPAR1 receptor were determined using a Chinese hamster ovary (CHO) stable transfected cell line expressing the LPAR1 receptor, through the HTRF cAMP kit (LANCE^{®} Ultra cAMP Kit).

The CHO cell line expressing the LPAR1 receptor was cultured in F-12 medium (Gibco, 117650054) containing 10% fetal bovine serum (FBS, Avantor, 76294-180) and 0.2 mg/mL hygromycin B (Solarbio, H8080-1g) at 37°C with 5% CO₂. Cell passage, recovery, and cryopreservation were performed according to conventional methods. The stable transfected cell line was cultured to 80% confluence, digested with trypsin to collect cells, counted, and then seeded at 5 µL/well in a 384-well plate (500 cells/5 µL). The 1× Stimulation Buffer was prepared according to the instructions of the kit (LANCE^{®} Ultra cAMP Kit). 1 µL of the test compound solution (diluted to 10× with 1× Stimulation Buffer) was added to the corresponding experimental wells, followed by centrifugation and incubation at 37°C for 10 minutes. Then, 4 µL/well of 7.5 µM Forskolin (Selleck, S2449) and 0.25 µM LPA buffer, prepared with 1× Stimulation Buffer, were added to the corresponding experimental wells, followed by incubation at 37°C for 30 minutes to induce cAMP production. 5 µL/well of Eu-cAMP (diluted with Detection buffer) was added to the corresponding experimental wells. 5 µL/well of ULight^{™}-anti-cAMP antibody (diluted with Detection buffer) was added to the corresponding experimental wells, followed by centrifugation and incubation at room temperature for 1 hour. After the incubation was completed, the readings at 665 nm and 620 nm under 330 nm excitation were detected using the HTS high-throughput drug screening multifunctional microplate reader (BMG, PHERAstar FSX). The IC₅₀ values were calculated by fitting nonlinear regression (dose response - variable slope) using GraphPad Prism software.

The IC₅₀ values obtained using the above method are shown in Table 2.

**Table 2: Inhibitory activity data of compounds against LPAR1**

| Compound No. | LPAR1 inhibitory activity IC₅₀ (nM) | Compound No. | LPAR1 inhibitory activity IC₅₀ (nM) |
|---|---|---|---|
| Control compound 1 | 407 | 454 | 60.0 |
| 053 | 117.4 | 455 | 60.7 |
| 101 | 204.7 | 456 | 60.6 |
| 109 | 23.9 | 461 | 23.4 |
| 113 | 19.6 | 462 | 48.7 |
| 114 | 9.2 | 462-A | 35.3 |
| 115 | 8.3 | 462-B | 51.3 |
| 118 | 83.9 | 463 | 38.2 |
| 208 | 44.1 | 463-A | 41.4 |
| 224-B | 126.8 | 463-B | 35.3 |
| 232 | 75.4 | 464 | 23.6 |
| 233 | 163.0 | 465 | 30.9 |
| 248 | 72.2 | 466 | 25.5 |
| 276 | 96.4 | 467 | 16.5 |
| 277 | 26.6 | 468 | 31.6 |
| 278 | 34.4 | 469 | 86.6 |
| 278-A | 46.2 | 470 | 24.1 |
| 278-B | 56.0 | 471 | 33.6 |
| 279 | 39.0 | 474 | 166.7 |
| 282-A | 87.6 | 476 | 76.5 |
| 290 | 190.3 | 477 | 46.3 |
| 294 | 194.6 | 478 | 2.1 |
| 296 | 83.8 | 485 | 52.3 |
| 299 | 13.6 | 486 | 34.1 |
| 304 | 134.1 | 487 | 35.2 |
| 304-A | 128.2 | 488 | 51.5 |
| 304-B | 55.0 | 489 | 14.1 |
| 305 | 91.2 | 490 | 8.6 |
| 305-A | 25.6 | 491 | 27.1 |
| 305-B | 81.5 | 492 | 8.6 |
| 308 | 93.2 | 493 | 8.5 |
| 309 | 83.2 | 494 | 50.8 |
| 313 | 156.2 | 495 | 26.1 |
| 323 | 14.8 | 496 | 45.4 |
| 334 | 106.9 | 497 | 46.2 |
| 335 | 113.4 | 498 | 6.0 |
| 340 | 83.9 | 499 | 11.5 |
| 340-A | 67.1 | 500 | 4.5 |
| 340-B | 66.9 | 501 | 14.7 |
| 402 | 47.2 | 502 | 7.2 |
| 427 | 59.0 | 503 | 8.5 |
| 428 | 49.4 | 504 | 6.4 |
| 429 | 33.4 | 506 | 20.1 |
| 430 | 63.2 | 507 | 73.3 |
| 432 | 16.6 | 508 | 4.0 |
| 444 | 48.4 | 510 | 32.1 |
| 448 | 6.8 | 512 | 37.0 |
| 449 | 5.6 | 513 | 26.5 |
| 451 | 108.6 | | |
| 452 | 33.7 | | |
| 453 | 15.0 | | |

Experimental conclusion: The compounds of the present disclosure have good inhibitory effects against LPAR1.

### Test Example 3: Pharmacokinetic study in rats

Male SPF-grade SD rats (Beijing Vital River Laboratory Animal Technology Co., Ltd.) with a body weight range of 200 to 250 g were used. After acclimatization, the rats were administered a clear solution of the compound of the present disclosure via oral gavage (Po, fasted overnight before administration with free access to water, food returned 4 hours after administration) and tail vein injection (Iv, free access to food and water throughout the administration). Blood samples were collected from the orbital venous plexus/jugular vein of rats at specified time points after administration (blood collection time points for the PO group: 0.167, 0.5, 1, 2, 4, 6, 8, and 24 h; for the IV group: 0.033, 0.167, 1, 2, 4, 6, 8, and 24 h). The concentration of compounds in plasma was detected by LC-MS/MS (AB SCIEX Qtrap4500). Data were analyzed using WinNonlin (version 5.2.1 Pharsight, Mountain View, CA) with a non-compartmental model to obtain PK parameters (including Cₘₐₓ, C₀, Tₘₐₓ, AUCₗₐₛₜ, AUC_{inf}, T_{1/2}, *etc.*)*.*

The results of the *in vivo* pharmacokinetic study in rats show that the compounds in the examples of the present disclosure all have good pharmacokinetic properties in rats, achieving high *in vivo* exposure and high oral bioavailability at relatively low doses. The *in vivo* pharmacokinetic data of representative compounds in rats are shown in Table 3 below.

**Table 3: Results of in vivo pharmacokinetic studies of compounds of the present disclosure in rats**

| Compound | Dosage of administration mg/kg | Route of administration | Cₘₐₓ (ng/mL) | AUCₗₐₛₜ (ng/mL* hr) | T_{1/2}(h) |
|---|---|---|---|---|---|
| Control compound 1 | 1 | Po | 154 | 626 | 2.37 |
| | | Iv | 1567 | 811 | 3.26 |
| | 3 | Po | 985 | 1536 | 2.59 |
| 002 | 3 | Po | ** | ** | 3.40 |
| | 1 | Iv | *** | * | 7.18 |
| 010-B | 3 | Po | ** | **** | 6.85 |
| | 1 | Iv | ** | *** | 8.56 |
| 023-A | 1 | Iv | *** | ** | 7.50 |
| 033 | 1 | Po | ** | ** | 5.31 |
| | | Iv | ** | ** | 5.93 |
| 105 | 1 | Po | * | * | 6.60 |
| | | Iv | ** | * | 4.72 |
| 125 | 1 | Iv | ** | * | 4.65 |
| 207-A | 1 | Po | * | *** | 6.96 |
| | | Iv | ** | *** | 10.20 |
| 210 | 1 | Po | * | ** | 6.08 |
| | | Iv | ** | *** | 6.18 |
| 212-B | 1 | Po | ** | *** | 7.96 |
| | | Iv | ** | *** | 7.43 |
| 213 | 1 | Po | * | *** | 9.97 |
| | | Iv | ** | *** | 10.40 |
| 213-B | 3 | Po | ** | **** | 9.60 |
| | 1 | Iv | ** | *** | 8.67 |
| 214 | 3 | Po | ** | **** | 8.21 |
| | 1 | Iv | ** | ** | 7.01 |
| 215 | 3 | Po | ** | *** | 4.49 |
| | 1 | Iv | ** | * | 5.03 |
| 216 | 3 | Po | ** | *** | 4.44 |
| | 1 | Iv | *** | * | 4.90 |
| 219-B | 1 | Po | ** | *** | 4.24 |
| | | Iv | ** | *** | 3.89 |
| 224 | 1 | Po | ** | *** | 3.76 |
| | | Iv | ** | *** | 4.78 |
| 232 | 1 | Po | * | ** | 14.00 |
| | | Iv | ** | *** | 14.50 |
| 237-A | 1 | Po | ** | *** | 4.00 |
| | | Iv | *** | *** | 5.19 |
| 244-B | 1 | Po | ** | *** | 4.07 |
| | | Iv | ** | *** | 5.05 |
| 245-B | 1 | Po | * | ** | 6.66 |
| | | Iv | ** | ** | 7.22 |
| 246-B | 1 | Po | * | ** | 4.40 |
| | | Iv | ** | *** | 7.01 |
| 248-A | 1 | Po | * | *** | 5.33 |
| | | Iv | ** | ** | 7.72 |
| 253 | 3 | Po | ** | *** | 3.45 |
| | 1 | Iv | *** | * | 4.06 |
| 254 | 1 | Po | * | ** | 5.13 |
| | | Iv | *** | ** | 7.04 |
| 260 | 1 | Po | * | *** | 9.12 |
| | | Iv | ** | *** | 8.44 |
| 263 | 1 | Po | * | *** | 10.20 |
| | | Iv | ** | ** | 13.80 |
| 265 | 1 | Po | * | ** | 8.50 |
| | 0.63 | Iv | ** | * | 7.81 |
| 276 | 1 | Iv | ** | ** | 3.69 |
| 278 | 1 | Po | * | *** | 4.50 |
| | | Iv | *** | *** | 5.24 |
| 279 | 1 | Po | ** | *** | 6.39 |
| | | Iv | *** | *** | 5.82 |
| 280-B | 3 | Po | ** | **** | 6.57 |
| | 1 | Iv | ** | *** | 6.47 |
| 282 | 1 | Po | * | * | 4.58 |
| | | Iv | ** | * | 6.07 |
| 283 | 1 | Po | * | *** | 6.90 |
| | | Iv | ** | *** | 7.36 |
| 285 | 1 | Po | * | ** | 6.12 |
| | | Iv | ** | *** | 9.86 |
| 291 | 1 | Po | ** | ** | 5.15 |
| | | Iv | ** | *** | 4.68 |
| 295 | 1 | Po | ** | **** | 3.49 |
| | | Iv | ** | *** | 3.46 |
| 305-B | 1 | Po | ** | *** | 3.49 |
| | | Iv | ** | ** | 3.52 |
| 306 | 1 | Po | * | ** | 7.20 |
| | | Iv | ** | ** | 7.66 |
| 309 | 1 | Po | ** | *** | 4.08 |
| | | Iv | ** | ** | 4.36 |
| 311 | 1 | Po | * | ** | 8.49 |
| | | Iv | ** | *** | 9.33 |
| 313 | 3 | Po | ** | **** | 5.59 |
| | 1 | Iv | ** | ** | 5.12 |
| 314 | 1 | Po | * | *** | 6.41 |
| | | Iv | ** | ** | 8.42 |
| 324 | 1 | Po | ** | *** | 5.73 |
| | | Iv | ** | ** | 12.1 |
| 340-B | 1 | Po | ** | *** | 4.25 |
| | | Iv | ** | *** | 4.77 |
| 355 | 1 | Po | * | * | 5.53 |
| | | Iv | ** | * | 6.65 |
| 359 | 1 | Po | * | * | 6.24 |
| | | Iv | ** | * | 7.63 |
| 360-A | 3 | Po | ** | *** | 3.49 |
| | 1 | Iv | ** | * | 5.25 |
| 361 | 1 | Po | * | * | 2.53 |
| | | Iv | ** | * | 3.03 |
| 365 | 1 | Po | * | * | 5.98 |
| | | Iv | ** | * | 7.21 |
| 370 | 1 | Po | * | * | 6.01 |
| | | Iv | ** | * | 5.01 |
| 429 | 1 | Po | * | ** | 5.70 |
| | | Iv | *** | ** | 6.87 |
| 444 | 1 | Po | * | ** | 3.61 |
| | | Iv | ** | * | 2.91 |
| 451 | 1 | Po | * | *** | 4.07 |
| | | Iv | ** | *** | 4.46 |
| 452 | 1 | Po | ** | *** | 3.37 |
| | | Iv | ** | * | / |
| 454 | 1 | Po | ** | *** | 2.16 |
| | | Iv | ** | ** | 2.61 |
| 462 | 1 | Po | ** | **** | 5.41 |
| | | Iv | **** | *** | 6.41 |
| 462-A | 1 | Po | ** | *** | 4.42 |
| | | Iv | **** | **** | 6.35 |
| 462-B | 1 | Po | ** | *** | 6.48 |
| | | Iv | **** | **** | 7.15 |
| 463 | 1 | Po | ** | *** | 5.47 |
| | | Iv | *** | ** | 5.44 |
| 468 | 1 | Po | * | *** | 2.77 |
| | | Iv | ** | ** | 2.35 |
| 492 | 1 | Po | ** | *** | 3.75 |
| | | Iv | ** | *** | 3.92 |
| 496 | 1 | Po | ** | **** | 5.89 |
| | | Iv | ** | *** | 4.77 |
| 497 | 1 | Po | * | *** | 5.40 |
| | | Iv | ** | *** | 4.94 |
| 506 | 1 | Po | ** | **** | 2.41 |
| | | Iv | **** | **** | 2.51 |

| | | | | | |
|---|---|---|---|---|---|
| In the table: ****: 10000 ng/mL*hr ≤ AUCₗₐₛₜ < 20000 ng/mL*hr, or 8000 ng/mL ≤ Cₘₐₓ < 12000 ng/mL; ***: 5000 ng/mL*hr ≤ AUCₗₐₛₜ < 10000 ng/mL*hr, or 5000 ng/mL ≤ Cₘₐₓ < 8000 ng/mL; **: 3000 ng/mL*hr ≤ AUCₗₐₛₜ < 5000 ng/mL*hr, or 800 ng/mL ≤ Cₘₐₓ < 5000 ng/mL; *: 1200 ng/mL*hr ≤ AUCₗₐₛₜ < 3000 ng/mL*hr, or 200 ng/mL ≤ Cₘₐₓ < 800 ng/mL; | | | | | |

The vehicle for the Po group compound was 5% DMSO + 5% ethanol + 60% PEG400 + 30% normal saline; the vehicle for the Iv group compound was 10% ethanol + 20% pure water + 70% PEG400 solution.

### Test Example 4: Pharmacokinetic study in monkeys

Cynomolgus monkeys (Suzhou Xishan Zhongke Laboratory Animal Co., Ltd.), aged 3 to 5 years, weighing 3 to 5 kg, after acclimatization, were administered the clear solution of the compounds of the present disclosure via oral gavage (Po, fasted before administration with free access to water) and intravenous bolus injection (Iv, free access to food and water throughout the administration), respectively. The vehicle for the Po group compound was 5% DMSO + 5% ethanol + 60% PEG400 + 30% normal saline, and the vehicle for the Iv group compound was 10% ethanol + 20% ultrapure water + 70% PEG400 solution. At specific time points after administration (blood collection time points for the Po group: 0.167, 0.5, 1, 2, 3, 4, 6, 9, 12, 24, 32, 48 h; for the Iv group: 0.083, 0.25, 1, 2, 3, 4, 6, 9, 12, 24, 32, 48 h), approximately 1 mL of whole blood was collected from the peripheral blood vessels of the non-dosing limb of each cynomolgus monkey. The concentration of the compound in plasma was detected by LC-MS/MS (AB SCIEX Qtrap4500), and the data were analyzed using WinNonlin (version 5.2.1 Pharsight, Mountain View, CA) via a non-compartmental model to obtain PK parameters of the cynomolgus monkeys (including Cₘₐₓ, C₀, Tₘₐₓ, AUCₗₐₛₜ, AUC_{inf}, T_{1/2}, *etc.*)*.*

The results of *in vivo* pharmacokinetic studies in cynomolgus monkeys show that the compounds of the present disclosure exhibit superior pharmacokinetic properties in cynomolgus monkeys, achieving higher *in vivo* exposure at lower doses, with higher bioavailability, longer half-life, higher clearance rate, and larger volume of distribution.

### Test Example 5: Study on metabolic stability in human primary hepatocytes

The cryopreserved human hepatocytes were suspended in culture medium to reach 1.5 × 10⁶ cells/mL. After AO/PI staining, cell counting was performed to determine viable cell density, followed by dilution with culture medium to 0.5 × 10⁶ viable cells/mL. Under these conditions, the following experiment was conducted: Cells were added to a 96-well plate and pre-incubated in a 37°C water bath for 10 min. The compounds of the present disclosure were added at a final concentration of 0.5 µM to initiate the reaction. Samples were taken at 0.5 min, 30 min, 60 min, 90 min, 120 min, and 240 min, respectively, and the reaction was then terminated. After vortex mixing, centrifugation was performed at 3220×g for 45 min, and the supernatant was collected for LC-MS/MS analysis. The T_{1/2} and CL were calculated according to the following formula:${T}_{1 / 2} = - 0.693 / slope , CLint = - slope * incubation volume / number of cells .$

The results show that the compounds of the present disclosure exhibit good metabolic stability in human primary hepatocytes.

### Test Example 6: Caco-2 cell permeability study

Caco-2 cells were seeded at 6.86 × 10⁵ cells/mL in a 96-well transwell plate, with the culture medium changed every other day. After 14 to 18 days of culture, the transepithelial electrical resistance (TEER) value was measured to assess the integrity of the cell monolayer. A TEER value greater than 230 ohm•cm² indicated that the Caco-2 monolayer was qualified. Transport experiments were conducted under intact cell membrane conditions: In group A to B, the compounds of the present disclosure (final concentrations of 10 µM, 243 µM) were added to the donor side of the Transwell plate, while buffer solution was added to the receiver side. The plate was then incubated at 37°C with 5% CO₂ for 2 hours. After the incubation was completed, samples were collected from both donor and receiver sides, added to termination agents containing internal standards, vortexed at 1000 rpm for 10 min, and centrifuged at 4000 rpm, 4°C for 30 min. The supernatant was collected and analyzed by LC-MS/MS. Group B to A was tested under the same conditions. Finally, Papp (10⁻⁶ cm/sec) and efflux ratio were calculated according to the following formulas: 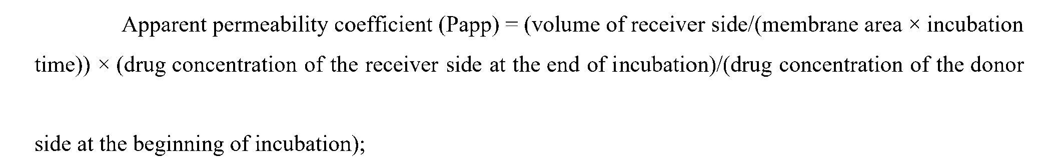
Apparent permeability coefficient (Papp) = (volume of receiver side/(membrane area × incubation time)) × (drug concentration of the receiver side at the end of incubation)/(drug concentration of the donor side at the beginning of incubation);$efflux ratio \left(ER\right) = Papp \left(B-A\right) / Papp \left(A-B\right) .$

The experimental results show that the compounds of the present disclosure exhibit high permeability with no significant efflux.

The above provides an exemplary description of the implementation of the technical solutions of the present disclosure. It should be understood that the scope of protection of the present disclosure is not limited to the above implementations. Any modifications, equivalent replacements, improvements, *etc.,* made by those skilled in the art within the essence and principles of the present disclosure shall be included within the scope of protection of the claims of the present disclosure.

## Claims

1. A compound of formula (I), a racemate thereof, a stereoisomer thereof, a tautomer thereof, an N-oxide thereof, or a pharmaceutically acceptable salt of any one of the foregoing: 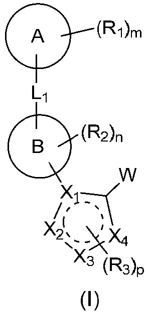 ring A is C₃₋₁₀ cycloalkyl, 3- to 10-membered heterocycloalkyl, or 5- to 10-membered heteroaryl; the definitions of ring B and R₁ are selected from one of the following cases (1), (2), and (3):
case (1): ring B is 5- to 7-membered heteroarylene;
each R₁ is independently OH, COOH, CN, oxo (=O), halogen, NH₂, 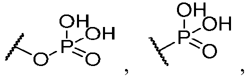 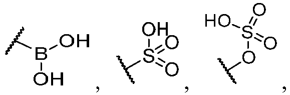 tetrazolyl, C₁₋₆ alkyl, C₁₋₆ alkoxy, -C₁₋₃ alkylene-COOH, -NHC(-O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, - C₁₋₃ alkylene-C(-O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(-O)NHS(-O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
case (2): ring B is 8- to 10-membered heteroarylene;
each R₁ is independently OH, CN, oxo (=O), halogen, NH₂, 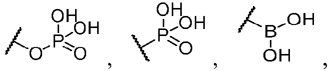 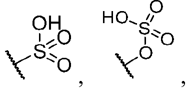 tetrazolyl, C₁₋₆ alkyl, C₁₋₆ alkoxy, -C₁₋₃ alkylene-COOH, -NHC(=O)OC₁₋₆ alkyl, - NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, - C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
case (3): ring B is C₆₋₁₀ arylene;
each R₁ is independently OH, CN, oxo (=O), halogen, NH₂, 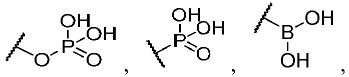 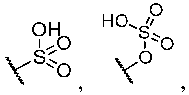 tetrazolyl, C₁₋₆ alkyl, C₁₋₆ alkoxy, -C₂₋₃ alkylene-COOH, -NHC(=O)OC₁₋₆ alkyl, - NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(-O)NHC(-O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, - C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₂₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(-O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
each Rₐ is independently C₁₋₄ alkyl, C₁₋₄ alkoxy, halogen, OH, oxo, CN, or NH₂; when the number of Rₐ is 2 or more, the OH and oxo are not attached to the same carbon atom;
L₁ is C₂₋₆ alkynylene, -C₂₋₆ alkynylene-O-, or -C₂₋₆ alkynylene-NH-; the C₂₋₆ alkynylene, -C₂₋₆ alkynylene-O-, and -C₂₋₆ alkynylene-NH- are each independently optionally substituted with 1, 2, or 3 R_{b} substituents;
each R_{b} is independently halogen, COOH, or C₁₋₆ alkyl; the C₁₋₆ alkyl is optionally substituted with 1, 2, or 3 COOH or halogen substituents;
each R₂ is independently halogen, NH₂, OH, oxo, CN, C₁₋₄ alkyl, C₁₋₄ alkoxy, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen or deuterium substituents;
X₁, X₂, X₃, and X₄ are each independently C, CH, CH₂, O, S, N, or NH; simultaneously, at least one of X₁, X₂, X₃, or X₄ is independently O, S, N, or NH;
the double line comprising solid and dashed lines represents a single bond or a double bond; simultaneously, 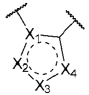 contains at least one double bond;
each R₃ is independently H, C₁₋₄ alkyl, C₁₋₄ alkoxy, halogen, NH₂, OH, oxo, CN, C₃₋₆ cycloalkyl, or 3-to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen or deuterium substituents;
W is 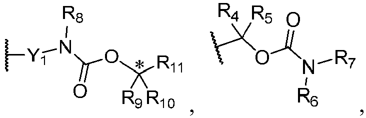 or -(CR₁₂R₁₃)_{q}-Y₂-R₁₅; the carbon atom marked with * is a chiral carbon atom or an achiral carbon atom; when the carbon atom is a chiral carbon atom, the configuration of the chiral carbon atom is R and/or S;
wherein R₆ and R₇ are each independently H, C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl is optionally substituted with 1, 2, or more R_{c} substituents; the C₃₋₈ cycloalkyl, 3- to 10-membered heterocyclyl, C₆₋₁₀ aryl, and 5- to 10-membered heteroaryl are each independently optionally substituted with 1, 2, or more Rₑ substituents;
each R_{c} is independently deuterium, halogen, -OH, -CN, -COOH, C₁₋₆ alkoxy, C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl, C₁₋₆ alkoxy, C₃₋₈ cycloalkyl, 3- to 10-membered heterocyclyl, C₆₋₁₀ aryl, and 5- to 10-membered heteroaryl are each independently optionally substituted with 1, 2, or more R_{c-1} substituents;
each R_{c-1} is independently deuterium, halogen, -OH, -CN, -COOH, C₁₋₆ alkoxy, or C₁₋₆ alkyl;
R₄, R₅, and R₈ are each independently H, deuterium, halogen, C₁₋₄ alkyl, or C₃₋₆ cycloalkyl; the C₁₋₄ alkyl is optionally substituted with 1, 2, or more Rⱼ substituents;
each Rⱼ is independently deuterium or halogen;
Y₁ is a chemical bond or C₁₋₆ alkylene; the C₁₋₆ alkylene is optionally substituted with 1, 2, or more R_{d} substituents;
each R_{d} is independently deuterium, halogen, or C₃₋₆ cycloalkyl;
R₉ and R₁₀ are each independently H, deuterium, or C₁₋₆ alkyl; the C₁₋₆ alkyl is optionally substituted with 1, 2, 3, or 4 R_{f} substituents;
R₁₁ is C₁₋₆ alkyl, C₆₋₁₀ aryl, 5- to 10-membered heteroaryl, C₃₋₈ cycloalkyl, or 3- to 8-membered heterocycloalkyl; the C₁₋₆ alkyl, C₆₋₁₀ aryl, 5- to 10-membered heteroaryl, C₃₋₈ cycloalkyl, and 3- to 8-membered heterocycloalkyl are each independently optionally substituted with 1, 2, or more R_{g} substituents;
Rₑ, R_{f}, and R_{g} are each independently deuterium, halogen, -OH, -CN, -COOH, C₁₋₆ alkoxy, C₁₋₆ alkyl, or C₃₋₆ cycloalkyl; the C₁₋₆ alkyl, C₁₋₆ alkoxy, and C₃₋₆ cycloalkyl are each independently optionally substituted with 1, 2, or more deuterium or halogen substituents;
Y₂ is a chemical bond, N(R₁₄), -C(=O)N(R₁₄)-, or O;
R₁₂, R₁₃, and R₁₄ are each independently H, deuterium, halogen, or C₁₋₆ alkyl; the C₁₋₆ alkyl is optionally substituted with 1, 2, or more halogen or deuterium substituents;
R₁₅ is C₁₋₆ alkyl, 3- to 10-membered heterocyclyl, or 5- to 10-membered heteroaryl; the C₁₋₆ alkyl, 3- to 10-membered heterocyclyl, and 5- to 10-membered heteroaryl are each independently optionally substituted with 1, 2, or more Rᵢ substituents;
each Rᵢ is independently deuterium, halogen, oxo, OH, COOH, NH₂, CN, C₃₋₆ cycloalkyl, C₁₋₆ alkyl, or C₁₋₆ alkoxy; the C₁₋₆ alkyl, C₃₋₆ cycloalkyl, and C₁₋₆ alkoxy are each independently optionally substituted with 1, 2, or more Rᵢ₋₁ substituents;
each Rᵢ₋₁ is independently deuterium, halogen, C₁₋₆ alkoxy, or C₃₋₆ cycloalkyl;
m is 0, 1, 2, 3, 4, or 5;
n and p are each independently 0, 1, 2, 3, or 4;
q is 0, 1, 2, or 3;
the heteroatoms in the 3- to 10-membered heterocycloalkyl, 5- to 7-membered heteroarylene, 8- to 10-membered heteroarylene, 3- to 6-membered heterocyclyl, 3- to 10-membered heterocyclyl, 5- to 10-membered heteroaryl, and 3- to 8-membered heterocycloalkyl are each independently selected from 1, 2, or 3 types of N, O, and S; the number of heteroatoms is independently 1, 2, or 3.

2. The compound of formula (I), the racemate thereof, the stereoisomer thereof, the tautomer thereof, the N-oxide thereof, or the pharmaceutically acceptable salt of any one of the foregoing according to claim 1, wherein ring A is C₃₋₁₀ cycloalkyl or 3- to 10-membered heterocycloalkyl;
in case (1): each R₁ is independently OH, COOH, CN, oxo, halogen, NH₂, 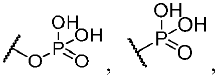 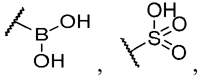 tetrazolyl, C₁₋₆ alkyl, C₁₋₆ alkoxy, -C₁₋₃ alkylene-COOH, -NHC(=O)OC₁₋₆ alkyl, - NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(-O)NHS(-O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (2): each R₁ is independently OH, CN, oxo, halogen, NH₂, 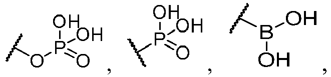 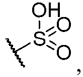
tetrazolyl, C₁₋₆ alkyl, C₁₋₆ alkoxy, -C₁₋₃ alkylene-COOH, -NHC(=O)OC₁₋₆ alkyl, -NHC(=O)C₁₋₆ alkyl, -C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (3): each R₁ is independently OH, CN, oxo, halogen, NH₂, 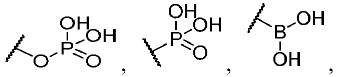 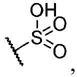 tetrazolyl, C₁₋₆ alkyl, C₁₋₆ alkoxy, -C₂₋₃ alkylene-COOH, -NHC(=O)OC₁₋₆ alkyl, -NHC(=O)C₁₋₆ alkyl, -C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₂₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(-O)NHS(-O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
R₆ and R₇ are each independently C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl is optionally substituted with 1, 2, or more R_{c} substituents; the C₃₋₈ cycloalkyl, 3- to 10-membered heterocyclyl, C₆₋₁₀ aryl, and 5- to 10-membered heteroaryl are each independently optionally substituted with 1, 2, or more Rₑ substituents;
preferably, ring A is C₃₋₁₀ cycloalkyl or 3- to 10-membered heterocycloalkyl;
in case (1): each R₁ is independently OH, COOH, CN, oxo, halogen, NH₂, 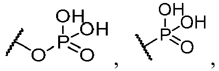 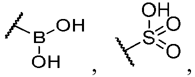 tetrazolyl, C₁₋₆ alkyl, C₁₋₆ alkoxy, -C₁₋₃ alkylene-COOH, -NHC(=O)OC₁₋₆ alkyl, - NHC(=O)C₁₋₆ alkyl, -C(-O)NHC₁₋₆ alkyl, -C(=O)OC₁₋₆ alkyl, -C(=O)NHS(=O)₂C₁₋₆ alkyl, - C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (2): each R₁ is independently OH, CN, oxo, halogen, NH₂, 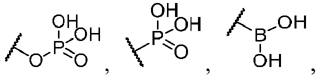 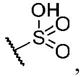 tetrazolyl, C₁₋₆ alkyl, C₁₋₆ alkoxy, -C₁₋₃ alkylene-COOH, -NHC(=O)OC₁₋₆ alkyl, -NHC(=O)C₁₋₆ alkyl, -C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(-O)NHS(-O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
in case (3): each R₁ is independently OH, CN, oxo, halogen, NH₂, 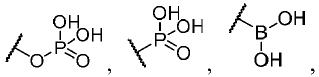 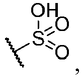 tetrazolyl, C₁₋₆ alkyl, C₁₋₆ alkoxy, -C₂₋₃ alkylene-COOH, -NHC(=O)OC₁₋₆ alkyl, -NHC(=O)C₁₋₆ alkyl, -C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, -C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₂₋₃ alkylene-COOH, -NHC(=O)OC₁₋₆ alkyl, -NHC(=O)C₁₋₆ alkyl, - C(=O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
each R₂ is independently halogen, NH₂, OH, oxo, CN, C₁₋₄ alkyl, C₁₋₄ alkoxy, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen substituents;
each R₃ is independently H, C₁₋₄ alkyl, C₁₋₄ alkoxy, halogen, NH₂, OH, oxo (=O), CN, C₃₋₆ cycloalkyl, or 3- to 6-membered heterocyclyl; the C₁₋₄ alkyl, C₁₋₄ alkoxy, C₃₋₆ cycloalkyl, and 3- to 6-membered heterocyclyl are each independently optionally substituted with 1, 2, or more halogen substituents;
R₆ and R₇ are each independently C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl is optionally substituted with 1, 2, or more R_{c} substituents; the C₃₋₈ cycloalkyl, 3- to 10-membered heterocyclyl, C₆₋₁₀ aryl, and 5- to 10-membered heteroaryl are each independently optionally substituted with 1, 2, or more Rₑ substituents;
each R_{c} is independently halogen, -OH, -CN, -COOH, C₁₋₆ alkoxy, C₁₋₆ alkyl, C₃₋₈ cycloalkyl, 3- to 10-membered heterocyclyl, C₆₋₁₀ aryl, or 5- to 10-membered heteroaryl; the C₁₋₆ alkyl, C₁₋₆ alkoxy, C₃₋₈ cycloalkyl, 3- to 10-membered heterocyclyl, C₆₋₁₀ aryl, and 5- to 10-membered heteroaryl are each independently optionally substituted with 1, 2, or more R_{c-1} substituents;
each R_{c-1} is independently halogen, -OH, -CN, -COOH, C₁₋₆ alkoxy, or C₁₋₆ alkyl;
R₉ and R₁₀ are each independently H or C₁₋₆ alkyl; the C₁₋₆ alkyl is optionally substituted with 1, 2, 3, or 4 R_{f} substituents;
Rₑ, R_{f}, and R_{g} are each independently halogen, -OH, -CN, -COOH, C₁₋₆ alkoxy, or C₁₋₆ alkyl; the C₁₋₆ alkyl and C₁₋₆ alkoxy are each independently optionally substituted with 1, 2, or more halogen substituents;
Y₂ is a chemical bond, NR₁₄, or O;
R₁₂, R₁₃, and R₁₄ are each independently H, halogen, or C₁₋₆ alkyl; the C₁₋₆ alkyl is optionally substituted with 1, 2, or more halogen substituents;
R₁₅ is 3- to 10-membered heterocyclyl or 5- to 10-membered heteroaryl; the 3- to 10-membered heterocyclyl and 5- to 10-membered heteroaryl are each independently optionally substituted with 1, 2, or more Rᵢ substituents;
each Rᵢ is independently halogen, oxo, OH, COOH, NH₂, CN, C₃₋₆ cycloalkyl, C₁₋₆ alkyl, or C₁₋₆ alkoxy; the C₁₋₆ alkyl is optionally substituted with 1, 2, or more Rᵢ₋₁ substituents;
each Rᵢ₋₁ is independently halogen, C₁₋₆ alkoxy, or C₃₋₆ cycloalkyl;
q is 0, 1, or 2.

3. The compound of formula (I), the racemate thereof, the stereoisomer thereof, the tautomer thereof, the N-oxide thereof, or the pharmaceutically acceptable salt of any one of the foregoing according to claim 1 or 2, wherein the compound of formula (I) satisfies one or more of the following conditions:
(1) in R₂, R₃, R₄, R₅, R₈, R_{d}, Rᵢ, Rᵢ₋₁, Rₑ, R_{f}, and R_{g}, each C₃₋₆ cycloalkyl is independently cyclopropyl, cyclobutyl, cyclopentyl, cyclohexyl, or bicyclo[1.1.1]pentyl;
(2) in R₆, R₇, R₁₁, and R_{c}, each C₃₋₈ cycloalkyl is independently cyclopropyl, cyclobutyl, cyclopentyl, cyclohexyl, bicyclo[1.1.1]pentyl, bicyclo[2.1.1]hexyl, spiro[2.2]pentyl, or spiro[2.3]hexyl;
(3) in R₆, R₇, R₁₁, and R_{c}, each C₆₋₁₀ aryl is independently phenyl or naphthyl, for example, phenyl;
(4) in R₂ and R₃, the heteroatom in each 3- to 6-membered heterocyclyl is independently N or O, and the number of the heteroatom is independently 1 or 2;
(5) in R₆, R₇, and R_{c}, each 3- to 10-membered heterocyclyl is independently 3- to 6-membered heterocyclyl, wherein the heteroatom in the 3- to 6-membered heterocyclyl is independently N, O, or S, and the number of the heteroatom is independently 1 or 2;
(6) in R₁₁, the heteroatom of the 3- to 8-membered heterocycloalkyl in the 3- to 8-membered heterocycloalkyl optionally substituted with 1, 2, or more R_{g} substituents is N, and the number of the heteroatom is independently 1 or 2;
(7) in ring A, the C₃₋₁₀ cycloalkyl is C₃₋₈ cycloalkyl, for example, cyclopropyl or C₄₋₈ cycloalkyl; for another example, C₃₋₆ cycloalkyl;
(8) in ring A, the 3- to 10-membered heterocycloalkyl is 4- to 8-membered heterocycloalkyl, for example, 4- to 6-membered heterocycloalkyl;
(9) in ring A, the 5- to 10-membered heteroaryl is 5- to 6-membered heteroaryl, for example, 5-membered heteroaryl or 6-membered heteroaryl; the heteroatom of the 5- to 6-membered heteroaryl, 5-membered heteroaryl, and 6-membered heteroaryl is N, and the number of the heteroatom is 1 or 2;
(10) in L₁, each C₂₋₆ alkynylene is independently ethynylene, 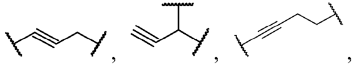 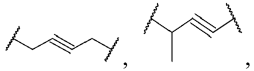 pentynylene, or hexynylene; preferably, each C₂₋₆ alkynylene is independently C₂₋₄ alkynylene, for example, 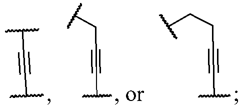
(11) in ring B, the C₆₋₁₀ arylene is phenylene or naphthylene;
(12) in ring B, the 5- to 7-membered heteroarylene is 6-membered heteroarylene, wherein the heteroatom in the 6-membered heteroarylene is N, and the number of the heteroatom is 1, 2, or 3;
(13) in R₁₅, the 3- to 10-membered heterocyclyl in the 3- to 10-membered heterocyclyl optionally substituted with 1, 2, or more Rᵢ substituents is independently 3- to 10-membered heterocycloalkyl or 3- to 10-membered heterocycloalkenyl, wherein the number of double bonds in the 3- to 10-membered heterocycloalkenyl is 1 or 2;
(14) in R₁₅, the 3- to 10-membered heterocyclyl in the 3- to 10-membered heterocyclyl optionally substituted with 1, 2, or more Rᵢ substituents is independently 3- to 8-membered heterocyclyl, for example, 3- to 6-membered heterocyclyl;
(15) in R₁₅, the 5- to 10-membered heteroaryl in the 5- to 10-membered heteroaryl optionally substituted with 1, 2, or more Rᵢ substituents is 5- to 6-membered heteroaryl, for example, 6-membered heteroaryl.

4. The compound of formula (I), the racemate thereof, the stereoisomer thereof, the tautomer thereof, the N-oxide thereof, or the pharmaceutically acceptable salt of any one of the foregoing according to any one of claims 1 to 3, wherein the compound of formula (I) satisfies one or more of the following conditions:
(1) in ring A, the C₃₋₁₀ cycloalkyl is cyclopropyl, cyclobutyl, cyclopentyl, cyclohexyl, cycloheptyl, cyclooctyl, bicyclo[1.1.1]pentyl, spiro[2.3]hexyl, bicyclo[2.1.1]hexyl, bicyclo[3.1.0]hexyl, bicyclo[3.2.0]heptyl, spiro[2.4]heptyl, or bicyclo[2.2.2]octyl, for example, 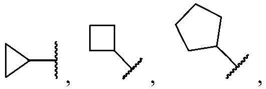 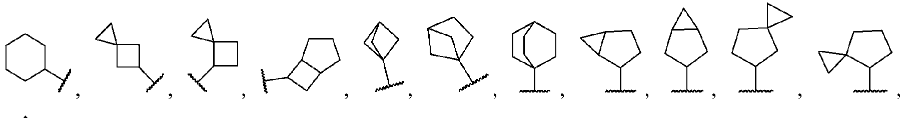 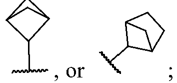
(2) in ring A, the 3- to 10-membered heterocycloalkyl is oxiranyl, oxetanyl, tetrahydrofuranyl, tetrahydropyranyl, oxepanyl, oxocanyl, aziridinyl, azetidinyl, pyrrolidinyl, piperidinyl, azepanyl, or azocanyl; for example, 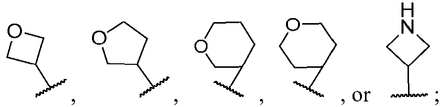
(3) in ring A, the 5- to 10-membered heteroaryl is pyridyl; for example, 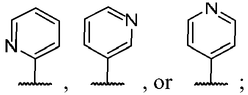
(4) in R₁, the halogen is fluorine, chlorine, or bromine;
(5) in R₁, each C₁₋₆ alkyl is independently C₁₋₄ alkyl; for example, methyl, ethyl, or n-propyl;
(6) in R₁, the C₁₋₆ alkoxy in the C₁₋₆ alkoxy optionally substituted with 1, 2, or more Rₐ substituents is independently C₁₋₃ alkoxy; for example, methoxy or ethoxy;
(7) in R₁, unsubstituted -C₁₋₃ alkylene-COOH is 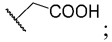
(8) in R₁, unsubstituted -NHC(=O)OC₁₋₆ alkyl is 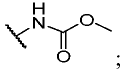
(9) in R₁, unsubstituted -C₁₋₃ alkylene-C(=O)NHC(=O)C₁₋₆ alkyl is 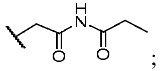
(10) in R₁, unsubstituted -C₁₋₃ alkylene-C(=O)NHS(=O)₂C₁₋₆ alkyl is 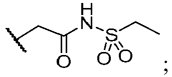
(11) in R₁, unsubstituted -C₁₋₃ alkylene-S(=O)₂OH is 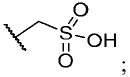
(12) in R₁, unsubstituted -C₁₋₃ alkylene-P(=O)(OH)₂ is 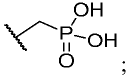
(13) in R₁, unsubstituted -C₁₋₃ alkylene-tetrazolyl is 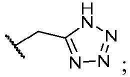
(14) in R₁, -NHS(=O)₂OH substituted with 1, 2, or more Rₐ substituents is 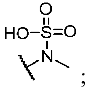
(15) in R₁, unsubstituted -C₁₋₃ alkylene-NHS(=O)₂OH is 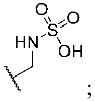
(16) in R₁, unsubstituted -C₁₋₃ alkylene-S(=O)₂C₁₋₆ alkyl is 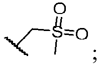
(17) in Rₐ, the halogen is fluorine, chlorine, or bromine;
(18) in R_{b}, the C₁₋₆ alkyl in the C₁₋₆ alkyl optionally substituted with 1, 2, or 3 COOH or halogen substituents is independently C₁₋₃ alkyl; for example, methyl;
(19) ring B is pyridinylene, pyrimidinylene, pyridazinylene, pyrazinylene, or triazinylene; for example, 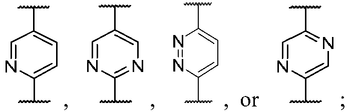 for another example, 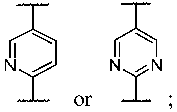 for yet another example, 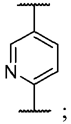
(20) in R₂, each halogen is independently fluorine, chlorine, or bromine;
(21) in R₂, the C₁₋₄ alkyl in the C₁₋₄ alkyl optionally substituted with 1, 2, or more halogen or deuterium substituents is methyl or ethyl;
(22) in R₃, the halogen is fluorine, chlorine, or bromine;
(23) in R₃, the C₁₋₄ alkyl in the C₁₋₄ alkyl optionally substituted with 1, 2, or more halogen or deuterium substituents is independently methyl or ethyl;
(24) in R₆ and R₇, the C₁₋₆ alkyl in the C₁₋₆ alkyl optionally substituted with 1, 2, or more R_{c} substituents is independently methyl, ethyl, n-propyl, n-butyl, n-pentyl, 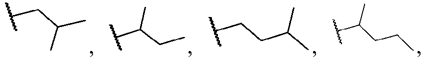 or 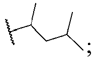
(25) in R₆ and R₇, the C₃₋₈ cycloalkyl in the C₃₋₈ cycloalkyl optionally substituted with 1, 2, or more Rₑ substituents is independently C₃₋₆ cycloalkyl, for example, cyclopentyl;
(26) in R₆ and R₇, the 5- to 10-membered heteroaryl in the 5- to 10-membered heteroaryl optionally substituted with 1, 2, or more Rₑ substituents is a monocyclic ring, for example, the 5- to 10-membered heteroaryl is independently 5- to 6-membered heteroaryl; for another example, the 5- to 10-membered heteroaryl is independently pyridyl or thiazolyl, for yet another example, 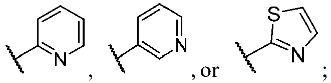
(27) in R_{c}, the halogen is fluorine, chlorine, or bromine;
(28) in R_{c}, the C₁₋₆ alkyl in the C₁₋₆ alkyl optionally substituted with 1, 2, or more R_{c-1} substituents is independently methyl or ethyl;
(29) in R_{c}, the C₃₋₈ cycloalkyl in the C₃₋₈ cycloalkyl optionally substituted with 1, 2, or more R_{c-1} substituents is independently cyclopropyl, cyclobutyl, cyclopentyl, bicyclo[1.1.1]pentyl, or spiro[2.2]pentyl; the spiro[2.2]pentyl is 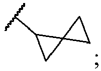 the bicyclo[1.1.1]pentyl is 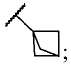
(30) in R_{c}, the 5- to 10-membered heteroaryl in the 5- to 10-membered heteroaryl optionally substituted with 1, 2, or more R_{c-1} substituents is a monocyclic ring, for example, the 5- to 10-membered heteroaryl is independently 5- to 6-membered heteroaryl; for another example, pyridyl, oxazolyl, or thiazolyl, for yet another example, 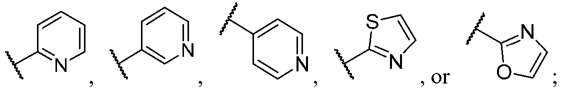
(31) in R_{c-1}, the halogen is fluorine, chlorine, or bromine;
(32) in R_{c-1}, the C₁₋₆ alkyl is C₁₋₃ alkyl; for example, methyl;
(33) in R₉ and R₁₀, the C₁₋₆ alkyl in the C₁₋₆ alkyl optionally substituted with 1, 2, 3, or 4 R_{f} substituents is independently C₁₋₃ alkyl; for example, methyl;
(34) in R_{f}, the halogen is fluorine, chlorine, or bromine;
(35) in R₁₁, the C₁₋₆ alkyl in the C₁₋₆ alkyl optionally substituted with 1, 2, or more R_{g} substituents is independently C₁₋₄ alkyl; for example, methyl, ethyl, n-propyl, n-butyl, 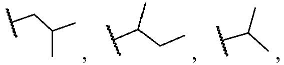 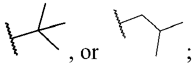 for another example, ethyl or n-propyl;
(36) in R₁₁, the C₃₋₈ cycloalkyl in the C₃₋₈ cycloalkyl optionally substituted with 1, 2, or more R_{g} substituents is independently C₃₋₆ cycloalkyl, wherein the C₃₋₆ cycloalkyl is a monocyclic, spiro, or bridged ring; the C₃₋₈ cycloalkyl may independently be cyclopropyl, cyclobutyl, cyclopentyl, cyclohexyl, bicyclo[1.1.1]pentyl, spiro[2.2]pentyl, spiro[2.3]hexyl, or bicyclo[2.1.1]hexyl; for another example, cyclopropyl, cyclobutyl, cyclopentyl, bicyclo[1.1.1]pentyl, spiro[2.3]hexyl, or bicyclo[2.1.1]hexyl; bicyclo[1.1.1]pentyl may be 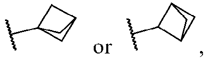 spiro[2.3]hexyl may be 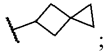 bicyclo[2.1.1]hexyl may be 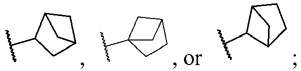
(37) in R₁₁, the 5- to 10-membered heteroaryl in the 5- to 10-membered heteroaryl optionally substituted with 1, 2, or more R_{g} substituents is independently 5- to 6-membered heteroaryl; preferably 6-membered heteroaryl; for example, pyridyl, for another example, 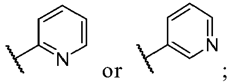
(38) in R_{g}, the halogen is fluorine, chlorine, or bromine;
(39) in R₁₅, the 3- to 10-membered heterocyclyl in the 3- to 10-membered heterocyclyl optionally substituted with 1, 2, or more Rᵢ substituents is independently 3- to 6-membered heterocycloalkyl; for example, azetidinyl, pyrrolidinyl, or piperidinyl, for another example, 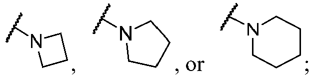
(40) in R₁₅, the 3- to 10-membered heterocyclyl in the 3- to 10-membered heterocyclyl optionally substituted with 1, 2, or more Rᵢ substituents is independently 3- to 6-membered heterocycloalkenyl, wherein the number of double bonds in the 3- to 6-membered heterocycloalkenyl is 1 or 2; for example, the 3- to 6-membered heterocycloalkenyl is 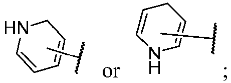 for another example, 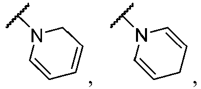 or 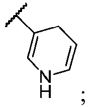
(41) in R₁₅, the 5- to 10-membered heteroaryl in the 5- to 10-membered heteroaryl optionally substituted with 1, 2, or more Rᵢ substituents is independently 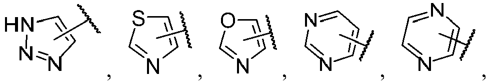 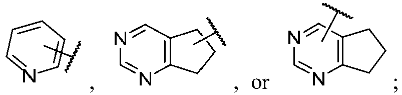 for example, 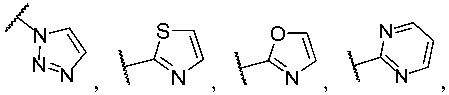 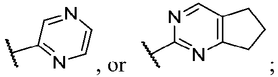
(42) in Rᵢ, the C₁₋₆ alkyl in the C₁₋₆ alkyl optionally substituted with 1, 2, or more Rᵢ₋₁ substituents is independently C₁₋₄ alkyl; for example, methyl, ethyl, n-propyl, n-butyl, or isopropyl; preferably, C₁₋₃ alkyl; for another example, methyl, ethyl, or isopropyl;
(43) in Rᵢ, the C₁₋₆ alkoxy is C₁₋₃ alkoxy; for example, ethoxy or isopropoxy;
(44) in Rᵢ, the C₃₋₆ cycloalkyl optionally substituted with 1, 2, or more Rᵢ₋₁ substituents is cyclopropyl, cyclobutyl, or bicyclo[1.1.1]pentyl; bicyclo[1.1.1]pentyl may be 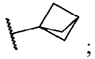 the C₃₋₆ cycloalkyl may be cyclopropyl, cyclobutyl, or bicyclo[1.1.1]pentyl;
(45) in Rᵢ₋₁, the C₃₋₆ cycloalkyl is cyclopropyl or cyclobutyl; and
(46) in Rᵢ₋₁, the C₁₋₆ alkoxy is methoxy or ethoxy.

5. The compound of formula (I), the racemate thereof, the stereoisomer thereof, the tautomer thereof, the N-oxide thereof, or the pharmaceutically acceptable salt of any one of the foregoing according to any one of claims 1 to 4, wherein the compound of formula (I) satisfies one or more of the following conditions:
(1) in R₁, the C₁₋₆ alkyl optionally substituted with 1, 2, or more Rₐ substituents is -CF₃, -CHF₂, 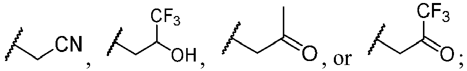
(2) in R₁, the -C₁₋₃ alkylene-COOH substituted with 1, 2, or more Rₐ substituents is 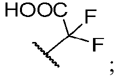
(3) in R_{b}, the C₁₋₆ alkyl substituted with 1, 2, or 3 COOH substituents is 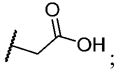 preferably, the C₂₋₆ alkynyl substituted with 1, 2, or 3 R_{b} substituents is 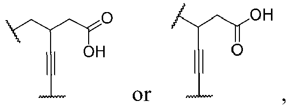 for example, 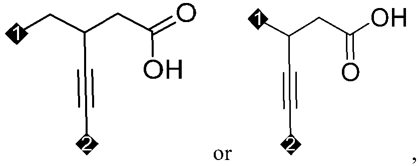 wherein the "1" position is connected to ring A, and the "2" position is connected to ring B;
(4) in R₂, the C₁₋₄ alkyl substituted with 1, 2, or more halogen or deuterium substituents is -CD₃, -CF₃, - CHF₂, or -CH₂F;
(5) in R₆ and R₇, the C₁₋₆ alkyl substituted with 1, 2, or more R_{c} substituents is independently 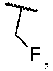 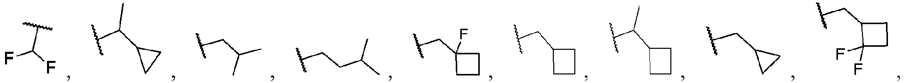 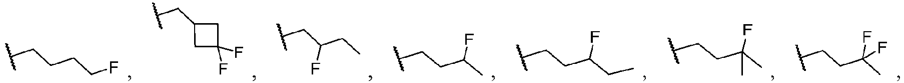 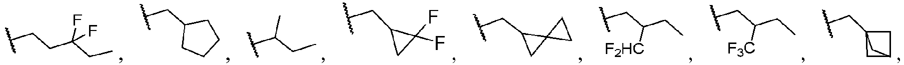 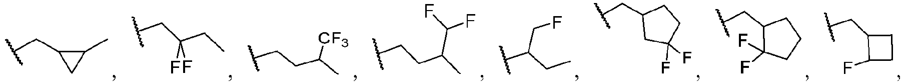 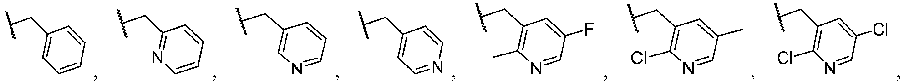 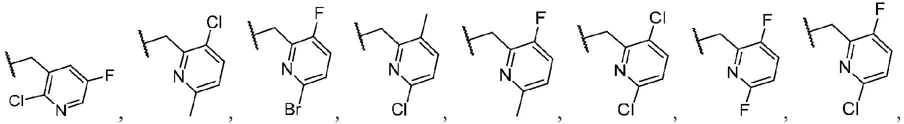 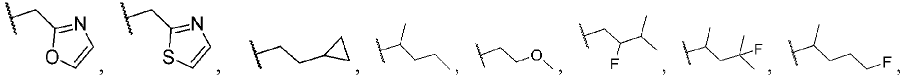 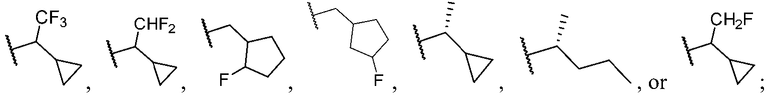
(6) in R₉ and R₁₀, the C₁₋₆ alkyl substituted with 1, 2, 3, or 4 R_{f} substituents is -CD₃, -CH₂F, or -CHF₂;
(7) in R₁₁, the C₃₋₈ cycloalkyl substituted with 1, 2, or more R_{g} substituents is 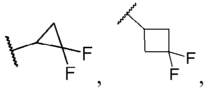 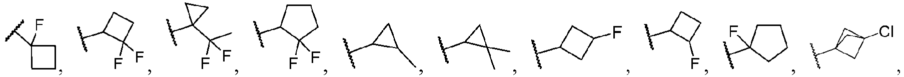 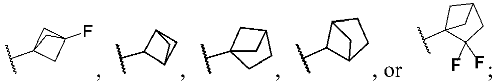
(8) in R₁₁, the C₆₋₁₀ aryl substituted with 1, 2, or more R_{g} substituents is 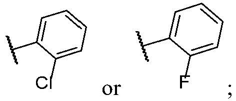
(9) in R₁₁, the 5- to 10-membered heteroaryl substituted with 1, 2, or more R_{g} substituents is 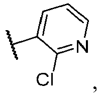 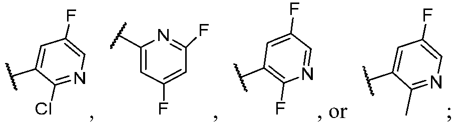 and
(10) in R₁₁, the C₁₋₆ alkyl substituted with 1, 2, or more R_{g} substituents is

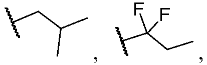

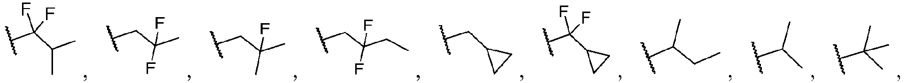

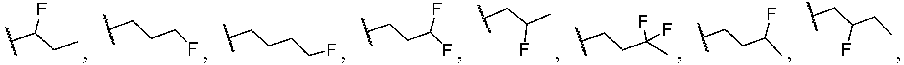

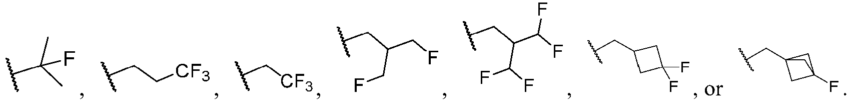

6. The compound of formula (I), the racemate thereof, the stereoisomer thereof, the tautomer thereof, the N-oxide thereof, or the pharmaceutically acceptable salt of any one of the foregoing according to claim 1, wherein the compound of formula (I) satisfies one or more of the following conditions:
(1) ring A is C₃₋₈ cycloalkyl, 4- to 6-membered oxacycloalkyl, or 4- to 6-membered azacycloalkyl; the number of the heteroatom in the 4- to 6-membered oxacycloalkyl and 4- to 6-membered azacycloalkyl is 1; or, ring A is C₄₋₈ cycloalkyl or 3- to 10-membered heterocycloalkyl, wherein the heteroatom in the 3- to 10-membered heterocycloalkyl is selected from 1, 2, or 3 types of N, O, and S, and the number of the heteroatom is 1, 2, or 3; or, ring A is 5- to 6-membered heteroaryl, wherein the heteroatom in the 5- to 6-membered heteroaryl is selected from 1, 2, or 3 types of N, O, and S, and the number of the heteroatom is 1, 2, or 3; or, ring A is C₃₋₈ cycloalkyl, 4- to 6-membered heterocycloalkyl, or 5- to 6-membered heteroaryl, wherein the heteroatom in the 4- to 6-membered heterocycloalkyl is N or O, and the number of the heteroatom is 1 or 2; the heteroatom in the 5- to 6-membered heteroaryl is N, and the number of the heteroatom is 1 or 2; for example, ring A is C₃₋₈ cycloalkyl;
(2) in case (1), R₁ is independently OH, COOH, F, Cl, Br, CN, 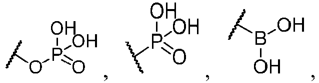 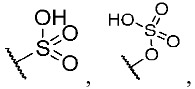 tetrazolyl, C₁₋₆ alkyl, C₁₋₆ alkoxy, -C₁₋₃ alkylene-COOH, -NHC(=O)OC₁₋₃ alkyl, - NHS(=O)₂OH, -C₁₋₃ alkylene-NHS(=O)₂OH, -C₁₋₃ alkylene-C(=O)NHC(=O)C₁₋₃ alkyl, -C₁₋₃ alkylene-C(=O)NHS(=O)₂C₁₋₃ alkyl, -C₁₋₃ alkylene-S(=O)₂OH, -C₁₋₃ alkylene-S(=O)₂C₁₋₆ alkyl, -C₁₋₃ alkylene-P(=O)(OH)₂, -C₁₋₃ alkylene-tetrazolyl, or -C₁₋₃ alkylene-C(=O)C₁₋₃ alkyl;
the C₁₋₆ alkyl, C₁₋₆ alkoxy, -C₁₋₃ alkylene-COOH, -NHC(=O)OC₁₋₃ alkyl, -NHS(=O)₂OH, -C₁₋₃ alkylene-NHS(=O)₂OH, -C₁₋₃ alkylene-C(=O)NHC(=O)C₁₋₃ alkyl, -C₁₋₃ alkylene-C(=O)NHS(=O)₂C₁₋₃ alkyl, -C₁₋₃ alkylene-S(=O)₂OH, -C₁₋₃ alkylene-S(=O)₂C₁₋₆ alkyl, -C₁₋₃ alkylene-P(=O)(OH)₂, -C₁₋₃ alkylene-tetrazolyl, and -C₁₋₃ alkylene-C(=O)C₁₋₃ alkyl are each independently optionally substituted with 1, 2, or more Rₐ substituents; preferably, R₁ is -C₁₋₃ alkylene-COOH; or, each R₁ is independently COOH or -C₁₋₃ alkylene-COOH;
or, in case (1), each R₁ is independently CN, oxo, halogen, NH₂, 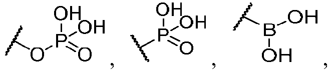 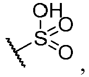 C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -C₁₋₃ alkyleneS(=O)₂NHC(=O)C₁₋₆ alkyl, -C₁₋₃ alkylene-P(=O)(OH)₂, or -C₁₋₃ alkylene-tetrazolyl;
the C₁₋₆ alkyl, C₁₋₆ alkoxy, -C₁₋₃ alkylene-COOH, -NHC(=O)OC₁₋₆ alkyl, -NHC(=O)C₁₋₆ alkyl, - NHS(=O)₂OH, -C₁₋₃ alkylene-NHS(=O)₂OH, -C(=O)NHC₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -C₁₋₃ alkyleneS(=O)₂NHC(=O)C₁₋₆ alkyl, -C₁₋₃ alkylene-P(=O)(OH)₂, and -C₁₋₃ alkylene-tetrazolyl are each independently optionally substituted with 1, 2, or more Rₐ substituents;
(3) in case (3), each R₁ is independently 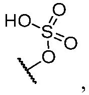 -NHS(=O)₂OH, or -C₁₋₃ alkylene-NHS(=O)₂OH; the -NHS(=O)₂OH and -C₁₋₃ alkylene-NHS(=O)₂OH are each independently optionally substituted with 1, 2, or more Rₐ substituents; each Rₐ is independently C₁₋₄ alkyl;
(4) in case (1), each Rₐ is independently methyl, oxo, OH, F, Cl, or CN;
(5) m is 0, 1, 2, or 3, for example, 1;
(6) each R₂ is independently halogen, -NH₂, C₁₋₄ alkyl, C₁₋₄ alkoxy, C₃₋₆ cycloalkyl, or 3- to 6-membered heterocyclyl; the C₁₋₄ alkyl is optionally substituted with 1, 2, or more halogen or deuterium substituents; preferably, each R₂ is independently halogen, C₁₋₄ alkyl, methoxy, or cyclopropyl; the C₁₋₄ alkyl is optionally substituted with 1, 2, or more halogen or deuterium substituents; or, each R₂ is independently halogen, C₁₋₄ alkyl, or C₃₋₆ cycloalkyl; the C₁₋₄ alkyl and C₃₋₆ cycloalkyl are each independently optionally substituted with 1, 2, or more halogen or deuterium substituents; for example, each R₂ is independently C₁₋₄ alkyl;
(7) n is 0 or 1;
(8) L₁ is C₂₋₆ alkynylene, wherein the C₂₋₆ alkynylene is optionally substituted with 1, 2, or 3 R_{b} substituents; preferably, L₁ is unsubstituted C₂₋₆ alkynylene;
(9) each R_{b} is independently C₁₋₆ alkyl; the C₁₋₆ alkyl is optionally substituted with 1, 2, or 3 COOH substituents; preferably, the C₁₋₆ alkyl is optionally substituted with 1 COOH substituent;
(10) X₁, X₂, X₃, and X₄ are each independently C, CH, CH₂, O, S, N, or NH; simultaneously, two or three of X₁, X₂, X₃, and X₄ are each independently O, S, N, or NH; for example, X₁ may be C or N; X₂ may be CH, O, S, or N; X₃ may be C, CH₂, O, S, or N; X₄ may be C, CH, N, or NH;
(11) each R₃ is independently H, C₁₋₄ alkyl, oxo, F, Cl, or Br; the C₁₋₄ alkyl is optionally substituted with 1, 2, or more halogen or deuterium substituents; preferably, each R₃ is independently H, C₁₋₄ alkyl, F, Cl, or oxo; the C₁₋₄ alkyl is optionally substituted with 1, 2, or more halogen or deuterium substituents; more preferably, each R₃ is independently C₁₋₄ alkyl, F, Cl, Br, or oxo; most preferably, each R₃ is independently C₁₋₄ alkyl;
(12) p is 0, 1, 2, or 3;
(13) R₄ and R₅ are each independently H, halogen, or C₁₋₄ alkyl, wherein the C₁₋₄ alkyl is optionally substituted with 1, 2, or more halogen substituents; or, R₄ and R₅ are each independently H or deuterium, for example, H;
(14) R₆ is H or C₁₋₆ alkyl; the C₁₋₆ alkyl is optionally substituted with 1, 2, or more R_{c} substituents;
(15) in R₆, each R_{c} is independently halogen, for example, fluorine, chlorine, or bromine;
(16) R₇ is C₁₋₆ alkyl, C₃₋₈ cycloalkyl, C₆₋₁₀ aryl, or 5- to 10-membered heteroaryl; the C₁₋₆ alkyl is optionally substituted with 1, 2, or more R_{c} substituents; the C₃₋₈ cycloalkyl, C₆₋₁₀ aryl, and 5- to 10-membered heteroaryl are each independently optionally substituted with 1, 2, or more Rₑ substituents; preferably, R₇ is C₁₋₆ alkyl, C₃₋₈ cycloalkyl, phenyl, or 5- to 6-membered heteroaryl; the C₁₋₆ alkyl is optionally substituted with 1, 2, or more R_{c} substituents; the C₃₋₈ cycloalkyl, phenyl, and 5- to 6-membered heteroaryl are each independently optionally substituted with 1, 2, or more Rₑ substituents; the heteroatom in the 5- to 6-membered heteroaryl is selected from 1 or 2 types of N, O, and S, and the number of the heteroatom is 1 or 2; more preferably, R₇ is C₁₋₆ alkyl, C₃₋₈ cycloalkyl, phenyl, or 5- to 6-membered heteroaryl; the C₁₋₆ alkyl is optionally substituted with 1, 2, or more R_{c} substituents;
(17) in R₇, each R_{c} is independently deuterium, halogen, C₁₋₄ alkyl, C₃₋₈ cycloalkyl, C₆₋₁₀ aryl, or 5- to 10-membered heteroaryl; the C₁₋₄ alkyl, C₃₋₈ cycloalkyl, C₆₋₁₀ aryl, and 5- to 10-membered heteroaryl are each independently optionally substituted with 1, 2, or more R_{c-1} substituents;
(18) in R₇, each R_{c-1} is independently deuterium, halogen, or C₁₋₆ alkyl;
(19) in R₇, each Rₑ is independently halogen or C₁₋₆ alkyl, for example, fluorine, chlorine, bromine, or methyl;
(20) R₈ is H, deuterium, halogen, or C₁₋₄ alkyl; for example, H or deuterium; for another example, H;
(21) Y₁ is a chemical bond;
(22) Y₁ is C₁₋₆ alkylene; the C₁₋₆ alkylene is optionally substituted with 1, 2, or more R_{d} substituents; preferably, Y₁ is unsubstituted C₁₋₄ alkylene;
(23) R_{f} is halogen or deuterium;
(24) R₉ is H or deuterium; for example, H;
(25) R₁₀ is H, deuterium, or C₁₋₄ alkyl; the C₁₋₄ alkyl is optionally substituted with 1, 2, 3, or 4 R_{f} substituents;
(26) R₁₁ is C₁₋₆ alkyl, C₆₋₁₀ aryl, 5- to 10-membered heteroaryl, or C₃₋₈ cycloalkyl; the C₁₋₆ alkyl, C₆₋₁₀ aryl, 5- to 10-membered heteroaryl, and C₃₋₈ cycloalkyl are each independently optionally substituted with 1, 2, or more R_{g} substituents; preferably, R₁₁ is C₁₋₆ alkyl, phenyl, 5- to 6-membered heteroaryl, or C₃₋₆ cycloalkyl; the C₃₋₆ cycloalkyl is a monocyclic, spiro, or bridged ring; the C₁₋₆ alkyl, phenyl, 5- to 6-membered heteroaryl, and C₃₋₆ cycloalkyl are each independently optionally substituted with 1, 2, or more R_{g} substituents; the heteroatom in the 5- to 6-membered heteroaryl is N, and the number of the heteroatom is 1 or 2; preferably, R₁₁ is C₁₋₆ alkyl, C₃₋₄ cycloalkyl, bicyclo[1.1.1]pentyl, spiro[2.3]hexyl, bicyclo[2.1.1]hexyl, bicyclo[1.1.1]pentyl, spiro[2.3]hexyl, or bicyclo[2.1.1]hexyl; the C₁₋₆ alkyl, C₃₋₄ cycloalkyl, bicyclo[1.1.1]pentyl, spiro[2.3]hexyl, bicyclo[2.1.1]hexyl, bicyclo[1.1.1]pentyl, spiro[2.3]hexyl, and bicyclo[2.1.1]hexyl are each independently optionally substituted with 1, 2, or more R_{g} substituents, for example, R₁₁ is C₁₋₆ alkyl or C₃₋₄ cycloalkyl; the C₁₋₆ alkyl and C₃₋₄ cycloalkyl are each independently optionally substituted with 1, 2, or more R_{g} substituents;
or, R₁₁ is cyclopentyl, cyclohexyl, phenyl, or 5- to 6-membered heteroaryl; the cyclopentyl, cyclohexyl, phenyl, and 5- to 6-membered heteroaryl are each independently optionally substituted with 1, 2, or more R_{g} substituents; for example, phenyl or 5- to 6-membered heteroaryl; the phenyl or 5- to 6-membered heteroaryl is independently optionally substituted with 1, 2, or more R_{g} substituents;
(27) each R_{g} is independently deuterium, halogen, C₁₋₆ alkyl, or C₃₋₆ cycloalkyl; the C₁₋₆ alkyl and C₃₋₆ cycloalkyl are each independently optionally substituted with 1, 2, or more deuterium or halogen substituents, for example, each R_{g} is independently deuterium or halogen; or each R_{g} is independently halogen or C₁₋₆ alkyl; the C₁₋₆ alkyl is optionally substituted with 1, 2, or more halogen substituents; preferably, each R_{g} is halogen;
(28) q is 0 or 1;
(29) Y₂ is a chemical bond, NH, -C(=O)N(CH₃)-, or O;
(30) R₁₂ and R₁₃ are each independently H, methyl, or F; or R₁₂ and R₁₃ are each independently H, deuterium, or halogen;
(31) R₁₄ is H, methyl, or F; or R₁₄ is H, deuterium, or C₁₋₆ alkyl;
(32) R₁₅ is C₁₋₆ alkyl, 3- to 6-membered heterocyclyl, or 5- to 6-membered heteroaryl; the C₁₋₆ alkyl, 3-to 6-membered heterocyclyl, and 5- to 6-membered heteroaryl are each independently optionally substituted with 1, 2, or more Rᵢ substituents; the heteroatom in the 3- to 6-membered heterocyclyl is N or O, and the number of the heteroatom is 1 or 2; the heteroatom in the 5- to 6-membered heteroaryl is N, O, or S, and the number of the heteroatom may be 1, 2, or 3;
(33) each Rᵢ is independently halogen, oxo, C₃₋₆ cycloalkyl, C₁₋₆ alkyl, or C₁₋₆ alkoxy; the C₁₋₆ alkyl, C₃₋₆ cycloalkyl, and C₁₋₆ alkoxy are each independently optionally substituted with 1, 2, or more Rᵢ₋₁ substituents; and
(34) each Rᵢ₋₁ is independently halogen, C₁₋₆ alkyl, C₃₋₆ cycloalkyl, or C₁₋₆ alkoxy.

7. The compound of formula (I), the racemate thereof, the stereoisomer thereof, the tautomer thereof, the N-oxide thereof, or the pharmaceutically acceptable salt of any one of the foregoing according to claim 6, wherein the compound of formula (I) satisfies one or more of the following conditions:
(1) ring A is cyclopropyl, cyclobutyl, cyclopentyl, cyclohexyl, cycloheptyl, cyclooctyl, bicyclo[1.1.1]pentyl, spiro[2.3]hexyl, bicyclo[2.1.1]hexyl, bicyclo[3.1.0]hexyl, bicyclo[3.2.0]heptyl, spiro[2.4]heptyl, bicyclo[2.2.2]octyl, oxiranyl, oxetanyl, tetrahydrofuranyl, tetrahydropyranyl, oxepanyl, oxocanyl, aziridinyl, azetidinyl, pyrrolidinyl, piperidinyl, azepanyl, azocanyl, or pyridyl; preferably, ring A is cyclopropyl, cyclobutyl, cyclopentyl, cyclohexyl, cycloheptyl, cyclooctyl, spiro[2.3]hexyl, spiro[2.4]heptyl, bicyclo[1.1.1]pentyl, bicyclo[2.1.1]hexyl, bicyclo[3.1.0]hexyl, bicyclo[3.2.0]heptyl, bicyclo[2.2.2]octyl, oxetanyl, tetrahydrofuranyl, tetrahydropyranyl, azetidinyl, or pyridyl; more preferably, ring A is 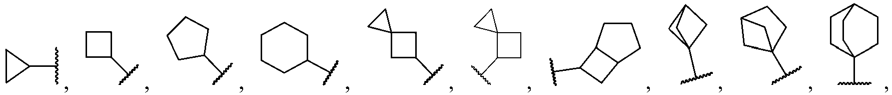 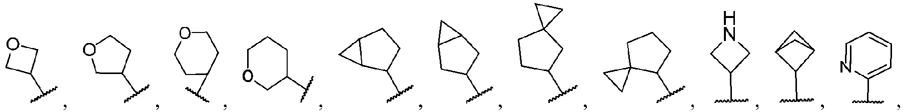 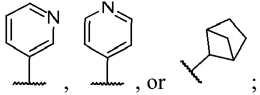 for example, cyclopropyl;
(2) in case (1), each R₁ is independently OH, COOH, F, Cl, CN, methyl, -CH₂F, -CHF₂, -CF₃, 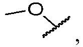 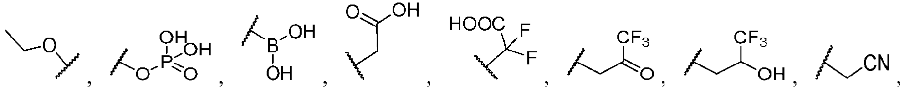 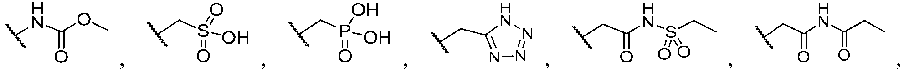 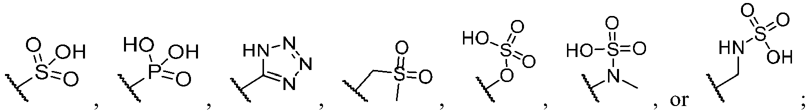 for example, OH, COOH, F, Cl, CN, methyl, -CH₂F, -CHF₂, -CF₃, 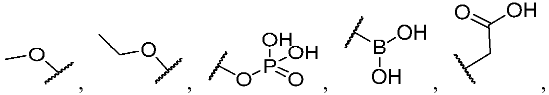 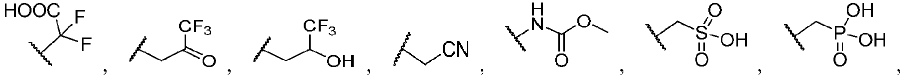 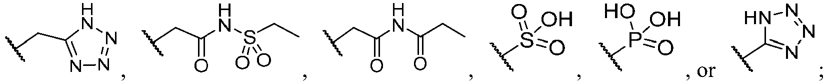
(3) in case (2), each R₁ is independently OH, F, Cl, CN, methyl, -CH₂F, -CHF₂, -CF₃, 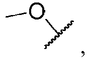 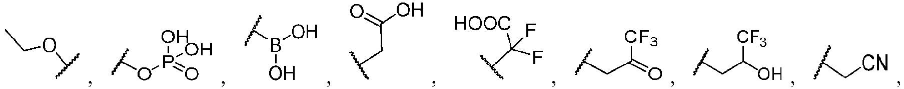 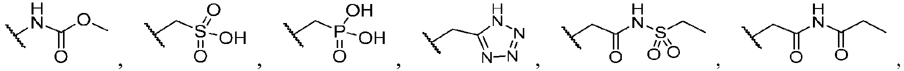 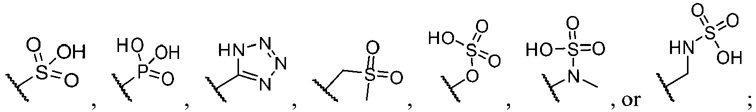
(4) in case (3), each R₁ is independently OH, F, Cl, CN, methyl, -CH₂F, -CHF₂, -CF₃, 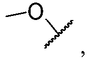 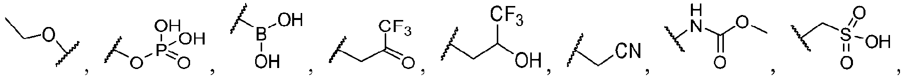 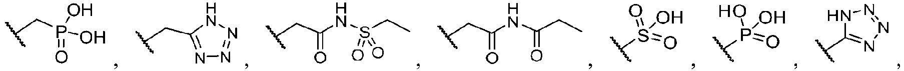 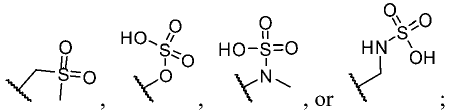 for example, 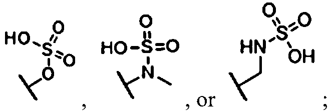
(5) each R₂ is independently methyl, ethyl, F, Cl, Br, -CD₃, -CF₃, -CHF₂, -CH₂F, -NH₂, -OCH₃, or cyclopropyl; for example, methyl, ethyl, F, Cl, -CD₃, -CF₃, -CHF₂, -CH₂F, -OCH₃, or cyclopropyl; for example, methyl, ethyl, F, Cl, -CD₃, -CF₃, -CHF₂, -OCH₃, or cyclopropyl; or, each R₂ is independently methyl, ethyl, F, Cl, Br, -CF₃, -CHF₂, -CH₂F, or -NH₂; for example, methyl, ethyl, F, Cl, -CF₃, or -CHF₂;
(6) L₁ is 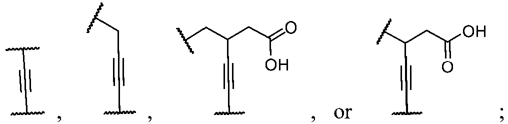 for example, 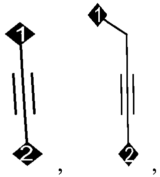 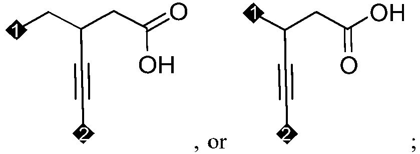 wherein the "1" position is connected to ring A, and the "2" position is connected to ring B;
(7) 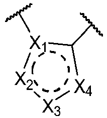 is triazolyl, pyrazolyl, imidazolyl, oxazolyl, tetrazolyl, isoxazolyl, thiazolyl, isothiazolyl, oxadiazolyl, furazanyl, thiadiazolyl, oxathiazolyl, 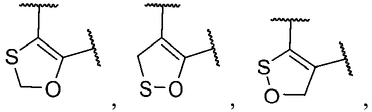 thienyl, furyl, or pyrrolyl; preferably, 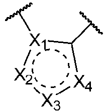 is 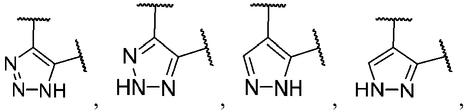 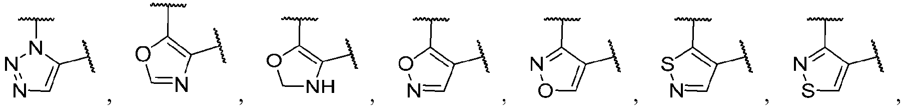 or 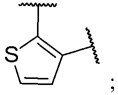 more preferably, 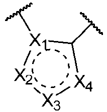 is 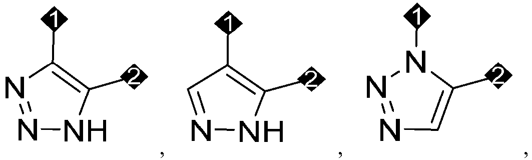 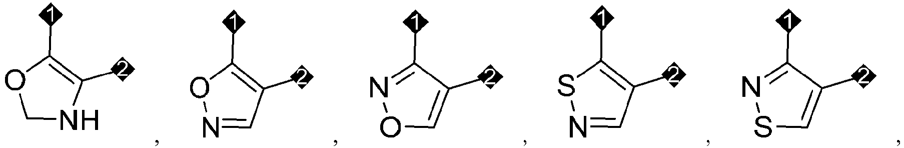 or 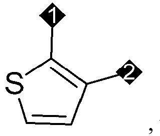 wherein the "1" position is connected to ring B, and the "2" position is connected to W;
(8) each R₃ is independently H, methyl, ethyl, oxo, F, Cl, -CD₃, -CF₃, -CHF₂, or -CH₂F; preferably, each R₃ is independently H, methyl, ethyl, -CD₃, oxo, or Cl; or, each R₃ is independently H, methyl, ethyl, oxo, F, Cl, -CF₃, -CHF₂, or -CH₂F; preferably, each R₃ is independently H, methyl, ethyl, oxo, or Cl;
(9) each R_{c} is independently F, methyl, -CF₃, -CHF₂, methoxy, cyclopropyl, cyclobutyl, cyclopentyl, 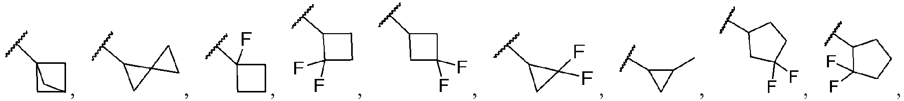 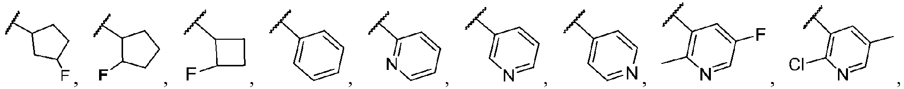 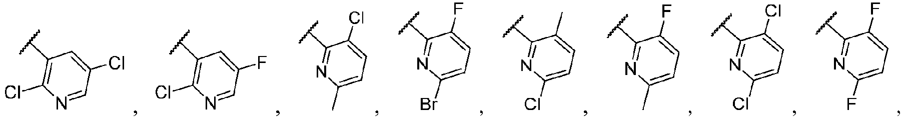 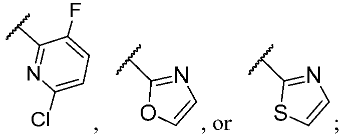
(10) R₁₀ is H, methyl, ethyl, n-propyl, isopropyl, -CD₃, 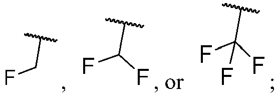 preferably, R₁₀ is H, methyl, -CD₃, 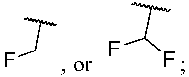 or, R₁₀ is H, methyl, ethyl, n-propyl, isopropyl, 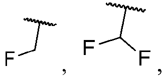 or 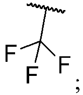 preferably, R₁₀ is H, methyl, 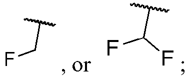
(11) each R_{g} is deuterium, F, Cl, methyl, ethyl, -CH₂F, CHF₂, CF₃, -CD₃, 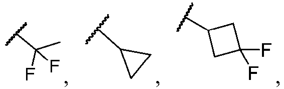 or 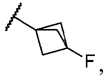 for example, deuterium, F, Cl, -CD₃, 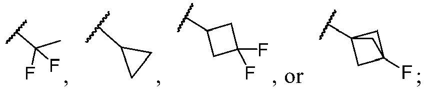 or each R_{g} is F, Cl, methyl, ethyl, -CH₂F, CHF₂, or CF₃, for example, F or Cl;
(12) R₁₁ is 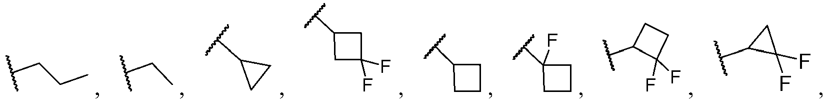 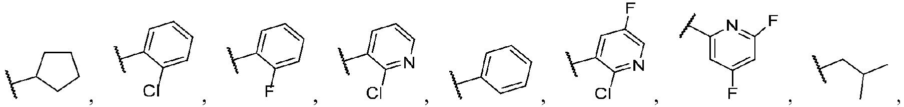 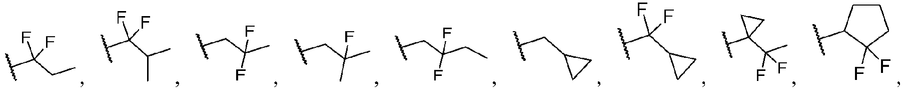 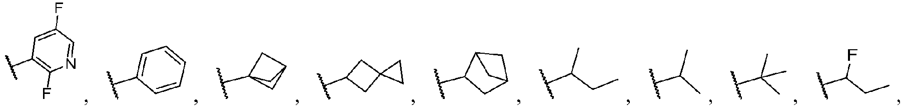 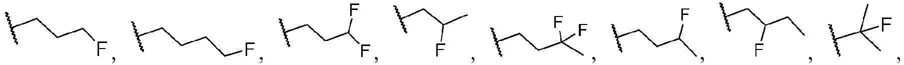 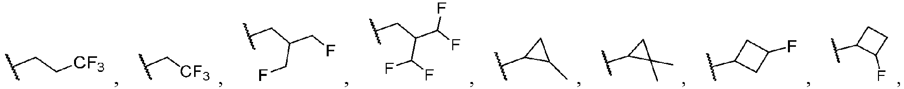 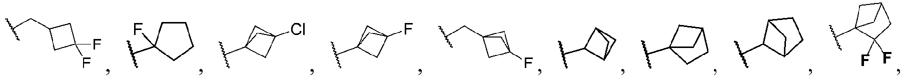 or 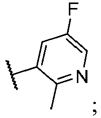 for example, R₁₁ is 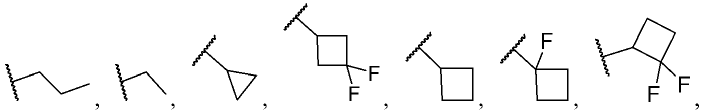 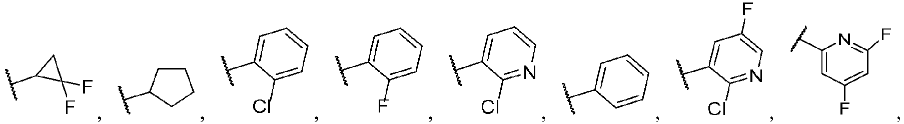 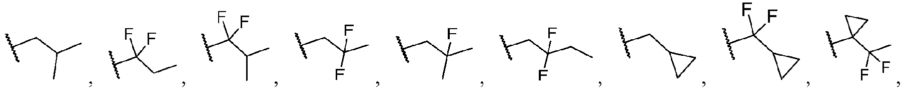 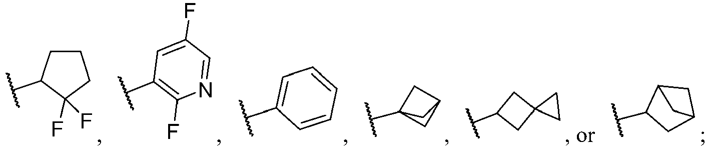
(13) W is -(CR₁₂R₁₃)_{q}-Y₂-R₁₅; for example, W is -N(R₁₄)-R₁₅, -C(R₁₂R₁₃)-R₁₅, -C(R₁₂R₁₃)-N(R₁₄)-R₁₅, - C(R₁₂R₁₃)-O-R₁₅, or -(CR₁₂R₁₃)₂-C(=O)-N(R₁₄)-R₁₅; preferably, W is -NH-R₁₅, -CH₂-R₁₅, -CH₂-NH-R₁₅, - CH₂-O-R₁₅, or 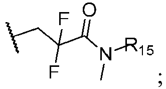 and
(14) each Rᵢ is independently methyl, n-propyl, isopropyl, n-butyl, F, oxo,

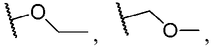

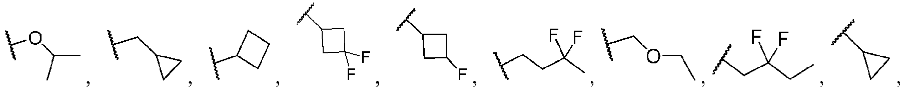

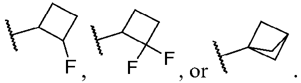

8. The compound of formula (I), the racemate thereof, the stereoisomer thereof, the tautomer thereof, the N-oxide thereof, or the pharmaceutically acceptable salt of any one of the foregoing according to any one of claims 6 to 7, wherein the compound of formula (I) satisfies one or more of the following conditions:
(1) 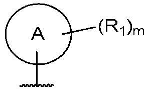 is 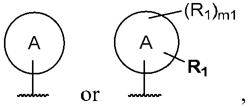 m1 is 0, 1, or 2; for example, 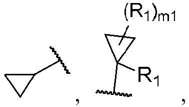 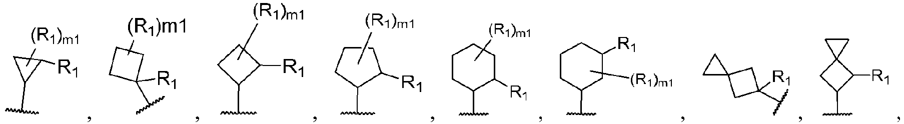 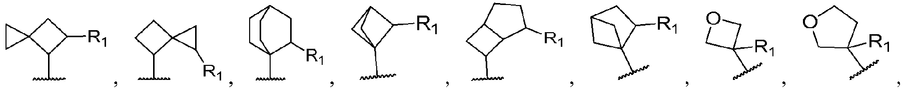 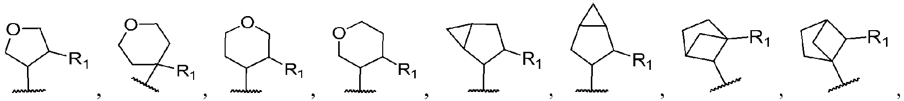 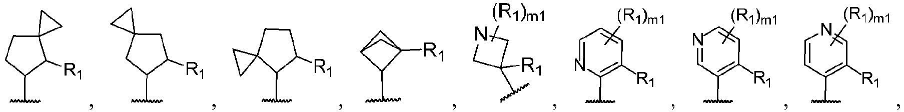 or 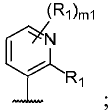 for another example, 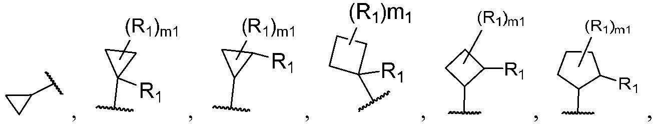 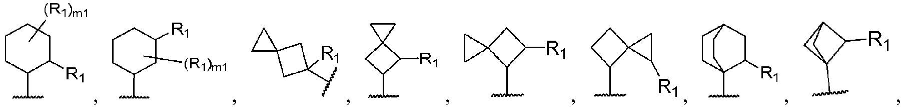 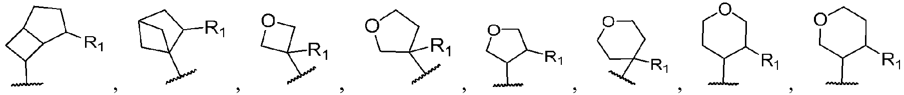 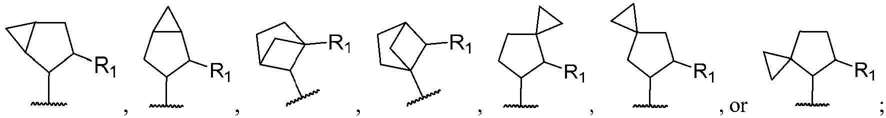 preferably, the 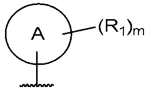 is 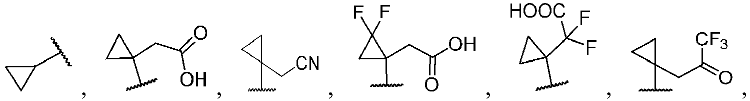 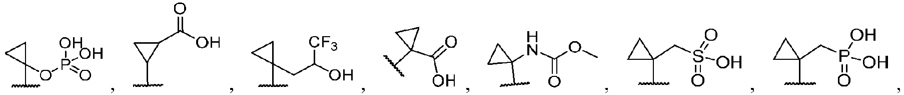 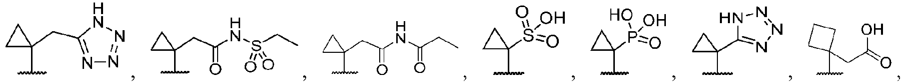 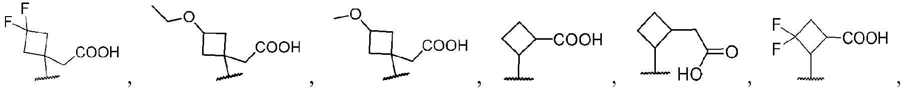 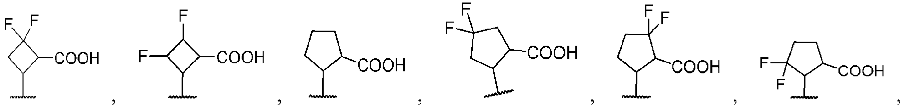 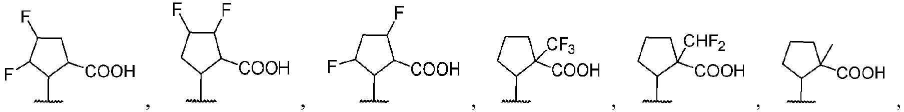 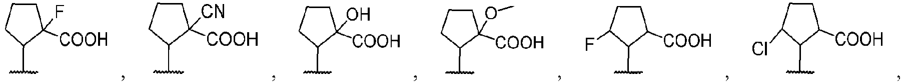 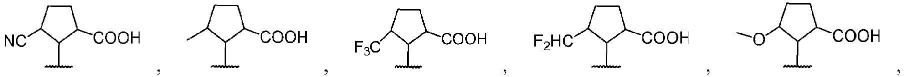 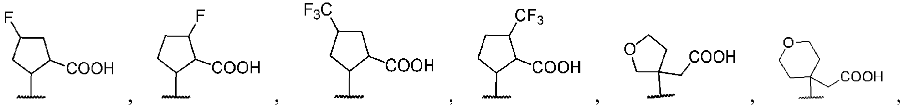 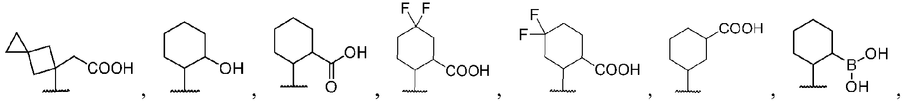 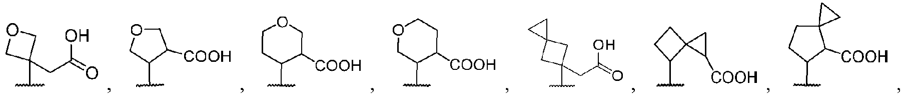 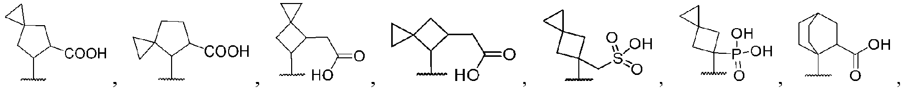 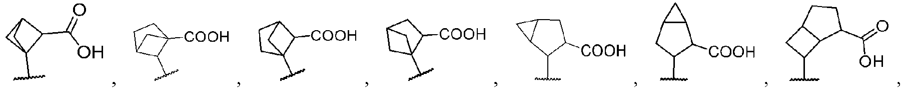 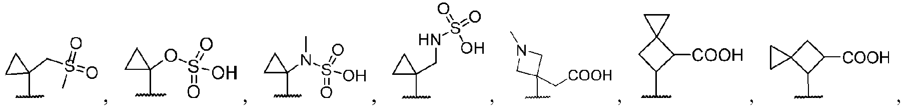 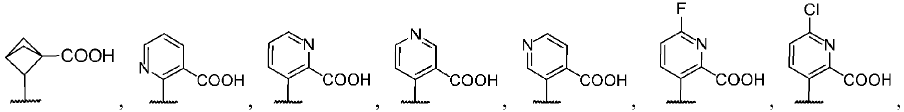 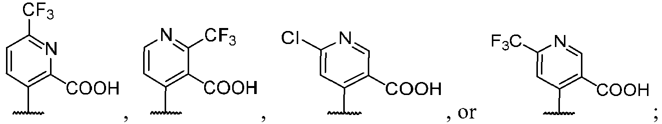
(2) 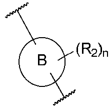 is 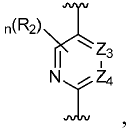 Z₃ and Z₄ are each independently C, CH, or N; preferably, 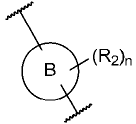 is 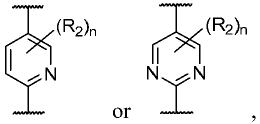 for example, 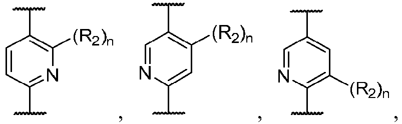 or 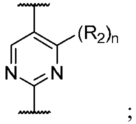 n is 0 or 1; for another example, 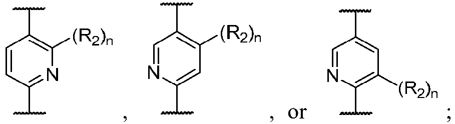 or, 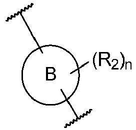 is 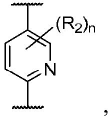 for example 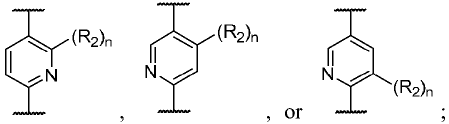 preferably, 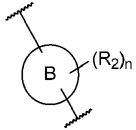 is 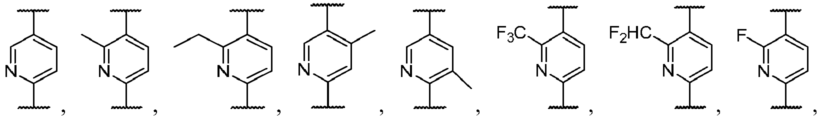 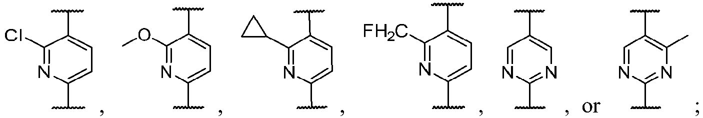 preferably, 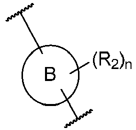 is 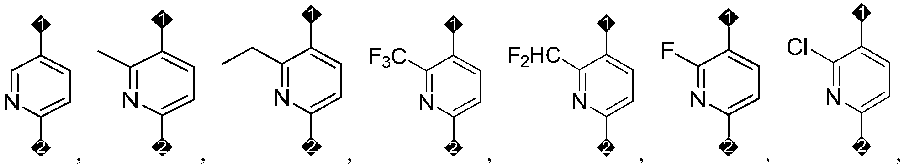 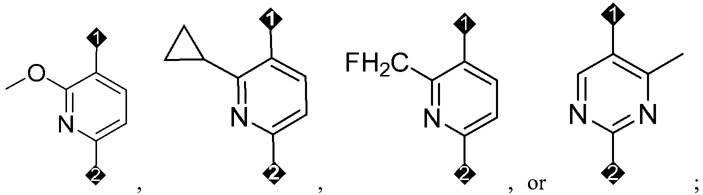 wherein the "1" position is connected to L₁, and the "2" position is connected to X₁ in 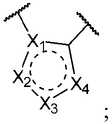
(3) 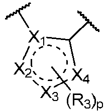 is 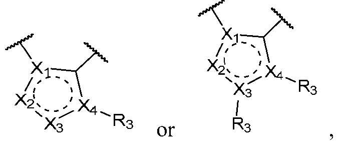 preferably, 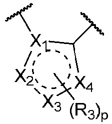 is 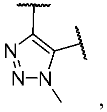 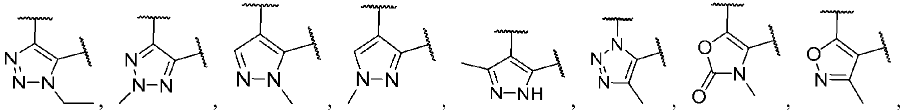 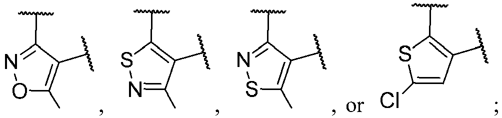 more preferably, 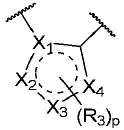 is 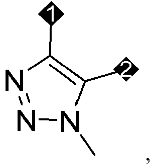 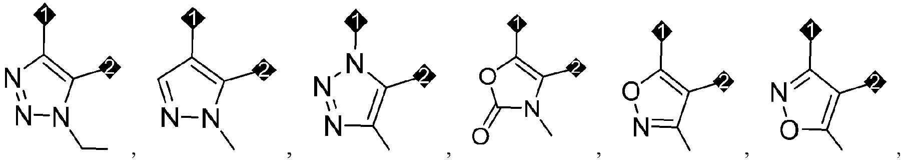 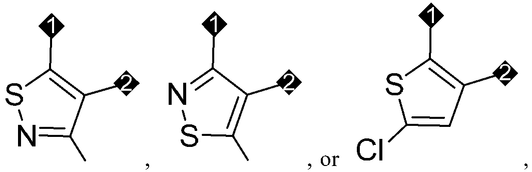 wherein the "1" position is connected to ring B, and the "2" position is connected to W;
(4) R₆ is H, methyl, ethyl, 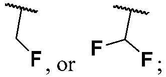
(5) R₇ is 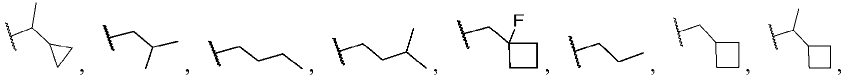 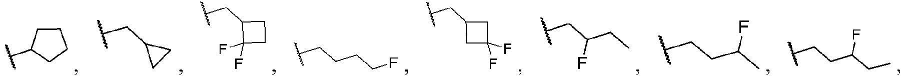 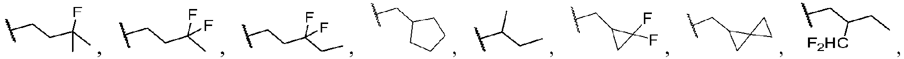 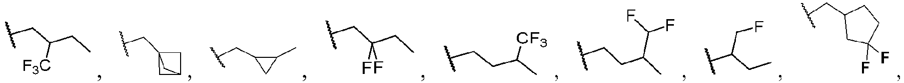 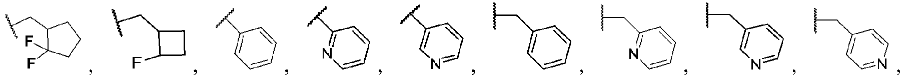 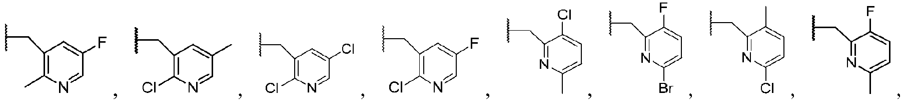 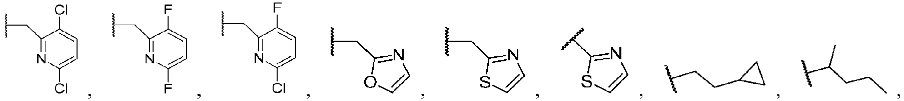 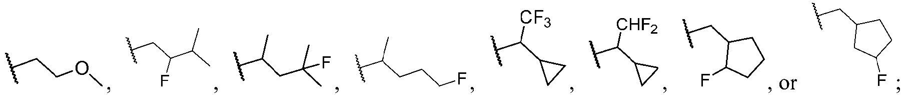
(6) 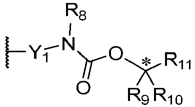 is 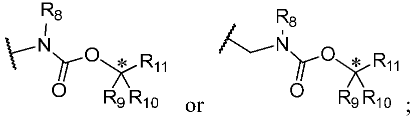
(7) 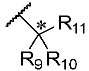 in 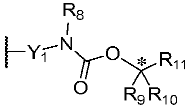 is 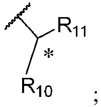 when the carbon atom marked with * is a chiral carbon atom, 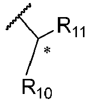 is 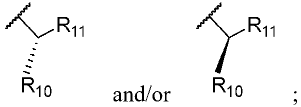 preferably, 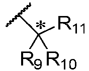 is 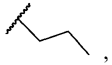 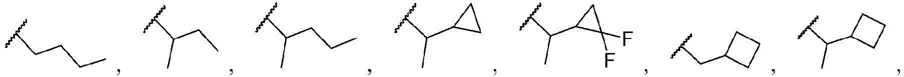 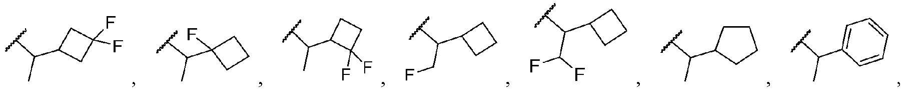 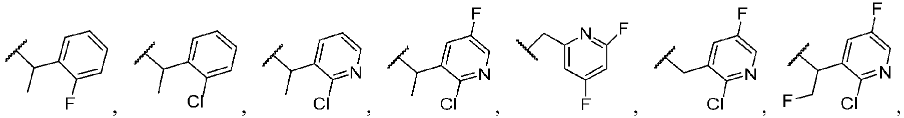 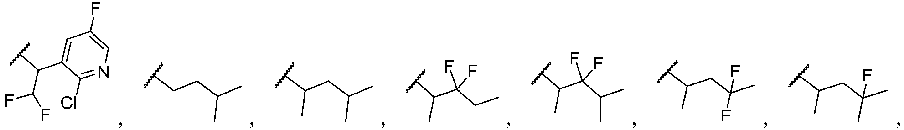 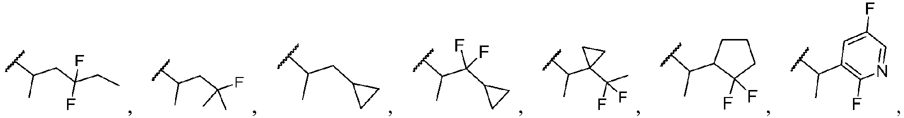 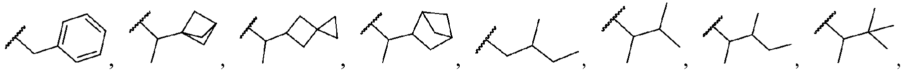 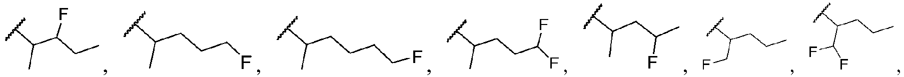 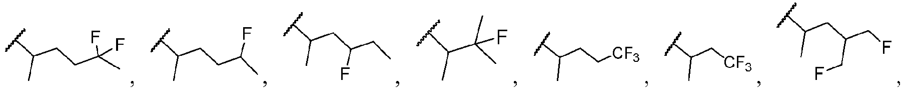 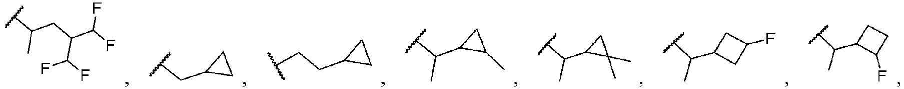 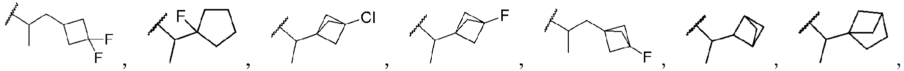 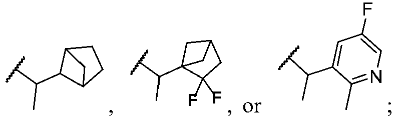 for example, 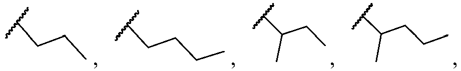 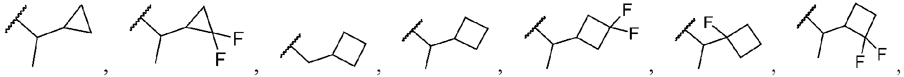 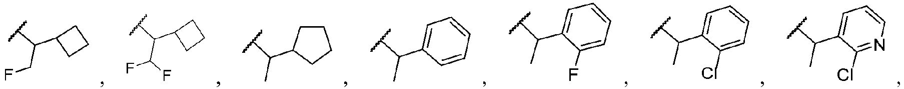 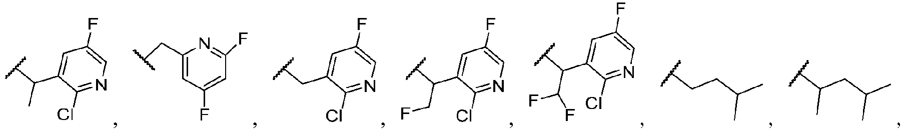 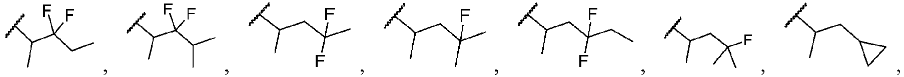 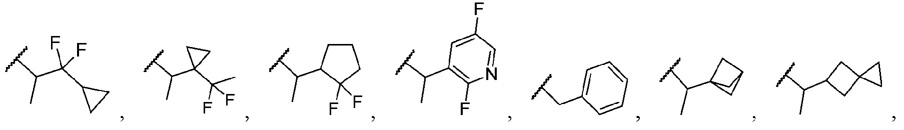 or 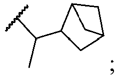 more preferably, 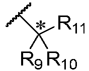 is 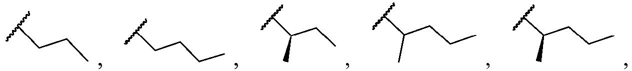 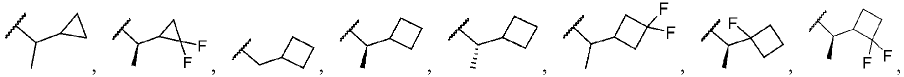 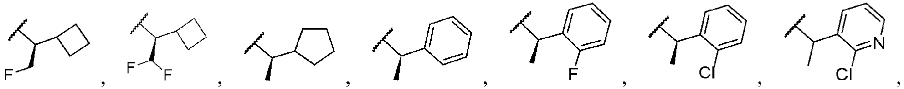 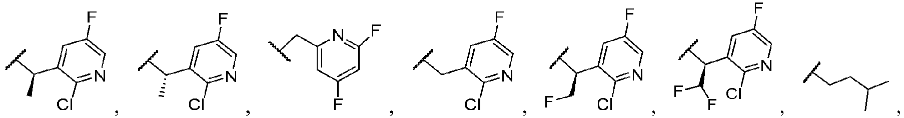 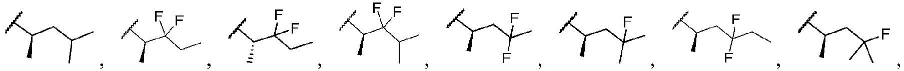 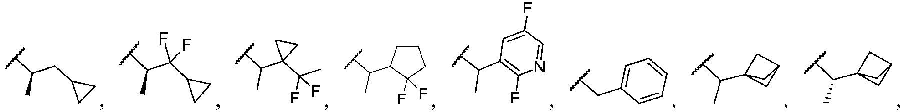 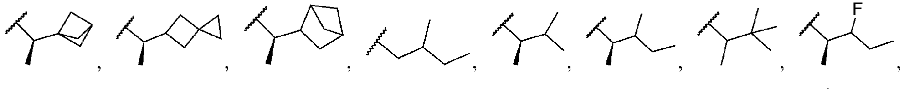 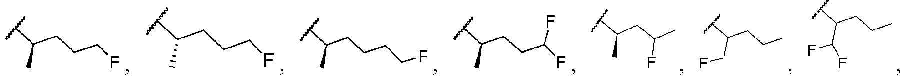 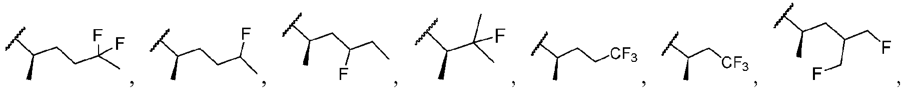 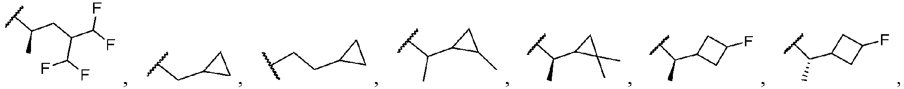 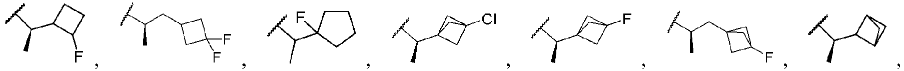 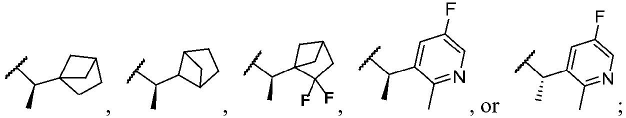 and
(8) R₁₅ is 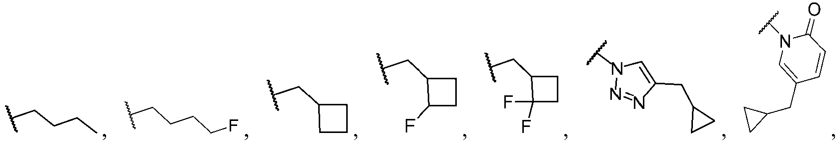 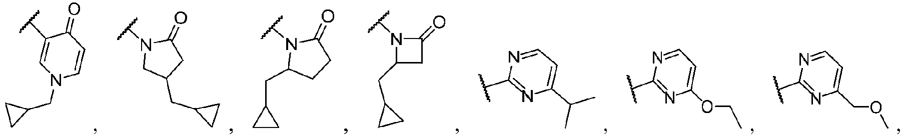 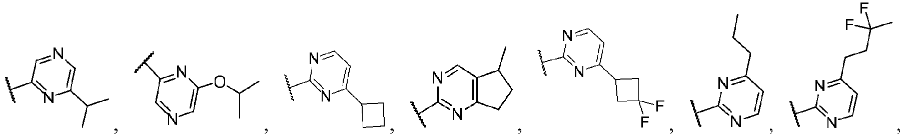 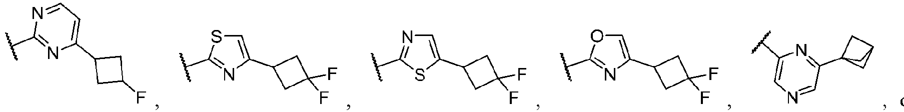 or

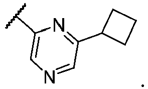

9. The compound of formula (I), the racemate thereof, the stereoisomer thereof, the tautomer thereof, the N-oxide thereof, or the pharmaceutically acceptable salt of any one of the foregoing according to claim 8, wherein the compound of formula (I) satisfies one or two of the following conditions:
(1) 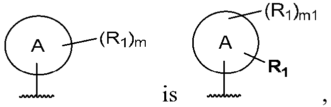 m1 is 0, 1, or 2; for example, 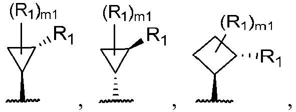 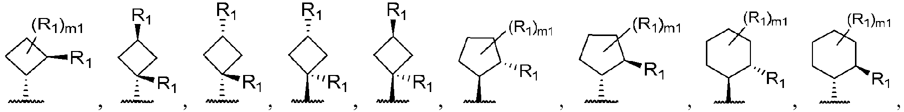 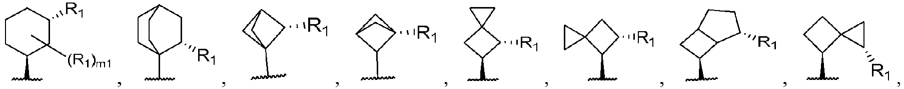 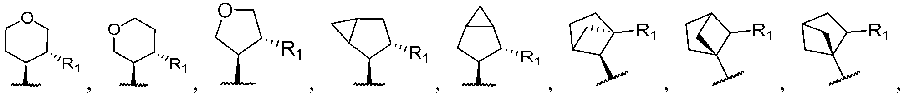 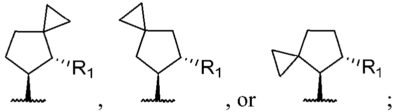 preferably, 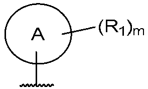 is 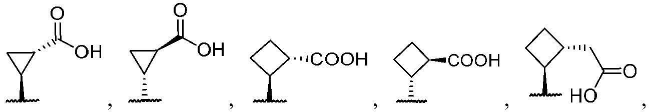 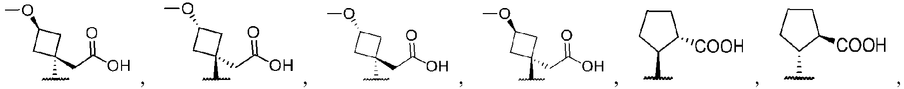 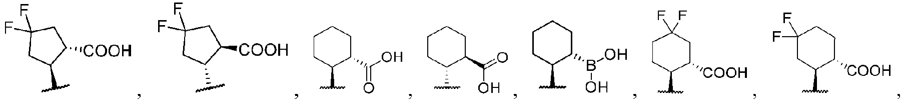 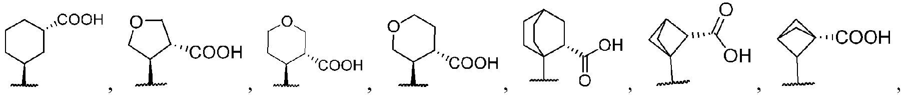 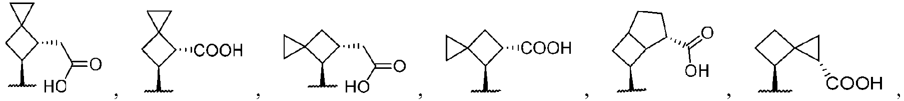 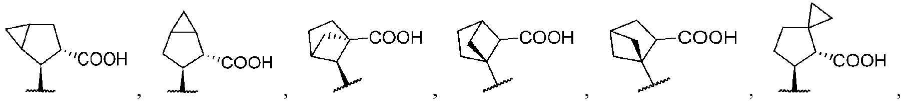 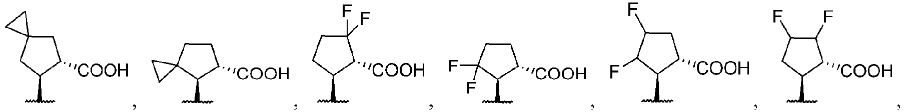 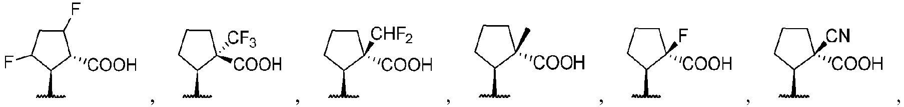 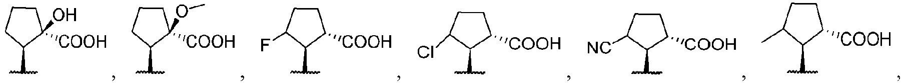 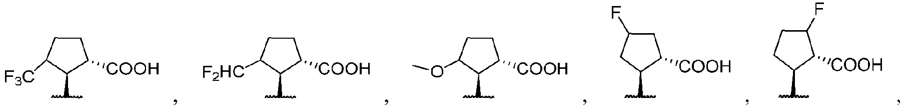 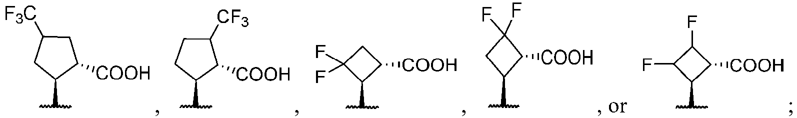 and
(2) 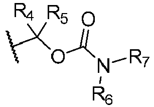 is 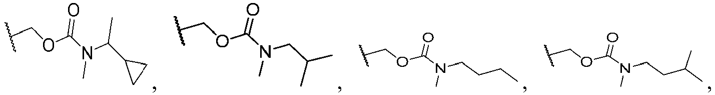 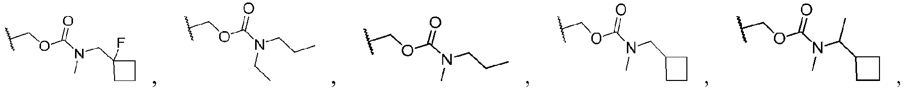 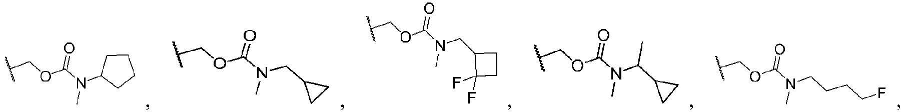 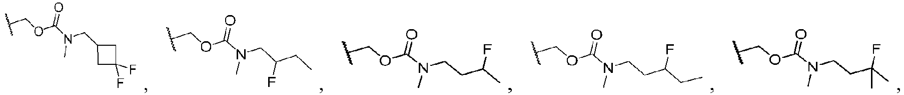 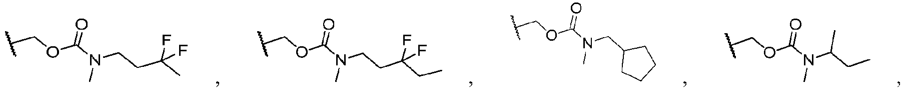 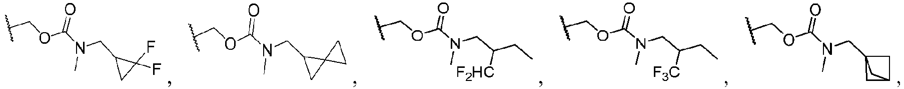 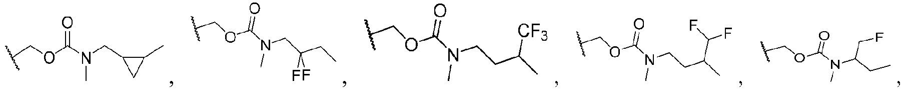 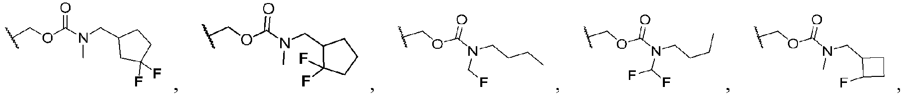 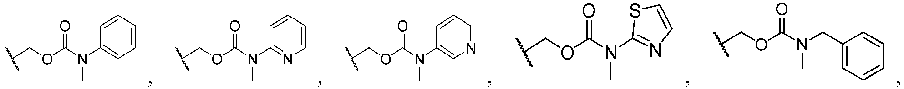 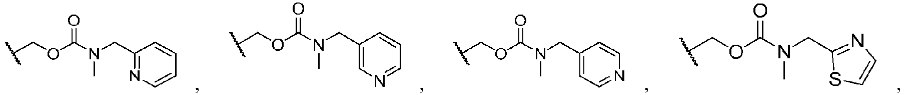 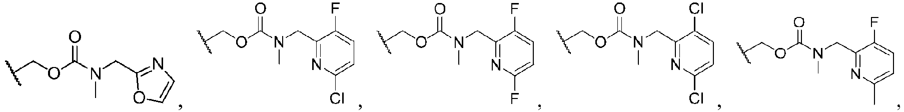 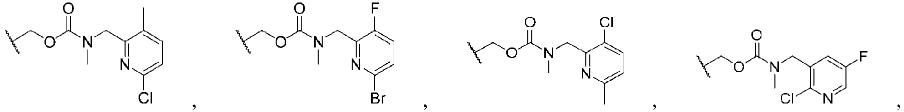 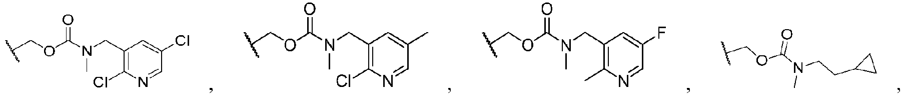 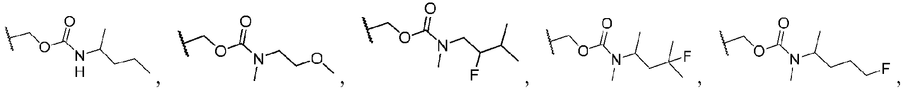 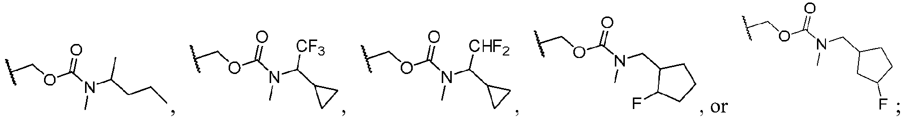 preferably, 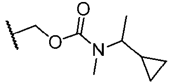 is 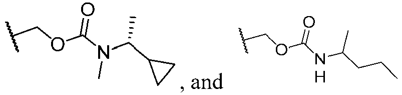 is

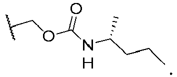

10. The compound of formula (I), the racemate thereof, the stereoisomer thereof, the tautomer thereof, the N-oxide thereof, or the pharmaceutically acceptable salt of any one of the foregoing according to claim 1, wherein 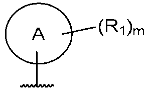 is 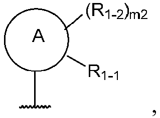 R₁₋₁ and R₁₋₂ are each independently defined as R₁ in any one of claims 1 to 9, and m2 is defined as m in any one of claims 1 to 9 or defined as m1 in any one of claims 8 to 9;
preferably, each R₁₋₂ is independently halogen, C₁₋₆ alkyl, C₁₋₆ alkoxy, OH, or CN; the C₁₋₆ alkyl and C₁₋₆ alkoxy are each independently optionally substituted with 1, 2, or more Rₐ substituents; for example, each R₁₋₂ is independently halogen, C₁₋₄ alkyl, C₁₋₄ alkoxy, OH, or CN; the C₁₋₄ alkyl and C₁₋₄ alkoxy are each independently optionally substituted with 1, 2, or more Rₐ substituents;
more preferably, in case (1), ring B is 6-membered heteroarylene; R₁₋₁ is OH, COOH, C₁₋₆ alkyl, 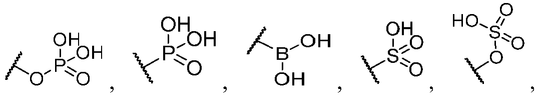 -C₁₋₃ alkylene-COOH, NHC(=O)OC₁₋₆ alkyl, - NHS(=O)₂OH, -C₁₋₃ alkylene-NHS(=O)₂OH, -C₁₋₃ alkylene-S(=O)₂OH, or -C₁₋₃ alkylene-S(=O)₂C₁₋₆ alkyl; the C₁₋₆ alkyl, -C₁₋₃ alkylene-COOH, NHC(=O)OC₁₋₆ alkyl, -NHS(=O)₂OH, -C₁₋₃ alkylene-NHS(=O)₂OH, - C₁₋₃ alkylene-S(=O)₂OH, and -C₁₋₃ alkylene-S(=O)₂C₁₋₆ alkyl are each independently optionally substituted with 1, 2, or more Rₐ substituents;
or, R₁₋₁ is COOH, 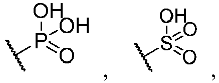 tetrazolyl, -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, or -C₁₋₃ alkylene-tetrazolyl; the -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, and -C₁₋₃ alkylene-tetrazolyl are each independently optionally substituted with 1, 2, or more Rₐ substituents;
in R₁₋₁, each Rₐ is independently C₁₋₄ alkyl, halogen, OH, oxo, or CN; when the number of Rₐ is 2 or more, the OH and oxo are not attached to the same carbon atom;
each R₁₋₂ is independently halogen, C₁₋₄ alkyl, C₁₋₄ alkoxy, OH, or CN; the C₁₋₄ alkyl and C₁₋₄ alkoxy are each independently optionally substituted with 1, 2, or more Rₐ substituents;
in R₁₋₂, each Rₐ is independently halogen.

11. The compound of formula (I), the racemate thereof, the stereoisomer thereof, the tautomer thereof, the N-oxide thereof, or the pharmaceutically acceptable salt of any one of the foregoing according to claim 1, wherein the compound of formula (I) is any one of the following schemes: 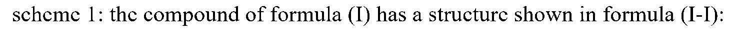 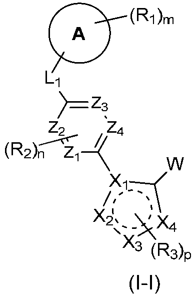
wherein Z₁, Z₂, Z₃, and Z₄ are each independently N or CH; simultaneously, at least one of Z₁, Z₂, Z₃, or Z₄ is N; n is 0, 1, or 2; ring A, L₁, R₁, R₂, R₃, W, X₁, X₂, X₃, X₄, m, p, and 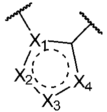 are as defined in any one of claims 1 to 10; 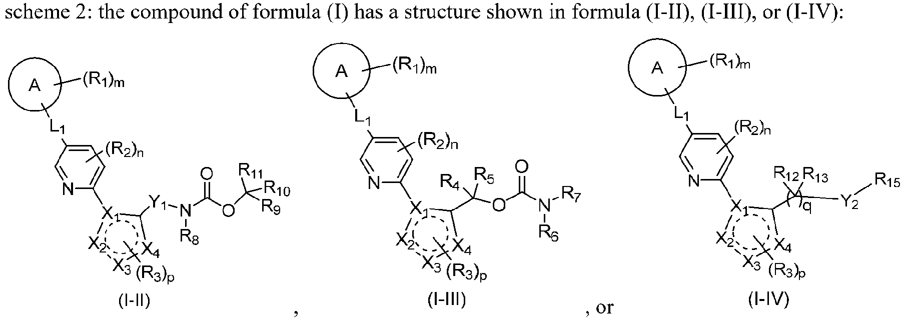
wherein L₁ is 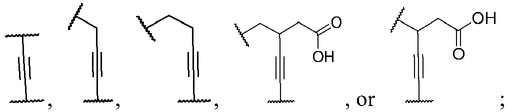
ring A, Y₁, Y₂, R₁, R₂, R₃, R₄, R₅, R₆, R₇, R₈, R₉, R₁₀, R₁₁, R₁₂, R₁₃, R₁₅, X₁, X₂, X₃, X₄, m, n, p, q, and 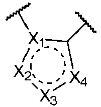 are as defined in any one of claims 1 to 10; 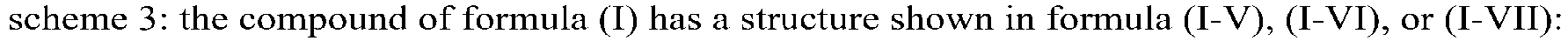 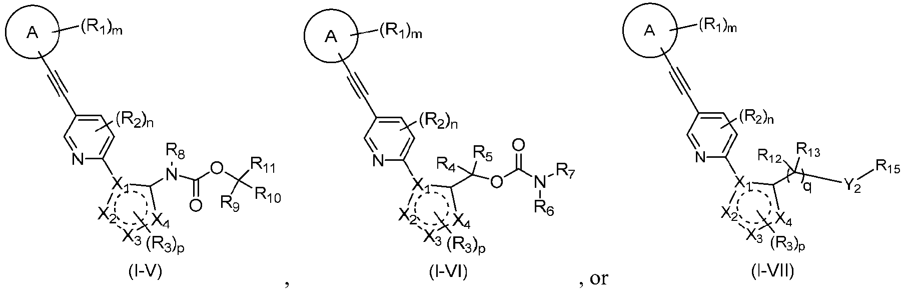
ring A, Y₂, R₁, R₂, R₃, R₄, R₅, R₆, R₇, R₈, R₉, R₁₀, R₁₁, R₁₂, R₁₃, R₁₅, X₁, X₂, X₃, X₄, m, n, p, q, and 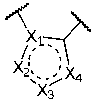 are as defined in any one of claims 1 to 10; 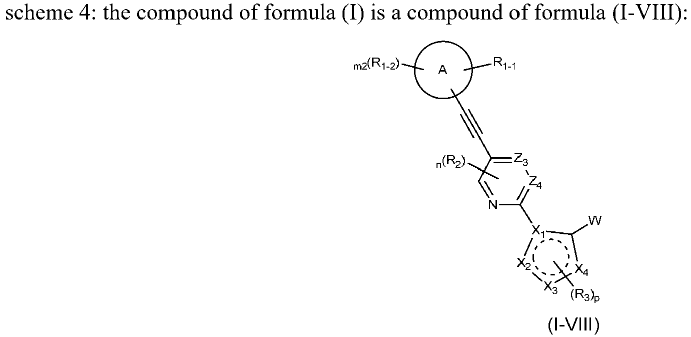
wherein R₁₋₁ is COOH, 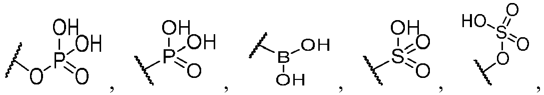
tetrazolyl, -C₁₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(=O)NHC₁₋₆ alkyl, -C(=O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, -C(=O)NHS(=O)C₁₋₆ alkyl, - C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, - S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, or -C₁₋₃ alkylene-tetrazolyl;
the -C₁₋₃ alkylene-COOH, -NHC(=O)OC₁₋₆ alkyl, -NHC(=O)C₁₋₆ alkyl, -NHS(=O)₂OH, -C₁₋₃ alkylene-NHS(=O)₂OH, -C(-O)NHC₁₋₆ alkyl, -C(-O)OC₁₋₆ alkyl, -C(=O)NHS(=O)₂C₁₋₆ alkyl, -C(=O)NHC(=O)C₁₋₆ alkyl, -C₁₋₃ alkylene-C(=O)NHC(=O)C₁₋₆ alkyl, -C₁₋₃ alkylene-C(=O)NHS(=O)₂C₁₋₆ alkyl, - C(=O)NHS(=O)C₁₋₆ alkyl, -C₁₋₃ alkylene-C(=O)NHS(=O)C₁₋₆ alkyl, -C₁₋₃ alkylene-S(=O)₂OH, -C₁₋₃ alkylene-S(=O)₂C₁₋₆ alkyl, -S(=O)₂NHC(=O)C₁₋₆ alkyl, -C₁₋₃ alkylene-S(=O)₂NHC(=O)C₁₋₆ alkyl, -C₁₋₃ alkylene-P(=O)(OH)₂, and -C₁₋₃ alkylene-tetrazolyl are each independently optionally substituted with **1, 2,** or more Rₐ substituents;
each R₁₋₂ is independently OH, CN, oxo (=O), halogen, NH₂, C₁₋₄ alkyl, or C₁₋₄ alkoxy; the C₁₋₄ alkyl is optionally substituted with 1, 2, or more halogen substituents;
each Rₐ is independently C₁₋₄ alkyl, C₁₋₄ alkoxy, or halogen;
Z₃ and Z₄ are each independently N or CH;
m2 is 0, 1, 2, or 3;
ring A, R₂, R₃, W, X₁, X₂, X₃, X₄, n, p, and 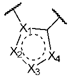 are as defined in any one of claims 1 to 10.

12. The compound of formula (I), the racemate thereof, the stereoisomer thereof, the tautomer thereof, the N-oxide thereof, or the pharmaceutically acceptable salt of any one of the foregoing according to claim 10, wherein the compound of formula (I) is any one of the following schemes: 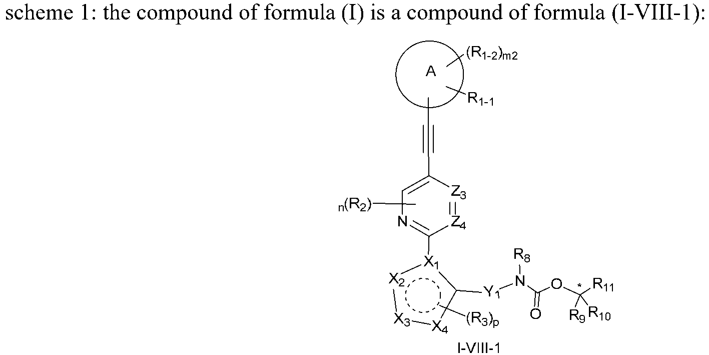
the carbon atom marked with * is a chiral carbon atom or an achiral carbon atom; when the carbon atom is a chiral carbon atom, the configuration of the chiral carbon atom is R and/or S;
ring A is C₃₋₁₀ cycloalkyl, 3- to 10-membered heterocycloalkyl, or 5- to 10-membered heteroaryl;
m2 is 0, 1, or 2;
R₁₋₁ is OH, COOH, C₁₋₆ alkyl, 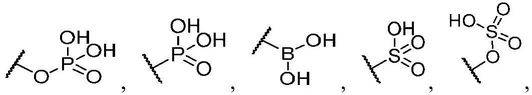 -C₁₋₃ alkylene COOH, -NHC(=O)OC₁₋₆ alkyl, -NHS(=O)₂OH, -C₁₋₃ alkylene-NHS(=O)₂OH, -C₁₋₃ alkylene-S(=O)₂OH, or - C₁₋₃ alkylene-S(=O)₂C₁₋₆ alkyl; the C₁₋₆ alkyl, -C₁₋₃ alkylene-COOH, NHC(=O)OC₁₋₆ alkyl, -NHS(=O)₂OH, - C₁₋₃ alkylene-NHS(=O)₂OH, -C₁₋₃ alkylene-S(=O)₂OH, and -C₁₋₃ alkylene-S(=O)₂C₁₋₆ alkyl are each independently optionally substituted with 1, 2, or more Rₐ substituents;
in R₁₋₁, each Rₐ is independently C₁₋₄ alkyl, halogen, OH, oxo, or CN; when the number of Rₐ is 2 or more, the OH and oxo are not attached to the same carbon atom;
each R₁₋₂ is independently halogen, C₁₋₆ alkyl, C₁₋₆ alkoxy, OH, or CN; the C₁₋₆ alkyl and C₁₋₆ alkoxy are each independently optionally substituted with 1, 2, or more Rₐ substituents;
in R₁₋₂, each Rₐ is independently halogen;
Z₃ and Z₄ are each independently C, CH, or N;
each R₂ is independently halogen, C₁₋₄ alkyl, or C₃₋₆ cycloalkyl; the C₁₋₄ alkyl and C₃₋₆ cycloalkyl are each independently optionally substituted with 1, 2, or more halogen or deuterium substituents;
n is 0 or 1;
X₁ is C or N;
X₂ is CH, O, S, or N;
X₃ is C, CH₂, O, S, or N;
X₄ is C, CH, N, or NH;
and at least one of X₁, X₂, X₃, or X₄ is independently O, S, or N;
the double line comprising solid and dashed lines represents a single bond or a double bond; simultaneously, 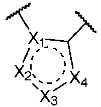 contains at least one double bond;
each R₃ is independently halogen, C₁₋₄ alkyl, or oxo;
p is 1 or 2;
Y₁ is a chemical bond or C₁₋₆ alkylene;
R₈ is H or deuterium;
R₉ is H or deuterium;
R₁₀ is H, deuterium, or C₁₋₆ alkyl; the C₁₋₆ alkyl is optionally substituted with 1, 2, 3, or 4 R_{f} substituents; each R_{f} is independently deuterium or halogen;
R₁₁ is C₁₋₆ alkyl, C₆₋₁₀ aryl, 5- to 10-membered heteroaryl, or C₃₋₈ cycloalkyl; the C₁₋₆ alkyl, C₆₋₁₀ aryl, 5-to 10-membered heteroaryl, and C₃₋₈ cycloalkyl are each independently optionally substituted with 1, 2, or more R_{g} substituents;
each R_{g} is independently deuterium, halogen, C₁₋₆ alkyl, or C₃₋₆ cycloalkyl; the C₁₋₆ alkyl and C₃₋₆ cycloalkyl are each independently optionally substituted with 1, 2, or more deuterium or halogen substituents;
the heteroatoms in the 3- to 10-membered heterocycloalkyl and 5- to 10-membered heteroaryl are each independently selected from 1, 2, or 3 types of N, O, and S, and the number of heteroatoms is independently 1, 2, or 3; 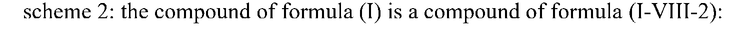 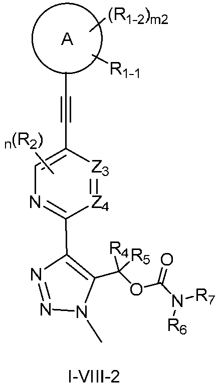
ring A is C₃₋₈ cycloalkyl, 4- to 6-membered oxacycloalkyl, or 4- to 6-membered azacycloalkyl; the number of heteroatoms in the 4- to 6-membered oxacycloalkyl and 4- to 6-membered azacycloalkyl is 1;
m2 is 0, 1, or 2;
R₁₋₁ is COOH, 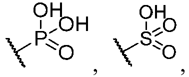 tetrazolyl, -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, or - C₁₋₃ alkylene-tetrazolyl; the -C₁₋₃ alkylene-COOH, -C₁₋₃ alkylene-C(=O)NHC(=O)C₁₋₆ alkyl, -C₁₋₃ alkylene-C(=O)NHS(=O)₂C₁₋₆ alkyl, -C₁₋₃ alkylene-S(=O)₂OH, -C₁₋₃ alkylene-P(=O)(OH)₂, and -C₁₋₃ alkylene-tetrazolyl are each independently optionally substituted with 1, 2, or more Rₐ substituents;
each R₁₋₂ is independently halogen, C₁₋₆ alkyl, C₁₋₆ alkoxy, OH, or CN; the C₁₋₆ alkyl and C₁₋₆ alkoxy are each independently optionally substituted with 1, 2, or more Rₐ substituents;
each Rₐ is independently halogen;
Z₃ and Z₄ are each independently C, CH, or N;
each R₂ is independently halogen, C₁₋₄ alkyl, C₁₋₄ alkoxy, or C₃₋₆ cycloalkyl; the C₁₋₄ alkyl, C₁₋₄ alkoxy, and C₃₋₆ cycloalkyl are each independently optionally substituted with 1, 2, or more halogen or deuterium substituents;
n is 0 or 1;
R₄ and R₅ are each independently H or deuterium;
R₆ is C₁₋₆ alkyl; the C₁₋₆ alkyl is optionally substituted with 1, 2, or more R_{c} substituents;
in R₆, each R_{c} is independently halogen;
R₇ is C₁₋₆ alkyl, C₃₋₈ cycloalkyl, C₆₋₁₀ aryl, or 5- to 10-membered heteroaryl; the C₁₋₆ alkyl is optionally substituted with 1, 2, or more R_{c} substituents; the C₃₋₈ cycloalkyl, C₆₋₁₀ aryl, and 5- to 10-membered heteroaryl are each independently optionally substituted with 1, 2, or more Rₑ substituents;
in R₇, each R_{c} is independently deuterium, halogen, C₁₋₆ alkyl, C₃₋₈ cycloalkyl, C₆₋₁₀ aryl, or 5- to 10-membered heteroaryl; the C₁₋₆ alkyl, C₃₋₈ cycloalkyl, C₆₋₁₀ aryl, and 5- to 10-membered heteroaryl are each independently optionally substituted with 1, 2, or more R_{c-1} substituents;
each R_{c-1} is independently deuterium, halogen, or C₁₋₆ alkyl;
each Rₑ is independently deuterium or halogen;
the heteroatoms in the 5- to 10-membered heteroaryl are each independently selected from N, O, and S, and the number of heteroatoms is independently 1, 2, or 3; 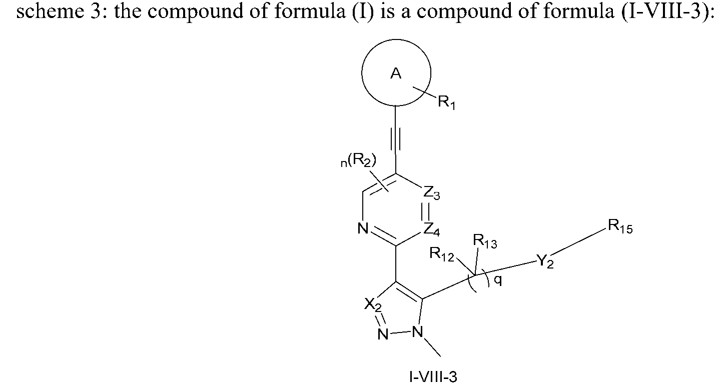
ring A is C₃₋₈ cycloalkyl;
R₁ is COOH or -C₁₋₃ alkylene-COOH;
each R₂ is independently C₁₋₄ alkyl;
n is 0 or 1;
Z₃ and Z₄ are each independently C, CH, or N;
X₂ is CH or N;
R₁₂ and R₁₃ are each independently H, deuterium, or halogen;
when q is 0, Y₂ is N(R₁₄), -C(=O)N(R₁₄)-, or O;
when q is 1, 2, or 3, Y₂ is a chemical bond, N(R₁₄), -C(=O)N(R₁₄)-, or O;
R₁₄ is H, deuterium, or C₁₋₆ alkyl; R₁₅ is C₁₋₆ alkyl, 3- to 10-membered heterocyclyl, or 5- to 10-membered heteroaryl; the C₁₋₆ alkyl, 3- to 10-membered heterocyclyl, and 5- to 10-membered heteroaryl are each independently optionally substituted with 1, 2, or more Rᵢ substituents;
each Rᵢ is independently halogen, oxo, C₃₋₆ cycloalkyl, C₁₋₆ alkyl, or C₁₋₆ alkoxy; the C₁₋₆ alkyl, C₃₋₆ cycloalkyl, and C₁₋₆ alkoxy are each independently optionally substituted with 1, 2, or more Rᵢ₋₁ substituents;
each Rᵢ₋₁ is independently halogen, C₁₋₆ alkyl, C₃₋₆ cycloalkyl, or C₁₋₆ alkoxy;
the heteroatoms in the 3- to 10-membered heterocycloalkyl and 5- to 10-membered heteroaryl are each independently selected from 1, 2, or 3 types of N, O, and S, and the number of heteroatoms is independently 1, 2, or 3.

13. The compound of formula (I), the racemate thereof, the stereoisomer thereof, the tautomer thereof, the N-oxide thereof, or the pharmaceutically acceptable salt of any one of the foregoing according to claim 1, wherein the compound of formula (I) is any one of the following compounds: 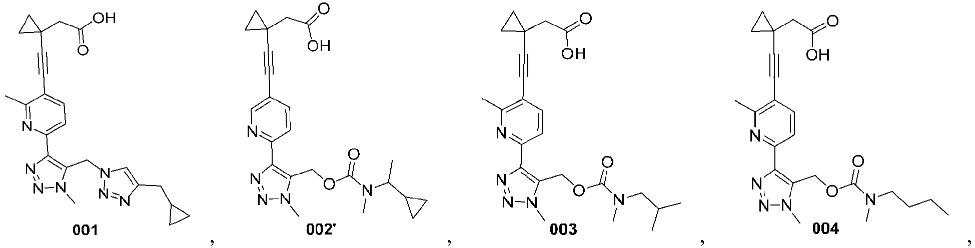 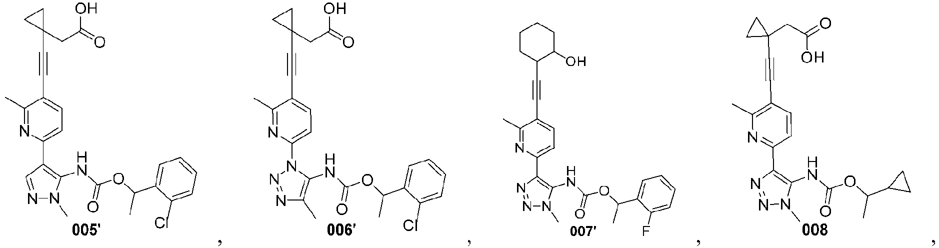 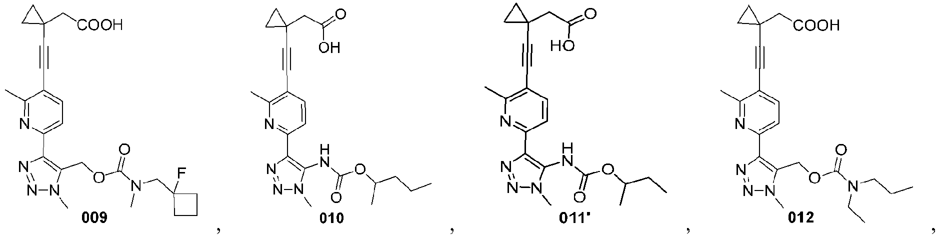 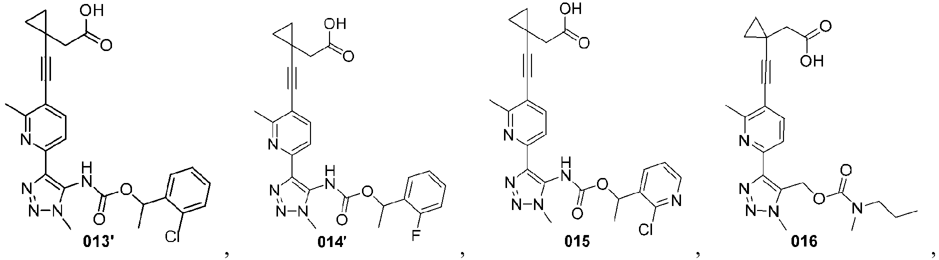 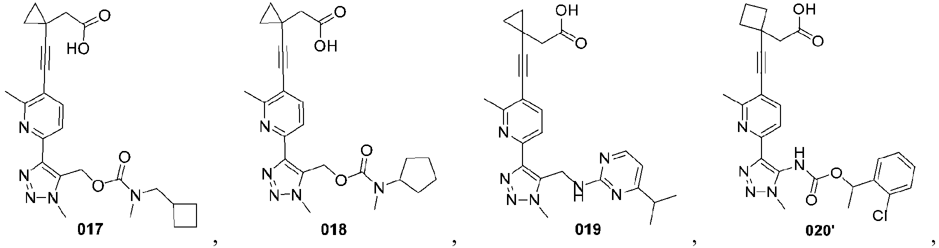 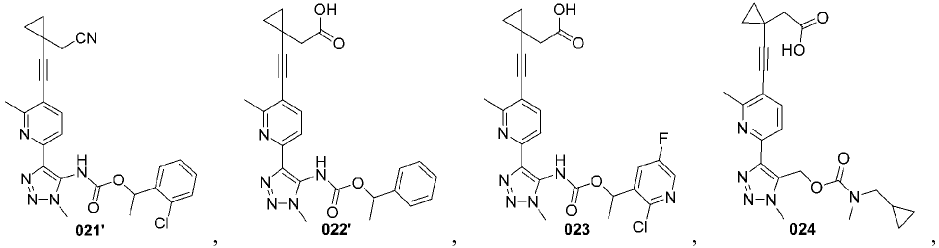 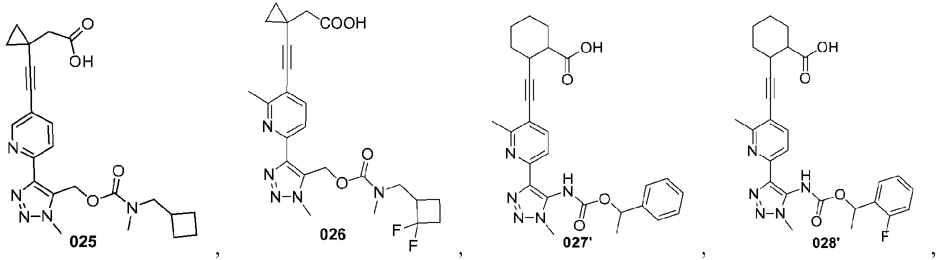 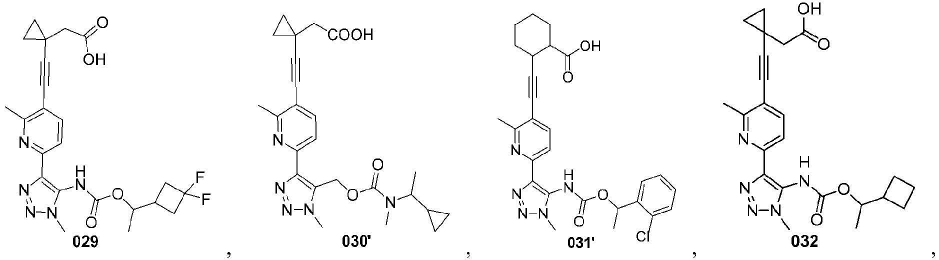 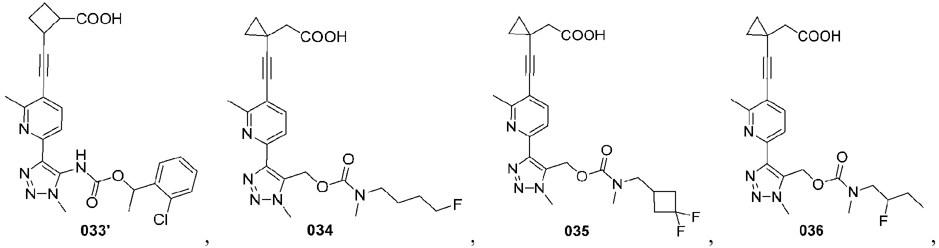 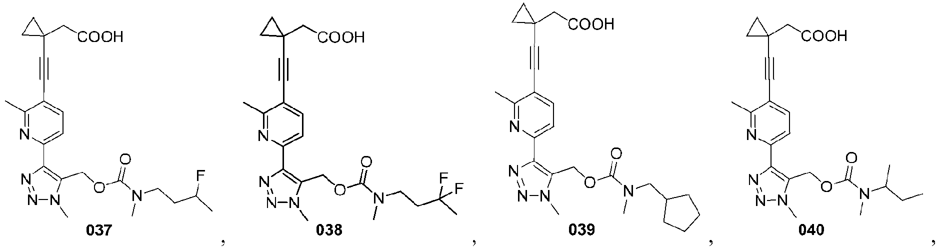 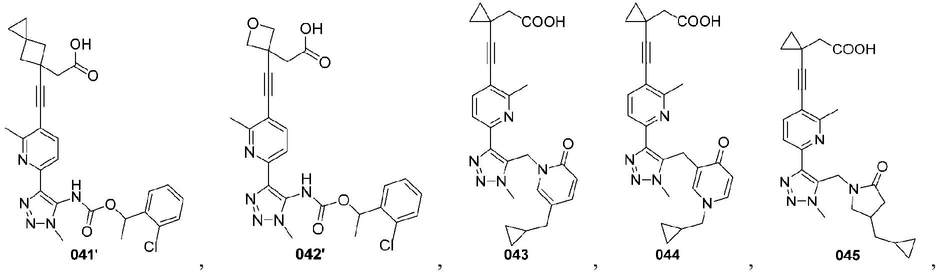 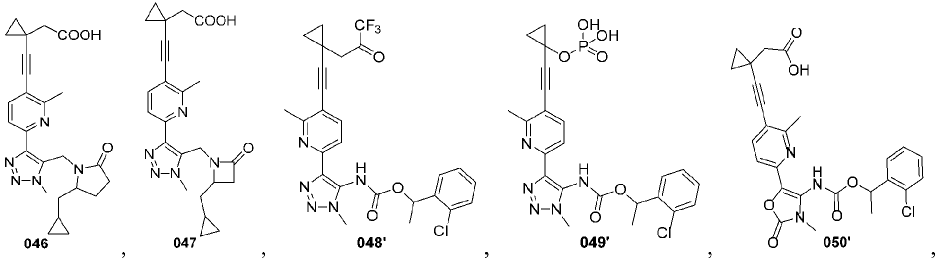 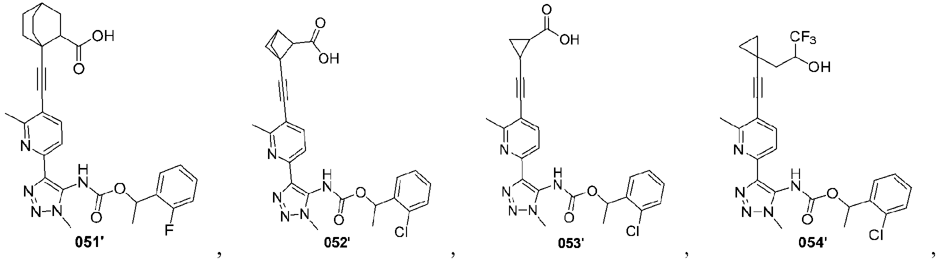 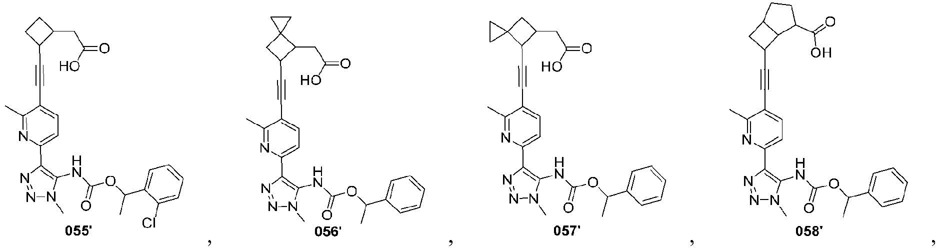 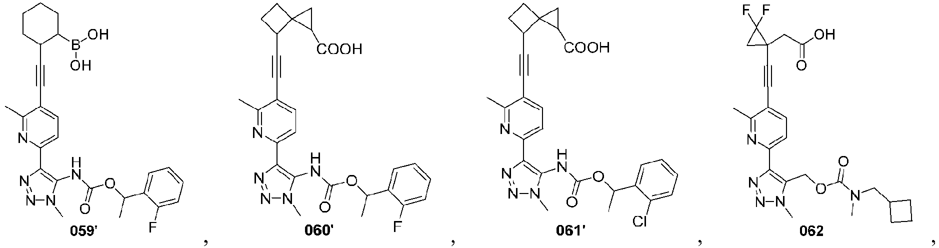 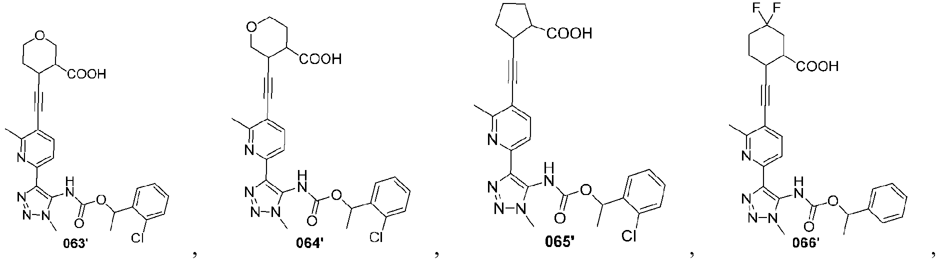 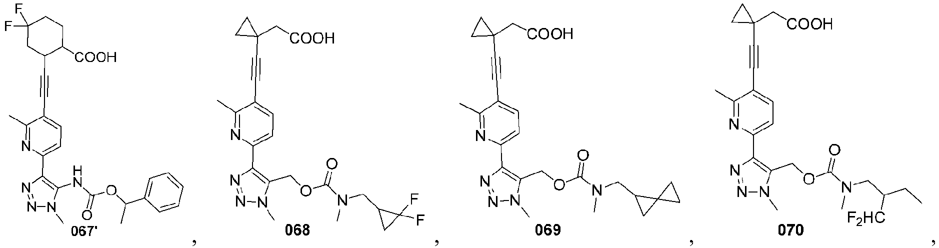 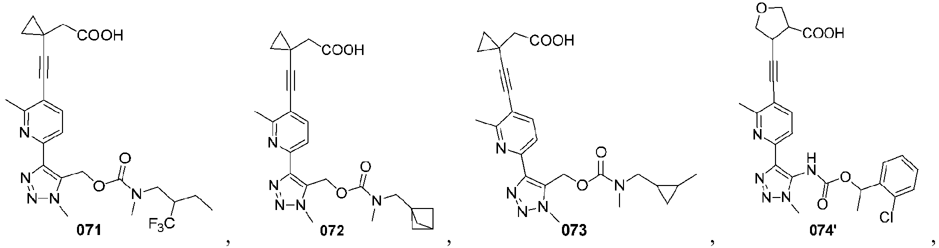 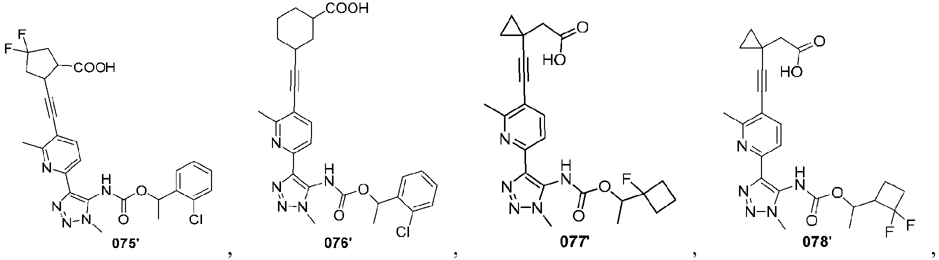 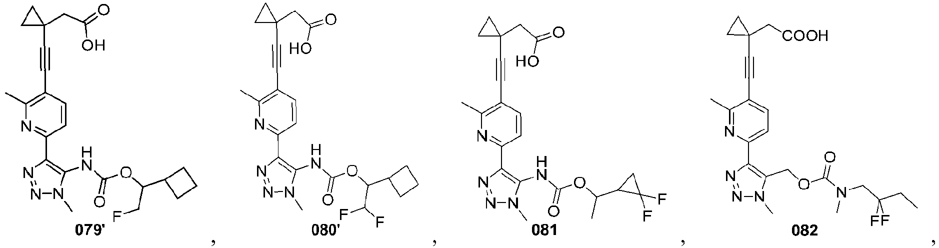 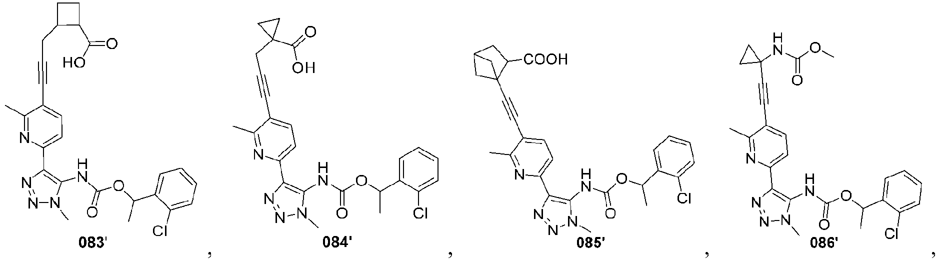 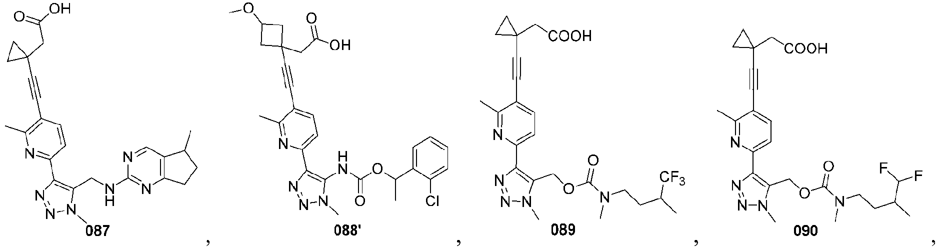 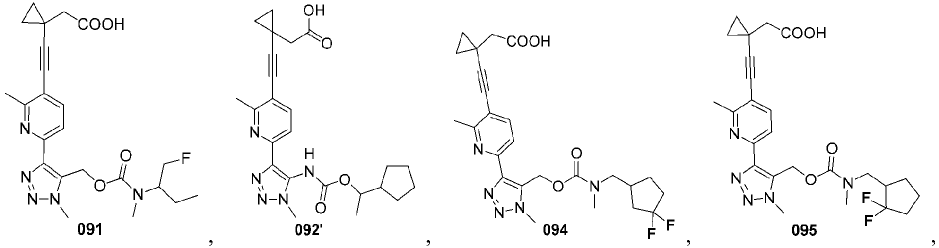 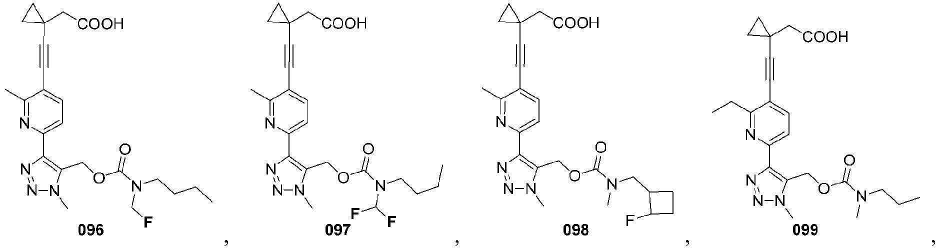 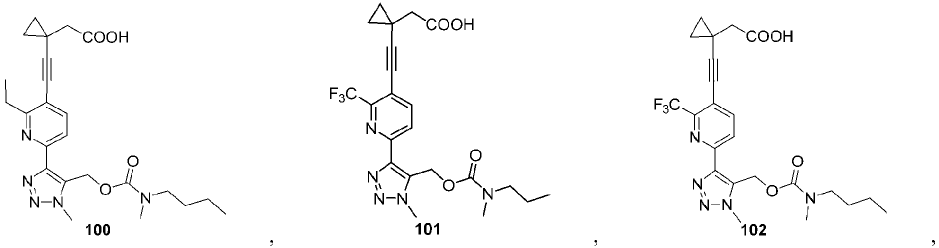 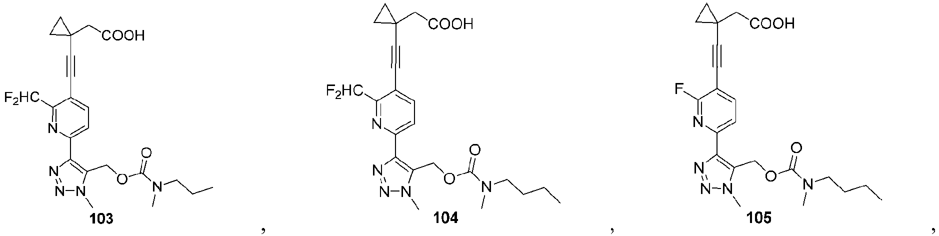 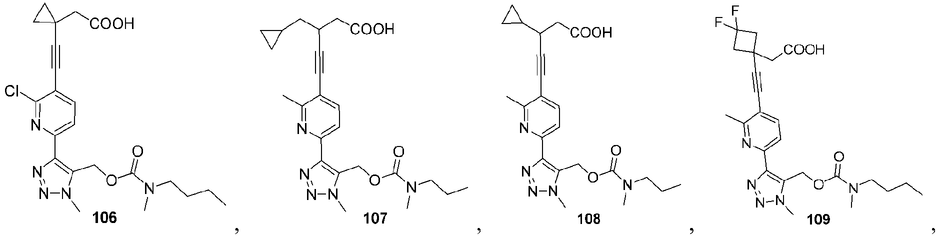 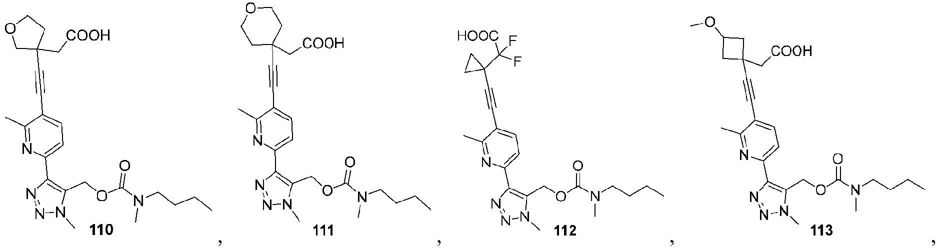 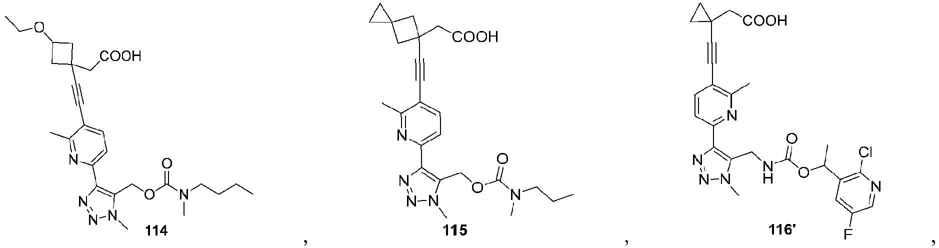 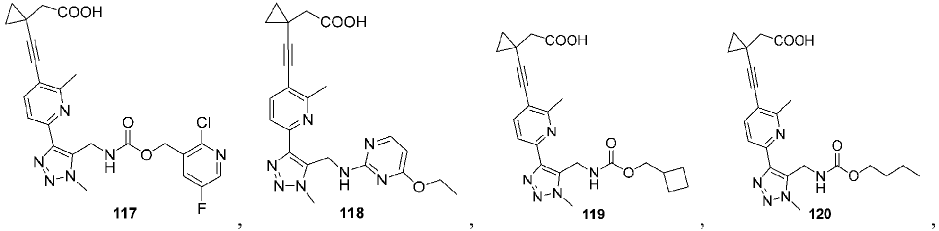 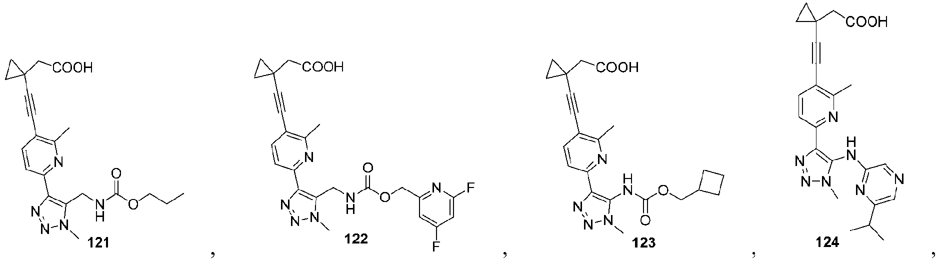 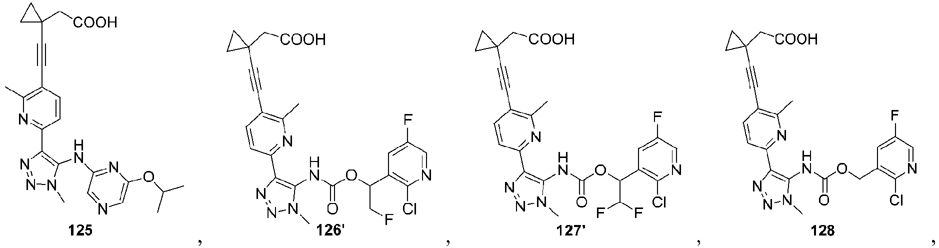 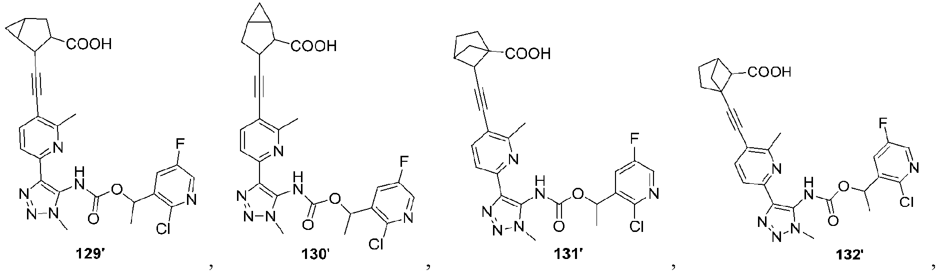 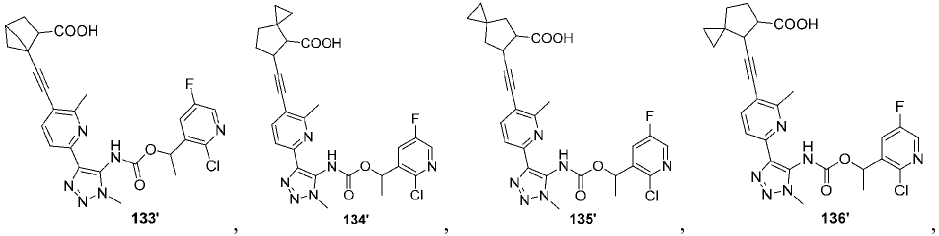 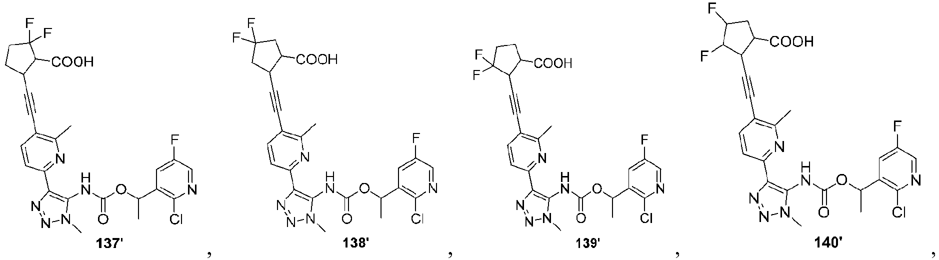 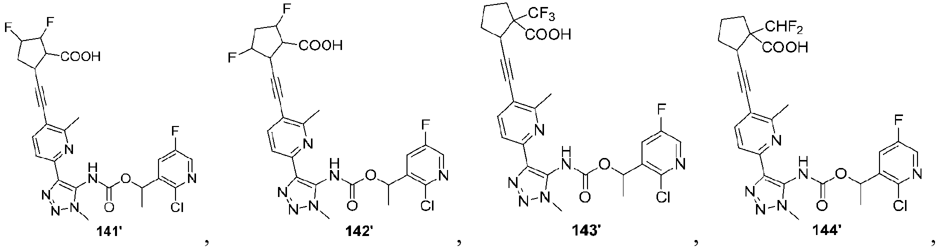 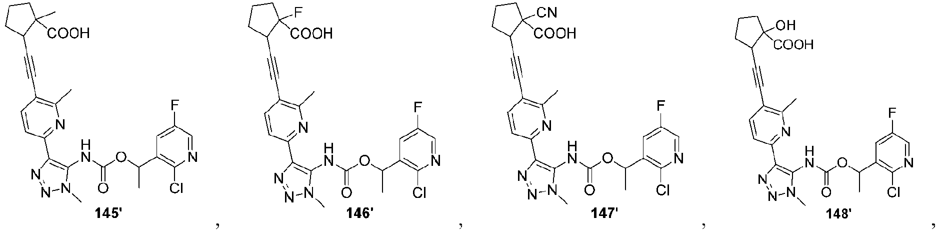 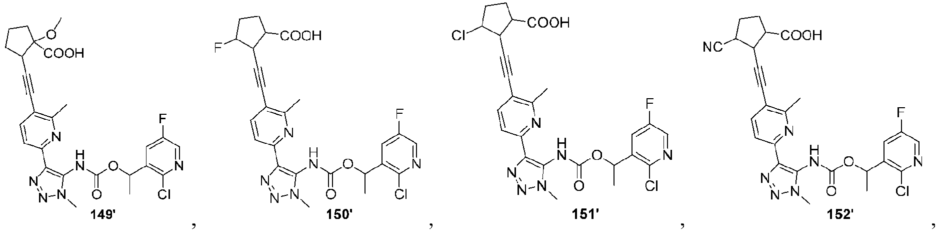 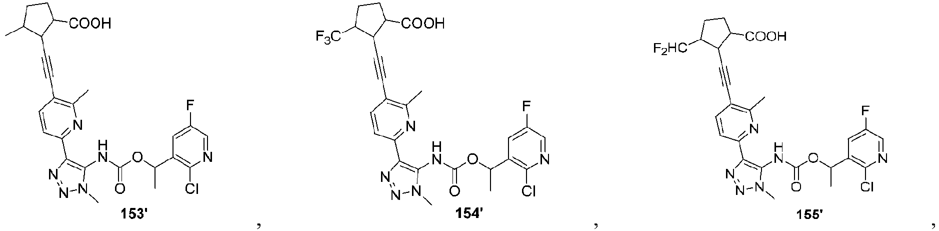 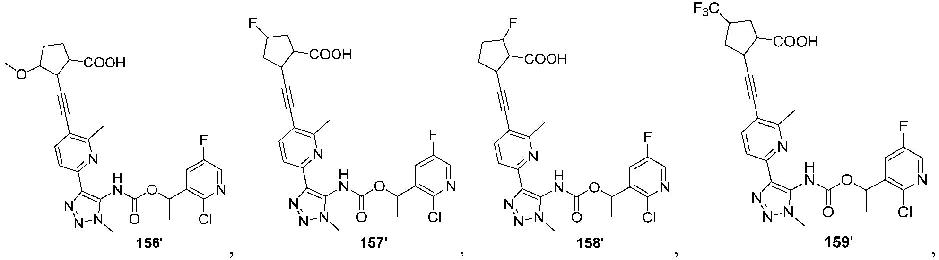 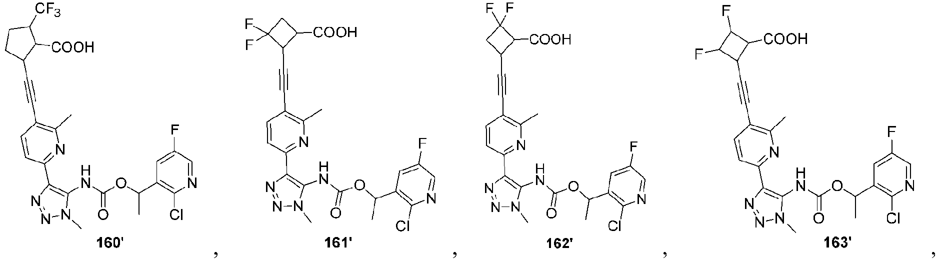 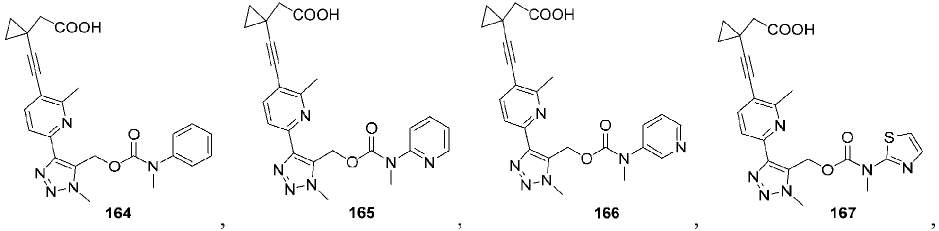 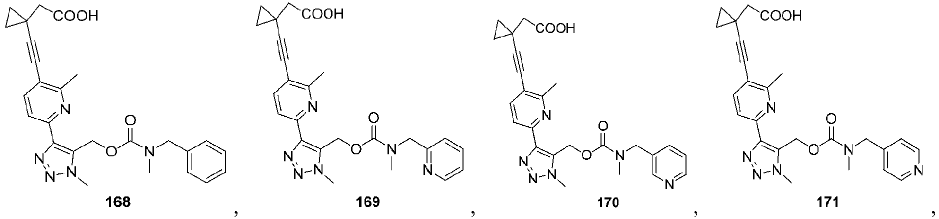 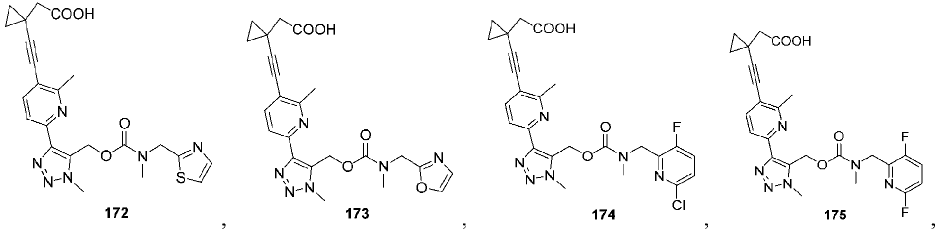 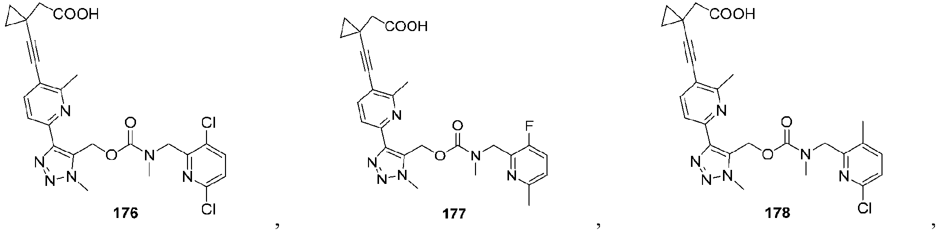 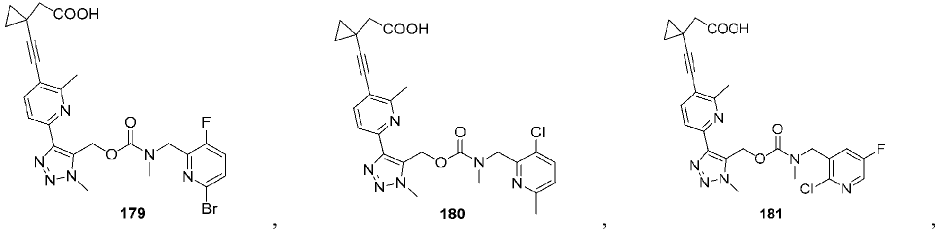 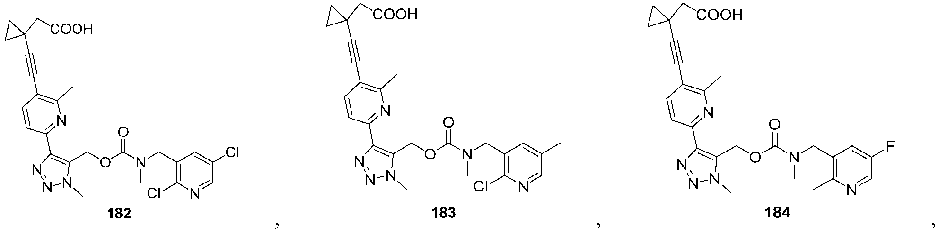 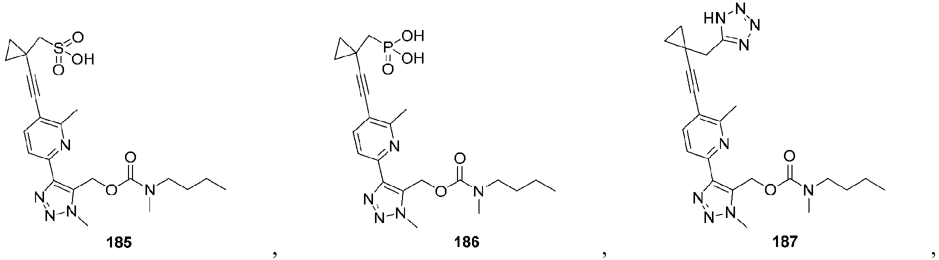 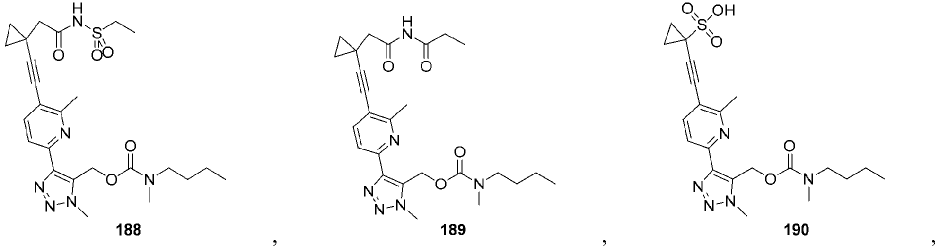 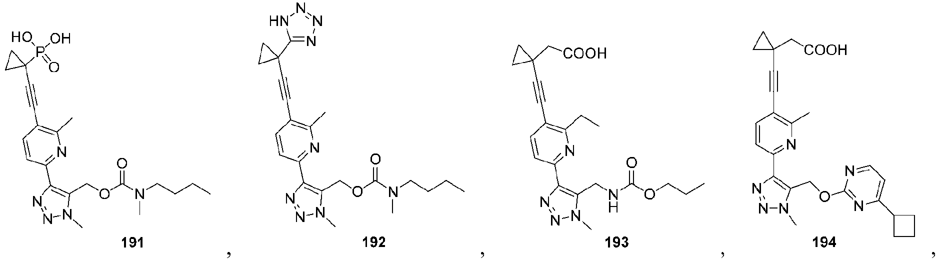 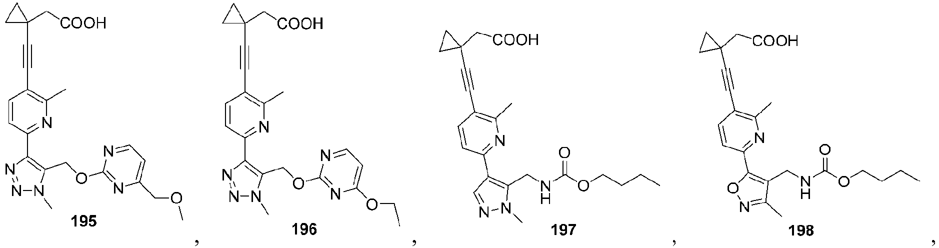 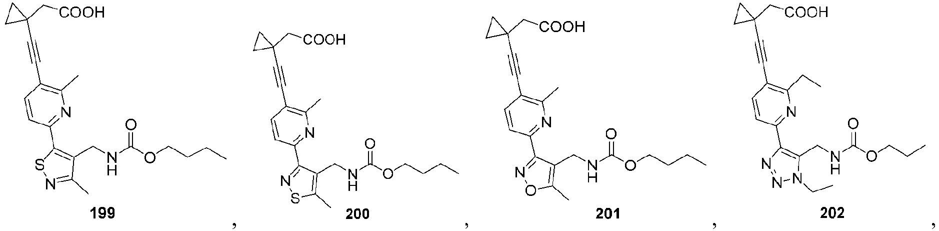 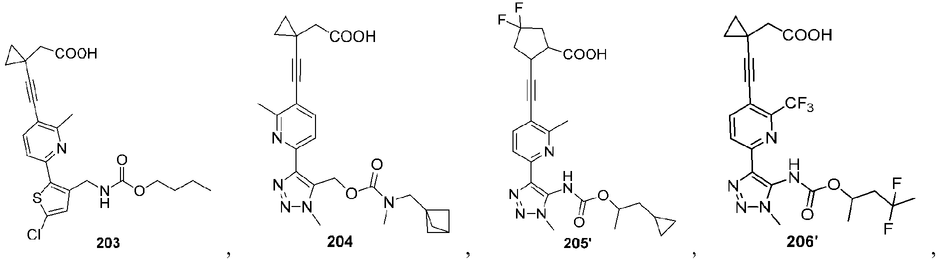 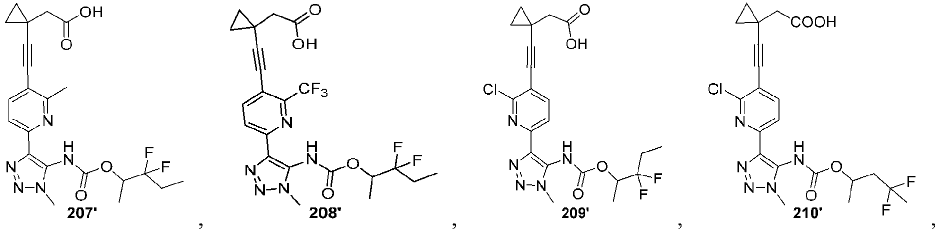 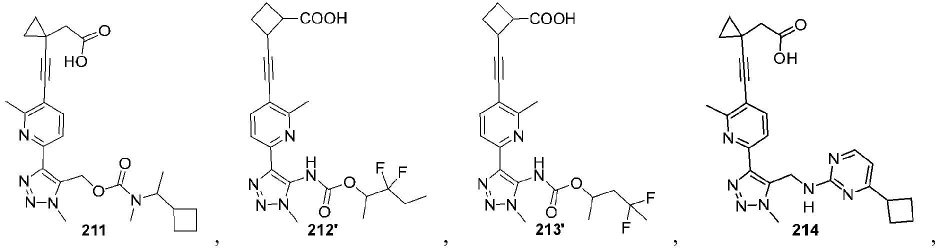 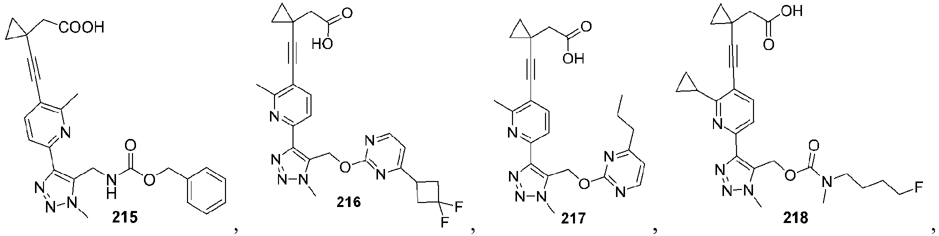 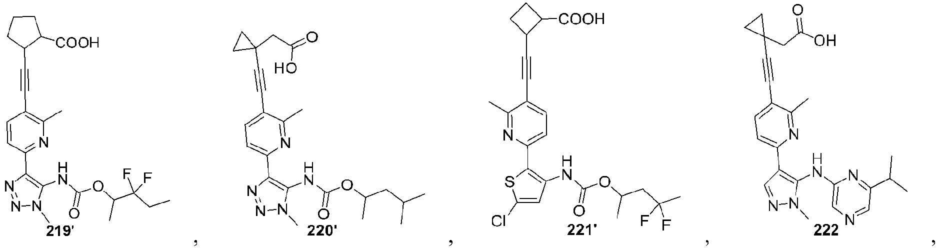 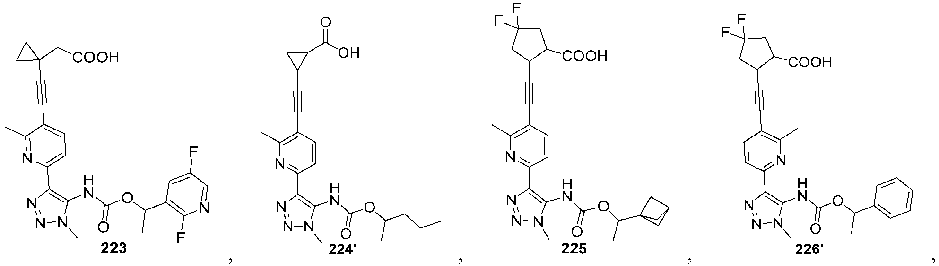 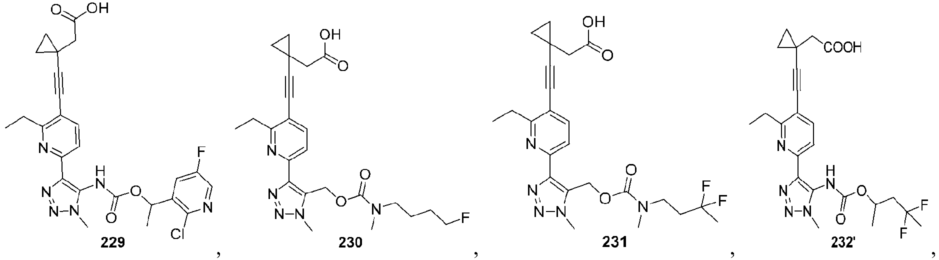 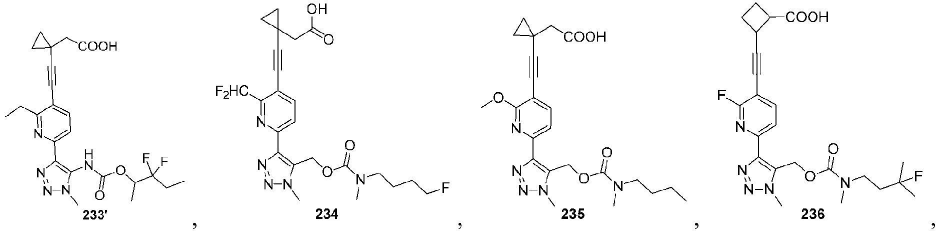 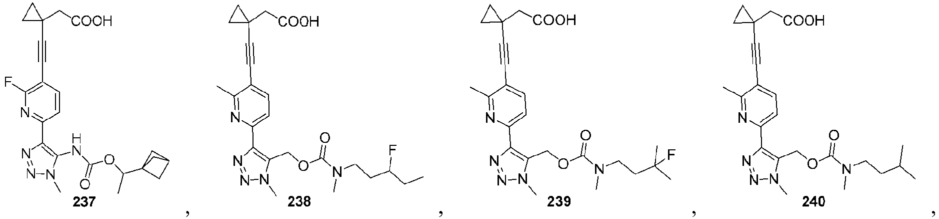 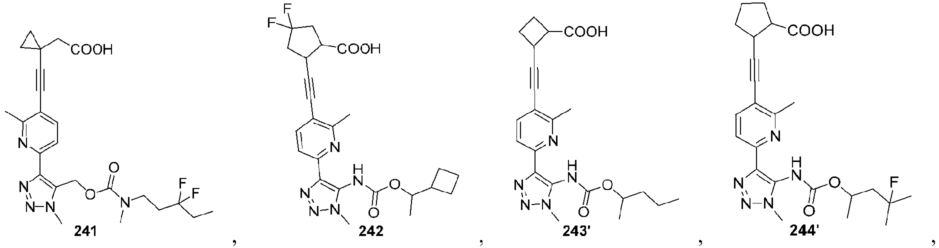 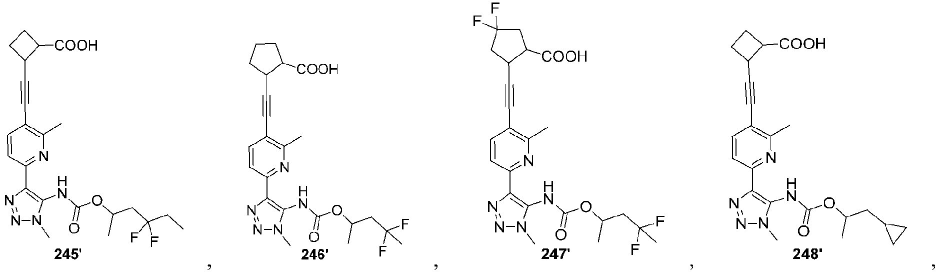 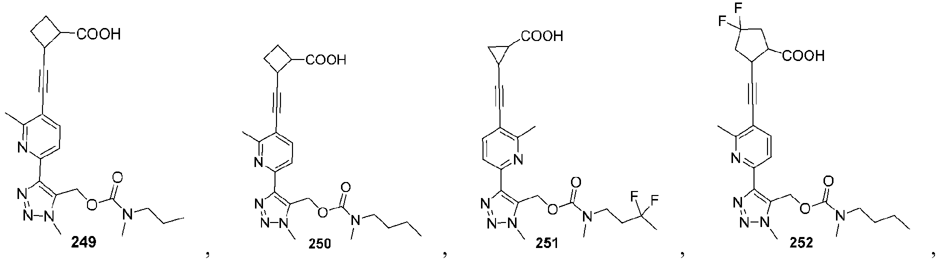 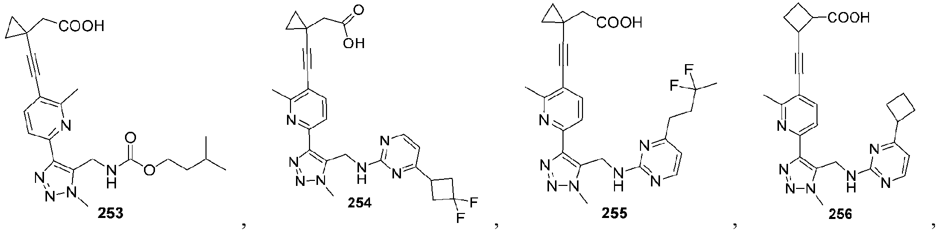 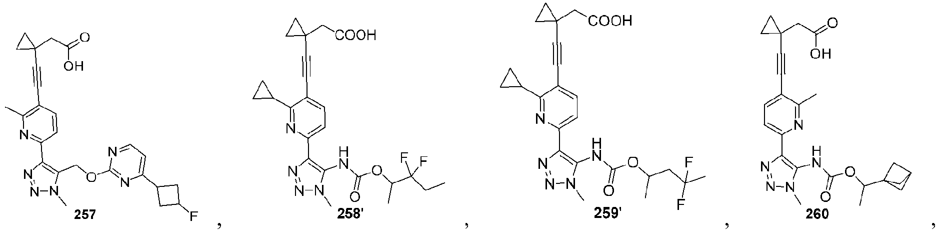 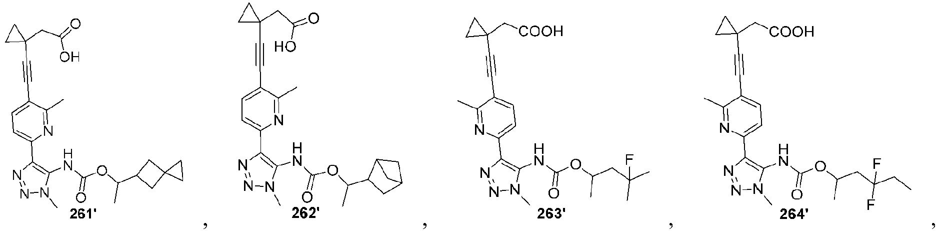 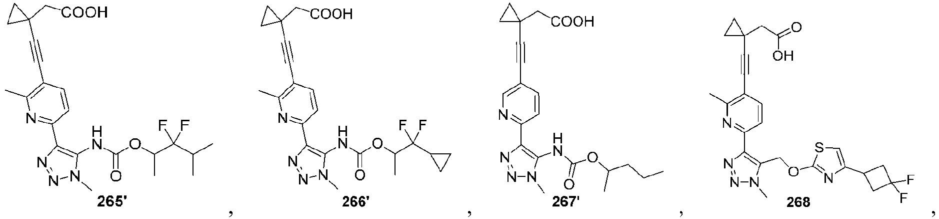 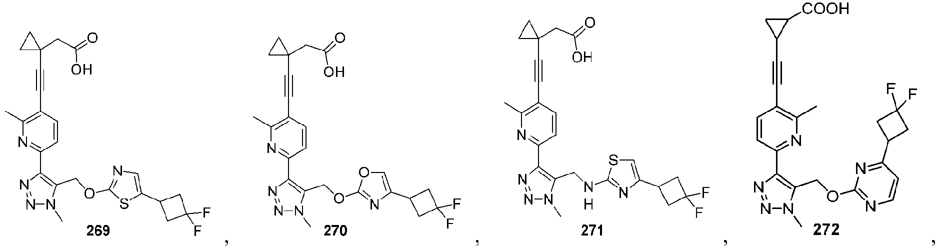 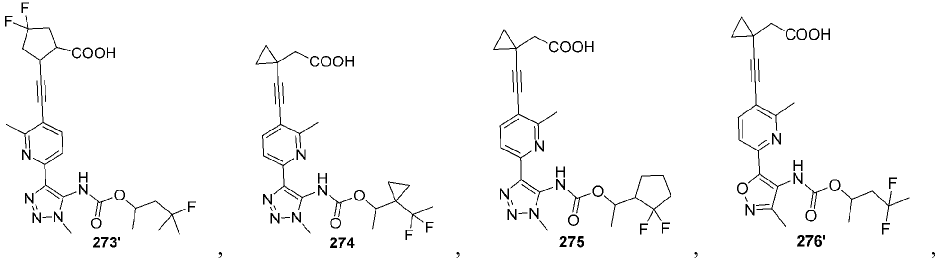 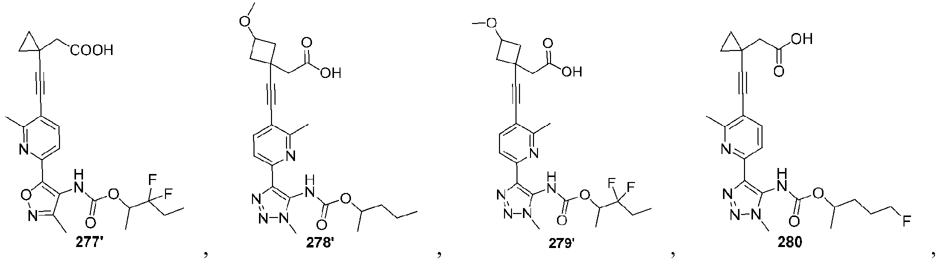 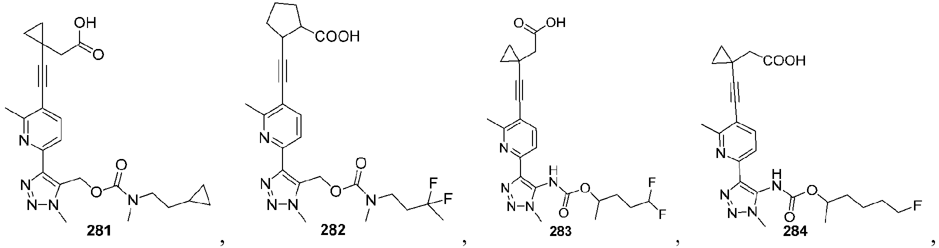 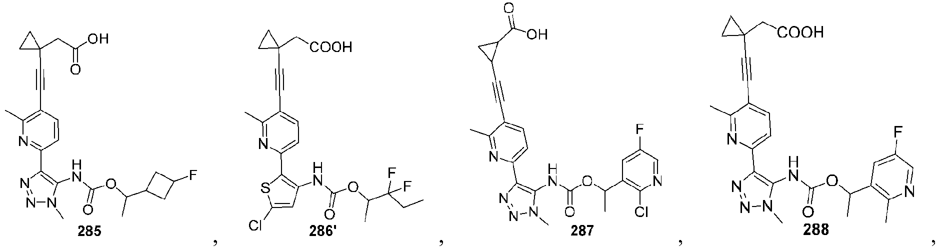 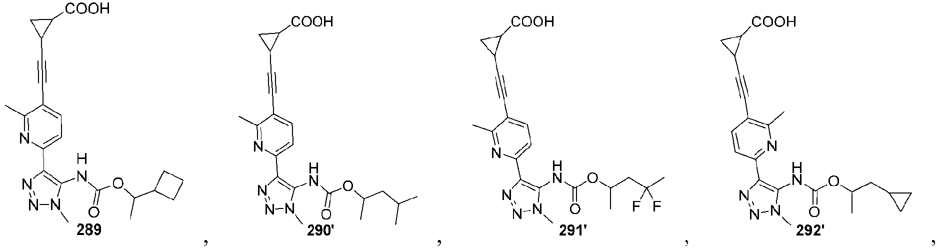 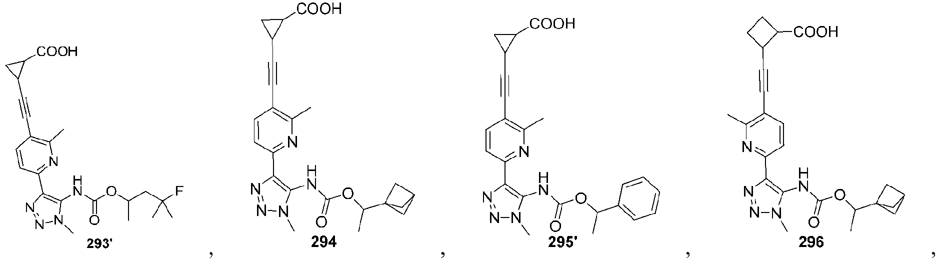 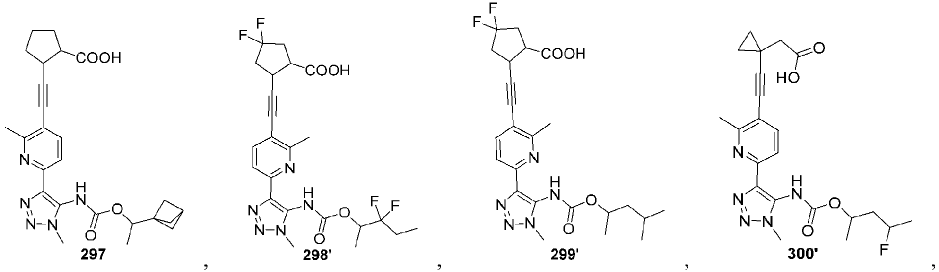 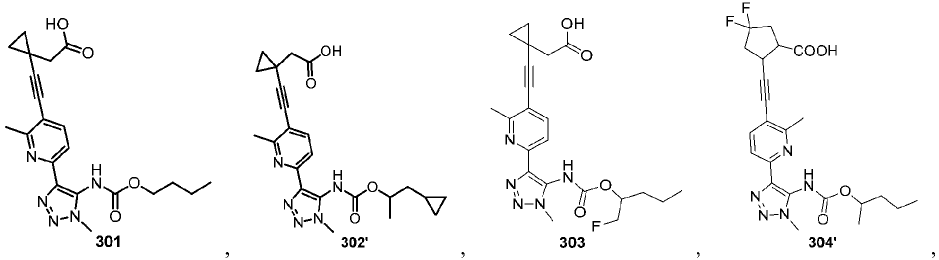 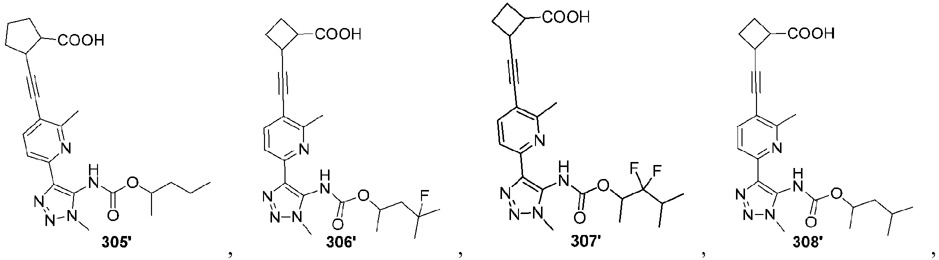 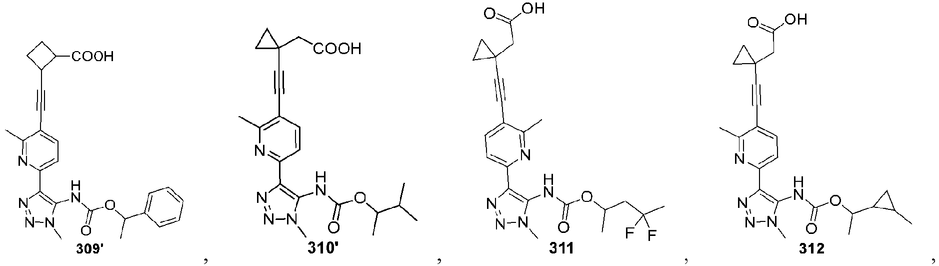 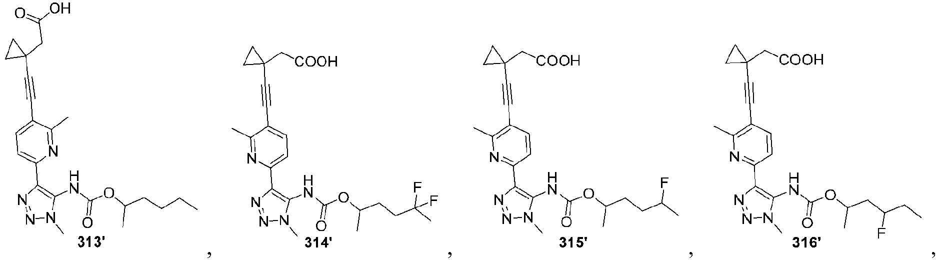 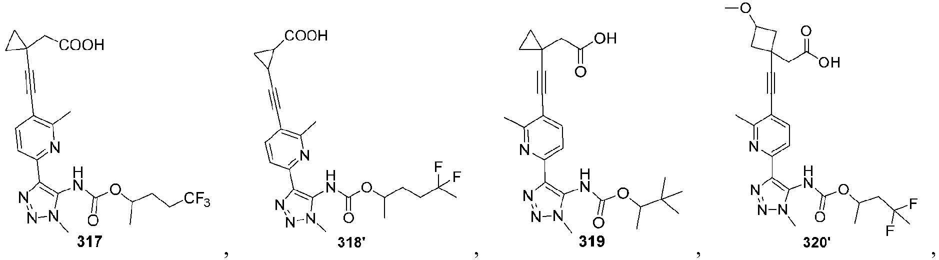 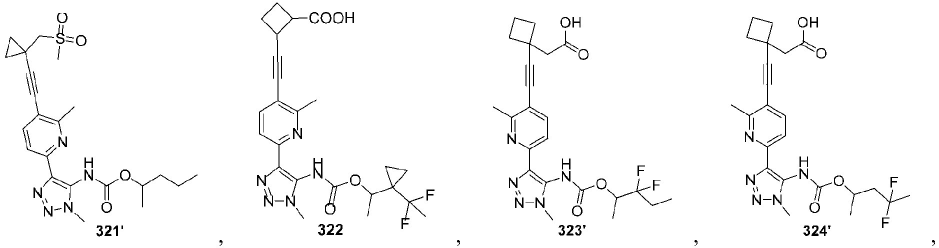 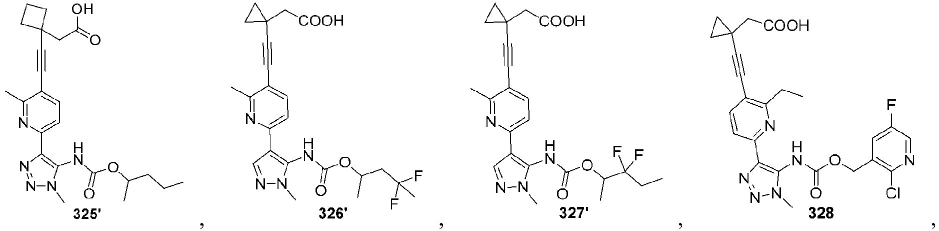 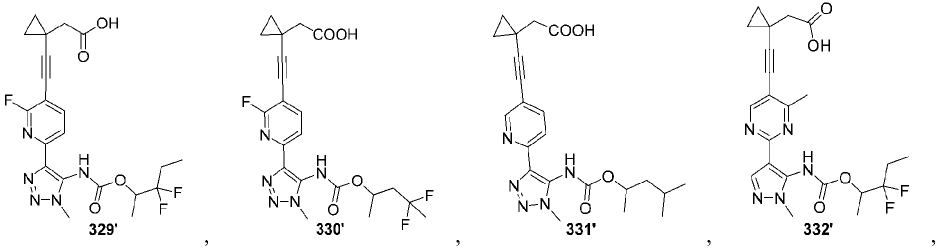 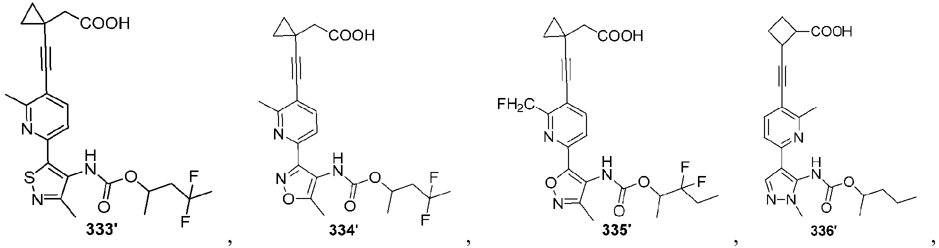 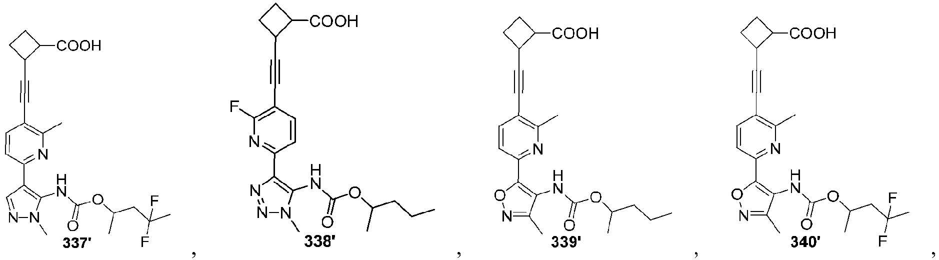 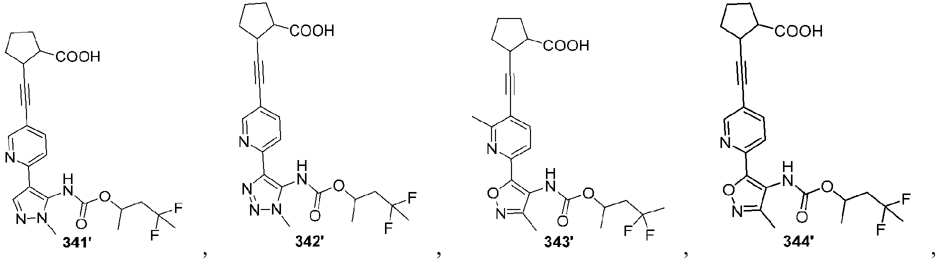 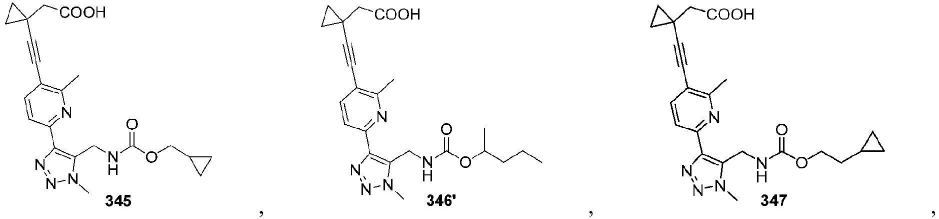 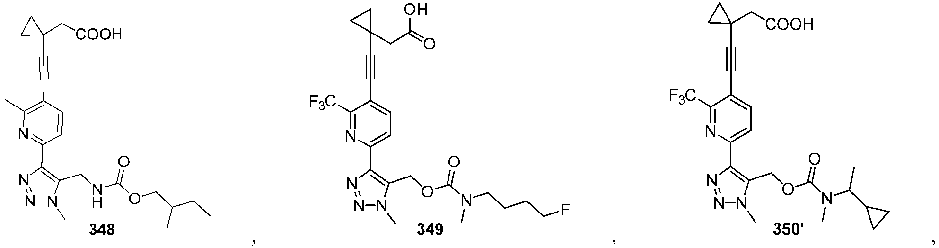 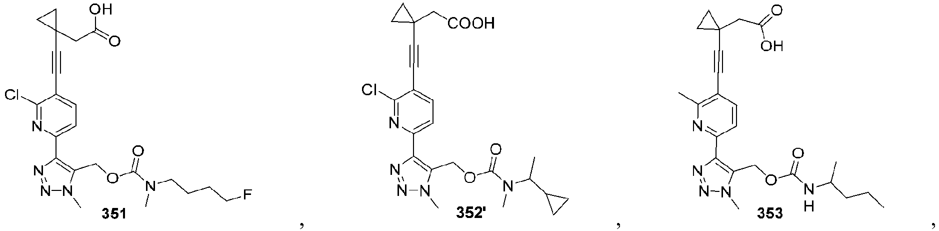 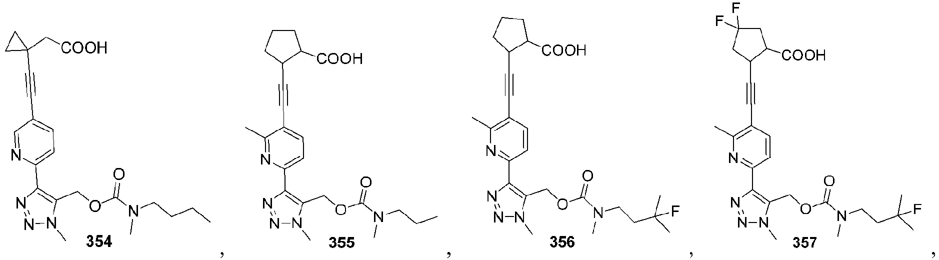 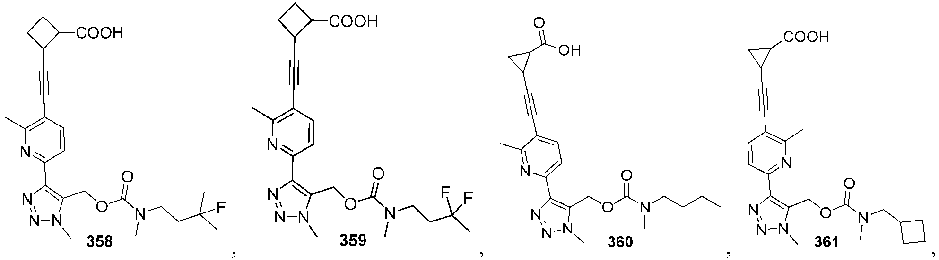 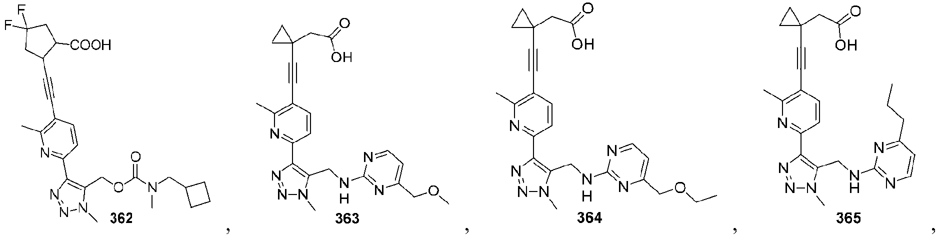 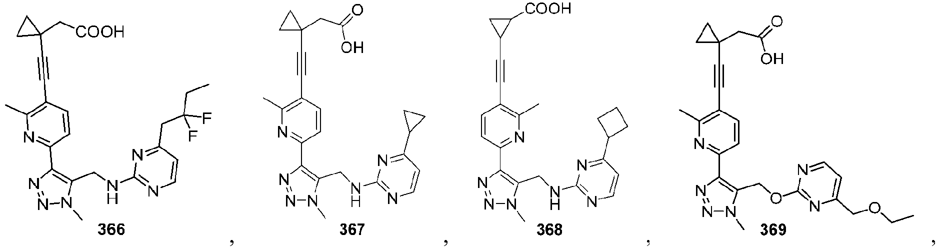 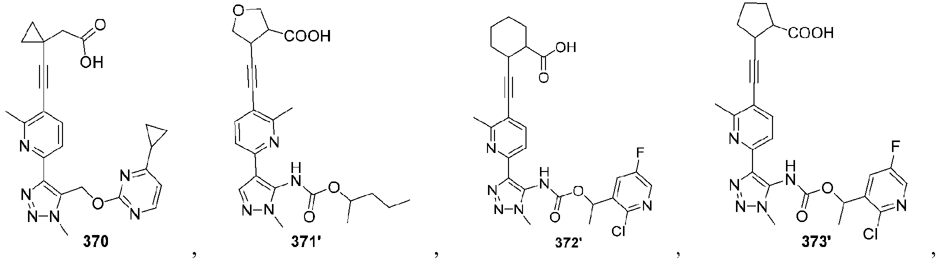 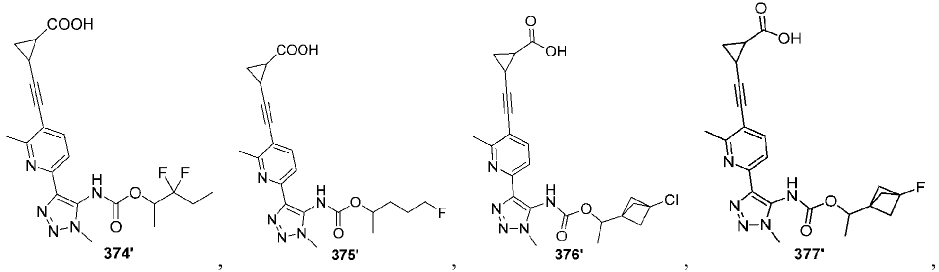 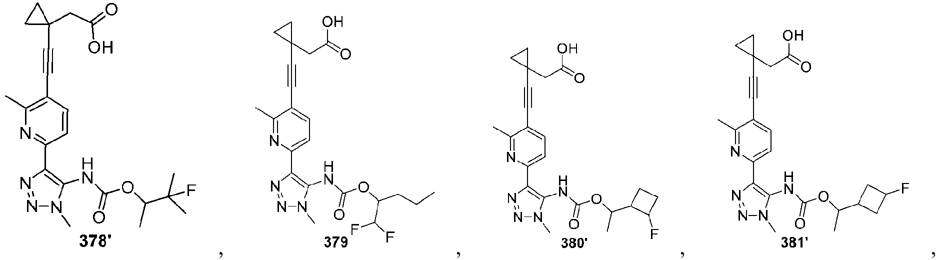 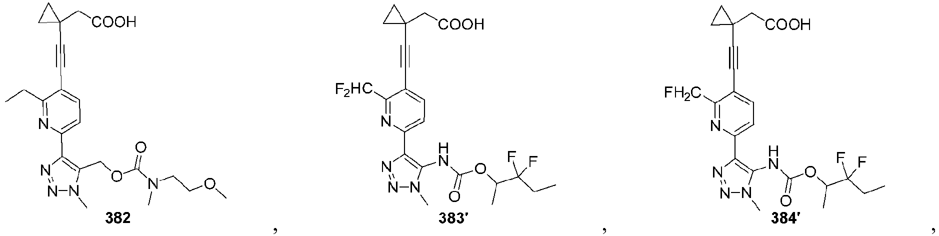 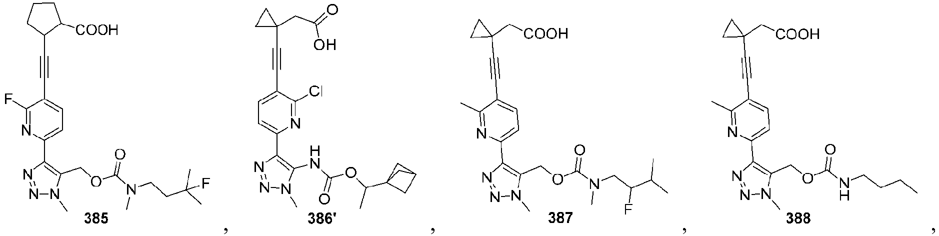 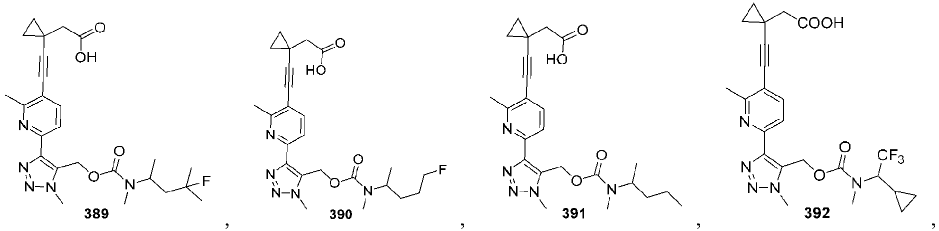 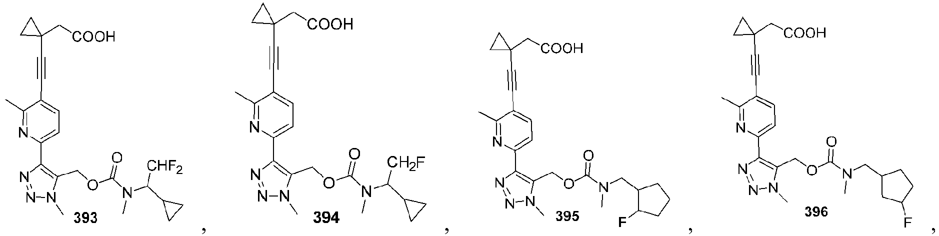 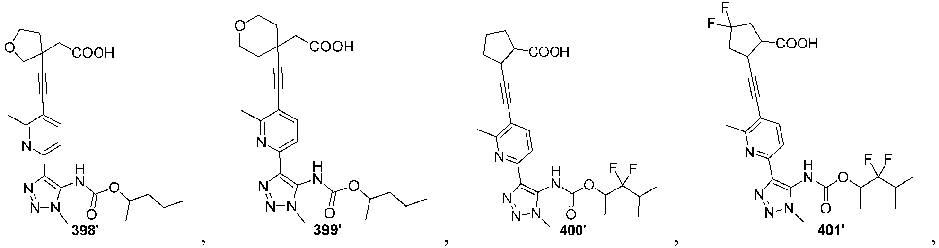 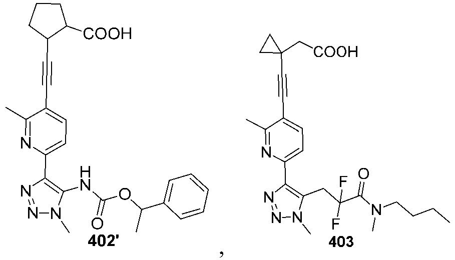 or its hydrochloride, 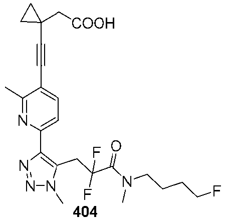 or its hydrochloride, 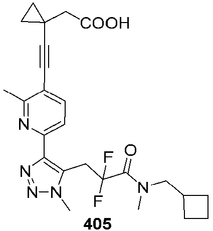 or its hydrochloride, 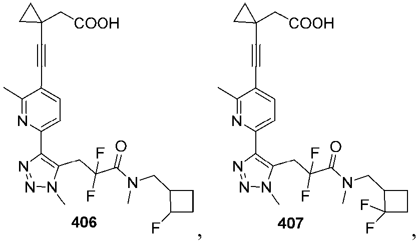 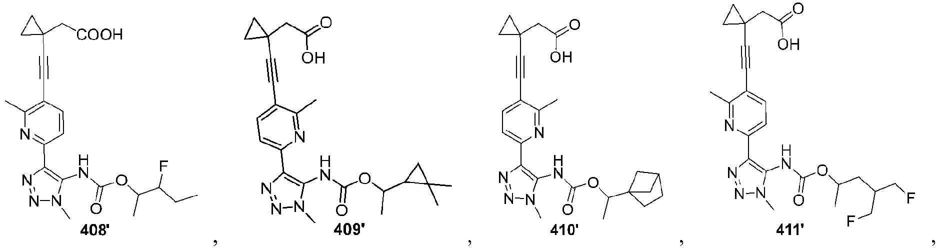 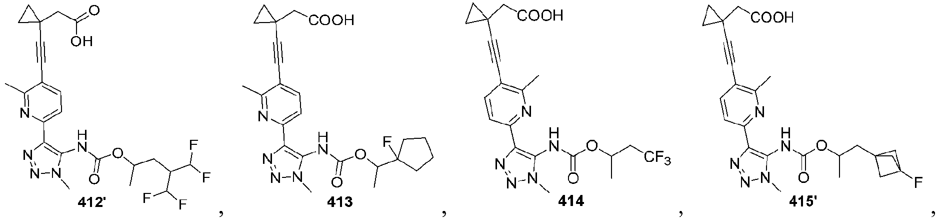 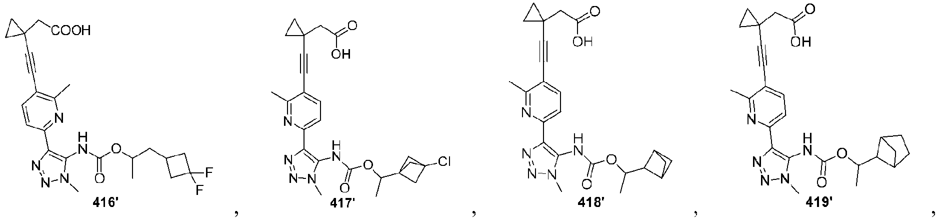 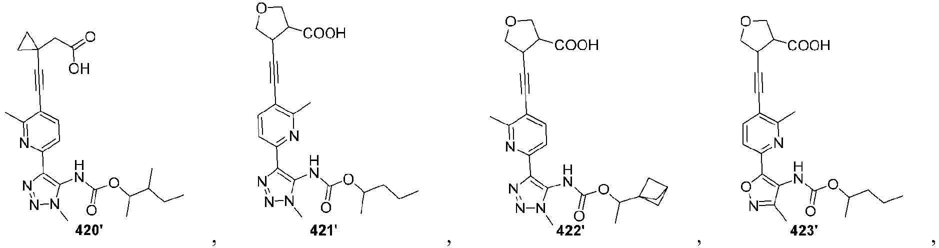 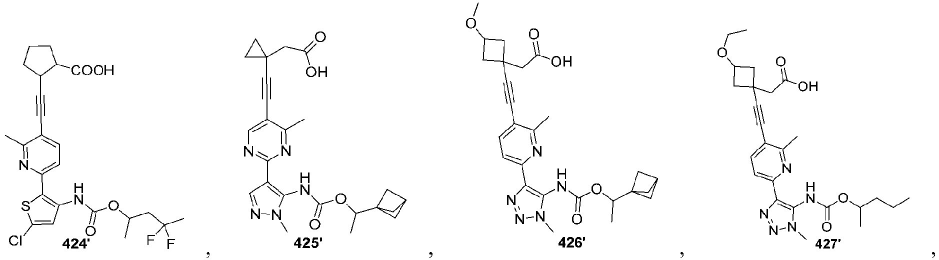 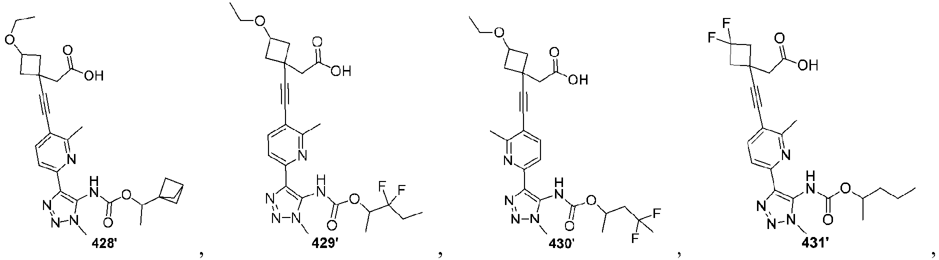 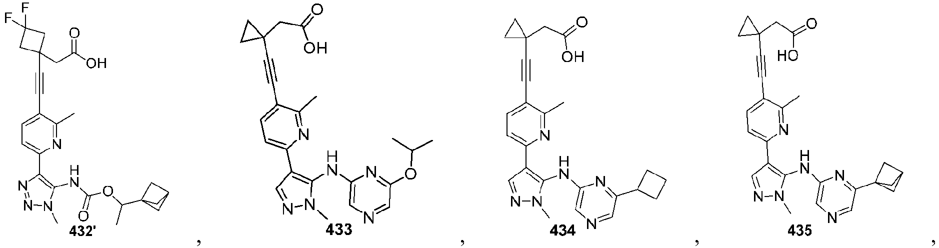 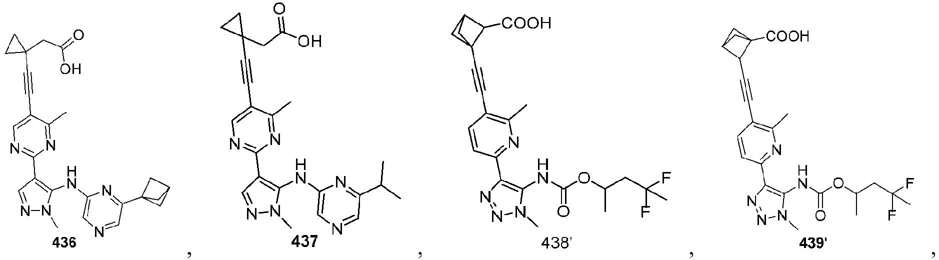 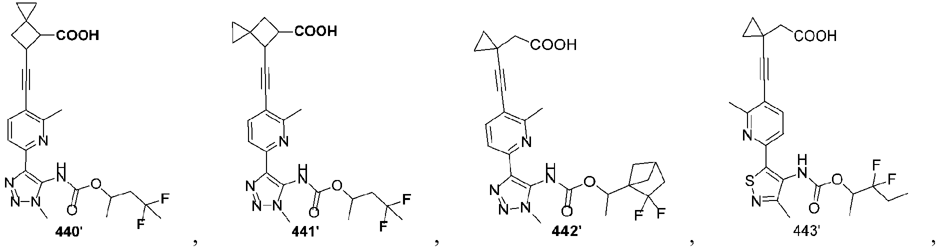 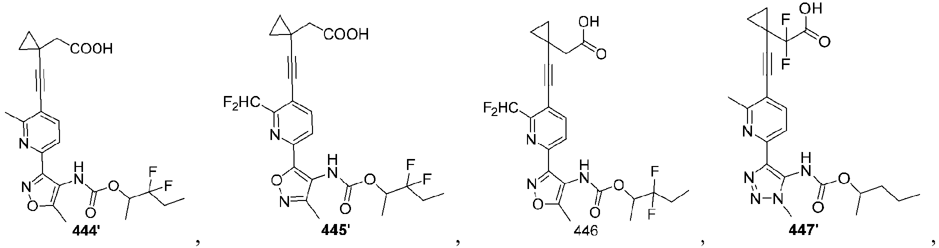 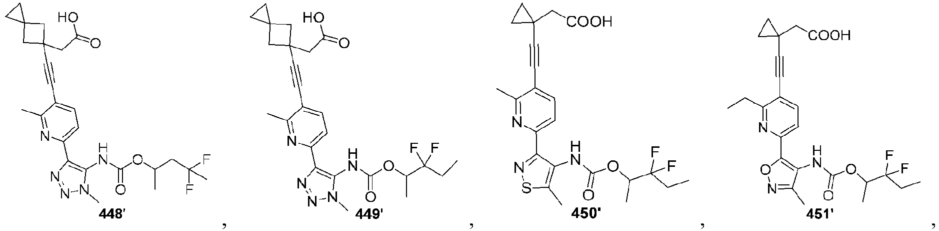 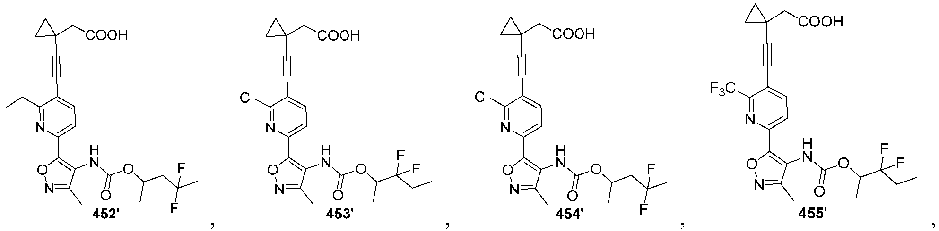 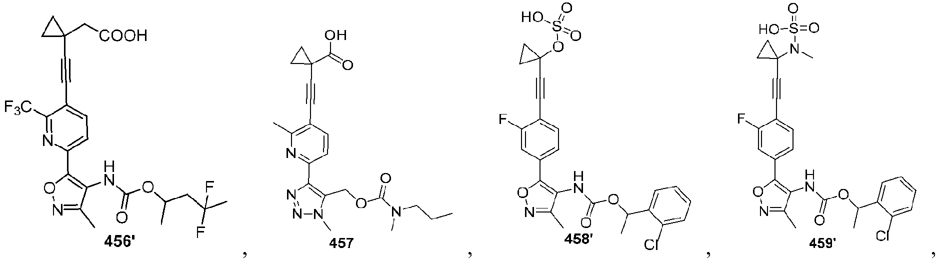 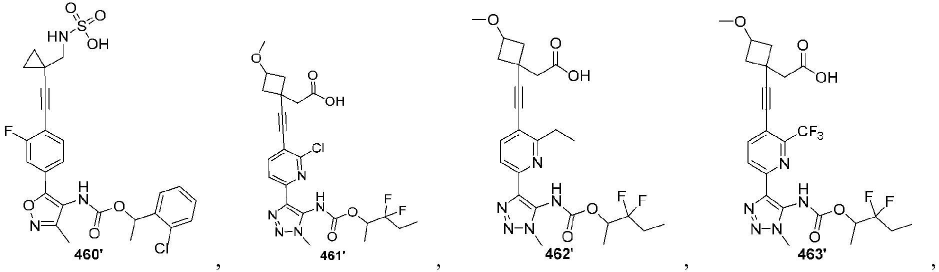 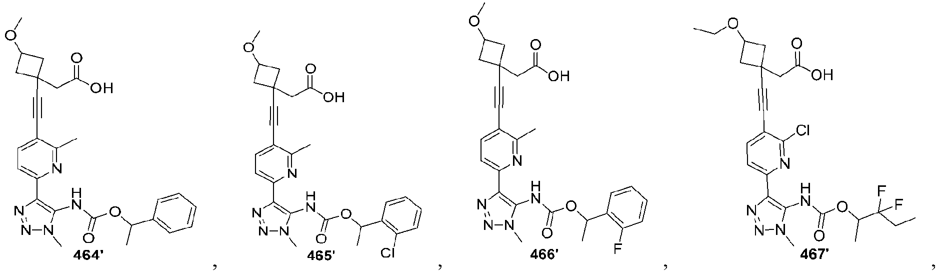 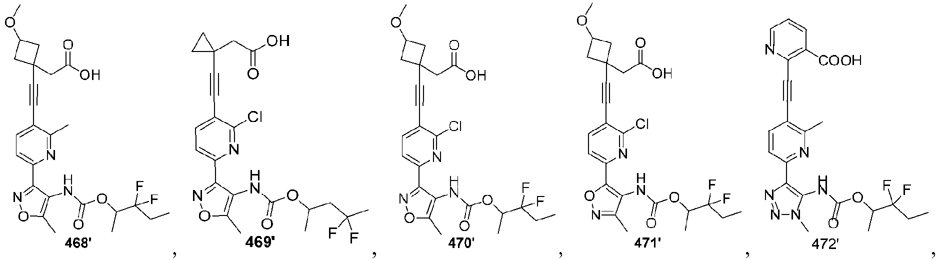 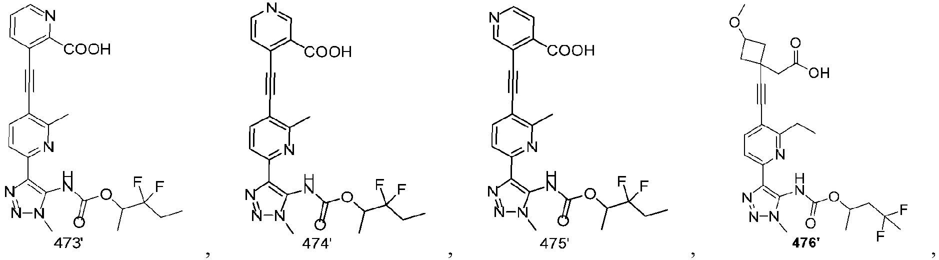 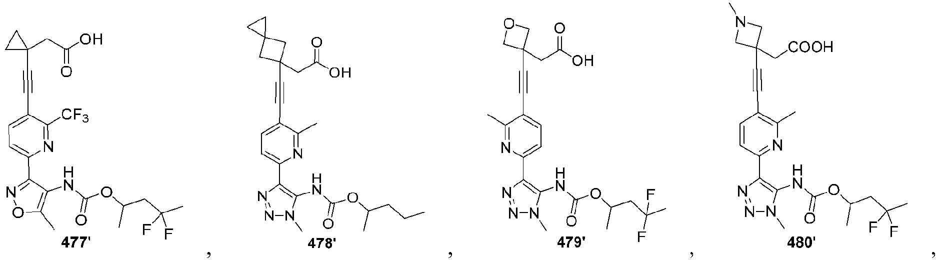 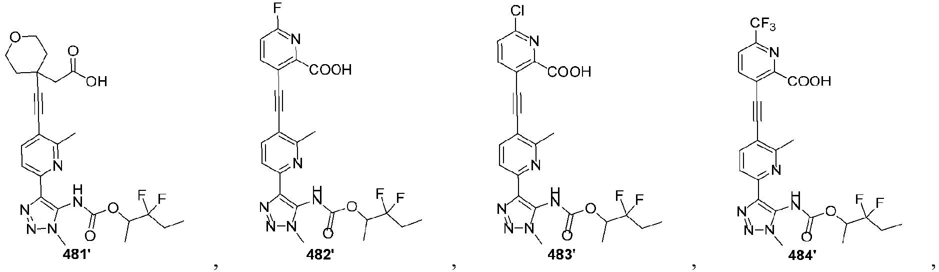 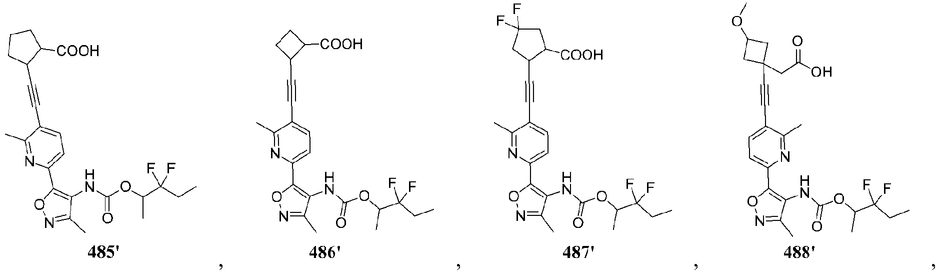 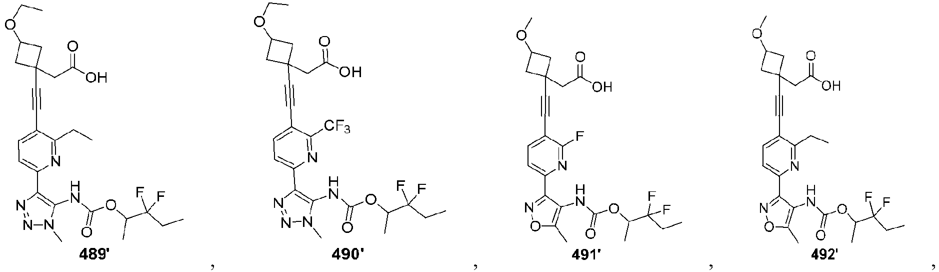 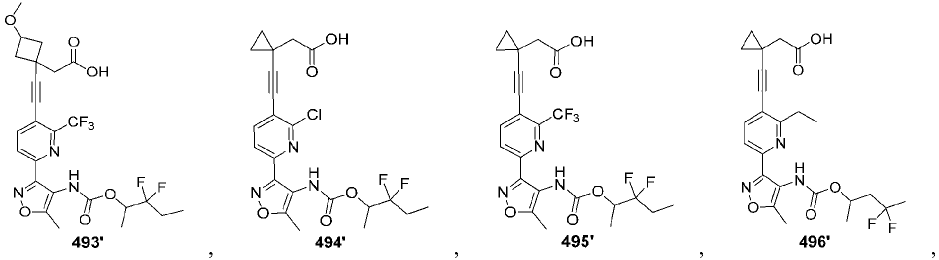 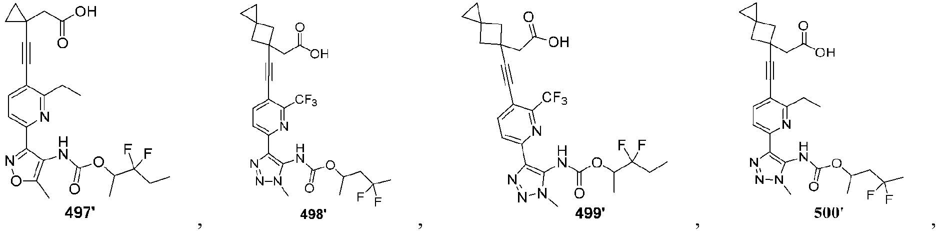 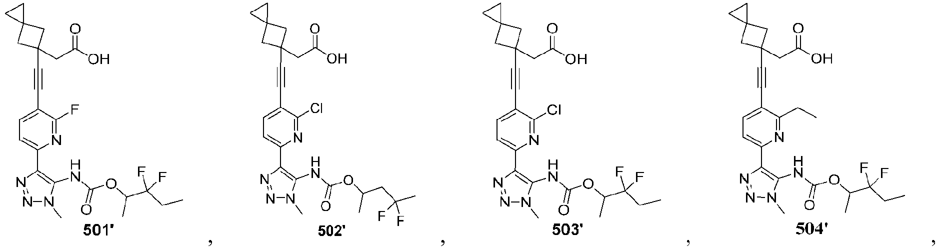 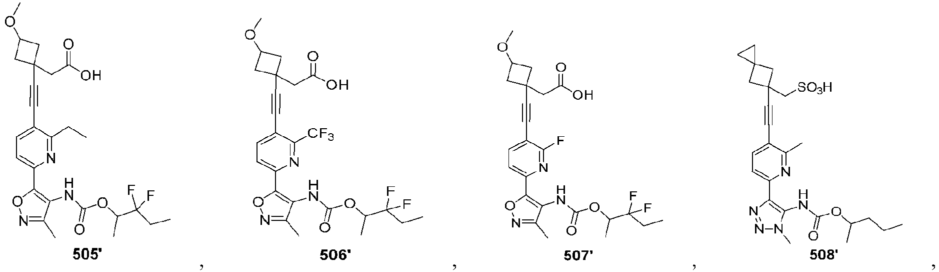 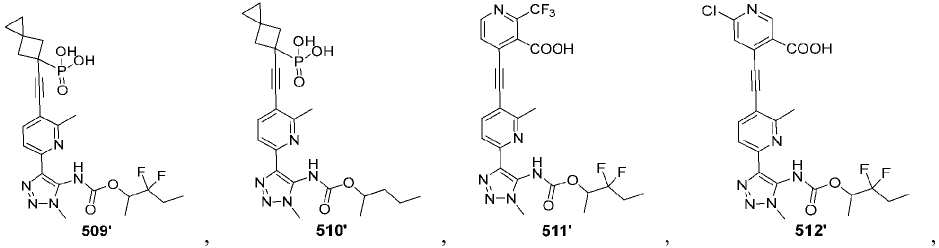 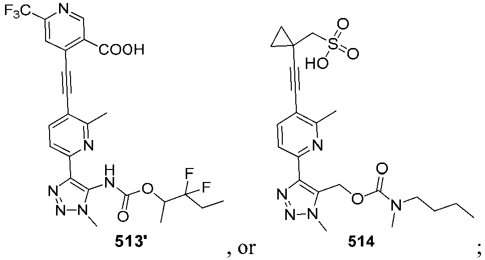 preferably, the compound of formula (I) is any one of the following compounds: 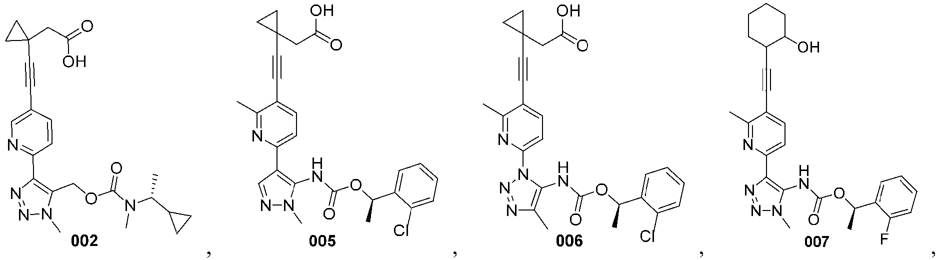 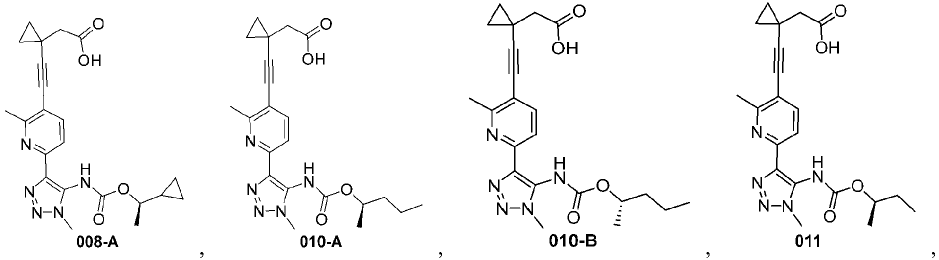 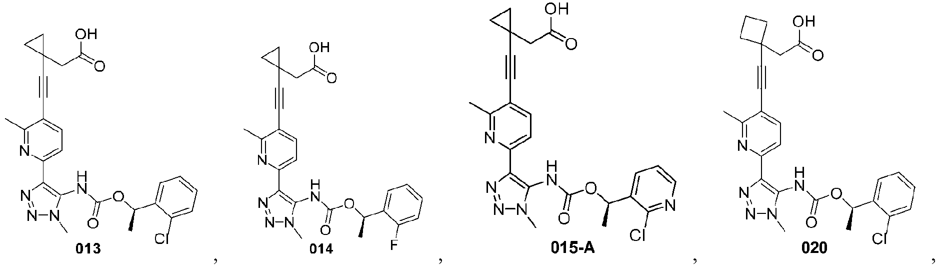 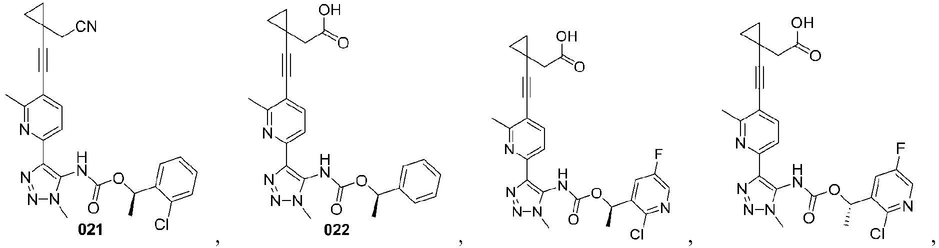 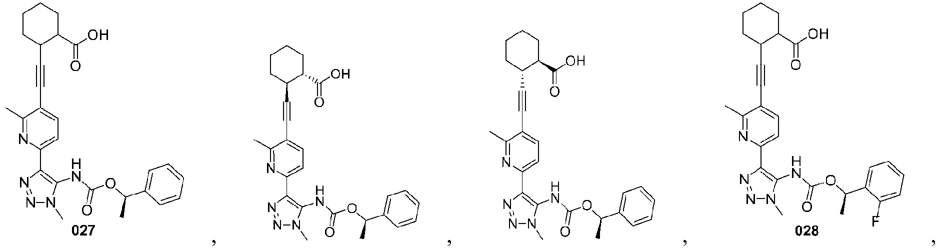 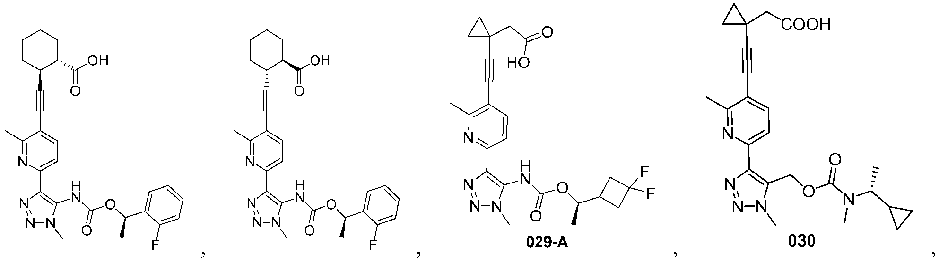 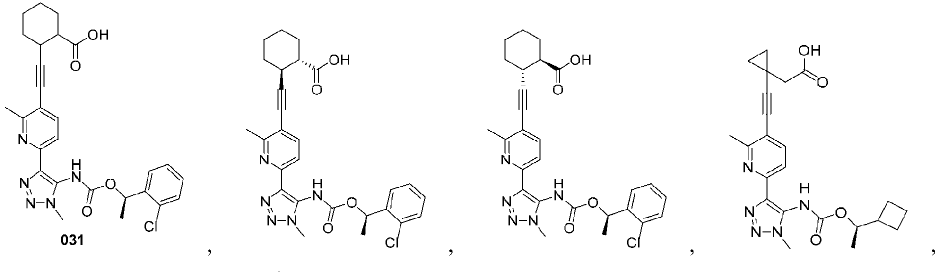 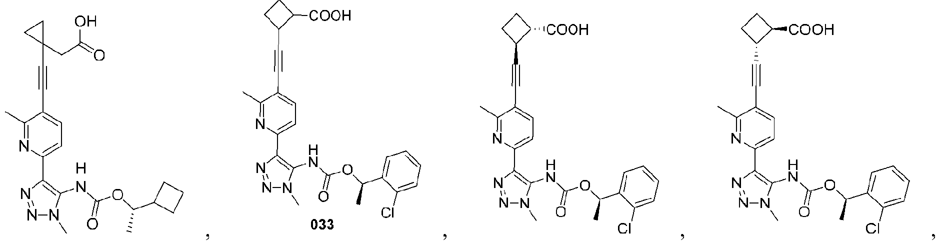 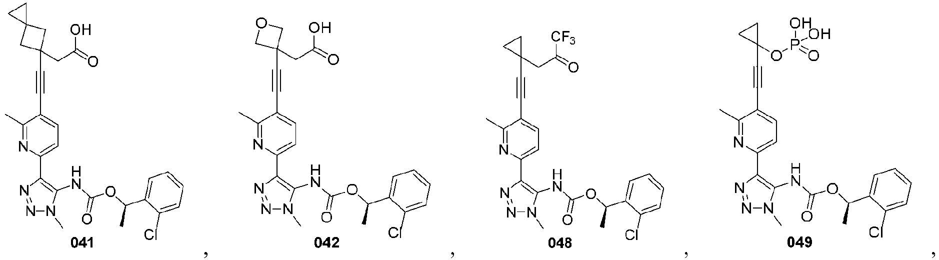 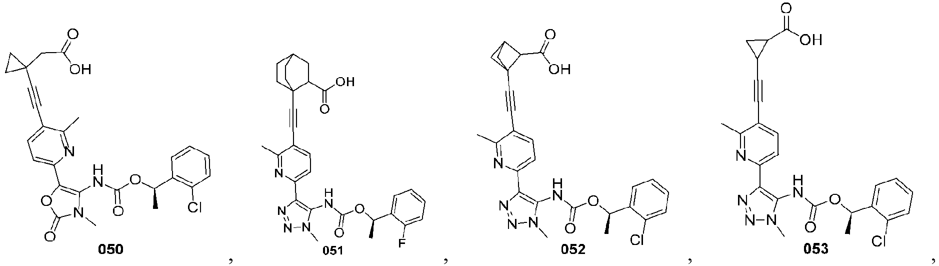 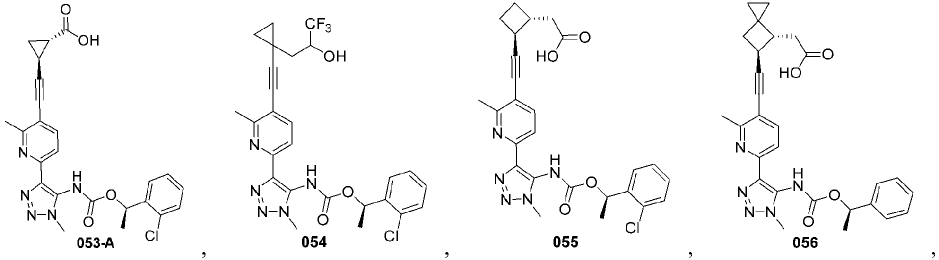 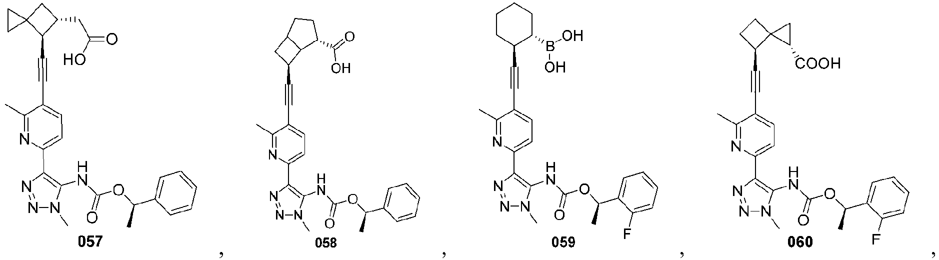 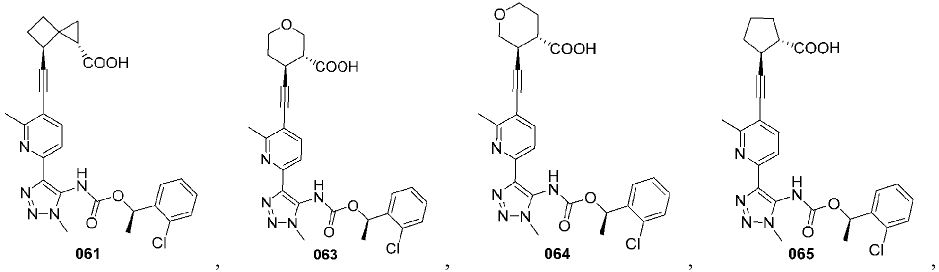 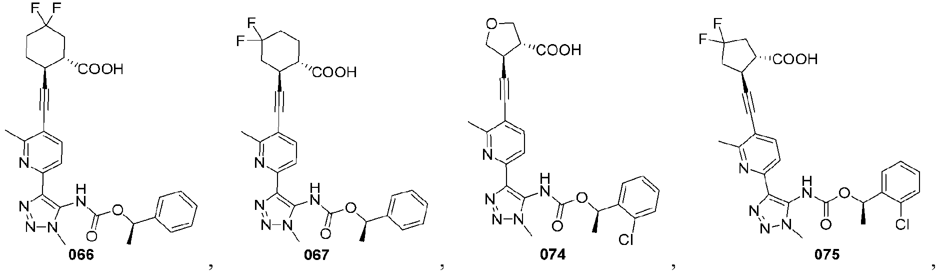 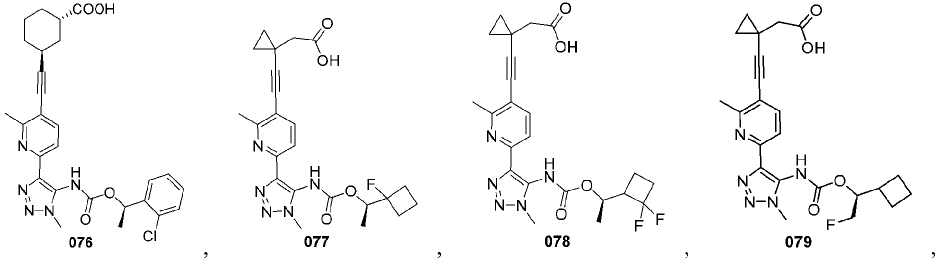 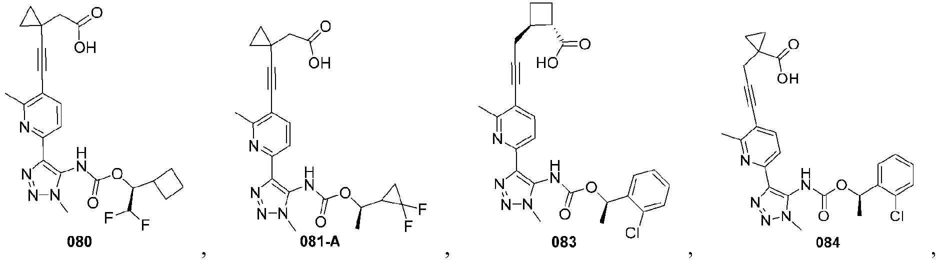 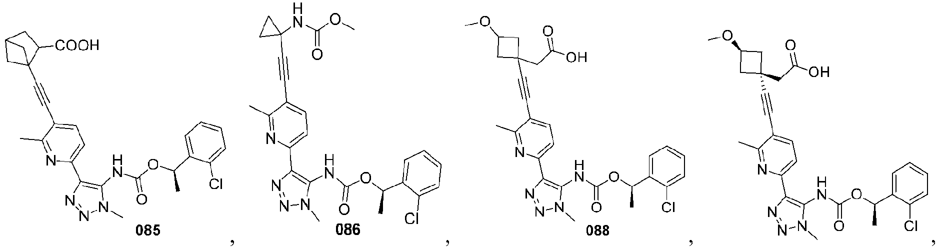 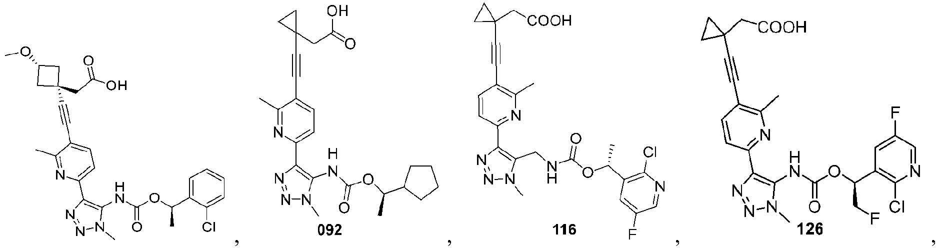 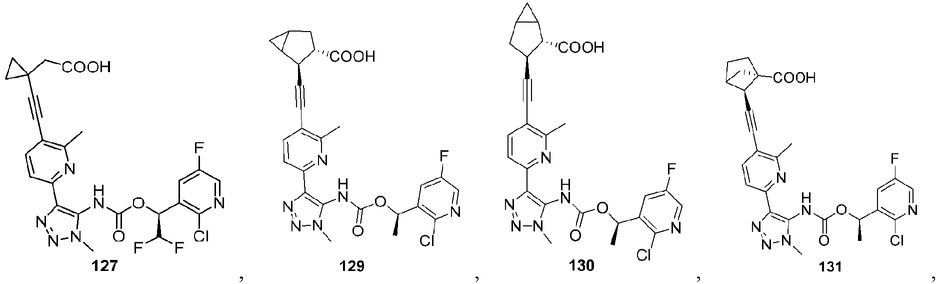 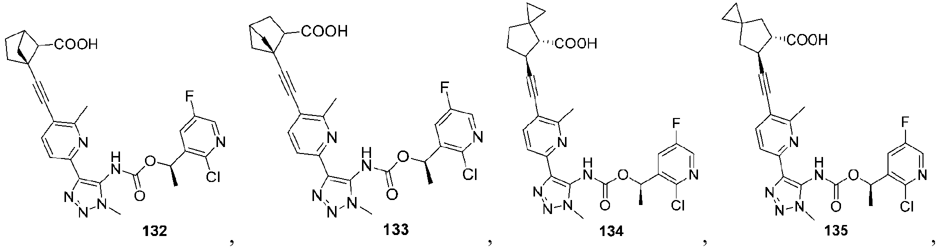 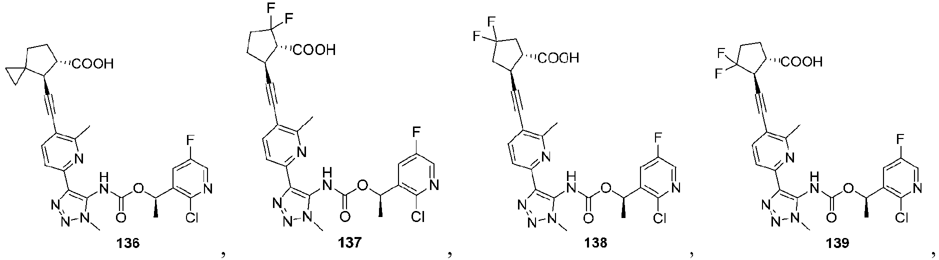 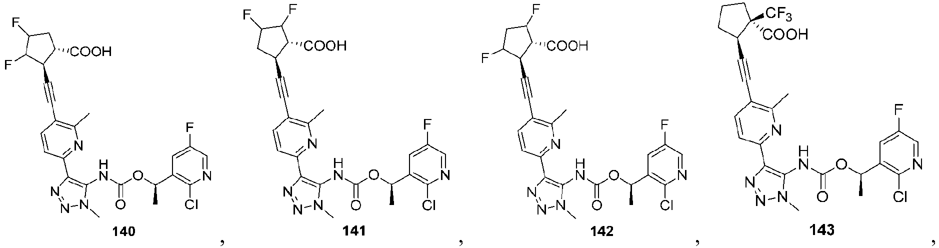 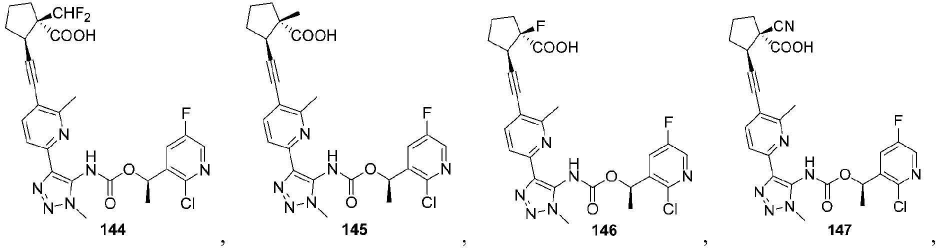 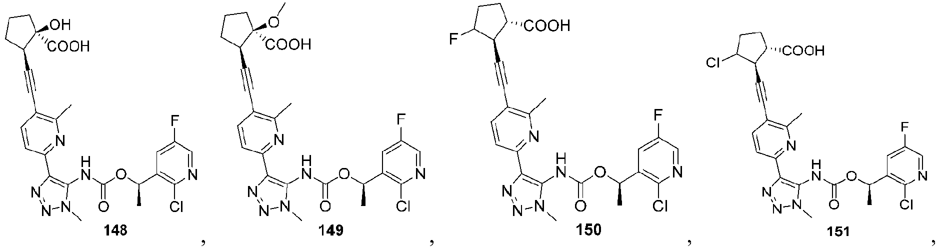 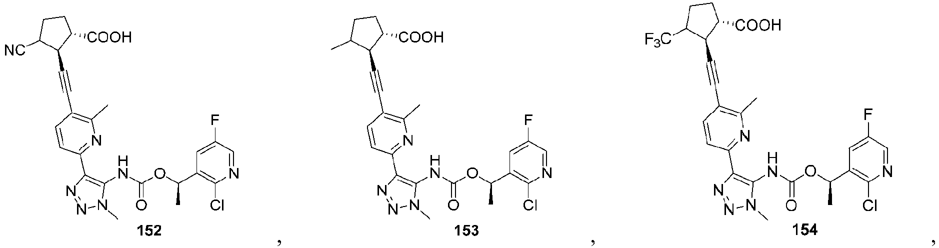 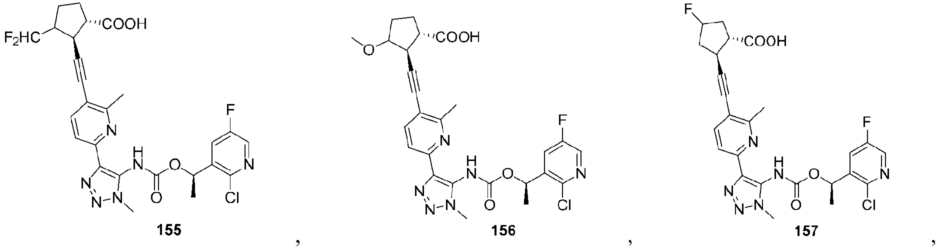 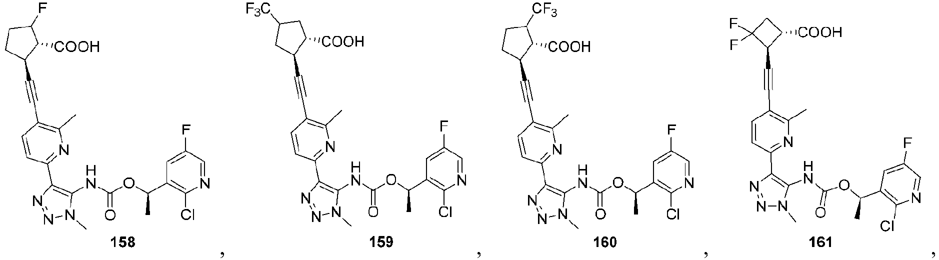 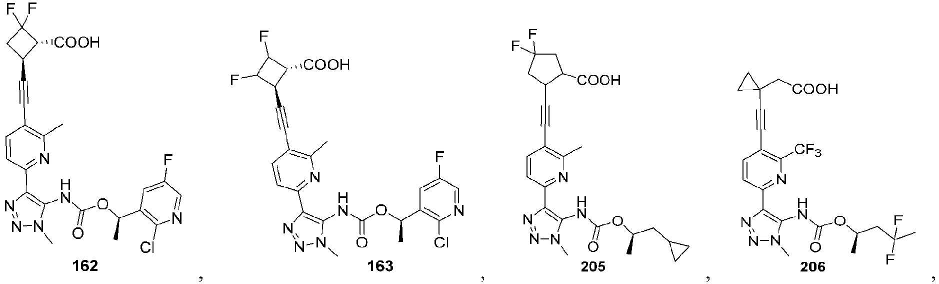 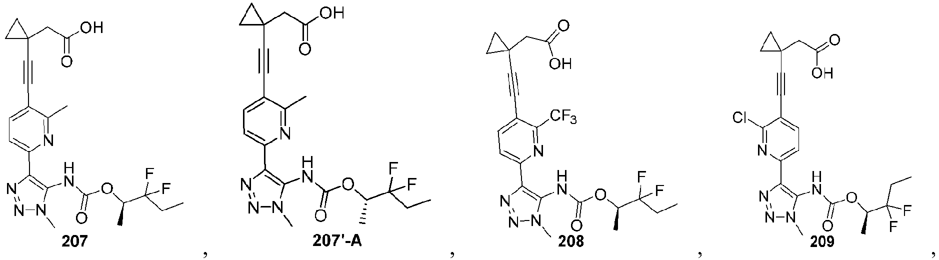 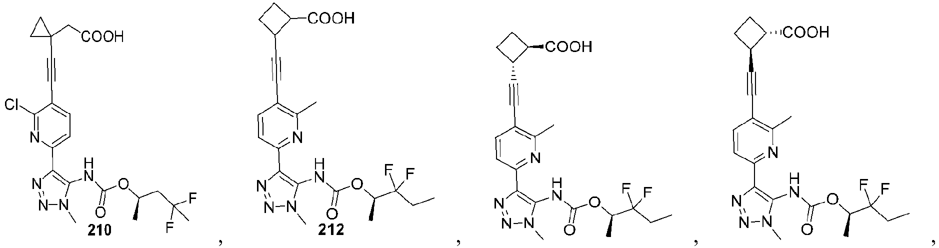 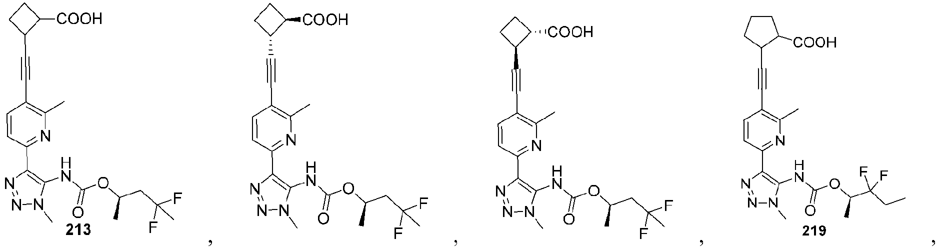 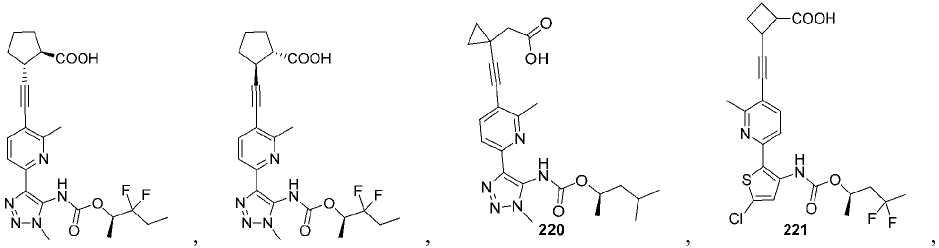 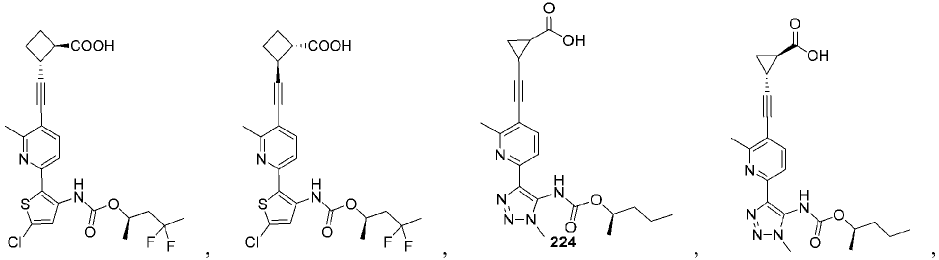 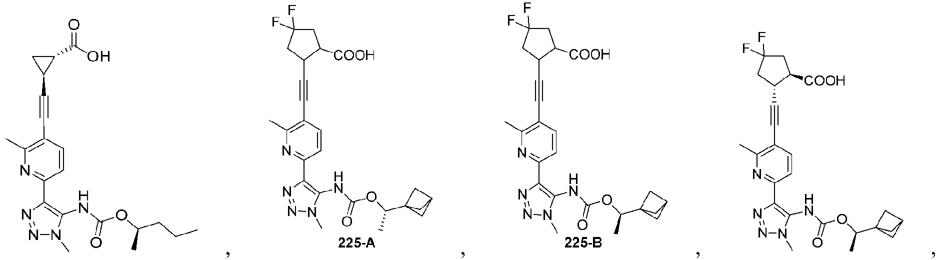 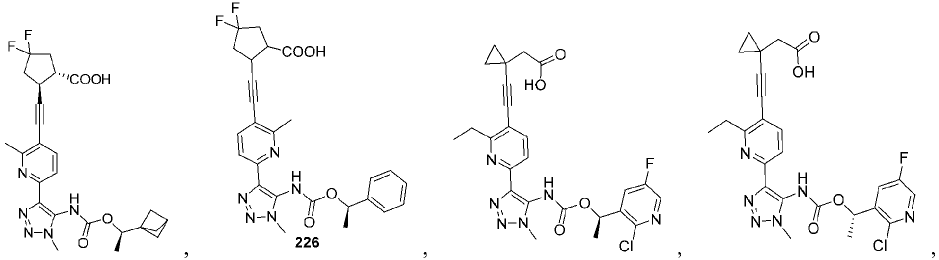 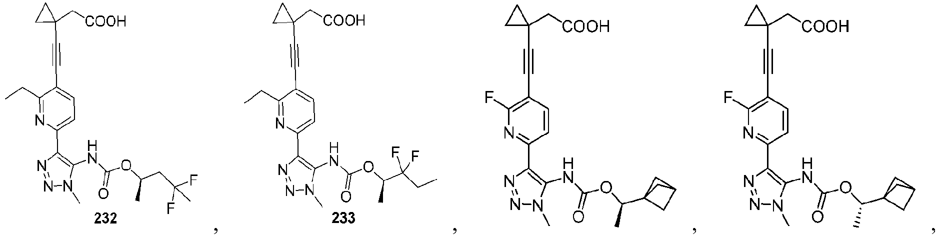 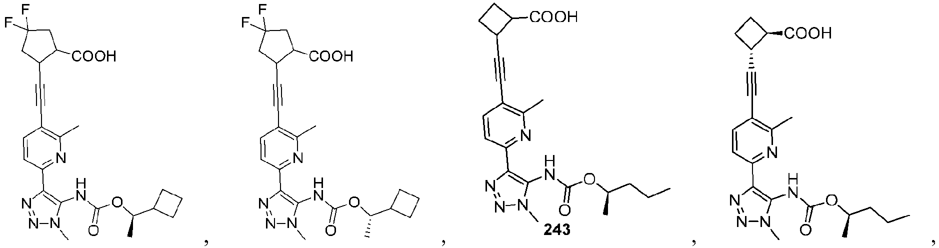 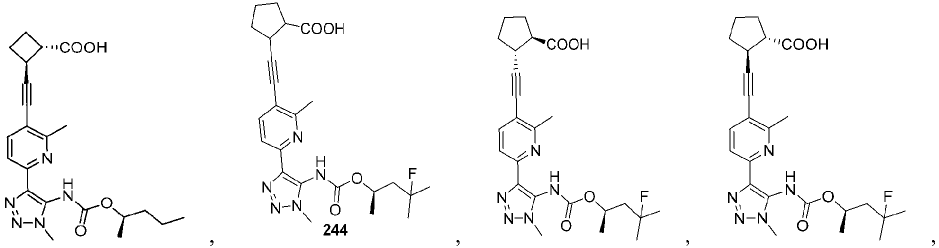 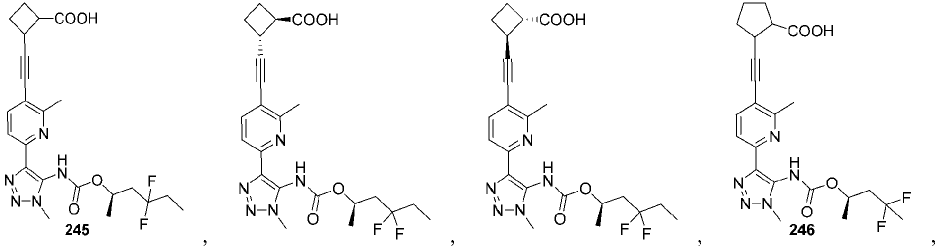 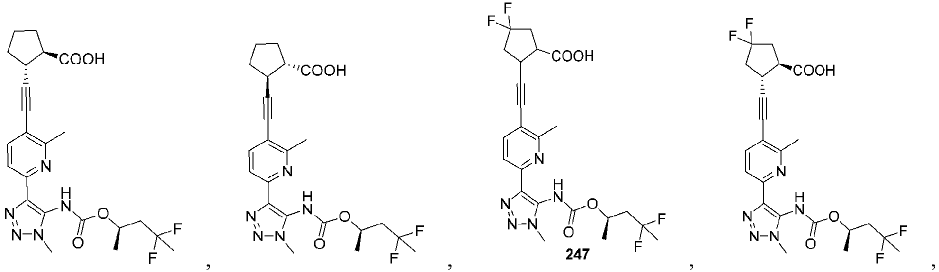 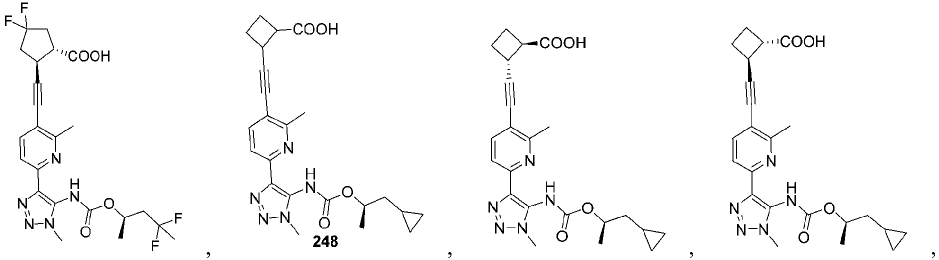 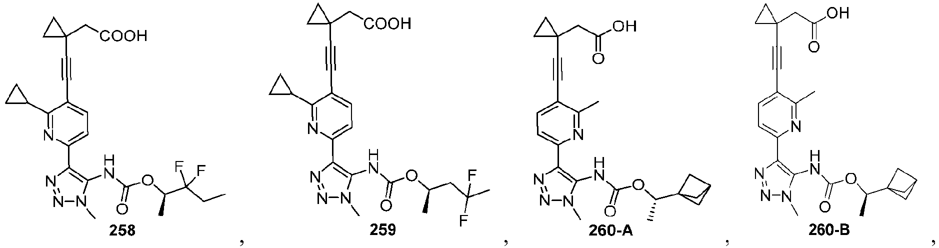 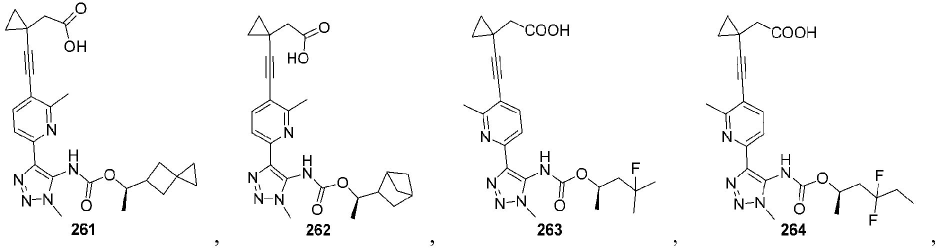 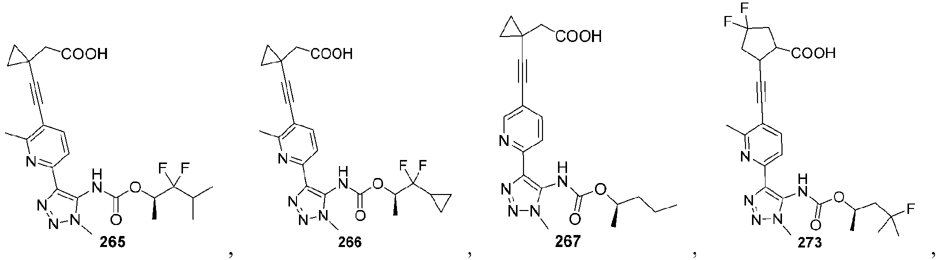 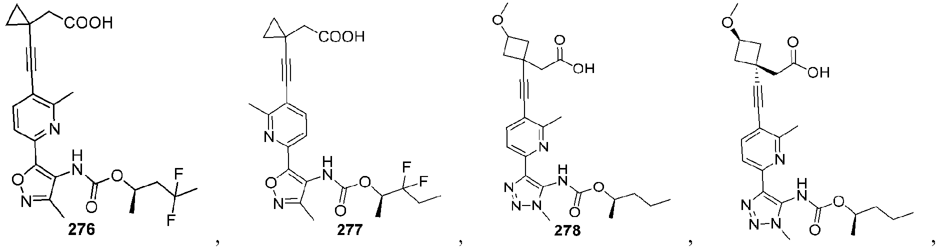 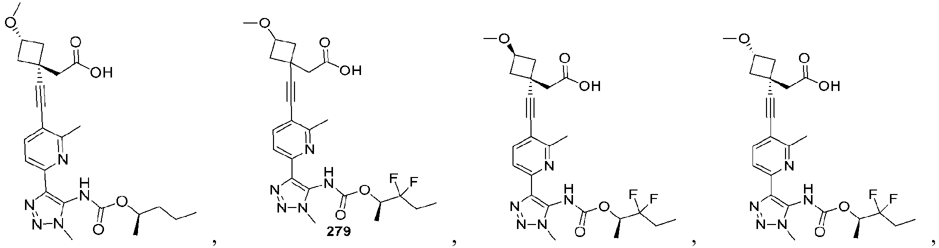 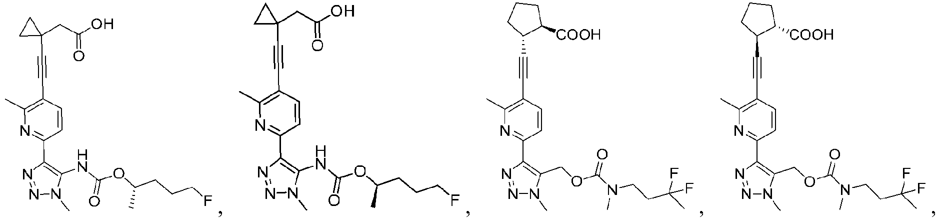 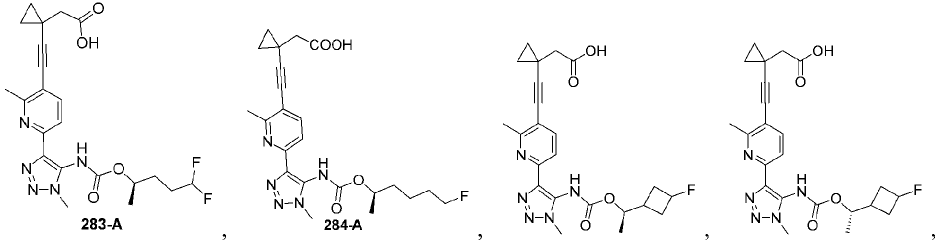 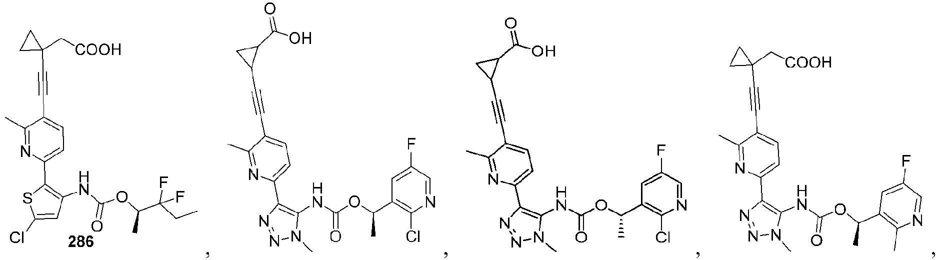 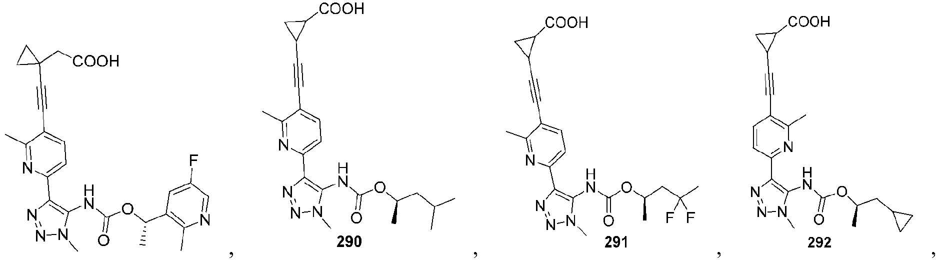 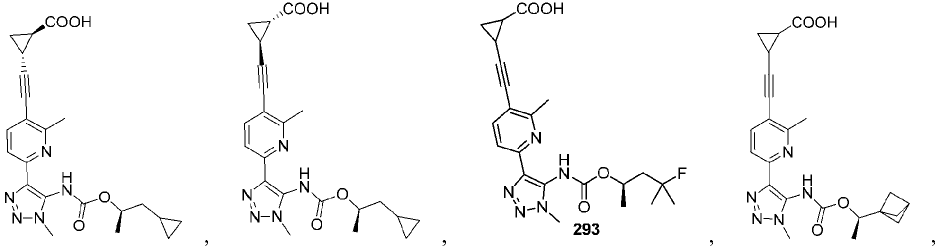 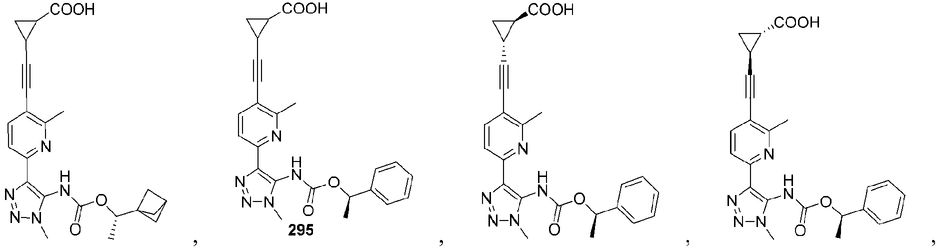 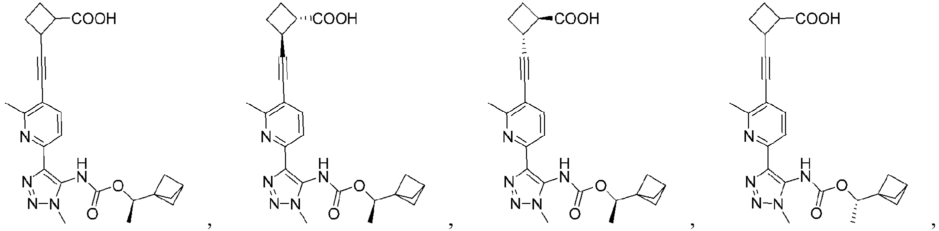 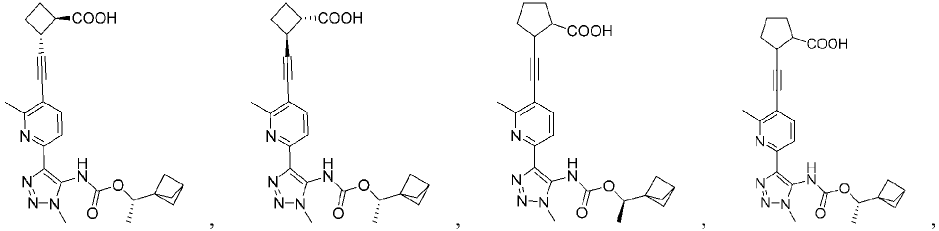 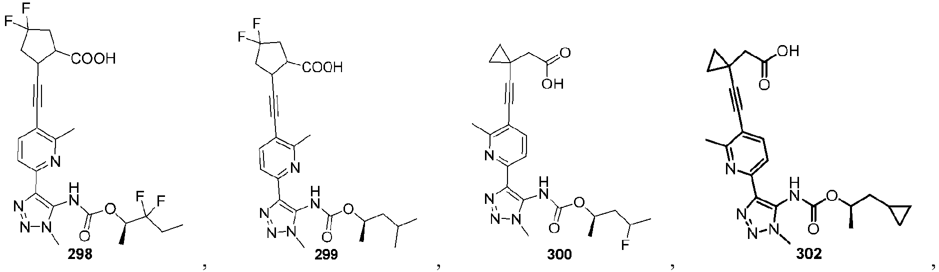 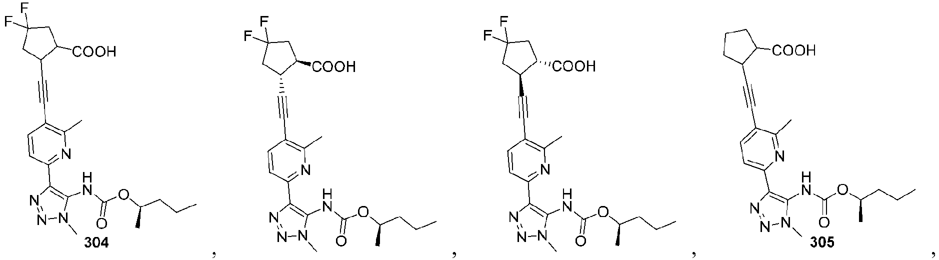 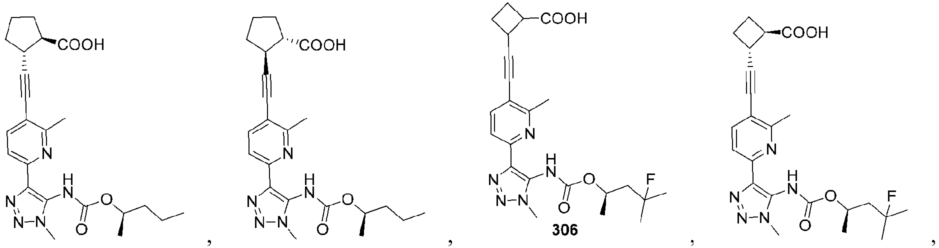 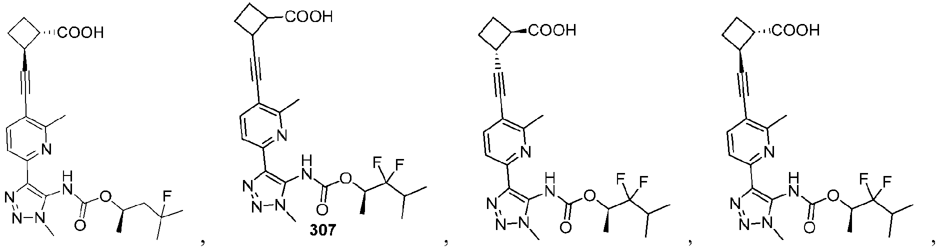 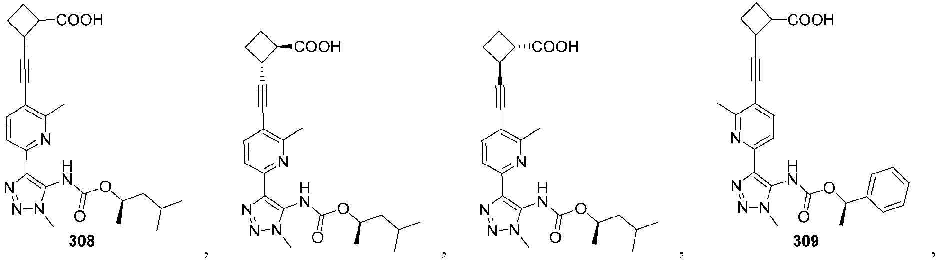 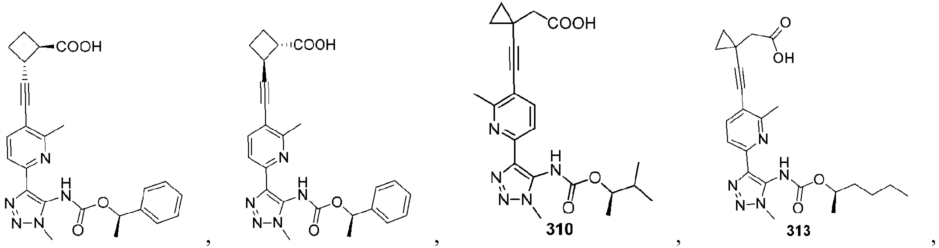 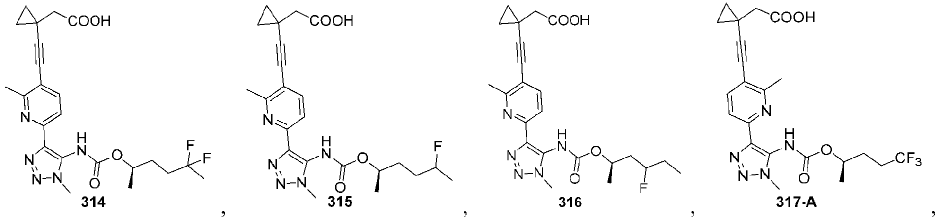 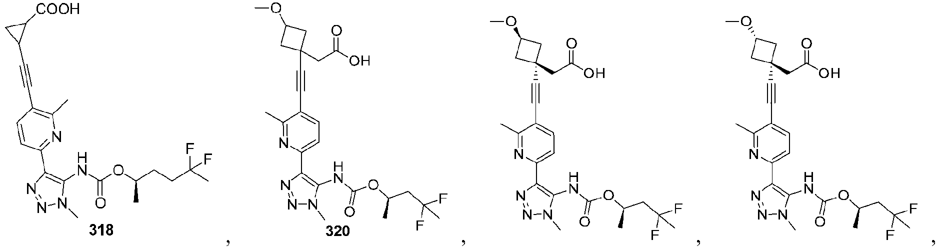 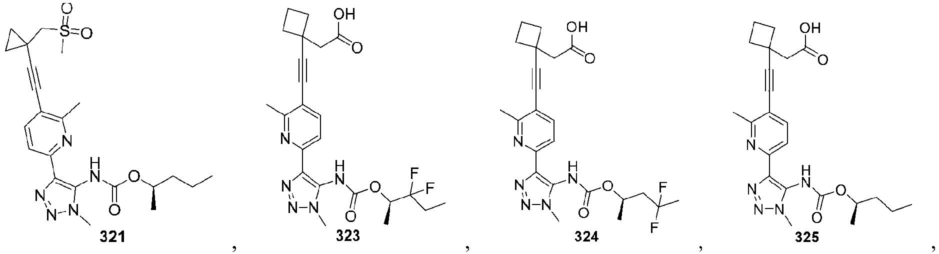 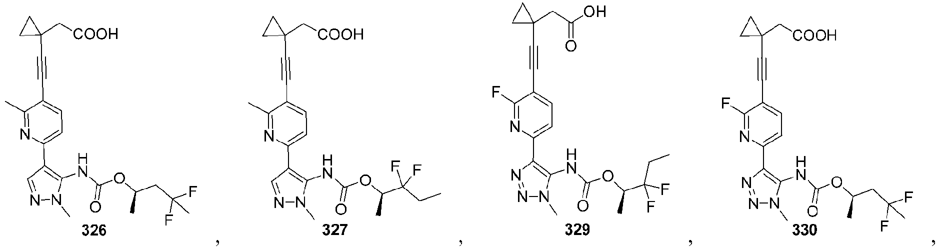 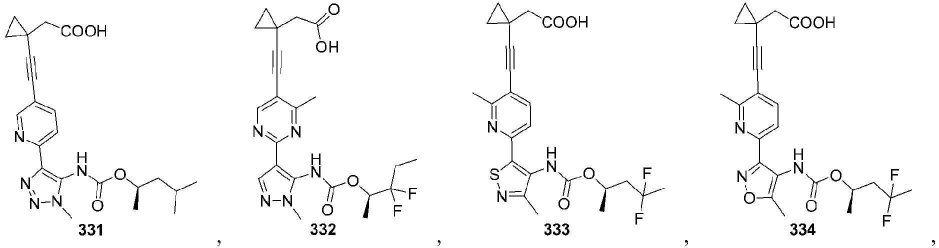 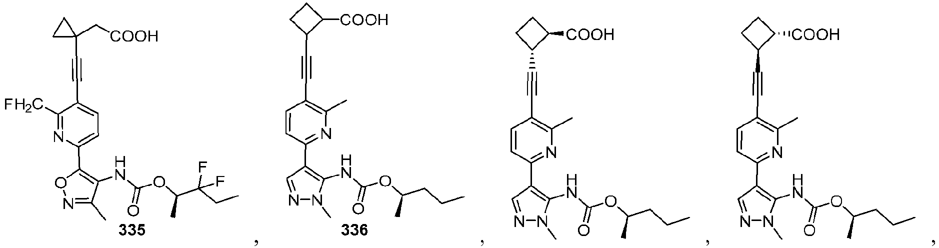 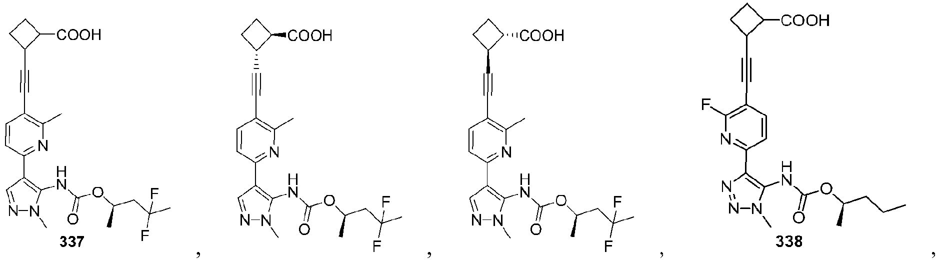 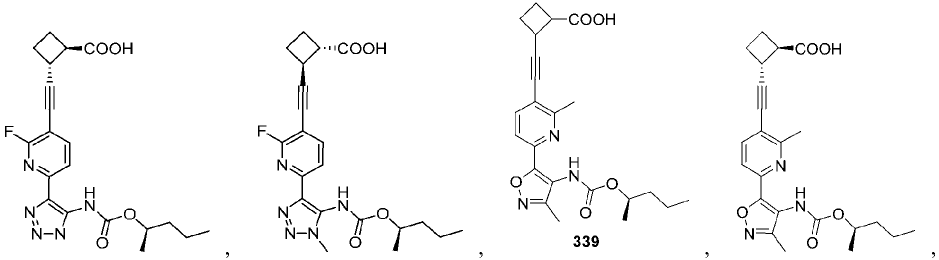 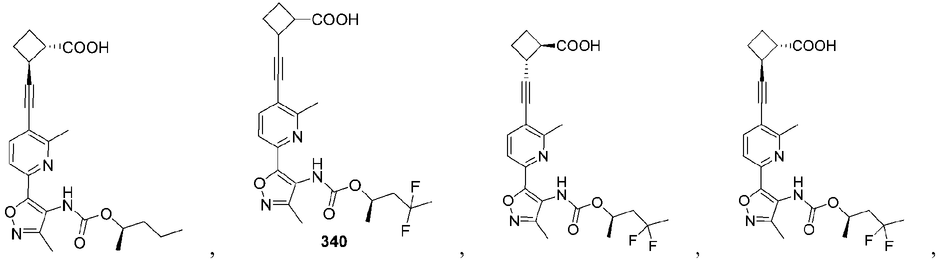 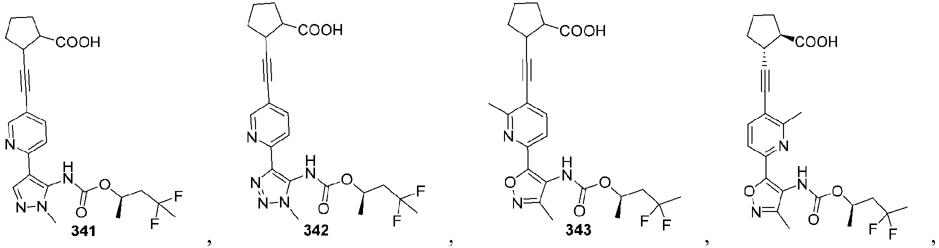 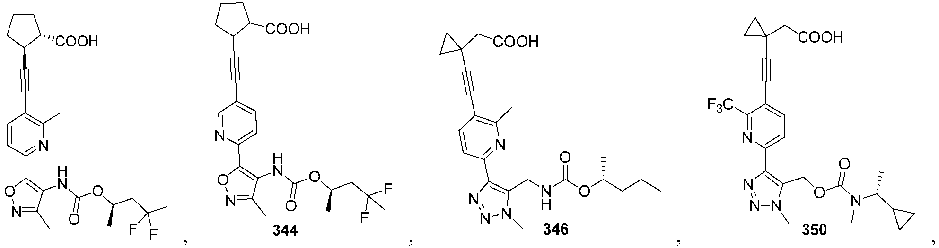 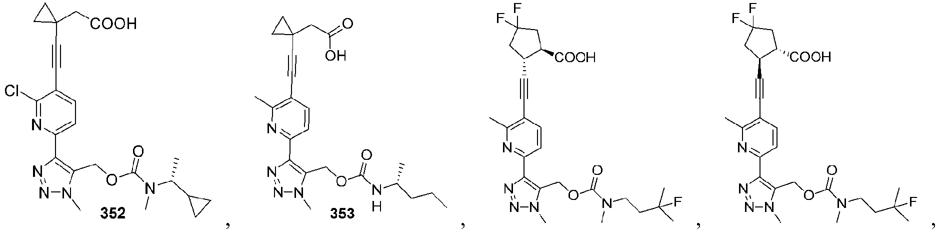 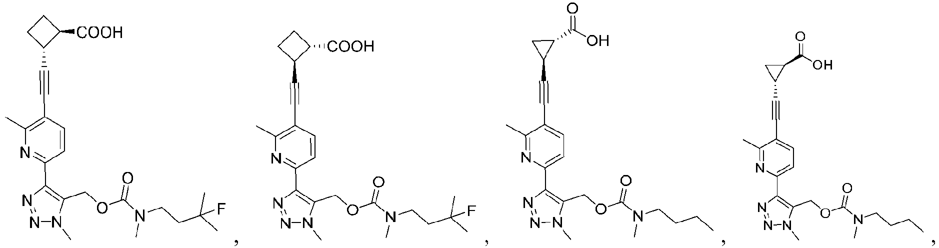 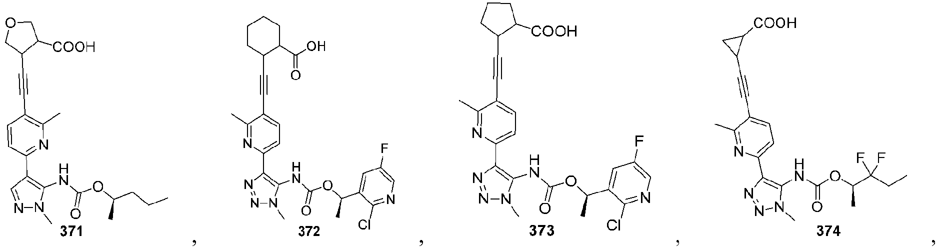 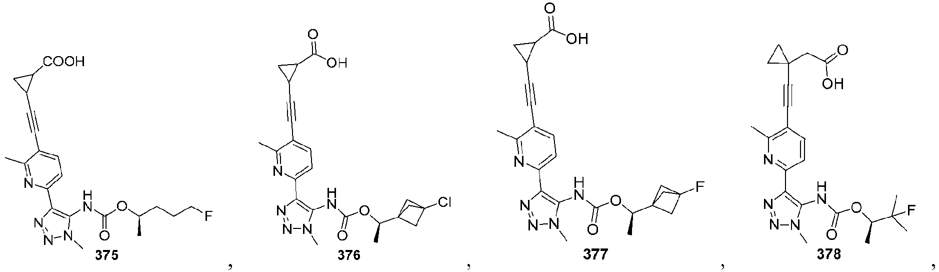 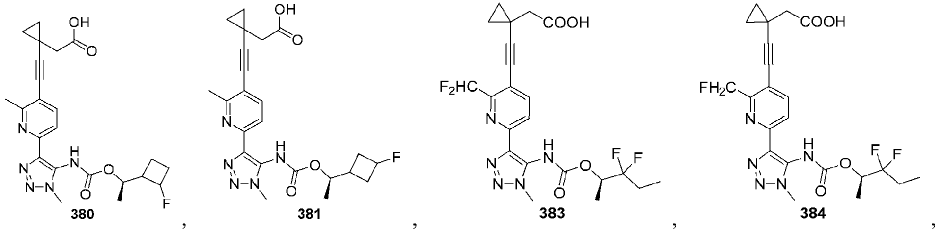 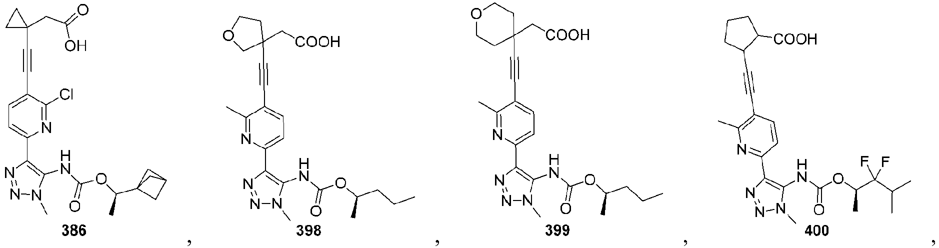 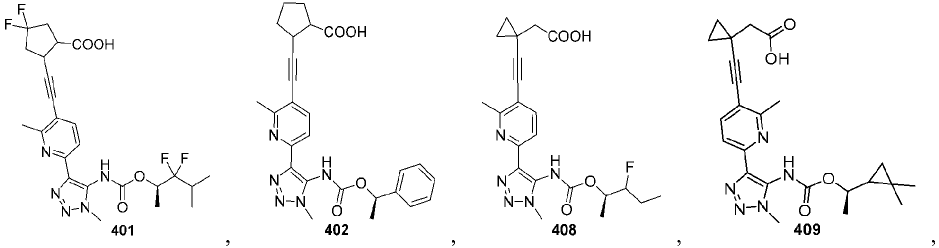 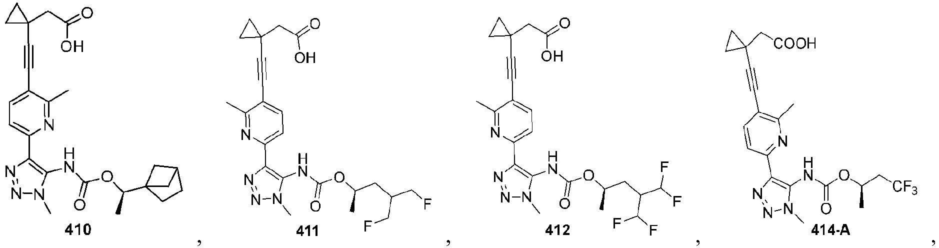 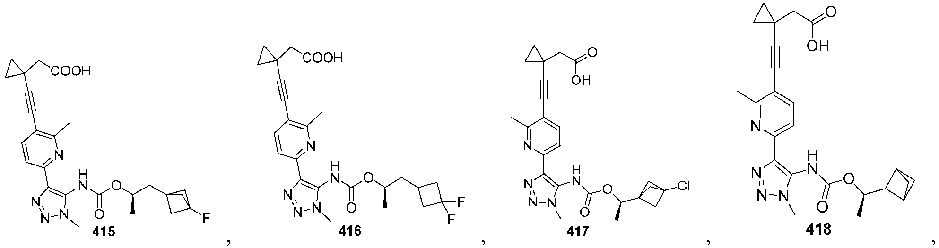 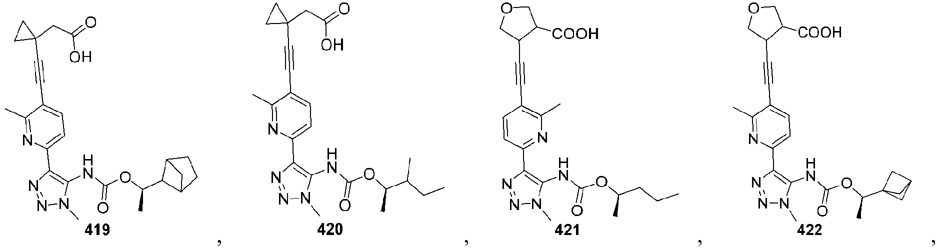 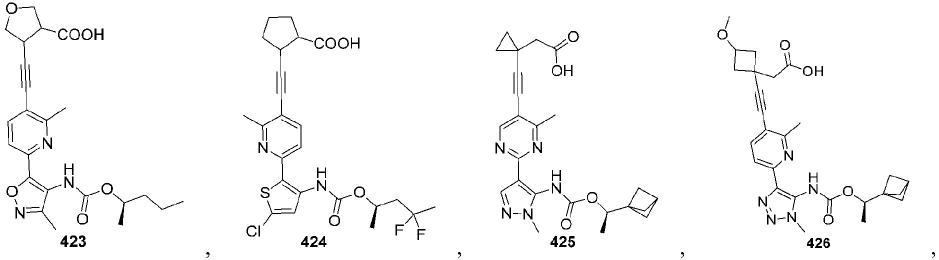 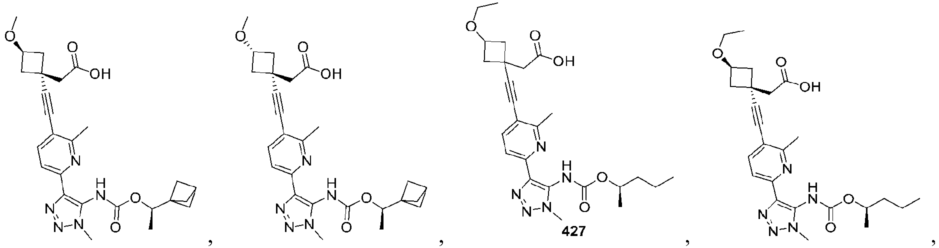 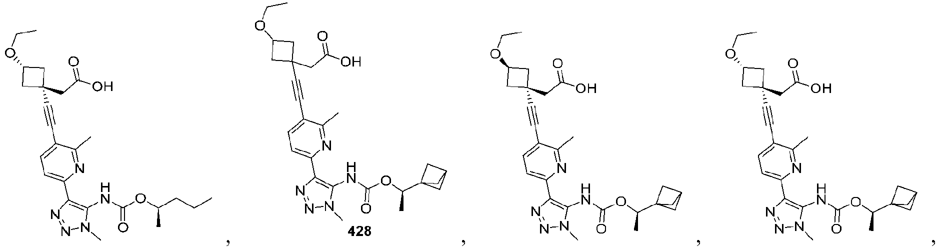 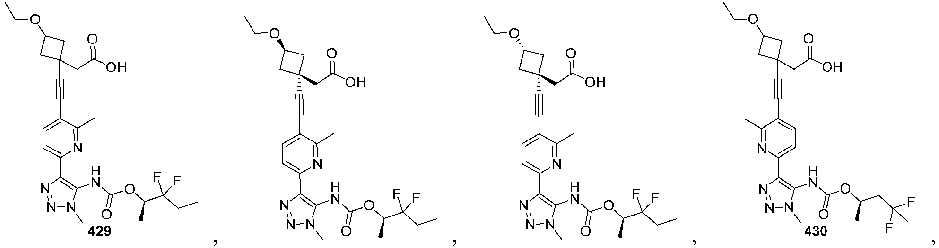 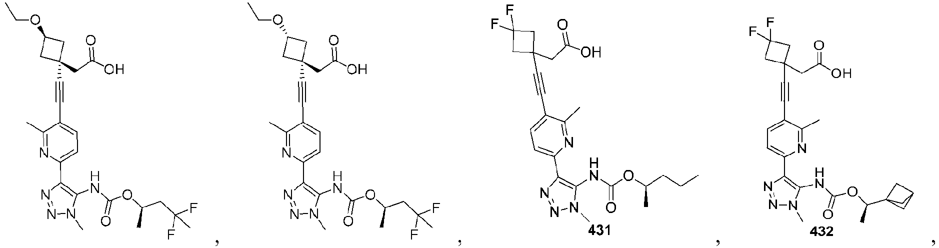 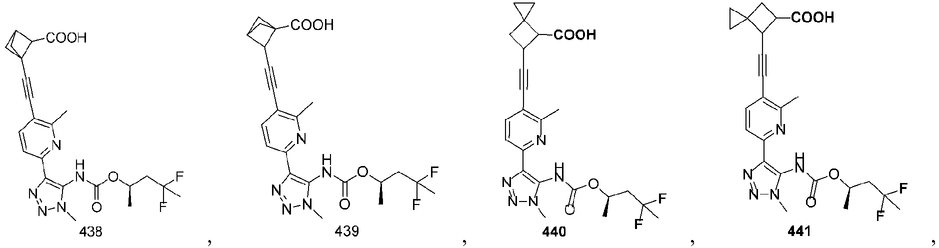 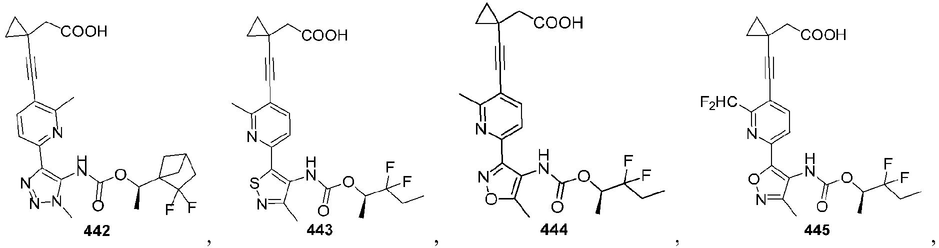 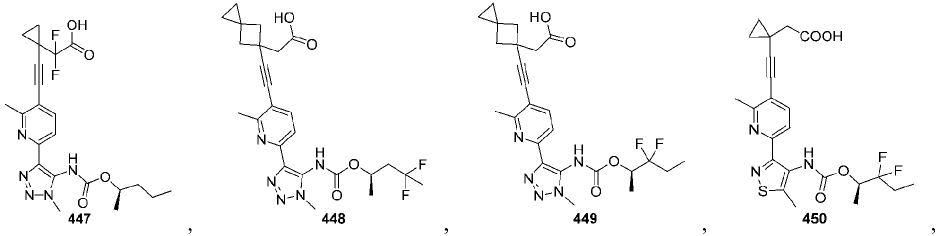 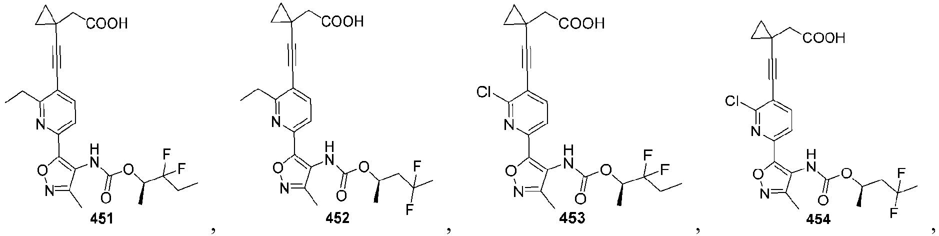 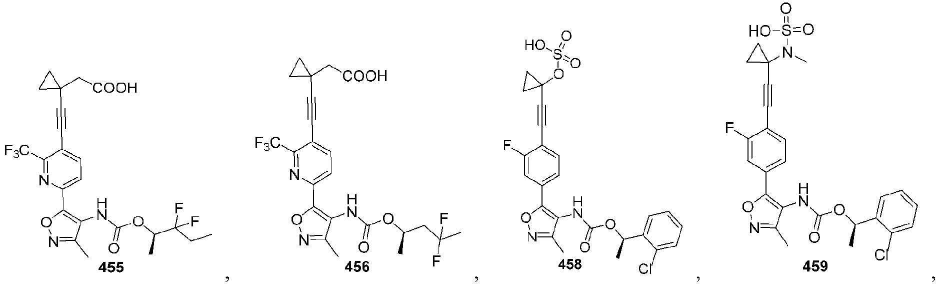 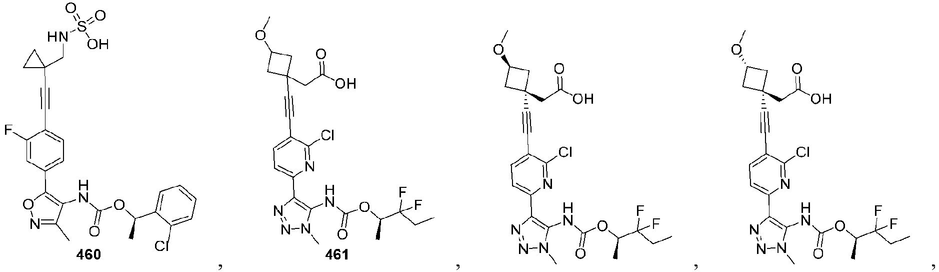 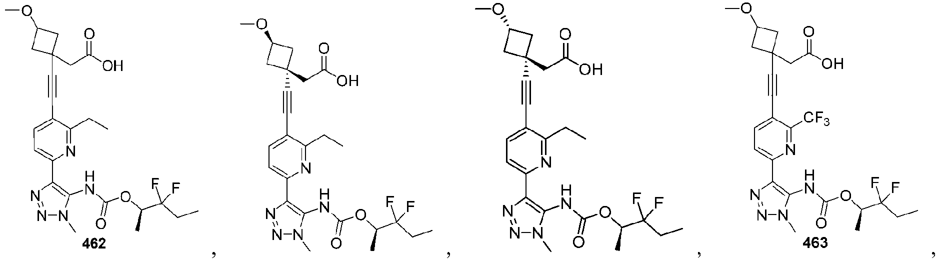 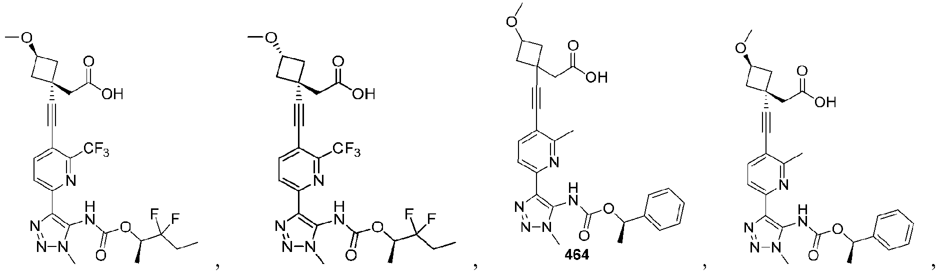 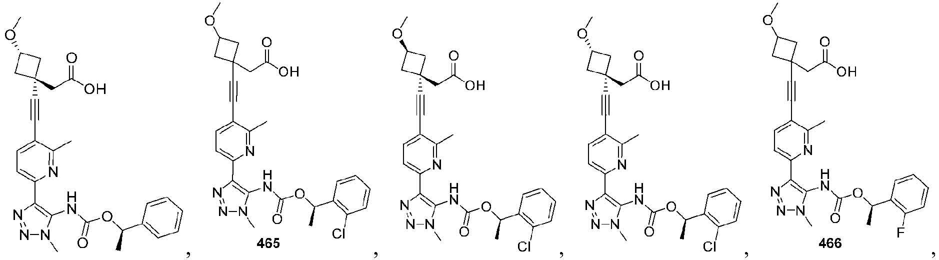 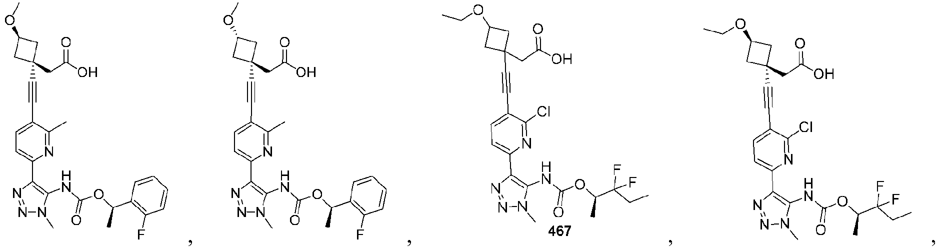 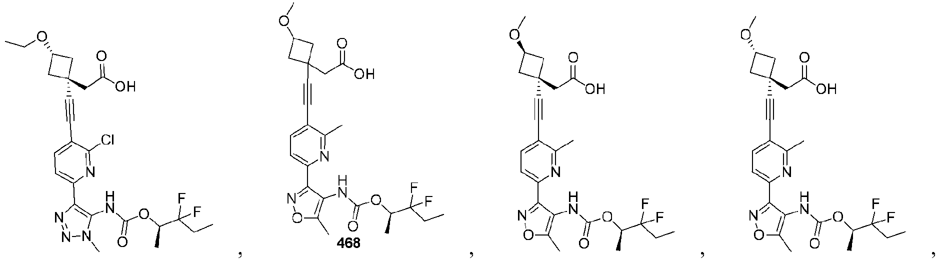 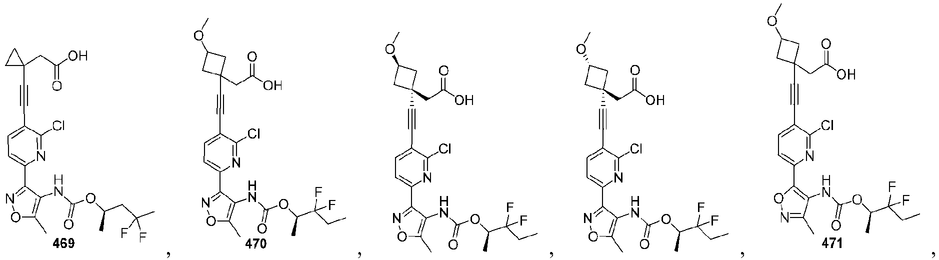 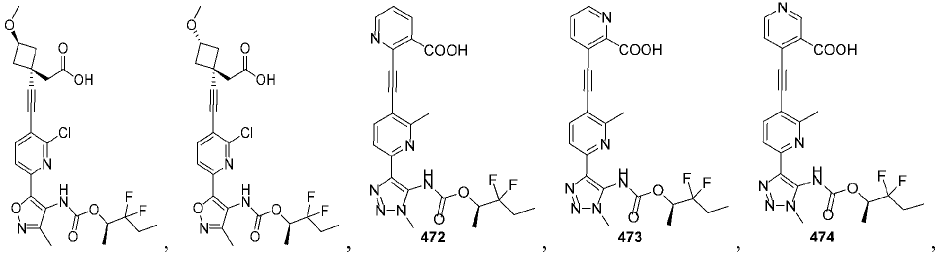 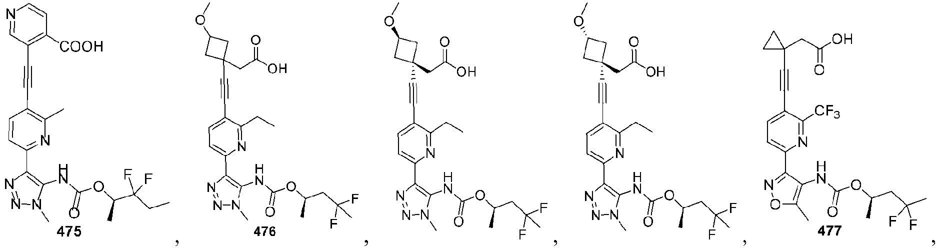 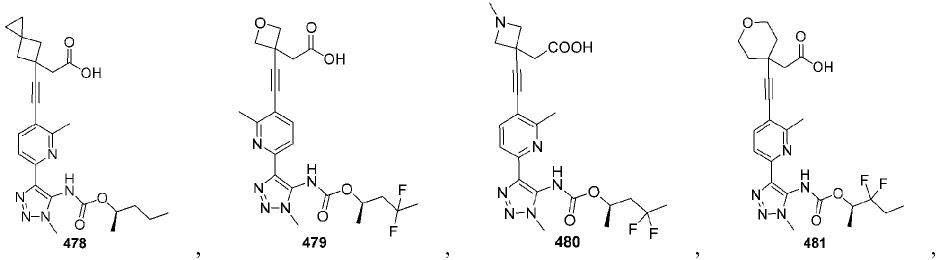 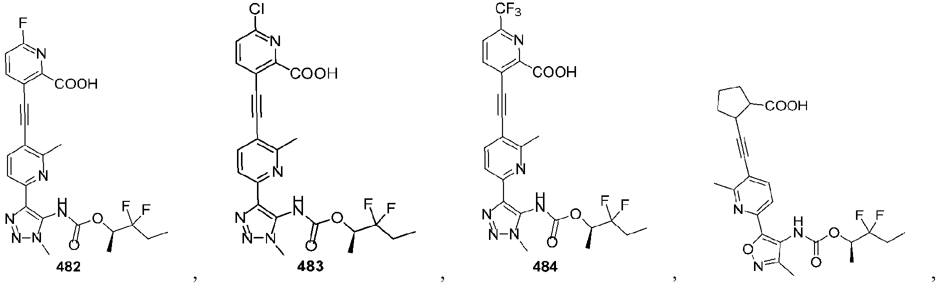 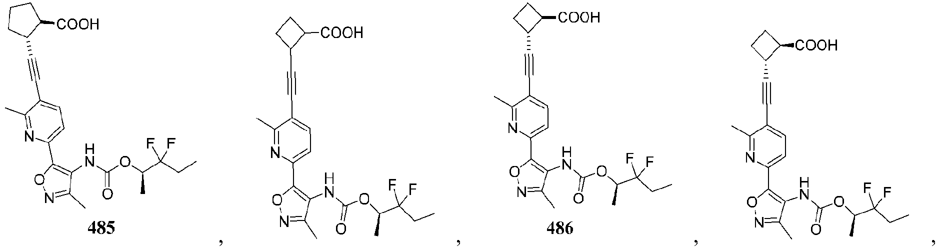 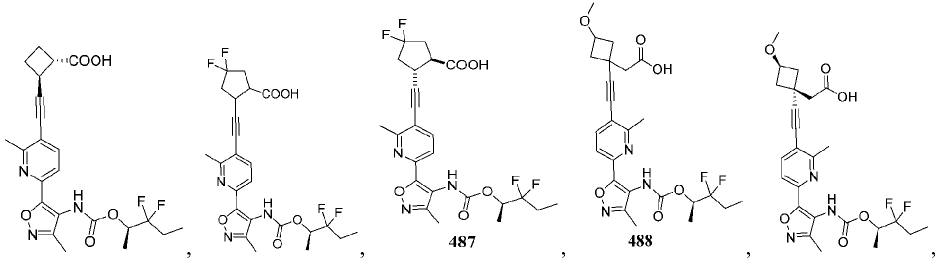 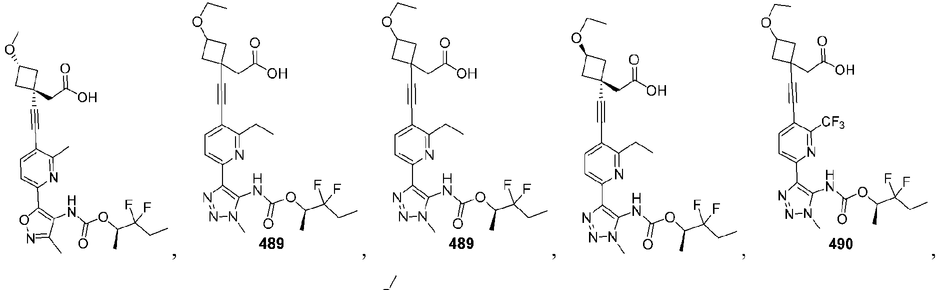 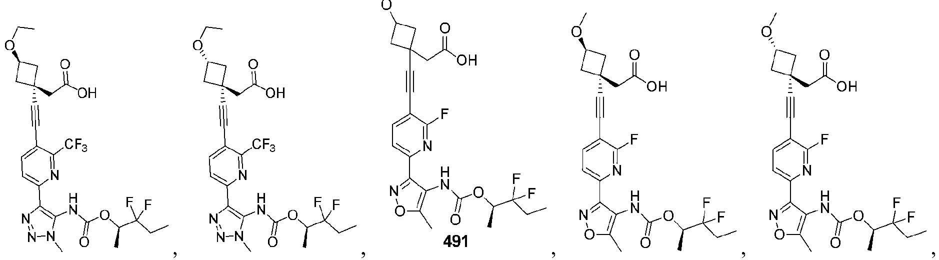 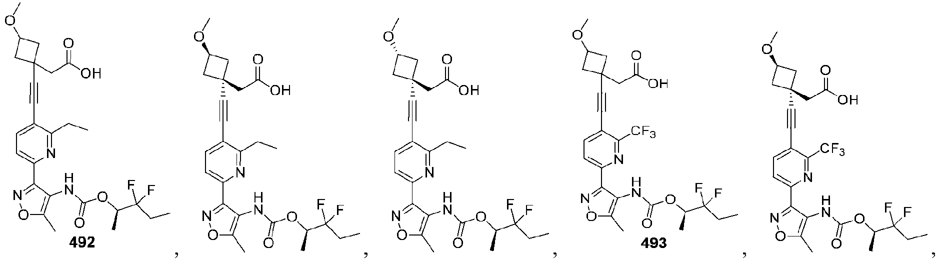 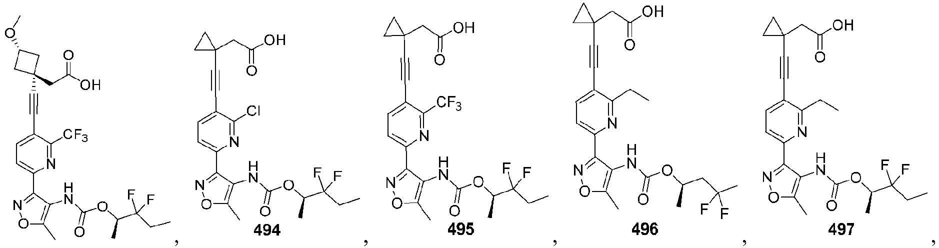 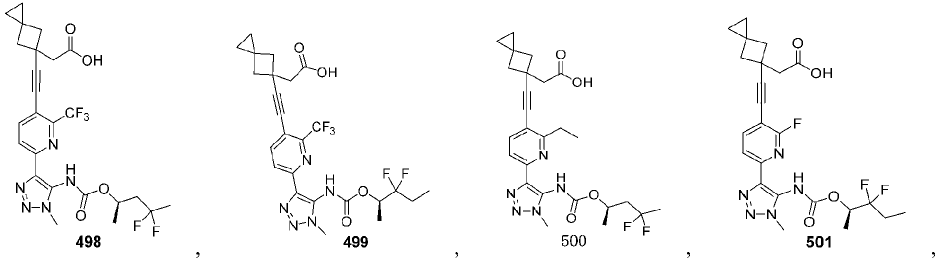 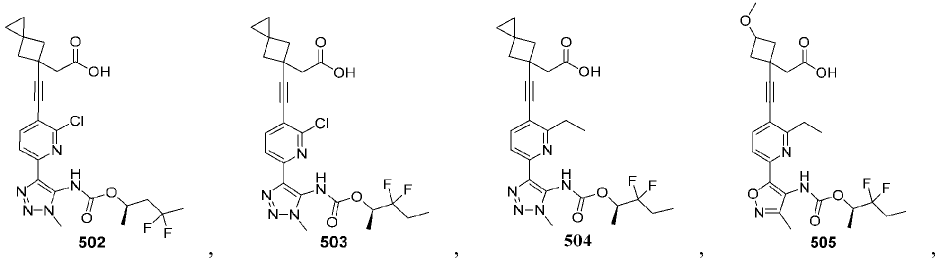 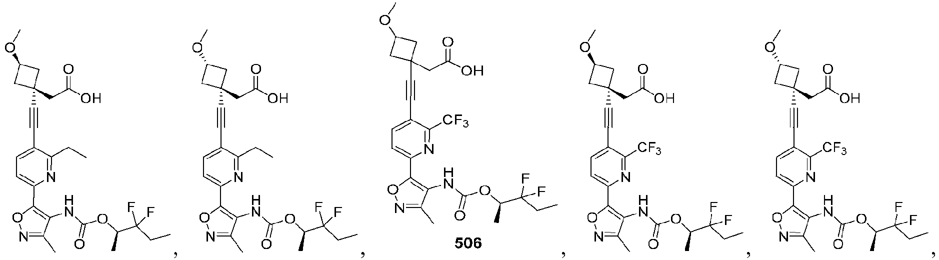 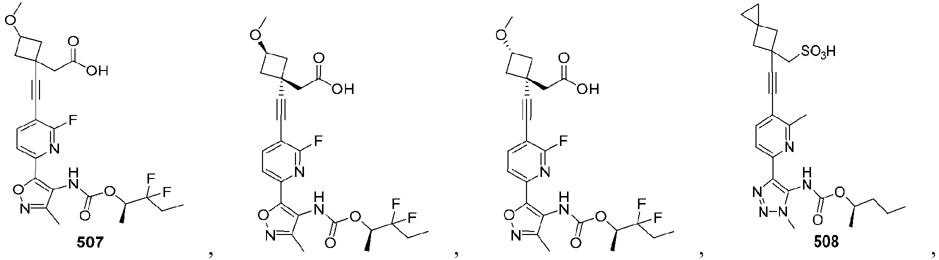 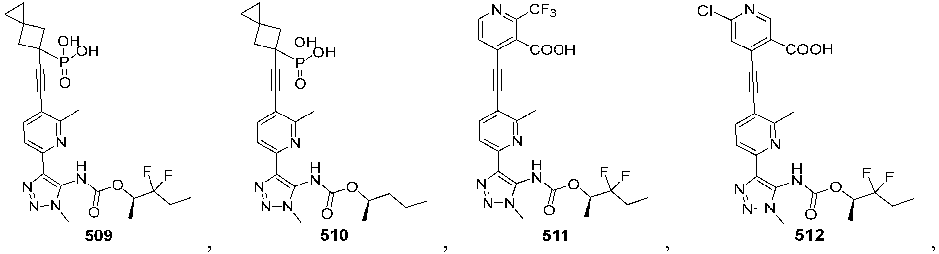 or

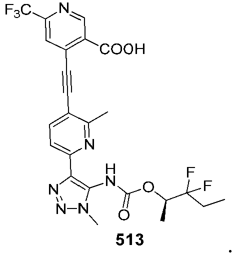

14. A preparation method for a compound of formula (I), wherein the method is the following method 1, method 2, method 3, or method 4:
method 1: subjecting a compound of formula I'-1A to an ester hydrolysis reaction in a solvent in the presence of a base to obtain the compound of formula (I); 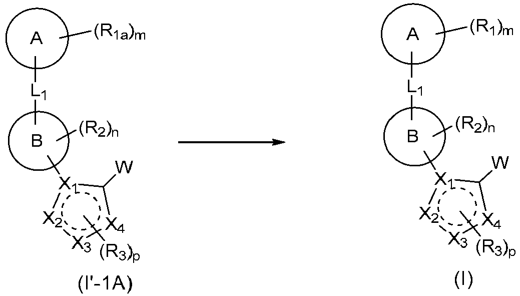
each R₁ₐ is C₁₋₆ alkyl substituted with 1, 2, or more R₁ₐ₋₁ substituents or -C(=O)O-C₁₋₆ alkyl; each R₁ₐ₋₁ is -C(=O)O-C₁₋₆ alkyl; each R₁ is C₁₋₆ alkyl substituted with 1, 2, or more COOH substituents or COOH; m is 1, 2, 3, 4, or 5;
ring A, ring B, L₁, R₂, X₁, X₂, X₃, X₄, R₃, W, m, n, and p are as defined in any one of claims 1 to 13;
method 2: subjecting a compound of formula I'-1B and a compound of formula I'-1C to a reaction as shown in the following formula in a solvent in the presence of a base, phosphine ligand, and catalyst to obtain the compound of formula (I); 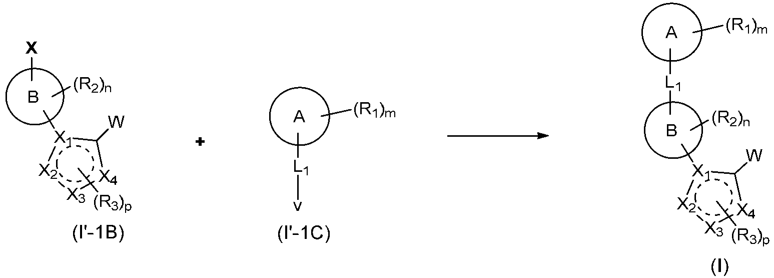
X is halogen; ring A, ring B, R₁, L₁, R₂, X₁, X₂, X₃, X₄, R₃, W, m, n, and p are as defined in any one of claims 1 to 13; V is H, TMS, or TBDMS;
method 3: subjecting a compound of formula I'-1D to a reaction in a solvent in the presence of a cyanide and catalyst to obtain the compound of formula (I); 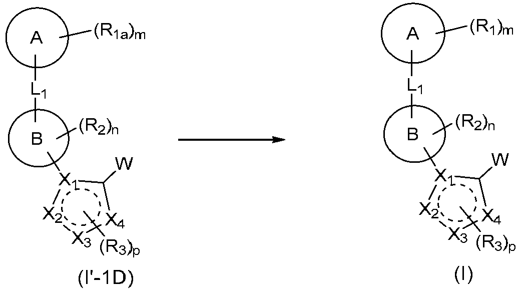
the cyanide is trimethylsilyl cyanide, potassium cyanide, or sodium cyanide;
each R₁ₐ is C₁₋₆ alkyl substituted with 1, 2, or more -OM substituents; each R₁ is C₁₋₆ alkyl substituted with 1, 2, or more cyano substituents or COOH; M is methanesulfonyl (Ms), p-toluenesulfonyl (Ts), or *p-*nitrobenzenesulfonyl (Ns);
ring A, ring B, L₁, R₂, X₁, X₂, X₃, X₄, R₃, W, m, n, and p are as defined in any one of claims 1 to 13;
method 4:
step 1) subjecting a compound of formula I'-1E with a compound of formula I'-1F or a salt thereof to a reaction in the presence of an acylation reagent to obtain a compound of formula I'-1G;
step 2) subjecting the compound of formula I'-1G to an ester hydrolysis reaction in a solvent in the presence of a base to obtain a compound of formula (I-I'); 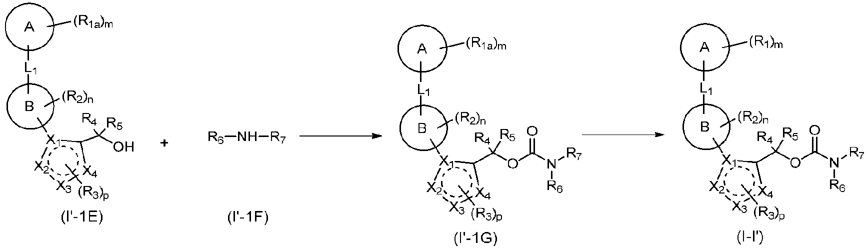
each R₁ₐ is C₁₋₆ alkyl substituted with 1, 2, or more R₁ₐ₋₁ substituents or -C(=O)O-C₁₋₆ alkyl; each R₁ₐ₋₁ is -C(=O)O-C₁₋₆ alkyl; each R₁ is C₁₋₆ alkyl substituted with 1, 2, or more COOH substituents or COOH; m is 1, 2, 3, 4, or 5;
ring A, ring B, L₁, R₂, X₁, X₂, X₃, X₄, R₃, R₄, R₅, R₆, R₇, m, n, and p are as defined in any one of claims 1 to 13.

15. A pharmaceutical composition comprising substance U and a pharmaceutical excipient, wherein substance U is the compound of formula (I), the racemate thereof, the stereoisomer thereof, the tautomer thereof, the N-oxide thereof, or the pharmaceutically acceptable salt of any one of the foregoing as defined in any one of claims 1 to 13.

16. Use of substance U or the pharmaceutical composition as defined in claim 15 in the manufacture of an LPAR1 antagonist, wherein substance U is the compound of formula (I), the racemate thereof, the stereoisomer thereof, the tautomer thereof, the N-oxide thereof, or the pharmaceutically acceptable salt of any one of the foregoing as defined in any one of claims 1 to 13.

17. Use of substance U or the pharmaceutical composition as defined in claim 15 in the manufacture of a medicament; wherein substance U is the compound of formula (I), the racemate thereof, the stereoisomer thereof, the tautomer thereof, the N-oxide thereof, or the pharmaceutically acceptable salt of any one of the foregoing as defined in any one of claims 1 to 13;
preferably, the medicament is a medicament for diagnosing, preventing, and/or treating a disease or condition mediated by an LPAR receptor;
preferably, the medicament is an LPAR1 antagonist;
preferably, the disease or condition is a fibrotic disease, a respiratory disease, pain, a neurological disease, a cardiovascular or cerebrovascular disease, an inflammatory disease, a kidney disease, a liver disease, an ocular disease, a cancer, a gastrointestinal disease, a urinary system disease, a metabolic disease, or transplant rejection;
preferably, the disease or condition is pulmonary fibrosis (e.g., idiopathic pulmonary fibrosis, progressive pulmonary fibrosis), renal fibrosis, hepatic fibrosis, skin fibrosis, intestinal fibrosis, ocular fibrosis, cardiac fibrosis, pancreatic fibrosis, interstitial lung disease, idiopathic interstitial pneumonia, asthma, chronic obstructive pulmonary disease, bronchospasm, cough, chronic cough, respiratory failure, silicosis, acute lung injury, acute respiratory distress, acute kidney injury, chronic kidney disease, diabetic nephropathy, alcoholic steatohepatitis, non-alcoholic fatty liver disease (e.g., non-alcoholic steatohepatitis), acute hepatitis, chronic hepatitis, cirrhosis, hepatic insufficiency, primary biliary cirrhosis, autoimmune disease, inflammation, arthritis, rheumatoid arthritis, scleroderma, Raynaud's phenomenon, chronic pruritus, lupus, cryptogenic fibrosing alveolitis, psoriasis, systemic sclerosis, collagen vascular disease, Alzheimer's disease, Parkinson's disease, neurodegenerative disease, traumatic brain injury, epilepsy, mental illness, sleep disorder, collagen vascular disease, myocardial infarction, stroke, thrombosis, atherosclerosis, heart failure, hypertension, irritable bowel syndrome, inflammatory bowel disease, digestive tract disease, gastrointestinal dysfunction, cancer pain, neuropathic pain, inflammatory pain, surgical pain, visceral pain, toothache, premenstrual pain, central pain, pain caused by burns, migraine, cluster headache, chronic pain, urinary incontinence, dysuria, cystitis, prostatic hyperplasia, urinary disturbance associated with prostatic hyperplasia, bladder neck sclerosis, underactive bladder, macular degeneration, diabetic retinopathy, breast cancer, pancreatic cancer, ovarian cancer, prostate cancer, glioblastoma, bone cancer, colon cancer, intestinal cancer, liver cancer, head and neck cancer, melanoma, multiple myeloma, chronic lymphocytic leukemia, tumor metastasis, or osteoporosis;
preferably, the disease or condition is interstitial lung disease, pulmonary fibrosis (particularly idiopathic pulmonary fibrosis), hepatic fibrosis, renal fibrosis, non-alcoholic fatty liver disease (e.g., non-alcoholic steatohepatitis), psoriasis, or scleroderma.
